(19) Europäisches Patentamt
European Patent Office
Office européen des brevets

(11) EP 3 724 205 B1

(12) **EUROPEAN PATENT SPECIFICATION**

(45) Date of publication and mention of the grant of the patent:
**22.06.2022 Bulletin 2022/25**

(21) Application number: **18830620.3**

(22) Date of filing: **14.12.2018**

(51) International Patent Classification (IPC):
***C07H 21/02*** *(2006.01)* ***A61K 31/7084*** *(2006.01)*
***C07H 21/04*** *(2006.01)* ***A61P 31/00*** *(2006.01)*
***A61P 31/12*** *(2006.01)* ***A61P 35/00*** *(2006.01)*

(52) Cooperative Patent Classification (CPC):
**C07H 21/02; A61K 31/7084; C07H 21/04**

(86) International application number:
**PCT/US2018/065677**

(87) International publication number:
**WO 2019/118839 (20.06.2019 Gazette 2019/25)**

(54) **CYCLIC DINUCLEOTIDES AS STING AGONISTS**

CYCLISCHE DINUKLEOTIDE ALS STING-AGONISTEN

DINUCLÉOTIDES CYCLIQUES UTILISÉS EN TANT QU'AGONISTES DE STING

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2017 US 201762599111 P**

(43) Date of publication of application:
**21.10.2020 Bulletin 2020/43**

(73) Proprietor: **Janssen Biotech, Inc.**
**Horsham, PA 19044 (US)**

(72) Inventors:
- **BEIGELMAN, Leonid**
  **South San Francisco, California 94080 (US)**
- **BIGNAN, Gilles**
  **Spring House, Pennsylvania 19477 (US)**
- **CONNOLLY, Peter J.**
  **Spring House,**
  **PA 19477 (US)**
- **EDWARDS, James Patrick**
  **Spring House, Pennsylvania 19477 (US)**
- **EMANUEL, Stuart**
  **Spring House, Pennsylvania 19477 (US)**
- **LAQUERRE, Sylvia**
  **Spring House, Pennsylvania 19477 (US)**
- **RICHTER, Mark**
  **Spring House, Pennsylvania 19477 (US)**
- **SCHEPENS, Wim Gert Griet**
  **2340 Beerse (BE)**
- **THATIKONDA, Santhosh Kumar**
  **South San Francisco, California 94080 (US)**
- **THURING, Johannes Wilhelmus John Fitzgerald**
  **2340 Beerse (BE)**
- **VIELLEVOYE, Marcel**
  **2340 Beerse (BE)**
- **WANG, Guangyi**
  **South San Francisco, California 94080 (US)**
- **ZHONG, Minghong**
  **San Francisco, California 94080 (US)**

(74) Representative: **Mitchell, Simon James**
**Murgitroyd & Company London**
**Euston House**
**24 Eversholt Street**
**London NW1 1AD (GB)**

(56) References cited:
**WO-A1-2017/161349 WO-A1-2020/016782**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 724 205 B1

**Description**

CROSS-REFERENCE TO RELATED APPLICATIONS

This Application claims priority to United States Provisional Patent Application No. 62/599,111, filed December 15, 2017.

FIELD OF THE INVENTION

The present invention relates to novel compounds which are STING (Stimulator of Interferon Genes) agonists and are useful for the treatment of disorders that are affected by the modulation of the STING protein. The invention also relates to pharmaceutical compositions comprising one or more of such compounds, processes to prepare such compounds and compositions, and use of such compounds or pharmaceutical compositions for the treatment of various diseases, syndromes and disorders. The invention may be involved in the activation of the downstream signaling pathway, further resulting in the activation of second messengers and growth factors, and the production of interferon involved in the innate and adaptive immunity. More particularly, the present invention relates to the use of such compounds or pharmaceutical compositions for the treatment of various infections, diseases, syndromes and disorders including, but not limited to, melanoma, colon cancer, breast cancer, prostate cancer, lung cancer, fibrosarcoma, and antiviral therapy.

BACKGROUND OF THE INVENTION

STING (stimulator of interferon genes), also known as TMEM173, MITA, MPYS, and ERIS, is a transmembrane receptor located inside the cell and a key sensor of cytosolic nucleic acids (Zhong B, et al. "The Adaptor Protein MITA Links Virus-Sensing Receptors to IRF3 Transcription Factor Activation". Immunity. 2008. vol. 29: 538-550). Recent studies have revealed the biology of STING and its role in mobilizing an innate immune response resulting in robust antitumor activity in mouse models. Activation of the STING pathway results in production of Type I interferons (mainly IFN-$\alpha$ and IFN-$\beta$) induced through the IRF3 (interferon regulatory factor 3) pathway. Activation of IRF3 is thought to be mediated by TBK1 that recruits and phosphorylates IRF3 thus forming an IRF3 homodimer capable of entering the nucleus to transcribe type I interferon and other genes (Liu S, et al. "Phosphorylation of innate immune adaptor proteins MAVS, STING, and TRIF induces IRF3 activation" Science. 2015: 2630-2637). TBK1 also activates the nuclear factor kappa-light-chain-enhancer of activated B cells pathway which leads to production of pro-inflammatory cytokines (IL-1$\alpha$, IL-1$\beta$, IL-2, IL-6, TNF-$\alpha$, etc.), via the oncogenic transcription factor NF-$_{K}$B. In addition, STING activates STAT6 (signal transducer and activator of transcription 6) to induce (Th2-type), increase (IL-12) or decrease (IL-10) production of various cytokines, including the chemokines CCL2, CCL20, and CCL26 (Chen H, et al. "Activation of STAT6 by STING Is Critical for Antiviral Innate Immunity" Cell. 2011, vol.14: 433-446). Direct phosphorylation of STING on Ser366 upon activation has also been reported to occur through TBK1 or ULK1 (Corrales, L. et al "Direct activation of STING in the tumor microenvironment leads to potent and systemic tumor regression and immunity" Cell Reports, 2015, vol.11: 1-13; Konno, H. et al. "Cyclic dinucleotides trigger ULK1 (ATG1) phosphorylation of STING to prevent sustained innate immune signaling" Cell, 2013, vol. 155: 688-698).

The natural ligand that binds to and activates STING (2',3')cyclic guanosine monophosphate-adenosine monophosphate (2',3'-cGAMP) and the enzyme responsible for its synthesis (cGAS, also known as C6orf150 or MB21D1) have been elucidated providing an opportunity to modulate this pathway. cGAMP is a high affinity ligand for STING produced in mammalian cells that serves as an endogenous second messenger to activate the STING pathway. It is a cyclic dinucleotide with a unique 2',3' linkage produced by cGAS in the presence of exogenous double-stranded DNA (e.g. that released by invading bacteria, viruses or protozoa) or of self-DNA in mammals (Wu et al., 2013; Sun, L. et al. "Cyclic GMP-AMP Synthase Is a Cytosolic DNA Sensor That Activates the Type I Interferon Pathway" Science, 2013, vol. 339: 786-791; Bhat N and Fitzgerald KA. "Recognition of Cytosolic DNA by cGAS and other STING-dependent sensors". Eur J Immunol. 2014 Mar; 44(3):634-40). STING activation can also occur through binding of exogenous (3',3) cyclic dinucleotides (c-di-GMP, c-di-AMP and 3'3'-cGAMP) that are released by invading bacteria (Zhang X, et al. "Cyclic GMP-AMP Containing Mixed Phosphodiester Linkages Is An Endogenous High-Affinity Ligand for STING" Molecular Cell, 2013, vol. 51: 226-235; Danilchanka, O and Mekalanos, JJ. "Cyclic Dinucleotides and the Innate Immune Response" Cell. 2013. vol. 154: 962-970).

Activation of the STING pathway triggers an immune response that results in generation of specific killer T-cells that can shrink tumors and provide long lasting immunity so they do not recur. The striking antitumor activity obtained with STING agonists in preclinical models has generated a high level of excitement for this target and small molecule compounds that can modulate the STING pathway have potential to treat both cancer and reduce autoimmune diseases.

Activation of the STING pathway also contributes to an antiviral response. Loss-of-functional response, either at the cellular or organism level, demonstrates an inability to control viral load in the absence of STING. Activation of

the STING pathway triggers an immune response that results in antiviral and proinflammatory cytokines that combat the virus and mobilize the innate and adaptive arms of the immune system. Ultimately, long-lasting immunity is developed against the pathogenic virus. The striking antiviral activity obtained with STING agonists in preclinical models has generated a high level of excitement for this target and small molecule compounds that can modulate the STING pathway have potential to treat chronic viral infections, such as hepatitis B.

Chronic hepatitis B virus (HBV) infection is a significant global health problem, affecting over 5% of the world population (over 350 million people worldwide and 1.25 million individuals in the U.S.). Despite the availability of certain HBV vaccines and therapies, the burden of chronic HBV infection continues to be a significant unmet worldwide medical problem due to suboptimal treatment options and sustained rates of new infections in most parts of the developing world. Current treatments are limited to only two classes of agents: interferon alpha and nucleoside analogues acting as inhibitors of the viral polymerase. Yet none of these therapies offer a cure to the disease, and drug resistance, low efficacy, and tolerability issues limit their impact. The low cure rates of HBV are attributed at least in part to the fact that complete suppression of virus production is difficult to achieve with a single antiviral agent However, persistent suppression of HBV DNA slows liver disease progression and helps to prevent hepatocellular carcinoma. Current therapy goals for HBV-infected patients are directed to reducing serum HBV DNA to low or undetectable levels, and to ultimately reducing or preventing the development of cirrhosis and hepatocellular carcinoma. There is, therefore, a need in the art for therapeutic agents that can increase the suppression of virus production and that can treat, ameliorate, or prevent HBV infection. Administration of such therapeutic agents to an HBV infected patient, either as monotherapy or in combination with other HBV treatments or ancillary treatments, may lead to significantly reduced virus burden, improved prognosis, diminished progression of the disease and enhanced seroconversion rates.

The potential therapeutic benefits of enhancing both innate and adaptive immunity make STING an attractive therapeutic target that demonstrates impressive activity by itself and can also be combined with other immunotherapies.

WO 2017/161349 and WO 2020/016782 A1 relate to cyclic dinucleotide compounds and methods of use.

SUMMARY OF THE INVENTION

The present invention provides a compound of Formula (I)

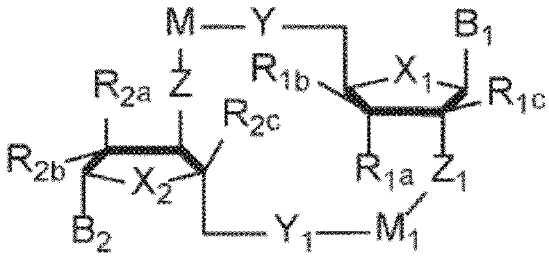

Formula (I)

which is a compound selected from the group consisting of the following compounds:

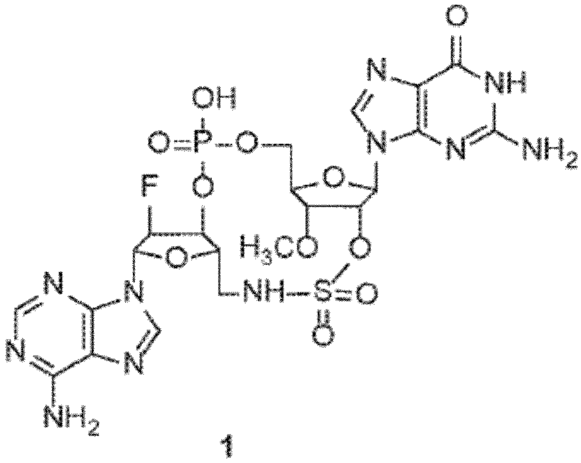

1

,

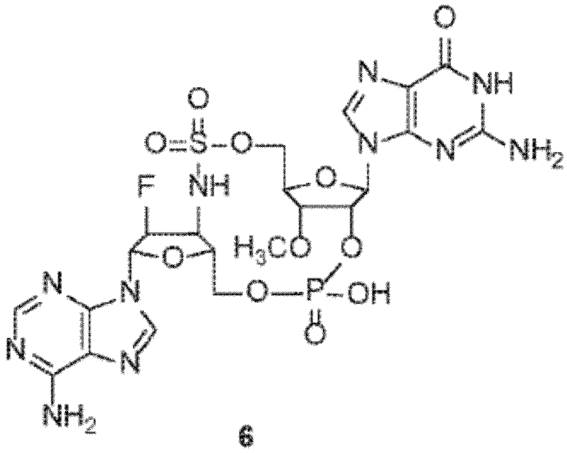

6

,

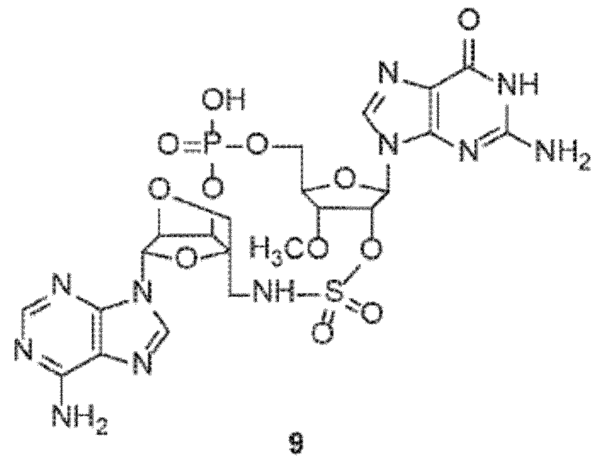

9

,

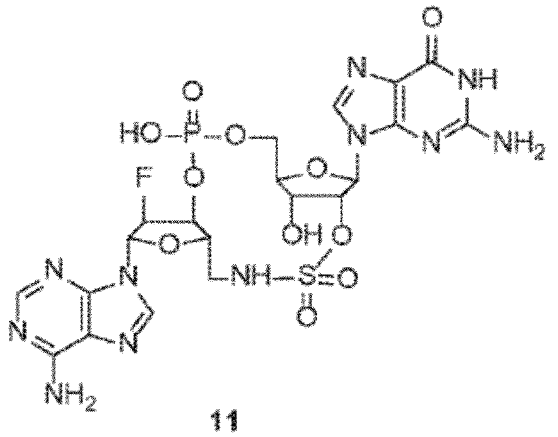

11

,

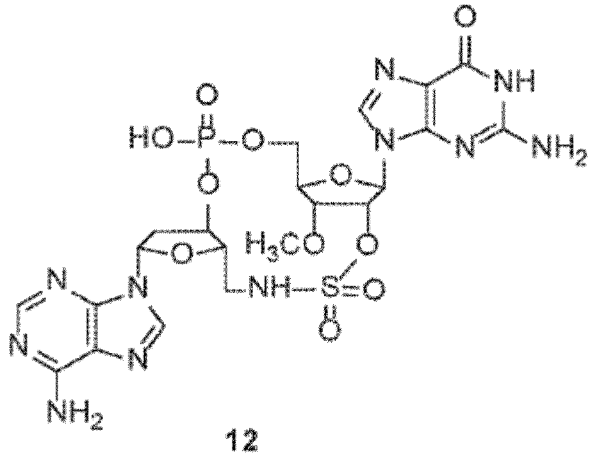

12

,

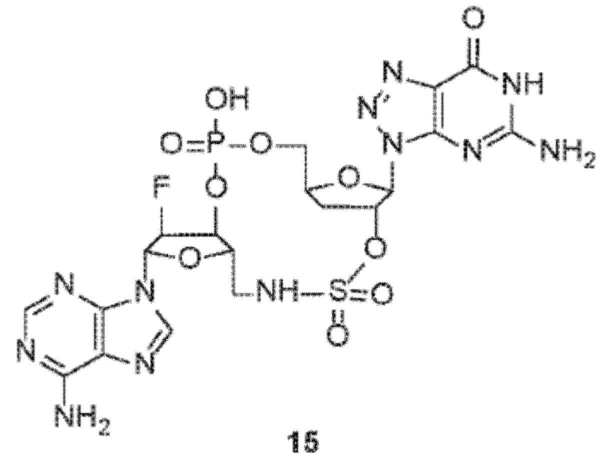

15

,

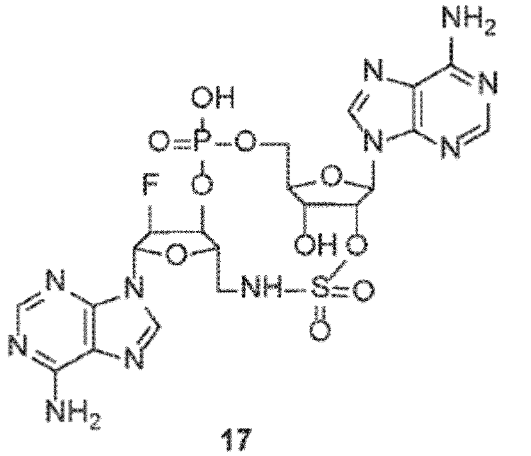

17

,

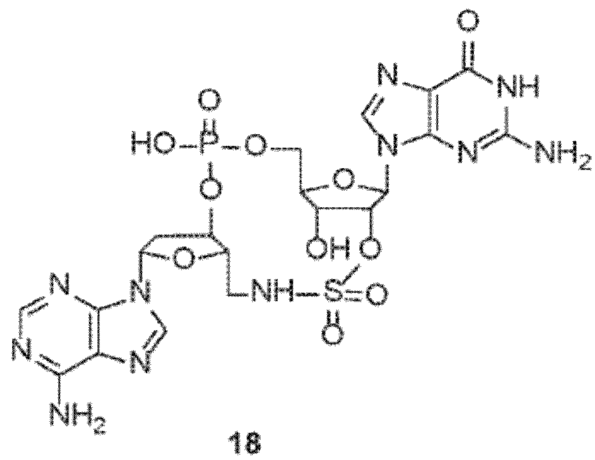

18

,

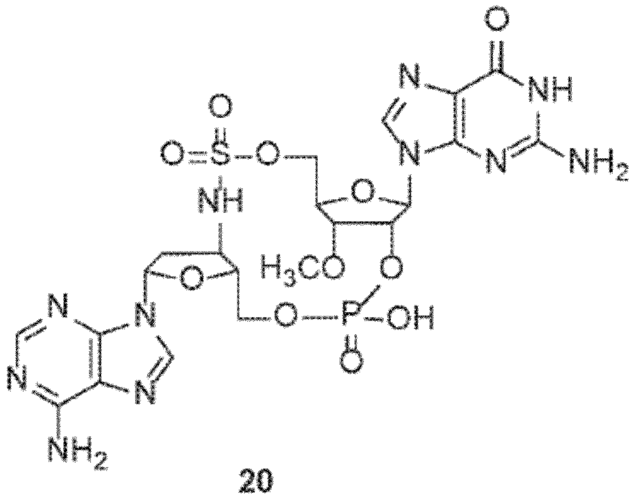

20

,

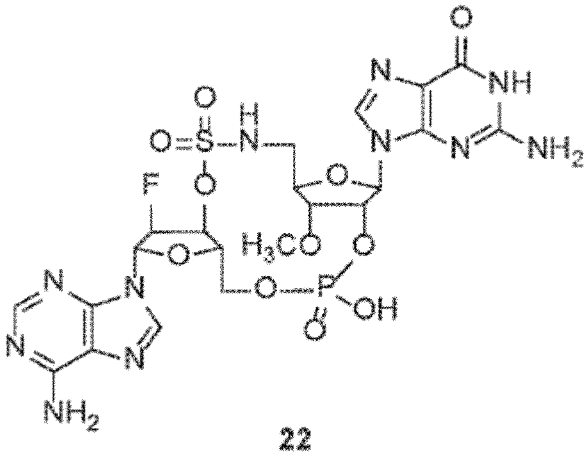

22

,

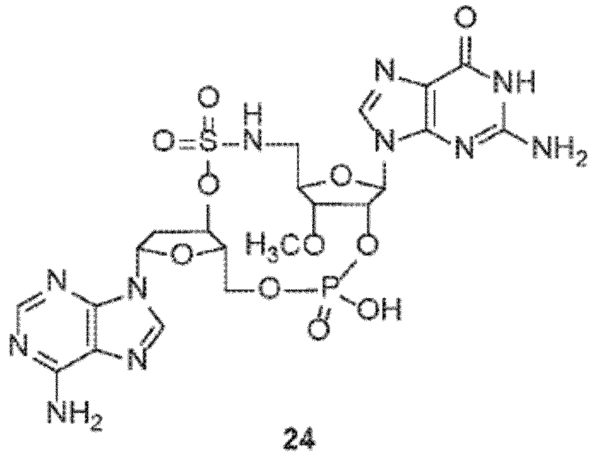

24

,

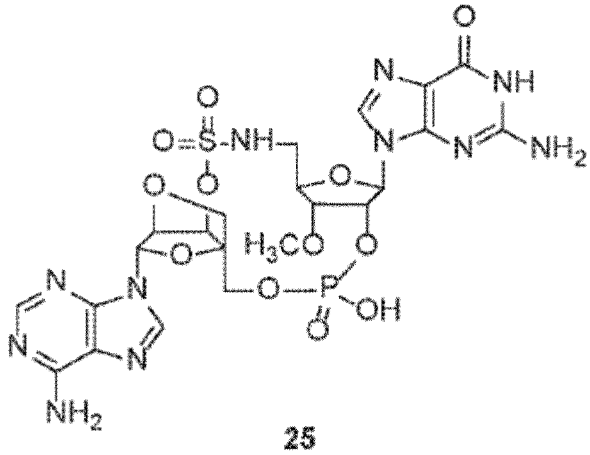

25

,

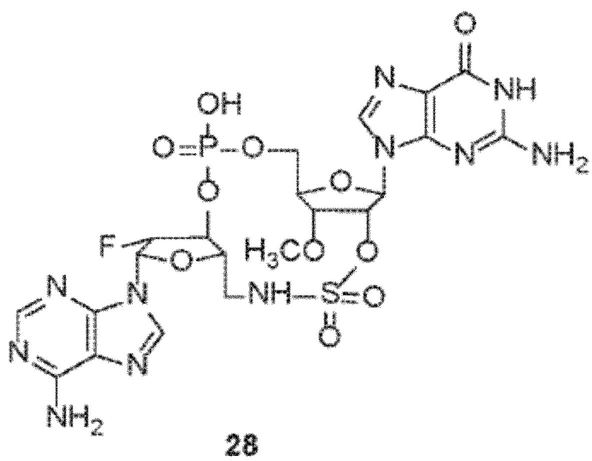

28

,

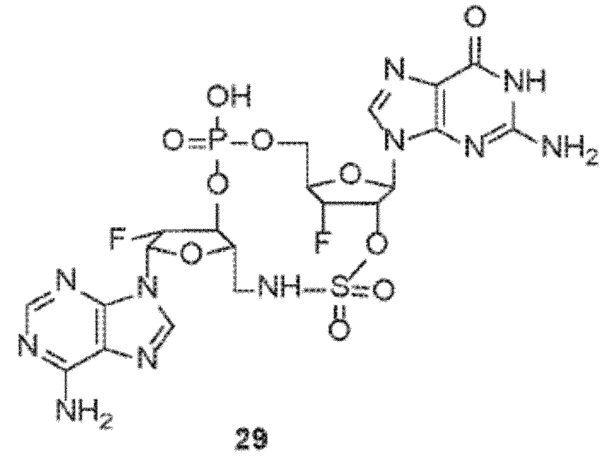

29

,

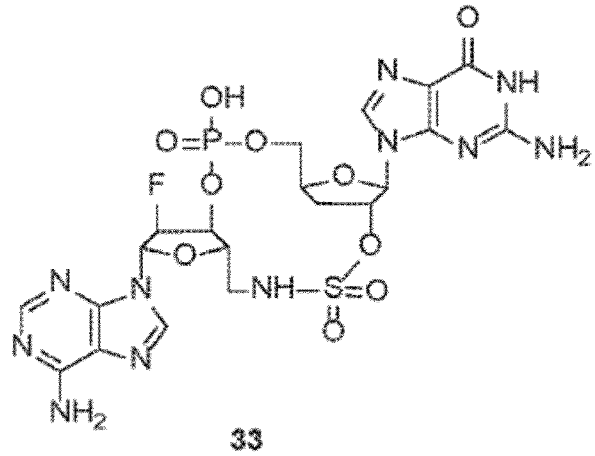

33

,

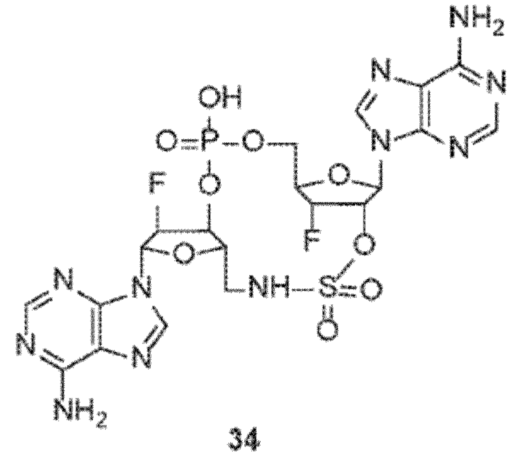

34

,

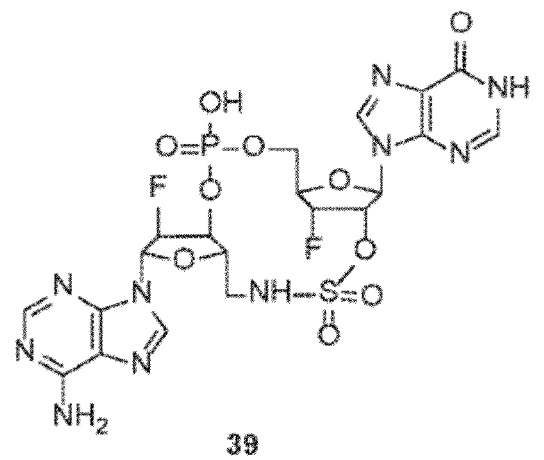

39

,

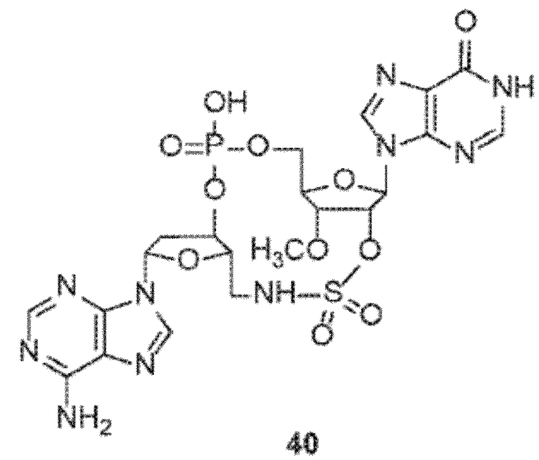

40

,

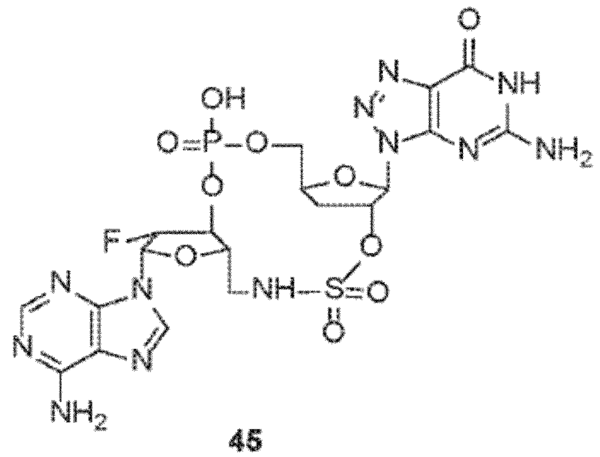

45

,

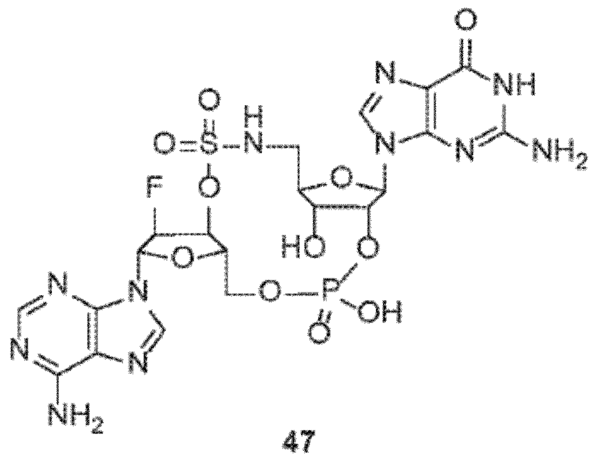

47

,

and

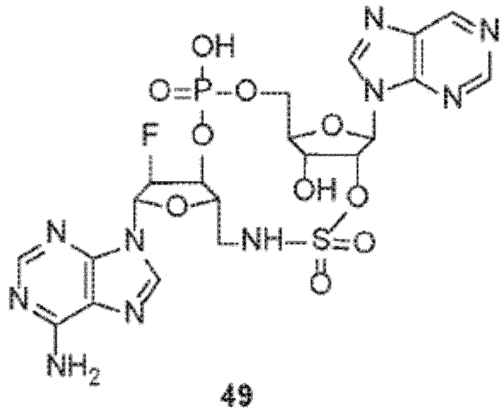

49

,

or a pharmaceutically acceptable salt form thereof.

Embodiments of the invention include a pharmaceutical composition comprising a compound of the invention and at least one of a pharmaceutically acceptable carrier, a pharmaceutically acceptable excipient, and a pharmaceutically acceptable diluent.

In embodiments of the invention, the composition is a solid oral dosage form.

In embodiments of the invention, the composition is a syrup, an elixir or a suspension.

Embodiments of the invention include the compound of the invention for use in treating a disease, syndrome, or condition modulated by STING.

Embodiments of the invention include the compound of the invention for use in treating a disease, syndrome, or condition, wherein said disease, syndrome, or condition is affected by the agonism of STING.

In embodiments of the invention, the disease, syndrome, or condition is cancer.

In embodiments of the invention, the cancer is melanoma, colon cancer, breast cancer, prostate cancer, lung cancer, or fibrosarcoma.

In embodiments of the invention, the disease, syndrome, or condition is a viral infection.

In embodiments of the invention, the viral infection is hepatitis B.

Embodiments of the invention include the compound of the invention, and an oncolytic virus or anti-cancer vaccine for use in treating a disease, syndrome, condition, or disorder, wherein said disease, syndrome, condition, or disorder is affected by the agonism of STING.

In embodiments of the invention, the anti-cancer vaccine is an antigen vaccine, whole cell vaccine, dendritic cell activating vaccine, DNA vaccine, Bacillus Calmette-Guérin (BCG) vaccine, Sipuleucel-T (Provenge), Talimogene laherparepvec (T-Vec; Imlygic™), oncolytic virus based vaccine, or adenovirus based vaccine.

The invention is defined in the claims. Further disclosure is provided for information.

DETAILED DESCRIPTION OF THE INVENTION

With reference to substituents, the term "independently" refers to the situation where when more than one substituent is possible, the substituents may be the same or different from each other.

The term "alkyl" whether used alone or as part of a substituent group, refers to straight and branched carbon chains having 1 to 8 carbon atoms. Therefore, designated numbers of carbon atoms (e.g., C1-8) refer independently to the number of carbon atoms in an alkyl moiety or to the alkyl portion of a larger alkyl-containing substituent. In substituent groups with multiple alkyl groups such as, (C1-6alkyl)2amino-, the C1-6alkyl groups of the dialkylamino may be the same or different.

The term "alkoxy" refers to an -O-alkyl group, wherein the term "alkyl" is as defined above.

The terms "alkenyl" and "alkynyl" refer to straight and branched carbon chains having 2 to 8 carbon atoms, wherein an alkenyl chain contains at least one double bond and an alkynyl chain contains at least one triple bond.

The term "cycloalkyl" refers to saturated or partially saturated, monocyclic or polycyclic hydrocarbon rings of 3 to 14 carbon atoms. Examples of such rings include cyclopropyl, cyclobutyl, cyclopentyl, cyclohexyl, cycloheptyl, and adamantyl.

The term "heterocyclyl" refers to a nonaromatic monocyclic or bicyclic ring system having 3 to 10 ring members that include at least 1 carbon atom and from 1 to 4 heteroatoms independently selected from N, O, and S. Included within the term heterocyclyl is a nonaromatic cyclic ring of 5 to 7 members in which 1 to 2 members are N, or a nonaromatic cyclic ring of 5 to 7 members in which 0, 1 or 2 members are N and up to 2 members are O or S and at least one member must be either N, O, or S; wherein, optionally, the ring contains 0 to 1 unsaturated bonds, and, optionally, when the ring is of 6 or 7 members, it contains up to 2 unsaturated bonds. The carbon atom ring members that form a heterocycle ring may be fully saturated or partially saturated.

The term "heterocyclyl" also includes two 5 membered monocyclic heterocycloalkyl groups bridged to form a bicyclic ring. Such groups are not considered to be fully aromatic and are not referred to as heteroaryl groups. When a heterocycle is bicyclic, both rings of the heterocycle are non-aromatic and at least one of the rings contains a heteroatom ring member. Examples of heterocycle groups include, and are not limited to, pyrrolinyl (including 2*H*-pyrrole, 2-pyrrolinyl or 3-pyrrolinyl), pyrrolidinyl, imidazolinyl, imidazolidinyl, pyrazolinyl, pyrazolidinyl, piperidinyl, morpholinyl, thiomorpholinyl, and piperazinyl. Unless otherwise noted, the heterocycle is attached to its pendant group at any heteroatom or carbon atom that results in a stable structure.

The term "aryl" refers to an unsaturated, aromatic monocyclic or bicyclic carbocyclic ring of 6 to 10 carbon members. Examples of aryl rings include phenyl and naphthalenyl.

The term "heteroaryl" refers to an aromatic monocyclic or bicyclic ring system having 5 to 10 ring members, which contains carbon atoms and from 1 to 4 heteroatoms independently selected from the group consisting of N, O, and S. Included within the term heteroaryl are aromatic rings of 5 or 6 members wherein the ring consists of carbon atoms and has at least one heteroatom member. Suitable heteroatoms include nitrogen, oxygen, and sulfur. In the case of 5 membered rings, the heteroaryl ring preferably contains one member of nitrogen, oxygen or sulfur and, in addition, up to 3 additional nitrogens. In the case of 6 membered rings, the heteroaryl ring preferably contains from 1 to 3 nitrogen atoms. For the case wherein the 6 membered ring has 3 nitrogens, at most 2 nitrogen atoms are adjacent. Examples of heteroaryl groups include furyl, thienyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, indolyl, isoindolyl, benzofuryl, benzothienyl, indazolyl, benzimidazolyl, benzothiazolyl, benzoxazolyl, benzisoxazolyl, benzothiadiazolyl, benzotriazolyl, quinolinyl, isoquinolinyl and quinazolinyl. Unless otherwise noted, the heteroaryl is attached to its pendant group at any heteroatom or carbon atom that results in a stable structure.

The term "halogen" or "halo" refers to fluorine, chlorine, bromine and iodine atoms.

Whenever the term "alkyl" or "aryl" or either of their prefix roots appear in a name of a substituent (e.g., arylalkyl, alkylamino) the name is to be interpreted as including those limitations given above for "alkyl" and "aryl." Designated numbers of carbon atoms (e.g., $C_1$-$C_6$) refer independently to the number of carbon atoms in an alkyl moiety, an aryl moiety, or in the alkyl portion of a larger substituent in which alkyl appears as its prefix root For alkyl and alkoxy substituents, the designated number of carbon atoms includes all of the independent members included within a given range specified. For example, $C_{16}$ alkyl would include methyl, ethyl, propyl, butyl, pentyl and hexyl individually as well as sub-combinations thereof (e.g., $C_{1-2}$, $C_{1-3}$, $C_{1-4}$, $C_{1-5}$, $C_{2-6}$, $C_{3-6}$, $C_{4-6}$, $C_{5-6}$, $C_{2-5}$. etc.).

In general, under standard nomenclature rules used throughout this disclosure, the terminal portion of the designated side chain is described first followed by the adjacent functionality toward the point of attachment Thus, for example, a "$C_1$-$C_6$ alkylcarbonyl" substituent refers to a group of the formula:

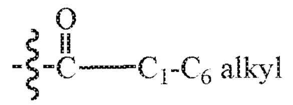

The term "R" at a stereocenter designates that the stereocenter is purely of the R-configuration as defined in the art; likewise, the term "S" means that the stereocenter is purely of the *S*-configuration. As used herein, the terms "*R" or "*S" at a stereocenter are used to designate that the stereocenter is of pure but unknown configuration. As used herein, the term "RS" refers to a stereocenter that exists as a mixture of the R- and *S*-configurations. Similarly, the terms "*RS" or "*SR" refer to a stereocenter that exists as a mixture of the *R*- and S-configurations and is of unknown configuration relative to another stereocenter within the molecule.

As used herein, the term "*" is used to designate a stereocenter that can exist as a pure but unknown configuration or mixture of the R- and S- configurations.

Compounds containing one stereocenter drawn without a stereo bond designation are a mixture of two enantiomers. Compounds containing two stereocenters both drawn without stereo bond designations are a mixture of four diastereomers. Compounds with two stereocenters both labeled "RS" and drawn with stereo bond designations are a two-component mixture with relative stereochemistry as drawn. Compounds with two stereocenters both labeled "*RS" and drawn with stereo bond designations are a two-component mixture with relative stereochemistry unknown. Unlabeled stereocenters drawn without stereo bond designations are a mixture of the R- and *S*-configurations. For unlabeled stereocenters drawn with stereo bond designations, the absolute stereochemistry is as depicted.

Unless otherwise noted, it is intended that the definition of any substituent or variable at a particular location in a molecule be independent of its definitions elsewhere in that molecule. It is understood that substituents and substitution patterns on the compounds of the present invention can be selected by one of ordinary skill in the art to provide compounds that are chemically stable and that can be readily synthesized by techniques known in the art as well as those methods set forth herein.

The term "subject" refers to an animal, preferably a mammal, most preferably a human, who has been the object of treatment, observation or experiment.

The term "therapeutically effective amount" refers to an amount of an active compound or pharmaceutical agent, including a compound of the present invention, which elicits the biological or medicinal response in a tissue system, animal or human that is being sought by a researcher, veterinarian, medical doctor or other clinician, which includes alleviation or partial alleviation of the symptoms of the disease, syndrome, condition, or disorder being treated.

The term "composition" refers to a product that includes the specified ingredients in therapeutically effective amounts, as well as any product that results, directly, or indirectly, from combinations of the specified ingredients in the specified amounts.

The term "STING agonist" is intended to encompass a compound that interacts with STING by binding to it and inducing downstream signal transduction characterized by activation of the molecules associated with STING function. This includes direct phosphorylation of STING, IRF3 and/or NF-$_{\kappa}$B and could also include STAT6. STING pathway activation results in increased production of type I interferons (mainly IFN-$\alpha$ and IFN-$\beta$) and expression of interferon-stimulated genes (Chen H, et al. "Activation of STAT6 by STING Is Critical for Antiviral Innate Immunity". Cell. 2011, vol.14: 433-446; and Liu S-Y, et al. "Systematic identification of type I and type II interferon-induced antiviral factors ". Proc. Natl. Acad. Sci. 2012:vol.109 4239-4244).

The term "STING-modulated" is used to refer to a condition affected by STING directly or via the STING pathway, including but not limited to, viral infections, diseases or conditions such as melanoma, colon cancer, breast cancer, prostate cancer, lung cancer, fibrosarcoma, and hepatitis B infection.

As used herein, unless otherwise noted, the term "disorder modulated by STING" shall mean any viral infection, disease, disorder or condition characterized in that at least one of its characteristic symptoms is alleviated or eliminated upon treatment with a STING agonist. Suitable examples include, but are not limited to melanoma, colon cancer, breast cancer, prostate cancer, lung cancer, fibrosarcoma, and hepatitis B.

As used herein, unless otherwise noted, the term "affect" or "affected" (when referring to a viral infection, disease, syndrome, condition or disorder that is affected by agonism of STING) includes a reduction in the frequency and / or severity of one or more symptoms or manifestations of said viral infection, disease, syndrome, condition or disorder; and / or include the prevention of the development of one or more symptoms or manifestations of said viral infection, disease, syndrome, condition or disorder or the development of the viral infection, disease, condition, syndrome or disorder.

The compounds of the instant invention are useful for treating or ameliorating a viral infection, disease, a syndrome, a condition or a disorder that is affected by the agonism of STING. Such methods comprise, consist of and/or consist essentially of administering to a subject, including an animal, a mammal, and a human in need of such treatment, amelioration and / or prevention, a therapeutically effective amount of a compound of Formula (I), or an enantiomer,

diastereomer, solvate or pharmaceutically acceptable salt thereof.

In particular, the compounds of Formula (I), or an enantiomer, diastereomer, solvate or pharmaceutically acceptable salt form thereof are useful for treating or ameliorating diseases, syndromes, conditions, or disorders such as melanoma, colon cancer, breast cancer, prostate cancer, lung cancer, fibrosarcoma, and hepatitis B.

More particularly, the compounds of Formula (I), or an enantiomer, diastereomer, solvate or pharmaceutically acceptable salt form thereof are useful for treating or ameliorating melanoma, colon cancer, breast cancer, prostate cancer, lung cancer, fibrosarcoma, and hepatitis B, comprising administering to a subject in need thereof a therapeutically effective amount of a compound of Formula (I), or an enantiomer, diastereomer, solvate or pharmaceutically acceptable salt form thereof as herein defined.

Some embodiments disclosed herein relate to compounds for use in methods of ameliorating and/or treating a viral infection including infections caused by *Hepadnaviridae* such as hepatitis B virus or HBV. The methods can include administering to a subject identified as suffering from a viral infection an effective amount of one or more compounds of Formula (I), or a pharmaceutically acceptable salt form thereof, or a pharmaceutical composition that includes one or more compounds of Formula (I), or a pharmaceutically acceptable salt form thereof.

Other embodiments disclosed herein relate to a compound for use in a method of ameliorating and/or treating a viral infection that can include contacting a cell infected with the virus with an effective amount of one or more compounds described herein (for example, a compound of Formula (I), or a pharmaceutically acceptable salt form thereof), or a pharmaceutical composition that includes one or more compounds described herein, or a pharmaceutically acceptable salt thereof. Still other embodiments described herein relate to using one or more compounds of Formula (I), or a pharmaceutically acceptable salt form thereof, in the manufacture of a medicament for ameliorating and/or treating a viral infection.

Yet still other embodiments described herein relate to one or more compounds of Formula (I), or a pharmaceutically acceptable salt form thereof, or a pharmaceutical composition that includes one or more compounds of Formula (I), or a pharmaceutically acceptable salt form thereof, that can be used for ameliorating and/or treating a viral infection. Some embodiments disclosed herein relate to a compound for use in a method of inhibiting replication of a virus that can include contacting a cell infected with the virus with an effective amount of one or more compounds of Formula (I), or a pharmaceutically acceptable salt form thereof, or a pharmaceutical composition that includes one or more compounds described herein, or a pharmaceutically acceptable salt form thereof.

Other embodiments described herein relate to using one or more compounds of Formula (I), or a pharmaceutically acceptable salt form thereof) in the manufacture of a medicament for inhibiting replication of a virus. Still other embodiments described herein relate to one or more compounds described herein (for example, a compound of Formula (I), or a pharmaceutically acceptable salt form thereof), or a pharmaceutical composition that includes one or more compounds described herein, or a pharmaceutically acceptable salt form thereof, that can be used for inhibiting replication of a virus.

In some embodiments, the viral infection can be a hepatitis B viral infection. The methods can include administering to a subject identified as suffering from HBV an effective amount of one or more compounds of Formula (I), or a pharmaceutically acceptable salt form thereof, or a pharmaceutical composition that includes one or more compounds of Formula (I), or a pharmaceutically acceptable salt form thereof.

Other embodiments disclosed herein relate to a compound for use in a method of ameliorating and/or treating a viral infection that can include contacting a cell infected with HBV with an effective amount of one or more compounds of Formula (I), or a pharmaceutically acceptable salt form thereof, or a pharmaceutical composition that includes one or more compounds of Formula (I), or a pharmaceutically acceptable salt form thereof. Still other embodiments described herein relate to using one or more compounds of Formula (I), or a pharmaceutically acceptable salt form thereof, in the manufacture of a medicament for ameliorating and/or treating HBV.

Yet still other embodiments described herein relate to one or more compounds of Formula (I), or a pharmaceutically acceptable salt form thereof, or a pharmaceutical composition that includes one or more compounds of Formula (I), or a pharmaceutically acceptable salt form thereof, that can be used for ameliorating and/or treating HBV. Some embodiments disclosed herein relate to a method of inhibiting replication of HBV that can include contacting a cell infected with the virus with an effective amount of one or more compounds of Formula (I), or a pharmaceutically acceptable salt form thereof, or a pharmaceutical composition that includes one or more compounds of Formula (I), or a pharmaceutically acceptable salt thereof.

Other embodiments described herein relate to using one or more compounds of Formula (I), or a pharmaceutically acceptable salt thereof) in the manufacture of a medicament for inhibiting replication of HBV. Still other embodiments described herein relate to one or more compounds of Formula (I), or a pharmaceutically acceptable salt thereof, or a pharmaceutical composition that includes one or more compounds of Formula (I), or a pharmaceutically acceptable salt form thereof, that can be used for inhibiting replication of HBV.

Embodiments of the present invention include a compound of Formula (I) as herein defined, or an enantiomer, diastereomer, solvate, or a pharmaceutically acceptable salt form thereof, wherein the substituents selected from one

or more of the variables defined herein (e.g. $B_2$, $X_2$, $R_{2a}$, $R_{2b}$, R2c, Z-M-Y, $Y_1$-$M_1$-$Z_1$, $B_1$, $X_1$, $R_{1a}$, $R_{1b}$, $R_{1c}$) are independently selected to be any individual substituent or any subset of substituents from those exemplified in the listing in Table 1, below.

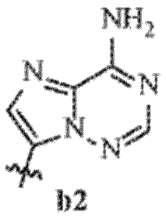
b2

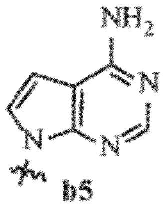
b5

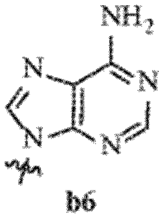
b6

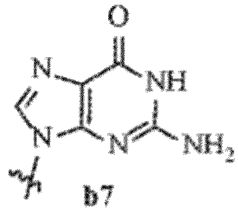
b7

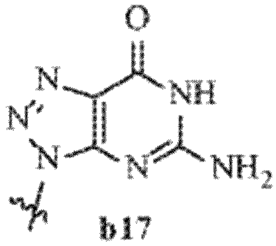
b17

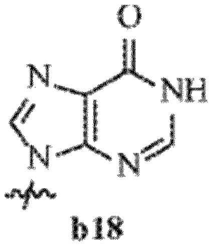
b18

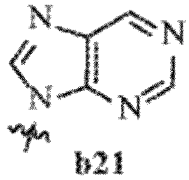
b21

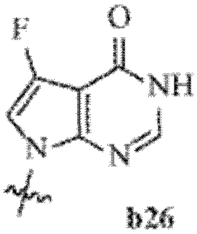
b26

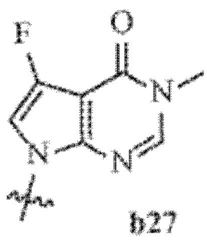
b27

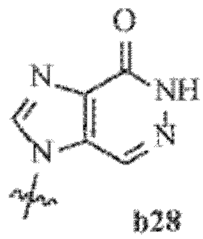
b28

Table 1.

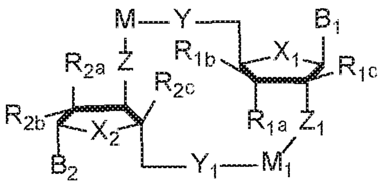
Formula (I)

| Cpd No. | $B_2$ | $X_2$ | $R_{2a}$ | $R_{2b}$ | $R_{2c}$ | Z-M-Y | $Y_1$-$M_1$-$Z_1$ | $B_1$ | $X_1$ | $R_{1a}$ | $R_{1b}$ | $R_{1c}$ |
|---|---|---|---|---|---|---|---|---|---|---|---|---|
| 1 | b6 | O | F | H | H | OP(O)(OH)O | NHS(O)$_2$O | b7 | O | $OCH_3$ | H | H |
| **2A** * | b6 | O | F | H | H | (*R)OP(O)(SH)O | NHS(O)$_2$O | b7 | O | $OCH_3$, | H | H |
| 3 * | b21 | O | OH | H | H | OS(O)$_2$NH | OP(O)(OH)O | b7 | O | $OCH_3$ | H | H |
| **4A** * | b6 | O | O | H | $CH_2$ to form a ring with $R_2$ | OS(O)$_2$NH | (*R)OP(O)(SH)O | b7 | O | $OCH_3$ | H | H |

(continued)

| Cpd No. | $B_2$ | $X_2$ | $R_{2a}$ | $R_{2b}$ | $R_{2c}$ | Z-M-Y | $Y_1$-$M_1$-$Z_1$ | $B_1$ | $X_1$ | $R_{1a}$ | $R_{1b}$ | $R_{1c}$ |
|---|---|---|---|---|---|---|---|---|---|---|---|---|
| **4B** * | b6 | O | O | H | $CH_2$ to form a ring with $R_2$ | OS $(O)_2$NH | (*S)OP (O)(SH)O | b7 | O | $OCH_3$ | H | H |
| 5 * | b6 | O | F | H | H | OP(O) (OH)O | OS $(O)_2$NH | b7 | O | $OCH_3$ | H | H |
| 6 | b6 | O | F | H | H | NHS $(O)_2$O | OP(O) (OH)O | b7 | O | $OCH_3$ | H | H |
| 7A * | b6 | O | F | H | H | (**R*)OP (O)(SH) O | NHS $(O)_2$O | b7 | O | H | $OCH_3$ | H |
| **7B*** | b6 | O | F | H | H | (**S*)OP (O)(SH) O | NHS $(O)_2$O | b7 | O | H | $OCH_3$ | H |
| **8** * | b5 | $CH_2$ | OH | H | H | OP(O) (OH)O | NHS $(O)_2$O | b7 | O | $OCH_3$ | H | H |
| 9 | b6 | O | O | H | $CH_2$ to form a ring with $R_2$ | OP(O) (OH)O | NHS $(O)_2$O | b7 | O | $OCH_3$ | H | H |
| * **10A** | b6 | O | O | H | $CH_2$ to form a ring forma with $R_2$ | (**R*)OP (O)(SH) O | NHS $(O)_2$O | b7 | O | $OCH_3$ | H | H |
| **11** | b6 | O | F | H | H | OP(O) (OH)O | NHS $(O)_2$O | b7 | O | OH | H | H |
| **12** | b6 | O | H | H | H | OP(O) (OH)O | NHS $(O)_2$O | b7 | O | $OCH_3$ | H | H |
| **13A** * | b6 | O | H | H | H | (**R*)OP (O)(SH) O | NHS $(O)_2$O | b7 | O | $OCH_3$ | H | H |
| **14A*** | b6 | O | F | H | H | (*K) OPP(O) $(BH_3)$O | NHS $(O)_2$O | b7 | O | $OCH_3$ | H | H |
| **15** | b6 | O | F | H | H | OP(O) (OH)O | NHS $(O)_2$O | b17 | O | H | H | H |
| **16A*** | b6 | O | F | H | H | (**R*)OP (O)(SH) O | NHS $(O)_2$O | b17 | O | H | H | H |
| **16B** * | b6 | O | F | H | H | (*5)OP (O)(SH) O | NHS $(O)_2$O | b17 | O | H | H | H |
| **17** | b6 | O | F | H | H | OP(O) (OH)O | NHS $(O)_2$O | b6 | O | OH | H | H |

(continued)

| Cpd No. | $B_2$ | $X_2$ | $R_{2a}$ | $R_{2b}$ | $R_{2c}$ | Z-M-Y | $Y_1$-$M_1$-$Z_1$ | $B_1$ | $X_1$ | $R_{1a}$ | $R_{1b}$ | $R_{1c}$ |
|---|---|---|---|---|---|---|---|---|---|---|---|---|
| **18** | b6 | O | H | H | H | OP(O)(OH)O | NHS(O)$_2$O | b7 | O | OH | H | H |
| **19A** * | b6 | O | H | H | H | (**R*)OS(O)$_2$NH | OP(O)(SH)O | b7 | O | $OCH_3$ | H | H |
| **19B** * | b6 | O | H | H | H | (**S*)OS(O)$_2$NH | OP(O)(SH)O | b7 | O | $OCH_3$ | H | H |
| 20 | b6 | O | H | H | H | NHS(O)$_2$O | OP(O)(OH)O | b7 | O | $OCH_3$ | H | H |
| 21A * | b6 | $CH_2$ | OH | H | H | (*R)OP(O)(SH)O | NHS(O)$_2$O | b7 | O | $OCH_3$ | H | H |
| 22 | b6 | O | F | H | H | OS(O)$_2$NH | OP(O)(OH)O | b7 | O | $OCH_3$ | H | H |
| 23A * | b6 | O | F | H | H | OS(O)$_2$NH | (**R*)OP(O)(SH)O | b7 | O | $OCH_3$ | H | H |
| 23B * | b6 | O | F | H | H | OS(O)$_2$NH | (*S)OP(O)(SH)O | b7 | O | $OCH_3$ | H | H |
| 24 | b6 | O | H | H | H | OS(O)$_2$NH | OP(O)(OH)O | b7 | O | $OCH_3$ | H | H |
| 25 | b6 | O | O | H | $CH_2$ to forma ring with $R_2$ | OS(O)$_2$NH | OP(O)(OH)O | b7 | O | $OCH_3$ | H | H |
| 26 * | b6 | O | F | H | H | OS(O)$_2$NH | OP(O)(OH)O | b7 | O | H | H | H |
| 27 * | b6 | O | F | H | H | OP(O)(OH)O | NHS(O)$_2$O | b28 | O | $OCH_3$ | H | H |
| 28 | b6 | O | H | F | H | OP(O)(OH)O | NHS(O)$_2$O | b7 | O | $OCH_3$ | H | H |
| 29 | b6 | O | H | F | H | OP(O)(OH)O | NHS(O)$_2$O | b7 | O | F | H | H |
| 30 * | b6 | O | OH | H | H | OP(O)(OH)O | NHS(O)$_2$O | b6 | O | OH | H | H |
| 31A* | b6 | O | H | F | H | (**R*)OP(O)(SH)O | NHS(O)$_2$O | b7 | O | F | H | H |
| 31B * | b6 | O | H | F | H | (**S*)OP(O)(SH)O | NHS(O)$_2$O | b7 | O | F | H | H |
| 32A * | b6 | O | F | H | H | OS(O)$_2$NH | (**R*)OP(O)(SH)O | b6 | O | F | H | H |
| 32B * | b6 | O | F | H | H | OS(O)$_2$NH | (*S)OP(O)(SH)O | b6 | O | F | H | H |
| 33 | b6 | O | F | H | H | OP(O)(OH)O | NHS(O)$_2$O | b7 | O | H | H | H |

(continued)

| Cpd No. | $B_2$ | $X_2$ | $R_{2a}$ | $R_{2b}$ | $R_{2c}$ | Z-M-Y | $Y_1$-$M_1$-$Z_1$ | $B_1$ | $X_1$ | $R_{1a}$ | $R_{1b}$ | $R_{1c}$ |
|---|---|---|---|---|---|---|---|---|---|---|---|---|
| 34 | b6 | O | F | H | H | OP(O)(OH)O | NHS(O)$_2$O | b6 | O | F | H | H |
| 35 * | b6 | O | F | H | H | OS(O)$_2$NH | OP(O)(OH)O | b6 | O | F | H | H |
| 36 * | b6 | O | H | H | H | OP(O)(OH)O | NHS(O)$_2$O | b6 | O | F | H | H |
| 37 * | b6 | O | F | H | H | OS(O)$_2$NH | OP(O)(OH)O | b17 | O | H | H | H |
| 38 * | b6 | O | H | F | H | OP(O)(OH)O | NHS(O)$_2$O | b7 | O | H | H | H |
| 39 | b6 | O | F | H | H | OP(O)(OH)O | NHS(O)$_2$O | b18 | O | F | H | H |
| 40 | b6 | O | H | H | H | OP(O)(OH)O | NHS(O)$_2$O | b18 | O | $OCH_3$ | H | H |
| 41* | b6 | O | F | OH | H | OS(O)$_2$NH | OP(O)(OH)O | b6 | O | OH | H | H |
| 42A * | b6 | O | F | H | H | (*R)OP(O)(SH)O | NHS(O)$_2$O | b6 | O | F | H | H |
| 42B * | b6 | O | F | H | H | (*S)OP(O)(SH)O | NHS(O)$_2$O | b6 | O | F | H | H |
| 43A* | b6 | O | F | H | H | (*R)OP(O)(SH)O | NHS(O)$_2$O | b7 | $CH_2$ | OH | H | H |
| 44A * | b6 | O | F | H | H | (*R)OP(O)CSH)O | NHS(O)$_2$O | b7 | O | H | H | H |
| 44B * | b6 | O | F | H | H | (*S)OP(O)(SH)O | NHS(O)$_2$O | b7 | O | H | H | H |
| 45 | b6 | O | H | F | H | OP(O)(OH)O | NHS(O)$_2$O | b17 | O | H | H | H |
| 46A* | b6 | O | F | H | H | OS(O)$_2$NH | (*R)OP(O)(SH)O | b7 | O | H | H | H |
| 46B * | b6 | O | F | H | H | OS(O)$_2$NH | (*S)OP(O)(SH)O | b7 | O | H | H | H |
| 47 | b6 | O | F | H | H | OS(O)$_2$NH | OP(O)(OH)O | b7 | O | OH | H | H |
| 48 * | b6 | O | OH | H | H | OP(O)(OH)O | NHS(O)$_2$O | b2 | O | OH | H | H |
| 49 | b6 | O | F | H | H | OP(O)(OH)O | NHS(O)$_2$O | b21 | O | OH | H | H |

(continued)

| Cpd No. | $B_2$ | $X_2$ | $R_{2a}$ | $R_{2b}$ | $R_{2c}$ | Z-M-Y | $Y_1$-$M_1$-$Z_1$ | $B_1$ | $X_1$ | $R_{1a}$ | $R_{1b}$ | $R_{1c}$ |
|---|---|---|---|---|---|---|---|---|---|---|---|---|
| 50A* | b6 | O | H | F | H | (**R*)OP (O)(SH) O | NHS $(O)_2O$ | b17 | O | H | H | H |
| 51 * | b6 | O | F | H | H | OP(O) (OH)O | NHS $(O)_2O$ | b17 | O | $OCH_3$ | H | H |
| 52 * | b18 | O | F | H | H | OP(O) (OH)O | O $(O)_2ONH$ | b7 | O | $OCH_3$ | H | H |
| 53 * | b18 | O | F | H | H | OS $(O)_2NH$ | OP(O) (OH)O | b7 | O | $OCH_3$ | H | H |
| 54* | b6 | O | F | H | H | OP(O) (OH)O | NHS $(O)_2O$ | b26 | O | $OCH_3$ | H | H |
| 55* | b6 | O | F | H | H | OP(O) (OH)O | NHS $(O)_2O$ | b27 | O | $OCH_3$ | H | H |
| * = not part of the invention. | | | | | | | | | | | | |

The compounds of Table 1 (including those which do not form part of the invention) are further represented below:

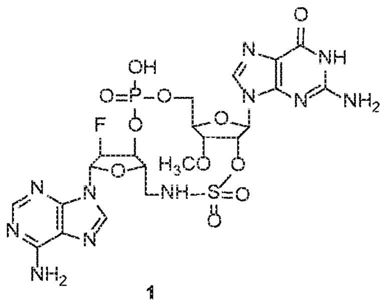

1

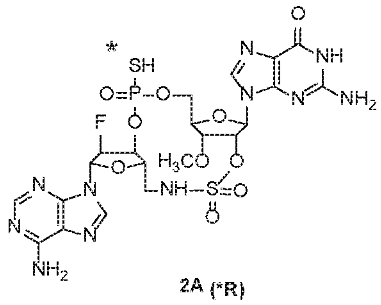

2A (*R)

,

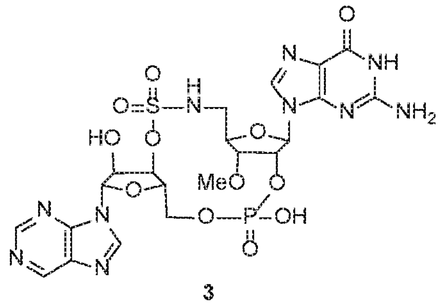

3

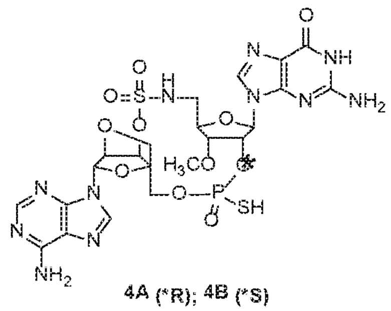

4A (*R); 4B (*S)
,

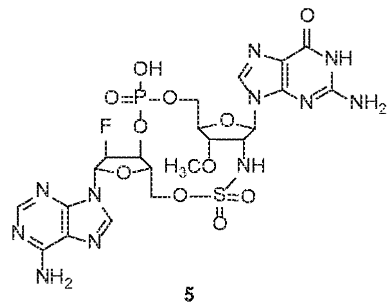

5
,

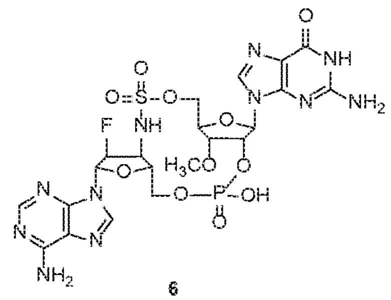

6
,

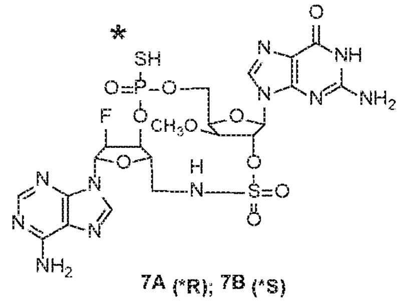

7A (*R); 7B (*S)

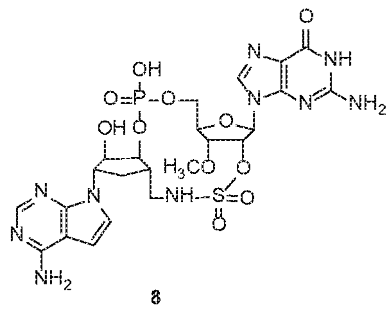

8

,

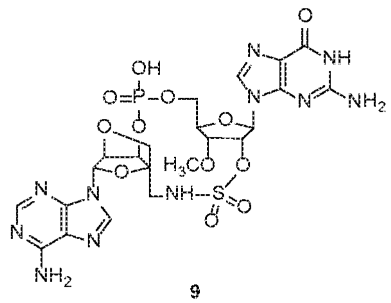

9

,

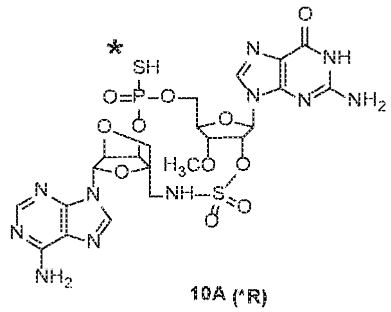

10A (*R)

,

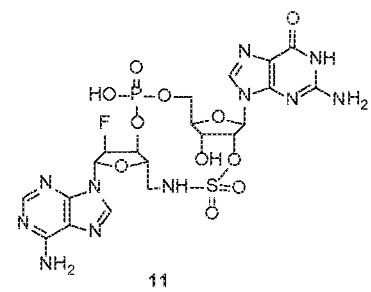

11

,

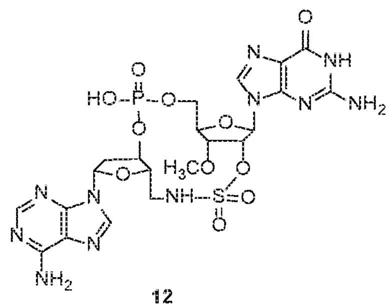

12

,

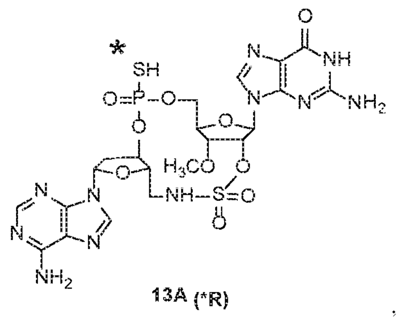

13A (*R) ,

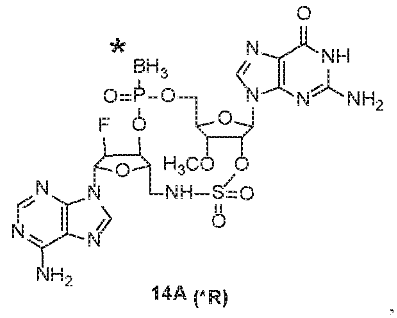

14A (*R) ,

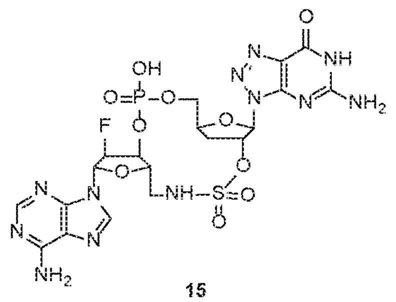

15 ,

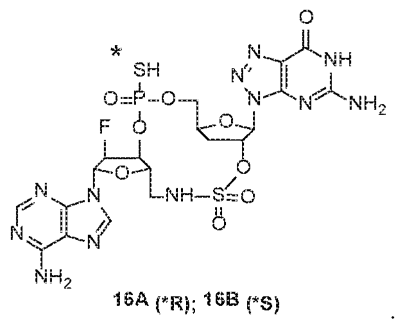

16A (*R); 16B (*S) ,

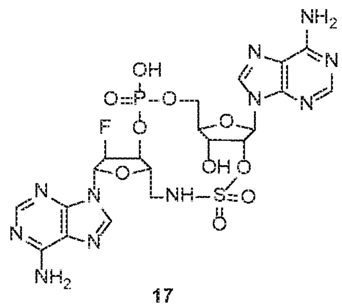

17 ,

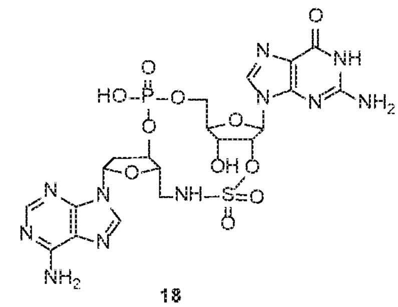

18

,

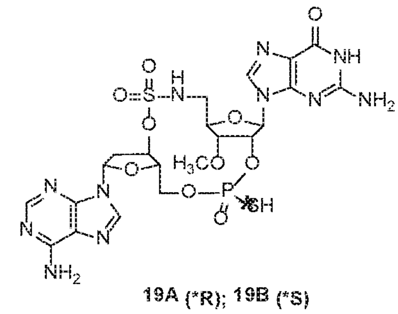

19A (*R); 19B (*S)

,

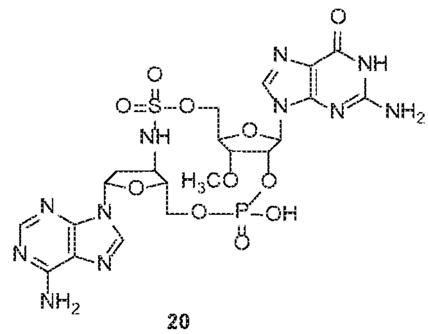

20

,

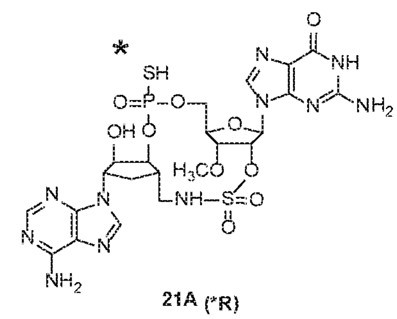

21A (*R)

,

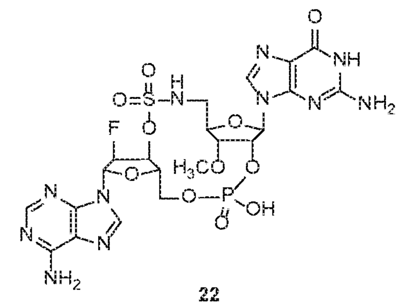

22

,

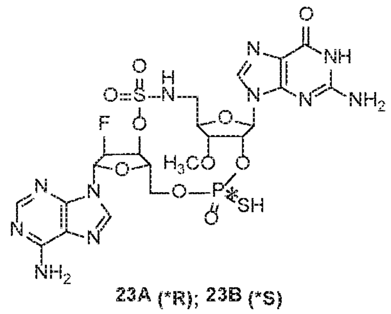

23A (*R); 23B (*S)

,

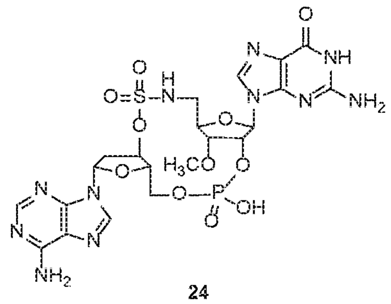

24

,

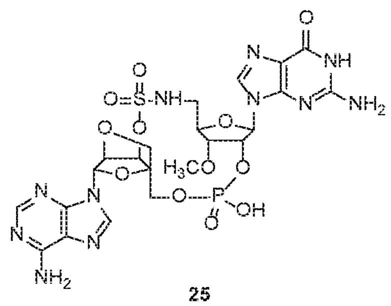

25

,

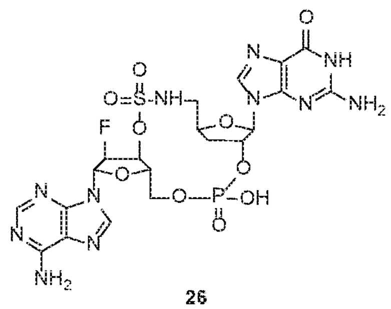

26

,

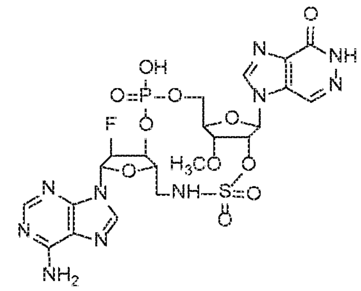

27

,

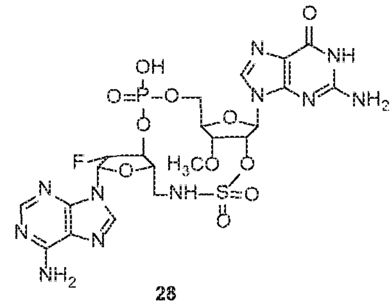

28

,

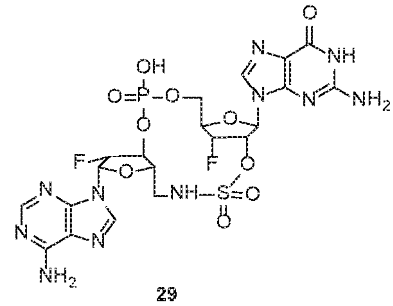

29

,

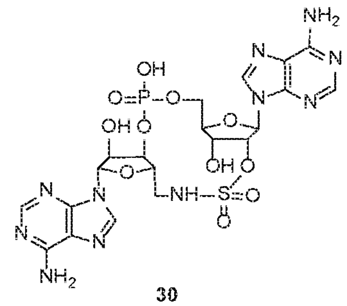

30

,

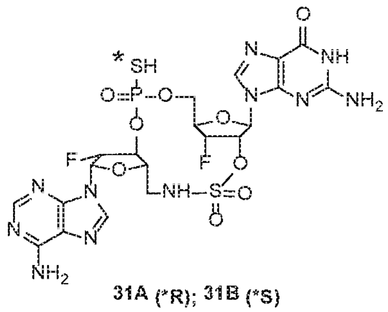

31A (*R); 31B (*S)

,

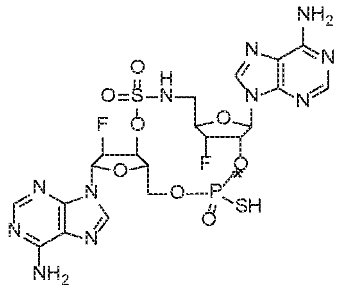

32A (*R); 32B (*S)

,

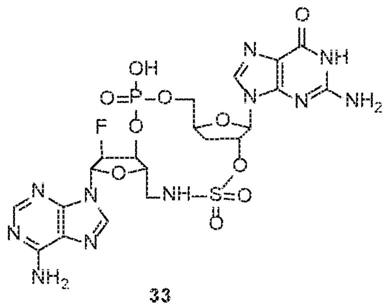

33

,

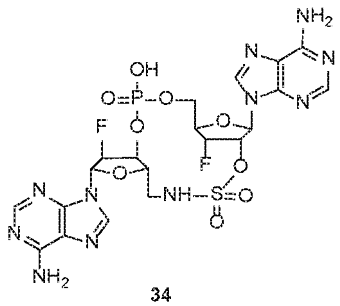

34

,

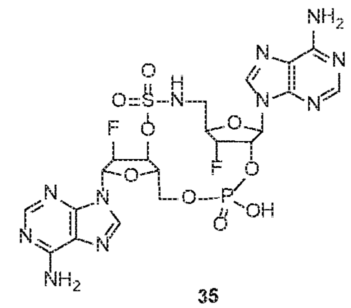

35 ,

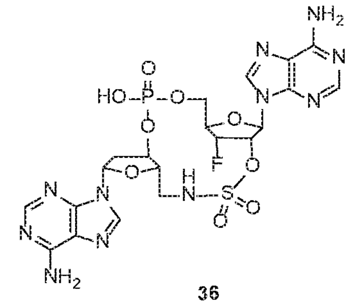

36 ,

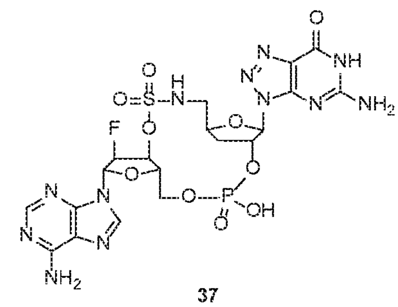

37 ,

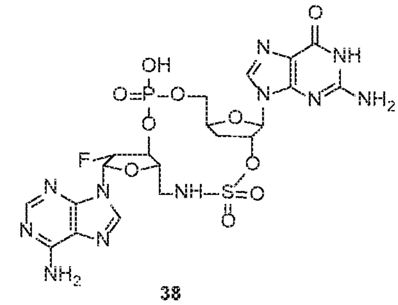

38 ,

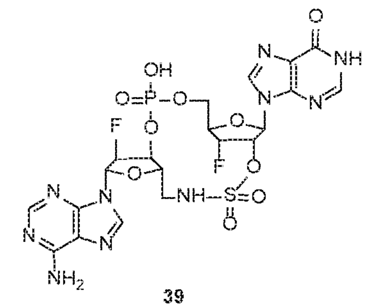

39 ,

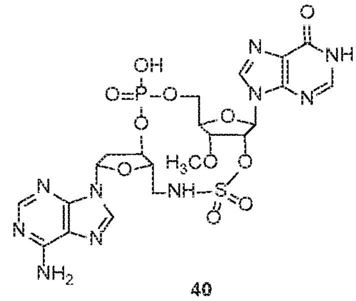

40

,

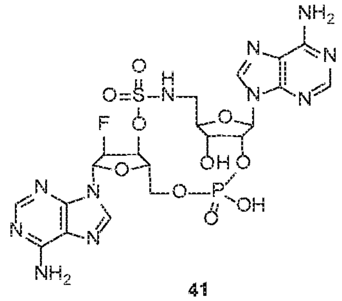

41

,

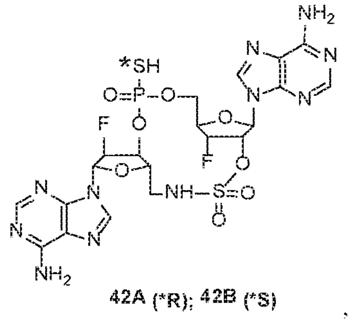

42A (*R); 42B (*S)

,

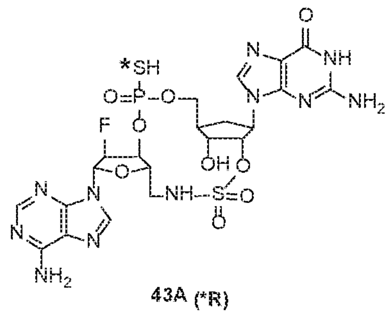

43A (*R)

,

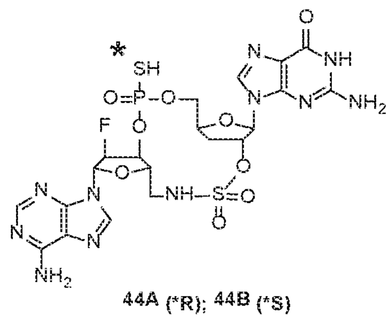

44A (*R); 44B (*S)

,

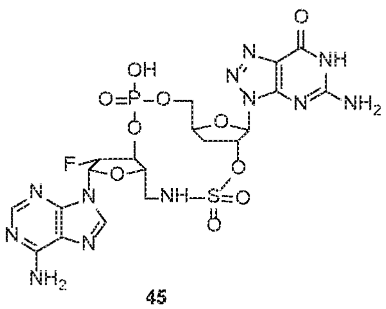

45

,

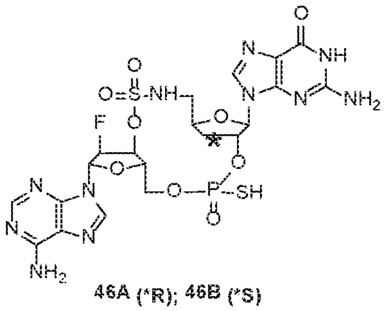

46A (*R); 46B (*S)

,

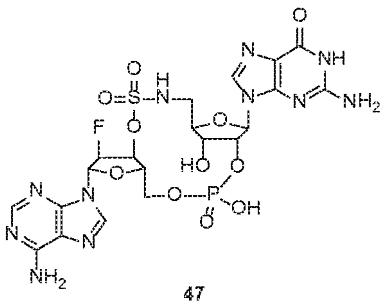

47

,

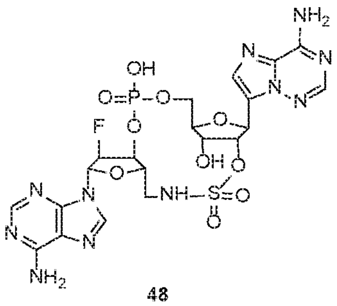

48

,

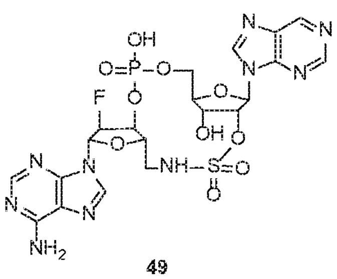

49

,

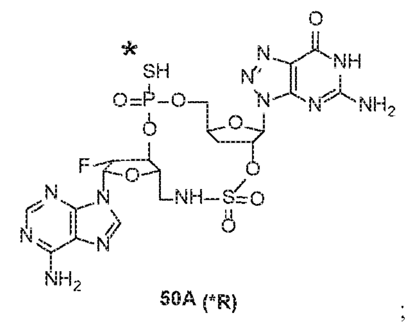

50A (*R)

;

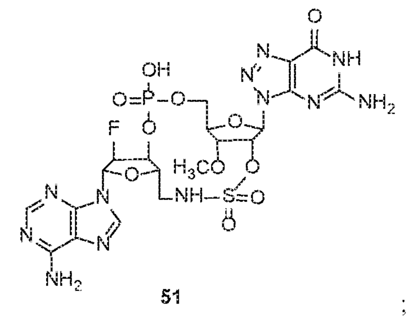

51

;

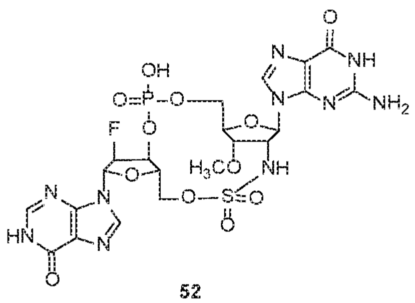

52

;

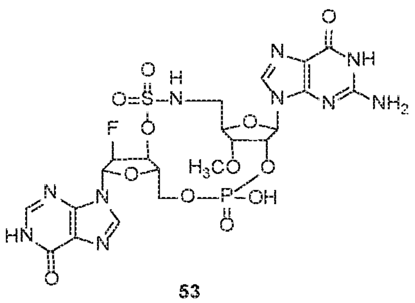

53

;

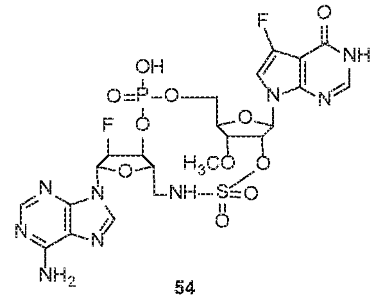

54

;

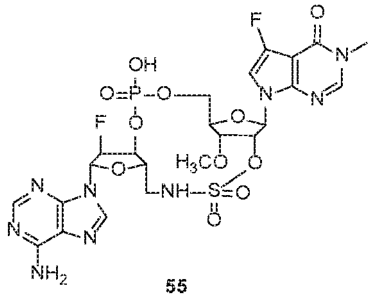

55

;

or a pharmaceutically acceptable salt form thereof.

For use in medicine, salts of compounds of Formula (I) refer to non-toxic "pharmaceutically acceptable salts." Other salts may, however, be useful in the preparation of compounds of Formula (I) or of their pharmaceutically acceptable salt forms thereof. Suitable pharmaceutically acceptable salts of compounds of Formula (I) include acid addition salts that can, for example, be formed by mixing a solution of the compound with a solution of a pharmaceutically acceptable acid such as, hydrochloric acid, sulfuric acid, fumaric acid, maleic acid, succinic acid, acetic acid, benzoic acid, citric acid, tartaric acid, carbonic acid or phosphoric acid. Furthermore, where the compounds of Formula (I) carry an acidic moiety, suitable pharmaceutically acceptable salts thereof may include alkali metal salts such as, sodium or potassium salts; alkaline earth metal salts such as, calcium or magnesium salts; and salts formed with suitable organic ligands such as, quaternary ammonium salts. Thus, representative pharmaceutically acceptable salts include acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylaisanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitiate, methylsulfate, mucate, napsylate, nitrate, N-methylglucamine ammonium salt, oleate, pamoate (embonate), palmitate, pantothenate, phosphate/diphosphate. polygalacturonate, salicylate, stearate, sulfate, subacetate, succinate, tannate, tartrate, teoclate, tosylate, triethiodide, and valerate.

Representative acids and bases that may be used in the preparation of pharmaceutically acceptable salts include acids including acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-bydroxy-2-naphthoic acid, nicotinic acid, nitric acid, oleic acid, orotic acid, oxalic acid, palmitic acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-bydroxyethyl)-moipiholine, piperazine, potassium hydroxide, 1-(2-hydroxyethyl)-pyrrolidine, sodium hydroxide, triethanolamine, tromethamine,

Where the compounds according to embodiments of this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention. The skilled artisan will understand that the term compound as used herein, is meant to include solvated compounds of Formula (I).

Where the processes for the preparation of the compounds according to certain embodiments of the invention give rise to mixture of stereoisomers, these isomers may be separated by conventional techniques such as, preparative chromatography. The compounds may be prepared in racemic form, or individual enantiomers may be prepared either by enantiospecific synthesis or by resolution. The compounds may, for example, be resolved into their component enantiomers by standard techniques such as, the formation of diastereomeric pairs by salt formation with an optically active acid such as, (-)-di-p-toluoyl-d-tartaric acid and/or (+)-di-p-toluoyl-l-tartaric acid followed by fractional crystallization and regeneration of the free base. The compounds may also be resolved by formation of diastereomeric esters or amides,

followed by chromatographic separation and removal of the chiral auxiliary. Alternatively, the compounds may be resolved using a chiral HPLC column.

One embodiment of the present invention is directed to a composition, including a pharmaceutical composition, comprising, consisting of, and/or consisting essentially of the (+)-enantiomer of a compound of Formula (I) wherein said composition is substantially free from the (-)-isomer of said compound. In the present context, substantially free means less than about 25 %, preferably less than about 10 %, more preferably less than about 5 %, even more preferably less than about 2 % and even more preferably less than about 1 % of the (-)-isomer calculated as

$$\%(+)\text{-enantiomer} = \frac{(mass(+)\text{-enantiomer})}{(mass(+)\text{-enantiomer}) + (mass(-)\text{-enantiomer})} \times 100$$

Another embodiment of the present invention is a composition, including a pharmaceutical composition, comprising, consisting of, and/or consisting essentially of the (-)-enantiomer of a compound of Formula (I) wherein said composition is substantially free from the (+)-isomer of said compound. In the present context, substantially free from means less than about 25 %, preferably less than about 10 %, more preferably less than about 5 %, even more preferably less than about 2 % and even more preferably less than about 1 % of the (+)-isomer calculated as

$$\%(-)\text{-enantiomer} = \frac{(mass(-)\text{-enantiomer})}{(mass(+)\text{-enantiomer}) + (mass(-)\text{-enantiomer})} \times 100$$

During any of the processes for preparation of the compounds of the various embodiments of the present invention, it may be necessary and/or desirable to protect sensitive or reactive groups on any of the molecules concerned. This may be achieved by means of conventional protecting groups such as those described in Protective Groups in Organic Chemistry, Second Edition, J.F.W. McOmie, Plenum Press, 1973; T.W. Greene & P.G.M. Wuts, Protective Groups in Organic Synthesis, John Wiley & Sons, 1991; and T.W. Greene & P.G.M. Wuts, Protective Groups in Organic Synthesis, Third Edition, John Wiley & Sons, 1999. The protecting groups may be removed at a convenient subsequent stage using methods known in the the art.

Even though the compounds of embodiments of the present invention (including their pharmaceutically acceptable salts and pharmaceutically acceptable solvates) can be administered alone, they will generally be administered in admixture with a pharmaceutically acceptable carrier, a pharmaceutically acceptable excipient and/or a pharmaceutically acceptable diluent selected with regard to the intended route of administration and standard pharmaceutical or veterinary practice. Thus, particular embodiments of the present invention are directed to pharmaceutical and veterinary compositions comprising compounds of Formula (I) and at least one pharmaceutically acceptable carrier, pharmaceutically acceptable excipient, and/or pharmaceutically acceptable diluent.

By way of example, in the pharmaceutical compositions of embodiments of the present invention, the compounds of Formula (I) may be admixed with any suitable binder(s), lubricant(s), suspending agent(s), coating agent(s), solubilizing agent(s), and combinations thereof.

Solid oral dosage forms such as, tablets or capsules, containing the compounds of the present invention may be administered in at least one dosage form at a time, as appropriate. It is also possible to administer the compounds in sustained release formulations.

Additional oral forms in which the present inventive compounds may be administered include elixirs, solutions, syrups, and suspensions; each optionally containing flavoring agents and coloring agents.

Alternatively, compounds of Formula (I) can be administered by inhalation (intratracheal or intranasal) or in the form of a suppository or pessary, or they may be applied topically in the form of a lotion, solution, cream, ointment or dusting powder. For example, they can be incorporated into a cream comprising, consisting of, and/or consisting essentially of an aqueous emulsion of polyethylene glycols or liquid paraffin. They can also be incorporated, at a concentration of between about 1 % and about 10 % by weight of the cream, into an ointment comprising, consisting of, and/or consisting essentially of a wax or soft paraffin base together with any stabilizers and preservatives as may be required. An alternative means of administration includes transdermal administration by using a skin or transdermal patch.

The pharmaceutical compositions of the present invention (as well as the compounds of the present invention alone) can also be injected parenterally, for example, intracavernosally, intravenously, intramuscularly, subcutaneously, intradermally, or intrathecally. In this case, the compositions will also include at least one of a suitable carrier, a suitable excipient, and a suitable diluent

For parenteral administration, the pharmaceutical compositions of the present invention are best used in the form of a sterile aqueous solution that may contain other substances, for example, enough salts and monosaccharides to make the solution isotonic with blood.

In addition to the above described routes of administration for the treatment of cancer, the pharmaceutical compositions may be adapted for administration by intratumoral or peritumoral injection. The activation of the immune system in this manner to kill tumors at a remote site is commonly known as the abscopal effect and has been demonstrated in animals with multiple therapueutic modalities, (van der Jeught, et al., Oncotarget, 2015, 6(3), 1359-1381). A further advantage of local or intratumoral or peritumoral administration is the ability to achieve equivalent efficacy at much lower doses, thus minimizing or eliminating adverse events that may be observed at much higher doses (Marabelle, A., et al., Clinical Cancer Research, 2014, 20(7), 1747-1756).

For buccal or sublingual administration, the pharmaceutical compositions of the present invention may be administered in the form of tablets or lozenges, which can be formulated in a conventional manner.

By way of further example, pharmaceutical compositions containing at least one of the compounds of Formula (I) as the active ingredient can be prepared by mixing the compound(s) with a pharmaceutically acceptable carrier, a pharmaceutically acceptable diluent, and/or a pharmaceutically acceptable excipient according to conventional pharmaceutical compounding techniques. The carrier, excipient, and diluent may take a wide variety of forms depending upon the desired route of administration (e.g., oral, parenteral, etc.). Thus, for liquid oral preparations such as, suspensions, syrups, elixirs and solutions, suitable carriers, excipients and diluents include water, glycols, oils, alcohols, flavoring agents, preservatives, stabilizers, coloring agents and the like; for solid oral preparations such as, powders, capsules, and tablets, suitable carriers, excipients and diluents include starches, sugars, diluents, granulating agents, lubricants, binders, disintegrating agents and the like. Solid oral preparations also may be optionally coated with substances such as, sugars, or be enterically coated so as to modulate the major site of absorption and disintegration. For parenteral administration, the carrier, excipient and diluent will usually include sterile water, and other ingredients may be added to increase solubility and preservation of the composition. Injectable suspensions or solutions may also be prepared utilizing aqueous carriers along with appropriate additives such as, solubilizers and preservatives.

A therapeutically effective amount of a compound of Formula (I) or a pharmaceutical composition thereof includes a dose range from about 0.01 mg to about 3000 mg, or any particular amount or range therein, in particular from about 0.05 mg to about 1000 mg, or any particular amount or range therein, or, more particularly, from about 0.05 mg to about 250 mg, or any particular amount or range therein, of active ingredient in a regimen of about 1 to about 4 times per day for an average (70 kg) human; although, it is apparent to one skilled in the art that the therapeutically effective amount for a compound of Formula (I) will vary as will the diseases, syndromes, conditions, and disorders being treated.

For oral administration, a pharmaceutical composition is preferably provided in the form of tablets containing about 1.0, about 10, about 50, about 100, about 150, about 200, about 250, and about 500 milligrams of a compound of Formula (I).

Advantageously, a compound of Formula (I) may be administered in a single daily dose, or the total daily dosage may be administered in divided doses of two, three and four times daily.

Optimal dosages of a compound of Formula (I) to be administered may be readily determined and will vary with the particular compound used, the mode of administration, the strength of the preparation and the advancement of the viral infection, disease, syndrome, condition or disorder. In addition, factors associated with the particular subject being treated, including subject gender, age, weight, diet and time of administration, will result in the need to adjust the dose to achieve an appropriate therapeutic level and desired therapeutic effect. The above dosages are thus exemplary of the average case. There can be, of course, individual instances wherein higher or lower dosage ranges are merited, and such are within the scope of this invention.

Compounds of Formula (I) may be administered in any of the foregoing compositions and dosage regimens or by means of those compositions and dosage regimens established in the art whenever use of a compound of Formula (I) is required for a subject in need thereof.

As STING protein agonists, the compounds of Formula (I) are useful in methods for treating or preventing a viral infection, disease, a syndrome, a condition or a disorder in a subject, including an animal, a mammal and a human in which the viral infection, disease, the syndrome, the condition or the disorder is affected by the modulation, including agonism, of the STING protein. Such methods comprise, consist of and/or consist essentially of administering to a subject, including an animal, a mammal, and a human, in need of such treatment or prevention, a therapeutically effective amount of a compound, salt or solvate of Formula (I).

In one embodiment, the present invention is directed to a compound of Formula (I), or a pharmaceutically acceptable salt form thereof, for the use in the treatment of cancer, and cancer diseases and conditions, or a viral infection.

Examples of cancer diseases and conditions for which compounds of Formula (I), or pharmaceutically acceptable salts or solvates thereof, may have potentially beneficial antitumor effects include, but are not limited to, cancers of the lung, bone, pancreas, skin, head, neck, uterus, ovaries, stomach, colon, breast, esophagus, small intestine, bowel, endocrine system, thyroid gland, parathyroid gland, adrenal gland, urethra, prostate, penis, testes, ureter, bladder, kidney or liver; rectal cancer; cancer of the anal region; carcinomas of the fallopian tubes, endometrium, cervix, vagina, vulva, renal pelvis, renal cell; sarcoma of soft tissue; myxoma; rhabdomyoma; fibroma; lipoma; teratoma; cholangiocarcinoma;

hepatoblastoma; angiosarcoma; hemagioma; hepatoma; fibrosarcoma; chondrosarcoma; myeloma; chronic or acute leukemia; lymphocytic lymphomas; primary CNS lymphoma; neoplasms of the CNS; spinal axis tumors; squamous cell carcinomas; synovial sarcoma; malignant pleural mesotheliomas; brain stem glioma; pituitary adenoma: bronchial adenoma; chondromatous hanlartoma; inesothelioma; Hodgkin's Disease or a combination of one or more of the foregoing cancers. Suitably the present invention relates to a method for treating or lessening the severity of cancers selected from the group consisting of brain (gliomas), glioblastomas, astrocytomas, glioblastoma multiforme, Bannayan-Zonana syndrome, Cowden disease, Lhermitte-Duclos disease, Wilm's tumor, Ewing's sarcoma, Rhabdomyosarcoma, ependymoma, medulloblastoma, head and neck, kidney, liver, melanoma, ovarian, pancreatic, adenocarcinoma, ductal madenocarcinoma, adenosquamous carcinoma, acinar cell carcinoma, glucagonoma, insulinoma, prostate, sarcoma, osteosarcoma, giant cell tumor of bone, thyroid, lymphoblastic T cell leukemia, chronic myelogenous leukemia, chronic lymphocytic leukemia, hairy-cell leukemia, acute lymphoblastic leukemia, acute myelogenous leukemia, chronic neutrophilic leukemia, acute lymphoblastic T cell leukemia, plasmacytoma, Immunoblastic large cell leukemia, mantle cell leukemia, multiple myeloma, megakaryoblastic leukemia, multiple myeloma, acute megakaryocytic leukemia, pro myelocytic leukemia, erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, vulval cancer, cervical cancer, endometrial cancer, renal cancer, mesothelioma, esophageal cancer, salivary gland cancer, hepatocellular cancer, gastric cancer, nasopharyngeal cancer, buccal cancer, cancer of the mouth, GIST (gastrointestinal stromal tumor) and testicular cancer.

In another embodiment, the present invention is directed to a compound of Formula (I), or a pharmaceutically acceptable salt form thereof, for use in the treatment of a disorder affected by the agonism of STING selected from the group consisting of melanoma, colon cancer, breast cancer, prostate cancer, lung cancer, fibrosarcoma, and hepatitis B.

The disclosed compounds of Formula (I) may be useful in combination with one or more additional compounds useful for treating HBV infection. These additional compounds may comprise other disclosed compounds and/or compounds known to treat, prevent, or reduce the symptoms or effects of HBV infection. Such compounds include, but are not limited to, HBV polymerase inhibitors, interferons, viral entry inhibitors, viral maturation inhibitors, literature-described capsid assembly modulators, reverse transcriptase inhibitors, immunomodulatory agents, TLR-agonists, and other agents with distinct or unknown mechanisms that affect the HBV life cycle or that affect the consequences of HBV infection.

In non-limiting examples, the disclosed compounds may be used in combination with one or more drugs (or a salt thereof) selected from the group comprising:

HBV reverse transcriptase inhibitors, and DNA and RNA polymerase inhibitors including, but not limited to, lamivudine (3TC, Zeffix, Heptovir, Epivir, and Epivir-HBV), entecavir (Baraclude, Entavir), adefovir dipivoxil (Hepsara, Preveon, bis-POM PMEA), tenofovir disoproxil fumarate (Viread, TDF or PMPA);
interferons including, but not limited to, interferon alpha (IFN-$\alpha$), interferon beta (IFN-$\beta$), interferon lambda (IFN-$\lambda$), and interferon gamma (IFN-$\gamma$);
viral entry inhibitors;
viral maturation inhibitors;
capsid assembly modulators, such as, but not limited to, BAY 41-4109;
reverse transcriptase inhibitors;
immunomodulatory agents such as TLR-agonists; and
agents of distinct or unknown mechanisms, such as, but not limited to, AT-61 ((E)-N-(1-chloro-3-oxo-1-phenyl-3-(piperidin-1-yl)prop-1-en-2-yl)benzamide),AT-130((E)-N-(1-bromo-1-(2-methoxyphenyl)-3-oxo-3-(piperidin-1-yl)prop-1-en-2-yl)-4-nitrobenzamide), and analogs thereof.

In one embodiment, the additional therapeutic agent is an interferon. The term "interferon" or "IFN" refers to any member of the family of highly homologous species-specific proteins that inhibit viral replication and cellular proliferation and modulate immune response. For example, human interferons are grouped into three classes: Type I, which includes interferon-alpha (IFN-$\alpha$), interferon-beta (IFN-$\beta$), and interferon-omega (IFN-$\omega$). Type II, which includes interferon-gamma (IFN-$\gamma$), and Type III, which includes interferon-lambda (IFN-$\lambda$). Recombinant forms of interferons that have been developed and are commercially available are encompassed by the term "interferon" as used herein. Subtypes of interferons, such as chemically modified or mutated interferons, are also encompassed by the term "interferon" as used herein. Chemically modified interferons may include pegylated interferons and glycosylated interferons. Examples of interferons also include, but are not limited to, interferon-alpha-2a, interferon-alpha-2b, interferon-alpha-nl, interferon-beta-la, interferon-beta-1b, interferon-lamda-1, interferon-lamda-2, and interferon-lamda-3. Examples of pegylated interferons include pegylated interferon-alpha-2a and pegylated interferon alpha-2b.

Accordingly, in one embodiment, the compounds of Formula (I) can be administered in combination with an interferon selected from the group consisting of interferon alpha (IFN-$\alpha$), interferon beta (IFN-$\beta$), interferon lambda (IFN-$\lambda$), and interferon gamma (IFN-$\gamma$). In one specific embodiment, the interferon is interferon-alpha-2a, interferon-alpha-2b,

or interferon-alpha-nl. In another specific embodiment, the interferon-alpha-2a or interferon-alpha-2b is pegylated. In a preferred embodiment, the interferon-alpha-2a is pegylated interferon-alpha-2a (PEGASYS). In another embodiment, the additional therapeutic agent is selected from immune modulator or immune stimulator therapies, which includes biological agents belonging to the interferon class.

Further, the additional therapeutic agent may be an agent that disrupts the function of other essential viral protein(s) or host proteins required for HBV replication or persistence.

In another embodiment, the additional therapeutic agent is an antiviral agent that blocks viral entry or maturation or targets the HBV polymerase such as nucleoside or nucleotide or non-nucleos(t)ide polymerase inhibitors. In a further embodiment of the combination therapy, the reverse transcriptase inhibitor or DNA or RNA polymerase inhibitor is Zidovudine, Didanosine, Zalcitabine, ddA, Stavudine, Lamivudine, Abacavir, Emtricitabine, Entecavir, Apricitabine, Atevirapine, ribavirin, acyclovir, famciclovir, valacyclovir. ganciclovir, valganciclovir, Tenofovir, Adefovir, PMPA, cidofovir, Efavirenz, Nevirapine, Delavirdine, or Etravirine.

In an embodiment, the additional therapeutic agent is an immunomodulatory agent that induces a natural, limited immune response leading to induction of immune responses against unrelated viruses. In other words, the immunomodulatory agent can effect maturation of antigen presenting cells, proliferation of T-cells and cytokine release (e.g., IL-12, IL-18, IFN-alpha, -beta, and - gamma and TNF-alpha among others),

In a further embodiment, the additional therapeutic agent is a TLR modulator or a TLR agonist, such as a TLR-7 agonist or TLR-9 agonist. In further embodiment of the combination therapy, the TLR-7 agonist is selected from the group consisting of SM360320 (9-benzyl-8-hydroxy-2-(2-methoxy-ethoxy)adenine) and AZD 8848 (methyl [3-({[3-(6-amino-2-butoxy-8-oxo-7,8-dihydro-9H-purin-9-yl)propyl][3-(4-morpholinyl)propyl]amino}methyl)phenyl]acetate).

In any of the methods provided herein, the method may further comprise administering to the individual at least one HBV vaccine, a nucleoside HBV inhibitor, an interferon or any combination thereof. In an embodiment, the HBV vaccine is at least one of RECOMBIVAX HB, ENGERIX-B, ELOVAC B, GENEVAC-B, or SHANVAC B.

In one embodiment, the methods described herein further comprise administering at least one additional therapeutic agent selected from the group consisting of nucleotide/nucleoside analogs, entry inhibitors, fusion inhibitors, and any combination of these or other antiviral mechanisms.

In another aspect, provided herein is method of treating an HBV infection in an individual in need thereof, comprising reducing the HBV viral load by administering to the individual a therapeutically effective amount of a disclosed compound alone or in combination with a reverse transcriptase inhibitor; and further administering to the individual a therapeutically effective amount of HBV vaccine. The reverse transcriptase inhibitor may be at least one of Zidovudine, Didanosine, Zalcitabine, ddA, Stavudine, Lamivudine, Abacavir, Emtricitabine, Entecavir, Apricitabine, Atevirapine, ribavirin, acyclovir, famciclovir, valacyclovir, ganciclovir, valganciclovir, Tenofovir, Adefovir, PMPA, cidofovir, Efavirenz, Nevirapine, Delavirdine, or Etravirine.

In another aspect, provided herein is a method of treating an HBV infection in an individual in need thereof, comprising reducing the HBV viral load by administering to the individual a therapeutically effective amount of a disclosed compound alone or in combination with an antisense oligonucleotide or RNA interference agent that targets HBV nucleic acids; and further administering to the individual a therapeutically effective amount of HBV vaccine. The antisense oligonucleotide or RNA interference agent possesses sufficient complementarity to the target HBV nucleic acids to inhibit replication of the viral genome, transcription of viral RNAs, or translation of viral proteins.

In another embodiment, the disclosed compound and the at least one additional therapeutic agent are co-formulated. In yet another embodiment, the disclosed compound and the at least one additional therapeutic agent are co-administered. For any combination therapy described herein, synergistic effect may be calculated, for example, using suitable methods such as the Sigmoid-$E_{max}$ equation (Holford & Scheiner, 19981, Clin. Pharmacokinet. 6: 429-453), the equation of Loewe additivity (Loewe & Muischnek, 1926, Arch. Exp. Pathol Pharmacol. 114: 313-326) and the median-effect equation (Chou & Talalay, 1984, Adv. Enzyme Regul. 22: 27-55). Each equation referred to above may be applied to experimental data to generate a corresponding graph to aid in assessing the effects of the drug combination. The corresponding graphs associated with the equations referred to above are the concentration-effect curve, isobologram curve and combination index curve, respectively.

In an embodiment of any of the methods of administering combination therapies provided herein, the method can further comprise monitoring or detecting the HBV viral load of the subject, wherein the method is carried out for a period of time including until such time that the HBV virus is undetectable.

The disclosed compounds of Formula (I) may be useful in combination with one or more additional compounds useful for treating cancer. These additional compounds may comprise other disclosed compounds and/or compounds known to treat, prevent, or reduce the symptoms or effects of said cancer.

In one embodiment, targeting STING with activation or inhibiting agents may be a promising approach for treating diseases and conditions in which modulation for the type I IFN pathway is beneficial, including inflammatory, allergic and autoimmune diseases, infectious diseases, cancer, pre-cancerous syndromes and as vaccine adjuvants (Dubensky et al., Therapeutic Advances in Vaccines 1(2013)131-143).

In one embodiment, the compounds of the present invention may be useful as adjuvants in a therapeutic strategy employing anti- cancer vaccines. In an embodiment, an anti-cancer vaccine includes inactivated or attenuated bacteria or viruses comprising the antigens of interest, purified antigens, live viral delivery vectors engineered to express and secrete the antigen of interest. Delivery vectors may also include attenuated bacterial delivery vectors expressing the antigens.

A further embodiment of the present invention includes a method for treating or lessening the severity of cancers by activating the immune system with a cancer vaccine, including but not limited to, antigen vaccines, whole cell vaccines, dendritic cell activating vaccines, DNA vaccines, Bacillus Calmette-Guérin (BCG) vaccine, Sipuleucel-T (Provenge), Talimogene laherparepvec (T-Vec; Imlygic™), oncolytic virus based vaccines, and adenovirus based vaccines.

Antigens and adjuvants that may be used in combination with the compounds of general Formula (I), or the pharmaceutically acceptable salt forms thereof, include B7 costimulatory molecule, interleukin-2, interferon-y, GM-CSF, CTLA-4 antagonists, OX-40/0X-40 ligand, CD40/CD40 ligand, sargramostim, levamisol, vaccinia virus, Bacille Calmette-Guerin (BCG), liposomes, alum, Freund's complete or incomplete adjuvant, detoxified endotoxins, mineral oils, surface active substances such as lipolecithin, pluronic polyols, polyanions, peptides, and oil or hydrocarbon emulsions. Adjuvants, such as aluminum hydroxide or aluminum phosphate, can be added to increase the ability of the vaccine to trigger, enhance, or prolong an immune response. Additional materials, such as cytokines, chemokines, and bacterial nucleic acid sequences, like CpG, a toll-like receptor (TLR) 9 agonist as well as additional agonists for TLR 2, TLR 4, TLR 5, TLR 7, TLR 8, TLR9, including lipoprotein, LPS, monophosphoryllipid A, lipoteichoic acid, imiquimod, resiquimod, and in addition retinoic acid- inducible gene I (RIG-I) agonists such as poly I:C, used separately or in combination with the described compositions are also potential adjuvants.

CLTA-4 and PD-I pathways are important negative regulators of immune response. Activated T-cells up-regulate CTLA-4, which binds on antigen-presenting cells and inhibits T-cell stimulation, IL-2 gene expression, and T-cell proliferation; these anti-tumor effects have been observed in mouse models of colon carcinoma, metastatic prostate cancer, and metastatic melanoma. PD-1 binds to active T-cells and suppresses T-cell activation; PD-1 antagonists have demonstrated anti-tumor effects as well.

CTLA-4 and PD-1 pathway antagonists that may be used in combination with the compounds of Formula (I) or Formula (Ia), or the pharmaceutically acceptable salt forms thereof, disclosed herein, include ipilimumab, tremelimumab, nivolumab, pembrolizumab, CT-OII, AMP-224, and MDX-II06.

"PD-1 antagonist" or "PD-1 pathway antagonist" means any chemical compound or biological molecule that blocks binding of PD-L1 expressed on a cancer cell to PD-1 expressed on an immune cell (T-cell, B-cell, or NKT-cell) and preferably also blocks binding of PD-L2 expressed on a cancer cell to the immune-cell expressed PD-1. Alternative names or synonyms for PD-1 and its ligands include: PDCD I, PD 1, CD279, and SLEB2 for PD-1; PDCDILI, PDL 1, B7HI, B7-4, CD274, and B7-H for PD-L1; and PDCDIL2, PDL2, B7-DC, Btdc, and CD273 for PD-L2. In any of the treatment method, medicaments and uses of the present disclosure in which a human individual is being treated, the PD-1 antagonist blocks binding of human PD-L1 to human PD-1, and preferably blocks binding of both human PD-L1 and PD-L2 to human PD-1. Human PD-1 amino acid sequences can be found in NCBI Locus No.: NP_005009. Human PD-L1 and PD-L2 amino acid sequences can be found in NCBI Locus No.: NP_054862 and NP_0795 I5, respectively.

Human PD-1 amino acid sequences can be found in NCBI Locus No.: NP_005009. Human PD-L1 and PD-L2 amino acid sequences can be found in NCBI Locus No.: NP_054862 and NP_0795 I5, respectively.

PD-1 antagonists useful in any of the treatment method, medicaments and uses of the present disclosure include a monoclonal antibody (mAb), or antigen binding fragment thereof, which specifically binds to PD-1 or PD-L1, and preferably specifically binds to human PD-1 or human PD-L1. The mAb may be a human antibody, a humanized antibody, or a chimeric antibody and may include a human constant region. In some embodiments, the human constant region is selected from the group consisting of IgG1, IgG2, IgG3, and IgG4 constant regions, and in preferred embodiments, the human constant region is an IgG1 or IgG4 constant region. In some embodiments, the antigen binding fragment is selected from the group consisting of Fab, Fab'-SH, F(ab')2, scFv, and Fv fragments.

Examples of mAbs that bind to human PD-L1, and useful in the treatment method, medicaments and uses of the present disclosure, are described in PCT International Patent Application Nos. WO2013/019906 and WO2010/077634 A1 and in U.S. Patent No. US8383796. Specific anti-human PD-L1 mAbs useful as the PD-1 antagonist in the treatment method, medicaments and uses of the present disclosure include MPDL3280A, BMS-936559, MEDI4736, MSB0010718C, and an antibody that comprises the heavy chain and light chain variable regions of SEQ ID NO:24 and SEQ ID N0:21, respectively, of WO2013/019906.

Other PD-1 antagonists useful in any of the treatment method, medicaments, and uses of the present disclosure include an immune-adhesion that specifically binds to PD-1 or PD-L1, and preferably specifically binds to human PD-1 or human PD-L1, e.g., a fusion protein containing the extracellular or PD-I binding portion of PD-LI or PD-L2 fused to a constant region such as an Fe region of an immunoglobulin molecule. Examples of immune-adhesion molecules that specifically bind to PD-1 are described in PCT International Patent Application Publication Nos. WO2010/027827 and WO2011/066342. Specific fusion proteins useful as the PD-1 antagonist in the treatment method, medicaments, and

uses of the present disclosure include AMP-224 (also known as B7-DCig), which is a PD-L2-FC fusion protein and binds to human PD-I.

Examples of cytotoxic agents that may be used in combination with the compounds of general formula (I), or pharmaceutically acceptable salts of the foregoing, include, but are not limited to, arsenic trioxide (sold under the tradename TRISENox®), asparaginase (also known as L-asparaginase, and Erwinia L-asparaginase, sold under the tradenames ELSPAR® and KIDROLASE®).

Chemotherapeutic agents that may be used in combination with the compounds of Formula (I), or pharmaceutically acceptable salts of the foregoing, disclosed herein include abiraterone acetate, altretamine, anhydrovinblastine, auristatin, bexarotene, bicalutamide, BMS 184476,2,3,4,5,6-pentafluoro-N-(3-fluoro-4-methoxyphenyl)benzene sulfonamide, bleomycin, N,N-dimethyl-L-valyl-L-valyl-N-methyl-L-valyl-L-prolyl-1-Lproline-t-butylamide, cachectin, cemadotin, chlorambucil, cyclophosphamide, 3',4'-didehydro-4'deoxy-8'-norvin-caleukoblastine, docetaxol, doxetaxel, cyclophosphamide, carboplatin, carmustine, cisplatin, cryptophycin, cyclophosphamide, cytarabine, dacarbazine (DTIC), dactinomycin, daunorubicin, decitabine dolastatin, doxorubicin (adriamycin), etoposide, 5-fluorouracil, finasteride, flutamide, hydroxyurea and hydroxyurea andtaxanes, ifosfamide, liarozole, lonidamine, lomustine (CCNU), MDV3100, mechlorethamine (nitrogen mustard), melphalan, mivobulin isethionate, rhizoxin,sertenef, streptozocin, mitomycin, methotrexate, taxanes, nilutamide, nivolumab, onapristone, paclitaxel, pembrolizumab, prednimustine, procarbazine, RPR109881, stramustine phosphate, tamoxifen, tasonermin, taxol, tretinoin, vinblastine, vincristine, vindesine sulfate, and vinflunine.

Examples of vascular endothelial growth factor (VEGF) receptor inhibitors include, but are not limited to, bevacizumab (sold under the trademark AVASTIN), axitinib (described in PCT International Patent Publication No. WOO1/002369), Brivanib Alaninate ((S)- ((R)-1-(4-(4-Fluoro-2-methyl-1H-indol-5-yloxy)-5-methylpyrrolo[2, 1-f][1,2,4]triazin-6- yloxy)propan-2-yl)2-aminopropanoate, also known as BMS-582664), motesanib (N-(2,3- dihydro-3,3-dimethyl-1H-indol-6-yl)-2-[ (4-pyridinylmethyl)amino]-3-pyridinecarboxamide and described in PCT International Patent Application Publication No. WO02/068470), pasireotide (also known as SO 230, and described in PCT International Patent Publication No. WO02/010192), and sorafenib (sold under the tradename NEXAVAR).

Examples of topoisomerase II inhibitors, include but are not limited to, etoposide (also known as VP-16 and Etoposide phosphate, sold under the tradenames TOPOSAR, VEPESID, and ETOPOPHOS), and teniposide (also known as VM-26, sold under the tradename VUMON).

Examples of alkylating agents, include but are not limited to, 5-azacytidine (sold under the trade name VIDAZA), decitabine (sold under the trade name of DACOGEN), temozolomide (sold under the trade names TEMCAD, TEMODAR, and TEMODAL), dactinomycin (also known as actinomycin-D and sold under the tradename COSMEGEN), melphalan (also known as L-PAM, L-sarcolysin, and phenylalanine mustard, sold under the tradename ALKERAN), altretamine (also known as hexamethylmelamine (HMM), sold under the tradename HEXALEN), carmustine (sold under the tradename BCNU), bendamustine (sold under the tradename TREANDA), busulfan (sold under the tradenames BUSULFEx® and MYLERAN®), carboplatin (sold under the tradename PARAPLATIN®), lomustine (also known as CCNU, sold under the tradename CEENU®), cisplatin (also known as CDDP, sold under the tradenames PLATINOL ® and PLATINOL ®-AQ), chlorambucil (sold under the tradename LEUKERAN®), cyclophosphamide (sold under the tradenames CYTOXAN® and NEOSAR®), dacarbazine (also known as DTIC, DIC and imidazole carboxamide, sold under the tradename DTIC-DoME®), altretamine (also known as hexamethylmelamine (HMM) sold under the tradename HEXALEN®), ifosfamide (sold under the tradename IFEx®), procarbazine (sold under the tradename MATULANE®), mechlorethamine (also known as nitrogen mustard, mustine and mechloroethamine hydrochloride, sold under the tradename MUSTARGEN®), streptozocin (sold under the tradename ZANOSAR®), thiotepa (also known as thiophosphoamide, TESPA and TSPA, and sold under the tradename THIOPLEx®.

Examples of anti-tumor antibiotics include, but are not limited to, doxorubicin (sold under the tradenames ADRIAMYCIN® and RUBEx®), bleomycin (sold under the tradename LENOXANE®), daunorubicin (also known as dauorubicin hydrochloride, daunomycin, and rubidomycin hydrochloride, sold under the tradename CERUBIDINE®), daunorubicin liposomal (daunorubicin citrate liposome, sold under the tradename DAUNOXoME®), mitoxantrone (also known as DHAD, sold under the tradename NOVAN IRONE®), epirubicin (sold under the tradename ELLENCE™), idarubicin (sold under the tradenames IDAMYCIN®, IDAMYCIN PFS®), and mitomycin C (sold under the tradename MUTAMYCIN®).

Examples of anti-metabolites include, but are not limited to, claribine (2- chlorodeoxyadenosine, sold under the tradename LEUSTATIN®), 5-fluorouracil (sold under the tradename ADRUCIL ®), 6-thioguanine (sold under the tradename PURINETHOL ®), pemetrexed (sold under the tradename ALIMTA ®), cytarabine (also known as arabinosylcytosine (Ara-C), sold under the tradename CYTOSAR-U®), cytarabine liposomal (also known as Liposomal Ara-C, sold under the tradename DEPOCYT™), decitabine (sold under the tradename DACOGEN®), hydroxyurea and (sold under the tradenames HYDREA®, DROXIATM and MYLOCEL™), fludarabine (sold under the tradename FLUDARA ®), floxuridine (sold under the tradename FUDR®), cladribine (also known as 2-chlorodeoxyadenosine (2-CdA) sold under the tradename LEUSTATIN™), methotrexate (also known as amethopterin, methotrexate sodium (MTX), sold under the trade names RHEUMA1REX® and TREXALL™), and pentostatin (sold under the tradename NIPENT®).

Examples of retinoids include, but are not limited to, alitretinoin (sold under the tradename PANRETIN®), tretinoin (all-trans retinoic acid, also known as ATRA, sold under the tradename VESANom®), Isotretinoin (13-c/s-retinoic acid, sold under the tradenames ACCUTANE®, AMNESTEEM®, CLARAvis®, CLARus®, DECUTAN®, ISOTANE®, Izo-TEcH®, ORATANE®, ISOTRET®, and SOTRET®), and bexarotene (sold under the tradename TARGRETIN®).

Abbreviations used in the instant specification, particularly the schemes and examples, are as follows:

| | |
|---|---|
| ACN | acetonitrile |
| AcOH | glacial acetic acid |
| ADDP | azodicarboxylic dipiperidide |
| aq. | Aqueous |
| Ar. | Argon |
| Bn or Bzl | benzyl |
| Bz | benzoyl |
| BINAP | 2,2'-bis(diphenylphosphino)-1,1'-binaphthyl |
| Boc | tert-butyloxycarbonyl |
| $CH_3CN$ | acetonitrile |
| conc. | concentrated |
| CV | Column Volume |
| dba | dibenzylideneacetone |
| DBU | 1,8-diazabicyclo[5.4.0]undec-7-ene |
| DCA | dichloroacetic acid |
| DCC | *N,N'*-dicyclohexyl-carbodiimide |
| DCE | 1,2-dichloroethane |
| DCM | dichloromethane |
| DDTT | 3-[(Dimethytaminomethylene)amino]-3H-1,2,4-dithiazole-5-thione |
| DEAD | diethyl azodicarboxylate |
| DIBAL | diisobutylaluminum hydride |
| DIPEA or DIEA | diisopropyl-ethyl amine |
| DMA | dimethylaniline |
| DMAP | 4-dimethylaminopyridine |
| DME | dimethoxyethane |
| DMF | *N,N*-dimethylformamide |
| DMP | Dess-Martin periodinane |
| DMSO | dimethylsulfoxide |
| DMTr | 4,4'-dimethoxytrityl |
| DPPA | diphenylphosphoryl azide |
| dppf | 1,1'-bis(diphenylphosphino)ferrocene |
| EA | ethyl acetate |
| EDCI | 1-ethy1-3-(3-dimethylaminopropyl) carbodiimide |
| ESI | electrospray ionization |
| EtOAc or EA | ethyl acetate |
| EtOH | ethanol |
| GCMS | gas chromatography-mass spectrometry |
| h or hr(s) | hour or hours |
| HEK | human embryonic kidney |
| HPLC | high performance liquid chromatography |
| LAH | lithium aluminum hydride |
| LDA | lithium diisopropylamide |
| LHMDS | lithium bis(trimethylsilyl)amide |
| Me | methyl |
| MEK | methyl ethyl ketone |
| MeOH | methanol |
| MeCN | acetonitrile |
| MHz | megahertz |
| min | minute or minutes |
| MS | mass spectrometry or molecular sieves |
| Ms | methanesulfonyl |
| NBS | *N*-bromosuccinimide |

| | |
|---|---|
| NIS | *N*-iodosuccinimide |
| NMM | *N*-methylmorpholine |
| NMP | *N*-methylpyrrolidone |
| NMR | nuclear magnetic resonance |
| PADS | phenylacetyl disulfide |
| PCC | pyridinium chlorochromate |
| PE | petrolum ether |
| RP | reverse-phase |
| rt or RT | room temperature |
| $R_t$ | retention time |
| Sec | second or seconds |
| SEM-Cl | 2-(trimethylsilyl)ethoxymethyl chloride |
| TBAF | tetrabutylammonium fluoride |
| TBS or TBDMS | t-butyldimethylsilyl |
| TBP | tributyl phosphate |
| TEA or $Et_3N$ | triethylamine |
| TEAA | triethylammoniumacetate |
| TFA | trifluoroacetic acid |
| THF | tetrahydrofuran |
| TIPS | triisopropylsilyl |
| TLC | thin layer chromatography |
| TMS | tetramethylsilane |
| Ts | 4-toluenesulfonyl |

Certain compounds 1-55 (including some not being part of the invention) according to Formula (I) may be prepared according to the process outlined in Scheme 1, below.

General Scheme 1

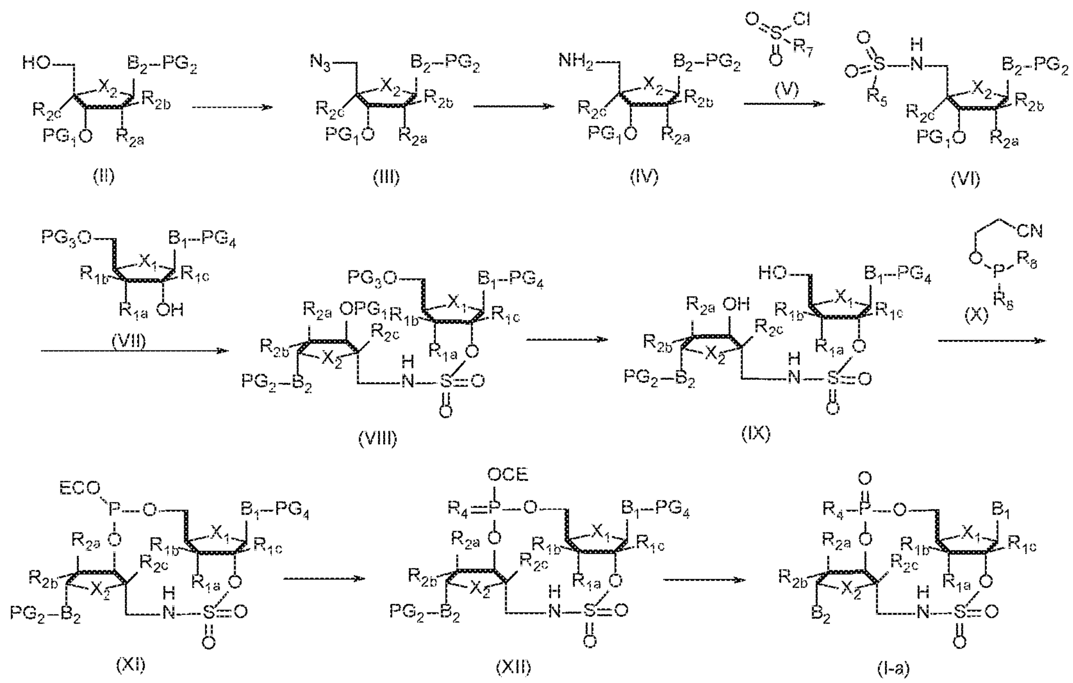


Accordingly, a suitably substituted compound of formula (II) in which $PG_1$ and $PG_2$ are protecting groups known to one of skill in the art, wherein $PG_1$ may be selected from acetyl, trimethylsilyl, tert-butyldimethyl silyl, benzyl, trityl, dimethoxytrityl or the like, and $PG_2$ may be selected from acyl, benzoyl, isobutyryl, or the like, a known compound or compound prepared by known methods, may be reacted with triphenylphosphine, sodium azide, in the presence of tetrabutylammonium iodide and carbon tetrabromide, in a suitably selected solvent or mixture of solvents such as DMF, THF, toluene, and the like, at a temperature ranging from about 0 °C to about 130 °C, to yield the corresponding compound of formula (III). Alternatively, a suitably substituted compound of formula (II), a known compound or compound prepared

by known methods, may be reacted with methanesulfonyl chloride, trifluoromethylsulfonyl chloride or the like, in the presence of a suitably selected base such as $Et_3N$, DIPEA, DMAP, and the like, in a suitably selected solvent or mixture of solvents such as $CHCl_3$, $CH_2Cl_2$, THF, pyridine, and the like, at a temperature ranging from about 0 °C to about 130 °C, to yield the corresponding mesyl or triflyl analogue, which may be further reacted with sodium azide in a suitably selected solvent or mixture of solvents such as DMF, THF, toluene, and the like, at a temperature ranging from about 0 °C to about 130 °C, to yield the corresponding compound of formula (III).

Yet another method may involve treating a suitably substituted compound of formula (II), with a combination of iodine, triphenyl phosphine and imidazole, in a suitable solvent such as pyridine, DMF, or the like; at a temperature ranging from about 0 °C to about 30 °C, to yield the corresponding iodo analogue, which may be further reacted with sodium azide in a suitably selected solvent or mixture of solvents such as DMF, THF, toluene, and the like, at a temperature ranging from about 0 °C to about 130 °C, to yield the corresponding compound of formula (III).

The compound of formula (III) may then be reacted with a source of hydrogen, under hydrogenation conditions, in the presence of a suitably selected catalyst or catalyst system, such as Pd/C, Pt, and the like, in a solvent such as MeOH, EtOH, EtOAc, and the like, to yield the corresponding compound of formula (IV). Alternatively, the compound of formula (III) may be reacted with triphenyl phosphine, in a suitable solvent such as THF, DMF, or the like; at a temperature ranging from about 20 °C to about 60 °C, followed by treatment with water at the same temperature to yield the corresponding compound of formula (IV).

The compound of formula (IV) may be reacted with a compound of formula (V) such as sulfuryl chloride, 4-nitrophenyl chlorosulfate, or the like, in the presence of a suitably selected base such as $Et_3N$, DIPEA, and the like, in a suitably selected solvent or mixture of solvents such as $CHCl_3$, $CH_2Cl_2$,THF, pyridine, and the like, at a temperature ranging from about -78 °C to about 50 °C, to yield the corresponding compound of formula (VI).

The compound of formula (VI) may then be reacted with a suitably substituted compound of formula (VII) in which $PG_3$ and $PG_4$ are protecting groups known to one of skill in the art, in which $PG_3$ might be selected from acetyl, trimethylsilyl, tert-butyldimethyl silyl, benzyl, trityl, dimethoxytrityl or the like, and $PG_2$ might be selected from acyl, benzoyl, isobutyryl, or the like, a known compound or compound prepared by known methods, in the presence of a suitably selected base such as $Et_3N$, DIPEA, DMAP, $Cs_2CO_3$ or the like, in a suitably selected solvent or mixture of solvents such as $CHCl_3$, $CH_2Cl_2$, THF, MeCN, pyridine, and the like, at a temperature ranging from about -10 °C to about 80 °C, to yield the corresponding compound of formula (VIII).

The alcohol protecting groups $PG_1$ and $PG_3$ of a compound of formula (VIII) may then be cleaved by methods well within the skill of persons versed in the art, in the presence of basic or acidic conditions, to yield the corresponding compound of formula (IX).

The compound of formula (IX) may then be reacted with a suitably substituted compound of formula (X) in which $R_8$ is halogen, diisopropylamino, or the like, a known compound or compound prepared by known methods, in the presence of a suitably activator such as tetrazole, DMAP, 5-ethylthio-1H-tetrazole, or the like, in a suitably selected solvent or mixture of solvents such as MeCN, $CH_2Cl_2$, THF, dioxane, and the like, at a temperature ranging from about -10 °C to about 60 °C, to yield the corresponding phosphite compound of formula (XI).

The compound of formula (XI) may then be reacted with an oxidant such as iodine, hydrogen peroxide, tert-butylperoxide, Beaucage reagent, DDTT, 3-amino-1,2,4-dithiazole-5-thione, PADS, and the like, or a $BH_3.SMe_2$, $BH_3$.THF complex, or the like, in a suitably selected solvent or mixture of solvents such as $CHCl_3$, $CH_2Cl_2$, THF, MeCN, dioxane, and the like, at a temperature ranging from about -10 °C to about 80 °C, to generate the compound of formula (XII) wherein $R_4$ is O, S or $BH_3$.

The compound of formula (XII) may then be deprotected using conditions basic conditions such as MeNH2, $tBuNH_2$, ammonium hydroxide, $Et_3N.3HF$ and the like, in a suitably selected solvent or mixture of solvents such as EtOH, water, iPrOH, and the like, at a temperature ranging from about -10 °C to about 120 °C, or by methods well within the skill of persons versed in the art, in the presence of basic or acidic conditions, to yield the corresponding compound of formula (I-a).

Alternatively, compounds of Formula (I) may be prepared according to the process outlined in General Scheme 2, below.

General Scheme 2

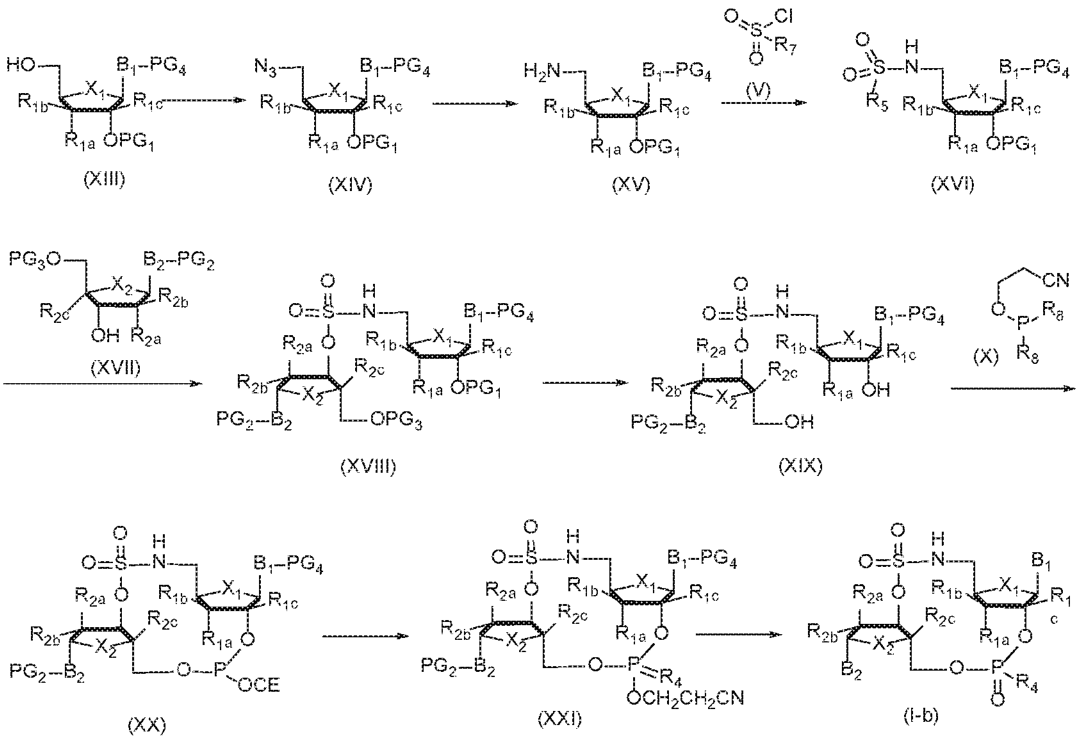


Accordingly, a suitably substituted compound of formula (XIII) in which $PG_1$ and $PG_4$ are protecting groups known to one of skill in the art, $PG_1$ may be selected from acetyl, trimethylsilyl, tert-butyldimethyl silyl, benzyl, trityl, dimethoxytrityl, or the like, and $PG_4$ might be selected from acyl, benzoyl, isobutyryl, or the like, a known compound or compound prepared by known methods, may be reacted with triphenylphosphine, sodium azide, in the presence of tetrabutylammonium iodide and carbon tetrabromide in a suitably selected solvent or mixture of solvents such as DMF, THF, toluene, and the like, at a temperature ranging from about 0 °C to about 130 °C, to yield the corresponding compound of formula (XIV).

Alternatively, a suitably substituted compound of formula (XIII), a known compound or compound prepared by known methods, may be reacted with methanesulfonyl chloride, trifluoromethylsulfonyl chloride, or the like, in the presence of a suitably selected base such as $Et_3N$, DIPEA, DMAP, or the like, in a suitably selected solvent or mixture of solvents such as $CHCl_3$, $CH_2Cl_2$, THF, pyridine, and the like, at a temperature ranging from about 0 °C to about 130 °C, to yield the corresponding mesyl or triflyl analogue, which may then be further reacted with sodium azide, in a suitably selected solvent or mixture of solvents such as DMF, THF, toluene, and the like, at a temperature ranging from about 0 °C to about 130 °C, to yield the corresponding compound of formula (XIV). Yet another method may involve treating a suitably substituted compound of formula (XIII), with a combination of iodine, triphenyl phosphine and imidazole, in a suitable solvent such as pyridine, DMF, or the like, at a temperature ranging from about 0 °C to about 30 °C, to yield the corresponding iodo analogue, which may be further reacted with sodium azide in a suitably selected solvent or mixture of solvents such as DMF, THF, toluene, and the like, at a temperature ranging from about 0 °C to about 130 °C, to yield the corresponding compound of formula (XIV).

The compound of formula (XIV) may then be reacted with a source of hydrogen, under hydrogenation conditions, in the presence of a suitably selected catalyst or catalyst system, such as Pd/C, Pt, and the like, in a solvent such as MeOH, EtOH, EtOAc, and the like, to yield the corresponding compound of formula (XV). Alternatively, the compound of formula (XIV) may be reacted with triphenyl phosphine, in a suitable solvent such as THF, DMF, or the like, at a temperature ranging from about 20 °C to about 60 °C, followed by treatment with water at the same temperature to yield the corresponding compound of formula (XV).

The compound of formula (XV) may be reacted with a compound of formula (V) such as sulfuryl chloride, 4-nitrophenyl chlorosulfate, or the like, in the presence of a suitably selected base such as $Et_3N$, DIPEA, or the like, in a suitably selected solvent or mixture of solvents such as $CHCl_3$, $CH_2Cl_2$, THF, pyridine, and the like, at a temperature ranging from about -78 °C to about 50 °C, to yield the corresponding compound of formula (XVI).

The compound of formula (XVI) may then be reacted with a suitably substituted compound of formula (XVII) in which $PG_2$ and $PG_3$ are protecting groups known to one of skill in the art, in which $PG_3$ may be selected from acetyl, trimethylsilyl, tert-butyldimethyl silyl, benzyl, trityl, dimethoxytrityl, or the like, and $PG_2$ may be selected from acyl, benzoyl,

isobutyryl, or the like, a known compound or compound prepared by known methods, in the presence of a suitably selected base such as $Et_3N$, DIPEA, DMAP, $Cs_2CO_3$ or the like, in a suitably selected solvent or mixture of solvents such as $CHCl_3$, $CH_2Cl_2$, THF, MeCN, pyridine, and the like, at a temperature ranging from about -10 °C to about 80 °C, to yield the corresponding compound of formula (XVIII). The alcohol protecting groups $PG_1$ and $PG_3$ in compound of formula (XVIII) may then be cleaved by methods well within the skill of persons versed in the art in the presence of basic or acidic conditions to yield the corresponding compound of formula (XIX).

The compound of formula (XIX) may then be reacted with a suitably substituted compound of formula (X) in which $R_8$ is halogen, diisopropylamino and the like, a known compound or compound prepared by known methods, in the presence of a suitably activator such as tetrazole, DMAP, 5-ethylthio-1H-tetrazole, or the like, in a suitably selected solvent or mixture of solvents such as MeCN. $CH_2Cl_2$, THF, dioxane, and the like, at a temperature ranging from about -10 °C to about 60 °C, to yield the corresponding phosphite compound of formula (XX).

The compound of formula (XX) may then be reacted with an oxidant such as iodine, hydrogen peroxide, tert-butylperoxide, Beaucage reagent, DDTT, 3-amino-1,2,4-dithiazole-5-thione, PADS and the like, or a $BH_3.SMe_2$, $BH_3.THF$ complex, or the like, in a suitably selected solvent or mixture of solvents such as $CHCl_3$, $CH_2Cl_2$, THF, MeCN, dioxane, and the like, at a temperature ranging from about -10 °C to about 80 °C, to generate the compound of formula (XXI) wherein $R_4$ is O, S or $BH_3$.

The compound of formula (XXI) may then be deprotected using conditions basic conditions such as $MeNH_2$, $tBuNH_2$, ammonium hydroxide, $Et_3N.3HF$ and the like, in a suitably selected solvent or mixture of solvents such as EtOH, water, iPrOH, and the like, at a temperature ranging from about -10 °C to about 120 °C, or by methods well within the skill of persons versed in the art, in the presence of basic or acidic conditions, to yield the corresponding compound of formula (I-b).

Alternatively, compounds of Formula (I) may be prepared according to the process outlined in General Scheme 3, below.

General Scheme 3

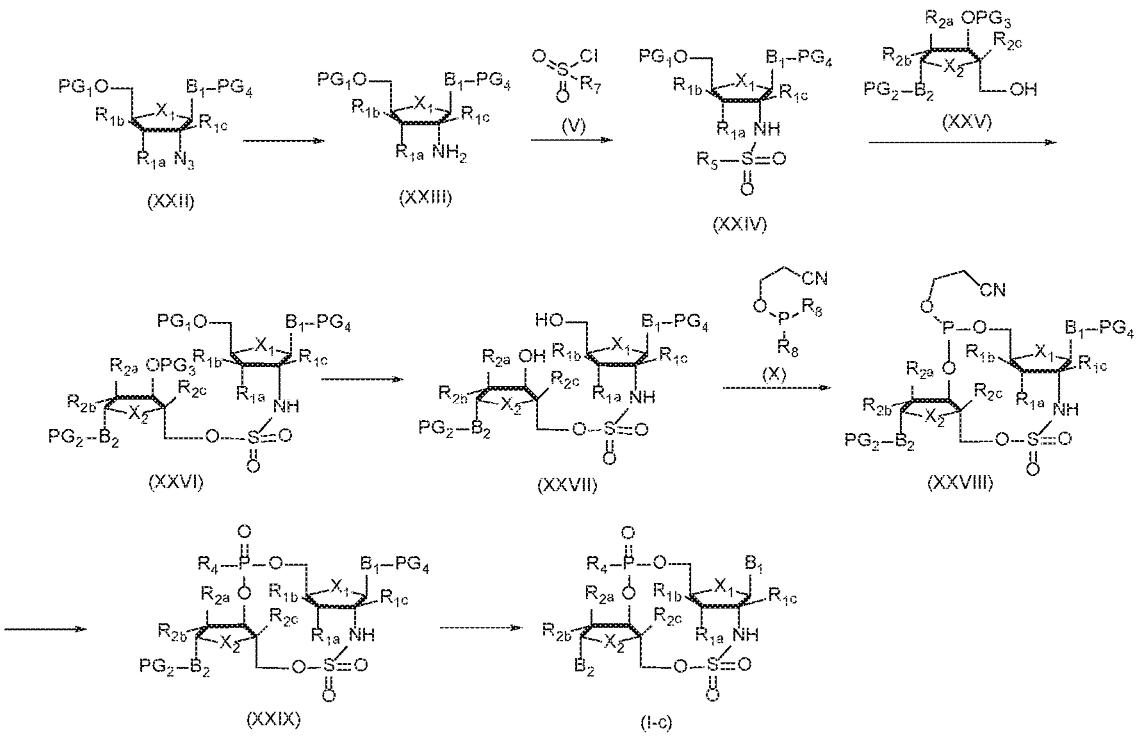


Accordingly, a suitably substituted compound of formula (XXII) in which $PG_1$ and $PG_4$ are protecting groups known to one of skill in the art, $PG_1$ may be selected from acetyl, trimethylsilyl, tert-butyldimethyl silyl, benzyl, trityl, dimethoxytrityl or the like, and $PG_4$ may be selected from acyl, benzoyl, isobutyryl, or the like, a known compound or compound prepared by known methods, may be reacted with a source of hydrogen, under hydrogenation conditions, in the presence of a suitably selected catalyst or catalyst system, such as Pd/C, Pt, and the like, in a solvent such as MeOH, EtOH, EtOAc, and the like, to yield the corresponding compound of formula (XXIII). Alternatively, the compound

of formula (XXII) may be reacted with triphenyl phosphine, in a suitable solvent such as THF, DMF, or the like, at a temperature ranging from about 20 °C to about 60 °C, followed by treatment with water at the same temperature to yield the corresponding compound of formula (XXIII).

The compound of formula (XXIII) may be reacted with a compound of formula (V) such as sulfuryl chloride, 4-nitrophenyl chlorosulfate, or the like, in the presence of a suitably selected base such as $Et_3N$, DIPEA, or the like, in a suitably selected solvent or mixture of solvents such as $CHCl_3$, $CH_2Cl_2$, THF, pyridine, and the like, at a temperature ranging from about -78 °C to about 50 °C, to yield the corresponding compound of formula (XXIV).

The compound of formula (XXIV) may then be reacted with a suitably substituted compound of formula (XXV) in which $PG_2$ and $PG_3$ are protecting groups known to one of skill in the art, in which $PG_3$ may be selected from acetyl, trimethylsilyl, tert-butyldimethyl silyl, benzyl, trityl, dimethoxytrityl, or the like, and $PG_2$ may be selected from acyl, benzoyl, isobutyryl, or the like, a known compound or compound prepared by known methods, in the presence of a suitably selected base such as $Et_3N$, DIPEA, DMAP, $Cs_2CO_3$, or the like, in a suitably selected solvent or mixture of solvents such as $CHCl_3$, $CH_2Cl_2$, THF, MeCN, pyridine, and the like, at a temperature ranging from about -10 °C to about 80 °C, to yield the corresponding compound of formula (XXVI).

The alcohol protecting groups $PG_1$ and $PG_3$ in a compound of formula (XXVI) may then be cleaved by methods well within the skill of persons versed in the art, in the presence of basic or acidic conditions, to yield the corresponding compound of formula (XXVII).

The compound of formula (XXVII) may then be reacted with a suitably substituted compound of formula (X) in which $R_8$ is halogen, diisopropylamino or the like, a known compound or compound prepared by known methods, in the presence of a suitable activator such as tetrazole, DMAP, 5-ethylthio-1H-tetrazole, or the like, in a suitably selected solvent or mixture of solvents such as MeCN, $CH_2Cl_2$, THF, dioxane, and the like, at a temperature ranging from about -10 °C to about 60 °C, to yield the corresponding phosphite compound of formula (XXVIII).

The compound of formula (XXVIII) may then be reacted with an oxidant such as iodine, hydrogen peroxide, tert-butylperoxide, Beaucage reagent, DDTT, 3-amino-1,2,4-dithiazole-5-thione, PADS or the like, or a $BH_3.SMe_2$, $BH_3$.THF complex, or the like, in a suitably selected solvent or mixture of solvents such as $CHCl_3$, $CH_2Cl_2$, THF, MeCN, dioxane, and the like, at a temperature ranging from about -10 °C to about 80 °C, to generate the compound of formula (XXIX) wherein $R_4$ is O, S or $BH_3$.

The compound of formula (XXIX) may then be deprotected using basic conditions such as $MeNH_2$, $tBuNH_2$, ammonium hydroxide, $Et_3N.3HF$, or the like, in a suitably selected solvent or mixture of solvents such as EtOH, water, iPrOH, and the like, at a temperature ranging from about -10 °C to about 120 °C, or by methods well within the skill of persons versed in the art, in the presence of basic or acidic conditions, to yield the corresponding compound of formula (I-c).

Alternatively, compounds of Formula (I) may be prepared according to the process outlined in General Scheme 4, below.

General Scheme 4

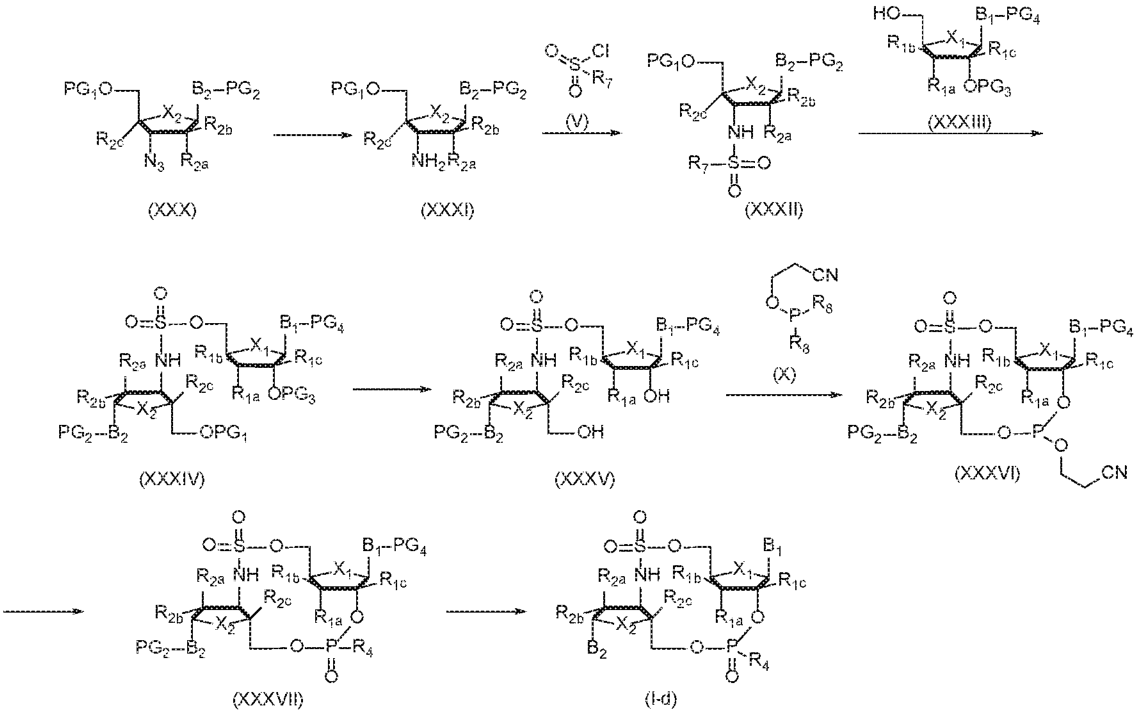


(XXX) (XXXI) (XXXII) (XXXIV) (XXXV) (XXXVI) (XXXVII) (I-d)

Accordingly, a suitably substituted compound of formula (XXX) in which $PG_1$ and $PG_2$ are protecting groups known to one of skill in the art, $PG_1$ might be selected from acetyl, trimethylsilyl, tert-butyldimethyl silyl, benzyl, trityl, dimethoxytrityl or the like, and $PG_2$ might be selected from acyl, benzoyl, isobutyryl, or the like, a known compound or compound prepared by known methods, may be reacted with a source of hydrogen, under hydrogenation conditions, in the presence of a suitably selected catalyst or catalyst system, such as Pd/C, Pt, and the like, in a solvent such as MeOH, EtOH, EtOAc, and the like, to yield the corresponding compound of formula (XXXI). Alternatively, the compound of formula (XXX) may be reacted with triphenyl phosphine, in a suitable solvent such as THF, DMF, or the like, at a temperature ranging from about 20 °C to about 60 °C, followed by treatment with water at the same temperature to yield the corresponding compound of formula (XXXI).

The compound of formula (XXXI) may be reacted with a compound of formula (V) such as sulfuryl chloride, 4-nitrophenyl chlorosulfate, or the like, in the presence of a suitably selected base such as $Et_3N$, DIPEA, or the like, in a suitably selected solvent or mixture of solvents such as $CHCl_3$, $CH_2Cl_2$, THF, pyridine, and the like, at a temperature ranging from about -78 °C to about 50 °C, to yield the corresponding compound of formula (XXXII).

The compound of formula (XXXII) may then be reacted with a suitably substituted compound of formula (XXXIII) in which $PG_3$ and $PG_4$ are protecting groups known to one of skill in the art, in which $PG_3$ may be selected from acetyl, trimethylsilyl, tert-butyldimethyl silyl, benzyl, trityl, dimethoxytrityl or the like, and $PG_2$ may be selected from acyl, benzoyl, isobutyryl, or the like, a known compound or compound prepared by known methods, in the presence of a suitably selected base such as $Et_3N$, DIPEA, DMAP, $Cs_2CO_3$, or the like, in a suitably selected solvent or mixture of solvents such as $CHCl_3$, $CH_2Cl_2$, THF, MeCN, pyridine, and the like, at a temperature ranging from about -10 °C to about 80 °C, to yield the corresponding compound of formula (XXXIV).

The alcohol protecting groups $PG_1$ and $PG_3$ in a compound of formula (XXXIV) may then be cleaved by methods well within the skill of persons versed in the art, in the presence of basic or acidic conditions, to yield the corresponding compound of formula (XXXV).

The compound of formula (XXXV) may then be reacted with a suitably substituted compound of formula (X) in which $R_8$ is halogen, diisopropylamino and the like, a known compound or compound prepared by known methods, in the presence of a suitably activator such as tetrazole, DMAP, 5-ethylthio-1H-tetrazole, or the like, in a suitably selected solvent or mixture of solvents such as MeCN, $CH_2Cl_2$, THF, dioxane, and the like, at a temperature ranging from about -10 °C to about 60 °C, to yield the corresponding phosphite compound of formula (XXXVI).

The compound of formula (XXXVI) may then be reacted with an oxidant such as iodine, hydrogen peroxide, tert-butylperoxide, Beaucage reagent, DDTT, 3-amino-1,2,4-dithiazole-5-thione, PADS, or the like, or a $BH_3.SMe_2$, $BH_3$.THF complex, or the like, in a suitably selected solvent or mixture of solvents such as $CHCl_3$, $CH_2Cl_2$, THF, MeCN, dioxane, and the like, at a temperature ranging from about -10 °C to about 80 °C, to generate the compound of formula

(XXXVII) wherein $R_4$ is O, S or $BH_3$.

The compound of formula (XXXVII) may then be deprotected using basic conditions such as $MeNH_2$, $tBuNH_2$, ammonium hydroxide, $Et_3N.3HF$, or the like, in a suitably selected solvent or mixture of solvents such as EtOH, WATER, iPrOH, and the like, at a temperature ranging from about -10 °C to about 120 °C, or by methods well within the skill of persons versed in the art, in the presence of basic or acidic conditions, to yield the corresponding compound of formula (I-d).

General Scheme 5

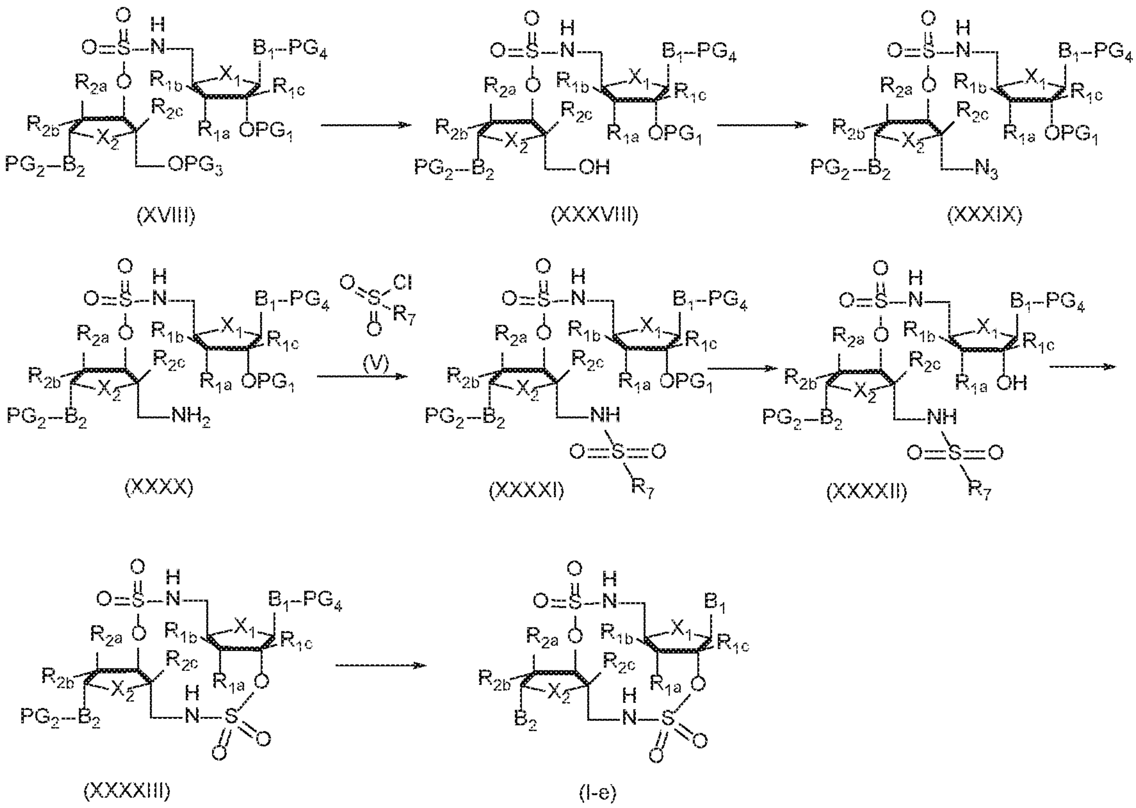


(XVIII) (XXXVIII) (XXXIX)

(XXXX) (XXXXI) (XXXXII)

(XXXXIII) (I-e)

Accordingly, the alcohol protecting group $PG_3$ in a compound of formula (XVIII) in which $PG_1$, $PG_2$, $PG_3$ and $PG_4$ are protecting groups known to one of skill in the art, $PG_1$ and $PG_3$ may be selected from acetyl, trimethylsilyl, tert-butyldimethyl silyl, benzyl, trityl, dimethoxytrityl, or the like, and $PG_2$ and $PG_4$ may be selected from acyl, benzoyl, isobutyryl, or the like, a known compound or compound prepared by known methods, may be cleaved selectively in the presence of the alcohol protecting group $PG_1$ by methods well within the skill of persons versed in the art, in the presence of basic or acidic conditions, to yield the corresponding compound of formula (XXXVIII).

The compound of formula (XXXVIII) may be reacted with triphenylphosphine, sodium azide, in the presence of tetrabutylammonium iodide and carbon tetrabromide, in a suitably selected solvent or mixture of solvents such as DMF, THF, toluene, and the like, at a temperature ranging from about 0 °C to about 130 °C, to yield the corresponding compound of formula (XXXIX). Alternatively, a suitably substituted compound of formula (XVIII), a known compound or compound prepared by known methods, may be reacted with methanesulfonyl chloride, trifluoromethylsulfonyl chloride or the like, in the presence of a suitably selected base such as $Et_3N$, DIPEA, DMAP, or the like, in a suitably selected solvent or mixture of solvents such as $CHCl_3$, $CH_2Cl_2$, THF, pyridine, and the like, at a temperature ranging from about 0 °C to about 130 °C, to yield the corresponding mesyl or triflyl analogue, which may be further reacted with sodium azide in a suitably selected solvent or mixture of solvents such as DMF, THF, toluene, and the like, at a temperature ranging from about 0 °C to about 130 °C, to yield the corresponding compound of formula (XXXIX).

Yet another method may involve treating a suitably substituted compound of formula (XVIII),with a combination of iodine, triphenyl phosphine and imidazole, in a suitable solvent like pyridine or DMF, or the like, at a temperature ranging from about 0 °C to about 30 °C, to yield the corresponding iodo analogue, which may be further reacted with sodium azide in a suitably selected solvent or mixture of solvents such as DMF, THF, toluene, and the like, at a temperature ranging from about 0 °C to about 130 °C, to yield the corresponding compound of formula (XXXIX).

The compound of formula (XXXIX) may then be reacted with a source of hydrogen, under hydrogenation

conditions, in the presence of a suitably selected catalyst or catalyst system, such as Pd/C, Pt, and the like, in a solvent such as MeOH, EtOH, EtOAc, or the like, to yield the corresponding compound of formula (XXXX). Alternatively, the compound of formula (XXXIX) may be reacted with triphenyl phosphine, in a suitable solvent such as THF, DMF, or the like, at a temperature ranging from about 20 °C to about 60 °C, followed by treatment with water at the same temperature to yield the corresponding compound of formula (XXXX).

The compound of formula (XXXX) may be reacted with a compound of formula (V) such as sulfuryl chloride, 4-nitrophenyl chlorosulfate, or the like, in the presence of a suitably selected base such as $Et_3N$, DIPEA, or the like, in a suitably selected solvent or mixture of solvents such as $CHCl_3$, $CH_2Cl_2$, THF, pyridine, and the like, at a temperature ranging from about -78 °C to about 50 °C, to yield the corresponding compound of formula (XXXXI). The alcohol protecting group $PG_1$ in a compound of formula (XXXIV) may then be cleaved by methods well within the skill of persons versed in the art, in the presence of basic or acidic conditions, to yield the corresponding compound of formula (XXXXII).

The compound of formula (XXXXII) may be reacted in the presence of a suitably selected base such as $Et_3N$, DIPEA, DMAP, $Cs_2CO_3$, or the like, in a suitably selected solvent or mixture of solvents such as $CHCl_3$, $CH_2Cl_2$, THF, MeCN, pyridine, and the like, at a temperature ranging from about -10 °C to about 80 °C, to yield the corresponding compound of formula (XXXXIII).

The compound of formula (XXXXIII) may then be deprotected using basic conditions such as $MeNH_2$, $tBuNH_2$, ammonium hydroxide, $Et_3N.3HF$ and the like, in a suitably selected solvent or mixture of solvents such as EtOH, water, iPrOH, and the like, at a temperature ranging from about -10 °C to about 120 °C, or by methods well within the skill of persons versed in the art, in the presence of basic or acidic conditions, to yield the corresponding compound of formula (I-e).

## General Scheme 6

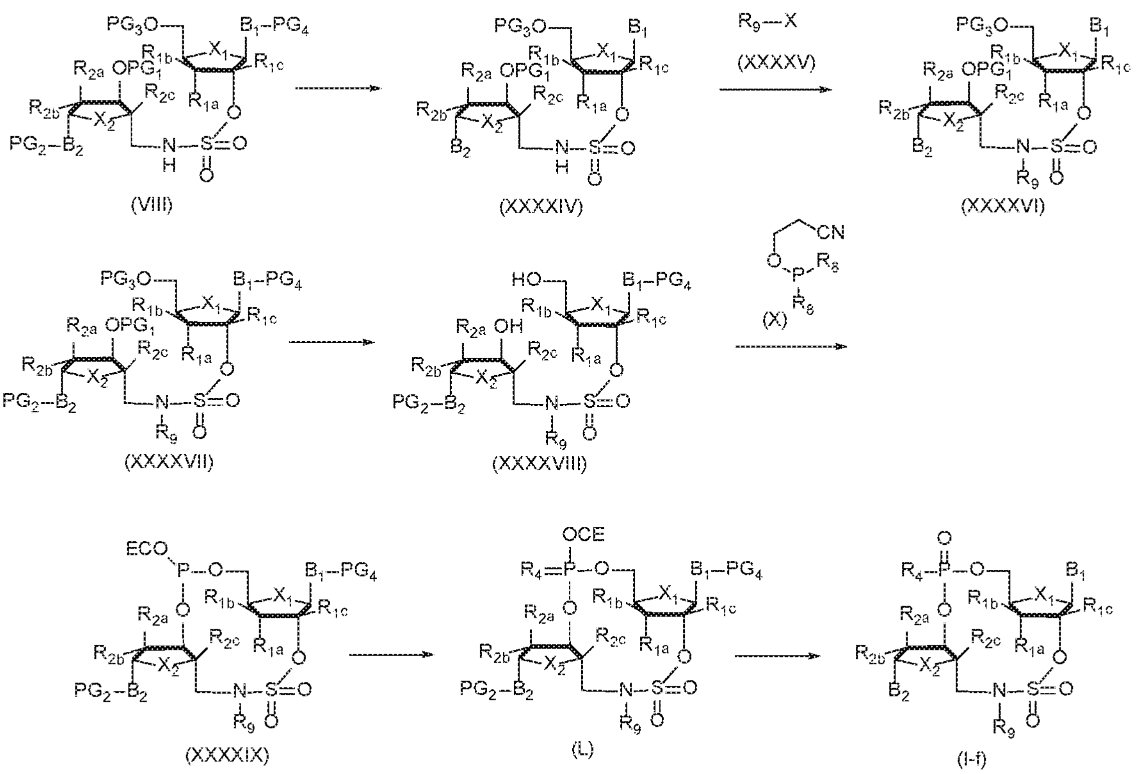


Accordingly, the alcohol protecting groups $PG_2$ and $PG_4$ in a compound of formula (VIII) in which $PG_1$, $PG_2$. $PG_3$ and $PG_4$ are protecting groups known to one of skill in the art, $PG_1$ and $PG_3$ may be selected from acetyl, trimethylsilyl, tert-butyldimethyl silyl, benzyl, trityl, dimethoxytrityl, or the like, and $PG_2$ and $PG_4$ may be selected from acyl, benzoyl, isobutyryl, or the like, a known compound or compound prepared by known methods, may be cleaved selectively in the presence of the alcohol protecting groups $PG_1$ and $PG_3$ by methods well within the skill of persons versed in the art, in the presence of basic or acidic conditions, to yield the corresponding compound of formula (XXXXIV).

The compound of formula (XXXXIV) may be reacted with a compound of formula (XXXXV) such as iodomethane or bromoethane wherein $R_9$ is optionally substituted $C_{1-3}$ alkyl, or the like, in the presence of a suitably selected base

such as $NaHCO_3$, $K_2CO_3$, $Et_3N$, DIPEA, or the like, in a suitably selected solvent or mixture of solvents such as $CHCl_3$, $CH_2Cl_2$, THF, DMF, and the like, at a temperature ranging from about -78 °C to about 80 °C, to yield the corresponding compound of formula (XXXXVI). The protecting groups $PG_2$ and $PG_4$ are then re-introduced in (XXXXVI) using protecting groups known to one of skill in the art, selected from acyl, benzoyl, isobutyryl, or the like, a known compound or compound prepared by known methods, in the presence of a suitably selected base such as $Et_3N$, DIPEA, DMAP, $Cs_2CO_3$, or the like, in a suitably selected solvent or mixture of solvents such as $CHCl_3$, $CH_2Cl_2$, THF, MeCN, pyridine, and the like, at a temperature ranging from about -10 °C to about 80 °C, to yield the corresponding compound of formula (XXXXVII).
The alcohol protecting groups $PG_1$ and $PG_3$ in a compound of formula (XXXXVII) may then be cleaved selectively in the presence of the protecting groups $PG_2$ and $PG_4$ by methods well within the skill of persons versed in the art, in the presence of basic or acidic conditions, to yield the corresponding compound of formula (XXXXVIII).
The compound of formula (XXXXVIII) may then be reacted with a suitably substituted compound of formula (X) in which $R_8$ is halogen, diisopropylamino or the like, a known compound or compound prepared by known methods, in the presence of a suitable activator such as tetrazole, DMAP, 5-ethylthio-1H-tetrazole, or the like, in a suitably selected solvent or mixture of solvents such as MeCN, $CH_2Cl_2$, THF, dioxane, and the like, at a temperature ranging from about -10 °C to about 60 °C, to yield the corresponding phosphite compound of formula (XXXXIX).

The compound of formula (XXXXIX) may then be reacted with an oxidant such as iodine, hydrogen peroxide, tert-butylperoxide, Beaucage reagent, DDTT, 3-amino-1,2,4-dithiazole-5-thione, PADS or the like, or a $BH_3.SMe_2$, $BH_3$.THF complex, or the like, in a suitably selected solvent or mixture of solvents such as $CHCl_3$, $CH_2Cl_2$, THF, MeCN, dioxane, and the like, at a temperature ranging from about -10 °C to about 80 °C, to generate the compound of formula (L) wherein $R_4$ is O, S or $BH_3$.

The compound of formula (L) may then be deprotected using basic conditions such as $MeNH_2$, $tBuNH_2$, ammonium hydroxide, $Et_3N.3HF$, or the like, in a suitably selected solvent or mixture of solvents such as EtOH, water, iPrOH, and the like, at a temperature ranging from about -10 °C to about 120 °C, or by methods well within the skill of persons versed in the art, in the presence of basic or acidic conditions, to yield the corresponding compound of formula (I-f).

Alternatively, compounds of Formula (I) may be prepared according to the process outlined in General Scheme 7, below.

Scheme 7

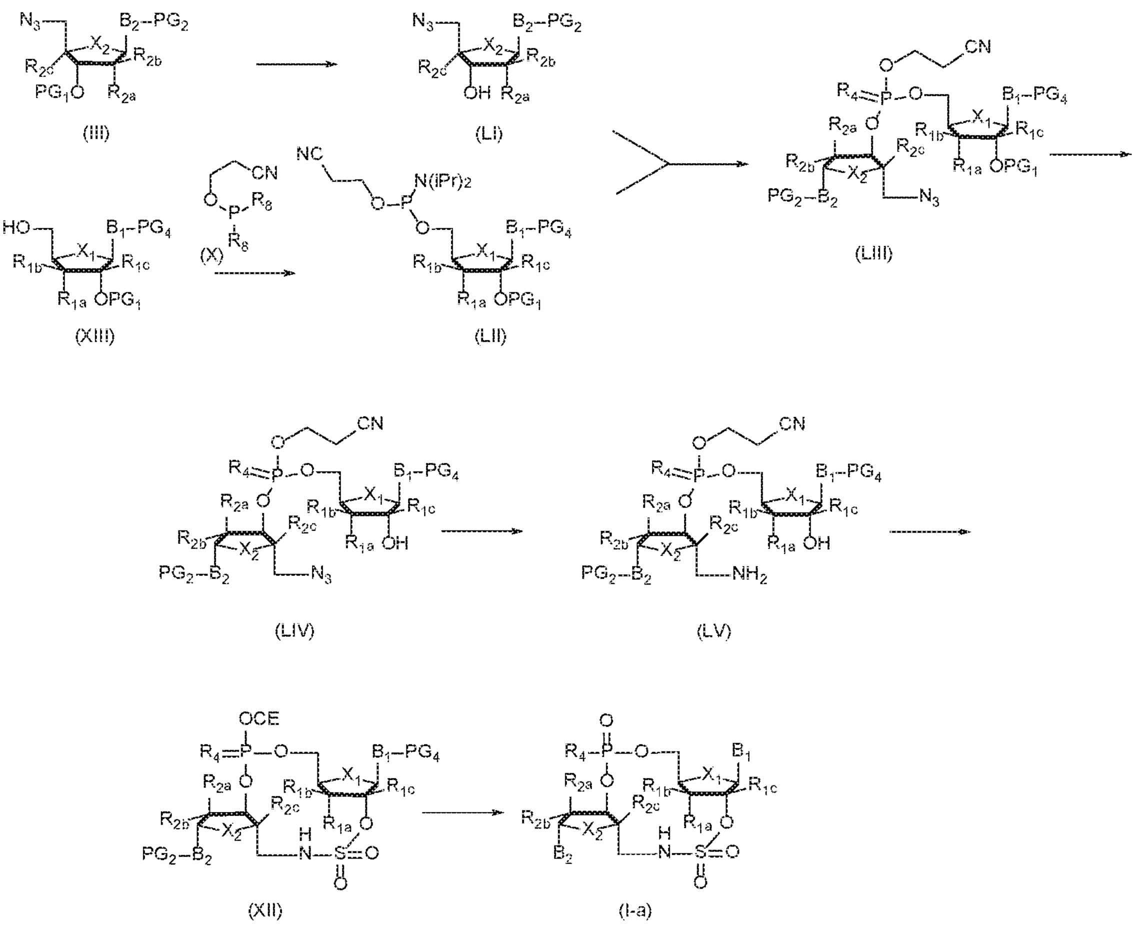

(III) (LI) (XIII) (X) (LII) (LIII) (LIV) (LV) (XII) (I-a)

Accordingly, the alcohol protecting groups $PG_1$ in a compound of formula (III) in which $PG_1$ and $PG_2$ are protecting groups known to one of skill in the art, $PG_1$ may be selected from acetyl, trimethylsilyl, tert-butyldimethyl silyl, benzyl, trityl, dimethoxytrityl, or the like, and $PG_2$ may be selected from acyl, benzoyl, isobutyryl, or the like, a known compound or compound prepared by known methods, may be cleaved selectively in the presence of the alcohol protecting groups $PG_2$ by methods well within the skill of persons versed in the art, in the presence of basic or acidic conditions, to yield the corresponding compound of formula (L1).

The compound of formula (XIII) in which $PG_1$ and $PG_4$ are protecting groups known to one of skill in the art, $PG_1$ may be selected from acetyl, trimethylsilyl, tert-butyldimethyl silyl, benzyl, trityl, dimethoxytrityl, or the like, and $PG_4$ may be selected from acyl, benzoyl, isobutyryl, or the like, a known compound or compound prepared by known methods, may then be reacted with a suitably substituted compound of formula (X) in which $R_8$ is halogen, diisopropylamino, or the like, a known compound or compound prepared by known methods, in the presence of a suitably activator such as tetrazole, DMAP, 5-ethylthio-1H-tetrazole, or the like, in a suitably selected solvent or mixture of solvents such as MeCN, $CH_2Cl_2$, THF, dioxane, and the like, at a temperature ranging from about -10 °C to about 60 °C, to yield the corresponding phosphite compound of formula (LII).

The compound of formula (LI) may then be reacted with the compound of formula (LII) in the presence or not of a suitable activator such as tetrazole, DMAP, 5-ethylthio-1H-tetrazole, or the like, in a suitably selected solvent or mixture of solvents such as MeCN, $CH_2Cl_2$, THF, DMF, dioxane, and the like, at a temperature ranging from about - 10 °C to about 60 °C, to yield the corresponding phosphite which is oxidized in situ with an oxidant such as iodine, hydrogen peroxide, tert-butylperoxide, Beaucage reagent, DDTT, 3-amino-1,2,4-dithiazole-5-thione, PADS or the like, or a $BH_3.SMe_2$, $BH_3$. THF complex, or the like, in a suitably selected solvent or mixture of solvents such as $CHCl_3$, $CH_2Cl_2$, THF, MeCN, dioxane, and the like, at a temperature ranging from about -10 °C to about 80 °C, to generate the compound of formula (LIII) wherein $R_4$ is O, S or $BH_3$.

The alcohol protecting group $PG_1$ in the compound of formula (LIII) is cleaved selectively in the presence of the alcohol protecting groups $PG_2$ and $PG_4$ by methods well within the skill of persons versed in the art, in the presence

of basic or acidic conditions, to yield the corresponding compound of formula (LIV).

The compound of formula (LIV) may then be reacted with a source of hydrogen, under hydrogenation conditions, in the presence of a suitably selected catalyst or catalyst system, such as Pd/C, Pt, and the like, in a solvent such as MeOH, EtOH, EtOAc, or the like, to yield the corresponding compound of formula (LV). Alternatively, the compound of formula (LIV) may be reacted with triphenyl phosphine, in a suitable solvent such as THF, DMF, or the like, followed by water at a temperature ranging from about 20 °C to about 60 °C, followed by treatment with water at the same temperature to yield the corresponding compound of formula (LV).

The compound of formula (LV) may be reacted with a reagent or known reagent such as sulfuryl chloride, 1,1'-sulfonyldiimidazole, 4-nitrophenyl chlorosulfate, or the like, in the presence or not of a suitably selected base such as $Et_3N$, DIPEA, or the like, in a suitably selected solvent or mixture of solvents such as $CHCl_3$, $CH_2Cl_2$, THF, pyridine, and the like, at a temperature ranging from about -78 °C to about 50 °C, to yield the corresponding compound of formula (XII).

The compound of formula (XII) may then be deprotected using acidic or basic conditions or by methods well known within the skill of persons versed in the art, to yield the corresponding compound of formula (I-a).

Specific Examples

Example 1

## Compound 1

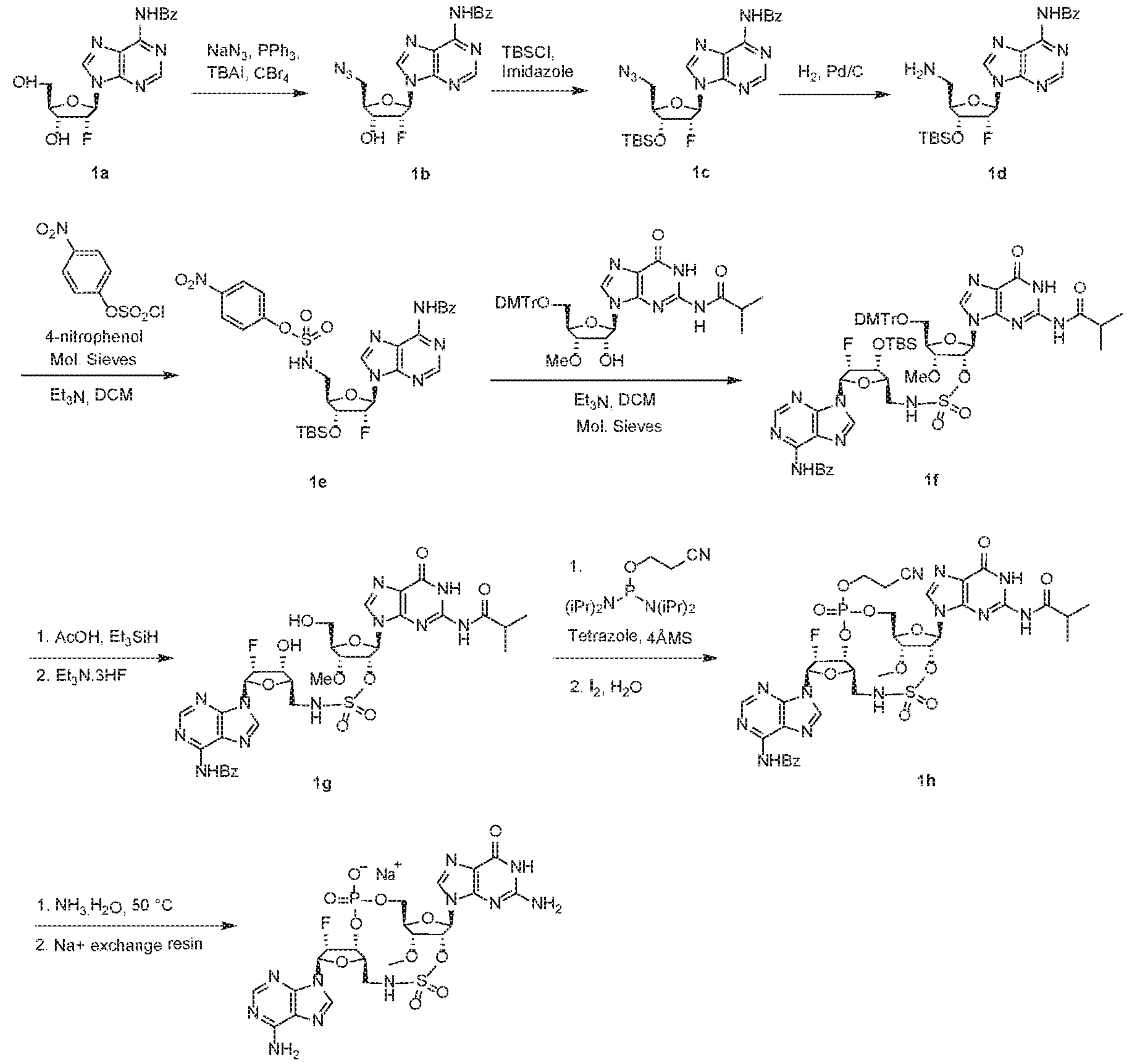


1a 1b 1c 1d 1e 1f 1g 1h

## Compound 1, sodium salt

Step 1: preparation of compound **1b**

Compound **1a** (5.48 g, 14.7 mmol, co-evaporated with anhydrous toluene (2x)) was dissolved in anhydrous DMF (87 mL). Triphenylphosphine (5.77 g, 22.0 mmol), sodium azide (3.06 g, 47.1 mmol), tetrabutylammonium iodide (1.08 g, 2.94 mmol) and carbon tetrabromide (7.30 g, 22.0 mmol) were added. The reaction mixture was stirred at room temperature overnight, followed by concentration under reduced pressure. The resulting residue was purified by flash column chromatography over silica gel (gradient elution: 0-10% MeOH in DCM) to give compound **1b** as a white solid powder (5.09 g, yield: 87%). ESI-MS: *m/z* 399.0 $[M+H]^+$.

Step 2: preparation of compound **1c**

Imidazole (0.51 g 7.5 mmol) and TBSCl (0.76 g, 5.0 mmol) were added to a solution of compound **1b** (1.0 g, 2.5 mmol) in DMF (15 mL), the reaction mixture was stirred at room temperature overnight. The mixture was poured into water and extracted with EtOAc, the combined organic layers were washed with brine, dried with anhydrous $Na_2SO_4$ and concentrated under reduced pressure. The crude product was purified by flash column chromatography over silica gel (gradient elution: 0 - 50% EtOAc in petroleum ether) to give compound **1c** as a white solid (1.2 g, yield: 93%). $^1H$ NMR (400 MHz, CHLOROFORM-d) δ ppm 0.18 (s, 6 H), 0.95 (s, 9 H), 3.52 (dd, J=13.6, 4.3 Hz, 1 H), 3.78 (dd, J=13.6,

3.0 Hz, 1 H), 4.25 (m, J=7.2, 3.5, 3.5 Hz, 1 H), 4.85 (ddd, J=18.8, 7.5, 4.5 Hz, 1 H), 5.53 (ddd, J=53.0, 4.5, 1.8 Hz, 1 H), 6.24 (dd, J=18.2, 1.9 Hz, 1 H), 7.50 - 7.58 (m, 2 H), 7.59 - 7.67 (m, 1 H), 7.99 - 8.08 (m, 2 H), 8.23 (s, 1 H), 8.80 (s, 1 H), 9.04 (br s, 1 H); ESI-MS: *m/z* 513.1 $[M+H]^+$.

Step 3: preparation of compound **1d**

A solution of compound **1c** (1.15 g, 2.24 mmol) in EtOAc (50 mL) was hydrogenated under atmospheric pressure at room temperature with Pd/C (20% on carbon, 132 mg, 0.224 mmol) as a catalyst After uptake of hydrogen, the catalyst was removed by filtration, and the filtrate was evaporated to give compound **1d** (1.1 g) as a white solid. The crude product was immediately used as such in the next step. ESI-MS: *m/z* 509.1 $[M+Na]^+$.

Step 4: preparation of compound **1e**

Compound **1d** (710 mg, 1.16 mmol), 4-nitrophenol (485 mg, 3.49 mmol) and $Et_3N$ (965 μL, 6.98 mmol) were dissolved in DCM (30 mL) followed by the addition of 4Å molecular sieves (500 mg). The resulting mixture was cooled to -78 °C, followed by the addition of 4-nitrophenyl chlorosulfate (830 mg, 3.49 mmol) in DCM (4 mL). The reaction mixture was stirred for 2.5 h at - 78 °C. Aqueous $NaHCO_3$ was added, the aqueous layer was separated and extracted with DCM The combined organic layers were dried over anhydrous $Na_2SO_4$, filtered and concentrated under reduced pressure. The crude product was purified by flash column chromatography over silica gel (gradient elution: 0 - 50% EtOAc in petroleum ether) to give compound **1e** as a white solid (546 mg, purity: 89%, yield: 56% starting from compound **ld).** $^1H$ NMR (400 MHz, CHLOROFORM-*d*) δ ppm 0.17 (s, 3 H), 0.18 (s, 3 H), 0.96 (s, 9 H), 3.64 - 3.75 (m, 2 H), 4.43 (br s, 1 H), 4.67 - 4.74 (m, 1 H), 5.51 (dt, *J*=51.8, 5.4 Hz, 1 H), 6.14 (dd, *J*=13.1, 5.5 Hz, 1 H), 7.41 (m, *J*=9.0 Hz, 2 H), 7.56 (t, *J*=7.8 Hz, 2 H), 7.65 (t, *J*=7.5 Hz, 1 H), 8.05 (d, *J*=7.3 Hz, 2 H), 8.09 (s, 1 H), 8.15 (m, *J*=9.0 Hz, 2 H), 8.54 (s, 1 H), 8.99 (br s, 1 H), 9.40 (br dd, *J*=6.5, 2.5 Hz, 1 H); $^{19}F$ NMR (376 MHz, CHLOROFORM-d) δ ppm -207.51 (br s, 1 F); ESI-MS: *m/z* 688.1 $[M+H]^+$.

Step 5: preparation of compound **1f**

A mixture of compound **1e** (546 mg (purity 92%), 0.73 mmol), 5'-O-(4,4'-Dimethoxytrityl)-N2-isobutyryl-3'-O-methyl-D-guanosine ([103285-33-2], 753 mg (purity 97%), 1.09 mmol) and 4Å molecular sieves (500 mg) in DCM (8 mL) was stirred at room temperature for 1 h under a nitrogen atmosphere. $Et_3N$ (503 μL, 3.64 mmol) was added and stirring was continued overnight The reaction mixture was concentrated under reduced pressure, the obtained residue was purified by flash column chromatography over silica gel (gradient elution: 0 - 90% EtOAc in petroleum ether) to give compound **1f** which was used as such in the next step. $^1H$ NMR (400 MHz, CHLOROFORM-*d*) δ ppm 0.14 (s, 3 H), 0.15 (s, 3 H), 0.75 (d, *J*=7.0 Hz, 3 H), 0.89 (d, *J*=6.8 Hz, 3 H), 0.93 (s, 9 H), 1.94 (spt, *J*=6.8 Hz, 1 H), 3.15 (dd, *J*=10.9, 3.4 Hz, 1 H), 3.45 (s, 3 H), 3.48 - 3.63 (m, 3 H), 3.75 (s, 3 H), 3.76 (s, 3 H), 4.12 - 4.21 (m, 1 H), 4.28 (t, *J*=5.3 Hz, 1 H), 4.36 (br s, 1 H), 4.63 - 4.72 (m, 1 H), 5.49 (dt, *J*=52.0, 5.1 Hz, 1 H), 5.75 (t, *J*=4.8 Hz, 1 H), 6.07 (d, *J*=4.5 Hz, 1 H), 6.12 (dd, *J*=13.4, 5.1 Hz, 1 H), 6.79 (m, *J*=8.4, 8.4 Hz, 4 H), 7.15 - 7.22 (m, 1 H), 7.25 (t, *J*=7.5 Hz, 2 H), 7.34 (m, *J*=8.3 Hz, 4 H), 7.43 - 7.55 (m, 4 H), 7.61 (t, *J*=7.3 Hz, 1 H), 7.72 (s, 1 H), 8.08 (d, *J*=8.3 Hz, 2 H), 8.09 (s, 1 H), 8.83 (s, 1 H), 9.14 (m, *J*=8.3 Hz, 2 H), 9.54 (br s, 1 H), 12.06 (br s, 1 H); $^{19}F$ NMR (376 MHz, CHLOROFORM-d) δ ppm -207.07 (br s, 1 F); ESI-MS: *m/z* 1240.6 $[M+Na]^+$.

Step 6: preparation of compound **1g**

To a solution of crude compound **1f** in MeCN (20 mL) was added acetic acid 80% (20 mL, 279.5 mmol) and triethylsilane (2 mL, 12.5 mmol), the reaction mixture was stirred at room temperature overnight EtOAc and aqueous $Na_2CO_3$ were added, the organic layer was separated, dried over anhydrous $Na_2SO_4$, filtered and the filtrate concentrated under reduced pressure. The resulting residue was dissolved in pyridine (10 mL) to which triethylamine (1.0 g, 10.0 mmol) and $Et_3N.3HF$ (807 mg, 5.0 mmol) were added. The reaction mixture was stirred at room temperature for 12 h. Subsequent concentration under reduced pressure resulted in a crude product which was purified by preparative reversed phase HPLC (Stationary phase: Phenomenex Gemini C18, 10 μm, 250 x 50 mm; Mobile phase: 10 mM aqueous ammonia bicarbonate (A) - MeCN (B); gradient elution 22 - 52% B in A over 11.2 min; flow rate: 22 mL/min) to give compound **1g** (yield: 27% starting from compound If). $^1H$ NMR (400 MHz, DMSO-$d_6$) δ ppm 1.11 (d, *J*=6.5 Hz, 6 H), 2.75 (spt, *J*=6.8 Hz, 1 H), 3.11 - 3.24 (m, 2 H), 3.39 (s, 3 H), 3.51 - 3.59 (m, 1 H), 3.59 - 3.69 (m, 1 H), 3.95 (m, *J*=3.7 Hz, 1 H), 4.09 (d, *J*=33 Hz, 1 H), 4.17 (dd, *J*=4.9, 3.3 Hz, 1 H), 4.57 (ddd, *J*=19.5, 7.3, 4.5 Hz, 1 H), 5.22 (br s, 1 H), 5.37 (t, *J*=5.5 Hz, 1 H), 5.57 (ddd, *J*=52.5, 4.5, 2.4 Hz, 1 H), 6.07 (d, *J*=6.5 Hz, 1 H), 6.33 (dd, *J*=19.3, 2.2 Hz, 1 H), 7.50-7.60 (m, 2 H), 7.61 - 7.70 (m, 1 H), 7.99 - 8.10 (m, 2 H), 8.22 (s, 1 H), 8.62 (s, 1 H), 8.73 (s, 1 H) (NH & OH where exchanged with $D_2O$); $^{19}F$ NMR (376 MHz, METHANOL-d4) δ ppm -205.32 (br s, 1 F); ESI-MS: *m/z* 802.2 $[M+H]^+$

Step 7: preparation of compound **1h**

A solution of compound **1g** (100 mg, 0.12 mmol) and 1H-tetrazole (2.2 mL of 0.45 M in MeCN, 0.93 mmol) in 1:1 MeCN / THF (14 mL) was treated with 4Å molecular sieves for 30 min before the addition of 2-cyanoethyl-*N*,*N*,*N'*,*N'*-tetra(isopropyl)phosphorodiamidite (70 mg, 0.23 mmol) in MeCN (6 mL). The resulting reaction mixture was stirred for 2 h after which an additional amount of tetrazole (0.55 mL of 0.45 M in MeCN, 0.25 mmol) was added followed by an additional 30 min of stirring. $I_2$ (0.5 M in a 8:1:1 mixture of THF / pyridine / WATER, 695 μL, 0.357 mmol) was added and stirring was continued overnight. The reaction mixture was quenched by the addition of saturated aqueous $Na_2S_2O_3$ and filtered, the filtrate was concentrated under reduced pressure. The residue was purified by preparative reverse phase HPLC (Stationary phase: Phenomenex Gemini C18, 10 μm, 250 x 50 mm; Mobile phase: 10 mM aqueous ammonia bicarbonate (A) - MeCN (B); gradient elution 20 - 50% B in A over 11.2 min; flow rate: 22 mL/min) to afford compound **1h** which was used as such in the subsequent deprotection step. ESI-MS: *m*/*z* 917.4 $[M+H]^+$ .

Step 8: preparation of **compound 1, sodium salt**

A solution of compound **1h** in a mixture of aqueous ammonia (25%, 9 mL) and EtOH (3 mL) was stirred at 50 °C overnight. The crude product obtained after concentration under reduced pressure was purified by preparative reverse phase HPLC (Stationary phase: Syneri Polar-RP, 5 μm, 100 x 30 mm; Mobile phase: 10 mM aqueous ammonia bicarbonate (A) - MeCN (B); gradient elution 0 - 25% B in A over 12 min; flow rate: 25 mL/min) to give compound 1 as the ammonium salt. Compound 1 was converted into the sodium salt by elution of an aqueous solution over a column packed with a cationic sodium ion-exchange resin affording 12.9 mg of **compound 1, sodium salt** as a white solid after lyophilization (yield: 13% starting from compound **1g**). $^1$H NMR (400 MHz, DMSO-$d_6$, spectrum recorded at 80 °C) δ ppm 3.31 (dd, *J*=13.2, 2.2 Hz, 1 H), 3.50 (s, 3 H), 3.60 (dd, *J*=13.5, 3.6 Hz, 1 H), 3.91 - 3.98 (m, 1 H), 4.20 (d, *J*=4.2 Hz, 1 H), 4.23 - 4.28 (m, 1 H), 4.28 - 4.31 (m, 1 H), 4.31 - 4.37 (m, 1 H), 5.22 - 5.34 (m, 1 H), 5.43 (dd, *J*=51.7, 4.4 Hz, 1 H), 5.99 (d, *J*=8.3 Hz, 1 H), 6.19 (br s, 1 H), 6.33 (d, *J*=19.5 Hz, 1 H), 6.58 (br s, 2 H), 7.08 (s, 2 H), 7.69 (s, 1 H), 7.87 (s, 1 H), 8.34 (br s, 1 H), 9.70 (br s, 1 H); $^{31}$P NMR (162 MHz, DMSO-$d_6$) δ ppm 56.05 (s, 1 P), 94.05 (br s, 1 P); ESI-MS: *m*/*z* 690.2 $[M+H]^+$.

Example 2

## Compound (*R) 2A

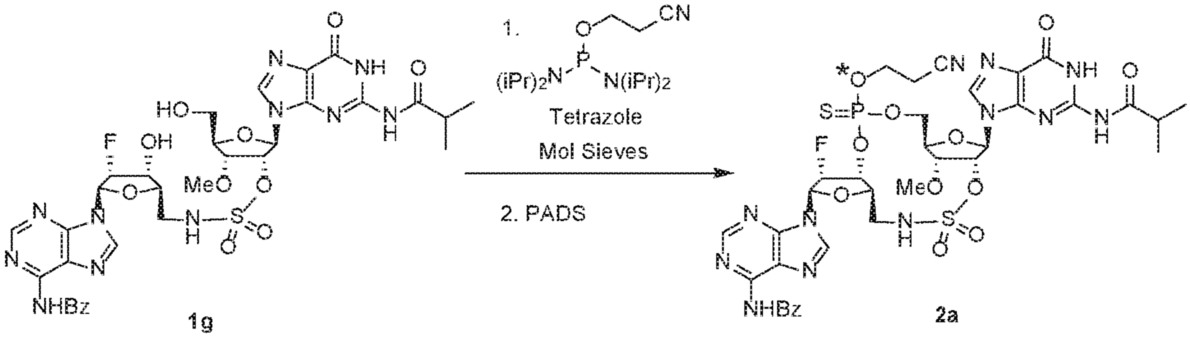


1g

2a

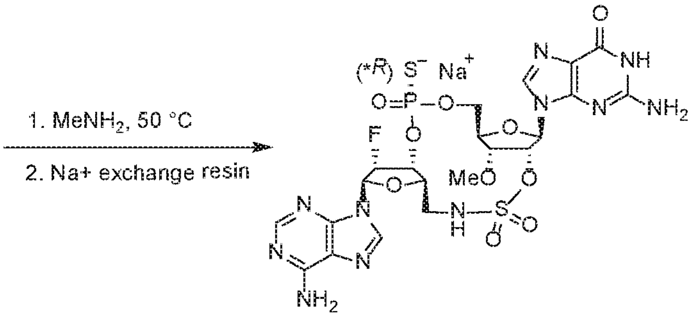


## Compound (*R) 2A, sodium salt

Step 1: preparation of compound **2a**

A solution of compound **1g** (230 mg, 0.28 mmol) and 1H-tetrazole (1.67 mL of a 3 - 4% in MeCN) in 1:1 MeCN / THF (12 mL) was treated with 4Å molecular sieves for 2 hours before the addition of 2-cyanoethyl-*N,N,N',N'*-tetra(isopropyl)phosphorodiamidite (78 mg, 0.26 mmol). The resulting reaction mixture was stirred for 30 min after which an additional amount of 2-cyanoethyl-*N,N,N',N'*-tetra(isopropyl)phosphorodiamidite (8.6 mg, 0.03 mmol) was added followed by stirring for an additional 15 min. Pyridine (15 mL) and phenylacetyl disulfide (PADS, 217 mg, 0.72 mmol) were added and stirring was continued for 40 min. Upon removal of the molecular sieves by filtration, EtOAc was added to the reaction mixture followed by extensive washes with brine and saturated aqueous $NaHCO_3$. The organic phase was dried over anhydrous $MgSO_4$, filtered and the filtrate concentrated under reduced pressure. The crude product was used as such in the subsequent deprotection step. ESI-MS: *m/z* 933.2 $[M+H]^+$..

Step 2: preparation of **compound (*R) 2A, sodium salt**

Crude compound **2a** was stirred in a 33% methylamine solution in ethanol (20 mL) at 50 °C for 4 h. The reaction mixture was concentrated under reduced pressure. The resulting crude product was dissolved in water, washed with EtOAc, lyophilized and purified by preparative reverse phase HPLC (Stationary phase: XBridge C18 OBD, 5 μm, 250 x 30 mm; Mobile phase: aqueous 0.25% ammonia bicarbonate (A) - MeCN (B); gradient elution: 0 - 15% B in A over 29 min, flow rate: 30 mL/min) to give **compound (*R) 2A** as a single diastereomer. Conversion into the sodium salt was performed as described in Example 1, Step 8 (23 mg, yield: 10% starting from compound 1g). $^1H$ NMR (400 MHz, DMSO-$d_6$, spectrum recorded at 80 °C) δ ppm 3.31 - 3.43 (m, 1 H), 3.49 (s, 3 H), 3.62-3.75 (m, 1 H), 3.92 - 4.00 (m, 1 H), 4.00 - 4.09 (m, 1 H), 4.20 (br s, 1 H), 4.31 (br s, 2 H), 5.33 - 5.50 (m, 2 H), 5.64 (br d, *J*=52.0 Hz, 1 H), 5.68 (dd, *J*=8.7, 4.1 Hz, 1 H), 6.03 (d, *J*=8.6 Hz, 1 H), 6.26 (s, 2 H), 6.33 (d, *J*=18.4 Hz, 1 H), 7.07 (s, 2 H), 7.88 (br s, 1 H), 8.29 (br s, 2 H), 8.66 (br s, 1 H), 10.30 (br s, 1 H); $^{31}P$ NMR (162 MHz, DMSO-$d_6$) δ ppm 52.18 (s, 1 P); ESI-MS: *m/z* 706.0 $[M+H]^+$.

Example 3

## Compound (**R*) 4A and Compound (**S*) 4B

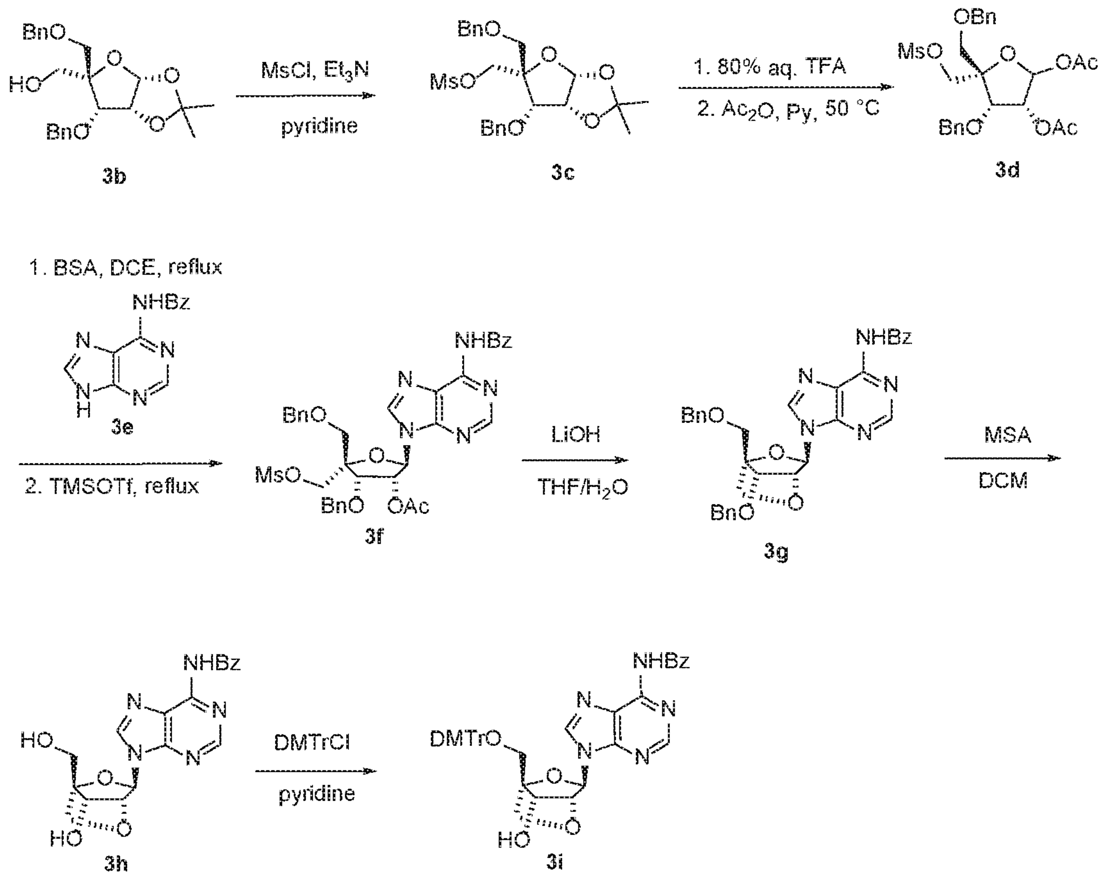


3b, 3c, 3d, 3e, 3f, 3g, 3h, 3i

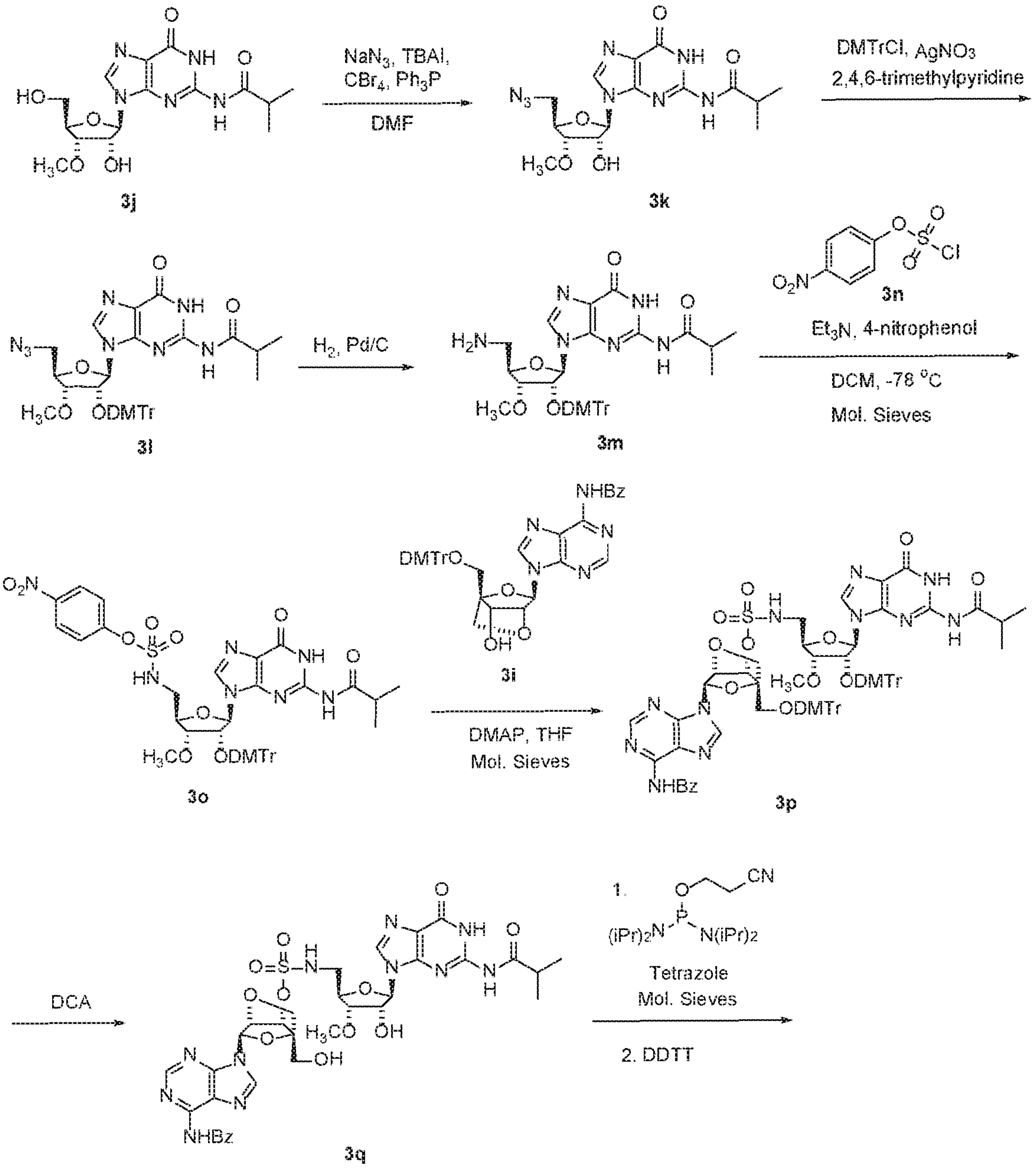


3j 3k 3l 3m 3n 3o 3i 3p 3q

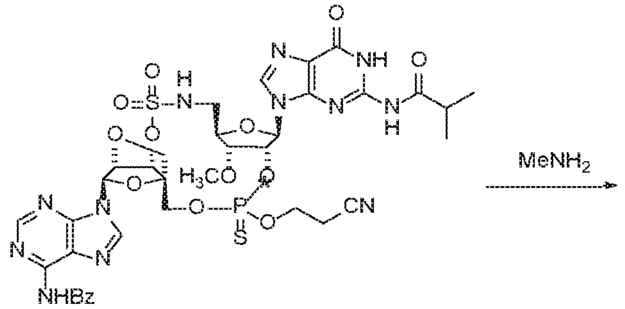


3r

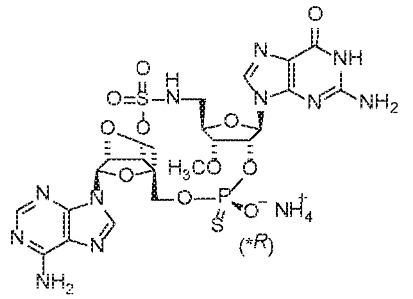

+

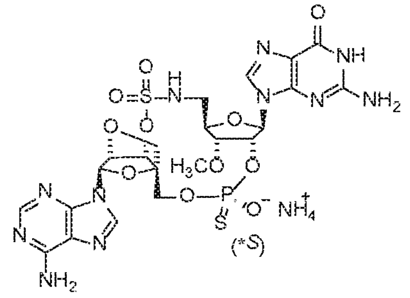

Compound (*R) 4A

Compound (*S) 4B

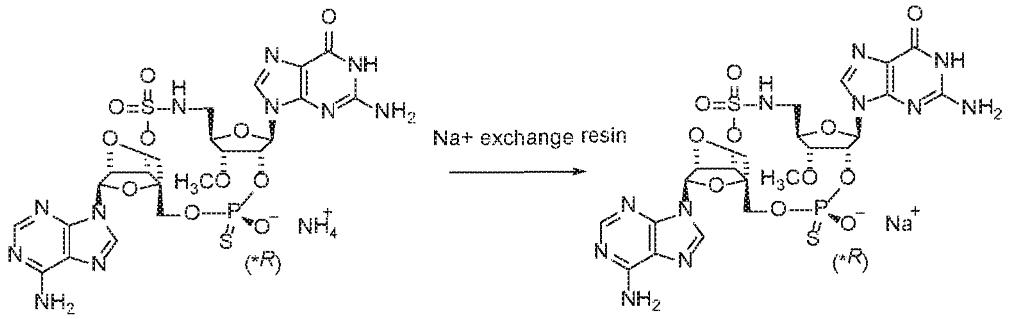


Compound (*R) 4A

Compound (*R) 4A, sodium salt

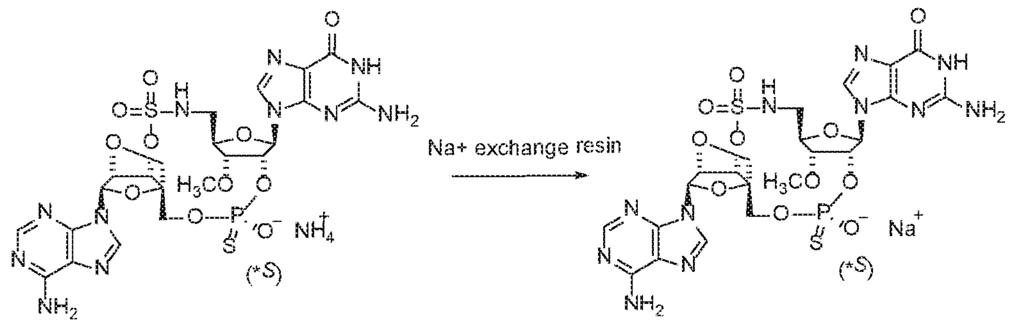


Compound (*S) 4B

Compound (*S) 4B, sodium salt

Step 1: Preparation of compound **3c**

To a solution of compound **3b** (2.0 g, 4.99 mmol, CAS# 153186-10-8) in pyridine (8 mL) was added MsCl (1.14 g, 9.99 mmol) slowly at 0 °C under $N_2$; after stirring for 1 h at room temperature, the reaction mixture was diluted with EtOAc (20 mL) and washed with water (3 x 20 mL). The aqueous phase was separated and extracted with EtOAc (3 x

15 mL). The combined organic layers were successively dried over anhydrous $Na_2SO_4$, filtered and concentrated under reduced pressure to afford compound 3c as a yellow oil (3.1 g).

$^1H$ NMR (400 MHz, $CDCl_3$) δ ppm 7.38 - 7.31 (m, 7H), 7.21 - 7.14 (m, 3H), 5.79 (d, J = 3.8 Hz, 1H), 4.87 (d, J = 11.8 Hz, 1H), 4.74 (d, J = 12.0 Hz, 1H),4.67 - 4.62 (m, 1H), 4.57 - 4.38 (m, 4H), 4.30 (d, J = 5.3 Hz, 1H), 3.63 - 3.58 (m, 1H), 3.53 - 3.48 (m, 1H), 3.63 - 3.47 (m, 1H), 3.07 (s, 3H), 2.36 (s, 3H), 1.71- 1.67 (m, 3H), 1.35 (s, 3H); ESI-MS: m/z = 501.2 $[M+Na]^+$.

Step 2: preparation of compound **3d**

A solution of **3c** (1.0 g, 2.1 mmol) in 80% aqueous trifluoroacetic acid (10 mL) was stirred at room temperature for 1 h. After removal of the solvent under reduced pressure, the residue was dissolved in DCM (20 mL) and washed with saturated aqueous $NaHCO_3$ (2 x 20 mL). The organic layer was successively dried over anhydrous $Na_2SO_4$, filtered and concentrated under reduced pressure to give a colorless oil (876 mg). The oil was co-evaporated with anhydrous pyridine (2 x 15 mL), dissolved in anhydrous pyridine (15 mL), and treated with $Ac_2O$ (815.8 mg, 7.99 mmol). After stirring overnight at 50 °C the reaction mixture was quenched with aqueous saturated $NaHCO_3$ (25 mL) and partitioned with EtOAc (2 x 20 mL). The organic layers were combined, successively washed with brine (15 mL), dried over anhydrous $Na_2SO_4$, filtered and concentrated under reduced pressure to yield a residue. The residue was purified by flash column chromatography on silica gel (0-30% EtOAc in petroleum ether) to afford **3d** as a colorless syrup (786 mg).

$^1H$ NMR (400 MHz, $CDCl_3$) δ ppm 7.42 - 7.18 (m, 12H), 6.38 (d, J=4.5 Hz, 1H), 6.15 (s, 1H), 5.36 (d, J=5.0 Hz, 1H), 5.30 (s, 1H), 5.17 (dd, J=4.8, 6.3 Hz, 1H), 2.99 (s, 3H), 2.96 (s, 1H), 2.19 - 2.10 (m, 4H), 2.06 (d, J=7.8 Hz, 1H), 1.90 (s, 3H). ESI-MS: m/z = 545 $[M+Na]^+$.

Step 3: preparation of compound **3f**

To a suspension of compound **3d** (200 mg, 0.38 mmol) and 6-N-benzoyladenine (3e, 109.8 mg, 0.46 mmol) in anhydrous 1,2-dichloroethane (5 mL) was added bis(trimethylsilyl)acetamide (BSA, 202.4 mg, 0.99 mmol). The mixture was refluxed for 1 hr and cooled to RT. TMSOTf (170 mg, 0.76 mmol) was added and the solution was heated at 110 °C for 16 h. The reaction mixture was diluted with DCM (15 mL), then poured into ice-cold saturated aqueous $NaHCO_3$ (20 mL), stirred for 0.5 h, and filtered. After separation of the two layers, the organic layer was successively washed with saturated aqueous $NaHCO_3$ (3 x 15 mL), dried over anhydrous $Na_2SO_4$, filtered and concentrated under reduced pressure to give a residue. Purification of the residue by flash column chromatography on silica gel (1-1.5% v/v MeOH/CH2C12) gave compound 3f (208 mg) as a light yellow solid. $^1H$ NMR (400 MHz, $CDCl_3$) δ ppm 8.80 (s, 1H), 8.59 (s, 1H), 8.04 (s, 1H), 7.87 (br d, J = 7.3 Hz, 2H), 7.54 - 7.44 (m, 1H), 7.44 - 7.34 (m, 2H), 7.29 - 7.03 (m, 10H), 6.18 (br d, J = 4.4 Hz, 1H), 5.96 - 5.84 (m, 1H), 4.70 (br d, J = 5.6 Hz, 1H), 4.56 - 4.29 (m, 1H), 4.55 - 4.22 (m, 4H), 4.21 (s,1H), 3.63 - 3.53 (m, 1H), 3.46 (br d, J=10.0 Hz, 1H), 2.84 - 2.75 (m, 1H), 2.86 - 2.75 (m, 1H), 2.87 - 2.71 (m, 1H), 1.99-1.92 (m, 1H), 1.91 (br s, 1H); ESI-MS: m/z = 702 $[M+H]^+$

Step 4: preparation of compound **3g**

To a solution of compound **3f** (5.86 g, 8.35 mmol) in a mixture of THF (50 mL) and water (35 mL) was added $LiOH.H_2O$ (1.75 g, 41.8 mmol) at 0 °C. After stirring the mixture for 5 h at room temperature, the reaction mixture was diluted with EtOAc (30 mL). The organic phase was washed with brine (30 mLx2) and the aqueous layer was extracted by EtOAc (50 mLx3). The combined organic layers were successively washed with saturated aqueous $NaHCO_3$ (3 x 15 mL), dried over anhydrous $Na_2SO_4$, filtered and concentrated under reduced pressure to give a residue. The residue was purified by flash column chromatography on silica gel (EtOAc : PE = 0-70%) to give compound **3g** (4.25 g) as a faint yellow solid. $^1H$ NMR (400 MHz, $CDCl_3$) δ ppm 9.00 (br s, 2H), 8.74 (s, 2H), 8.23 (s, 2H), 8.02 (br d, J=7.3 Hz, 3H), 7.60 (br d, J=7.3 Hz, 2H), 7.52 (br t, J=7.5 Hz, 3H), 7.38 - 7.16 (m, 15H), 6.09 (s, 2H), 4.79 (s, 2H), 4.66 - 4.49 (m, 6H), 4.24 (s, 2H), 4.18 - 4.05 (m, 3H), 3.99 (br d, J=7.8 Hz, 2H), 3.90 - 3.70 (m, 3H), 2.03 (s, 2H), 1.75 (br s, 3H), 1.24 (br t, J=7.1 Hz, 2H); ESI-MS: m/z = 564.1 $[M+H]^+$.

Step 5: preparation of compound **3h**

To a stirred solution of compound **3g** (3.3 g, 5.85 mmol) in DCM (50 mL) was added at 0 °C methanesulfonic acid (28.9 g, 0.3 mol). After stirring at 0 °C for 2.5 h, the reaction was combined with a previous batch and a suspension of $NaHCO_3$ (75 g, 0.90 mol) in DCM (180 mL) was added using a pressure equalizing dropping funnel. After stirring the reaction mixture for 1.5 h, MeOH (10 mL) was added and the mixture stirred for another 0.5 hr (pH -7-8). The reaction mixture was filtered and the filtrate was concentrated under reduced pressure to give a light yellow solid, purified by flash column chromatography on silica gel (0-10% MeOH in DCM, 25 mL/min) to give compound **3h** as an off-white solid

(3.75 g). $^1$H NMR (400 MHz,$CD_3OD$) δ ppm 8.73 (s, 1H), 8.56 (s, 1H), 8.13 - 8.06 (m, 2H), 7.71 - 7.62 (m, 1H), 7.61 - 7.53 (m, 2H), 6.14 (s, 1H), 4.63 (s, 1H), 4.38 (s, 1H), 4.09 (d, J = 7.8 Hz, 1H), 3.97 (s, 2H), 3.92 (d, J = 7.8 Hz, 1H).

Step 6: preparation of compound **3i**

To a mixture of compound **3h** (4.3 g, 11.2 mmol) in pyridine (50 mL) was added a solution of DMTrCl (4.56 g, 13.4 mmol) in pyridine (20 mL) dropwise at 0 °C. After stirring at RT for 2 h, the reaction mixture was diluted with ethyl acetate (100 mL) then washed successively with saturated aqueous $NaHCO_3$ (80 mLx3) and brine (80 mL x 2). The organic layers were combined and successively dried over anhydrous $Na_2SO_4$, filtered and concentrated under reduced pressure to give a residue. The residue was purified by flash column chromatography on silica gel (0-100% EtOAc in Petroleum ether) to give compound **3i** as a white solid (7.8 g). $^1$H NMR (400 MHz, $CD_3OD$) δ ppm 8.74 (s, 1H), 8.50 (s, 1H), 8.09 (d, J=7.3 Hz, 2H), 7.70 - 7.62 (m, 1H), 7.60 - 7.53 (m, 2H), 7.48 (d, J=7.5 Hz,2H), 7.36 (dd, J=3.0, 9.0 Hz, 4H), 7.33 - 7.27 (m, 1H), 7.26 - 7.18 (m, 1H), 6.87 (d, J=8.8 Hz, 4H), 6.17 (s, 1H), 4.65 (s, 1H), 4.49 (s, 1H), 4.10 (q, J=7.3 Hz, 1H), 4.05 - 3.98 (m, 2H), 3.77 (s, 6H), 3.65 - 3.58 (m, 1H), 3.54 - 3.48 (m, 1H); ESI-MS: m/z = 686 $[M+H]^+$.

Step 7: preparation of compound **3k**

To a stirred suspension of compound **3j** (5.0 g, 13.61 mmol, CAS# 160107-07-3), triphenylphosphine (4.28 g, 16.33 mmol), TBAI (502 mg, 1.36 mmol) and $NaN_3$ (3.3 g, 50.76 mmol) in DMF (60 mL) was added $CBr_4$ (5.41 g, 16.33 mmol) in one portion. After stirring at 20 °C for 12 h, the reaction mixture was partitioned with 80 mL of saturated aqueous $NaHCO_3$ and DCM (100 mL x 3). The organic layers were combined and successively dried over anhydrous $Na_2SO_4$, filtered and concentrated under reduced pressure to give a residue. The residue was purified by flash column chromatography on silica gel (0-100% EtOAc in petroleum ether to flush $Ph_3PO$, then switched to 0-10% MeOH in DCM) to give **3k** as a white solid (4.26 g). $^1$H NMR (400 MHz, $CD_3OD$) δ ppm 8.16 (s, 1H), 5.96 (d, J=4.9 Hz, 1H), 4.82 (t, J=5.3 Hz, 1H), 4.27 - 4.21 (m, 1H), 3.97 (t, J=4.9 Hz, 1H), 3.66 (d, J=4.4 Hz, 2H), 3.52 (s, 3H), 2.78 - 2.69 (m, 1H), 1.24 (d, J=6.8 Hz, 6H); ESI-MS: m/z = 393.1 $[M+H]^+$.

Step 8: preparation of compound **3l**

To a solution of **3k** (4.07 g, 10.37 mmol), 2,4,6-trimethylpyridine (1.63 g, 13.48 mmol) and $AgNO_3$ (2.29 g, 13.48 mmol) in DCM (40 mL) was added DMTrCl (4.57 g, 13.48 mmol) at 0°C. After stirring at 25°C for 3 h, the red suspension was diluted with DCM (50 mL) and filtered through a pad of diatomaceous earth. The filtrate was partitioned between DCM/water (50/30 mL) and washed with brine (30 mL). The organic layers were combined and successively dried over anhydrous $Na_2SO_4$, filtered and concentrated under reduced pressure to give a residue. The residue was purified by flash column chromatography on silica gel (0-5% MeOH in DCM) to give **3l** as a white solid (7.0 g). $^1$H NMR (400 MHz, $CD_3OD$) $\delta$ 7.94 (s, 1H), 7.35-7.28 (m, 1H), 7.22(d, J=8.8Hz, 1H), 7.18-7.12(m, 2H),7.09 (d, J=8.8Hz, 1H), 6.72 (d, J=8.8Hz, 1H), 6.61(d, J=8.8Hz, 1H), 6.03(d, J=7.7Hz, 1H), 4.94 (br dd, J=5.2,7.2 Hz, 1H), 4.87 (s, 7H), 4.16 (t, J=5.7Hz,1H), 4.07 (q, J=7.1Hz, 1H), 3.71 (d, J=13.7Hz, 4H), 3.61 (dd, J=6.6, 12.8Hz, 1H), 3.29(s,3H), 3.21 (s, 2H), 2.75 (quin, J=6.8Hz, 1H), 2.68 (d, J=4.6Hz, 1H), 1.99 (s, 2H), 1.28-1.17 (m, 6H); ESI-MS: m/z = 695.3 $[M+H]^+$.

Step 9: preparation of compound **3m**

A suspension of **3l** (1.2 g, 1.72 mmol) and 10% wet Pd/C (1.0 g, 0.49 mmol) in ethyl acetate (100 mL) was hydrogenated at 20 °C under 15 psi for 4 h. The reaction mixture was filtered through a pad of diatomaceous earth and the filtrate concentrated under reduced pressure to give a residue purified by flash column chromatography on silica gel (0-10% MeOH in DCM) to give **3m** as a white solid (682 mg). $^1$H NMR (400 MHz, $CD_3OD$) $\delta$ 7.89 (s, 1H), 7.33 (dd, J=1.5, 7.8 Hz, 1H), 7.30 - 7.29 (m, 1H), 7.36 - 7.29 (m, 1H), 7.21 (d, J=8.8 Hz, 2H), 7.18 -7.10 (m, 3H), 7.08 (d, J=8.8 Hz, 2H), 6.71 (d, J=8.8 Hz, 2H), 6.59 (d, J=8.8 Hz, 2H), 5.98 (d, J=7.5 Hz, 1H), 5.03 (dd, J=4.8, 7.5 Hz, 1H), 4.21 (dd, J=3.4, 9.9 Hz, 1H), 3.69 (d, J=16.3 Hz, 6H), 3.26 (s, 3H), 3.16 (dd, J=10.0, 13.1 Hz, 1H), 2.88 - 2.75 (m, 3H), 1.31 - 1.21 (m, 8H); ESI-MS: m/z = 669.3 $[M+H]^+$.

Step 10: preparation of compound **3o**

A solution of 4-nitrophenyl chlorosulfate **3n** (2.45g, 10.31 mmol, J. Chem. Soc., Perkin Trans. 1, 2002, 485-495) in dry DCM (5 mL) was added rapidly to a mixture of **3m** (2.3 g, 3.44 mmol), 4-nitrophenol (1.43 g, 10.31 mmol), $Et_3N$ (2.09 g, 20.63 mmol) and activated 4Å molecular sieves (≈ 4 g) in dry DCM (50 mL) under $N_2$ at -78°C. After warming up to room temperature (12 °C) and stirring for 2 h, the reaction mixture was diluted with DCM (20 mL) and filtered through a pad of diatomaceous earth. The filtrate was combined with previous two other batches and partitioned between

DCM / saturated aqueous $NaHCO_3$ (100, 3 x 70 mL). The organic layers were combined and successively dried over anhydrous $Na_2SO_4$, filtered and concentrated under reduced pressure to give a residue. The residue was purified by flash column chromatography on silica gel (0-100% ethyl acetate in petroleum ether) to give **3o** as a yellow solid (4.8 g). $^1H$ NMR (400 MHz, $CDCl_3$) $\delta$ 12.18 (br s, 1H), 9.18 (s, 1H), 8.32 - 8.26 (m, 3H), 7.74 (s, 1H), 7.37 - 7.28 (m, 4H), 7.25 - 7.11 (m, 6H), 7.08 (d, J=8.8 Hz, 1H), 6.73 (d, J=9.0 Hz, 2H), 6.63 (d, J=8.8 Hz, 2H), 5.89 (d, J=8.8 Hz, 1H), 4.99 (dd, J=5.0, 8.5 Hz, 1H), 4.29 (s, 1H), 3.80 - 3.74 (m, 4H), 3.72 (s,1H), 3.50 - 3.44 (m, 1H), 3.38 - 3.29 (m, 1H), 3.20 (s, 3H), 2.77 (d, J=5.0 Hz, 1H), 2.55 - 2.43 (m, 1H), 1.31 - 1.27 (m, 4H), 1.15 (d, J=6.8 Hz, 3H).

ESI-MS: m/z = 870 $[M+H]^+$.

Step 11: preparation of compound **3p**

The mixture of **3o** and **3i** was co-evaporated with THF (30 mL x 3) before use. A mixture of **3o** (4.12 g, 4.74 mmol), **3i** (2.5 g, 3.64 mmol) and activated 4Å molecular sieves (~2 g) in dry THF (50 mL) was stirred under $N_2$ at RT for 1 h. DMAP (2.22 g, 18.22 mmol) was added in one portion and the reaction mixture stirred at 40 °C for 12h. The mixture was diluted with DCM (40 mL), filtered through a pad of diatomaceous earth and concentrated under reduced pressure to give a residue. The residue was purified by flash column chromatography on silica gel (0-2% MeOH in DCM) to give **3p** as a light yellow solid (4.6 g). $^1H$ NMR (400 MHz, $CDCl_3$) $\delta$ 12.31 (s, 1H), 9.25 (s, 1H), 9.11 (br s, 1H), 8.78 (s, 1H), 8.52 - 8.29 (m, 2H), 8.16 - 8.01 (m, 5H), 7.77 (s, 1H), 6.69(d, J=8.8 Hz, 2H), 6.60 (d, J=9.0 Hz, 2H), 6.21 (s, 1H), 5.84 (d, J=8.8 Hz, 1H), 5.36 (s, 1H), 5.06 (s, 1H), 4.86 (dd, J=4.9, 8.7 Hz, 1H), 4.06 - 3.93 (m, 2H),3.73 (d, J=2.8 Hz, 9H), 3.70 (s, 3H), 3.67 - 3.58 (m, 1H), 3.34 (d, J=11.0 Hz, 1H), 3.15 (s, 3H), 3.06 (dd, J=2.3, 12.5 Hz, 1H), 2.96 - 2.84 (m, 1H), 2.62 - 2.49(m, 2H), 1.28 - 1.24 (m, 5H), 1.18 (d, J=6.8 Hz, 3H); ESI-MS: m/z=1417 $[M+H]^+$.

Step 12: preparation of compound **3q**

To a stirred solution of **3p** (4.6 g, 3.25 mmol) in DCM (84 mL) was added 6% DCA in DCM (44 mL, 32.3 mmol, 10.0 eq.) at RT under $N_2$. After stirring at RT for 30 min, the reaction mixture was quenched with pyridine (2.8 g,11 eq.) and the resulting colorless solution was concentrated under reduced pressure to give a colorless residue purified by flash column chromatography on silica gel (0-7% MeOH in DCM) to give **3q** as a white solid (1.9 g). The above solid was further purified by reverse phase preparative HPLC (Column: Phenomenex Synergi Max-RP 250 x 50mm x 10 $\mu$m; mobile phase water (10mM $NH_4HCO_3$)-MeCN, Begin B 15, End B 45; Flow Rate: 90 mL/min Gradient Time: 18 min followed by B 100 for 3 min) to generate 2 fractions of **3q** as a white solid after lyophilization. Fraction 1: 876 mg and fraction 2: 743 mg. $^1H$ NMR (400 MHz, $CD_3OD$) $\delta$ 8.52 (s, 1H), 8.36 (s, 1H), 8.01 (d, J=7.1 Hz, 2H), 7.86 (s, 1H), 7.65 (d, J=7.3 Hz, 1H), 7.60 - 7.54 (m, 2H),6.20 (s,1H), 5.71 (d, J=4.9 Hz, 1H), 5.22 (d, J=14.4 Hz, 2H), 4.67 - 4.59 (m, 1H), 4.06 (d, J=3.7 Hz, 2H), 4.02 (br d, J=4.6 Hz, 3H), 4.10 - 3.99 (m,1H), 3.99-3.93(m, 1H), 3.51 - 3.44 (m, 4H), 3.40 (d, J=4.6 Hz, 1H), 2.73 - 2.65 (m, 1H), 1.21 (dd, J=5.4, 6.6 Hz, 6H); ESI-MS: m/z=812.2 $[M+H]^+$.

Step 13: preparation of compound **3r**

THF was freshly distilled over Na/benzophenone and $CH_3CN$ was freshly distilled over $CaH_2$.

Vacuum-dried diol **3q** (300 mg, 0.37 mmol) was co-evaporated with a mixture of $CH_3CN$ / THF (10/6 mLx3) and dissolved in a mixture of $CH_3CN$ / THF (10/6 mL). It was added 800 mg of activated 4Å Molecular Sieves and a solution of 1H-tetrazole in $CH_3CN$ (6.56 mL, 0.45M, prepared by dissolving 945 mg of tetrazole in 30 mL of dry $CH_3CN$, followed by addition of 800 mg of 4Å molecular sieves and then stirred for 1h under Argon before use) and the mixture was bubbled with Argon for 15 min. After stirring the white suspension for 1hr at 8 °C under Argon, a solution of 2-cyanoethyl N,N,N',N'-tetraisopropylphosphorodiamidite in $CH_3CN$ (5.62 mL, 0.59 mmol, 0.105M in $CH_3CN$, prepared by dissolving 759 mg of phosphorodiamidite reagent in 24 mL of $CH_3CN$, followed by addition of 800 mg of 4Å molecular sieves and then stirred for 1h under Argon before use) was added dropwise over 60 min. The resulting white suspension was stirred for 1hr at 8°C under Argon. More $CH_3CN$ (6 mL) was added and after stirring for 1hr at 30°C, additional tetrazole (1.64 mL, 0.74 mmol, 0.45M in $CH_3CN$) was added. After stirring for an additional 2 hr, a solution of DDTT (380 mg, 1.84 mmol) in pyridine (10 mL) was added rapidly. After stirring for 30 min, the mixture was filtered through a pad of diatomaceous earth; the filtrate was combined with another batch and concentrated under reduced pressure to give a residue dissolved in DCM (8 mL) and purified by flash column chromatography on silica gel (12 g, 0-6% MeOH in DCM, 25 mL/min) to afford **3r** (275 mg) as a light yellow solid used directly into the next step without further purification ESI-MS: m/z = 943.5 $[M+H]^+$.

Step 14: preparation of **compounds (*R) 4A and (*S) 4B**

A solution of compound 3r (275 mg, 0.292 mmol) in $MeNH_2$ (27-30% in EtOH, 5 mL) was stirred at 80°C for 4

hr. The reaction mixture was combined with another batch and concentrated under reduced pressure to give a residue; the residue was dissolved in a mixture of $CH_3CN/H_2O$ (1/8 mL) and washed with DCM (8 mL x 3). The aqueous layer was lyophilized to give a yellow gum (382 mg) then further dissolved in a mixture of $CH_3CN/H_2O$ (4/1). Purification by reverse phase preparative HPLC (Column: Agela Durashell C18 150 x 25 x 5 μm; mobile phase water (0.04% $NH_3$WATER+10mM $NH_4HCO_3$)- $CH_3CN$; Begin B 12, End B 25; Flow Rate: 25 mL/min Gradient Time: 12 min followed by B 100 for 3 min); desired fractions were collected and lyophilized to generate **compound (**R*) 4A, ammonium salt** (39.8 mg, first eluting isomer) as a white solid and **compound (**S*) 4B, ammonium salt** (65.9 mg, second eluting isomer) as a white solid.

Final conversion into the sodium salt was done by elution of an aqueous solution over a column packed with Dowex 50WX8 Na ion-exchange resin to give **compound (**R*) 4A, sodium salt** and **compound (**S*) 4B, sodium salt** as white fluffy solid after lyophilization.

**Compound (**R*) 4A**. $^1$H NMR (400 MHz, 60 °C, DMSO-$d_6$) δ ppm 3.34 - 3.44 (m, 1 H), 3.45 - 3.56 (m, 1 H), 3.52 (s, 3 H), 3.83 (dd, *J*=10.7, 2.0 Hz, 1 H), 3.98 (d, *J*=8.3 Hz, 1 H), 4.09 (br s, 1 H), 4.16 (br s, 1 H), 4.23 (d, *J*=8.3 Hz, 1 H), 4.67 (dd, *J*=11.1, 7.8 Hz, 1 H), 4.98 (br s, 1 H), 5.14 (br s, 1 H), 5.31 (ddd, *J*=13.4, 9.0, 4.4 Hz, 1 H), 5.78 (d, *J*=8.8 Hz, 1 H), 6.07 (s, 1 H), 6.13 (br s, 2 H), 7.15 (br s, 2 H), 7.92 (s, 1 H), 8.16 (s, 1 H), 8.17 (s, 1 H) $^{31}$P NMR (162 MHz, DMSO-$d_6$) δ 55.83 (s, 1 P); ESI-MS: m/z = 716.2 [M+H]$^+$.

**Compound (**S*) 4B.** $^1$H NMR (400 MHz, 80 °C, DMSO-$d_6$) δ ppm 3.21 - 3.39 (m, 2 H), 3.54 (s, 3 H), 3.87 (dd, J=11.5, 4.3 Hz, 1 H), 3.92 (d, J=8.1 Hz, 1 H), 3.99 (br s, 1 H), 4.14 (d, J=8.1 Hz, 1 H), 4.30 (br s, 1 H), 4.37 (dd, J=11.6, 5.0 Hz, 1 H), 4.84 (br s, 1 H), 5.06 (br s, 1 H), 5.31 (ddd, J=12.3, 9.0, 4.2 Hz, 1 H), 5.79 (d, J=9.0 Hz, 1 H), 6.03 (s, 1 H), 6.12 (br s, 2 H), 6.99 (br s, 2 H), 7.99 (s, 1 H), 8.18 (s, 1 H), 8.20 (s, 1 H); $^{31}$P NMR (162 MHz, DMSO-$d_6$) δ 52.88 (s, 1 P); ESI-MS: m/z = 716.2 [M+H]$^+$.

Example 4

# Compound 52

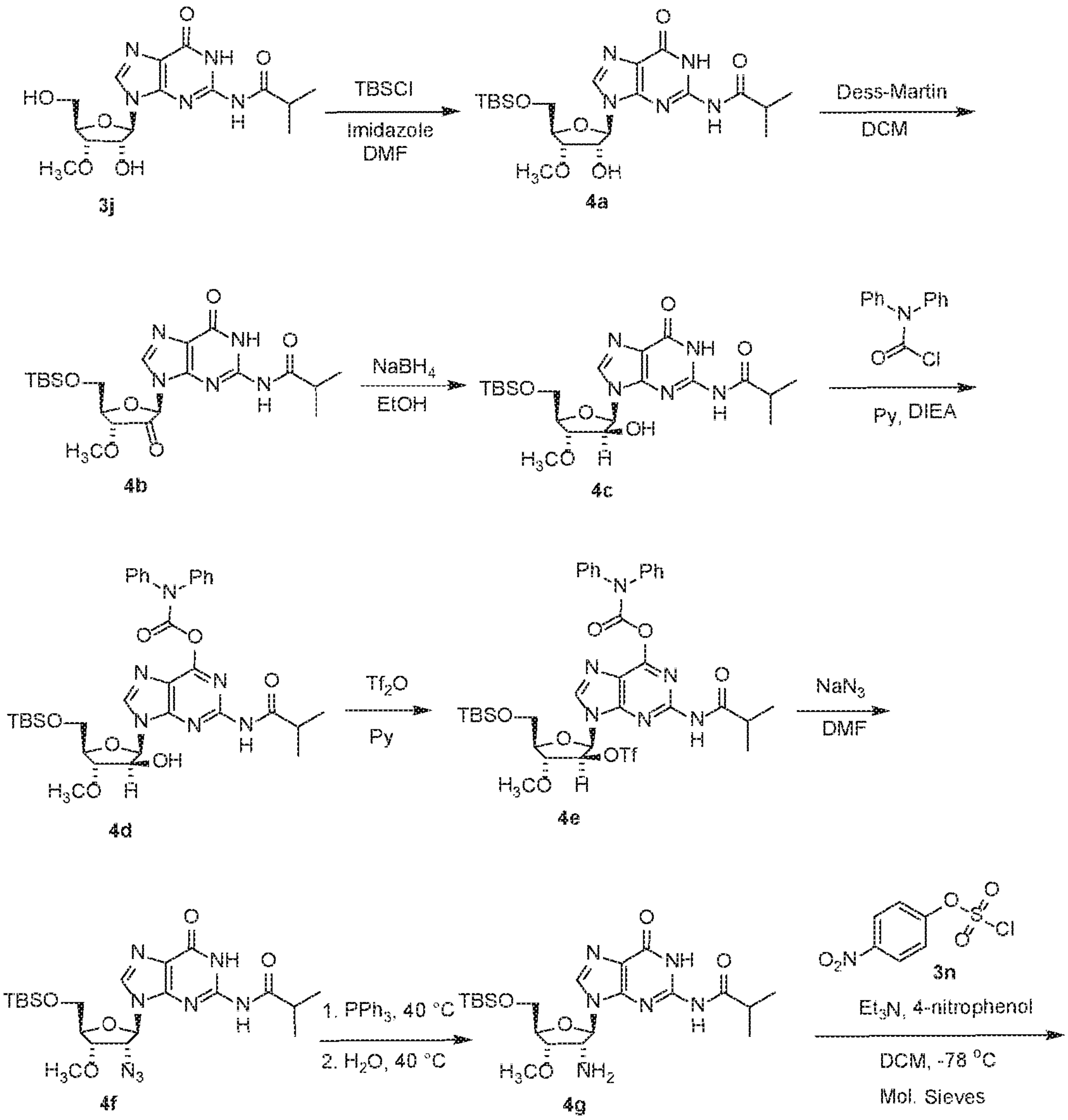
3j
TBSCl
Imidazole
DMF
4a
Dess-Martin
DCM
4b
NaBH4
EtOH
4c
Py, DIEA
4d
Tf2O
Py
4e
NaN3
DMF
4f
1. PPh3, 40 °C
2. H2O, 40 °C
4g
3n
Et3N, 4-nitrophenol
DCM, -78 °C
Mol. Sieves

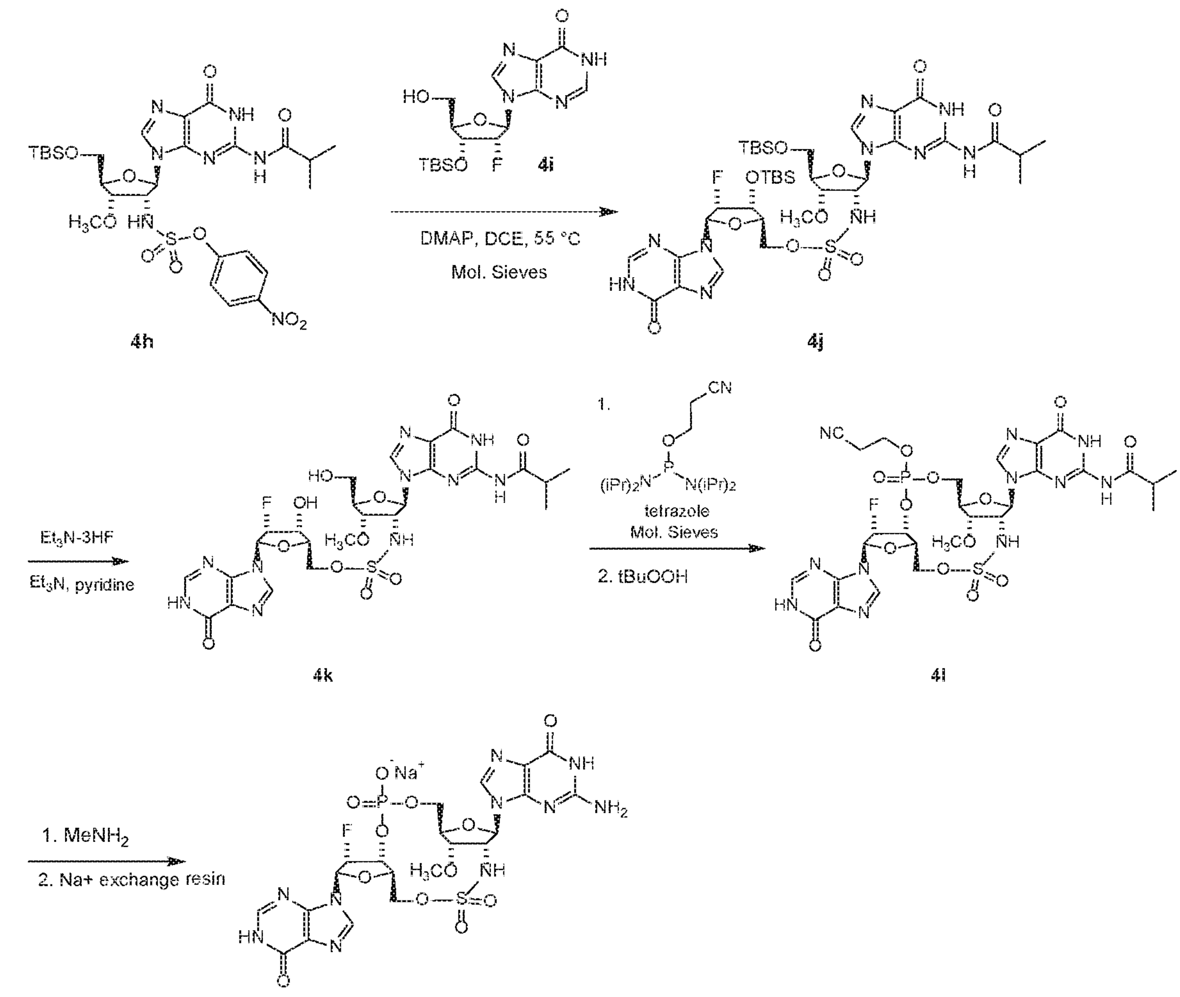


**Compound 52, sodium salt**

Step 1: Preparation of compound **4a**

To a solution of compound **3j** (9.6 g, 26.13 mmol, CAS# 160107-07-3) in DMF (80 mL) was added imidazole (3.56 g, 52.26 mmol) and TBSCl (4.73 g, 31.36 mmol) at 0 °C. After stirring the mixture at 25 °C for 2 h, the reaction was diluted with EtOAc (200 mL) and $H_2O$ (100 ml); organic layer was successively dried over anhydrous $Na_2SO_4$, filtered, and evaporated under reduced pressure. The residue was purified by flash column chromatography on silica gel (gradient elution: 0 - 5% MeOH in DCM) to give compound **4a** (9.7 g, 76%) as a white solid. $^1H$ NMR (400 MHz, DMSO-d6) $\delta$ =12.12 (br s, 1H), 9.48 (br s, 1H), 8.05 (s, 1H), 5.89 (d, J=4.8 Hz, 1H), 4.56 (t, J=4.9 Hz, 1H), 4.21 (q, J=3.4 Hz, 1H), 3.98 (t, J=4.6 Hz, 1H), 3.93 (dd, J=3.5, 11.5 Hz, 1H), 3.79 (dd, J=2.8, 11.5 Hz, 1H), 3.47 (s, 3H), 2.75 (td, J=6.9, 13.6 Hz, 1H), 1.27 (s, 3H), 1.25 (s, 3H), 0.91 (s, 9H), 0.10 (s, 6H); ESI-MS: m/z=482.3 $[M+H]^+$

Step 2: Preparation of compound **4b**

To a solution of compound **4a** (10 g, 20.76 mmol) in DCM (200 mL) was added Dess Martin periodinane (14.97 g, 35.29 mmol) at 0 °C. After stirring the mixture at 30 °C for 12 hours. The reaction mixture was diluted with DCM (300 mL); organic layer was washed with aqueous saturated $Na_2S_2O_3$ (100 mL) and aqueous saturated $NaHCO_3$ (50 mL). Organic layer was concentrated under pressure to give a residue. The residue (combined with silica gel: 15 g) was purified by flash column chromatography on silica gel (gradient elution: 0 -100% EtOAc in petroleum ether,) to give compound **4b** (9.0 g, 80%) as a white solid. ESI-MS: m/z=498.3 $[M+H7]^+$

Step 3: Preparation of compound **4c**

To a solution of compound **4b** (9 g, 18.77 mmol) in EtOH (200 mL) was added $NaBH_4$ (1.06 g, 28.15 mmol) at

0°C. After stirring the solution at 0 °C for 0.5 hours, the mixture was diluted with EtOAc (500 mL) and aqueous saturated $NH_4Cl$ (100 mL). Organic layer was concentrated under reduced pressure to give a residue (9.0 g). The residue (combined with silica gel: 15 g) was purified by flash column chromatography on silica gel (gradient elution: 0 - 2% MeOH in DCM, V/V) to give compound **4c** (4.8 g, 53%) as a white solid and compound **4a** (1.5 g, 17%) as a white solid. Compound **4d:** $^1H$ NMR (400 MHz, DMSO-d6) $\delta$ =12.06 (s, 1H), 11.70 (s, 1H), 8.00 (s, 1H), 6.05 (d, *J*=4.6 Hz, 1H), 5.85 (d, *J*=5.5 Hz, 1H), 4.31 - 4.26 (m, 1H), 3.91 - 3.77 (m, 4H), 3.39 (s, 3H), 2.80 - 2.70 (m, 1H), 1.11 (d, *J*=6.8 Hz, 6H), 0.89 (s, 9H), 0.07 (s, 6H)
ESI-MS: m/z=482.3 $[M+H]^+$

Step 4: Preparation of compound **4d**

To a solution of compound **4c** (1.8 g, 3.74 mmol) in pyridine (20 mL) was added DIEA (1.45 g, 11.21) and diphenylcarbamic chloride (1.12 g, 4.86 mmol) at 25 °C. The mixture was stirred at 25 °C for 3 h. The reaction mixture was combined with 2 other batches and worked up. The mixture was partitioned between ethyl acetate (150 mL) and $H_2O$ (100 mL). The organic layer was washed with brine (100 ml) and evaporated under reduced pressure to give a residue. The residue was purified by flash column chromatography on silica gel (0% to 100 % EA/PE, V/V) to give compound **4d** (6 g, 70 % from 4.8 g of compound **4c)** as a yellow solid. $^1H$ NMR (400 MHz, CHLOROFORM-d) $\delta$= 8.34 (s, 1H), 8.00 (s, 1H), 7.44 (br d, J=7.3 Hz, 4H), 7.37 (t, J=7.8 Hz, 4H), 7.25 (br s, 2H), 6.22 (d, J=3.0 Hz, 1H), 4.38 (br s, 1H), 4.16 - 4.12 (m, 1H), 4.00 - 3.93 (m, 2H), 3.83(dd, J=2.5, 11.0 Hz, 1H), 3.51 (s, 3H), 2.94 (br s, 1H), 1.27 (dd, J=1.0, 6.8 Hz, 6H), 0.94 (s, 9H), 0.14 (s, 6H); ESI-MS: m/z=677.4 $[M+H]^+$.

Step 5: Preparation of compound **4e**

To a solution of compound **4d** (5 g, 7.39 mmol) in pyridine (100 mL) was added $Tf_2O$ (16.67 g, 59.1 mmol,) at 0 °C. After stirring the mixture at 0 °C for 1.5 h, the reaction was partitioned between DCM (200 mL) and $H_2O$ (100 mL). The organic layer was separated and the aqueous phase extracted with DCM (100 ml). Organic layers were then combined and successively dried with anhydrous $Na_2SO_4$, filtered and evaporated under reduced pressure to give a residue. The residue was combined with another batch and purified by flash column chromatography on silica gel (0% to 30 % EtOAc /petroleum ether, V/V) to give compound **4e** (4.0 g, as a white solid. ESI-MS: m/z=809.5 $[M+H]^+$.

Step 6: Preparation of compound **4f**

To a solution of compound **4e** (4.0 g, 4.94 mmol) in DMF (40 mL) was added $NaN_3$ (3.5 g, 54.15 mmol) at 25 °C. After stirring the reaction at 25°C for 12 hours, the mixture was diluted with aqueous saturated $NaHCO_3$ (30 mL) to adjust the pH > 9 and extracted with ethyl acetate (100 mL x 3). Organic layers were combined and successively washed with brine (100 mL), dried with anhydrous $Na_2SO_4$, filtered and evaporated under reduced pressure to give a yellow solid. The residue (combined with silica gel: 10 g) was purified by flash column chromatography on silica gel (gradient elution: 0 - 100% ethyl acetate in petroleum ether) to give compound **4f** (1.8 g, 67%) as a yellow solid.

$^1H$ NMR (400 MHz, CHLOROFORM-d) $\delta$= 11.99 (br s, 1H), 8.20 (br s, 1H), 8.05 (s, 1H), 5.96 (br d, *J*=4.4 Hz, 1H), 4.25 - 4.11 (m, 3H), 3.99 (br d, *J*=11.7 Hz, 1H), 3.82 (br d, *J*=11.7 Hz, 1H), 3.53 (d, *J*=2.4 Hz, 3H), 2.70 - 2.58 (m, 1H), 1.30 (br d, *J*=6.8 Hz, 6H), 0.94 (s, 9H), 0.13 (s, 6H); ESI-MS: m/z=507.3 $[M+H]^+$.

Step 7: Preparation of compound **4g**

To a solution of compound **4f** (1.8 g, 3.55 mmol) in THF (20 mL) was added triphenylphosphine (1.3 g, 4.97 mmol) at 25 °C. After stirring the reaction at 40 °C for 2 hours, water (10 mL) was added to the solution at 40 °C and the mixture was stirred for 12 hours. The mixture was then diluted with DCM (50 mL) and washed with brine (2x50 mL). Organic layers were combined and concentrated under pressure to give a yellow solid (2.0 g). The residue was purified by flash column chromatography on silica gel (gradient elution: 0% - 10% MeOH in DCM) to give the compound **4g** (1.5 g, 81%) as a white solid. $^1H$ NMR (400 MHz, DMSO-$d_6$) $\delta$ = 8.15 (s, 1H), 5.57 (d, *J*=8.0 Hz, 1H), 4.07 - 4.00 (m, 1H), 3.91 (dd, *J*=5.3, 8.0 Hz, 1H), 3.75 - 3.63 (m, 3H), 3.37 (s, 3H), 2.77 - 2.69 (m, 1H), 1.10 (d, *J*=6.8 Hz, 6H), 0.86 (s, 9H), 0.04 (s, 6H); ESI-MS: m/z=481.3 $[M+H]^+$

Step 8: Preparation of compound **4h**

To a solution of compound **4g** (1.5 g, 2.81 mmol) in DCM (100 mL) was added 4-nitrophenol (3.12 g, 22.47 mmol), triethylamine (1.7 g, 16.8 mmol) and 4Å molecular sieves (2.0 g). After stirring the mixture at -78 °C for 0.5 h, 4-nitrophenyl chlorosulfate **3n** (2 g, 8.42 mmol) in DCM (20 mL) was added to the solution at -78 °C. After stirring at -78

°C for 15 min and stirred at 0 °C for 1 hour, the mixture was filtered and diluted with DCM (100 ml). Organic layer was washed with aqueous saturated $NaHCO_3$ (3x50 ml) and concentrated under pressure to give a yellow solid (2.5 g). The residue was combined with another batch (combined with silica gel: 6 g) and purified by flash column chromatography on silica gel (gradient elution: 0 - 100% ethyl acetate in DCM) to give compound **4h** (1.68 g, 75% from 2.4 g compound 74) as a yellow solid. $^1H$ NMR (400 MHz, $CD_3CN$) $\delta$ = 11.89 (br s, 1H), 9.27 (br s, 1H), 8.15 (br d, *J*=9.3 Hz, 2H), 7.94 - 7.90 (m, 1H), 7.23 (br d, *J*=9.0 Hz, 2H), 5.80 (br d, *J*=8.3 Hz, 1H), 4.81 (br d, *J*=7.3 Hz, 1H), 4.24 (br s, 1H), 3.97 (br d, *J*=4.5 Hz, 1H), 3.83 (br s, 2H), 3.48 - 3.39 (m, 3H), 2.73 - 2.58 (m, 1H), 1.19 (br dd, *J*=3.3, 6.0 Hz, 6H), 0.94 (s, 9H), 0.12 (s, 6H); ESI-MS: m/z=682.3 $[M+H]^+$

Step 9: Preparation of compound **4j**

A solution of compound **4h** (1.68 g, 2.47 mmol), compound **4i** (731 mg, 1.9 mmol) and 4Å Molecular sieves (2.0 g) in DCE (135 mL) was stirred under $N_2$ for 30 min at 25 °C, followed by addition of DMAP (1.16 g, 9.49 mmol). After stirring at 55 °C (oil temperature) for 12 hours, the mixture was filtered and the solution partitioned with DCM (100 mL) and brine (50 mL). The organic layer was washed with aqueous saturated $NaHCO_3$ (3×100 mL); organic layer was dried over anhydrous $Na_2SO_4$, filtered and the solvent evaporated under reduced pressureto give the crude product (2.5 g). The crude product (2.5 g, crude) (combined with silica gel: 5 g) was purified by flash column chromatography on silica gel (gradient elution: 0 - 10% MeOH in DCM) to give compound **4j** (1.2 g, 52%) as a light yellow solid. ESI-MS: m/z=927.4 $[M+H]^+$

Step 10: Preparation of compound **4k**

To a solution of compound **4j** (1.2 g, 1.29 mmol) in pyridine (24 mL) was added TEA (1.31 g, 12.94 mmol) and $Et_3N$-3HF (1.0, 6.47 mmol) at 15 °C. After stirring the solution at 35 °C for 12 hours, THF (20 mL) and trimethyl(propoxy)silane (3.4 g, 25.89 mmol) were added at 25°C and the reaction mixture was stirred for another 3 hours. The reaction mixture was concentrated under pressure to give a residue (2 g). The residue (combined with silica gel: 4 g) was purified by flash column chromatography on silica gel (gradient elution: 0 - 10% MeOH in DCM) to give crude compound **4k** (900 mg). The crude product was purified by reverse phase preparative HPLC (Method: Column: Waters Xbridge Prep OBD 5μm C18 150×30, Condition: water (10mM $NH_4HCO_3$)-ACN B: 5,End B 35, Gradient Time (min): 7, 100%B Hold Time (min): 1, Flow Rate (ml/min): 25) to give pure compound **4k** (0.57 g, 70%) as a white solid. $^1H$ NMR (400 MHz, DMSO-$d_6$) $\delta$ = 8.17 (d, *J*=4.4 Hz, 2H), 8.08 (s, 1H), 6.21 (d, *J*=19.6 Hz, 1H), 5.84 (d, *J*=8.6 Hz, 1H), 5.51 - 5.26 (m, 1H), 5.13 (br s, 1H), 4.72 (dd, *J*=5.4, 8.8 Hz, 1H), 4.53 - 4.38 (m, 1H), 4.22 - 4.16 (m, 1H), 4.14 - 4.10 (m, 1H), 4.09 - 4.00 (m, 2H), 3.86 (d, *J*=5.4 Hz, 1H), 3.63 - 3.46 (m, 2H), 3.25 (s, 3H), 2.75 (td, *J*=6.8, 13.6 Hz, 1H), 1.11 (dd, *J*=2.1, 6.7 Hz, 6H)
$^{19}F$ NMR (376 MHz, DMSO-d6) -201.424 (s, 1F); ESI-MS: *m*/*z* = 699.3 $[M+H]^+$

Step 11: Preparation of compound **4l**

$CH_3CN$ was freshly distilled over $CaH_2$ before use. Compound **4l** (152 mg, 0.218 mmol) was dissolved in DMF (2 mL) and $CH_3CN$ (6 mL), to which were added 0.3 g of 4A MS (powder) and a solution of 1H-tetrazole (3.87 mL, 0.45 M, prepared by dissolved 472.5 mg of tetrazole in 15 mL of dry $CH_3CN$, followed by addition of 1 g of 4Å MS and then stirred for 0.5 hr under $N_2$ before use). A solution of 2-cyanoethyl N,N,N',N'-tetraisopropyl phosphorodiamidite (131.15 mg, 0.43 mmol) in $CH_3CN$ (0.8 mL) was added drop-wise over 20 min vial a syringe. The resulting white suspension was further stirred for 2 hr at 25 °C under $N_2$. TBHP (0.218 mL, 1.09 mmol, 5 M in decane) was then added to the above solution at 25 °C. After stirring the reaction at 25 °C for 1 h, the mixture was diluted with DCM (20 mL) and $CH_3OH$ (3 mL), filtered through a pad of diatomaceous earth and concentrated under vacuum to give a colorless oil; the oil was purified by flash column chromatography on silica gel (4 g, 0-11.5% MeOH in DCM) to give compound **4l** (151 mg, 68%) as a white solid. ESI-MS: m/z=814.3 $[M+H]^+$.

Step 12: Preparation of **compound 52, sodium salt**

A solution of compound **4l** (150 mg, 0.18 mmol) in EtOH (2 mL) was treated with $MeNH_2$ (5 mL, 30% in EtOH). After stirring the reaction at 25 °C for 2 h, and at 35 °C for 1 h, the solvent was concentrated under reduced pressure to give a colorless oil. The residue was dissolved into $H_2O$ (20 mL) and $CH_3CN$ (5 mL), then washed with DCM (20 mLx 2). The aqueous phase was then lyophilized to give the crude product (80 mg, crude) as a light yellow solid. The crude product (80 mg, crude) was purified by reverse phase preparative HPLC (Method: Column, Waters Xbridge Prep OBD 5μm C18 150x30; Condition water (10mM $NH_4HCO_3$) (A)-ACN (B) Begin B 0 End B 25; Gradient Time (min) 7; 100%B Hold Time (min) 1 FlowRate(ml/min) 25 Injections 12) to give **compound 52, ammonium salt** (35 mg, 26%) as a white

solid. $^1H$ NMR (400 MHz, $D_2O$) δ = 8.29 (s, 1H), 8.19 (br d, J=9.5 Hz, 2H), 6.61 (br d, J=18.6 Hz,1H), 6.08 (br d, J=8.8 Hz, 1H), 5.90 - 5.72 (m, 1H), 5.39 (br d, J=19.6 Hz, 1H), 5.20 (br s, 1H), 4.76 (br s, 1H), 4.72 (br s, 1H), 4.49 (br d, J=10.3 Hz, 3H), 4.35 (br d, J=4.3 Hz, 1H), 4.17 (q, J=6.2 Hz, 1H), 3.67 (s, 3H); $^{19}F$ NMR (376.5MHz, $D_2O$) -199.859; $^{31}P$ NMR (162MHz, $D_2O$) -1.639; ESI-MS: m/z=691.1 $[M+H]^+$.

**Conversion to sodium salt**

Dowex 50W × 8, 200-400 (5 mL, H form) was added to a beaker and washed with deionized $H_2O$ (20 mL). Then to the resin was added 15% $H_2SO_4$ in de-ionized $H_2O$ (20 mL), the mixture was gently stirred for 15 min, and decanted (15 mL). The resin was transferred to a column with 15% $H_2SO_4$ in de-ionized $H_2O$ and washed with 15% $H_2SO_4$ (at least 4 CV), and then with de-ionized $H_2O$ until it was neutral. The resin was transferred back into the beaker, 15% NaOH in de-ionized $H_2O$ (20 mL) solution was added, and mixture was gently stirred for 15 min, and decanted (1 x). The resin was transferred to the column and washed with 15% NaOH in $H_2O$ (at least 4 CV), and then with de-ionized $H_2O$ until it was neutral. The CDN **(compound 52,**35 mg, 0.049 mmol)) was dissolved in de-ionized $H_2O$ (8 mL), added to the top of the column, and eluted with de-ionized $H_2O$. Appropriate fractions were pooled together and lyophilized to get sodium salt form (30 mg, purity: 91%) as white solid. The salt form (30 mg) was purified by reverse phase preparative HPLC (Method: Column, Waters Xbridge Prep OBD 5μm C18 150×30; Condition water (10mM $NH_4HCO_3$) (A)-ACN(B) Begin B 0 End B 30; Gradient Time(min) 7; 100%B Hold Time(min) 1 FlowRate(ml/min) 25) to give the product (25 mg) as a white solid. The product (25 mg, purity: 97.07%) was purified a second time by by reverse phase preparative HPLC (Method: Column, Waters Xbridge Prep OBD 5μm C18 150×30; Condition water (10mM $NH_4HCO_3$) (A)-ACN (B) Begin B 0 End B 30; Gradient Time (min) 7; 100%B Hold Time (min) 1 Flow Rate (ml/min) 25) to give **compound 52, ammonium salt** which was treated with the ion exchange resin DOWEX 50W × 8,200-400 to give **compound 52, sodium salt** (16.1 mg, 65%) as a white solid. $^1H$ NMR (400 MHz, $D_2O$) δ = 8.01 (s, 1H), 7.93 (s, 1H), 7.85 (s, 1H), 6.37 (br d, J=17.3 Hz, 1H), 5.83 (d, J=9.0 Hz, 1H), 5.59 (br d, J=3.5 Hz, 0.5H), 5.46 (br d, J=3.5 Hz, 0.5H), 5.15 - 5.02 (m, 1H), 4.97 (br d, J=4.5 Hz, 1H), 4.62 - 4.52 (m, 2H), 4.49 (br s, 1H), 4.29 - 4.19 (m, 3H), 4.10 (br d, J=4.5 Hz, 1H), 3.43 (s, 3H); $^{19}F$ NMR (376.5MHz, $D_2O$) -200.863; $^{31}P$ NMR (162MHz, $D_2O$) -1.676
ESI-MS: m/z=691.2 $[M+H]^+$.

Example 5

**Compound 6**

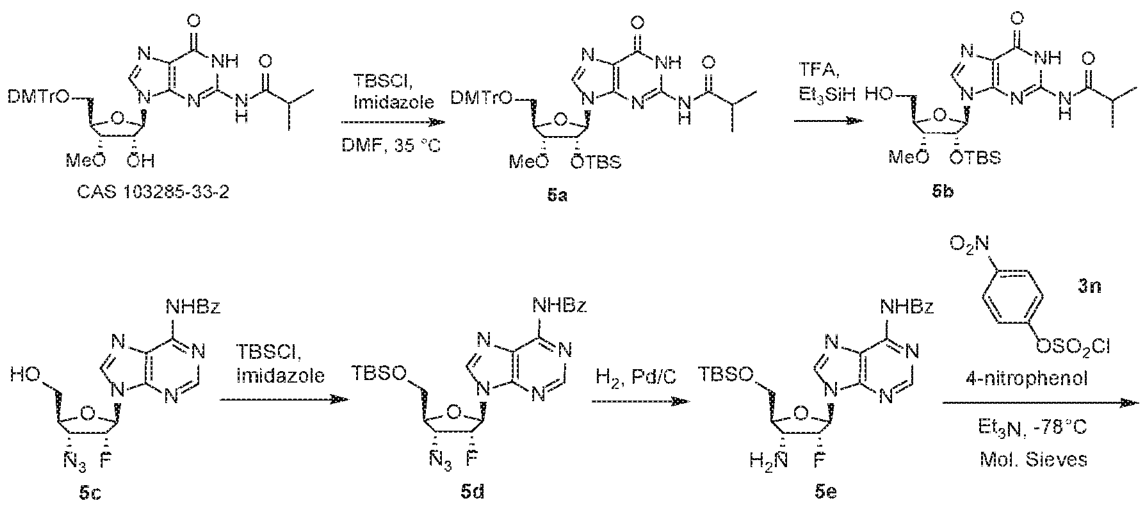

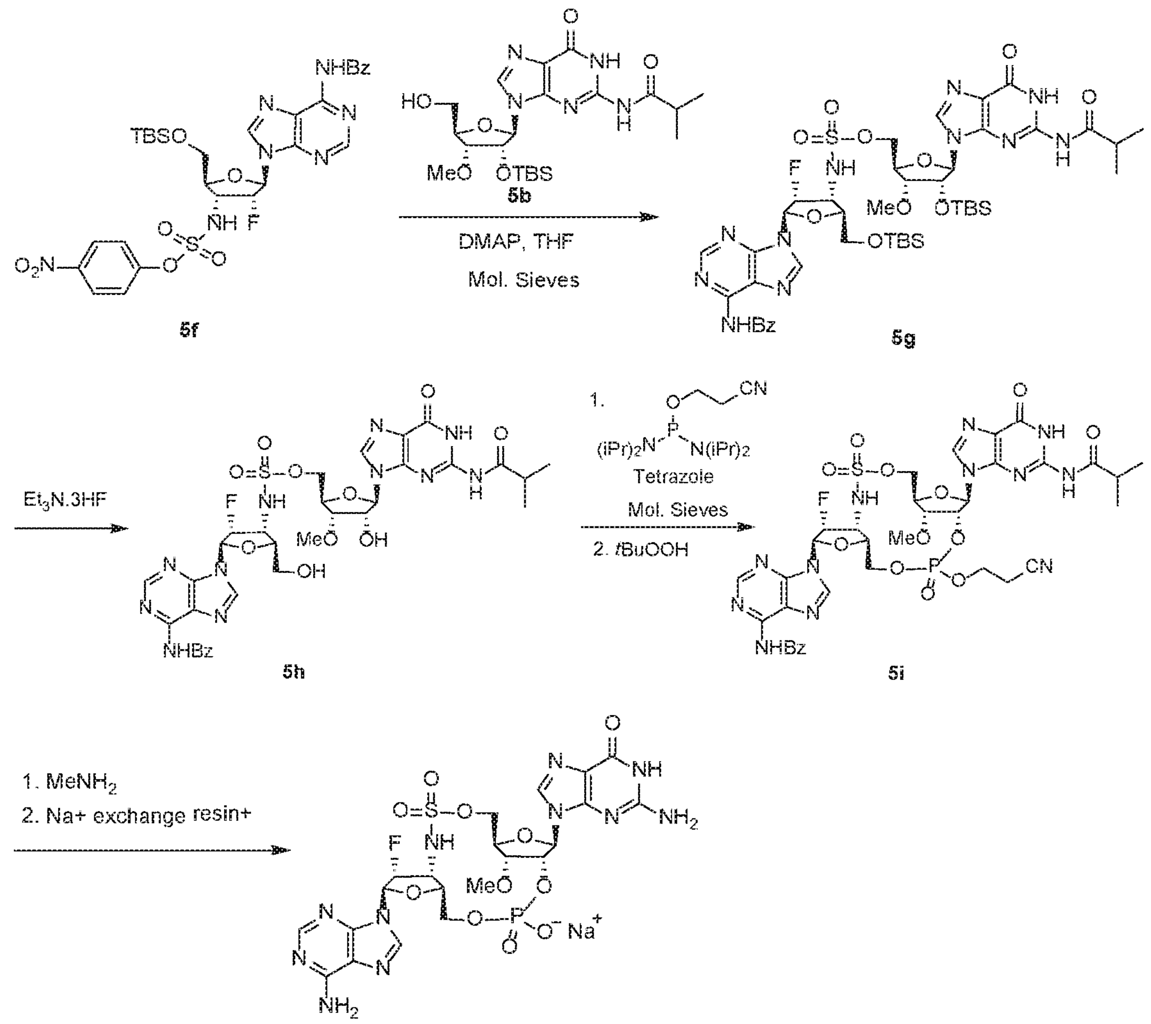


Compound 6, sodium salt

Step 1: Preparation of compound **5a**

A solution of 5'-O-(4,4'-dimethoxytrityl)-N2-isobutyryl-3'-O-methyl-D-guanosine [CAS 103285-33-2] (5.2 g, 7.76 mmol) in DMF (50 mL), to which imidazole (2.38 g, 34.94 mmol) and TBSC1 (3.51 g, 23.29 mmol) were added, was stirred for 6 h at 35 °C. The reaction mixture was quenched with saturated aqueous $NaHCO_3$ and extracted with DCM The combined organic layers were washed with brine, dried over $Na_2SO_4$, filtered and concentrated. The crude product was purified by flash column chromatography over silica gel (gradient elution: 0 - 15% MeOH in DCM) to give compound **5a** which was used as such in the next step. ESI-MS: m/z 784.4 $[M+H]^+$.

Step 2: Preparation of compound **5b**

TFA (2 mL, 26.12 mmol) and $Et_3$ SiH (8 mL, 50.03 mmol) were added to a solution of the above compound **5a** in DCM (160 mL) at 0 °C. The resulting mixture was stirred at 0 °C for 30 min, after which stirring was continued at room temperature for 4 h. The reaction solution was quenched with aqueous $NaHCO_3$ and extracted with DCM. The combined organic layers were washed with brine, dried over $Na_2SO_4$, filtered and concentrated. The crude product was purified by column chromatography over silica gel (gradient elution: 0 - 5% MeOH in DCM) to give compound **5b** as a white solid (2.9 g). $^1H$ NMR (400 MHz, CHLOROFORM-*d*) δ ppm 12.12 (br s, 1 H), 8.36 (br s, 1 H), 7.71 (s, 1 H), 5.72 (d, *J*=7.5 Hz, 1 H), 5.41 (br s, 1 H), 4.78 (dd, *J*=7.5, 5.1 Hz, 1 H), 4.30 (br s, 1 H), 3.98 (dd, *J*=12.6, 2.2 Hz, 1 H), 3.84 (d, *J*=5.1 Hz, 1 H), 3.71 (br s, 1 H), 3.54 (s, 3 H), 2.68 (spt, *J*=6.9 Hz, 1 H), 1.28 (d, *J*=6.8 Hz, 3 H), 1.29 (d, *J*=6.8 Hz, 3 H), 0.81 (s, 9 H), -0.07 (s, 3 H), -0.29 (s, 3 H); ESI-MS: *m*/*z* 482.1 $[M+H]^+$.

Step 3: Preparation of compound **5d**

A solution of 5c [CAS 2241580-02-7] (2 g, 5.02 mmol) in DMF (10 mL), to which imidazole (1.02 g,15.06 mmol) and TBSCl (1.51 g, 10.04 mmol) were added, was stirred for 2 h at room temperature. The reaction mixture was diluted with EtOAc and washed with water. The aqueous layer was extracted with EtOAc. The combined organic layers were washed with saturated aqueous $NaHCO_3$ and brine, dried over $Na_2SO_4$, filtered and concentrated. The crude product was purified by column chromatography over silica gel (gradient elution: 0 - 15% MeOH in DCM) to afford compound **5d** as a yellow solid (2.57 g, yield: 100%). ESI-MS: *m/z* 513.2 $[M+H]^+$.

Step 4: Preparation of compound **5e**

A solution of compound **5d** (1.285 g, 2.51 mmol) in EtOAc (150 mL) was hydrogenated at room temperature under atmospheric pressure for 2 h using 10% of Pd/C (2.95 g) as catalyst. The reaction mixture was filtered and the filtrate concentrated under reduced pressure. The reaction was repeated on the same scale, the crude product of both reactions was combined for purification by column chromatography over silica gel (gradient elution: 0-5% MeOH in DCM) to afford compound **5e** (1.69, yield: 69%) as a white solid. ESI-MS: *m/z* 487.1 $[M+H]^+$.

Step 5: Preparation of compound **5f**

A solution of compound **5e** (1.05 g, 2.16 mmol) in DCM (40 ml), to which 4-nitrophenol (900 mg, 6.47 mmol), $Et_3N$ (1.79 mL, 12.95 mmol) and activated molecular sieves were added, was stirred at room temperature for 30 min. The mixture was cooled to -78 °C, after which 4-nitrophenyl chlorosulfate (1.54 g, 6.47 mmol) in DCM (10 mL) was added, stirring was continued for 2.5 h at -78 °C. The reaction mixture was filtered and washed with aqueous $NaHCO_3$, the aqueous washing layers were extracted with DCM. The combined organic layers were dried with $Na_2SO_4$, filtered and concentrated under reduced pressure. The crude product was purified by column chromatography over silica gel (gradient elution: 0 - 100% EtOAc in petroleum ether) to give compound 5f as a white solid (1.24 g, yield: 83.5%). ESI-MS: *m/z* 688.2 $[M+H]^+$.

Step 6: Preparation of compound **5g**

Activated molecular sieves were added to a solution of compound **5b** (105 mg, 0.218 mmol) and sulfamate **5f** (180 mg, 0.262 mmol) in dry THF (2 mL), the resulting mixture was stirred at room temperature for 1 h under $N_2$. Next, DMAP (133 mg, 1.09 mmol) was added to initiate the reaction, the reaction mixture was stirred at room temperature for 18 h. The molecular sieves were removed by filtration and the filtrate was concentrated under reduced pressure. The residue was purified by column chromatography over silica gel (gradient elution: 0 - 5% MeOH in DCM) to afford compound **5g** as a yellow solid (157 mg, yield: 70%). $^1H$ NMR (400 MHz, $CDCl_3$) $\delta$ ppm 12.18 (s, 1H), 9.07 (s, 1H), 8.78 (s, 1H), 8.39 (s, 1H), 8.05 (d, *J*=7.3 Hz, 2H), 7.66 - 7.59 (m, 1H), 7.58 - 7.49 (m, 3H), 7.19 (br s, 1H), 6.32 (d, *J*=17.8 Hz, 1H), 5.60 - 5.42 (m, 2H), 5.60 - 5.42 (m, 1H), 4.80 - 4.63 (m, 2H), 4.49 (dd, *J*=3.8, 11.0 Hz, 1H), 4.36 - 4.27 (m, 2H), 4.17 - 4.02 (m, 2H), 3.80 (dd, *J*=2.3, 4.8 Hz, 1H), 3.51 (s, 3H), 2.75 - 2.63 (m, 1H), 1.33 (d, *J*=6.8 Hz, 3H), 1.27 (d, *J*=6.8 Hz, 3H), 0.91 (s, 9H), 0.77 (s, 9H), 0.89 (s, 3H), 0.91 (s, 3H), -0.08 (s, 3H), -0.28 (s, 3H). ESI-MS: *m/z* 1030.5 $[M+H]^+$.

Step 7: Preparation of compound **5h**

$Et_3N$ (2.62 g, 25.92 mmol) and triethylamine trihydrofluoride (6.28 g, 51.83 mmol) were added to solution of compound **5g** (890 mg, 0.864 mmol) in pyridine (10 mL), the resulting reaction mixture was stirred under $N_2$ at room temperature for 18 h. The mixture was filtered and concentrated under reduced pressure. The residue was purified by column chromatography over silica gel (gradient elution: 0 - 10% MeOH in DCM) followed by purification by preparative reversed phase HPLC (Stationary phase: Phenomenex Synergi Max-RP, 10 $\mu$M, 250 $\times$ 50 mm; Mobile phase: water (A) - MeCN (B); gradient elution) to give compound **5h** as a white solid (397 mg, yield: 57%). $^1H$ NMR (400 MHz, DMSO-$d_6$) $\delta$ ppm 12.07 (s, 1 H), 11.60 (s, 1 H), 11.20 (s, 1 H), 8.89 (br d, *J*=9.0 Hz, 1 H), 8.71 (d, *J*=22.8 Hz, 2 H), 8.14 (s, 1 H), 8.05 (d, *J*=7.3 Hz, 2 H), 7.60 - 7.72 (m, 1 H), 7.47 - 7.60 (m, 2 H), 6.46 (d, *J*=19.9 Hz, 1H), 5.84 (d, *J*=5.7 Hz, 1H), 0.00 (d, *J*=6.1 Hz, 1H), 5.66 (dd, *J*=52.1, 4.5 Hz, 1 H), 5.22 (br t, *J*=5.1 Hz, 1 H), 4.50 - 4.69 (m, 2 H), 4.22 - 4.42 (m, 3 H), 4.05 - 4.16 (m, 1 H), 3.93 (t, *J*=4.1 Hz, 1 H), 3.82 (br dd, *J*=12.2, 4.4 Hz, 1 H), 3.54 - 3.68 (m, 1 H), 3.42 (s, 3 H), 2.75 (spt, *J*=6.9 Hz, 1 H), 1.12 (d, *J*=6.9 Hz, 6 H); ESI-MS: *m/z* 802.3 $[M+H]^+$.

Step 8: Preparation of compound **5i**

A solution of compound **5h** (200 mg, 0.25 mmol) and 1*H*-tetrazole (1.82 mL of a 3 - 4% in MeCN, dried on 3Å

molecular sieves before use) in dry THF / MeCN (1:1, 12 mL, dried on 3Å molecular sieves before use) was treated with activated 3Å molecular sieves for 2 h under $N_2$ after which 2-cyanoethyl-*N*,*N*,*N'*,*N'*-tetra(isopropyl)phosphorodiamidite (75 mg, 0.25 mmol) was added in one portion. The reaction mixture was shaken overnight An additional amount of 2-cyanoethyl-*N,N,N',N'*-tetra(isopropyl)phosphorodiamidite (22.5 + 15 mg, 0.075 + 0.05 mmol) was added in two portions with a time interval of 2 h, after which shaking was continued for 90 min. A solution of *t*BuOOH (68 μL of a 5.5 M solution in decane, 0.37 mmol) was added, the reaction mixture was shaken for 30 min. The molecular sieves were removed by filtration and rinsed with dichloromethane. The filtrate was washed with brine and concentrated under reduced pressure. The crude product was purified by column chromatography over silica gel (gradient elution: 0 - 10% MeOH in DCM) give compound **5i** (30 mg, yield: 13%). ESI-MS: *m*/*z* 917.5 $[M+H]^+$.

Step 9: Preparation of **compound 6, sodium salt**

The above compound 5i (30 mg, 0.033 mmol) was stirred in a 33% methylamine solution in ethanol (1 mL) at room temperature until complete conversion (ca. 2 h). The crude product, obtained after concentration under reduced pressure, was triturated in MeCN. Final conversion into the sodium salt was done by elution of an aqueous solution over a column packed with a cationic Na ion-exchange resin affording **compound 6, sodium salt** as a white fluffy solid (14 mg, yield: 60%). $^1$H NMR (400 MHz, DMSO-$d_6$, 61 °C) δ ppm 10.39 (br s, 1 H), 8.32 (s, 1 H), 8.19 (s, 1 H), 7.97 (s, 1 H), 7.07 (br s, 2 H), 6.31 (br s, 2 H), 6.24 (dd, *J*=15.7, 2.7 Hz, 1 H), 5.78 (d, *J*=9.0 Hz, 1 H), 5.44 (br d, *J*=53.9 Hz, 1 H), 5.11 (td, *J*=9.3, 4.0 Hz, 1 H), 4.42 - 4.57 (m, 1 H), 4.13 - 4.25 (m, 4 H), 4.11 (d, *J*=3.9 Hz, 1 H), 3.90 - 4.03 (m, 1 H), 3.79 - 3.88 (m, 1 H), 3.53 (s, 3 H);
$^{31}$P NMR (162 MHz, DMSO-$d_6$) δ ppm -1.45 (s, 1 P); ESI-MS: *m*/*z* 690.3 $[M+H]^+$.

<u>Example 6</u>

## Compound 9

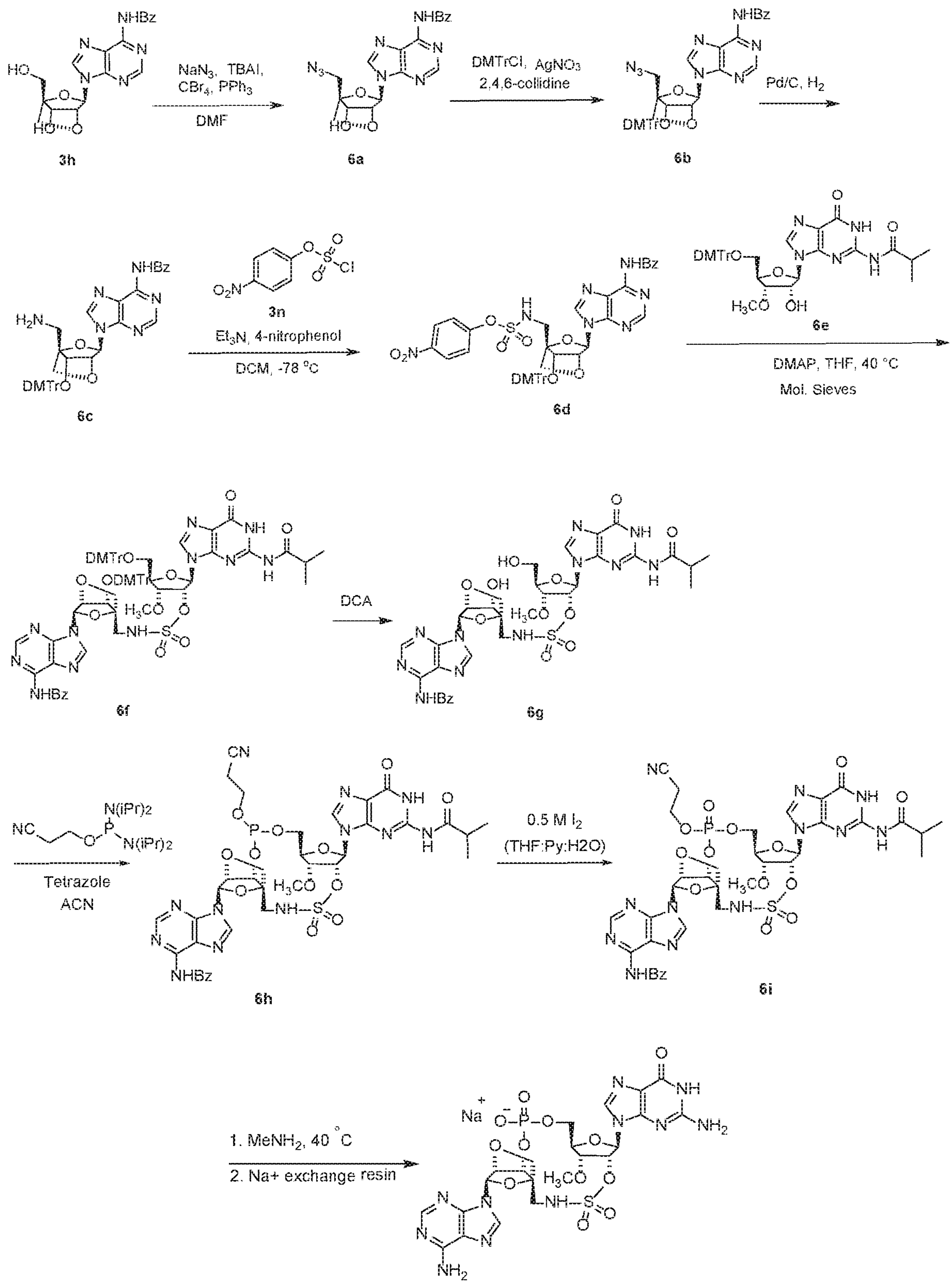


## Compound 9, sodium salt

Step 1: preparation of compound **6a**

Compound **3h** (1.0 g, 2.61 mmol) was co-evaporated with anhydrous toluene: $CH_3CN$ (v:v=1:1, 6 mL × 2 ) and next dissolved in anhydrous DMF (18.8 mL). It was then added triphenylphosphine (1.02 g, 3.91 mmol), $NaN_3$ (0.63 g, 9.73 mmol), tetrabutylammonium iodide (192.7 mg, 0.52 mmol) and $CBr_4$ (1.3 g, 3.91 mmol) at RT. After stirring the reaction at RT for 12h, the reaction was quenched with brine (10 mL) and partitioned with EtOAc (20 mL). Saturated aqueous $NaHCO_3$ (20 mL) solution was added to the mixture followed by extraction with EtOAc (20 mL × 3). Organic layers were then combined and successively dried over anhydrous $Na_2SO_4$, filtered and concentrated under reduced pressure to give a residue. The residue was purified by flash column chromatography on silica gel (EtOAc : Petroleum Ether = 0 to 100%, followed by MeOH : DCM = 0 to 5%) to give **6a** as a yellow powder. ESI-MS: m/z 408.9 $[M+H]^+$.

Step 2: preparation of compound **6b**

To a solution of **6a** (1.24 g, 3.04 mmol) in DCM (16 mL) was added 2,4,6-collidine (1.4 g, 11.56 mmol), $AgNO_3$ (1.96 g, 11.56 mmol) and DMTrCl (1.54 g, 4.56 mmol) at 15 °C. After stirring at 15 °C for 5 hr, the reaction mixture was quenched with MeOH (20 mL) and diluted with DCM (40 mL). Organic layer was successively washed with brine (20 mLx2), dried over anhydrous $Na_2SO_4$, filtered and concentrated under reduced pressure to give a residue. The residue was purified by flash column chromatography on silica gel (EtOAc : Petroleum Ether = 0 to 100%) to give **6b** (1.99 g) as white solid. $^1H$ NMR (400 MHz, $CDCl_3$) $\delta$7.17 - 7.00 (m, 6H), 6.93 (s, 4H), 6.73 - 6.49 (m, 4H), 5.28 (s, 2H), 4.10 (d, J=7.3 Hz, 3H), 3.79 - 3.56 (m, 5H), 3.47 (br d, J=4.6 Hz, 1H), 3.37 - 3.11 (m, 2H), 2.94 (s, 1H), 2.87 (s, 1H), 2.61 (s, 6H), 2.31 (s, 3H), 2.03 (s, 1H), 1.72 - 1.56 (m, 5H), 1.53 - 1.35 (m, 2H), 1.24 (t, J=7.2 Hz, 1H), 0.99 (t, J=7.3 Hz, 3H). ESI-MS: m/z 711.2 $[M+H]^+$.

Step 3: preparation of compound **6c**

A suspension **of 6b** (1.99 g, 2.8 mmol) with 10% Pd/C wet (3.3 g, 2.8 mmol) as a catalyst in EtOAc (100 mL) was hydrogenated (15 psi) at RT (~15°C) for 5 h. The catalyst was filtered off and the filtrate was evaporated under reduced pressure to give crude 6c (1.31 g) as a white solid, used directly into the next step without any further purification ESI-MS: m/z=685.2 $[M+H]^+$.

Step 4: preparation of compound **6d**

Compound 6c (213 mg, 0.31 mmol) was co-evaporated with anhydrous toluene: $CH_3CN$ (v:v=1:1, 3x2 mL) and dissolved in anhydrous DCM (8 mL); it was then added 4-nitrophenol (129.8 mg, 0.93 mmol), $Et_3N$ (188.8 mg, 1.86 mmol) and activated 4Å molecular sieves (~3 g) under the $N_2$ at RT (~10°C); the mixture was cooled down to -78°C followed by the rapid addition of 4-nitrophenyl chlorosulfate **3n** (221.74 mg, 0.93 mmol) under $N_2$ at -78°C. After stirring at -78°C for 3hr, the reaction mixture was diluted with DCM (30 mL) and filtered through a pad of diatomaceous earth. The filtrate was partitioned between DCM/saturated aqueous $NaHCO_3$ (30/45 mL) and the aqueous layer extracted with DCM (15 mLx3). Organic layers were combined and successively washed with brine (20 mL×2), dried over anhydrous $Na_2SO_4$, filtered and concentrated under reduced pressure to give a residue. The residue was purified by flash column chromatography on silica gel (EtOAc : Petroleum Ether=0 to 90%) to give **6d** (142 mg) as a yellow solid. $^1H$ NMR (400 MHz, $CDCl_3$) $\delta$9.25 - 9.05 (m, 2H), 8.35 - 8.26 (m, 1H), 8.05 - 7.94 (m, 4H), 7.76 - 7.64 (m, 1H), 7.58 (br t, J=6.7 Hz, 1H), 7.50(t, J=7.5 Hz, 2H), 7.38 - 7.27 (m, 4H), 7.24 (s, 1H), 7.20 - 7.10 (m, 7H), 6.65 (br d, J=8.8 Hz, 2H), 6.62 (br d, J=7.9 Hz, 2H), 5.86 (s, 1H), 4.53 (s, 1H), 4.41(d, J=7.6 Hz, 1H), 4.09 (q, J=7.3 Hz, 2H), 4.02 - 3.93 (m, 2H), 3.79 (br d, J=12.5 Hz, 1H), 3.71 (s, 3H), 3.70 (s, 3H), 2.88 (s, 1H), 2.01 (s, 3H), 1.22 (t, J=7.2 Hz, 3H). ESI-MS: m/z= 886.2 $[M+H]^+$.

Step 5: preparation of compound **6f**

Compound **6d** was co-evaporated with anhydrous toluene : $CH_3CN$ (1:1, 6 mL x 2 ) before use. A mixture of **6d** (699 mg, 0.79 mmol), **6e** (739.81 mg, 1.1 mmol, CAS# 103285-33-2) and activated 4Å Molecular Sieves (~ 5 g) in dry THF (20 mL) was stirred at RT for 1 h under $N_2$. DMAP (481.97 mg, 3.94 mmol) was added in one portion and the reaction mixture was stirred at 40°C (oil temperature) for 5 hr under $N_2$. The reaction mixture was diluted with DCM (15 mL) and filtered through a pad of diatomaceous earth. The filtrate was concentrated under reduced pressure to give a yellow residue. The residue was purified by flash column chromatography on silica gel (MeOH:DCM=0 to 10%) to afford **6f** (969 mg) as a pale yellow solid. ESI-MS: m/z= 1416.2 $[M+H]^+$.

Step 6: preparation of compound **6g**

DCA (6% in DCM, 10.8 mL) was added under $N_2$ to a solution of compound 6f (1.1 g, 0.78 mmol) in DCM (22 mL), the resulting red solution was stirred at RT for 30 min then quenched with pyridine (12 mL). The clear reaction mixture was concentrated under reduced pressure to give a colorless residue which was purified by flash column chromatography on silica gel (MeOH : DCM = 0 to 10%) to give compound **6g** (740 mg) as a white solid. $^1H$ NMR (400 MHz, $CD_3OD$) $\delta$ 8.67 (s, 1H), 8.40 (s, 1H), 8.14 - 8.09 (m, 3H), 7.71 - 7.65 (m, 1H), 7.62 - 7.56 (m, 2H), 6.19 (d, J=5.3 Hz, 1H), 6.03 (s, 1H), 5.53 (t, J=5.4 Hz, 1H), 4.84 (br s, 1H), 4.48 (s, 1H), 4.43 (s, 1H), 4.32 (t, J=4.8 Hz, 1H), 4.14 (br d, J=3.8 Hz, 1H), 3.99 (d, J=8.0 Hz, 1H), 3.83 - 3.77 (m, 1H), 3.73 - 3.66 (m, 2H), 3.60 (s, 3H), 3.54 (d, J=13.3 Hz, 1H), 3.30 - 3.23 (m, 2H), 2.67 (td, J=6.7, 13.7 Hz, 1H), 1.17 (dd, J=2.4, 6.9 Hz, 6H). ESI-MS: m/z= 812.2 $[M+H]^+$.

Step 7: preparation of compound **6i**

Compound **6g** (105 mg, 0.129 mmol) was co-evaporated with mixture of anhydrous Toluene: Acetonitrile (1:1, v/v, 3 $\times$ 30 mL) then dissolved in anhydrous THF (12 mL). 4 Å Molecular sieves powder (0.3 g) and 0.45 M Tetrazole in $CH_3CN$ (2.3 mL, 1.03 mmol) were added and the resulting heterogeneous mixture was bubbled with Argon for 4 min. After stirring this mixture at RT for 10 min, a solution of 2-cyanoethyl-*N*,*N*,*N'*,*N'*-tetra(isopropyl)phosphorodiamidite in $CH_3CN$ (59 mg, 0.19 mmol, 3.0 mL $CH_3CN$) was added over 30 min at RT. After stirring the reaction for 1.5 hr, the mixture was filtered off then washed with THF (15 mL). (Compound **6h.** MS: m/z 911 $[M+H]^+$). The resulting mixture was used directly in the next step. 0.5 M Iodine solution (in THF:water:Py 8:1:1, v/v/v) was added until the color persists. After stirring the reaction mixture at RT for 30 min, the mixture was diluted with EtOAc (30 mL) and excess iodine quenched with saturated aqueous $Na_2S_2O_3$ (until discoloration). Layers were separated; organic layer was washed with saturated aqueous $NaHCO_3$ (1 $\times$ 20 mL) and brine (1 $\times$ 20 mL). Aqueous layer was back extracted with EtOAc (1 $\times$ 20 mL). Combined organic layers were evaporated to dryness, the resulting crude material was purified by flash column chromatography on silica gel (MeOH in DCM: 0 to 15%, v/v) to generate compound **6i** (75 mg); ESI-MS: m/z 927 $[M+H]^+$.

Step 8: preparation of compound **9**

A solution of compound **6i** (75 mg, 0.08 mmol) in $MeNH_2$ (33% in EtOH, 6 mL) was stirred at 40°C for 2 hr 30 min, concentrated under reduced pressure.

The resulting crude solid was washed with DCM (15 mL) and the precipitate was filtered off and purified by reverse phase preparative HPLC (column: Synergi 4$\mu$, Hydro RP, 250 mm $\times$ 30 mm $\times$ 10 $\mu$M, Mobile Phase: Buffer A: 50 mM Triethylammonium acetate in WATER; Buffer B: 50 mM Triethylammoniumacetate in $CH_3CN$; gradient: 0-40% of B over 30 min, flow rate: 24 mL/min) to afford compound 9 (18.2 mg) as the TEAA salt. Final conversion into the sodium salt was done by elution of an aqueous solution over a column packed with a cationic sodium ion-exchange resin to give **compound 9, sodium salt** (17.3 mg). $^1H$ NMR (400 MHz, $D_2O$): $\delta$ 7.96 (s, 1H), 7.72 (s, 1H), 7.12 (s, 1H), 5.90-6.12 (m, 3H), 4.97 (s, 1H), 4.79 (s, 1H), 4.60 (m, 1H), 4.41 (t, J = 5.6 Hz, 1H), 4.25 (d, J = 4.0 Hz, 1H), 4.05 (d, J = 8.0 Hz, 1H), 3.90-4.01 (m, 1H), 3.85 (d, J = 8.0 Hz, 1H), 3.55 (d, J = 12.8 Hz, 1H), 3.41 (s, 3H), 3.31 (d, J = 12.8 Hz). $^{31}P$ NMR (162 MHz, $D_2O$): $\delta$ -1.46 (s, 1P). ESI-MSs: m/z: 698 $[M-1]^-$.

Example 7

## Compound (*R) 14A

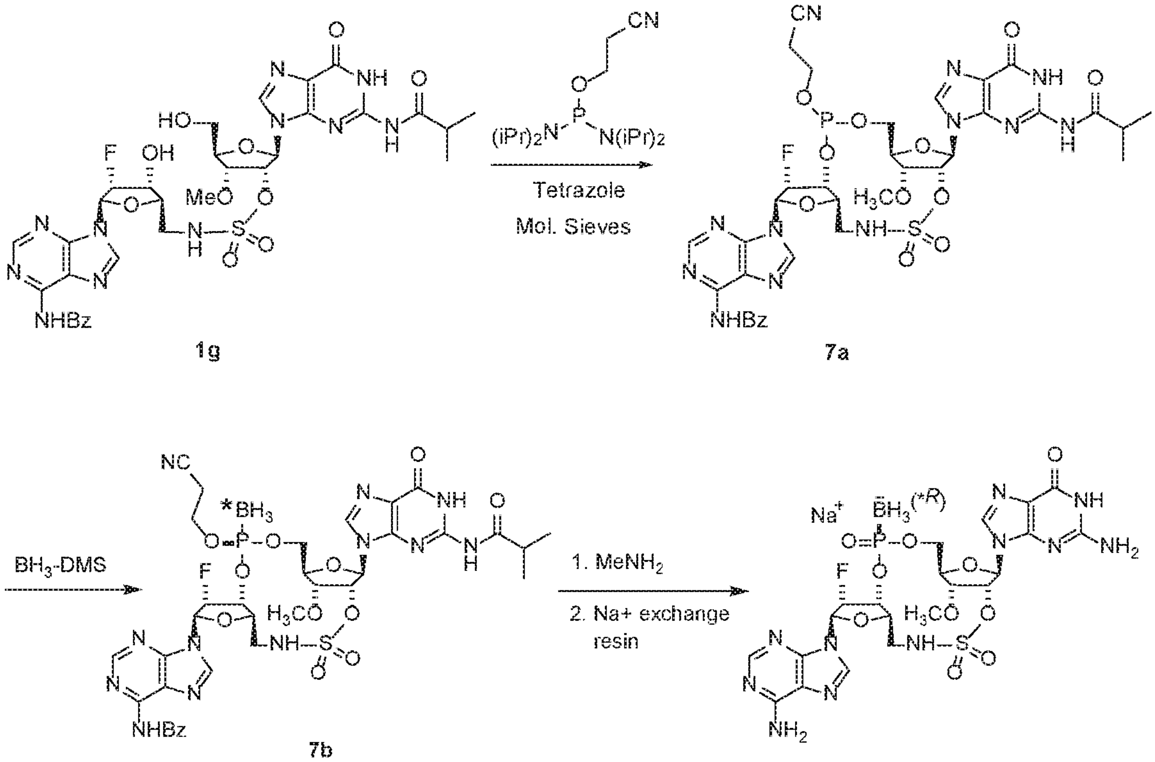


1g

7a

7b

Compound (*R) 14A, sodium salt

Step 1: preparation of compound **7b**

Compound **1g** (80 mg, 0.099 mmol) was co-evaporated with mixture of anhydrous Toluene: Acetonitrile (1:1, v/v, 3 × 15 mL) then dissolved in anhydrous THF (10 mL). 4 Å Molecular sieves powder (0.3 g) and 0.45 M Tetrazole in $CH_3CN$ (1.3 mL, 0.598 mmol) were added and the resulting heterogeneous mixture was bubbled with Argon for 4 min. After stirring this mixture at RT for 10 min, a solution of 2-cyanoethyl-*N*,*N*,*N'*,*N'*-tetra(isopropyl)phosphorodiamidite in $CH_3CN$ (48 mg, 0.16 mmol, in 3.0 mL $CH_3CN$) was added over 30 min at RT. After stirring the reaction for 1.5 hr, the mixture was filtered off and washed with THF (15 mL). (Compound **7a.** MS: m/z 901 $[M+H]^+$). The resulting mixture was used directly into the next step.

Borane dimethyl sulfide complex solution (2.0 M in THF, $BH_3$-DMS, 180 μL, 0.35 mmol) was added very slowly for 5 min at 0 °C. After stirring the reaction for 20 min at RT, the reaction mixture was quickly filtered off, diluted with EtOAc (50 mL) and quenched with water (20 mL). Layers were separated; organic layer was washed with water (1 × 20 mL) and brine (1 × 20 mL); aqueous layer was back extracted with EtOAc (1 × 20 mL). Organic layers were then combined and evaporated to dryness. The residue was purified by flash column chromatography on silica gel (0 -15% MeOH in DCM, v/v) to give 7b (48 mg). ESI-MS: m/z 915 $[M+H]^+$.

Step 2: preparation of **compound (*R) 14A**

A solution of compound **7b** (48 mg) in $MeNH_2$ (33% in EtOH, 6 mL) was stirred at 40 °C for 2 hr and concentrated under reduced ressure. The resulting crude solid was washed with DCM (15 mL) and the precipitate was filtered off and purified by reverse phase preparative HPLC (column: Synergi 4μm, Hydro RP, 250 mm × 4.6 mm, Mobile Phase: Buffer A: 50 mM Triethylammonium acetate in water; Buffer B: 50 mM triethylammonium acetate in $CH_3CN$, gradient: 0-40% of B over 30 min, flow rate 24 mL/min) to give **compound 14** (20.2 mg) as a TEAA salt. Final conversion into the sodium salt was done by elution of an aqueous solution over a column packed with a cationic sodium ion-exchange resin to give **compound (*R) 14A, sodium salt** (18.8 mg). $^1H$ NMR (400 MHz, $D_2O$) $\delta$7.92 (s, 1H), 7.81 (s, 1H), 7.08 (s, 1H), 6.17-6.24 (d, J = 20.8 Hz, 1H), 5.95 (d, J = 7.2 Hz, 1H), 5.45 (s, 1H), 5.29 (s, 0.5H), 5.16 (s, 0.5H), 4.70-4.85 (m, 1H), 4.49 (s, 1H), 4.33 (d, J = 8 Hz, 1H), 4.27 (s, 1H), 4.05-4.15 (m, 1H), 3.96 (d, J = 10.8 Hz, 1H), 3.64 (d, J = 12.4 Hz, 1H), 3.44 (s, 1H),

3.30-3.44 (m, 2H), 0.3 (br.s, 3H); $^{31}P$ NMR (162 MHz, $D_2O$) $\delta$ 95.57 ppm (s, 1 P) $^{19}F$ NMR (379 MHz, $D_2O$) $\delta$ broad peak -196.84 ppm (s, IF); ES-MS: m/z: 686.8 $[M-H]^-$.

Example 8

**Compound 41**

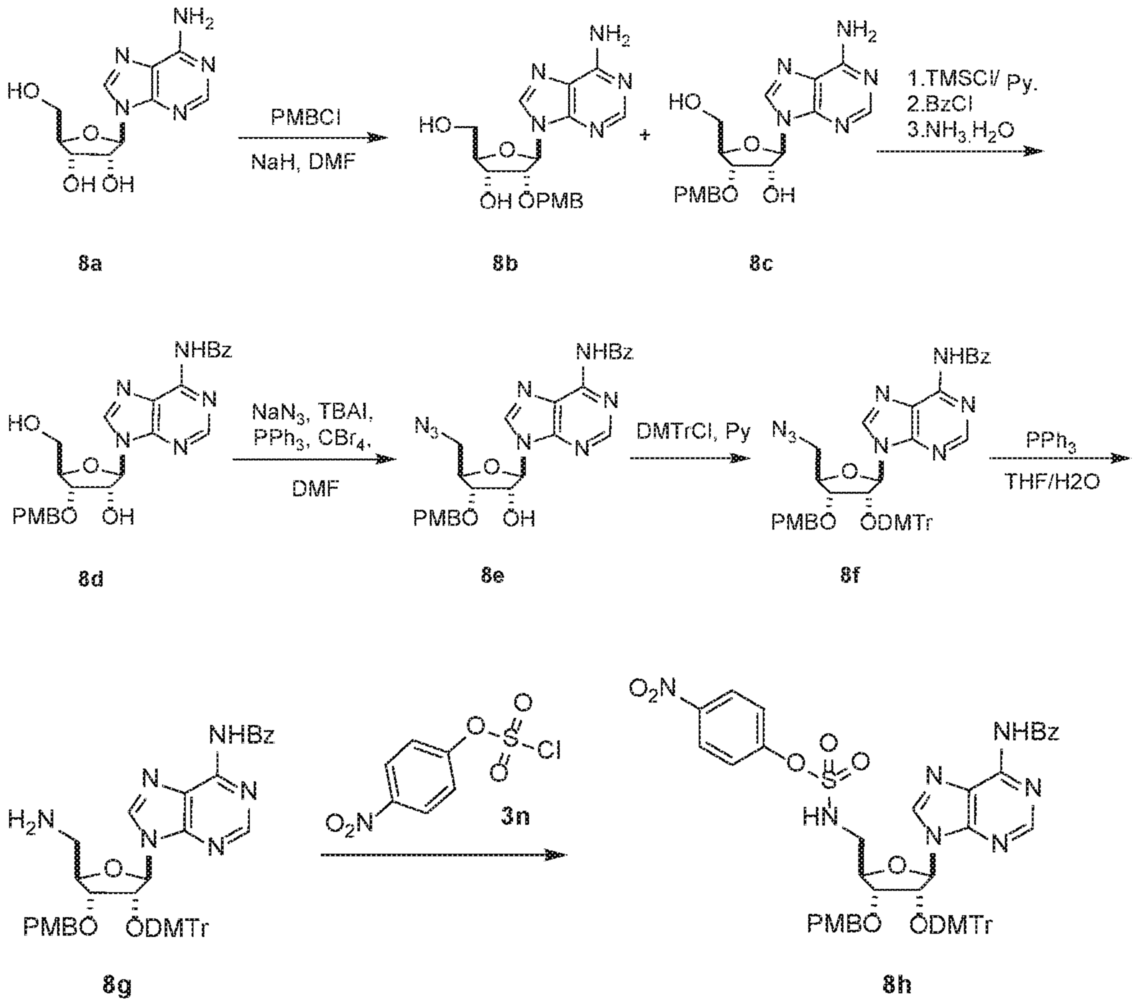


8a 8b 8c

8d 8e 8f

8g 8h

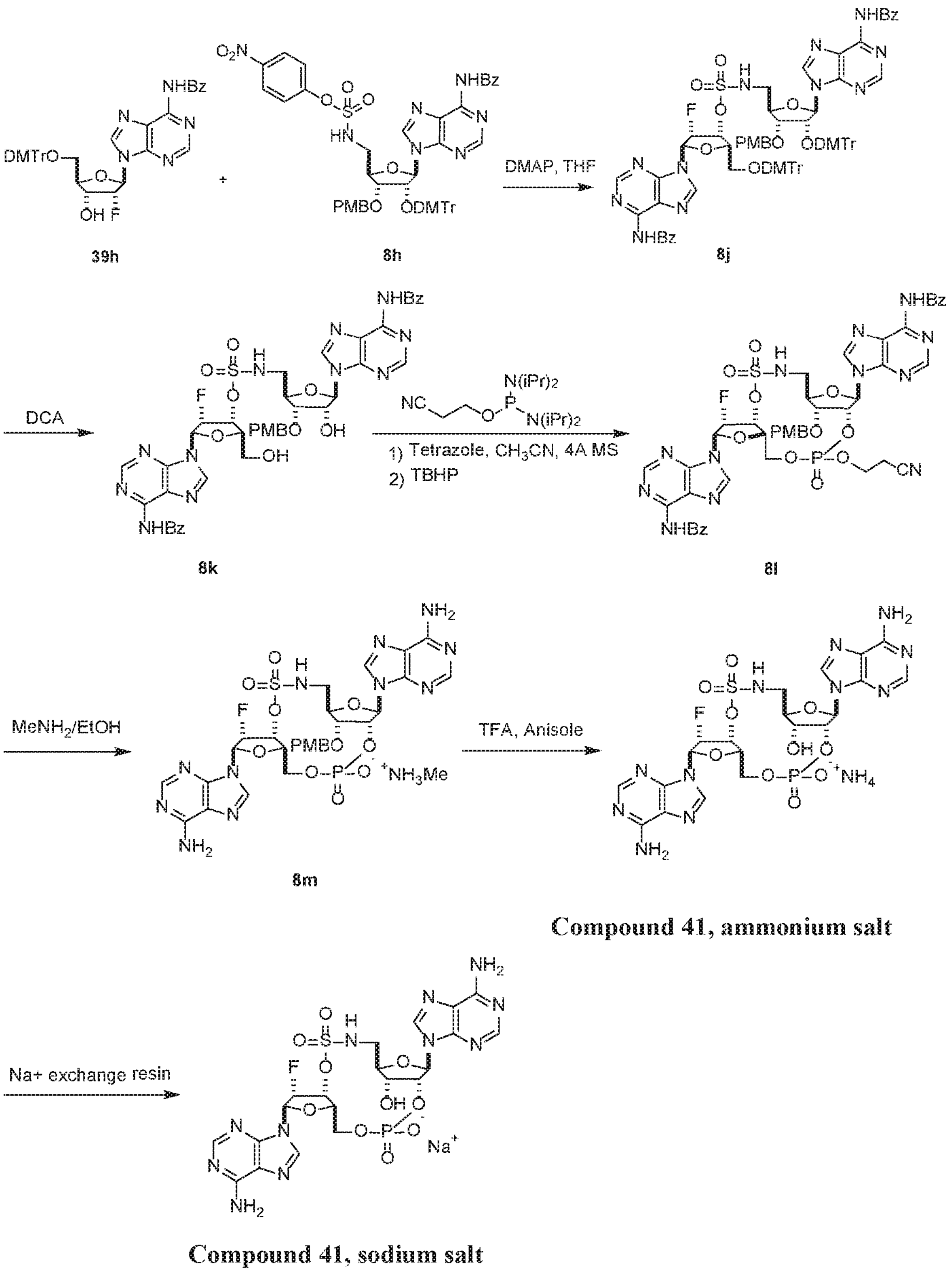


39h

8h

8j

8k

8l

8m

Compound 41, ammonium salt

Compound 41, sodium salt

Step 1: preparation of compounds **8b** and **8c**

NaH (60% in mineral oil, 4.864g, 121.61 mmol) was added to a solution of adenosine **8a** (25 g, 93.549 mmol) in DMF (800 mL) at -5 °C. After stirring the mixture for 1.5 h at -5°C, a solution of 4-methoxybenzyl chloride (15.15 g, 112.26 mmol) in DMF (50 mL) was added dropwise over 2h. After addition was complete, the reaction was warmed up to RT and stirred for 18 h. Water (15 mL) was added to the reaction and the mixture was stirred at 15°C for 10 min. The resulting mixture was combined with other reactions and DMF was evaporated under high vacuum. The suspension was diluted with MeOH, filtered and the filtrate concentrated under reduced pressure to give a residue. The residue was

purified by flash column chromatography on silica gel (DCM : MeOH = 100 : 0 ~ 20:1) to give **8b** and **8c;** the mixture was separated by reverse phase preparative HPLC (Column: Phenomenex Synergi Max-RP 250 × 50mm × 10 μm; Condition: Water-MeCN Begin B 2% End B 36%; Gradient Time: 18 min; 100%B Hold Time: 14 min; Flow Rate: 100 mL/min) to give **8b** (75 g) and **8c** (15.5 g), both as white solids (66% overall yield).

Compound **8b.** $^1$H NMR (400 MHz, DMSO-$d_6$) δ 8.31 (s, 1H), 8.09 (s, 1H), 7.36 (s, 2H), 7.06 (d, J = 8.4 Hz, 2H), 6.72 (d, J = 8.8 Hz, 2H), 6.03 (d, J = 6.4 Hz, 1H), 5.51 (dd, J = 4.4, 7.2 Hz, 1H), 5.32 (d, J = 4.8 Hz, 1H), 4.71 - 4.46 (m, 2H), 4.43 - 4.30 (m, 2H), 4.03 (br d, J = 2.8 Hz, 1H), 3.74 - 3.61 (m, 4H), 3.61 - 3.49 (m, 1H); ESI-MS: m/z = 388.1 [M+H]$^+$.

Compound 8c: $^1$H NMR (400 MHz, DMSO-$d_6$) δ 8.35 (s, 1H), 8.13 (s, 1H), 7.49 - 7.30 (m, 4H), 6.93 (d, J = 8.4 Hz, 2H), 5.92 (d, J = 6.4 Hz, 1H), 5.57 (d, J = 6.4 Hz, 1H), 5.51 (dd, J = 4.4, 7.3 Hz, 1H), 5.64 - 5.40 (m, 1H), 5.56 - 5.37 (m, 1H), 4.89 - 4.73 (m, 1H), 4.72 - 4.62 (m, 1H), 4.62 - 4.49 (m, 1H), 4.60 - 4.48 (m, 1H), 4.15 - 3.98 (m, 2H), 3.75 (s, 3H), 3.70 - 3.60 (m, 1H), 3.53 (ddd, J = 3.6, 7.6, 11.8 Hz, 1H); ESI-MS: m/z = 388.1 [M+H]$^+$

Step 2: preparation of compound **8d**

To a solution of compound **8c** (10 g, 25.81 mmol) in pyridine (200 mL) was added chlorotrimethylsilane (14.74 mL, 116.162 mmol) at 0 °C dropwise. After stirring the reaction mixture for 2h, it was cooled to 0 °C and benzoyl chloride (6 mL) was added dropwise at 0 °C for 30 min. The reaction mixture was stirred at RT overnight and quenched with water (30 mL) carefully at 0 °C, aqueous ammonia (60 mL) was then added dropwise at 0 °C. After stirring the mixture for 1.5 h, the reaction was diluted with DCM (800 mL). The organic layer was successively washed with brine (200 mL × 3), dried over anhydrous $Na_2SO_4$, filtered and concentrated under reduced pressure to give a residue. The residue was triturated from EtOAc (100 mL) to afford compound **8d** (9.8 g) as white solid. $^1$H NMR (400 MHz, DMSO-$d_6$) δ 11.21 (s, 1H), 8.72 (d, J = 13.2 Hz, 2H), 8.06 - 7.89 (m, 2H), 7.71 - 7.58 (m, 1H), 7.57 - 7.46 (m, 2H), 7.31 (d, J = 8.8 Hz, 2H), 6.90 (d, J = 8.8 Hz, 2H), 6.05 (d, J = 6.0 Hz, 1H), 5.67 (d, J = 6.4 Hz, 1H), 5.18 (t, J = 5.6 Hz, 1H), 4.91 - 4.76 (m, 1H), 4.73 - 4.59 (m, 1H), 4.53 (d, J = 11.6 Hz, 1H), 4.17 - 4.04 (m, 2H), 3.72 (s, 3H), 3.66 (td, J = 4.4, 12.0 Hz, 1H), 3.53 (ddd, J = 3.6, 6.0, 12.0 Hz, 1H); ESI-MS: m/z = 388.1 [M+H]$^+$

Step 3: preparation of compound **8e**

To a stirred suspension of compound **8d** (7 g, 14.24 mmol), triphenylphosphine (5.6 g, 21.36 mmol), TBAI (1.05 g, 2.85 mmol) and $NaN_3$ (6.67g, 102.54 mmol) in DMF (90 mL) was added $CBr_4$ (7.085 g, 21.363 mmol) portion-wise at 0°C resulting in a yellow suspension. After stirring ON at 35°C, the mixture was poured into aqueous saturated $NaHCO_3$ solution (500 mL) under stirring condition. The mixture was extracted with DCM (200 mLx3). Organic layers were then combined and successively dried over anhydrous $Na_2SO_4$, filtered and concentrated under reduced pressure to give a residue. The residue was purified by flash column chromatography on silica gel (gradient elution: 0 - 2% MeOH in DCM) to give **8e** (6.4 g) as white solid. ESI-MS: m/z = 517.2 [M+H]$^+$

Step 4: preparation of compound **8f**

To a solution of **8e** (6.0 g, 11.61 mmol, co-evaporated with pyridine twice before use) in pyridine (100 mL) was added DMTrCl (7.87 g, 23.23 mmol). After stirring the reaction mixture at 80 °C overnight, it was diluted with EA (800 mL) and washed with saturated $NaHCO_3$ (200 mLx2) and brine (200 mLx2). Organic layer was dried over anhydrous $Na_2SO_4$, filtered and concentrated under reduced pressure to give a residue. The residue was purified by flash column chromatography on silica gel (Petroleum Ether / EtOAc) to give **8f** as a yellow solid (6.8 g).

$^1$H NMR (400 MHz, DMSO-$d_6$) δ 11.21 (s, 1H), 8.64 (s, 1H), 8.55 (s, 1H), 8.58 - 8.45 (m, 1H), 8.04 (d, J = 7.2 Hz, 2H), 7.74 - 7.59 (m, 1H), 7.58 - 7.47 (m, 2H), 7.28 (d, J = 8.8 Hz, 2H), 7.22 (dd, J = 2.8, 6.8 Hz, 2H), 7.15 - 7.07 (m, 5H), 6.98 (d, J = 8.8 Hz, 2H), 6.91 (d, J = 8.8 Hz, 2H), 6.72 (d, J = 8.8 Hz, 2H), 6.60 (d, J = 8.8 Hz, 2H), 6.27 (d, J = 7.6 Hz, 1H), 5.24 (dd, J = 4.4, 7.6 Hz, 1H), 4.30 (d, J = 10.4 Hz, 1H), 4.22 (dd, J = 4.8, 7.8 Hz, 1H), 4.05 - 3.99 (m, 1H), 3.80 - 3.58 (m, 10H), 3.21 (dd, J = 4. 8, 12.8 Hz, 1H), 2.56 (d, J = 4.4 Hz, 1H); ESI-MS: m/z = 819.4 [M+H]$^+$.

Step 5: preparation of compound **8g**

To a mixture of **8f** (6.8 g, 8.3 mmol) in THF (80 mL) was added $PPh_3$ (3.27 g, 12.46 mmol) in one portion; the mixture was stirred at 40°C for 2 hr under $N_2$, followed by addition of water (30 mL), then further stirred for another 12 hr resulting in a colorless solution. The mixture was combined with another scale-up. Most of the volatile was removed under reduced pressure and the residual aqueous layer was partitioned between DCM/water. The layer was collected and extracted with DCM (200 mLx3). Organic layers were then combined, dried over anhydrous $Na_2SO_4$, filtered and concentrated under reduced pressure to give a residue. The residue was purified by flash column chromatography on silica gel (gradient elution: 0-5% MeOH in DCM) to give **8g** (6.4 g, 89% overall yield) as a white solid. $^1$H NMR (400

MHz, DMSO-$d_6$) $\delta$ ppm 8.74 - 8.65 (m, 1H), 8.54 (s, 1H), 8.13 - 8.01 (m, 2H), 7.74 - 7.63 (m, 1H), 7.62 - 7.51 (m, 2H), 7.31 (d, J = 8.8 Hz, 2H), 7.24 (dd, J = 3.2, 6.4 Hz, 2H), 7.19 - 7.07 (m, 5H), 6.97 (dd, J = 8.8, 18.0 Hz, 4H), 6.73 (d, J = 8.8 Hz, 2H), 6.62 (d, J = 9.2 Hz, 2H), 6.25 (d, J = 7.6 Hz, 1H), 5.16 (dd, J = 4.4, 7.6 Hz, 1H), 4.30 (d, J = 10.8 Hz, 1H), 4.16 - 4.04 (m, 2H), 4.01 - 3.95 (m, 1H), 3.84 - 3.59 (m, 9H), 2.72 (d, J = 4.8 Hz, 1H), 2.67 - 2.58 (m, 1H), 2.67 - 2.58 (m, 1H); ESI-MS: m/z = 794.4 $[M+H]^+$.

Step 6: preparation of compound **8h**

A solution of 4-nitrophenyl chlorosulfate (5.57 g, 23.46 mmol) in dry DCM (5 mL) was added rapidly to a mixture of **8g** (6.2 g, 7.82 mmol), 4-nitrophenol (3.26 g, 23.46 mmol), $Et_3N$ (4.75 g, 46.92 mmol) in dry DCM (20 mL) under $N_2$ at -78 °C, then warmed to room temperature over 1.5 hr. The mixture was transferred into a separatory funnel, washed with aqueous saturated $NaHCO_3$ (200 mL × 4). Organic layer was dried over anhydrous $Na_2SO_4$, filtered and concentrated under reduced pressure to give a residue. The residue was purified by flash column chromatography on silica gel (gradient elution: 0 -100% EtOAc in Petroleum ether) to give **8h** (6.2 g) as a white solid. $^1H$ NMR (400 MHz, DMSO-$d_6$) $\delta$ 11.32 (br s, 1H), 9.29 (br t, J = 5.6 Hz, 1H), 8.72 (s, 1H), 8.33 - 8.24 (m, 2H), 8.13 (s, 1H), 8.07 (d, J = 7.2 Hz, 2H), 7.72 - 7.63 (m, 1H), 7.60 - 7.46 (m, 4H), 7.31 (d, J = 8.4 Hz, 2H), 7.27 - 7.19 (m, 2H), 7.18 - 7.06 (m, 5H), 6.95 (d, J = 8.0 Hz, 4H), 6.73 (d, J = 9.2 Hz, 2H), 6.60 (d, J = 9.2 Hz, 2H), 6.30 (d, J = 7.8 Hz, 1H), 5.11 (dd, J = 4.8, 7.8 Hz, 1H), 4.39 - 4.22 (m, 2H), 4.10 - 3.93 (m, 2H), 3.83 - 3.59 (m, 10H), 2.84 (d, J = 4.8 Hz, 1H); ESI-MS: m/z = 994.2 $[M+H]^+$.

Step 7: preparation of compound **8j**

A suspension of **39h** (1.3 g, 1.92 mmol), **8h** (2.48 g, 2.5 mmol) and molecular sieves (1.5 g) in dry THF (20 mL) was stirred under $N_2$ for 30 min at room temperature, followed by addition of DMAP (0.94 g, 7.7 mmol). After stirring overnight at 45 °C under $N_2$, the reaction mixture was filtered through a pad of diatomaceous earth and the filtrate was concentrated under reduced pressure to give a yellow residue; it was dissolved in DCM (300 mL) and washed with aqueous saturated $NaHCO_3$ (100 mL x 3). Organic layer was dried over anhydrous $Na_2SO_4$, filtered and concentrated under reduced pressure to give a residue. The residue was purified by flash column chromatography on silica gel (gradient elution: 0 -70% EtOAc in PE) to give **8j** (2.7 g) as white solid. ESI-MS: m/z = 766.2 $[M+H]^+$

Step 8: preparation of compound **8k**

To a mixture of **8j** (2.7 g, 1.76 mmol) in DCM (80 mL) was added water (318 mg, 17.64 mmol) and DCA (335 mg, 4.06 mmol) resulting in a yellow solution. After stirring the mixture at RT for 1.5 hours, it was added MeOH (2 mL), followed by addition of pyridine (558.1 mg) resulting in colorless solution, which was further stirred for 15 min. The solvent was concentrated under reduced pressure and the residue was purified by flash column chromatography on silica gel (gradient elution: 0 - 2% MeOH in DCM) to give compound **8k** (1.28 g) as a white solid. $^1H$ NMR (400 MHz, DMSO-$d_6$) $\delta$ 11.26 (br d, J = 10.8 Hz, 2H), 9.02 - 8.50 (m, 5H), 8.05 (d, J = 7.6 Hz, 4H), 7.79 - 7.62 (m, 2H), 7.61 - 7.48 (m, 4H), 7.36 (d, J = 8.4 Hz, 2H), 6.93 (d, J = 8.4 Hz, 2H), 6.50 (dd, J = 2.4, 16.8 Hz, 1H), 6.09 (d, J = 5.6 Hz, 1H), 5.99 - 5.83 (m, 1H), 5.79 (br d, J = 6.4 Hz, 1H), 5.53 - 5.27 (m, 2H), 5.11 - 4.93 (m, 1H), 4.79 - 4.53 (m, 2H), 4.42 - 4.29 (m, 1H), 4.27 - 4.03 (m, 2H), 3.89 - 3.71 (m, 4H), 3.68 - 3.56 (m, 1H), 3.55 - 3.38 (m, 2H); $^{19}F$ NMR (376MHz, DMSO-$d_6$) = -202.67 (s, IF); ESI-MS: m/z = 926.3 $[M+H]^+$

Step 9: preparation of compound **8l**

THF was freshly distilled over sodium/benzophenone and $CH_3CN$ was freshly distilled over $CaH_2$ before use.

To a solution of **8k** (200 mg, 0.21 mmol, dried by lyophilization ) in THF (6mL), was added 4Å Molecular Sieves (800 mg, powder) and a solution of 1H-tetrazole (4.8 mL, 0.45 M, 945 mg of tetrazole (dried by lyophilization) in 30 mL of dry $CH_3CN$, followed by addition of 4Å MS (1 g, powder), stirred for 1 hr under $N_2$ prior use). After purging the flask with $N_2$. A solution of 2-cyanoethyl-*N,N,N',N'*-tetra(isopropyl)phosphorodiamidite (117 mg, 0.39 mmol) in THF (0.8 mL) was added drop-wise over 25 min vial a syringe; after stirring the reaction mixture at RT for 1.5 hr, a solution of TBHP (0.34 mL, 1.73 mmol, 5M). after stirring the mixture for an additional 30 min, the reaction mixture was concentrated under reduced pressure and the residue was purified by flash column chromatography on silica gel (gradient elution: 0-5% DCM in MeOH) to give 81 as a white solid (153 mg, 0.14 mmol). $^1H$ NMR (400 MHz, $CDCl_3$) $\delta$ 9.85 (br s, 1H), 9.79 - 9.72 (m, 1H), 8.89 (s, 1H), 8.87 - 8.84 (m, 1H), 8.64 - 8.59 (m, 1H), 8.20 - 8.14 (m, 1H), 8.10 - 8.04 (m, 4H), 8.02 - 7.95 (m, 3H), 7.65 - 7.56 (m, 3H), 7.55 - 7.45 (m, 6H), 6.05 - 5.94 (m, 1H), 4.81 - 4.71 (m, 2H), 4.66 (br d, J = 11.6 Hz, 1H), 4.52 - 4.47 (m, 1H), 4.36 - 4.21 (m, 4H), 4.01 (br dd, J = 4.4, 8.8 Hz, 1H), 3.92 - 3.80 (m, 6H), 2.67 (t, J = 6.0 Hz, 1H), 2.43 - 2.28 (m, 2H); $^{19}F$ NMR (376 MHz, $CDCl_3$) $\delta$-196.54 - -197.10 (m, 1F); $^{31}P$ NMR (162 MHz, $CDCl_3$) $\delta$-2.49 (s, IP), -4.38 (s, IP); ESI-MS: m/z = 780.2 $[M/2+H]^+$

Step 10: preparation of compound **8m**

A solution of **8l** (153 mg, 0.14 mmol) in $MeNH_2$/EtOH (5 mL) was stirred at RT for 2 hr. The volatile was evaporated under reduced pressure to give a white solid dissolved in water (20 mL); the aqueous layer was washed with DCM (10 mL × 3). The aqueous layer was lyophilized to give 114 mg of 8m as a white solid.

Step 11: preparation of **compound 41, ammonium salt**

A solution of anisole (158 mg, 1.46 mmol) in TFA (1.57 mL, 20.47 mmol) was cooled down to 0 °C and added to **8m** (114 mg). After stirring the reaction mixture at 0 °C for 2.5 hr, the TFA was removed by blowing with a flow of nitrogen gas at 0 °C. The remaining of the reaction mixture was quenched with $MeNH_2$ (33% solution in EtOH, 1.6 mL) at 0°C and evaporated to dryness under reduced pressure. The residue was partitioned between DCM/water (15 mL × 3 / 15 mL). The aqueous layer was lyophilized to give a white residue further purified by reverse phase preparative HPLC (column: Xbridge 150×30mm×10 μm, Condition: A: water (10 mM $NH_4HCO_3$)-ACN: MeCN, beginning: B 5%, End B: 35%; Flow Rate (mL/min) 25.) to afford **compound 41 ammonium salt** as a white solid (45 mg). $^1$H NMR (400 MHz, DMSO-$d_6$) $\delta$ ppm 10.63 (br s, 1H), 8.44 - 8.37 (m, 1H), 8.25 (br s, 1H), 8.04 (br s, 2H), 7.38 - 6.89 (m, 2H), 6.41 (br d, J =19.6 Hz, 1H), 6.13(d, J = 8.4 Hz, 1H), 6.01 - 5.83 (m, 1H), 5.60 (br s, 1H), 5.31 (br s, 1H), 4.45 (br d, J = 5.2 Hz, 1H), 4.28 (br s, 2H), 4.12 - 4.04 (m, 1H), 3.99 (br d, J = 11.2Hz, 1H); $^{19}$F NMR (376 MHz, DMSO-$d_6$) $\delta$ -198.36 - -200.33 (m, IF); $^{31}$P NMR (162 MHz, DMSO-$d_6$) $\delta$ ppm -3.40 (br s, IP); ESI-MS: m/z = 659.9 $[M+H]^+$

Step 12: preparation of **compound 41, sodium salt**

Dowex 50W × 8, 200-400 (9 mL, H form) was added to a beaker (for 45mg of **compound 41 ammonium salt)** and washed with deionized water (50 mL). Then to the resin was added 15% $H_2SO_4$ in deionized water, the mixture was gently stirred for 5 min, and decanted (50 mL). The resin was transferred to a column with 15% $H_2SO_4$ in deionized water and washed with 15% $H_2SO_4$ (at least 4 CV [Column Volume]), and then with deionized water until it was neutral. The resin was transferred back into the beaker, 15% NaOH in deionized water solution (20 mL) was added, and mixture was gently stirred for 5 min, and decanted (1 ×). The resin was transferred to the column and washed with 15% NaOH in water (at least 4 CV), and then with deionized WATER until it was neutral. **compound 41 ammonium salt** (45 mg) was dissolved in minimum amount of deionized water, added to the top of the column, and eluted with deionized water. Desired fractions were pooled together and lyophilized to give **compound 41, sodium salt** (31.4 mg) as a white solid. $^1$H NMR ($D_2O$, 400 MHz) $\delta$ 8.06 (s, 1H), 8.00 (s, 1H), 7.62-7.87 (m, 1H), 7.28 (br s, 1H), 6.24 (br d, J = 12.8 Hz, 1H), 6.03 (br d, J = 8.0 Hz, 1H), 5.49-5.74 (m, 1H), 5.22-5.31 (m, 1H), 5.11 (br d, J = 16.4 Hz, 1H), 4.53 (br s, 1H), 4.44 (br d, J = 4.4 Hz, 1H), 4.39 (brs, 1H), 4.34 (br d, J = 10.4 Hz, 1H), 3.98 (br d, J = 8.8 Hz, 1H), 3.71-3.79 (m, 1H), 3.60-3.70 (m, 1H); $^{19}$F NMR (376 MHz, $D_2O$) $\delta$-201.23 (br s, IF), $^{31}$P NMR (162 MHz, $D_2O$) $\delta$-2.76 (br s, IP); ESI-MS: m/z = 660.1 $[M+H]^+$

Example 9

## Compound 8

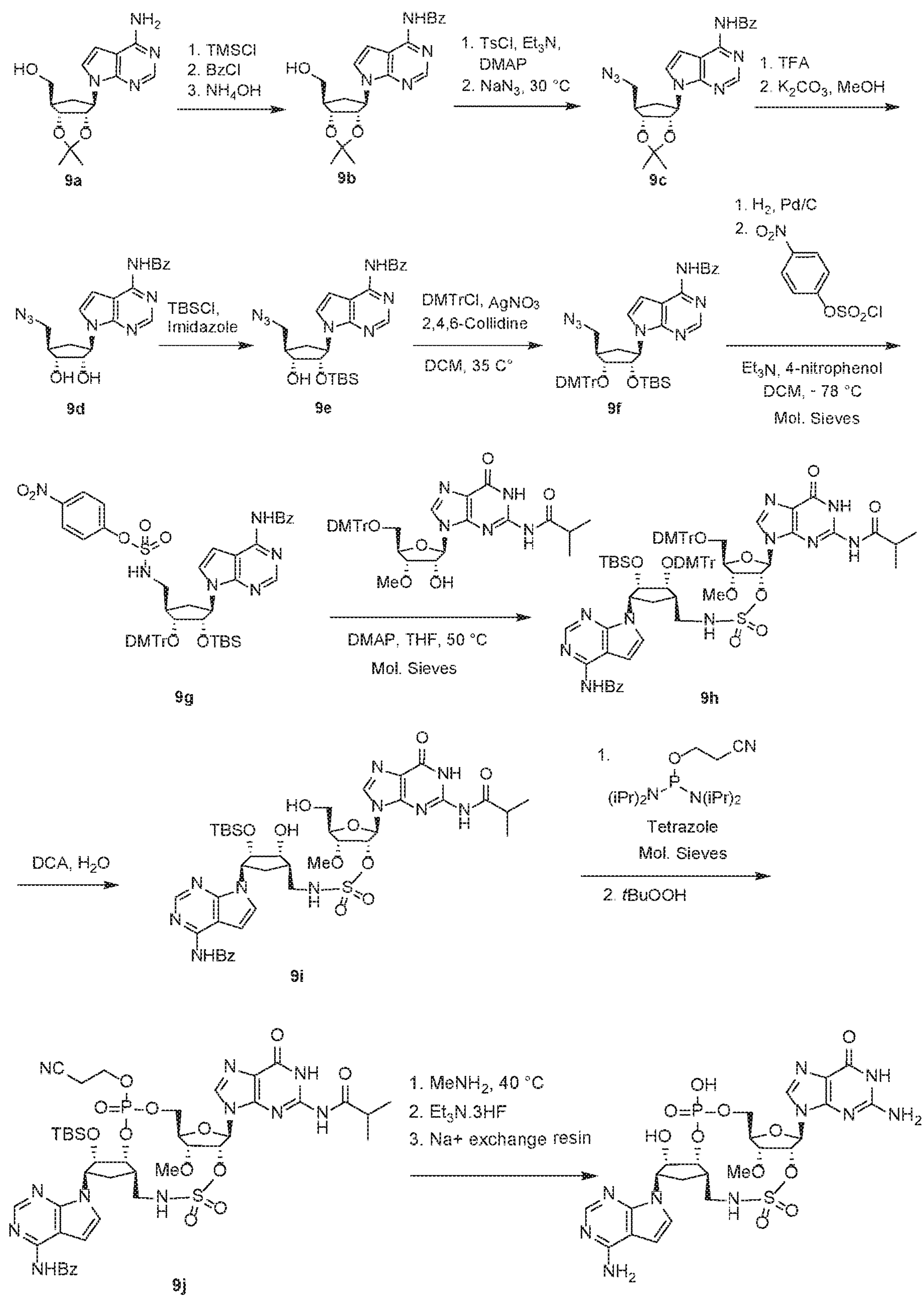


Compound 8, sodium salt

Step 1: Preparation of compound **9b**

A solution of compound **9a** (8.3 g, 27.27 mmol, CAS# 2086765-82-2) in dry pyridine (160 mL) at 0 °C to which chlorotrimethylsilane (14.8 g, 136.36 mmol) was added dropwise, was stirred at 0 °C for 1.5 h. Benzoyl chloride (19.2 g, 136.36 mmol) was added dropwise and stirring was continued at room temperature for 3 h. The reaction solution, which was cooled in an ice-bath, was quenched by the addition of water (50 mL) and stirred overnight at room temperature. Aqueous ammonia (70 mL of 25% solution) was added and stirring was continued for an extra hour. The reaction solution was extracted with EtOAc, the combined organic layers were washed with brine, dried with $Na_2SO_4$, filtered and concentrated under reduced pressure. Purification was done by column chromatography over silica gel (gradient elution: 0 - 90% EtOAc in petroleum ether) to give compound **9b** as a yellow solid (9.1 g, yield: 80%). $^1$H NMR (400 MHz, DMSO-$d_6$) δ ppm 11.11 (s, 1H), 8.60 (s, 1H), 8.07 (d, *J*=7.3 Hz, 2H), 7.69 (d, *J*=3.8 Hz, 1H), 7.66 - 7.61 (m, 1H), 7.57 - 7.51 (m, 2H), 6.68 (d, *J*=3.5 Hz, 1H), 5.13 (td, *J*=6.3, 12.0 Hz, 1H), 4.92 (t, *J*=6.8 Hz, 1H), 4.80 (t, *J*=5.3 Hz, 1H), 4.55 (dd, J=4.4,7.2 Hz, 1H), 3.59 - 3.46 (m, 2H), 2.32 - 2.20 (m, 2H), 2.16 - 2.03 (m, 1H), 1.49 (s, 3H), 1.23 (s, 3H);
ESI-MS: m/*z* 409.1 $[M+H]^+$.

Step 2: Preparation of compound **9c**

DMAP (448 mg, 3.76 mmol) and tosyl chloride (2.80 g, 14.69 mmol) were added to a solution of compound **9b** (3.0 g, 7.35 mmol) and $Et_3N$ (2.33 g, 22.04 mmol) in DCM (60 mL) under ice cooling. The reaction mixture was stirred at room temperature for 4 h. The mixture was quenched with water and extracted with DCM. The organic layer was washed with brine, dried with anhydrous $Na_2SO_4$ and evaporated under reduced pressure to give the tosylated compound. The crude product was dissolved in DMF (30 mL), sodium azide (2.34 g, 36.00 mmol) was added and the resulting reaction mixture was stirred at 30 °C overnight The reaction solution was cooled to room temperature, diluted with saturated aqueous $Na_2CO_3$, and extracted with EtOAc. The organic layer was separated, washed with brine, dried with anhydrous $Na_2SO_4$ and evaporated under reduced pressure. The crude product was purified by silica column chromatography (gradient elution: 0 - 40% EtOAc in petroleum ether) to give compound **9c** as a yellow solid (2.1 g, yield: 62%). $^1$H NMR (400 MHz, DMSO-$d_6$) δ ppm 11.13 (s, 1H), 8.60 (s, 1H), 8.10 - 8.03 (m, 2H), 7.69 (d, *J*=3.8 Hz, 1H), 7.67 - 7.61 (m, 1H), 7.57 - 7.51 (m, 2H), 6.69 (d, *J*=3.8 Hz, 1H), 5.16 (td, *J*=6.3, 12.0 Hz, 1H), 4.99 - 4.89 (m, 1H), 4.55 (dd, *J*=5.1, 7.2 Hz, 1H), 3.63 - 3.46 (m, 2H), 2.41 - 2.27 (m, 2H), 2.20 - 2.07 (m, 1H), 1.50 (s, 3H), 1.24 (s, 3H); ESI-MS: *m*/*z* 434.1 $[M+H]^+$.

Step 3: Preparation of compound **9d**

TFA (40 mL) was added to a solution of compound **9c** (5.4 g, 12.46 mmol) in DCM (100 mL). The reaction mixture was stirred at room temperature overnight. The residue obtained after concentration under reduced pressure was re-dissolved in MeOH (20 mL) followed by the addition of saturated aqueous $K_2CO_3$ (40 mL). The resulting mixture was stirred for 20 min after which it was extracted with DCM The combined organic layers were washed with brine, dried with anhydrous $Na_2SO_4$ and evaporated under reduced pressure to give compound **9d** which was used as such in the next step. $^1$H NMR (400 MHz, DMSO-$d_6$) δ ppm 11.08 (s, 1H), 8.57 (s, 1H), 8.07 (br d, *J*=7.5 Hz, 2H), 7.67 - 7.61 (m, 2H), 7.57 - 7.51 (m, 2H), 6.66 (d, *J*=3.5 Hz, 1H), 5.10 - 5.01 (m, 1H), 4.97 (d, *J*=6.5 Hz, 1H), 4.89 (d, *J*=4.8 Hz, 1H), 4.27 (td, *J*=6.1, 8.1 Hz, 1H), 3.84 - 3.80 (m, 1H), 3.60 - 3.48 (m, 2H), 2.34 - 2.25 (m, 1H), 2.23 - 2.12 (m, 1H), 1.67 - 1.55 (m, 1H); ESI-MS: *m*/*z* 394.0 $[M+H]^+$.

Step 4: Preparation of compound **9e**

The above compound **9d** was dissolved in DCM (90 mL), followed by the addition of imidazole (2.42 g, 35.52 mmol) and TBSCl (2.68 g, 17.76 mmol). The reaction mixture was stirred at room temperature overnight after which it was poured into water and extracted with DCM. The combined organic layers were washed with brine, dried with anhydrous $Na_2SO_4$, filtered and concentrated under reduced pressure to give **9e** and its 3'-regioisomer. Separation was done by preparative reversed phase HPLC (Stationary phase: Phenomenex Synergi, 10 μm Max-RP, 250 × 50 mm; Mobile phase: WATER (A) - MeCN (B); gradient elution) to give compound **9e** (2.3 g, yield: 50% from **9c)** as the first eluting isomer. $^1$H NMR (400 MHz, DMSO-$d_6$) δ ppm 11.05 (br s, 1 H), 8.56 (s, 1 H), 8.07 (d, *J*=7.8 Hz, 2 H), 7.60 - 7.68 (m, 2 H), 7.47 - 7.59 (m, 2 H), 6.68 (d, *J*=3.5 Hz, 1H), 5.10 - 5.23 (m, 1 H), 4.63 (d, *J*=5.5 Hz, 1 H), 4.37 (dd, *J*=8.0, 5.8 Hz, 1 H), 3.74 - 3.84 (m, 1H), 3.48 - 3.62 (m, 2 H), 2.10 - 2.30 (m, 2 H), 1.66 - 1.85 (m, 1 H), 0.64 (s, 9 H), -0.16 (s, 3 H), -0.37 (s, 3 H); ESI-MS: *m*/*z* 508.1 $[M+H]^+$.

Step 5: Preparation of compound **9f**

DMTrCl (3.87 g, 11.43 mmol), AgNO3 (4.85 g, 28.56 mmol) and 2,4,6-collidine (3.46 g, 28.56 mmol) were added to a solution of compound **9e** (2.9 g, 5.71 mmol) in DCM (50 mL), the resulting mixture was stirred at 35 °C overnight. The reaction solution was filtered and concentrated under reduced pressure. The crude product was purified by silica column chromatography (gradient elution: 0 - 20% EtOAc in petroleum ether), followed by purification by preparative reversed phase HPLC (Stationary phase: Phenomenex Synergi Max-RP, 10 μm, 250 × 50 mm, Mobile phase: 0.1% aqueous TFA (A) - MeCN (B); gradient elution) to give compound **9f** (3.6 g, yield, 78%).

$^1H$ NMR (400 MHz, DMSO-$d_6$) δ ppm 11.04 (s, 1 H), 8.59 (s, 1 H), 8.08 (d, J=7.4 Hz, 2 H), 7.66 (d, J=3.5 Hz, 1 H), 7.61 - 7.65 (m, 1 H), 7.55 (q, J=7.3 Hz, 4 H), 7.40 (t, J=8.6 Hz, 4 H), 7.27 - 7.35 (m, 2 H), 7.18 - 7.26 (m, 1H), 6.85 - 6.96 (m, 4 H), 6.68 (d, J=3.5 Hz, 1 H), 5.50 (q, J=9.1 Hz, 1 H), 4.42 (dd, J=9.1, 4.1 Hz, 1 H), 3.74 (s, 3 H), 3.73 (s, 3 H), 3.64 (br d, J=3.9 Hz, 1 H), 3.27 - 3.37 (m, 1 H), 3.20 (dd, J=12.3, 5.5 Hz, 1 H), 2.26 - 2.37 (m, 1 H), 1.71 - 1.84 (m, 1 H), 1.59 - 1.71 (m, 1 H), 0.60 - 0.78 (m, 9 H), -0.27 - -0.18 (m, 3 H), - 0.59 - -0.51 (m, 3 H); ESI-MS: m/z 810.1 $[M+H]^+$.

Step 6: Preparation of compound **9g**

A solution of compound **9f** (2.3 g, 2.84 mmol) in EtOAc (80 mL) was stirred under $H_2$ (50 psi) at 35 °C overnight in the presence of Pd/C (10% on carbon, 2.0 g). The catalyst was removed by filtration over Diatomaceous earth and the filter cake was washed with EtOAc. The filtrate was concentrated under reduced pressure to give the compound amine which was immediately used as such in the next step. The crude product was dissolved in DCM (90 mL), followed by the addition of 4-nitrophenol (2.81 g, 20.21 mmol), $Et_3N$ (1.23 g, 12.12 mmol) and activated molecular sieves. The resulting mixture was cooled to -78 °C under $N_2$ after which 4-nitrophenyl chlorosulfate (2.88 g, 12.12 mmol) in DCM (10 mL) was added dropwise, the reaction solution was allowed to warm to room temperature and stirred overnight. Molecular sieves were removed by filtration. The filtrate was washed with saturated aqueous $NaHCO_3$, dried with $Na_2SO_4$ and concentrated. The crude product was purified by silica column chromatography (gradient elution: 0 - 20% EtOAc in petroleum ether) to give compound **9g** (1.4 g, yield: 80% (LCUV purity: 76%)).

ESI-MS: *m*/*z* 986.6 $[M+H]^+$.

Step 7: Preparation of compound **9h**

A reaction flask was charged with DMAP (0.55 g, 4.47 mmol), dry THF (6 mL) and activated 3Å molecular sieves. The resulting mixture was shaken at room temperature under inert atmosphere for 2 h. Simultaneously, a solution of 5'-O-(4,4'-Dimethoxytrityl)-N2-isobutyryl-3'-O-methyl-D-guanosine [103285-33-2], (0.33 g, 0.46 mmol) and a solution of compound **9g** (1.16 g, 0.89 mmol), each in dry THF (2 × 6 mL), were dried on activated 3Å molecular sieves (ca. 2 h). Both solutions 5'-O-(4,4'-Dimethoxytrityl) -N2-isobutyryl-2'-O-methylguanosine and compound **9g** respectively) were successively transferred to the reaction flask. The reaction mixture was stirred at 50 °C for 18 h after which it was cooled to room temperature and diluted with DCM. The molecular sieves were removed by filtration and thoroughly rinsed with DCM. The filtrate was washed with saturated aqueous $NaHCO_3$ and brine, dried with $MgSO_4$, filtered, and concentrated under reduced pressure. The crude product was purified by silica column chromatography (gradient elution: 0 - 10% MeOH in DCM) to give compound **9h** (1.17 g, yield: 86%). ESI-MS: *m*/*z* 1515.9 $[M+H]^+$.

Step 8: Preparation of compound **9i**

A solution of compound **9h** (1.13 g, 0.75 mmol) in DCM (20 mL) was treated with DCA (250 μL, 2.98 mmol) in the presence of water (67 μL, 3.73 mmol) for 2.5 h. The reaction mixture was quenched by the addition of pyridine (360 μL, 4.47 mmol). The resulting solution residue was brought as such on a silica column for purification (gradient elution: 0 -10% MeOH in DCM) to give compound **9i** as a white solid (595 mg, yield: 87%).

$^1H$ NMR (400 MHz, CHLOROFORM-*d*) δ ppm 12.01 (br s, 1 H), 9.70 (br s, 1 H), 8.71 (br s, 1 H), 8.53 (s, 1 H), 8.02 - 8.07 (m, 2 H), 7.87 (s, 1 H), 7.57 - 7.63 (m, 1 H), 7.49 - 7.57 (m, 2 H), 7.13 (d, *J*=3.7 Hz, 1 H), 7.00 (d, *J*=3.6 Hz, 1 H), 6.12 (d, *J*=4.0 Hz, 1 H), 5.49 (t, *J*=4.5 Hz, 1 H), 4.74 - 4.83 (m, 2 H), 4.39 (br t, *J*=5.3 Hz, 1 H), 4.12 - 4.27 (m, 1 H), 4.04 - 4.10 (m, 1 H), 3.97 - 4.04 (m, 1 H), 3.74 - 3.82 (m, 1 H), 3.57 (s, 3 H), 3.37 - 3.46 (m, 1 H), 3.17 - 3.26 (m, 1 H), 2.65 (spt, *J*=7.0 Hz, 1 H), 2.45 - 2.54 (m, 1 H), 2.39 - 2.49 (m, 1 H), 1.96 - 2.03 (m, 1 H), 1.17 (d, *J*=6.6 Hz, 3 H), 1.18 (d, *J*=6.6 Hz, 3 H), 0.75 (s, 9 H), - 0.23 (s, 3 H), -0.44 (s, 3 H); ESI-MS: *m*/*z* 911.6 $[M+H]^+$.

Step 9: Preparation of compound **9j**

A solution of compound **9i** (530 mg, 0.58 mmol) in dry THF / MeCN (1:1, 108 mL, dried on activated 3Å molecular sieves before use) was treated with activated 3Å molecular sieves under an inert atmosphere (ca. 2 h shaking). 1*H*-tetrazole (5.0 mL of a 3 - 4% in MeCN, 1.73 mmol, dried on activated 3Å molecular sieves before use) was added and the mixture was shaken for 1 h at room temperature. Next, 2-cyanoethyl-*N,N,N',N'*-tetra(isopropyl)phosphorodiamidite (180 µL, 0.58 mmol) was added at once and shaking was continued for 18 h. Finally, two equal portions of *t*BuOOH (2 × 120 µL of a 5.5 M solution in decane, 2 × 0.63 mmol) were added with a time interval of 30 min after which the reaction mixture was shaken for an extra 2 h. Molecular sieves were removed by filtration and rinsed with DCM The filtrate was washed with a mixture of saturated aqueous $Na_2S_2O_3$ and saturated aqueous $NaHCO_3$, and brine, dried with $MgSO_4$, filtered and concentrated under reduced pressure. The crude product was purified by silica column chromatography (gradient elution: 0 -10% MeOH in DCM) to give compound **9j** (130 mg, yield: 21%). $^1$H NMR (400 MHz, CHLOROFORM-*d*) δ ppm -0.04 (br s, 3 H) 0.11 (s, 3 H) 0.85 (s, 9 H) 0.93 (br d, *J*=6.9 Hz, 3 H) 1.10 (d, *J*=6.9 Hz, 3 H) 2.42 - 2.52 (m, 1 H) 2.52 - 2.69 (m, 2 H) 2.89 (td, *J*=5.9, 2.8 Hz, 2 H) 2.93 (br d, *J*=5.7 Hz, 1 H) 3.37 - 3.50 (m, 1 H) 3.56 - 3.67 (m, 4 H) 4.35 (d, *J*=2.8 Hz, 1 H) 4.37 - 4.48 (m, 4 H) 4.53 (dt, *J*=5.3, 3.7 Hz, 1 H) 4.80 (br s, 1 H) 4.99 (ddd, *J*=11.0, 5.3, 3.7 Hz, 1 H) 5.27 - 5.32 (m, 1 H) 5.95 - 6.02 (m, 2 H) 7.03 (d, *J*=3.7 Hz, 1 H) 7.12 (d, *J*=3.7 Hz, 1 H) 7.52 - 7.58 (m, 2 H) 7.58 - 7.65 (m, 1 H) 7.59 - 7.67 (m, 1 H) 8.04 (s, 1 H) 8.12 (d, *J*=7.3 Hz, 2 H) 8.93 (br s, 1 H) 9.10 (br s, 1 H) 10.66 (br s, 1 H) 12.10 (br s, 1 H); $^{31}$P NMR (162 MHz, CHLOROFORM-*d*) δ ppm -3.52 (s, 1 P); ESI-MS: *m/z* 1026.6 $[M+H]^-$.

Step 10: Preparation of **compound 8, sodium salt**

Compound **9j** (118 mg, 0.11 mmol) was stirred in a 33% methylamine solution in ethanol (5.9 mL) at 40 °C until complete conversion (ca. 3 h), after which the reaction solution was cooled to room temperature and concentrated under reduced pressure. The residue was dissolved in pyridine (6 mL), followed by the addition of $Et_3N$ (460 µL, 3.31 mmol) and triethylamine trihydrofluoride (280 µL, 1.66 mmol). The reaction mixture was stirred at 45 °C for 18 h. Isopropoxytrimethylsilane (1.17 mL, 6.62 mmol) was added and stirring was continued at room temperature for 2 days. The residue obtained after concentration under reduced pressure was triturated in anhydrous acetonitrile, the obtained precipitate was further purified by preparative reversed phase HPLC (Stationary phase: RP XBridge C18 OBD, 10 µm, 150 × 50 mm, Mobile phase: 0.25% aqueous ammonia bicarbonate (A) - MeOH (B); gradient elution) to give **compound 8**. Final conversion into the sodium salt was done by elution of an aqueous solution over a column packed with a cationic sodium ion-exchange resin affording **compound 8, sodium salt** as a white fluffy solid (32 mg, yield: 41%). $^1$H NMR (400 MHz, DMSO-$d_6$) δ ppm 10.31 (br s, 1 H), 8.04 (s, 1 H), 7.94 (s, 1 H), 7.76 (br s, 1 H), 7.21 (d, *J*=3.3 Hz, 1 H), 6.74 (d, *J*=3.1 Hz, 1 H), 6.48 - 6.60 (m, 5 H), 6.02 (d, *J*=8.4 Hz, 1 H), 5.92 (dd, *J*=8.4, 4.3 Hz, 1 H), 4.80 - 4.93 (m, 1 H), 4.63 (br s, 1 H), 4.34 - 4.43 (m, 1 H), 4.30 (d, *J*=3.8 Hz, 2 H), 4.18 (dd, *J*=12.2, 3.6 Hz, 1 H), 4.08 (dd, *J*=12.5, 2.8 Hz, 1 H), 3.50 (s, 3 H), 3.16 (br d, *J*=6.1 Hz, 2 H), 2.25 - 2.37 (m, 2 H), 1.45 -1.57 (m, 1 H); $^{31}$P NMR(162 MHz, DMSO-$d_6$) δ ppm 1.42 (s, 1 P); ESI-MS: *m/z* 685.3 $[M+H]^+$.

Example 10

Compound 48

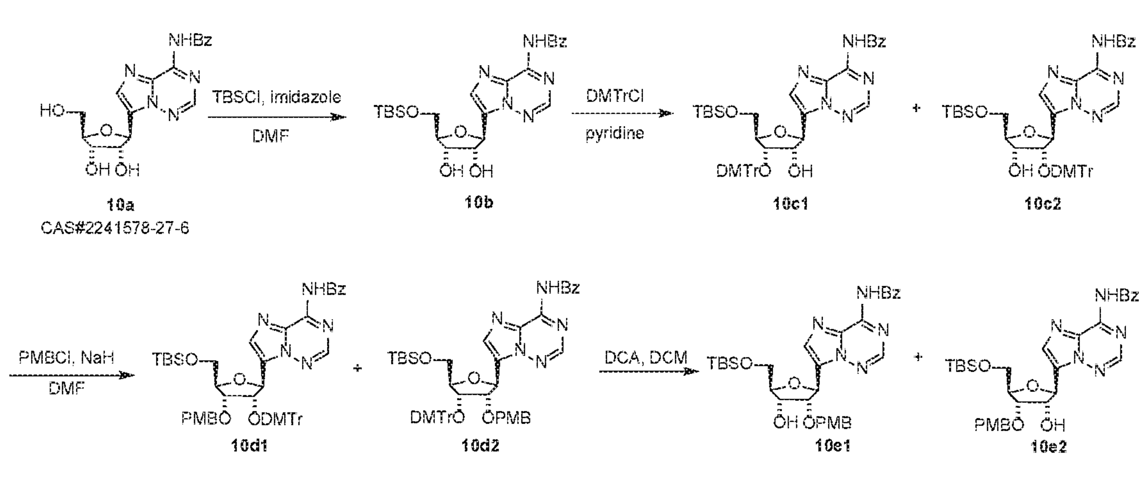

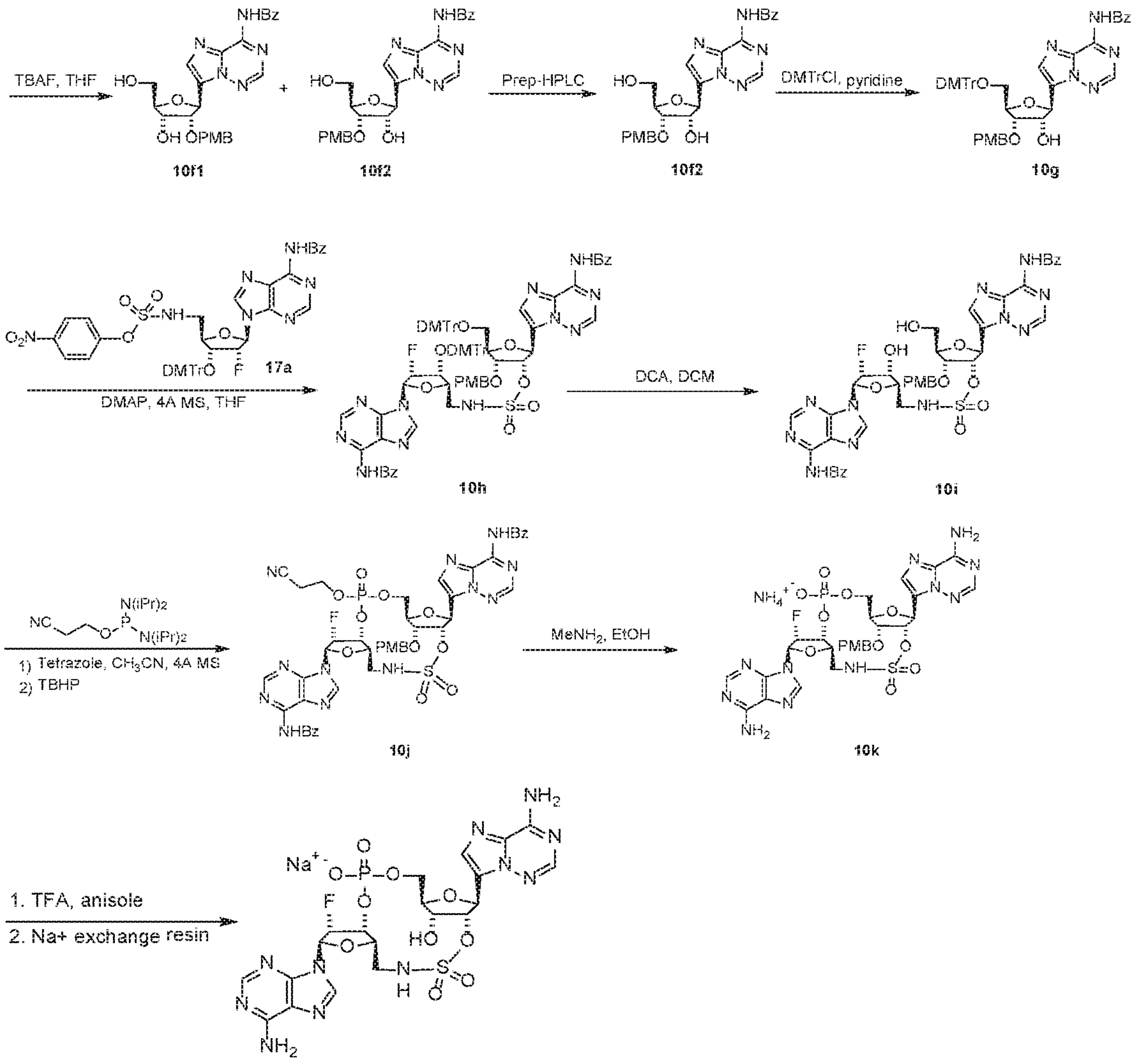


10f1 10f2 10f2 10g 17a 10h 10i 10j 10k

**Compound 48, sodium salt**

Step 1: preparation of compound **10b**

To a solution of compound **10a** (0.8 g, 2.15 mmol) in DMF (6 mL) was added imidazole (439.98 mg, 6.46 mmol) and TBSCl (616.94 mg, 4.09 mmol). After stirring at RT for 1.5h., the mixture was with another batch and diluted with EA (800 mL). Organic layer was successively washed with aqueous saturated $NaHCO_3$ (200 mL×2), brine (200 mLx2), dried over anhydrous $Na_2SO_4$, filtered and concentrated under reduced pressure to give a residue. The residue was purified by flash column chromatography on silica gel (eluted with 0-70% EA in PE) to give compound **10b** (5.5 g) as a white solid. 1H NMR (400 MHz, DMSO-$d_6$) δ 11.44 (br s, 1H), 8.66 (br s, 1H), 8.17 - 7.92 (m, 2H), 7.85 (br s, 1H), 7.69 - 7.59 (m, 1H), 7.57 - 7.33 (m, 2H), 5.44 - 5.09 (m, 2H), 5.02 (br d, *J*= 5.4 Hz, 1H), 4.30 (br s, 1H), 4.01 (br d, *J*= 4.0 Hz, 1H), 3.87 (br d, *J*= 3.6 Hz, 1H), 3.80 - 3.61 (m, 2H), 0.84 (s, 9H), 0.01 (d, *J*= 4.4 Hz, 6H); ESI-MS: m/z = 486.2 $[M+H]^+$.

Step 2: preparation of compound **10c1** and **10c2**

Compound **10b** was co-evaporated with pyridine (10 mL) twice prior use. To a solution of compound **10b** (1.7 g, 0.88 mmol) in pyridine (15 mL) was added DMTrCl (41.78 g, 5.25 mmol). After stirring at RT for 2 h., the mixture was combined with another batch and diluted with EtOAc (500 mL). Organic layer was successively washed with aqueous saturated $NaHCO_3$ (150 mLx2), brine (150 mLx2), dried over anhydrous $Na_2SO_4$, filtered and concentrated under reduced pressure to give a residue. The residue was purified by flash column chromatography on silica gel (eluted with 0-70% EtOAc in PE) to give mixture of **10c1** and **10c2** (3.9 g). ESI-MS: m/z = 788.4 $[M+H]^+$.

Step 3: preparation of compound **10d1** and **10d2**

To a solution of **10c1** and **10c2** (2.5 g, 3.17 mmol) in DMF (25 mL) was added NaH (60% in mineral oil, 482.3 mg, 12.05 mmol) at 0°C. The mixture was stirred for 1 hr at 0 °C and a solution of 4-methoxybenzyl chloride (745.31 mg, 4.76 mmol) in DMF (5 mL) was added dropwise over 10 min. After stirring at RT for 2 h, the mixture was quenched with WATER (5 mL) dropwise and diluted with EA (500 mL Organic layer was successively washed with aqueous saturated $NaHCO_3$ (150 mL×2), brine (150 mLx2), dried over anhydrous $Na_2SO_4$, filtered and concentrated under reduced pressure to give a residue. The residue was purified by flash column chromatography on silica gel (eluted with 0-40% EA in PE) to give mixture of **10d1** and **10d2** (1.9 g). ESI-MS: m/z = 908.4 $[M+H]^+$.

Step 3: preparation of compounds **10e1** and **10e2**

A solution of **10e1** and **10e2** (1.9 g, 2.09 mmol) in DCM (30 mL) was treated with water (0.38 mL, 20.92 mmol) and DCA (0.69 mL, 8.37 mmol). The resulting yellow solution was stirred at RT for 2 h. It was then added to MeOH (0.15 mL), followed by addition of pyridine (662 mg), resulting in a colorless solution, which was further stirred for 15 min. The mixture was evaporated under reduced pressure to give a residue purified by by flash column chromatography on silica gel to give a mixture of compounds **10e1** and **10e2** (1.05 g) as a yellow solid. ESI-MS: m/z = 606.1 $[M+H]^+$.

Step 4: preparation of compounds **10f1** and **10f2**

A mixture of compounds **10e1** and **10e2** (1.2 g, 1.98 mmol) in THF (12 mL) was treated with TBAF (3 mL, 1 M) in one portion. The mixture was stirred at RT for 3 h and quenched with aqueous saturated $NaHCO_3$ (150 mL). The organic layer was dried (anhydrous $Na_2SO_4$), filtered and evaporated under reduced pressure to give a residue, which was purified by flash column chromatography on silica gel (5% MeOH in DCM) to give a mixture of compounds **10f1** and **10f2.** The mixture of **10f1** and **10f2** was combined with another batch and triturated with MeOH; a precipitate formed out of MeOH; after filtration and washing with small amount of cold MeOH, it was identified as the major pure isomer compound **10f2** (659 mg); mother liquors were concentrated and purified by reverse phase preparative HPLC (Column: Waters Xbridge Prep OBD 10μm C18 150x30, Condition: A: water (10mM $NH_4HCO_3$)-ACN: MeCN, beginning: B 25%, End B: 45%; Flow Rate (mL/min) 25) to give more of compound **10f2** (97 mg) and the minor isomer compound **10f1** (200 mg) each one as a white solid.

Compound **10f1**: $^1H$ NMR (400 MHz, DMSO-$d_6$) δ 8.51 (s, 1H), 8.03 (br d, *J* = 8.0 Hz, 2H), 7.81 (s, 1H), 7.67 - 7.63 (m, 1H), 7.57 - 7.53 (m, 2H), 7.17 (d, *J*= 8.0 Hz, 2H), 6.80 (d, *J* = 8.0 Hz, 2H), 5.28 (d, *J* = 8.0 Hz, 1H), 5.09 (br d, *J* = 4.0 Hz, 1H), 4.83 (br t, *J*= 4.0 Hz, 1H), 4.64 (d, *J*= 12 Hz, 1H), 4.47 (d, *J* = 12 Hz, 1H), 4.28 (t, *J*= 4.0 Hz, 1H), 4.20 (q, *J*= 4.0 Hz, 1H), 3.91 (q, *J* = 4.0 Hz, 1H), 3.70 (s, 3H), 3.61 - 3.58 (m, 1H), 3.51 - 3.47 (m, 1H); ESI-MS: m/z = 492.2 $[M+H]^+$.

Compound **10f2**: $^1H$ NMR (400 MHz, DMSO-$d_6$) δ 8.61 (br s, 1H), 8.03 (br d, *J* = 8.0 Hz, 2H), 7.92 (br s, 1H), 7.69 - 7.65 (m, 1H), 7.58 - 7.54 (m, 2H), 7.33 (d, *J*= 8.0 Hz, 2H), 6.92 (d, *J* = 8.0 Hz, 2H), 5.33 (d, *J* = 8.0 Hz, 1H), 5.21 (br d, *J* = 8.0 Hz, 1H), 4.87 (t, *J* = 8.0 Hz, 1H), 4.66 (d, *J* = 8.0 Hz, 1H), 4.57 - 4.49 (m, 2H), 4.01 (q, *J* = 4.0 Hz, 1H), 3.95 (t, *J* = 4.0 Hz, 1H), 3.75 (s, 3H), 3.60 - 3.55 (m, 1H), 3.50 - 3.45 (m, 1H)
ESI-MS: m/z = 492.3 $[M+H]^+$.

Step 5: preparation of compound **10g**

To a solution of N-(7-((2S,3S,4S,5R)-3-hydroxy-5-(hydroxymethyl)-4-((4-methoxybenzyl)oxy)tetrahydrofuran-2-yl)imidazo[2,1-f][1,2,4]triazin-4-yl)benzamide, compound **10f2** (400 mg, 0.81 mmol) in pyridine (6 mL) was added DMTrCl (496 mg, 1.46 mmol) at RT. After stirred at RT overnight, the mixture was combined with another crude batch and diluted with DCM (50 mL). The organic layer was successively washed with aqueous saturated $NaHCO_3$ (50 mL × 3), dried with anhydrous $Na_2SO_4$, filtered and concentrated to give a residue. The residue was purified by flash column chromatography on silica gel (0~100% EtOAc in p etroleum ether and 0~10% MeOH in DCM) to give compound **10g** (450 mg) as a white solid. Unreacted compound **10f2** (160 mg) was recovered as a white solid. $^1H$ NMR (400 MHz, DNEO-$d_6$) 11.50 (br s, 1H), 8.67 (br s, 1H), 7.99 (br d, *J*= 6.0 Hz, 1H), 7.86 (br s, 1H), 7.65 (br d, *J* = 6.4 Hz, 1H), 7.54 (br t, *J* = 7.6 Hz, 2H), 7.29-7.35 (m, 2H), 7.23-7.29 (m, 2H), 7.17-7.23 (m, 7H), 6.83 (td, *J* = 6.0, 3.2 Hz, 6H), 5.45 (br d, *J* = 4.8 Hz, 1H), 5.30 (br s, 1H), 4.63 (br d, *J* = 11.6 Hz, 1H), 4.43 (br d, *J* = 12.0 Hz, 1H), 4.12 (br s, 1H), 4.07 (br s, 1H), 3.72 (s, 9H), 3.20 (br d, *J* = 7.6 Hz, 1H), 3.01 (br d, *J*= 5.6 Hz, 1H); ESI-MS: m/z = 794.2 $[M+H]^+$.

Step 5: preparation of compound **10h**

A stirred suspension of compound **10g** (450 mg, 0.56 mmol), sulfamate **17a** (744.735 mg, 0.850 mmol) and

4Å MS (1 g) in dry THF (6 mL) was treated at 25 °C with DMAP (277 mg, 2.27 mmol). After stirring the yellow suspension at 45 °C for 18 h under $N_2$, the reaction mixture was filtered through a pad of diatomaceous earth and the filtrate concentrated under reduced pressure to give a yellow residue; the residue was dissolved in DCM (100mL) and washed with aqueous saturated $NaHCO_3$ (100 mL × 4). The organic layer was dried with anhydrous $Na_2SO_4$, filtered and concentrated to give a residue. The residue was purified by flash column chromatography on silica gel (gradient elution: 0 - 100% EtOAc in PE) to give compound **10h** (604 mg) as a yellow solid. ESI-MS: m/z = 766.8 $[M+H]^+$.

Step 6: preparation of compound **10i**

A solution of compound **10h** (704 mg, 0.46 mmol) in water (0.08 mL) and DCM (15 mL) was treated with DCA (118.6 mg, 0.92 mmol) at room temperature for 4 h, then quenched with pyridine (0.5 mL). After stirring at RT for 10 min, the mixture was concentrated in vacuo to give a residue. The residue was purified by flash column chromatography on silica gel (gradient elution: 0 -100% EtOAc in PE) to give compound **10i** (370 mg) as a solid. $^1$H NMR (400 MHz, DMSO-$d_6$) δ 11.46 (br s, 1H), 11.24 (s, 1H), 8.70 (s, 1H), 8.62 (s, 1H), 8.45 (br t, *J* = 4.0 Hz, 1H), 8.03 (br t, *J* = 8.0 Hz, 6H), 7.67 - 7.63 (m, 2H), 7.57 - 7.53 (m, 4H), 7.30 (br d, *J* = 8.0 Hz, 2H), 6.91 (br d, *J* =8.0 Hz, 2H), 6.38 - 6.33 (m, 1H), 5.89 (br d, *J*= 8.0 Hz, 1H), 5.65 (br s, 1H), 5.51 (br s, 1H), 5.38 (br s, 1H), 4.95 (br s, 1H), 4.63 - 4.50 (m, 4H), 4.32 (br t, *J* = 4.0 Hz, 1H), 4.04 - 3.99 (m, 3H), 3.74 (s, 3H), 3.59 (br d, *J* = 12.0 Hz, 2H), 3.29 - 3.22 (m, 1H); $^{19}$F NMR (376MHz, DMSO-$d_6$) δ -202.69 (td, J = 18.8, 52.6 Hz, 1F); ESI-MS: m/z = 926.5 $[M+H]^+$.

Step 7: preparation of compound **10j**

NOTE: THF was freshly distilled over Na/benzophenone and $CH_3CN$ was freshly distilled over $CaH_2$ before use. To a solution of compound **10i** (220 mg, 0.24 mmol, dried by lyophilization) dissolved in THF (4 mL) was added 4A MS (powder, 800 mg) and a solution of 1H-tetrazole (5.28 mL, 0.45 M, prepared by dissolved 945 mg of tetrazole (dried by lyophilization) in 30 mL of dry $CH_3CN$, followed by addition of 1 g of 4A MS and then stirred for 1hr under $N_2$ before use). The vessel flask was purged several times with $N_2$. A solution of 2-cyanoethyl-N,N,N',N'-tetraisopropylphosphorodiamidite (129 mg, 0.43 mmol) in THF (2 mL) was added drop-wise over 20 min vial a syringe. After stirring at RT for 1.5 hr., a solution of TBHP (0.38 mL, 1.9 mmol, 5M) was added and the mixture was stirred for another 30 min and concentrated under reduced pressure to give a residue. The residue was purified by flash column chromatography on silica gel ((gradient elution: 0 -10% DCM in MeOH, $R_f$= 0.6) to give compound **10j** as a white solid (308 mg). ESI-MS: m/z = 1041.4 $[M+H]^+$.

Step 8: preparation of compound 10k

Compound **10j** (308 mg) was treated with $MeNH_2$/EtOH (2 mL) and stirred at RT for 2.5 hr. The reaction mixture was concentrated under reduced pressure and the residue purified by reverse phase preparative HPLC (Column: Waters Xbridge Prep OBD 5μm C18 150x30, Condition: A: water (10mM $NH_4HCO_3$)-ACN: MeCN, beginning: B 5%, End B: 35%; Flow Rate (mL/min) 25) to give compound **10k** (32 mg) as a white solid.

ESI-MS: m/z = 780.3 $[M+H]^+$.

Step 9: preparation of **compound 48, sodium salt**

A solution of anisole (43.43 mg, 0.4 mmol) in TFA (0.31 mL, 4.02 mmol) was cooled down to 0°C. The solution was added to compound **10k** (32 mg, 0.04 mmol). After stirring the mixture at 0° C for 2.5 hr., most of the TFA was removed by blowing with a flow of nitrogen gas at 0 °C. The remaining reaction mixture was quenched with $MeNH_2$, 33% solution in EtOH (0.31 mL) at 0°C. The reaction mixture was evaporated under reduced pressure and the residue partitioned between DCM/water (30 mL × 3 / 15 mL) and the aqueous layer washed with DCM (30 mL × 2). Aqueous layer was lyophilized to give a residue. The residue was purified by reverse phase preparative HPLC (Waters Xbridge Prep OBD C18 5μm C18 150x30, Condition: A: water (10mMN$H_4HCO_3$)-ACN: ACN, beginning: B 5%, End B: 35%; Flow Rate (mL/min) 25.) to give **compound 48, ammonium salt** (19.2 mg) as a white solid. $^1$H NMR (400 MHz, $D_2O$) δ 8.32 (s, 1H), 8.00 (d, *J* = 3.2 Hz, 2H), 7.49 (s, 1H), 6.57 - 6.50 (m, 1H), 5.81 - 5.75 (m, 1H), 5.73 - 5.65 (m, 2H), 5.54 (br d, *J* =4.4 Hz, 1H), 5.39 - 5.27 (m, 1H), 4.80 (d, *J*= 4.2 Hz, 1H), 4.46 (br s, 1H), 4.42 - 4.34 (m, 1H), 4.24 - 4.17 (m, 1H), 3.82 (br dd, *J*= 3.2, 13.4 Hz, 1H), 3.61(br d, *J* = 13.2 Hz, 1H); $^{19}$F NMR (376MHz, $D_2O$) δ -197.15-197.29 (m, IF); $^{31}$P NMR (162 MHz, $D_2O$) δ -1.83 (s, 1P); ESI-MS: m/z = 660.1 $[M+H]^+$.

**Sodium salt conversion**

Dowex 50W × 8, 200-400 (5 mL, H form) was added to a beaker and washed with deionized water (25 mL).

Then to the resin was added 15% $H_2SO_4$ in deionized water, the mixture was gently stirred for 5 min, and decanted (25 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 ×). The resin was transferred to the column and washed with 15% NaOH in water (at least 4 CV), and then with deionized water until it was neutral. **Compound 48, ammonium salt** (19.2 mg) was dissolved in DI water (2 mL) and added to the top of the column, and eluted with deionized water. Appropriate fractions were pooled together and lyophilized to **compound 48, sodium salt** (16 mg) as a white solid. $^1H$ NMR (400 MHz, $D_2O$) δ 8.04 (br s, 1H), 7.73 (s, 1H), 7.56 (s, 1H), 6.83 (br s, 1H), 6.33 - 6.25 (m, 1H), 5.68 - 5.60 (m, 1H), 5.46 (br d, *J* = 9.6 Hz, 1H), 5.32(br d, *J* = 4.0 Hz, 1H), 5.20 (br d, *J* = 4.4 Hz, 1H), 5.06 - 4.90 (m, 1H), 4.38 (br d, *J*= 9.2 Hz, 1H), 4.30 (br s, 1H), 4.17 (br dd, *J* = 6.0, 10.4 Hz, 1H), 4.02 (brd, *J* =11.6 Hz, 1H), 3.67 (br d, *J*= 12.8 Hz, 1H), 3.41 (br d, *J* = 13.2 Hz, 1H); $^{31}P$ NMR (162MHz, $D_2O$) δ -2.02 (s, IP); $^{19}F$ NMR (376MHz, $D_2O$) δ -196.74 (br s, IF); ESI-MS: m/z = 660.1 [M+H] $^+$.

Example 11

Compound 11

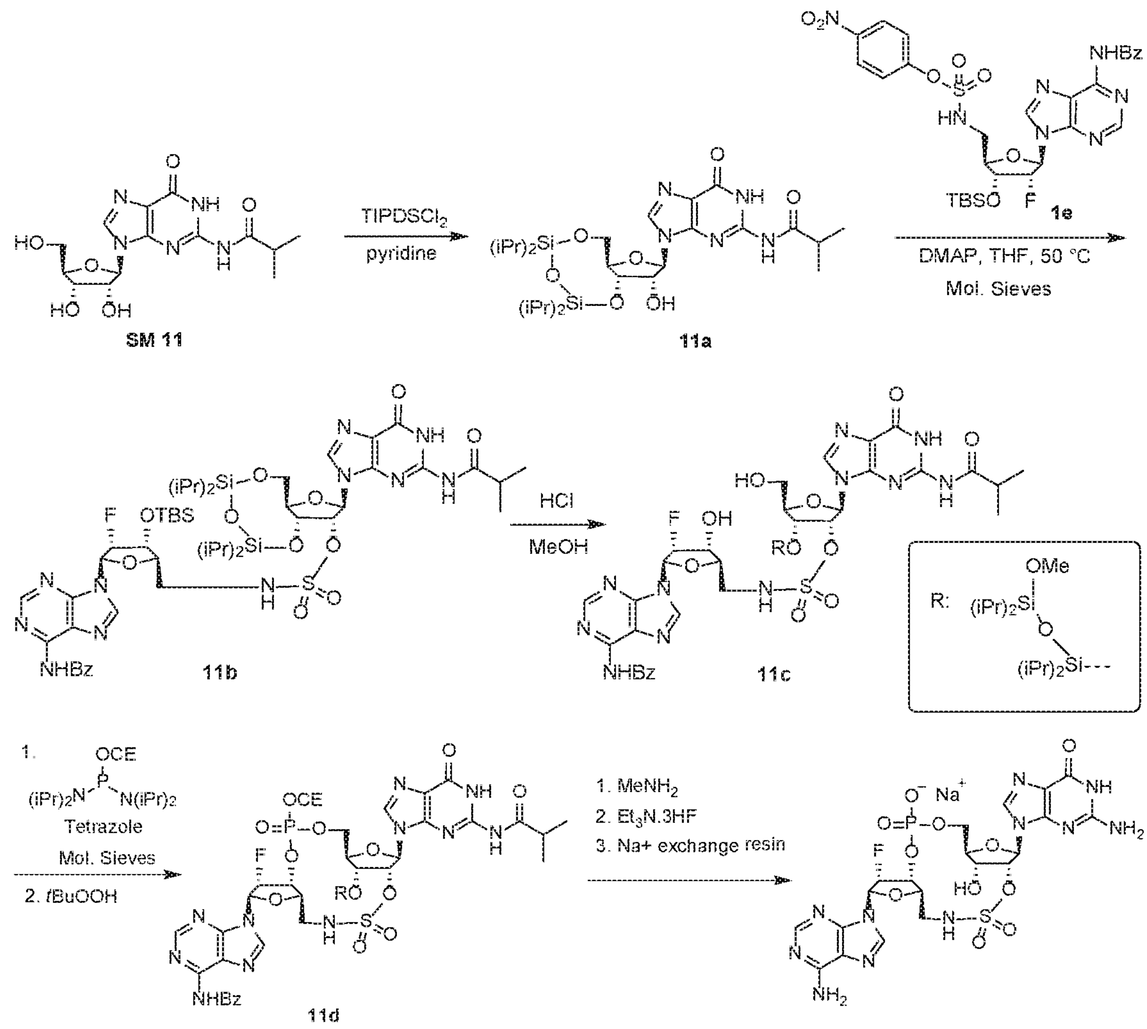


Compound 11, sodium salt

Step 1: preparation of compound **11a**

TIPDSCl$_2$ (6.70 g, 21.23 mmol) was added to a solution of **SM 11**, *N*-isobutyrylguanosine (5.0 g, 14.15 mmol) in pyridine (50 mL). The reaction mixture was stirred for 12 h at room temperature after which it was concentrated under reduced pressure. The residue product was purified by silica column chromatography (gradient elution: 0 - 10% MeOH in DCM) to give compound **11a** as a white foam (5.5 g, yield: 65%). $^1$H NMR (400 MHz, DMSO-$d_6$) δ ppm 12.12 (s, 1 H), 11.76 (s, 1 H), 8.05 (s, 1 H), 5.79 (s, 1 H), 5.71 (d, *J*=4.9 Hz, 1 H), 4.28 - 4.39 (m, 2 H), 4.13 (br dd, *J*=12.9, 2.6 Hz, 1 H), 4.00 - 4.07 (m, 1 H), 3.94 (br dd, *J*=12.9, 2.7 Hz, 1 H), 2.78 (spt, *J*=6.8 Hz, 1 H), 1.11 (d, *J*=6.6 Hz, 6 H), 0.93 - 1.08 (m, 28 H); ESI-MS: *m/z* 596.2 [M+H]$^+$.

Step 2: preparation of compound **11b**

Activated molecular sieves were added to a solution of compound **11a** (1.28 g, 1.92 mmol) and compound **1e** (1.0 g, 1.28 mmol) in dry THF (60 mL); the resulting mixture was stirred for 1 h under N$_2$. Next, DMAP (783 mg, 6.41 mmol) was added to initiate the reaction. The reaction mixture was stirred at 50 °C overnight The molecular sieves were removed by filtration and rinsed with DCM, the filtrate was washed with saturated aqueous NaHCO$_3$. The aqueous phase was extracted with DCM The combined organic layers were dried with Na$_2$SO$_4$, filtered and concentrated under reduced pressure. Compound **11b** (520 mg, yield: 36%) was obtained as a yellow solid after purification by silica column chromatography (gradient elution: 0 - 100% EtOAc in petroleum ether followed by 0 - 2% MeOH in DCM). $^1$H NMR (400 MHz, DMSO-$d_6$) δ ppm 12.08 (br s, 1 H), 11.56 (br s, 1 H), 11.28 (br s, 1 H), 8.70 (s, 1 H), 8.61 (s, 1 H), 8.54 (br s, 1 H), 8.09 (s, 1 H), 8.04 (s, 2 H), 7.61 - 7.73 (m, 1 H), 7.49 - 7.59 (m, 2 H), 6.31 - 6.44 (m, 1 H), 6.06 (d, *J*=1.5 Hz, 1 H), 5.73 (br d, *J*=53.5 Hz, 1 H), 5.31 (br d, *J*=5.8 Hz, 1 H), 4.79 - 4.95 (m, 1 H), 4.64 (t, *J*=6.9 Hz, 1 H), 3.84 - 4.10 (m, 4 H), 3.41 (m, *J*=13.3 Hz, 1 H), 3.11 - 3.25 (m, 1 H), 2.75 (spt, *J*=6.5 Hz, 1 H), 0.85 - 1.15 (m, 43 H), 0.15 (s, 3 H), 0.14 (s, 3 H); ESI-MS: *m/z* 1144.4 [M+H]$^+$.

Step 3: preparation of compound **11c**

A solution of compound **11b** (1.3 g, 0.98 mmol) in dry MeOH (55 mL, dried on molecular sieves) was treated with HCl (0.19 mL of 2 M in Et$_2$O, 13.7 mmol) for 4 h. The reaction mixture was quenched by the addition of saturated aqueous NaHCO$_3$ followed by extraction with DCM. The organic phase was dried with anhydrous MgSO$_4$, filtered and concentrated under reduced pressure. The crude product was purified by silica column chromatography (gradient elution: 0-5% MeOH in DCM) to the give compound **11c** (670 mg, yield: 65%). $^1$H NMR (400 MHz, DMSO-$d_6$) δ ppm 1.01 - 1.08 (m, 28 H), 1.12 (d, *J*=6.8 Hz, 3 H), 1.67 - 1.75 (m, 1 H), 2.20 - 2.32 (m, 1 H), 2.43 - 2.54 (m, 1 H), 2.62 (br d, *J*=14.3 Hz, 1 H), 2.79 (spt, *J*=6.9 Hz, 1 H), 2.85 - 2.96 (m, 1 H), 3.51 (s, 3 H), 3.52 - 3.57 (m, 2 H), 3.75 - 3.83 (m, 1 H), 4.39 - 4.48 (m, 1 H), 4.50 (d, *J*=4.2 Hz, 1 H), 5.02 (q, *J*=9.6 Hz, 1 H), 5.09 (t, *J*=4.5 Hz, 1 H), 5.25 (dd, *J*=9.8, 4.1 Hz, 1 H), 5.52 (ddd, *J*=52.4, 4.4, 2.6 Hz, 1 H), 5.68 (br d, *J*=5.9 Hz, 1 H), 6.30 (dd, *J*=18.5, 2.6 Hz, 1 H), 7.56 (t, *J*=7.7 Hz, 2 H), 7.61 - 7.69 (m, 1 H), 8.02 - 8.07 (m, 2 H), 8.11 (br s, 1 H), 8.14 (s, 1 H), 8.57 (s, 1 H), 8.71 (s, 1 H), 11.22 (br s, 1 H), 11.33 (br s, 1 H), 12.09 (br s, 1 H); ESI-MS: *m/z* 1060.7 [M+H]$^+$.

Step 4: preparation of compound **11d**

A solution of compound **11c** (340 mg, 0.32 mmol) and 1H-tetrazole (4.48 mL of a 3 - 4% in MeCN, dried on 3Å molecular sieves before use) in dry THF / MeCN (1:1, 11 mL, dried on 3Å molecular sieves before use) was treated with activated 3Å molecular sieves for 2 h under N$_2$ after which 2-cyanoethyl-*N,N,N',N'*-tetra(isopropyl)phosphorodiamidite (96 mg, 0.32 mmol) was added in one portion. The reaction mixture was shaken for 2 h. An additional amount of 2-cyanoethyl-*N,N,N',N'*-tetra(isopropyl)phosphorodiamidite (19 mg, 0.064 mmol) was added and shaking was continued for 2 h. A solution of *t*BuOOH (93 μL of 5.5 M solution in decane, 0.51 mmol) was added, the reaction mixture was shaken for 30 min. The molecular sieves were removed by filtration and rinsed with dichloromethane. The filtrate was washed with brine and concentrated under reduced pressure. The crude product was purified by column chromatography over silica gel (gradient elution: 0-5% MeOH in DCM) give compound **11d** (100 mg, yield: 26.5%). ESI-MS: *m/z* 1177.6 [M+H]$^+$.

Step 5: preparation of **compound 11, sodium salt**

The above compound **11d** (100 mg, 0.085 mmol) was stirred in a 33% methylamine solution in ethanol (2 mL) at room temperature until complete conversion (ca. 3 h), after which the reaction solution was concentrated under reduced pressure and triturated in MeCN. The precipitate was dissolved in a mixture of pyridine (684 μL mL) and Et$_3$N (590 μL). Triethylamine trihydrofluoride (57 mg, 0.34 mmol) was added, the resulting reaction mixture was stirred at

room temperature until complete conversion (note: precipitation of desired product observed. The residue, obtained after concentration under reduced pressure, was triturated in MeCN, the obtained precipitate was further purified by preparative reversed phase HPLC (Stationary phase: XBridge C18 OBD, 5 μm, 250 × 30 mm, Mobile phase: 0.25% ammonia bicarbonate (A) - MeCN (B); gradient elution) to give **compound 11.** Final conversion into the sodium salt was done by elution of an aqueous solution over a column packed with a cationic Na ion-exchange resin affording **compound 11, sodium salt** as a white fluffy solid (24.5 mg, yield: 41%). $^1$H NMR (600 MHz, DMSO-$d_6$) δ ppm 10.03 (br s, 1 H), 9.49 (br s, 1 H), 8.27 (br s, 1 H), 7.85 (s, 1 H), 7.60 (br s, 1 H), 7.25 (br s, 2 H), 6.41 - 6.79 (m, 2 H), 6.36 (br d, *J*=20.0 Hz, 1 H), 6.08 - 6.22 (m, 1 H), 6.04 (br d, *J*=7.9 Hz, 1 H), 5.72 (br s, 1 H), 5.43 (br dd, *J*=51.7, 4.3 Hz, 1 H), 5.25 - 5.34 (m, 1 H), 4.54 (br t, *J*=4.0 Hz, 1 H), 4.33 - 4.43 (m, 1 H), 4.29 - 4.33 (m, 1 H), 4.11 (br t, *J*=3.9 Hz, 1 H), 3.85 - 3.94 (m, 1 H), 3.56 (br dd, *J*=13.0, 2.6 Hz, 1 H), 3.34 (br d, *J*=12.6 Hz, 1 H); $^{31}$P NMR (162 MHz, DMSO-$d_6$) δ ppm -2.34 (s, 1 P); ESI-MS: *m/z* 675.1 [M+H]$^+$.

Example 12

**Compound 29**

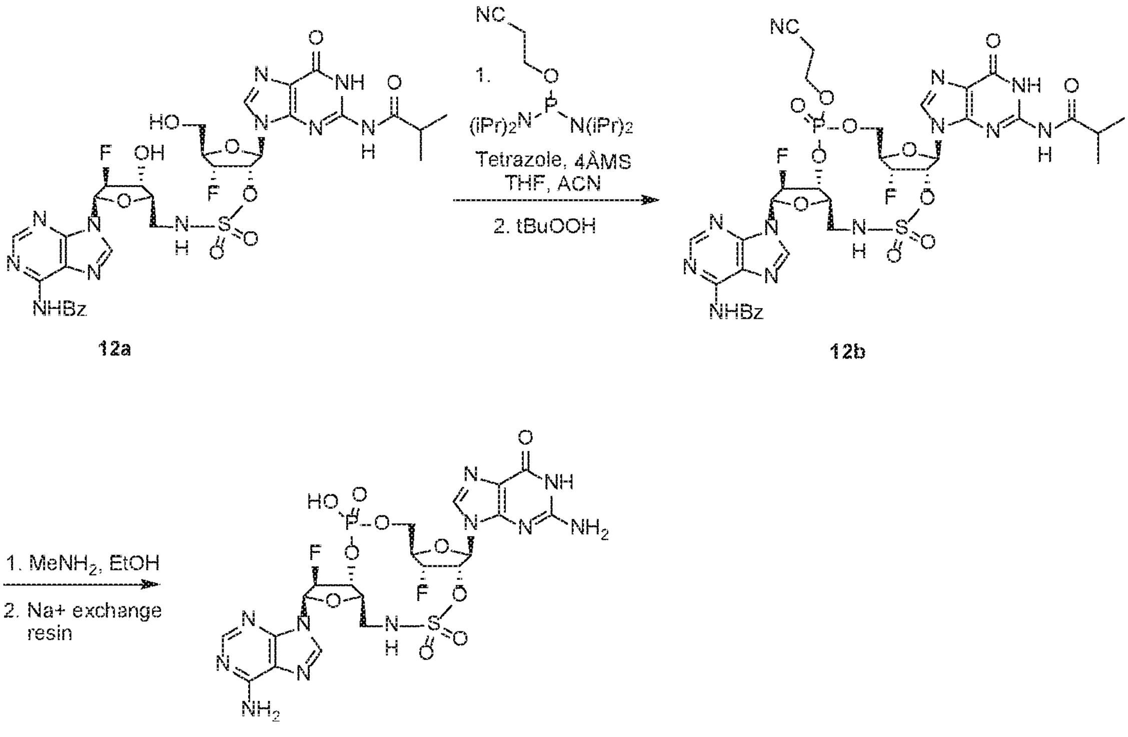


12a

12b

**Compound 29, sodium salt**

Step 1: Preparation of compound **12b**

Compound **12a** (300 mg, 0.38 mmol) was dissolved in a mixture of anhydrous ACN (33 mL) and anhydrous THF (33 mL). 1H-tetrazole (3.3 mL, 1.13 mmol) and 3Å molecular sieves were added. The mixture was shaken for 2 h at RT and then 2-cyanoethyl-N,N,N',N'-tetraisopropylphosphorodiamidite (0.16 mL, 0.49 mmol) was added at once via syringe. The reaction mixture was shaken at RT for 4 hours. An additional amount of 2-cyanoethyl-N,N,N',N'-tetraisopropylphosphorodiamidite (0.078 mL, 0.25 mmol) was added. The reaction mixture was shaken at RT for 1 hour and then tert-butyl hydroperoxide solution 5.5 M in decane (0.075 mL, 0.41 mmol) was added. The reaction mixture was shaken at RT for 30 minutes, and then filtered. The molecular sieves were washed three times with dichloromethane. The combined filtrate was washed with a mixture of a saturated $Na_2S_2O_3$ solution and a saturated $NaHCO_3$ solution, washed with brine, dried with $MgSO_4$, filtered and the solvents of the filtrate evaporated. The residue was purified over a $SiO_2$ column using a gradient from 100% DCM to 10% MeOH in DCM The fractions containing product were combined

and the solvents were evaporated to give **12b** (47 mg, 12% yield). ESI-MS: m/z 905.4 [M+H]$^+$.

Step 2: Preparation of **compound 29, sodium salt**

Compound **12b** (47 mg, 0.045 mmol) in methylamine, 33% solution in ethanol (2.5 mL, 20.2 mmol) was stirred at 40°C for 3 h. The reaction mixture was evaporated to dryness under reduced pressure. The residue was triturated in 10 mL anhydrous acetonitrile. The precipitate collected by filtration and washed with anhydrous acetonitrile. The residue was purified with reverse phase preparative HPLC (Stationary phase: RP XBridge Prep C18 OBD-10μm,50×150mm, Mobile phase: 0.25% $NH_4HCO_3$ solution in water, MeOH). The solvents of the pure fraction were removed by lyophilization. The residue was dissolved in water and purified over a prewashed (water) column filled with a ion-exchange resin resin. The solvents of the resulting solution were removed by lyophilization to give **compound 29, sodium salt** as a white solid (27 mg, 87% yield). $^1$H NMR (400 MHz, DMSO-$d_6$) $\delta$ ppm 3.18 - 3.28 (m, 1 H) 3.29 - 3.40 (m, 1 H) 4.02 - 4.10 (m, 2 H) 4.17 - 4.25 (m, 1 H) 4.29 - 4.48 (m, 1 H) 4.36 (br s, 1 H) 5.09 (br dd, J=13.6, 7.1 Hz, 1 H) 5.23 - 5.49 (m, 2 H) 5.86 - 6.00 (m, 2 H) 6.34 - 6.42 (m, 1 H) 6.61 (br s, 2 H) 6.96 (br s, 2 H) 7.92 (s, 1 H) 8.11 (d, J=2.4 Hz, 1 H) 8.15 (s, 1 H); ESI-MS: m/z 678.2 [M+H]$^+$.

<u>Example 13</u>

**Compound 15**

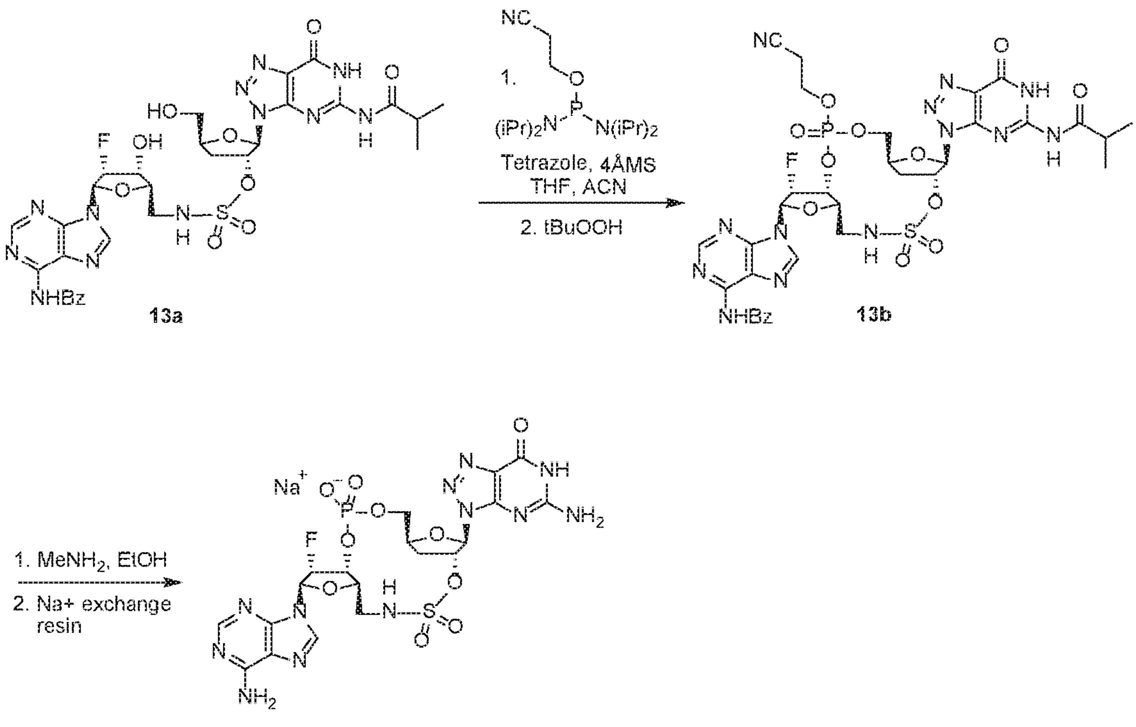


13a 13b

**Compound 15, sodium salt**

Step 1: Preparation of compound **13b**

Compound **13a** (355 mg, 0.46 mmol) was dissolved in a mixture of anhydrous ACN (40 mL) and anhydrous THF (40 mL). 1H-tetrazole (4.1 mL, 1.4 mmol) and 3Å molecular sieves were added. The mixture was shaken for 2 hours at RT and then 2-cyanoethyl-N,N,N',N'-tetraisopropylphosphorodiamidite (0.19 mL, 0.61 mmol) was added at once via syringe. The reaction mixture was shaken at RT for 4 hours. An additional amount of 2-cyanoethyl-N,N,N',N'-tetraisopropylphosphorodiamidite (0.18 mL, 0.57 mmol) was added. The reaction mixture was shaken at RT for 1 hour and then tert-butyl hydroperoxide solution 5.5 M in decane (0.093 mL, 0.51 mmol) was added. The reaction mixture was shaken at RT for 30 minutes. The reaction mixture was filtered. The molecular sieves were washed three times with

dichloromethane. The combined filtrate was washed with a mixture of a saturated $Na_2S_2O_3$ solution and aqueous saturated $NaHCO_3$ solution, washed with brine, dried with $MgSO_4$, filtered and the solvents of the filtrate evaporated. The residue was purified over a $SiO_2$ column using a gradient from 100% DCM to 10% MeOH in DCM. The fractions containing product were combined and the solvents were evaporated to give **13b** (34 mg, 5% yield). ESI-MS: m/z 888.4 $[M+H]^+$.

Step 2: Preparation of **compound 15, sodium salt**

Compound **13b** (34 mg, 0.023 mmol) in methylamine, 33% solution in ethanol (2 mL, 16 mmol) was stirred at 40°C for 3 hours. The reaction mixture was evaporated to dryness under reduced pressure. The residue was triturated in 10 mL anhydrous acetonitrile. The precipitate was filtered off and washed with anhydrous acetonitrile. A purification was performed with reverse phase preparative HPLC (Stationary phase: RP XBridge Prep C18 OBD-10μm,50×150mm, Mobile phase: 0.25% $NH_4HCO_3$ solution in water, MeOH). The solvents of the pure fraction were removed by lyophilization. The residue was dissolved in water and filtered over a prewashed (water) column packed with a cationic sodium ion-exchange resing. The solvents of the resulting solution were removed by lyophilization to give **compound 15, sodium salt** (23 mg, 100% yield).as a white solid. $^1H$ NMR (400 MHz, DMSO-$d_6$) $\delta$ ppm 2.00 - 2.13 (m, 1 H) 2.49 (m, J=3.9, 1.7, 1.7 Hz, 1 H) 3.29 - 3.37 (m, 1 H) 3.57 (dd, J=13.6, 2.6 Hz, 1 H) 3.85 - 3.97 (m, 1 H) 4.03 - 4.17 (m, 1 H) 4.27 (br d, J=9.0 Hz, 1 H) 4.45 - 4.55 (m, 1 H) 4.73 - 4.88 (m, 1 H) 5.10 (br d, J=22.4 Hz, 1 H) 5.42 (dd, J=51.1, 4.1 Hz, 1 H) 6.11 (br d, J=4.1 Hz, 1 H) 6.32 (d, J=18.7 Hz, 1 H) 6.86 - 7.03 (m, 2 H) 7.07 (br s, 2 H) 7.85 (br s, 1 H) 8.15 (s, 1 H) 8.35 (s, 1 H) 8.40 - 8.87 (m, 1 H); ESI-MS: m/z 661.3 $[M+H]^+$.

Example 14

**Compound 30**

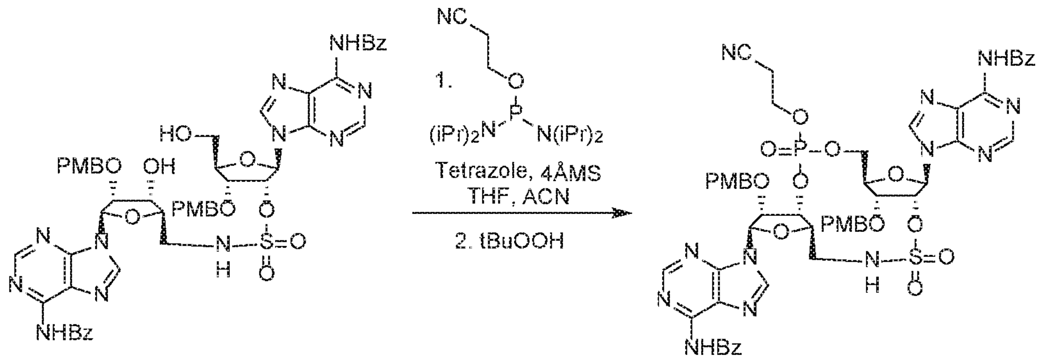


**14a** **14b**

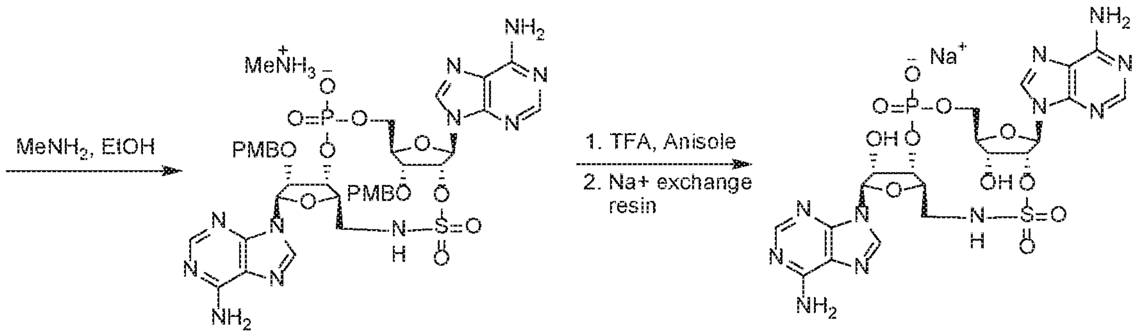


**14c methylammonium salt** **Compound 30, sodium salt**

Step 1: Preparation of compound **14b**

Compound **14a** (275 mg, 0.26 mmol) was dissolved in anhydrous ACN (40 mL). 1H-tetrazole (2.3 mL, 1.8 mmol) and 3Å molecular sieves were added. The mixture was shaken for 1 hour at RT and then 2-cyanoethyl-N,N,N',N'-tetraisopropylphosphoro-diamidite (0.084 mL, 0.26 mmol) was added at once via syringe. The reaction mixture was shaken at RT for 4 hours. An additional amount of 2-cyanoethyl-N,N,N',N'-tetraisopropylphosphorodiamidite (0.084 mL, 0.26 mmol) was added. The reaction mixture was shaken at RT for 1 hour and then tert-butyl hydroperoxide solution 5.5 M in decane (0.062 mL, 0.34 mmol) was added. The reaction mixture was shaken at RT for 18 hours. The reaction mixture was filtered. The molecular sieves were washed three times with dichloromethane. The combined filtrate was washed with a mixture of a saturated $Na_2S_2O_3$ solution and aqueous saturated $NaHCO_3$ solution, washed with brine, dried with $MgSO_4$, filtered and the solvents of the filtrate evaporated. The residue was purified over a $SiO_2$ column using a gradient from 100% DCM to 10% MeOH in DCM. The fractions containing product were combined and the solvents were evaporated to give **14b** (30 mg, 5% yield). ESI-MS: m/z 1159.6 $[M+H]^+$.

Step 2: Preparation of compound **14c, methylammonium salt**

Compound **14b** (30 mg, 0.014 mmol) in methylamine, 33% solution in ethanol (3 mL, 24.3 mmol) was stirred at 45°C for 1 hour. The reaction mixture was evaporated to dryness under reduced pressure. The residue was triturated in 10 mL anhydrous acetonitrile. The precipitate was filtered off and washed with anhydrous ACN to give **compound 14c, methylammonium salt** as a white solid (11 mg, 71% yield). ESI-MS: m/z 898.5 $[M+H]^+$.

Step 3: Preparation of **compound 30, sodium salt**

A solution of anisole (0.011 mL, 0.1 mmol) in TFA (0.076 mL, 1 mmol) was cooled down to 0°C. The cold solution was added to **compound 14c** (11 mg, 0.01 mmol). The reaction mixture was stirred at 0°C for 75 minutes. The majority of the TFA was removed by blowing with a flow of nitrogen gas at 0°C. The remaining reaction mixture was quenched by the addition of methylamine, 33% solution in ethanol (0.12 mL, 1 mmol) at 0°C. The reaction mixture was evaporated to dryness. The residue was purified with reverse phase preparative HPLC (Stationary phase: RP XBridge Prep C18 OBD-10μm,50×250mm Mobile phase: 0.25% $NH_4HCO_3$ solution in water, $CH_3CN$). The solvents of the pure fraction were removed by lyophilization. The residue was dissolved in water and filtered over a prewashed (water) column filled with a cationic sodium ion-exchange resin. The fractions containing product were combined. The solvents of the resulting solution were removed by lyophilization to give **compound 30, sodium salt** as a white solid (3 mg, 45% yield). $^{31}P$ NMR (162 MHz, $D_2O$) $\delta$ ppm -1.27 (s, 1 P); ESI-MS: m/z 658.3 $[M+H]^+$.

Example 15

**Compound 38**

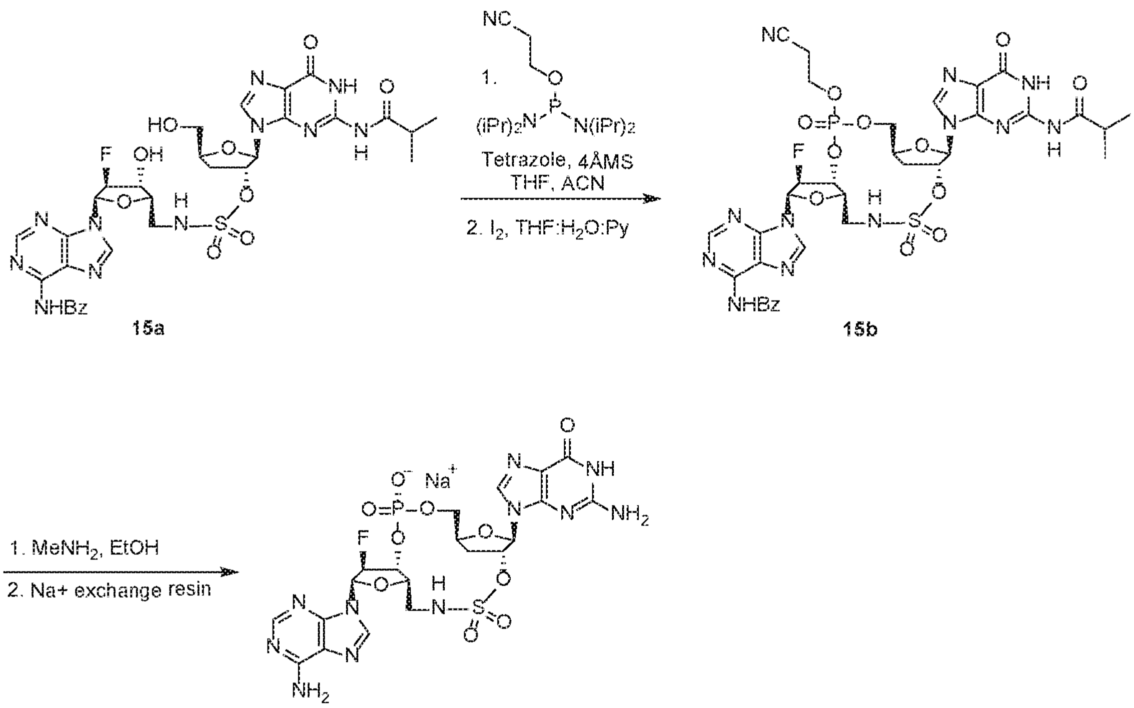


15a 15b

**Compound 38, sodium salt**

Preparation of compound **15b**

The diol **15a** (210 mg, 0.272 mmol) was co-evaporated with a mixture of anhydrous toluene: acetonitrile (1:1, v/v, 3×30 mL) then dissolved in anhydrous THF (20 mL). 4 Å Molecular sieves powder (0.8 g) and 0.45 M tetrazole in MeCN (4.8 mL, 2.17 mmol) were added. The resulting heterogeneous mixture was bubbled with Argon for 4 min. After stirring this mixture at RT for 10 min, a solution of 2-cyanoethyl-*N,N,N',N'*-tetra(isopropyl)phosphorodiamidite (131 mg, 0.43 mmol) in MeCN (3 mL) was added to this over 30 min at RT. After stirring for 90 min, the reaction filtered, then washed with THF (15 mL). 0.5 M Iodine (in THF:water:Py 8:1:1, v/v/v) was added until the color persists. After stirring the reaction at RT for 30 min, the reaction mixture was diluted with EtOAc (30 mL), excess iodine was quenched with aqueous saturated $Na_2S_2O_3$ (until discoloration). Phases were separated; organic phase was washed with aqueous saturated $NaHCO_3$ (1 × 20 mL), aqueous saturated NaCl (1 × 20 mL). Aqueous phase was back extracted with EtOAc (1 × 20 mL). The combined organic phase evaporated to dryness, the resulting crude material was purified by flash

column chromatography over silica gel (0-15% MeOH in dichloromethane) to give **15b** (120 mg). ESI-MS: m/z 873 M+H]$^+$.

Preparation of **Compound 38 sodium salt**

Compound **15b** (120 mg) was subjected to 33% methylamine solution in ethanol (15 mL) at RT. After stirring the reaction at 40 °C for 2 h, the mixture was concentrated under reduced pressure. The resulting crude solid was washed with DCM (15 mL) and the precipitate was collected by filtration and purified by reverse phase preparative HPLC (column: Synergi 4μm, Hydro RP, 250 mm × 30 mm, Mobile Phase: Buffer A: 50 mM Triethylammonium acetate in water; Buffer B: 50 mM Triethylammoniumacetate [TEAA] in $CH_3CN$, gradient: 0-40% of B over 30 min, flow rate 24 mL/min) to give **Compound 38** (7.1 mg) as a TEAA salt ESI-MS: m/z: 658 [M-1]$^-$.

Salt conversion: Dowex 50W × 8, 200-400 (5 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 ×). The resin was transferred to the column and washed with 15% NaOH in water (at least 4 CV), and then with deionized water until it was neutral. **Compound 38 TEAA salt** (24.2 mg) were dissolved in minimum amount of deionized water and $CH_3CN$ (1:1, v/v), added to the top of the column, and eluted with deionized water. Appropriate fractions were pooled together and lyophilized to **Compound 38 sodium salt** (6.3 mg) as a white solid. $^1H$ NMR (400 MHz, $D_2O$) $\delta$ 7.98 (s, 1H), 7.82 (s, 1H), 7.49 (s, 1H), 6.37 (dd, J = 5.2 Hz, 11.6 Hz, 1H), 5.75-5.90 (m, 2H), 5.49 (t, J = 4.8 Hz, 0.5H), 5.37 (t, J = 4.8 Hz, 0.5H), 5.01-5.15 (m, 1H), 4,51 (s, 1H), 4.26 (d, J = 3.6 Hz, 1H), 4.00-4.10 (m, 2H), 3.35-3.50 (m, 2H), 2.45-2.70 (m, 2H). $^{31}P$ NMR (162 MHz, $D_2O$) $\delta$ -1.416 ppm (s, IP); $^{19}F$ NMR (379 MHz, $D_2O$) $\delta$ -196 ppm (broad peak, IF); ESI-MS: m/z: 658.4 [M-1]$^-$.

<u>Example 16</u>

Compound 28

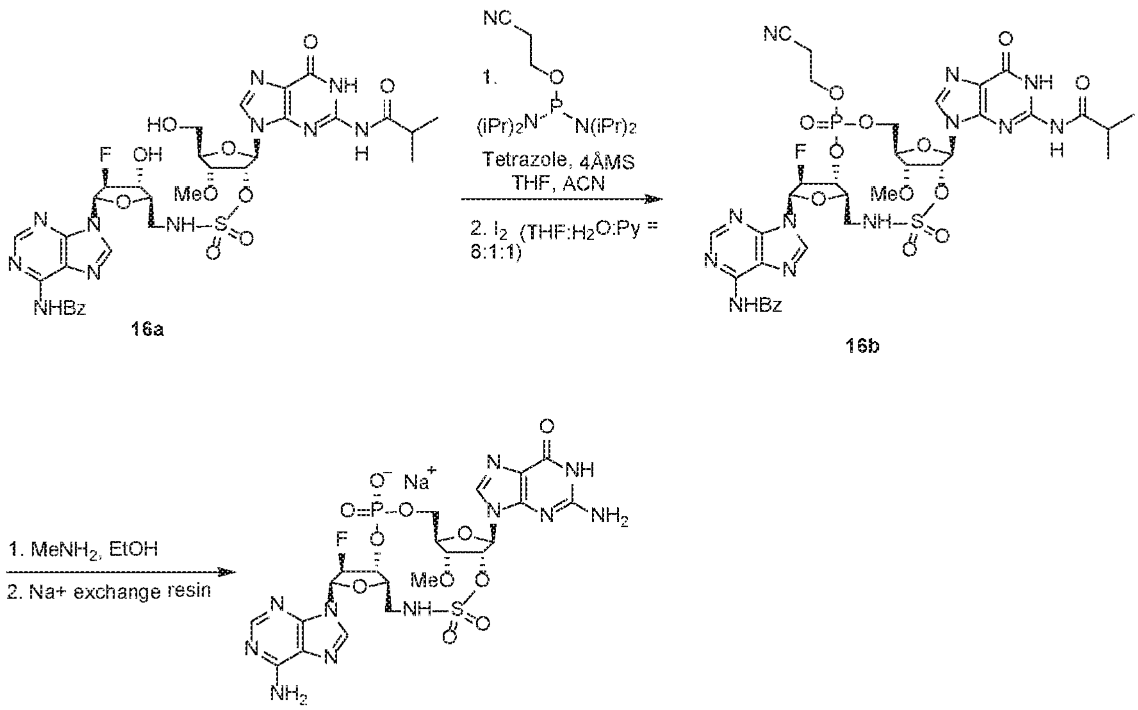


Compound 28, sodium salt

Preparation of compound **16b**

Compound **16a** (140 mg, 0.174 mmol) was co-evaporated with a mixture of anhydrous Toluene: Acetonitrile (1:1, v/v, 3 × 30 mL) then dissolved in anhydrous THF (15 mL). 4Å Molecular sieves powder (0.5 g) and 0.45 M tetrazole in acetonitrile (2.32 mL, 1.04 mmol) were added. The resulting heterogeneous mixture was bubbled with Ar for 4 min. After stirring the mixture at RT for 10 min, a solution of 2-cyanoethyl-*N,N,N',N'*-tetra(isopropyl)phosphorodiamidite (84 mg, 0.28 mmol, 1.6 eq) in $CH_3CN$ (3.0 mL) was added to this over 30 min at RT. After stirring the reaction for 90 min, the mixture was filtered then washed with THF (15 mL). The resulting compound phosphite (MS: *m/z* 901 $[M+H]^+$) was used directly in the next step. 0.5 M Iodine (in THF:water:Py 8:1:1, *v/v/v*) was added to this until the color persists. After stirring the mixture at RT for 30 min, the reaction was diluted with EtOAc (30 mL); excess iodine was quenched with aqueous saturated $Na_2S_2O_3$ (until discoloration). The phases were separated; the organic phase was washed with aqueous saturated $NaHCO_3$ (1 × 20 mL) and brine (1 × 20 mL). The aqueous layer was back extracted with EtOAc (1 × 20 mL). The combined organic layers were evaporated to dryness to afford a residue. The residue was purified by flash column chromatography over silica gel (0-15% MeOH in dichloromethane, *v/v*) to give compound **16b** (80 mg). ESI-MS: *m/z* 917 $[M+H]^+$.

Preparation of **compound 28, sodium salt**

Compound **16b** (80 mg) was subjected to 33% methylamine solution in ethanol (6 mL) at RT. After stirring the mixture at 40 °C for 2 h, the reaction was concentrated under reduced pressure to give a solid. The resulting crude solid was washed with DCM (15 mL) and the precipitate was collected by filtration, and purified by reverse phase preparative HPLC (column: Synergi 4μ, Hydro RP, 250 mm × 30 mm, Mobile Phase: Buffer A: 50 mM Triethylammonium acetate in water; Buffer B: 50 mM Triethylammoniumacetate in $CH_3CN$, gradient: 0-40% of B over 30 min, flow rate 24 mL/min) to give **compound 28** (22.4 mg) as a TEAA salt. Final conversion into the sodium salt was done by elution of an aqueous solution over a column packed with Cationic Na ion-exchange resin to afford **compound 28, sodium salt** as a white fluffy solid after lyophilization (21.9 mg).

$^1H$ NMR (400 MHz, $D_2O$) $\delta$ ppm 7.87 (s, 1H), 7.80 (s, 1H), 7.19 (s, 1H), 6.32 (t, *J* = 5.6 Hz, 1H), 5.97 (d, *J* = 8.0 Hz, 1H), 5.91 (s, 1H), 5.50 (s, 0.5H), 5.37 (s, 0.5H), 4.98-5.10 (m, 1H), 4.49 (s, 1H), 4.10-4.30 (m, 4H), 3.52 (d, *J* = 11.6 Hz, 1H), 3.44 (s, 3H), 3.34 (d, *J*= 12.4 Hz); $^{19}F$ NMR (379 MHz, $D_2O$) $\delta$ broad peak -196.61 ppm; $^{31}P$ NMR (162 MHz, $D_2O$) $\delta$ -1.38 ppm; ESI-MS: *m/z*: 688.8 $[M-1]^-$.

Example 17

## Compound 33

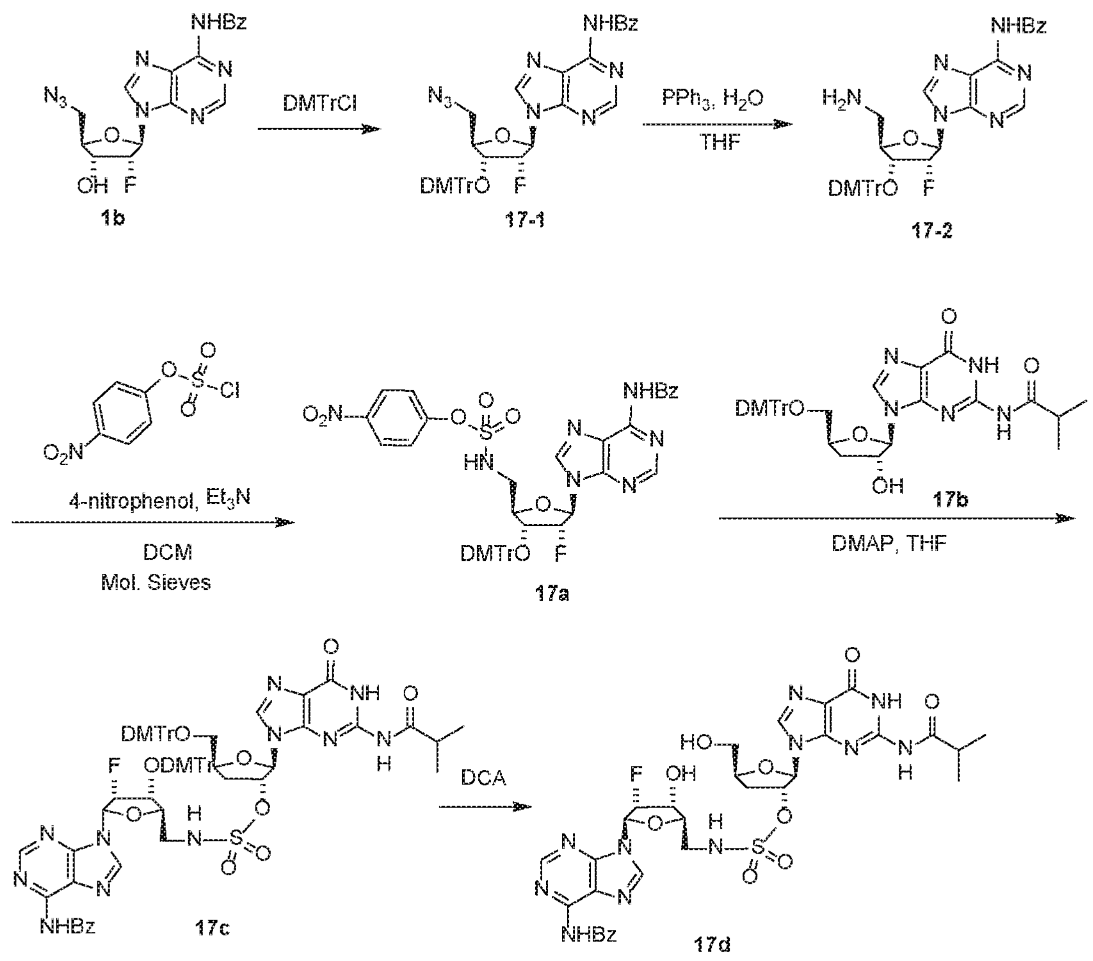
1b
DMTrCl
17-1
PPh3, H2O
THF
17-2
4-nitrophenol, Et3N
DCM
Mol. Sieves
17a
17b
DMAP, THF
17c
DCA
17d

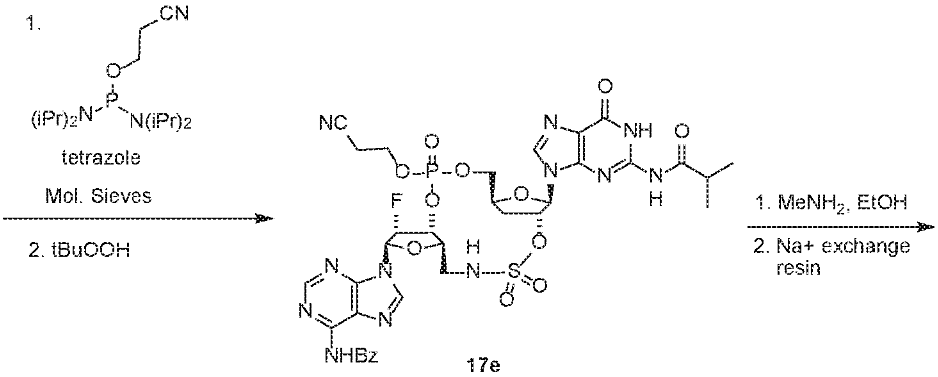


17e

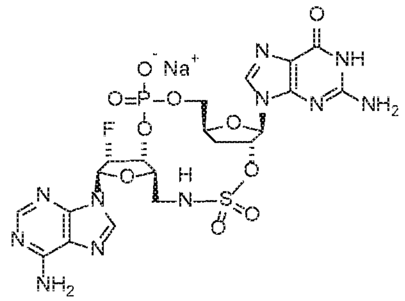

Compound 33, sodium salt

Step 1: preparation of Compound **17-1**

NOTE: compound **1b** was co-evaporated with pyridine (60 mL) twice before use.

To a solution of compound **1b** (6g, 15.06 mmol) in pyridine (60 mL) was added DMTrCl (10.2 g, 30.12 mmol) and DMAP (920 mg, 7.53 mmol) at 5 °C. The reaction mixture was stirred at 80 °C for 18 hr resulting in a yellow solution. The reaction mixture was combined with another batch and worked-up. Volatile was removed under vacuum and the residue was dissolved in DCM (150 mL), then slowly poured into aqueous saturated $NAHCO_3$ (100 mL) with vigorous stirring. The aqueous layer was extracted with DCM (100 mLx2). Organic layers were combined, dried over anhydrous $Na_2SO_4$, filtrated and concentrated under reduced pressure to give a yellow residue. The residue was purified by flash chromatography on silica gel (0-100% EtOAc in Petroleum ether) to give compound **17-1** (12.6 g, 88% from 7.9 g of compound **1b**) as a yellow solid. ESI-MS: m/z = 701.1 $[M+H]^+$.

Step 2: preparation of Compound **17-2**

$PPh_3$ (6.6 g, 25.1 mmol) was added to a solution of compound 17-1 (12.6 g, 17.98 mmol) in THF (100 mL) in one portion; the mixture was stirred at 40 °C for 2 hr under $N_2$, followed by addition of $H_2O$ (50 mL); the mixture was stirred for 12 hr resulting in a colorless solution. Solvent was concentrated under reduced pressure and the residual aqueous layer was partitioned between $DCMZH_2O$ (80/30 mL). The aqueous layer was was extracted with DCM (40 mLx2). Organic layers were then combined, dried over anhydrous $Na_2SO_4$, filtrated and concentrated under reduced pressure to give a white solid purified by flash column chromatography on silica gel (0-5% MeOH in DCM) to give compound **17-2** (11.6 g) as a white solid. $^1H$ NMR (400 MHz, $CDCl_3$) $\delta$ 8.93 (br s, 1H), 8.71 (s, 1H), 8.31 (s, 1H), 8.01 (br d, *J*= 7.3 Hz, 2H), 7.33 - 7.19 (m, 4H), 6.82 (dd, *J*= 6.9, 8.9 Hz, 4H), 6.17 (dd, *J*= 1.5, 17.6 Hz, 1H), 4.64 (ddd, *J*= 4.4, 7.6, 19.4 Hz, 1H), 4.57 - 4.36 (m, 1H), 4.18 - 4.08 (m, 1H), 3.77 (d, *J*= 5.3 Hz, 6H), 2.94 (dd, *J*= 2.4, 14.2 Hz, 1H), 2.64 (dd, *J*= 4.3, 14.3 Hz, 1H); $^{19}F$ NMR (376 MHz, $CDCl_3$) $\delta$ -197.03 (br s, IF); ESI-MS: m/z = 675.1 $[M+H]^+$.

Step 3: preparation of Compound **17a**:

A solution of 4-nitrophenyl chlorosulfate (768 mg, 3.23 mmol) in dry $CH_2Cl_2$ (5 mL) was added rapidly to a mixture of compound **17-2** (727 mg, 1.07 mmol), 4-nitrophenol (449 mg, 3.23 mmol), $Et_3N$ (654 mg, 6.46 mmol) and activated 4Å molecular sieves ($\approx$ 1 g) in dry $CH_2Cl_2$ (15 mL) under $N_2$ at -78 °C. The mixture was warmed to RT gradually

over 1.5 hr. The reaction mixture was combined with other batches and filtered through a pad of diatomaceous earth. The filtrate was transferred to a separatory funnel, washed with aqueous saturated $NaHCO_3$ (200 mLx4). The organic layer was dried over anhydrous $Na_2SO_4$, filtrated and concentrated under reduced pressure to give a yellow residue purified by flash column chromatography on silica gel (0-100% EtOAc in petroleum ether) to give compound **17a** (16 g) as a light yellow solid. $^1H$ NMR (400 MHz, $CDCl_3$) δ 8.93 - 8.81 (m, 2H), 8.41 (s, 1H), 8.11-7.92 (m, 5H), 7.67 -7.58 (m, 1H), 6.86 (br t, *J* = 7.7 Hz, 4H), 6.20 (br dd, *J* = 5.1, 13.7 Hz, 1H), 5.34 - 5.23 (m, 2H), 5.16 (br t, *J* = 5.1 Hz, 1H), 4.73 (br s, 1H), 3.90(br s, 1H), 3.79 (d, *J* = 6.4 Hz, 6H), 3.23 (br d, J=13.2 Hz, 1H), 2.89 (br dd, *J* = 8.7,12.6 Hz, 1H); $^{19}F$ NMR (376MHz, $CDCl_3$) δ -199.28 - -205.90 (m, 1F); ESI-MS: m/z = 876.1 $[M+H]^+$.

Step 4: preparation of compound **17c**

A solution of **17a** (2053 mg, 2.34 mmol), compound **17b** (1000 mg, 1.56 mmol) and molecular sieves (6 g) in THF (30 mL) was stirred under $N_2$ for 30 min at RT; DMAP (954.8 mg, 7.81 mmol) was added and the mixture was stirred at 45 °C (oil temperature) for 12 hr. The reaction mixture was filtered and to it was added DCM (50 mL) and brine (20 mL). The organic layer was washed with aqueous saturated $NaHCO_3$ (3 × 50 mL), dried over anhydrous $Na_2SO_4$, filtered and evaporated under reduced pressure to give a residue. The residue was combined with silica gel (6 g) was purified by flash column chromatography over silica gel (PE/ EA from 10% to 100% and DCM/MeOH = 0% to 5%) to give **17c** (1.8 g) as a light yellow solid. $^1H$ NMR (400 MHz, DMSO-$d_6$) δ 12.10 (s, 1H), 11.46 (s, 1H), 11.23 (s, 1H), 11.05 (s, 1H), 8.65 (s, 1H), 8.47 (s, 1H), 8.13-8.10 (m, 1H), 8.02 (d, J=7.3 Hz, 2H), 7.68 -7.63 (m, 1H), 7.58-7.46 (m, 4H), 7.41-7.31 (m, 6H), 7.29-7.23 (m, 3H), 7.20-7.11 (m, 7H), 6.91 (dd, J=6.4, 8.3 Hz, 4H), 6.75 (dd, J=8.9, 12.3 Hz, 4H), 6.39-6.30 (m, 1H), 6.16 (s, 1H), 5.34 (br, d, J=5.9 Hz, 1H), 4.91 (br, s, 1H), 4.86 - 4.73 (m, 2H), 4.45 (br, d, J=5.4 Hz, 1H), 3.69 (s, 12H), 3.21-3.08 (m, 2H), 2.96-2.88 (m, 1H), 2.73 (m, 2H), 2.07 (m, 1H), 1.02 (m, 6H); $^{19}F$ NMR (376 MHz, DMSO-$d_6$) δ -197.64 (s, IF); ESI-MS: m/z=689.1 $[M/2+H]^+$.

Step 5: preparation of compound **17d**

To a solution of **17c** (1.8 g, 1.31 mmol) in DCM (30 mL) was added water (235.5 mg, 13.08 mmol) and DCA (337.233 mg, 2.615 mmol) resulting in a red solution. The solution was stirred at 25 °C for 12 hours at RT. It was then added MeOH (5 mL) until the solution was clear followed by pyridine (1034.4 mg, 13.08 mmol). After stirring the mixture at 25 °C for 2 hours, the solution was concentrated under pressure to give a residue (3.0 g). The residue was combined with silica gel (6.0 g) and purified by flash column chromatography over silica gel (0-8% DCM in MeOH) to give **17d** (0.9 g) as a white solid. $^1H$ NMR (400 MHz, DMSO-$d_6$) δ 12.12 (s, 1H), 11.57 (s, 1H), 11.26 (s, 1H), 8.75 (s, 1H), 8.60 (s, 1H), 8.47 (t, J=5.9 Hz, 1H), 8.21 (s, 1H), 8.07-8.02 (m, 2H), 7.69 -7.63 (m, 1H), 7.60-7.52 (m, 2H), 6.33 (dd, J=2.0, 19.3 Hz, 1H), 6.08 (d, J=1.7 Hz, 1H), 5.92 (d, J=6.4 Hz, 1H), 5.70-5.50 (m, 1H), 5.30 (br, d, J=6.1 Hz, 1H), 5.02 (t, J=5.4 Hz, 1H), 4.67-4.53 (m, 1H), 4.27 (br, d, J=3.9 Hz, 1H), 4.11 (q, J=5.1 Hz, 1H), 4.05-3.99 (m, 1H), 3.66-3.55 (m, 1H), 3.46 (td, J=4.9, 12.2 Hz, 1H), 2.78 (td, 1H), 2.24 (td, 1H), 1.12 (d, 6H);
$^{19}F$ NMR (376 MHz, DMSO-$d_6$) δ -202.156 (s, 1F); ESI-MS: m/z = 772.1 $[M+H]^+$ .

Step 5: preparation of compound **17e**

THF was freshly distilled over Na/benzophenone and $CH_3CN$ was freshly distilled over $CaH_2$ before use.

To a solution of **17e** (100 mg, 0.130 mmol) in tetrahydrofuran (3 mL) was added 4Å MS (powder, 1g) and the mixture was stirred for 20 min. After 20 min, a solution of 1H-tetrazole (0.45 M in acetonitrile, 2.3 mL) was added at 25 °C, followed by addition of a solution of 2-cyanoethyl-*N,N,N',N'*-tetra(isopropyl)phosphorodiamidite (78.1 mg, 0.26 mmol, diluted in 1 mL of THF) at 25 °C. After stirring for 1.5 hr at RT, tert-butyl hydroperoxide (0.12 mL, 0.65 mmol) was added at 25 °C and the solution was stirred for 1.5 h. The reaction mixture was concentrated under pressure to give a yellow residue. The residue was combined with silica gel (2 g) and purified by flash column chromatography over silica gel (0-15% MeOH in DCM) to give 17e (80 mg) as a light yellow solid. ESI-MS: *m/z* = 887.5 $[M+H]^+$.

Step 4: Preparation of **compound 33, sodium salt**

Compound **17e** (80 mg, 0.090 mmol) was treated with $MeNH_2$ (50% in EtOH, 5.0 mL). After stirring at 25 °C for 4 hours, the solution was concentrated under pressure to give a residue. The residue was purified by reverse phase preparative HPLC (Column: Agela Durashell 5μm C18 150x25, Condition: water (10 mM $NH_4HCO_3$)-$CH_3CN$ Begin B: 5, End B 35, Gradient Time (min): 9, 100%B Hold Time(min): 0, Flow Rate (mL/min): 25) to give **compound 33, ammonium salt** (15 mg) as a white solid. Final conversion into the sodium salt was done by elution of an aqueous solution over a column packed with a cationic solium ion-exchange resin to afford **compound 33, sodium salt** as a white fluffy solid after lyophilization (25.5 mg) .

$^1H$ NMR (400 MHz, $D_2O$) $\delta$ ppm 8.16 (br, s, 1H), 7.88 (s, 1H), 7.15 (br, s, 1H), 6.45 (br, d, *J* = 19.8 Hz, 1H), 6.19 (br, d, *J* = 7.3 Hz, 1H), 6.00 (br, d, *J* = 7.1 Hz, 1H), 5.43-5.29 (m, 1H), 5.28-5.18 (m, 1H), 4.63-4.51 (m, 2H), 4.31-4.22 (m, 1H), 4.16-4.04 (m, 1H), 3.78 (br d, *J* = 13.2 Hz, 1H), 3.45 (br, d, *J* = 13.2 Hz, 1H), 2.80-2.60 (m, 2H); $^{19}F$ NMR (376 MHz, $D_2O$) $\delta$ ppm -196.95 (s, IF); $^{31}P$ NMR (162 MHz, $D_2O$) $\delta$ ppm -1.422 (s, IP); ESI-MS: m/z=660.2 [M+H]$^+$.

Example 18

Compound 34

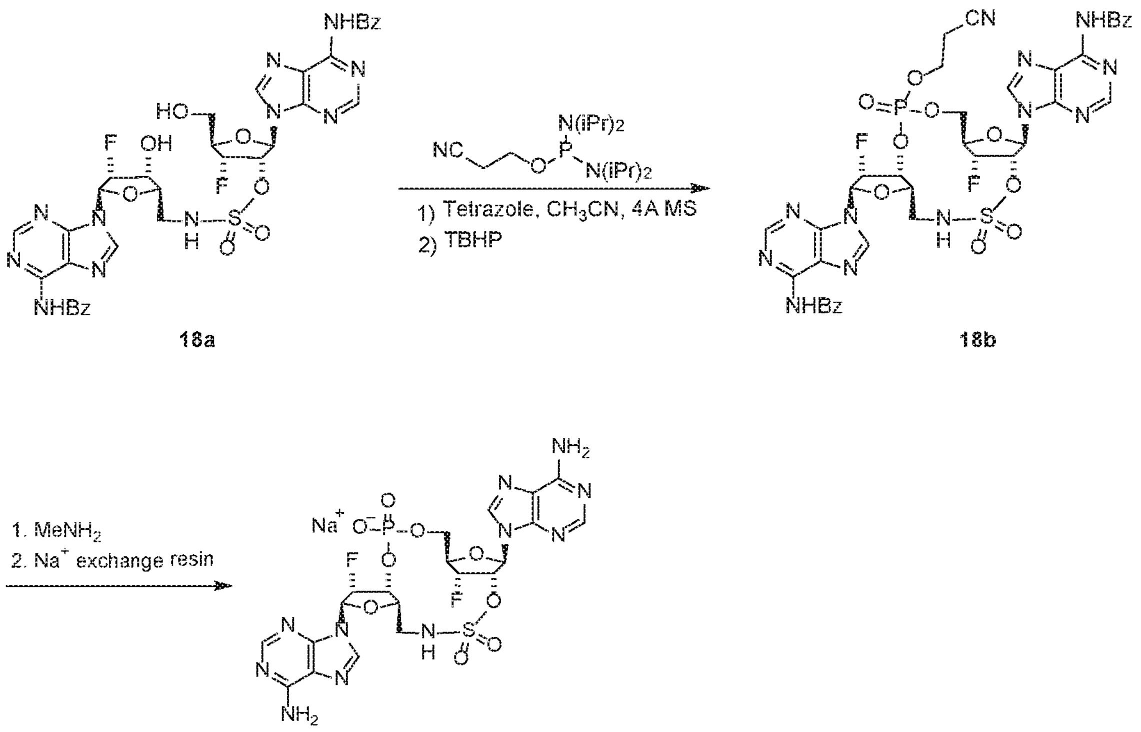


Compound 34, sodium salt

Step 1: preparation of compound **18b**

THF was freshly distilled over Na/benzophenone and $CH_3CN$ was freshly distilled over $CaH_2$ before use.

To a solution of **18a** (100 mg, 0.127 mmol) in THF (2 mL), was added 4A MS (powder, 0.5 g) and a solution of 1H-tetrazole (2.25 mL, 0.45 M, prepared by dissolved 945 mg of tetrazole in 30 mL of dry $CH_3CN$, followed by addition of 1 g of 4A MS and then stirred for 1hr under $N_2$ before use); then purged with $N_2$ several times. A solution of 2-cyanoethyl-N,N,N',N'-tetra(isopropyl)phosphorodiamidite (61 mg, 0.203 mmol) in THF (0.6 mL) was added drop-wise over 5 min. After stirring for 4 hr at RT, a solution of TBHP (124 μl, 0.62 mmol, 5M in decane) was added under $N_2$. After stirring for 30 min, the reaction mixture was combined with a previous batch and filtered through a pad of diatomaceous earth and the filtrate concentrated under reduced pressure to give a colorless oil. The oil was purified by flash column chromatography over silica gel (12 g, 35 mL/min, firstly eluted with 50-80% THF in petroleum ether, then switched to 0-2% MeOH in DCM) to give **18b** as a white solid (89 mg). $^1H$ NMR (400 MHz, CHLOROFORM-d) δ 8.82 (s, 2H), 8.60 (s, 1H), 8.08 (s, 1H), 7.95 (br d, J=7.5 Hz, 2H), 7.81 (br s, 2H), 7.60 - 7.50 (m, 1H), 7.47 - 7.37 (m, 3H), 7.37 - 7.28 (m, 2H), 6.95 (s, 1H), 6.36 (br d, *J* = 17.6 Hz, 2H), 6.23 - 5.80 (m, 2H), 5.67 - 5.39 (m, 1H), 4.87 - 4.56 (m, 3H), 4.53 - 4.37 (m, 3H), 3.84 - 3.67 (m, 2H), 3.48 - 3.32 (m, 1H), 2.87 (br t, *J* = 5.9 Hz, 4H); $^{31}P$ NMR (162 MHz, CHLOROFORM-d) δ -5.30 (s, IP); ESI-MS: m/z = 923.4 [M+H] $^+$.

Step 2: preparation of **compound 34, sodium salt**

**Compound 18b** (98 mg, 0.096 mmol) was treated with a solution of methyl amine in EtOH (30% in EtOH, 5mL). After stirring at 40°C for 1 h, volatile solvents were evaporated under reduced pressure. The resulting white solid was dissolved in a mixture of water/$CH_3CN$ (15/4 mL), then washed with DCM (10 mL x3). The aqueous layer was lyophilized to give **compound 34** (61 mg) as a white solid. ESI-MS: m/z = 662.2 $[M+H]^+$.

The solid was purified by reverse phase preparative HPLC (Column: Xbridge 10μm 150 × 30mm, Condition: water (10 mM $NH_4HCO_3$)-$CH_3CN$ Begin B: 8, End B 38, Gradient Time(min): 7, 100%B Hold Time(min): 0, Flow Rate (mL/min): 25) to give **compound 34, ammonium salt** as a white solid. ESI-MS: m/z = 662.3 $[M+H]^+$; $^1H$ NMR (400 MHz, D2O) δ 8.50 - 8.11 (m, 1H), 8.08 - 7.86 (m, 1H), 7.81 - 7.56 (m, 1H), 6.95 - 6.58 (m, 1H), 6.41 - 6.01 (m, 2H), 5.76 - 5.05 (m, 4H), 5.01 - 4.76 (m, 1H), 4.45 - 3.93 (m, 3H), 3.81 - 3.23 (m, 2H); $^{19}F$ NMR (376 MHz, D2O) δ-196.50 -197.49, -197.63, -198.85; $^{31}P$ NMR (162 MHz, D2O) δ-2.25 (1s, IP)

Preparation of **compound 34, sodium salt**

Salt conversion: Dowex 50W × 8, 200-400 (6 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 ×). The resin was transferred to the column and washed with 15% NaOH in water (at least 4 CV), and then with deionized water until it was neutral. **Compound 34 TEAA salt** (44 mg) was dissolved in in a mixture of deionized water: $CH_3CN$ (4:1), added to the top of the column, and eluted with deionized water. Appropriate fractions were pooled together and lyophilized to **Compound 34 sodium salt** (43.5 mg) as a white solid. $^1H$ NMR (400 MHz, D2O) δ ppm 8.29 (s, 1H), 7.98 (s, 1H), 7.68 (s, 1H), 6.75 (br s, 1H), 6.31 - 6.17 (m, 2H), 5.73 - 5.58 (m, 1H), 5.57 - 5.39 (m, 1H), 5.31 - 5.12 (m, 1H), 4.96 - 4.82 (m, 1H), 4.68 (br d, *J*= 2.4 Hz, 1H), 4.33 (br d, *J* = 9.0 Hz, 1H), 4.25 - 4.06 (m, 2H), 3.62 (dd, *J*= 2.4, 13.4 Hz, 1H), 3.43 (br d, *J*= 13.0 Hz, 1H); $^{31}P$ NMR (162MHz, D2O) δ ppm -2.15 (s, 1P); $^{19}F$ NMR (376 MHz, D2O) δ ppm -196.86 (s, IF), -198.50 (s, IF); ESI-MS: m/z = 662.2 $[M+H]^+$.

Example 19

## Compound 12

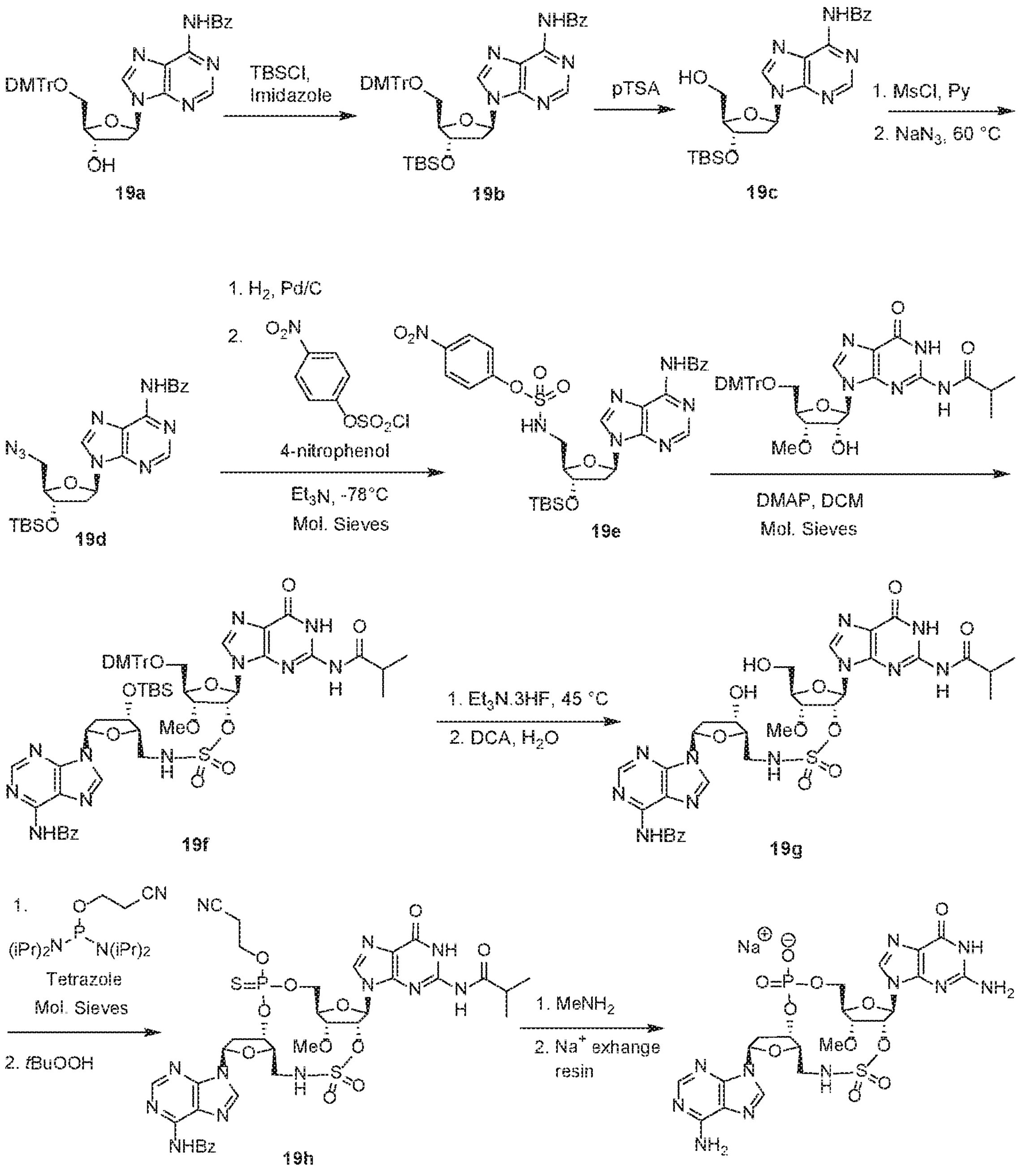


**Compound 12, sodium salt**

Step 1: preparation of compound **19b**

Imidazole (31 g, 456.1 mmol) and TBSCl (45.8 g, 304.0 mmol) were successively added to a solution of N6-Benzoyl-5'-O-(4,4'-dimethoxytrityl)-2'-deoxy adenosine (19a, [64325-78-6], 50.0 g, 76.0 mmol) in dry DMF (750 mL). The mixture was stirred at room temperature until complete conversion (ca. 3 h) after which the reaction solution was diluted with EtOAc and washed with water. The organic layer was dried over anhydrous $Na_2SO_4$ filtered and concentrated under reduced pressure. The obtained crude residue was re-precipitated with DCM and hexane to get compound **19b** as a white foam (68 g, crude). ESI-MS: *m/z* 771.0 [M+H]$^+$.

Step 2: preparation of compound **19c**

Compound **19b** (68.0 g, 88.3 mmol) was dissolved in $CHCl_3$ (1564 mL) and cooled to 0° C. A solution of pTSA monohydrate (20.1 g, 105.9 mmol) in methanol was added. The reaction mixture was stirred for 30 min after which saturated aqueous $NaHCO_3$ was added for quenching. The aqueous layer was extracted with DCM. The combined organic phases were dried over anhydrous $Na_2SO_4$. filtered and concentrated under reduced pressure. Purification was done by column chromatography over silica gel (gradient elution: 0 - 1% MeOH in DCM) to give compound **19c** as an off-white foam (27 g). $^1$H NMR (500 MHz, DMSO-$d_6$) δ ppm: 11.20 (s, 1H), 8.75 (s, 1H), 8.69 (s, 1H), 8.05 (d, *J*= 7.6 Hz, 2H), 7.65 (t, *J* = 7.6 Hz, 1H), 7.55 (t, *J* = 7.6 Hz, 2H), 6.47 (t, *J* = 6.9 Hz, 1H), 5.06 (t, *J* = 5.5 Hz, 1H), 4.65 (m, 1H), 3.90 (q, *J* = 4.1 Hz, 1H), 3.63 (m, 1H), 3.53 (m, 1H), 2.92 (m, 1H), 2.36 (qd, *J*= 6.4, 2.8 Hz, 1H), 0.92 (s, 9H), 0.13 (s, 6H); ESI-MS: *m*/*z* 470.0 $[M+H]^+$.

Step 3: preparation of compound **19d**

Mesyl chloride (5.1 mL, 66.7 mmol) was added dropwise to a solution of compound **19c** (27.0 g, 57.5 mmol) in dry pyridine (135 mL) at 0° C. The reaction mixture was stirred at 0° C until complete conversion (ca. 3 h) after which it was quenched with methanol and concentrated under reduced pressure. The obtained residue was dissolved in EtOAc and washed with saturated aqueous $NaHCO_3$. The organic layer was dried over anhydrous $Na_2SO_4$, filtered and concentrated under reduced pressure to give the mesylated product. The crude product was dissolved in dry DMF (256 mL) followed by the addition of sodium azide (30.5 g, 468.0 mmol). The reaction mixture was stirred at 60° C for 5 h after which it was cooled to room temperature, diluted with EtOAc, and washed with saturated aqueous $NaHCO_3$ and water. The organic layer was dried over anhydrous $Na_2SO_4$, filtered and concentrated under reduced pressure. Purification was done by column chromatography over silica gel (gradient elution: 0 - 1% MeOH in DCM) to give compound **19d** as a foam (24 g, yield: 84%). $^1$H NMR (500 MHz, DMSO-$d_6$) δ ppm: 11.19 (s, 1H), 8.77 (s, 1H), 8.70 (s, 1H), 8.05 (d, *J* = 6.9 Hz, 2H), 7.65 (t, *J* = 7.2 Hz, 1H), 7.55 (t, *J*= 7.6 Hz, 2H), 6.51 (t, *J*= 6.9 Hz, 1H), 4.71 (m, 1H), 4.01 (m, 1H), 3.65 (q, *J*= 6.4 Hz, 1H), 3.57 (dd, *J*= 13.1, 4.8 Hz, 1H), 3.06 (m, 1H), 2.41 (qd, *J*= 6.7, 4.0 Hz, 1H), 0.92 (s, 9H), 0.14 (s, 6H); ESI-MS: *m*/*z* 495.0 $[M+H]^+$.

Step 4: preparation of compound **19e**

A solution of compound **19d** (10.0 g, 20.2 mmol) in MeOH (100 mL) was hydrogenated under atmospheric pressure at room temperature on Pd/C (20% on carbon, 1 g). The reaction mixture was filtered over diatomaceous earth, the diatomaceous earth was rinsed with MeOH. The filtrate was concentrated under reduced pressure to give the compound amine as a white foam (9.4 g, crude). The crude product (9.4 g) was dissolved in DCM (395 mL), followed by the addition of 4-nitrophenol (8.38 g, 60.2 mmol), $Et_3N$ (16.9 mL, 120.4 mmol) and activated molecular sieves. The resulting mixture was cooled to -78°C under $N_2$ after which a solution of 4-nitrophenyl chlorosulfate (14.28 g, 60.2 mmol) in DCM (45 mL) was added dropwise, stirring was continued until complete conversion (ca. 2 h). The reaction mixture was warmed to room temperature and washed with saturated aqueous $NaHCO_3$ and water. The organic phase was dried with anhydrous $Na_2SO_4$, filtered and concentrated under reduced pressure. The crude product was purified by silica column chromatography (gradient elution: 0 - 50% EtOAc in hexane) to give compound **19e** as a foam (6.4 g, yield: 47%). $^1$H NMR (500 MHz, DMSO-$d_6$) δ ppm: 11.20 (s, 1H), 9.09 (t, *J* = 5.9 Hz, 1H), 8.70 (d, *J*= 2.1 Hz, 2H), 8.29 (td, *J*= 6.2, 3.9 Hz, 2H), 8.05 (d, *J*= 7.6 Hz, 2H), 7.65 (t, *J*= 7.2 Hz, 1H), 7.54 (m, 4H), 6.50 (t, *J*= 6.9 Hz, 1H), 4.66 (m, 1H), 4.01 (m, 1H), 3.51 (dt, *J*= 14.2, 5.7 Hz, 1H), 3.39 (m, 1H), 3.05 (m, 1H), 2.38 (qd, *J*= 6.7, 2.8 Hz, 1H), 0.91 (s, 9H), 0.13 (s, 6H); ESI-MS: m/z 670.0 $[M+H]^+$

Step 5: preparation of compound **19f**

5'-O-(4,4'-Dimethoxytrityl)-N2-isobutyryl-3'-O-methyl-D-guanosine ([103285-33-2], 2.5 g, 3.73 mmol), compound **19e** (2.9 g, 4.48 mmol) and DMAP (2.27 g, 18.6 mmol) were each separately dissolved in dry DCM (3 x 10.0 mL) and dried on activated 3Å molecular sieves for at least 2 h under an inert atmosphere. Next, the 5'-O-(4,4'-dimethoxytrityl)-N2-isobutyryl-3'-O-methyl-D-guanosine and compound **22c** solutions were successively transferred to the reaction flask containing the DMAP solution. The reaction mixture was stirred for 22 h. The molecular sieves were removed by filtration and thoroughly washed with dichloromethane. The filtrate was washed with saturated aqueous $NaHCO_3$, brine and saturated aqueous $NH_4Cl$. The combined organic phases were dried over $Na_2SO_4$, filtered, and concentrated under reduced pressure. The residue was purified by silica column chromatography (gradient elution: 0 - 1% MeOH in DCM) to give compound **19f** (2.04 g, yield: 45.5%). $^1$H NMR(500 MHz, DMSO-$d_6$) δ ppm: 12.10 (s, 1H), 11.54 (s, 1H), 11.20 (s, 1H), 8.70 (s, 1H), 8.65 (s, 1H), 8.18 (s, 1H), 8.05 (d, *J* = 6.9 Hz, 2H), 7.65 (t, *J* = 7.2 Hz, 1H), 7.56 (t, *J*= 7.6 Hz, 2H), 7.29 (d, *J*= 7.6 Hz, 2H), 7.24 (t, *J* = 7.6 Hz, 2H), 7.18 (t, *J* = 9.6 Hz, 5H), 6.81 (dd, *J* = 8.3, 6.2 Hz, 4H), 6.46 (t,

*J* = 6.9 Hz, 1H), 6.16 (d, *J* = 4.1 Hz, 1H), 5.55 (t, *J* = 4.8 Hz, 1H), 4.63 (m, 1H), 4.38 (t, *J* = 5.2 Hz, 1H), 4.12 (q, *J*= 4.6 Hz, 1H), 3.86 (dd, *J*= 9.0, 6.2 Hz, 1H), 3.71 (s, 6H), 3.37 (s, 3H), 3.23 (m, 5H), 2.97 (m, 1H), 2.74 (m, 1H), 1.09 (m, 6H), 0.88 (s, 9H), 0.11 (s, 6H); ESI-MS: *m/z* 1201.0 $[M+H]^+$.

Step 6: preparation of compound **19g**

$Et_3N$ (11.61 mL, 83.3 mmol) and $Et_3N.3HF$ (1.35 mL, 8.33 mmol) were added to a solution of compound **19f (2.5** g, 2.08 mmol) in pyridine (16.5 mL). The reaction mixture was stirred at 45 °C until complete conversion (ca. 5 h) and then cooled to room temperature. Isopropoxytrimethylsilane (5.9 mL, 33.28 mmol) was added and stirring was continued overnight. Concentration under reduced pressure gave the crude 5'-deprotected compound which was re-dissolved in DCM (59.0 mL). Water (0.17 mL, 9.65 mmol) and dichloroacetic acid (6.3 mL of 10% in DCM, 7.72 mmol) were added, the resulting reaction mixture was stirred for 1 h (full conversion) after which it was quenched by the addition of pyridine (0.77 mL, 9.65 mmol) and some drops of methanol. The residue obtained after concentration under reduced pressure was purified by column chromatography over silica gel (gradient elution: 0 - 9% MeOH in DCM) to give compound **19g** (1.4 g, yield: 86%). $^1$H NMR (500 MHz DMSO-$d_6$) δ ppm: 12.08 (s, 1H), 11.62 (s, 1H), 11.19 (s, 1H), 8.70 (s, 1H), 8.60 (s, 1H), 8.25 (s, 1H), 8.05 (d, *J* = 7.6 Hz, 2H), 7.65 (t, *J*= 7.2 Hz, 1H), 7.56 (t, *J*= 7.6 Hz, 2H), 6.42 (t, *J* = 6.9 Hz, 1H), 6.08 (d, *J* = 6.2 Hz, 1H), 5.45 (d, *J* = 4.1 Hz, 1H), 5.38 (dd, *J*= 6.5, 5.2 Hz, 1H), 5.26 (t, *J*= 5.2 Hz, 1H), 4.36 (d, *J* = 2.1 Hz, 1H), 4.18 (q, *J*= 2.5 Hz, 1H), 4.10 (m, 1H), 3.79 (td, *J*= 5.9, 3.2 Hz, 1H), 3.61 (dtd, *J* = 37.3, 8.3, 3.8 Hz, 2H), 3.41 (s, 3H), 3.09 (d, *J* = 15.1 Hz, 3H), 2.83 (m, 1H), 2.74 (td, *J* = 13.4, 6.7 Hz, 1H), 2.33 (qd, *J* = 6.7, 3.6 Hz, 1H), 1.11 (q, *J* = 3.4 Hz, 6H); ESI-MS: m/z 784.0 $[M+H]^+$.

Step 7: preparation of compound **19h**

A solution of compound **19g** (500 mg, 0.638 mmol) and 1H-tetrazole (5.59 mL of a 3 - 4% in MeCN) in dry THF (26 mL) was treated with 3Å molecular sieves for 2 h under inert atmosphere. 2-Cyanoethyl-N,N,N',N'-tetra(isopropyl)phosphorodiamidite (250 mg, 0.829 mmol) was added in one portion and the reaction mixture was shaken overnight. tBuOOH (174 μL of 5.5 M solution in decane, 0.96 mmol) was added and shaking was continued for 1 h. The molecular sieves were removed by filtration and extensively rinsed with DCM The filtrate was concentrated under reduced pressure to give crude compound **19h** which was used directly in the next step. ESI-MS: *m/z* 899.5 $[M+H]^+$.

Step 8: preparation of **compound 12, sodium salt**

Crude compound **19h** was stirred in a 33% methylamine solution in ethanol (10 mL) at room temperature for 2 h. The reaction mixture was concentrated under reduced pressure. The crude product was triturated in MeCN followed by preparative reversed phase HPLC purification (Stationary phase: XBridge C18 OBD, 5 μm, 250 x 30 mm; Mobile phase: aqueous 0.25% ammonia bicarbonate (A) - MeOH (B); gradient elution) to give **compound 12, ammonium salt**. Conversion into the sodium salt was done by elution of an aqueous solution over a column packed with IR Na ion-exchange resin to give **compound 12, sodium salt** as a white fluffy solid after lyophilization (43 mg, yield: 10%). $^1$H NMR (600 MHz, DMSO-$d_6$, 60 °C) δ ppm 10.36 (br s, 1 H), 8.54 (br s, 1 H), 8.33 (s, 1 H), 7.99 (s, 1 H), 7.98 (s, 1 H), 7.11 (s, 2 H), 6.76 (br s, 2 H), 6.35 (t, J=6.9 Hz, 1 H), 5.98 (br s, 2 H), 5.14 (br s, 1 H), 4.28 (br s, 2 H), 4.19 (s, 1 H), 4.09 (s, 1 H), 3.86 - 3.95 (m, 1 H), 3.48 (s, 3 H), 3.34 - 3.46 (m, 1 H), 3.09 - 3.26 (m, 1 H), 2.87 - 2.99 (m, 1 H), 2.56 - 2.65 (m, 1 H); $^{31}$P NMR (162 MHz, DMSO-$d_6$) δ ppm -1.88 (s, 1 P);
ESI-MS: *m/z* 670.2 $[M-H]^-$.

Example 20

## Compound 36

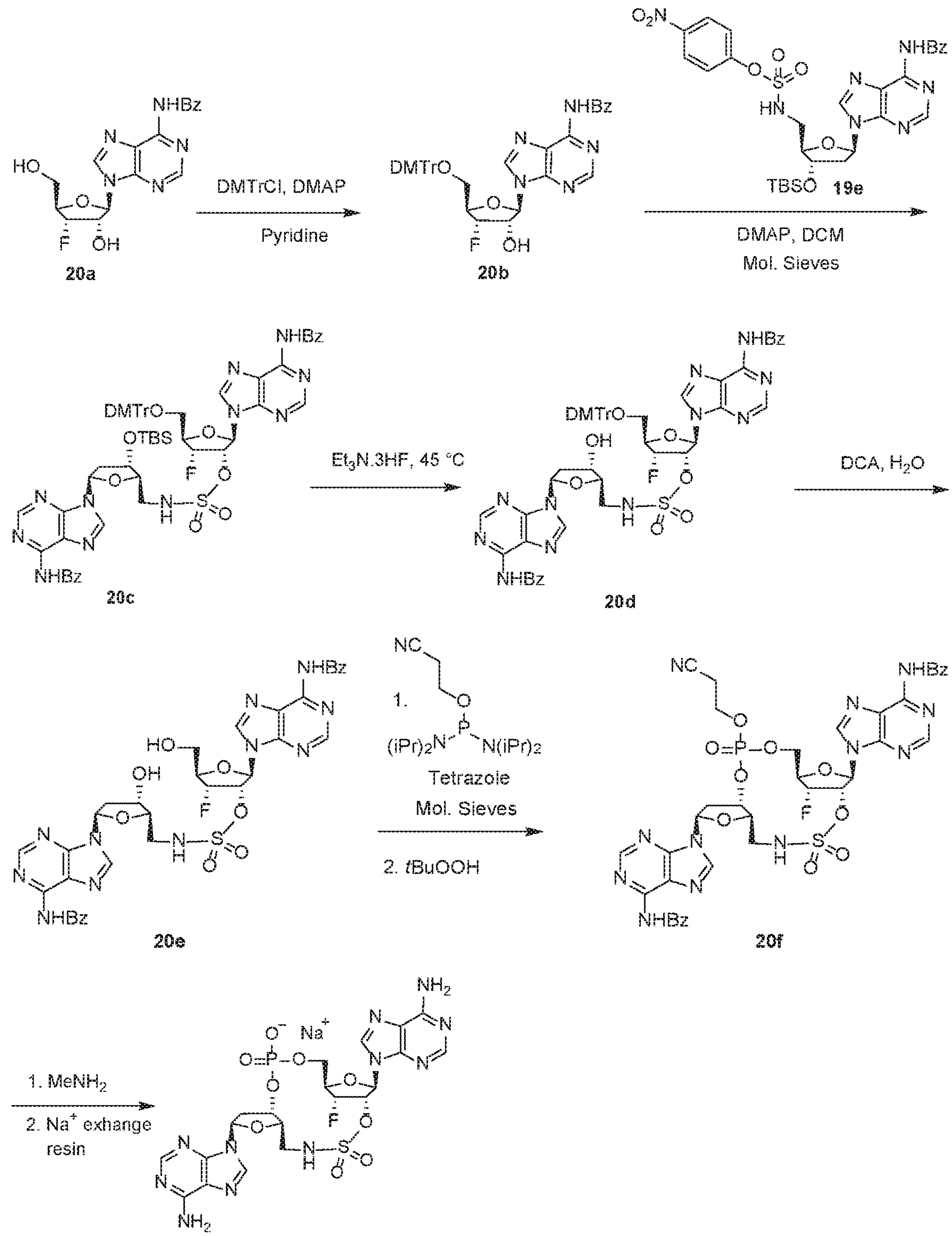


20a, 20b, 20c, 20d, 20e, 20f

**Compound 36, sodium salt**

Step 1: Preparation of compound **20b**

A solution of N-benzoyl-3'-deoxy-3'-fluoro-adenosine **20a** [CAS 129054-67-7] (7.4 g, 18.5 mmol) in dry pyridine (138 mL), to which DMAP (1.13 g, 9.2 mmol) and DMTrCl (10 g, 29.7 mmol) (portionwise) were added, was stirred at

room temperature until complete conversion (5 h). The reaction mixture was quenched with methanol (15 mL) and concentrated under reduced pressure. The obtained residue was dissolved in ethyl acetate and washed with water. The organic layer was dried over anhydrous $Na_2SO_4$, filtered, and concentrated under reduced pressure. Purification was done by column chromatography over silica gel (gradient elution: 0 - 2% MeOH in DCM) to give compound **20b** as a white foam (9.8 g, yield: 78%). $^1$H NMR (500 MHz DMSO-$d_6$) δ ppm: 3.31 (q, *J*=5.0 Hz, 1 H), 3.36 (t, J=5.2 Hz, 1 H), 3.73 (s, 6 H), 4.41 (dt, J=26.4, 4.6 Hz, 1 H), 5.19 (m, 1 H), 5.28 (dd, J=11.7, 7.6 Hz, 1 H), 6.09 (dd, *J*=6.9, 4.8 Hz, 2 H), 6.87 (m, 4 H), 7.24 (m, 7 H), 7.39 (d, J=6.9 Hz, 2 H), 7.56 (t, J=7.6 Hz, 2 H), 7.65 (m, 1 H), 8.06 (d, J=6.9 Hz, 2 H), 8.62 (d, J=3.4 Hz, 2 H), 11.27 (s, 1 H); ESI-MS: *m/z* 676.1 $[M+H]^+$.

Step 2: Preparation of compound **20c**

A reaction flask was charged with DMAP (2.62 g, 21.4 mmol), dry DCM (50 mL) and activated 3Å molecular sieves. The resulting mixture was stirred at room temperature for at least 2 h under inert atmosphere. Simultaneously, a solution of compound **20b** (2.9 g, 4.29 mmol) and a solution of compound **19e** (3.4 g, 5.15 mmol), each in dry DCM (2 x 50 mL), were dried on activated 3Å molecular sieves (ca. 2 h). Both solutions (compound **20b** and compound 19c respectively) were successively transferred to the reaction flask. The resulting reaction mixture was stirred for 24 h. The molecular sieves were removed by filtration and thoroughly rinsed with dichloromethane. The filtrate was washed with saturated aqueous $NaHCO_3$, brine and saturated aqueous $NH_4Cl$, dried over $Na_2SO_4$, filtered, and concentrated under reduced pressure. The residue was purified by silica column chromatography (gradient elution: 0 - 1% MeOH in DCM) to give compound **20c** (4.2 g, yield: 81%). $^1$H NMR (500 MHz DMSO-*$d_6$*) δ ppm: 11.23 (d, *J*= 36.5 Hz, 2H), 8.70 (s, 1H), 8.63 (d, *J*= 20.0 Hz, 2H), 8.53 (s, 1H), 8.04 (q, *J*= 3.4 Hz, 4H), 7.65 (m, 2H), 7.55 (td, *J* = 7.6, 4.8 Hz, 4H), 7.35 (d, *J* = 7.6 Hz, 2H), 7.22 (m, 7H), 6.83 (m, 4H), 6.43 (q, *J* = 7.1 Hz, 2H), 6.05 (td, *J* = 11.0, 6.2 Hz, 1H), 5.64 (d, *J* = 54.4 Hz, 1H), 4.53 (m, 2H), 3.88 (t, *J* = 3.1 Hz, 1H), 3.70 (s, 6H), 3.37 (m, 2H), 3.20 (m, 2H), 2.97 (m, 1H), 0.88 (s, 9H), 0.09 (d, *J* = 2.1 Hz, 6H); ESI-MS: *m/z* 1206 $[M+H]^+$.

Step 3: Preparation of compound **20d**

$Et_3N$ (19.4 mL, 139.4 mmol) and $Et_3N.3HF$ (2.2 mL, 13.9 mmol) were added to a solution of compound **20d** (4.2 g, 3.4 mmol) in pyridine (70 mL). The reaction mixture was stirred at 45 °C until complete conversion (ca. 5 h) and then cooled to room temperature. Isopropoxytrimethylsilane (9.9 mL, 55.7 mmol) was added and stirring was continued overnight. Finally, the reaction mixture was concentrated under reduced pressure and purified by column chromatography over silica gel (gradient elution: 0 - 4% MeOH in DCM) to give compound **20d** as a foam (3.0 g, yield: 78%).

$^1$H NMR (500 MHz DMSO-$d_6$) δ ppm: 11.21 (s, 1H), 8.70 (s, 1H), 8.62 (d, *J*= 9.6 Hz, 2H), 8.53 (s, 1H), 8.04 (m, 4H), 7.65 (m, 2H), 7.55 (td, *J*= 7.7, 3.7 Hz, 4H), 7.36 (d, *J* = 7.6 Hz, 2H), 7.21 (m, 7H), 6.83 (m, 4H), 6.43 (dd, *J* = 16.2, 6.5 Hz, 2H), 6.04 (td, *J*= 11.0, 6.2 Hz, 1H), 5.66 (dt, *J*= 53.5, 3.4 Hz, 1H), 5.46 (s, 1H), 4.49 (dd, *J*= 24.1, 2.8 Hz, 1H), 4.39 (s, 1H), 3.88 (m, 1H), 3.71 (s, 6H), 3.40 (q, *J*= 5.3 Hz, 2H), 3.20 (m, 2H), 2.85 (q, *J*= 6.9 Hz, 1H); ESI-MS: *m/z* 1090 $[M+H]^+$.

Step 4: Preparation of compound **20e**

Compound **20d** (3.0 g, 2.7 mmol) was dissolved in DCM (84 mL), water (250 μL, 13.7 mmol) and dichloroacetic acid (9.1 mL of 10% in DCM, 10.9 mmol) were added. The resulting reaction mixture was stirred for 1 h (complete conversion) after which it was quenched by the addition of pyridine (1.1 mL, 13.7 mmol) and some drops of methanol. The residue obtained after concentration under reduced pressure was purified by silica gel column chromatography (gradient elution: 0 - 6% MeOH in DCM) to give compound **20e** (1.9 g, yield: 87%). $^1$H NMR (500 MHz DMSO-$d_6$) δ ppm: 11.28 (s, 1H), 11.21 (s, 1H), 8.72 *(d, J* = 4.1 Hz, 3H), 8.62 (s, 1H), 8.04 (d, *J* = 8.3 Hz, 4H), 7.64 (m, 2H), 7.55 (q, *J* = 6.9 Hz, 4H), 6.40 (m, 2H), 5.53 (t, *J*= 5.9 Hz, 1H), 5.42 (t, *J*= 5.2 Hz, 1H), 4.45 (dt, *J* = 26.9, 3.4 Hz, 1H), 4.35 (s, 1H), 3.79 (t, *J* = 7.2 Hz, 1H), 3.68 (d, *J* = 20.0 Hz, 2H), 3.10 (m, 3H), 2.84 (m, 1H), 2.32 (q, *J*= 3.4 Hz, 1H); ESI-MS: *m/z* 790 $[M+H]^+$.

Step 5: Preparation of compound **20f**

A solution of compound **20e** (500 mg, 0.63 mmol) and 1H-tetrazole (5.54 mL of a 3 - 4% in MeCN) in dry THF (31 mL) was treated with activated 3Å molecular sieves for 2 h under an inert atmosphere after which 2-cyanoethyl-N,N,N',N'-tetra(isopropyl)phosphorodiamidite (210 mg, 0.70 mmol) was added in one portion. The reaction mixture was shaken for 2 h after. An additional amount of 2-cyanoethyl-N,N,N',N'-tetra(isopropyl)phosphorodiamidite (95 mg, 0.32 mmol) was added and shaking was continued overnight. *t*BuOOH (196 μL of 5.5 M solution in decane, 1.08 mmol) was added and the reaction mixture was shaken for an extra hour. The molecular sieves were removed by filtration and

extensively rinsed with DCM. The filtrate was washed with brine and saturated aqueous $NaHCO_3$, dried with $MgSO_4$, filtered and concentrated under reduced pressure. The residue was purified by silica column chromatography (gradient elution: 0 - 10% MeOH in DCM) to give compound **20f (56** mg, yield: 10%). ESI-MS: *m/z* 905.5 $[M+H]^+$.

Step 6: Preparation of **compound 36, sodium salt**

Compound **20f** (56 mg, 0.062 mmol) was stirred in a 33% methylamine solution in ethanol (10 mL) at room temperature for 2 h. The reaction mixture was concentrated under reduced pressure. The crude product was triturated in MeCN followed by preparative reversed phase HPLC purification (Stationary phase: XBridge C18 OBD, 5 μm, 250 x 30 mm; Mobile phase: aqueous 0.25% ammonia bicarbonate (A) - MeCN (B); gradient elution) to give **compound 36, ammonium salt.** Conversion into the sodium salt was done by elution of an aqueous solution over a column packed with IR Na ion-exchange resin to afford **compound 36, sodium salt** as a white fluffy solid after lyophilization (17.5 mg, yield: 42%). $^1H$ NMR (600 MHz, DMSO-$d_6$, 81 °C) δ ppm 8.71 (br s, 1 H), 8.34 (br s, 1 H), 8.14 (s, 1 H), 8.07 (s, 1 H), 6.94 (br s, 2 H), 6.90 (br s, 2 H), 6.32 (t, J=7.1 Hz, 1 H), 6.22 (br s, 1 H), 5.68 (br d, J=26.8 Hz, 1 H), 5.42 (br d, J=51.9 Hz, 1 H), 5.10 (br s, 1 H), 4.46 (br d, *J*=25.5 Hz, 1 H), 4.10 - 4.19 (m, 1 H), 4.04 (br s, 1 H), 3.79 (br s, 1 H), 3.18 (m, *J*=11.4 Hz, 1 H), 2.92 (s, 1 H), 2.53 (br s, 1 H); $^{31}P$ NMR (162 MHz, DMSO-$d_6$) δ ppm - 1.74 (s, 1 P); ESI-MS: *m/z* 644.4 $[M+H]^+$.

Example 21

## Compound 29

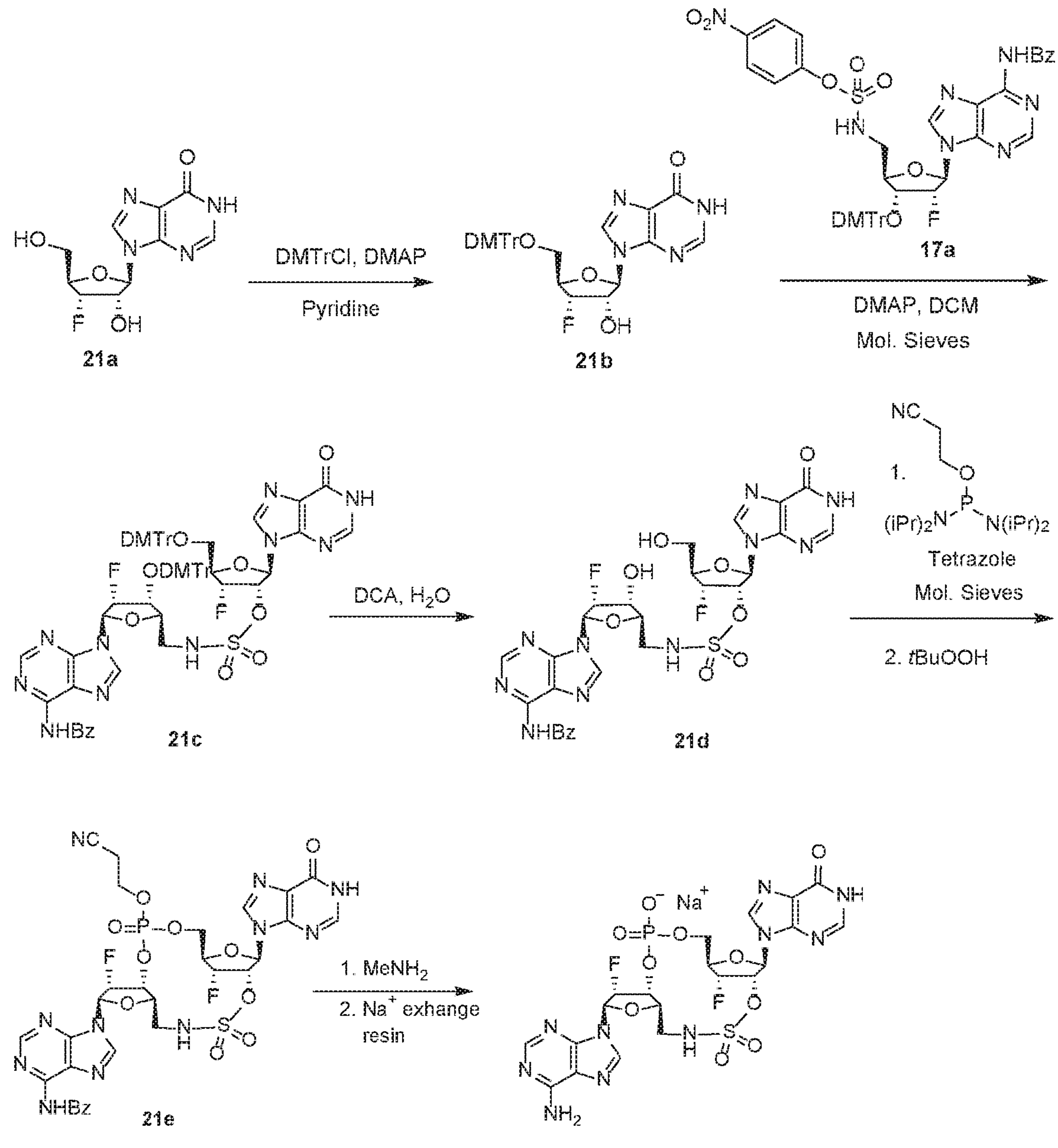


## Compound 29, sodium salt

Step 1: Preparation of compound **21b**

A solution of 3'-deoxy-3'-fluoroinosine **21a** [CAS 117517-20-1] (2.2 g, 8.14 mmol) in dry pyridine (33 mL), to which DMAP (0.49 g, 4.0 mmol) and DMTrCl (4.4 g, 13 mmol) (portion wise) were added, was stirred at room temperature until complete conversion (ca. 2.5 h). The reaction mixture was quenched with methanol (10 mL) and concentrated under reduced pressure. The obtained residue was dissolved in ethyl acetate and washed with water. The organic layer was dried over anhydrous $Na_2SO_4$, filtered and concentrated under reduced pressure. Purification was done by column chromatography over silica gel (gradient elution: 0 - 2% MeOH in DCM) to give compound **21b** as an off-white foam (3.3 g, yield: 71%). $^1H$ NMR (500 MHz DMSO-$d_6$) $\delta$ ppm: 8.22 (d, J=2.1 Hz, 1H), 7.93 (d, J=1.4 Hz, 1H), 7.36 (d, J=8.3 Hz, 2H), 7.28 (t, J=7.6 Hz, 2H), 7.23 (m, 5H), 6.86 (dd, J=8.3, 6.2 Hz, 4H), 5.92 (d, J=7.6 Hz, 1H), 5.14 (dd, J=54.1, 4.5 Hz, 1H), 5.02 (dq, J=23.2, 3.9 Hz, 1H), 4.35 (dt, J=25.9, 4.3 Hz, 1H), 3.74 (s, 6H), 3.29 ( dq, J=37.5, 5.2 Hz, 2H); ESI-MS:m/z

572.0 [M+H]+.

Step 2: Preparation of compound **21c**

Compound **21b** (0.66 g, 1.15 mmol), sulfamate **17a** (1.21 g, 1.38 mmol) and DMAP (0.704 g, 5.76 mmol) were each separately dissolved in dry DCM (3 x 20.0 mL) and dried on activated 3Å molecular sieves for at least 2 h under an inert atmosphere. Next, the compound **21b** and sulfamate **17a** solutions were successively transferred to the reaction flask containing the DMAP solution. The resulting reaction mixture was stirred for 24 h. The molecular sieves were removed by filtration and thoroughly rinsed with dichloromethane. The filtrate was successively washed with saturated aqueous $NaHCO_3$ and saturated aqueous $NH_4Cl$, dried over $Na_2SO_4$, filtered, and concentrated under reduced pressure. The residue was purified by silica column chromatography (gradient elution: 0 - 2% MeOH in DCM) to give compound **21c** as an off-white foam (0.475 g, yield: 31%).

$^1$H NMR (500 MHz DMSO-$d_6$) δ ppm: 12.47 (s, 1H), 11.23 (s, 1H), 8.66 (s, 1H), 8.56 (s, 1H), 8.48 (s, 1H), 8.19 (s, 1H), 8.03 (d, J=7.6 Hz, 2H), 7.84 (s, 1H), 7.65 (t, J=7.2 Hz, 1H), 7.55 (t, J=7.6 Hz, 2H), 7.48 (d, J=7.6 Hz, 2H), 7.36 (q, J=4.8 Hz, 4H), 7.32 (m, 4H), 7.23 (t, J=7.6 Hz, 4H), 7.19 (t, J=7.9 Hz, 5H), 6.90 (dd, J=9.0, 6.9 Hz, 4H), 6.81 (dd, J=9.0, 6.2 Hz, 4H), 6.34 (m, 1H), 6.23 (d, J=6.2 Hz, 1H), 5.78 (m, 2H), 5.50 (d, J=53.7 Hz, 1H), 4.72 (m, 2H), 4.43 (d, J=24.1 Hz, 1H), 4.01 (t, J=7.9 Hz, 1H), 3.74 (s, 1H), 3.70 (t, J=2.4 Hz, 12H), 3.25 (dd, J=10.7, 3.8 Hz, 1H), 2.99 (d, J=13.1 Hz, 1H), 2.70 (dd, J=14.5, 9.0 Hz, 1H); ESI-MS: *m/z* 1310.0 [M+H]+.

Step 3: Preparation of compound **21d**

A solution of compound **21c** (0.453 g, 0.34 mmol) in DCM (12.6 mL), to which DCA (1.14 mL of 10% in DCM, 1.38 mmol) and water (31 μL, 1.72 mmol) were added, was stirred at room temperature until complete deprotection (ca. 2 h). The reaction mixture was quenched by the addition of pyridine (0.14 mL, 1.72 mmol) and some drops of methanol. The resulting suspension was stirred for 20 min, filtered and dried to get compound **21d** (0.21 g, yield: 86%). $^1$H NMR (500 MHz DMSO-$d_6$) δ ppm: 12.5 (d, J=3.4 Hz, 1H), 11.25 (s, 1H), 8.75 (s, 1H), 8.69 (t, *J=6.0* Hz, 1H), 8.61 (s, 1H), 8.35 (s, 1H), 8.09 (d, *J*=3.4 Hz, 1H), 8.05 (d, 7.6 Hz, 2H), 7.66 (t, *J* = 7.2 Hz, 1H), 7.56 (t, J=7.6 Hz, 2H), 6.32 (dd, J=20.0, 2.1 Hz, 1H), 6.21 (d, J=7.6 Hz, 1H), 5.85 (d, J=6.2 Hz, 1H), 5.60 (m, 1H), 5.49 (m, 3H), 5.34 (d, J=4.8 Hz, 1H), 4.57 (m, 1H), 4.40 (dt, *J*=26.9, 3.4 Hz, 1H), 3.92 (q, J=6.0 Hz, 1H), 3.65 (t, *J=4.1* Hz, 2H), 3.17 (t, *J*=6.2 Hz, 2H); ESI-MS: *m/z* 706.0 [M+H]+.

Step 4: Preparation of compound **21e**

A solution of compound **21d** (260 mg, 0.37 mmol) and 1H-tetrazole (2.15 mL of a 3 - 4% in MeCN, dried on activated 3Å molecular sieves) in DMF / THF (1:2, 30 mL, dried on activated 3Å molecular sieves) was treated with 3Å molecular sieves for 2 h under an inert atmosphere after which 2-cyanoethyl-N,N,N',N'-tetra(isopropyl) phosphorodiamidite (129 μL, 0.41 mmol) was added in one portion. The resulting reaction mixture was shaken at room temperature overnight An additional amount of 2-cyanoethyl-N,N,N',N'-tetra(isopropyl)phosphorodiamidite (23 μL, 0.074 mmol) was added and shaking was continued for an extra day to obtain full conversion. *t*BuOOH (134 μL of 5.5 M solution in decane, 0.74 mmol) was added and the reaction mixture was shaken for an extra hour. Molecular sieves were removed by filtration and rinsed with dichloromethane. The filtrate was extensively washed with water, dried with $MgSO_4$, filtered and concentrated under reduced pressure. The residue was purified by silica column chromatography (gradient elution: 0 - 5% MeOH in DCM) to give compound **21e** (20 mg, yield: 7%).

ESI-MS: *m/z* 820.4 [M+H]+.

Step 5: Preparation of **compound 29, sodium salt**

Compound **21e** (20 mg, 0.024 mmol) was stirred in a 33% methylamine solution in ethanol (10 mL) at room temperature until complete conversion (~1 h). The reaction mixture was concentrated under reduced pressure. The resulting crude product was triturated in MeCN followed by preparative reversed phase HPLC purification (Stationary phase: XBridge C18 OBD, 5 μm, 250 x 30 mm; Mobile phase: aqueous 0.25% ammonia bicarbonate (A) - MeCN (B); gradient elution) to give **compound 29, ammonium salt.** Final conversion into the sodium salt was done by elution of an aqueous solution over a column packed with a cationic sodium ion-exchange resin to afford **compound 29, sodium salt** as a white fluffy solid after lyophilization (13.5 mg, yield: 80%). $^{31}$P NMR (162 MHz, DMSO-$d_6$) δ ppm -2.06 (s, 1 P); ESI-MS: *m/z* 663.3 [M+H]+.

Example 22

**Compound 49**

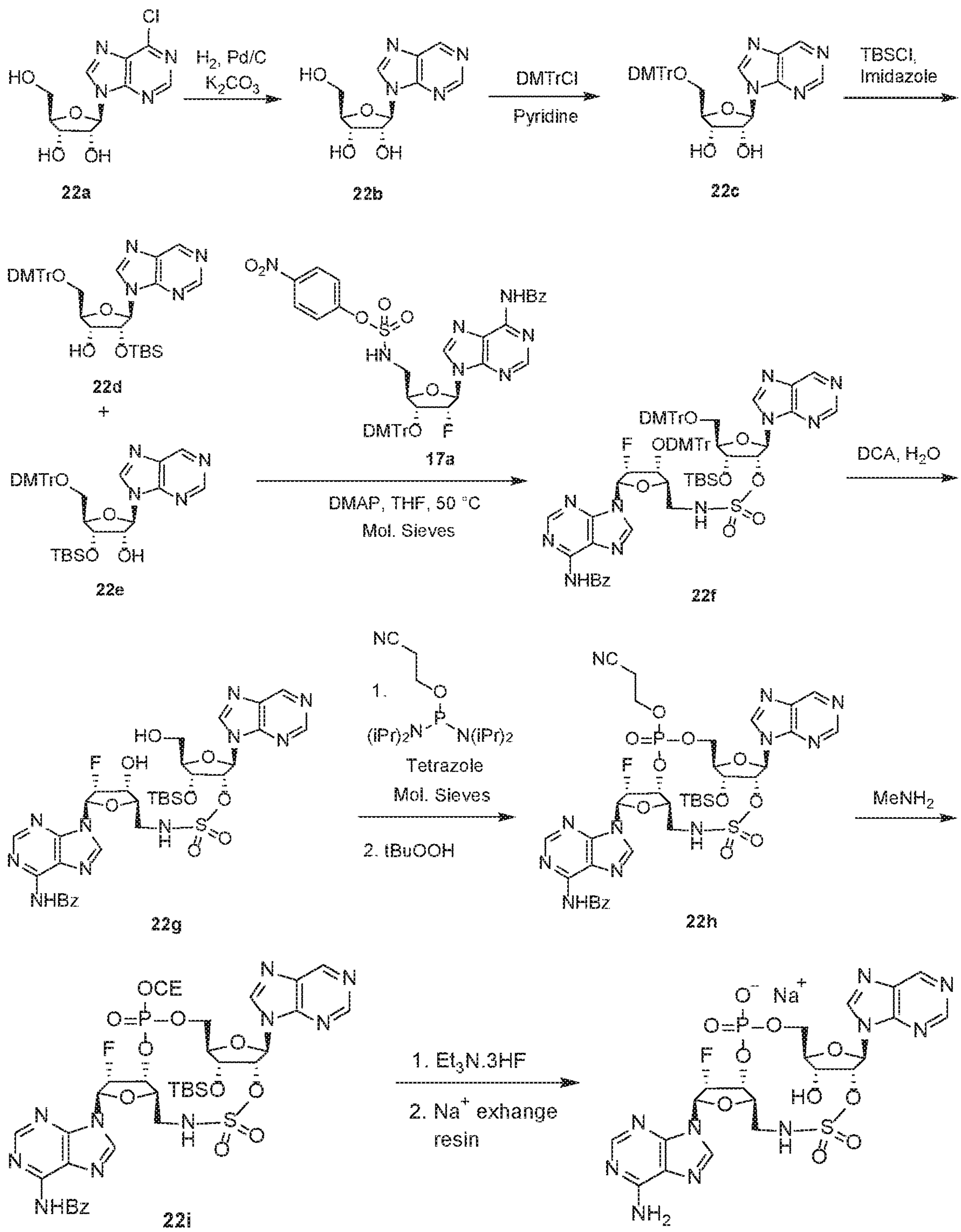


**Compound 49, sodium salt**

Step 1: Preparation of compound **22b**

A solution of 6-chloropurine riboside **22a** (10.0 g, 34.88 mmol, CAS# 5399-87-1) in THF (200 mL) was hydrogenated under atmospheric pressure at room temperature on Pd/C (10% on carbon, 8 g) in the presence of $K_2CO_3$ (9.64 g, 69.77 mmol). The reaction mixture was filtered over diatomaceous earth, the diatomaceous earth was successively rinsed with THF and 5:1 THF/MeOH. The combined filtrates were concentrated under reduced pressure to give crude compound **22b** (6.3 g) which was used as such in the next step. ESI-MS: m/z 253.1 $[M+H]^+$.

Step 2: Preparation of compound **22c**

A solution of crude compound **22b** (6.1 g) and DMTrCl (11.93 g, 35.20 mmol) in dry pyridine (300 mL) was stirred at room temperature until complete conversion (ca. 2 h). The reaction mixture was diluted with DCM and washed with brine. The aqueous layer was reextracted with DCM. The combined organic layers were dried over anhydrous $Na_2SO_4$, filtered, and concentrated under reduced pressure. Purification was done by column chromatography over silica gel (gradient elution: 1 - 5% MeOH in DCM) to give compound **22c** as a white solid (11.7 g). $^1H$ NMR (400 MHz, DMSO-$d_6$) $\delta$ ppm 9.21 (s, 1 H), 8.89 (s, 1 H), 8.74 (s, 1 H), 7.33 (d, J=6.8 Hz, 2 H), 7.15 - 7.26 (m, 7 H), 6.80 (t, *J*=9.3 Hz, 4 H), 6.07 (d, J=4.5 Hz, 1 H), 5.62 (d, *J*=5.6 Hz, 1 H), 5.29 (d, J=5.8 Hz, 1 H), 4.78 (q, J=5.1 Hz, 1 H), 4.34 (q, J=5.3 Hz, 1 H), 4.11 (q, *J*=4.8 Hz, 1 H), 3.72 (s, 3 H), 3.71 (s, 3 H), 3.21 - 3.25 (m, 2 H); ESI-MS: m/z 555.1 $[M+H]^+$.

Step 3: Preparation of compounds **22d** and **22e**

A solution of compound **22c** (6.7 g, 12.1 mmol) in DMF (70 mL) to which imidazole (2.47 g, 14.5 mmol) and TBSCl (2.19 g, 2.39 mmol) were added, was stirred at room temperature overnight. The reaction mixture was diluted with EtOAc and washed with brine. The organic phase was dried with anhydrous $Na_2SO_4$, filtered and concentrated under vacuum. The crude product was purified by silica column chromatography (gradient elution: 10 - 33% EtOAc in petroleum ether) to give compound **22d** (3.3 g, yield: 40%) as the first eluting isomer and compound **22e** (3.5 g, yield: 43%) as the second eluting isomer. Compound **22d:** $^1H$ NMR (400 MHz, DMSO-$d_6$) $\delta$ ppm 9.22 (s, 1 H), 8.87 (s, 1 H), 8.74 (s, 1 H), 7.38 (d, J=7.5 Hz, 2 H), 7.16 - 7.29 (m, 7 H), 6.83 (dd, J=8.5, 4.9 Hz, 4 H), 6.09 (d, J=4.8 Hz, 1 H), 5.23 (d, *J*=6.0 Hz, 1 H), 4.89 (t, J=4.9 Hz, 1 H), 4.27 (q, J=5.2 Hz, 1 H), 4.14 (q, *J*=4.5 Hz, 1 H), 3.72 (s, 6 H), 3.29 (br d, J=4.6 Hz, 2 H), 0.72 (s, 10 H), -0.05 (s, 3 H), -0.17 (s, 3 H); ESI-MS: *m*/*z* 669.2 $[M+H]^+$.

Compound **22e:** $^1H$ NMR (400 MHz, DMSO-$d_6$) $\delta$ ppm 9.20 (s, 1 H), 8.87 (s, 1 H), 8.80 (s, 1 H), 7.29 - 7.40 (m, 2 H), 7.12 - 7.27 (m, 7 H), 6.82 (m, J=8.8, 6.8 Hz, 4 H), 6.04 (d, *J*=5.0 Hz, 1 H), 5.48 (d, *J=6.0* Hz, 1 H), 4.92 (q, J=5.2 Hz, 1 H), 4.52 (t, J=4.6 Hz, 1 H), 4.05 - 4.13 (m, 1 H), 3.72 (s, 6 H), 3.36 (dd, J=10.5, 4.5 Hz, 1 H), 3.15 (dd, J=10.5, 5.0 Hz, 1 H), 0.84 (s, 9 H), 0.09 (s, 3 H), 0.05 (s, 3 H); ESI-MS: *m*/*z* 669.2 $[M+H]^+$.

Step 4: Preparation of compound **22f**

Activated 3Å molecular sieves were added to a reaction flask charged with compound **22e** (1.31 g, 1.96 mmol) and sulfamate **17a** (1.88 g, 2.14 mmol) in dry THF (85 mL). The resulting mixture was stirred at room temperature for at least 2 h under inert atmosphere. Simultaneously, a solution of DMAP (1.2 g, 9.82 mmol) in dry THF (15 mL) was dried on activated 3Å molecular sieves, after which it was transferred to the reaction flask. The resulting reaction mixture was stirred at 50 °C overnight The molecular sieves were removed by filtration and thoroughly rinsed with dichloromethane. The filtrate was washed with water and saturated aqueous $NaHCO_3$, dried over $Na_2SO_4$, filtered, and concentrated under reduced pressure. The residue was purified by silica column chromatography (gradient elution: 0 - 2% MeOH in DCM) to give compound **22f.**

Step 5: Preparation of compound **22g**

A solution of compound **22f** (2.4 g, 1.71 mmol) in DCM (80 mL), to which DCA (0.563 mL, 6.83 mmol) and water (154 $\mu$L, 8.54 mmol) were added, was stirred at room temperature until complete deprotection (ca. 1 h). The reaction mixture was quenched by the addition of pyridine (688 $\mu$L, 8.54 mmol) in methanol (1 mL) and washed with water. The organic layer was dried with anhydrous $Na_2SO_4$, filtered and concentrated under reduced pressure. The residue was purified by silica column chromatography (gradient elution: 0 - 10% MeOH in DCM) to give compound **22g.** $^1H$ NMR (400 MHz, DMSO-*$d_6$)* $\delta$ ppm 11.26 (s, 1 H), 9.17 (s, 1 H), 8.89 (s, 1 H), 8.83 (s, 1 H), 8.71 (s, 1 H), 8.57 (s, 1 H), 8.44 (t, J=5.8 Hz, 1 H), 8.00 - 8.08 (m, 2 H), 7.61 - 7.75 (m, 1 H), 7.49 - 7.59 (m, 2 H), 6.34 (d, *J=6.0* Hz, 1 H), 6.28 (dd, J=19.2, 2.1 Hz, 1 H), 5.79 (d, J=6.1 Hz, 1 H), 5.39 - 5.61 (m, 2 H), 5.21 - 5.32 (m, 1 H), 4.69 (dd, J=4.8, 2.8 Hz, 1 H), 4.39 - 4.55 (m, 1 H), 4.02 (q, J=3.5 Hz, 1 H), 3.78 - 3.87 (m, 1 H), 3.66 - 3.76 (m, 1 H), 3.54 - 3.62 (m, 1 H), 2.95 - 3.14 (m, 2 H), 0.91 (s, 9 H), 0.13 (s, 3 H), 0.13 (s, 3 H); ESI-MS: *m*/*z* 801.3 $[M+H]^+$.

Step 6: Preparation of compound **22h**

A solution of compound **22g** (100 mg, 0.129 mmol) and 1*H*-tetrazole (2.22 mL of a 0.45 M solution in MeCN, 0.99 mmol) in dry THF (3 mL) was treated with 4Å molecular sieves for 5 min under $N_2$ after which 2-cyanoethyl-N,N,N',N'-tetra(isopropyl)phosphorodiamidite (75 mg, 0.25 mmol) in dry THF (1 mL) was added dropwise over 10 min via a syringe (note: THF was freshly distilled over Na/benzophenone and MeCN was freshly distilled over $CaH_2$ before use). The resulting reaction mixture was stirred for 1 h at room temperature. A solution of *t*BuOOH (114 μL of 5-6 M solution in decane, 0.624 mmol) was added via a syringe and stirring was continued for another 30 min. The mixture was diluted with DCM (20 mL), filtered through a pad of Diatomaceous earth and concentrated. The crude product was purified by flash chromatography on silica gel (gradient elution: 0 - 6% MeOH in DCM) to give compound **22h** (66 mg, 57% yield) as a white solid. ESI-MS: *m*/*z* = 916.3 $[M+H]^+$.

Step 7: Preparation of compound **22i**

Compound **22h** (66 mg, 0.072 mmol) was stirred in a 30% methylamine solution in ethanol (5 mL) at room temperature for 4 h. The reaction mixture was concentrated under reduced pressure. The residue was dissolved in water, washed with DCM and lyophilized. The crude product was purified by preparative reversed phase HPLC (Stationary phase: Agela Durashell C18, 5 μm, 150 x 25 mm; Mobile phase: 10 mM aqueous ammonia bicarbonate (A) - MeCN (B); gradient elution) to give compound **22i** (11.6 mg, yield: 14.5%). $^1$H NMR (400 MHz, D20) δ ppm 9.57 (d, J=1.5 Hz, 1H), 9.35 (dd, J=1.5, 9.5 Hz, 2H), 8.71 (d, J=1.3 Hz, 1H), 8.09 (d, J=1.8 Hz,1H), 7.04 (d, J=8.3 Hz, 1H), 6.86 (br, d, J=19.6 Hz, 1H), 6.18 - 5.91 (m, 2H), 5.84-5.65 (m, 1H), 5.24 (br, s, 1H), 4.88-4.80 (m, 3H), 4.74 -4.59 (m, 1H), 4.05 (br, dd, J=5.4, 14.4 Hz, 1H), 3.96-3.81 (m, 1H), 1.44 (d, J=1.3 Hz, 9H), 0.73-0.60 (m, 6H); $^{19}$F NMR (376 MHz, D20) δ ppm -198.496 (s, IF); $^{31}$P NMR (162 MHz, D20) δ ppm -198.496 (s, IF); ESI -MS: *m*/*z* = 759.2 $[M+H]^+$.

Step 8: Preparation of **compound 49, sodium salt**

A solution of compound **22i** (40 mg, 0.053 mmol) in pyridine (3 mL), to which $Et_3N$ (320 mg, 3.16 mmol) and triethylamine trihydrofluoride (254 mg, 1.58 mmol) were added, was stirred at 50 °C for 4 h. The reaction mixture was cooled to temperature and diluted with THF (2 mL). Isopropoxytriethylsilane (697 mg, 5.27 mmol) was added and stirring was continued at room temperature for 1.5 h. The residue, obtained after concentration under reduced pressure, was purified by preparative reversed phase HPLC (Stationary phase: XBridge C18 OBD, 5 μm, 150 x 25 mm; Mobile phase: 10 mM aqueous ammonia bicarbonate (A) - MeCN (B); gradient elution) to give **compound 49, ammonium salt.** Final conversion into the sodium salt was done by elution of an aqueous solution over a column packed with a cationic sodium ion-exchange resin to afford **compound 49, sodium salt** as a white solid after lyophilization (22.5 mg, yield: 58%).

$^1$H NMR (400 MHz, D20) δ ppm 8.95 (s, 1H), 8.76 (s, 1H), 8.53 (s, 1H), 8.13 (s, 1H), 6.73-6.58 (m, 1H), 6.53 (d, J=8.0 Hz, 1H), 6.42-6.33 (m, 1H), 5.93 (br, dd, J=4.3, 7.8 Hz, 1H), 5.50-5.30 (m, 1H), 5.19-5.04 (m, 1H), 4.88-4.81 (m, 1H), 4.55-4.44 (m, 2H), 4.39 - 4.32 (m, 1H), 4.25 (ddd, J=2.3, 4.9, 12.2 Hz, 1H), 3.71 (br, d, J=13.1 Hz, 1H), 3.46 (br, d, J=12.8 Hz, 1H); $^{19}$F NMR (376 MHz, D20) δ ppm -197.119 (s, IF); $^{31}$P NMR (162 MHz, D20) δ ppm -1.984 (s, IP); ESI -MS: *m*/*z* = 645.2 $[M+H]^+$.

Example 23

## Compound (**R*) 16A and Compound (**S*) 16B

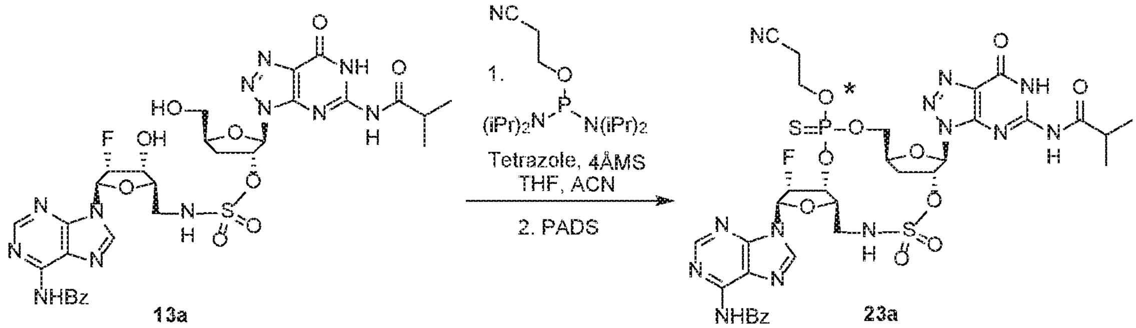


13a

23a

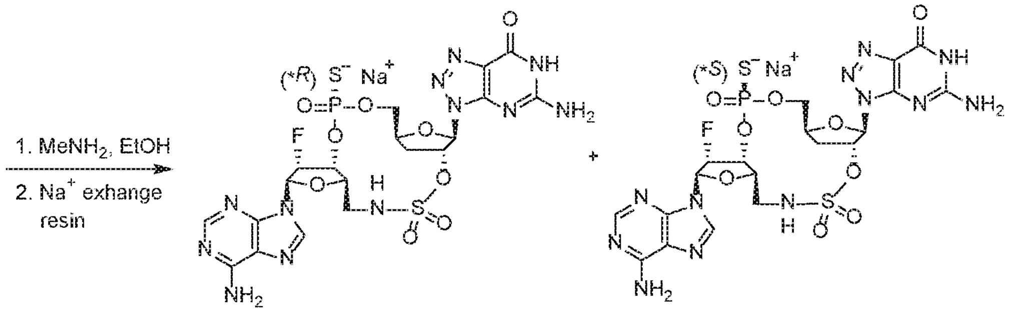


+

**Compound (**R*) 16A, sodium salt** **Compound (**S*) 16B, sodium salt**

Step 1: Preparation of compound **23a**

Compound **13a** (900 mg, 1.16 mmol) was dissolved in a mixture of anhydrous ACN (103 mL) and anhydrous THF (103 mL). 1H-tetrazole (10.2 mL, 3 - 4% in MeCN, dried on 3Å molecular sieves before use ) and 3Å molecular sieves were added. The mixture was shaken for 2 hours at RT and then 2-cyanoethyl-N,N,N',N'-tetraisopropylphosphorodiamidite (0.48 mL, 1.51 mmol) was added at once via syringe. The reaction mixture was shaken at RT for 4 hours. An additional amount of 2-cyanoethyl-N,N,N',N'-tetraisopropylphosphorodiamidite (0.48 mL, 1.51 mmol) was added. The reaction mixture was shaken at RT for 1 hour and then phenylacetyl disulfide (0.7 g, 2.33 mmol) was added. The reaction mixture was shaken at RT for 30 minutes. The reaction mixture was filtered. The molecular sieves were washed three times with dichloromethane. The combined filtrate was washed with a mixture of a saturated $Na_2S_2O_3$ solution and a saturated $NaHCO_3$ solution, washed with brine, dried with $MgSO_4$, filtered and the solvents of the filtrate evaporated. The residue was purified by column chromatography over silica (gradient elution: 0 - 100% MeOH in DCM) to give compound **23a** (152 mg, yield: 11%). ESI-MS: m/z 904.3 $[M+H]^+$.

Step 2: Preparation of **compound (*R) 16A** and **compound (**S*) 16B**

Compound **23a** (152 mg, 0.12 mmol) in methylamine, 33% solution in ethanol (7 mL, 56 mmol) was stirred at 40°C for 3 hours. The reaction mixture was evaporated to dryness under reduced pressure. The residue was triturated in 10 mL anhydrous acetonitrile. The precipitate was collected by filtration and washed with anhydrous acetonitrile. A purification/separation of the two epimers was performed with reverse phase preparative HPLC (Stationary phase: RP XBridge Prep C18 OBD-10μm,50×150mm, Mobile phase: 0.25% $NH_4HCO_3$ solution in water, MeOH). The solvents of the two pure fractions were removed by lyophilization. The residues were dissolved in water and filtered over a prewashed (water) column packed with a cationic sodium ion-exchange resin to give to give **compound (*R) 16A, sodium salt** (37 mg, 29% yield) and **compound (*S) 16B, sodium salt** (5 mg, 6% yield), both as a white solids.

**Compound (*R) 16A**, **sodium salt:** $^1H$ NMR (400 MHz, DMSO-$d_6$) δ ppm 2.58 - 2.69 (m, 1 H) 2.95 - 3.07 (m, 1 H) 3.41 (dd, J=13.2, 2.6 Hz, 1 H) 3.58 - 3.68 (m, 1 H) 4.01 (td, J=11.8, 6.5 Hz, 1 H) 4.18 (ddd, J=13.5, 11.3, 4.1 Hz, 1 H) 4.36 (br d, J=8.1 Hz, 1 H) 4.47 - 4.56 (m, 1 H) 5.18 - 5.29 (m, 1 H) 5.75 - 5.94 (m, 1 H) 5.79 (m, J=9.0, 4.1 Hz, 1 H) 6.13 (d, J=3.7 Hz, 1 H) 6.37 (d, J=19.1 Hz, 1 H) 6.78 (br s, 2 H) 7.15 (br s, 2 H) 7.99 (s, 1 H) 8.38 (s, 1 H) 9.65 (br

s, 1 H); ESI-MS: m/z 677.0 $[M+H]^+$.

**Compound (*S) 16B, sodium salt:** $^{31}P$ NMR (162 MHz, D2O) δ ppm 55.80 (s, 1 P). ESI-MS: m/z 677.3 $[M+H]^+$.

Example 24

**Compound (*R) 13A**

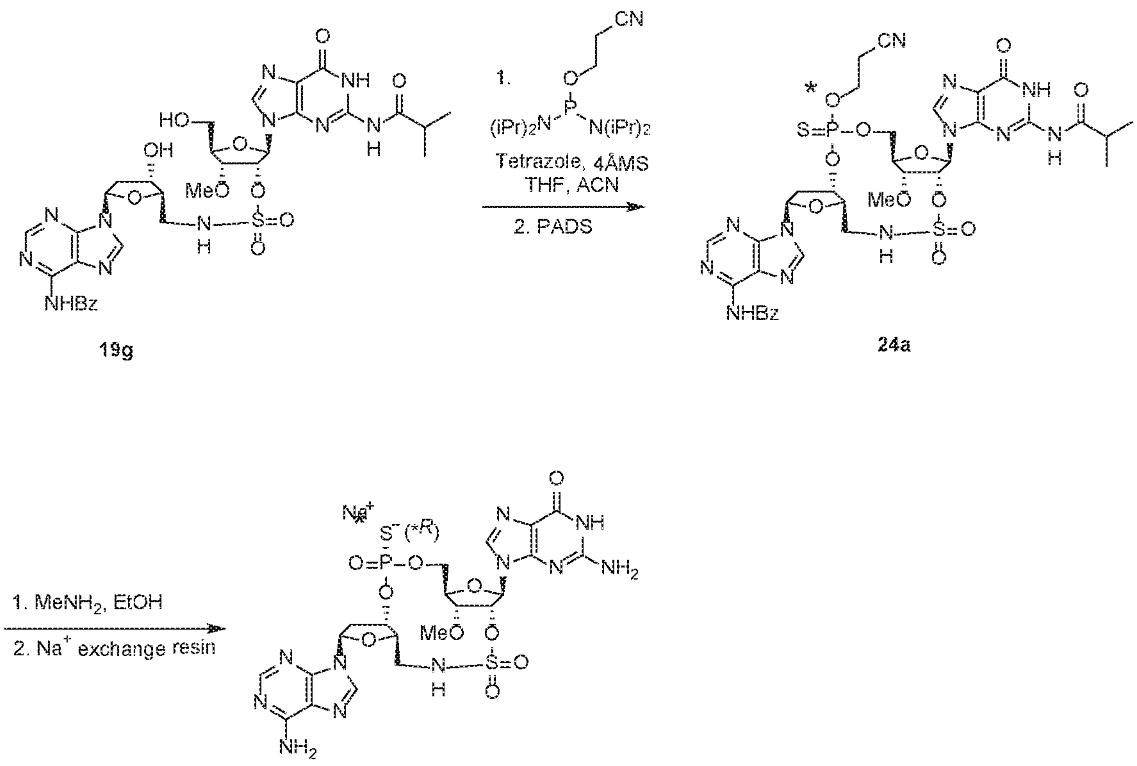


19g

24a

**Compound (*R) 13A, sodium salt**

Step 1: Preparation of compound **24a**

Compound **19g** (0.6 g, 0.77 mmol) was dissolved in a mixture of anhydrous ACN (55 mL) and anhydrous THF (55 mL). 1H-Tetrazole (8.94 mL, 3 - 4% in MeCN, dried on 3Å molecular sieves before use) and 3Å molecular sieves were added. The mixture was shaken for 1 hour at RT and then 2-cyanoethyl-N,N,N,N-tetraisopropyl-phosphorodiamidite (0.24 mL, 0.77 mmol) was added at once via syringe. The reaction mixture was shaken at RT for 2.5 h and then phenylacetyl disulfide (PADS, 0.46 g, 1.53 mmol) was added. The reaction mixture was shaken at room temperature for 18 h. The molecular sieves were removed by filtration and rinsed with dichloromethane. The combined filtrate was washed with a mixture of a saturated $Na_2S_2O_3$ solution and a saturated $NaHCO_3$ solution, washed with brine, dried with $MgSO_4$, filtered and the solvents of the filtrate evaporated. The residue was purified by column chromatography over silica gel (gradient elution: 0 - 100% MeOH in DCM) to give compound **24a** (184 mg, yield: 26%). $^1H$ NMR (400 MHz, DMSO-$d_6$) δ ppm 1.13 (dd, J=6.7, 1.4 Hz, 6 H) 2.69 - 2.87 (m, 2 H) 3.07 (t, J=5.9 Hz, 2 H) 3.25 - 3.30 (m, 1 H) 3.35 - 3.47 (m, 2 H) 3.53 (s, 3 H) 3.55 - 3.65 (m, 1 H) 4.31 - 4.48 (m, 4 H) 4.51 - 4.61 (m, 2 H) 5.14 (dd, J=8.5, 4.1 Hz, 1 H) 5.38 - 5.52 (m, 1 H) 6.20 (d, J=8.5 Hz, 1 H) 6.60 (dd, J=8.5, 6.1 Hz, 1 H) 7.56 (t, J=7.4 Hz, 2 H) 7.65 (t, J=7.0 Hz, 1 H) 8.05 (d, J=7.5 Hz, 2 H) 8.35 (s, 1 H) 8.44 (br s, 1 H) 8.73 (s, 1 H) 8.76 (s, 1 H) 11.22 (br s, 1 H) 11.73 (s, 1 H) 12.11 (br s, 1 H); $^{31}P$ NMR (162 MHz, DMSO-$d_6$) δ ppm 65.02 (s, 1 P); ESI-MS: m/z 915.5 $[M+H]^+$.

Step 2: Preparation of **compound (*R) 13A**, **sodium salt**

Compound **24a** (184 mg, 0.2 mmol) in methylamine, 33% solution in ethanol (10 mL, 81 mmol) was stirred at 45°C for 1 h. The reaction mixture was evaporated to dryness under reduced pressure. The residue was triturated in 3 mL anhydrous acetonitrile. The precipitate was collected by filtration and washed with anhydrous acetonitrile. A purification was performed with reverse phase preparative HPLC (Stationary phase: RP XBridge Prep C18 OBD-10μm,50 x 150mm, Mobile phase: 0.25% $NH_4HCO_3$ solution in water, MeOH). The solvents of the pure fractions were removed by lyophilization. The residue was dissolved in water and filtered over a prewashed (water) column filled with ion-exchange resin IR120 $Na^+$ form. The solvents of the resulting solution were removed by lyophilization to give **compound (*R) 13A**, **sodium salt** (93 mg, 66% yield) as a white fluffy solid. $^1H$ NMR (400 MHz, DMSO-$d_6$) δ ppm 10.59 (br s, 1 H), 8.42 (s, 1 H), 8.37 (s, 1 H), 8.05 (s, 1 H), 7.23 - 7.33 (m, 2 H), 7.28 (br s, 2 H), 6.52 (br s, 2 H), 6.35 (dd, J=8.4, 6.0 Hz, 1 H), 6.02 (d, J=8.5 Hz, 1 H), 5.48 (br dd, J=8.6, 4.0 Hz, 1 H), 5.17 (br s, 1 H), 4.36 (br s, 1 H), 4.12 (s, 2 H), 3.95 - 4.04 (m, 1 H), 3.88 - 3.95 (m, 1 H), 3.48 - 3.59 (m, 1 H), 3.47 (s, 3 H), 3.18 (dd, J=14.0,4.3 Hz, 1 H), 3.06 (ddd, J=13.9, 8.2, 5.9 Hz, 1 H), 2.53 - 2.68 (m, 1 H); $^{31}P$ NMR (162 MHz, DMSO-$d_6$) δ ppm 52.21 (s, 1 P); ESI-MS: *m/z* 688.3 $[M+H]^+$.

<u>Example 25</u>

**Compound (*R) 31A and compound (*S) 31B**

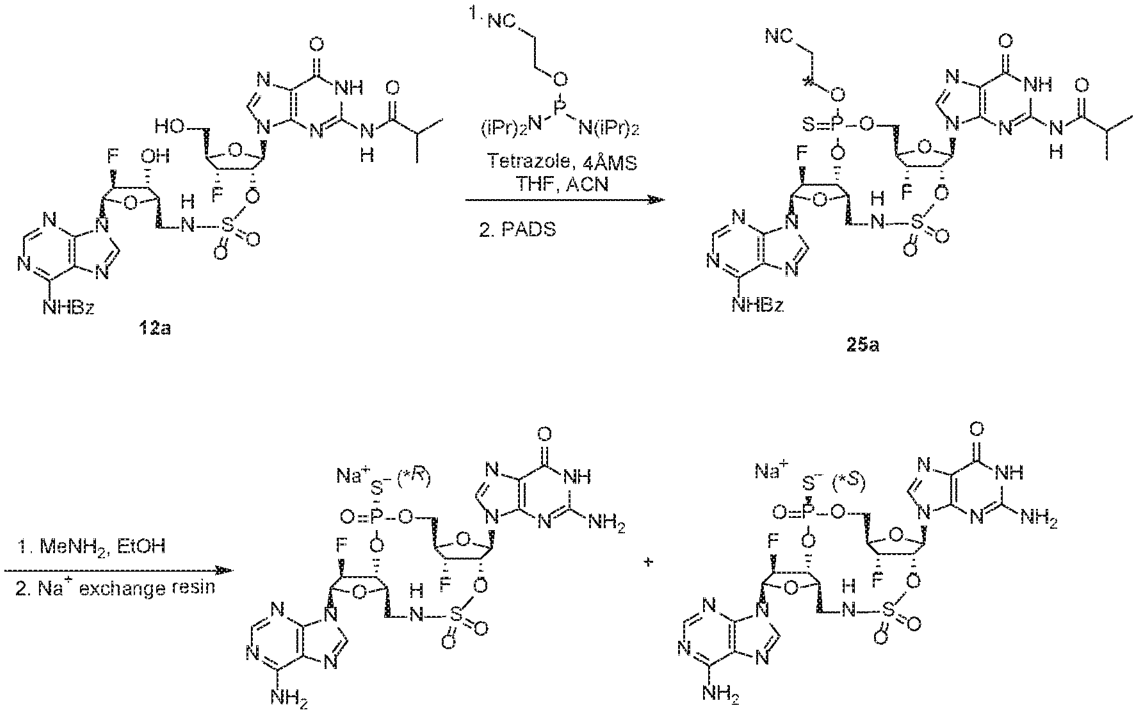


12a

25a

**Compound (*R) 31A, sodium salt** **Compound (*S) 31B, sodium salt**

Step 1: Preparation of compound **25a**

Compound **12a** (300 mg, 0.38 mmol) was dissolved in a mixture of anhydrous ACN (33 mL) and anhydrous THF (33 mL). 1H-Tetrazole (4.4 mL, 3 - 4% in MeCN, dried on 3Å molecular sieves before use) and 3Å molecular sieves were added. The mixture was shaken for 1 hour at RT and then 2-cyanoethyl-N,N,N',N'-tetraisopropylphosphorodiamidite (0.12 mL, 0.38 mmol) was added at once via syringe. The reaction mixture was shaken at room temperature for 4.5 hours. An additional amount of 2-cyanoethyl-N,N,N',N'-tetraisopropylphosphorodiamidite (0.06 mL, 0.19 mmol) was added. The reaction mixture was shaken at RT for 18 hours and then phenylacetyl disulfide (0.23 g, 0.75 mmol) was

added. The reaction mixture was shaken at RT for 2 hours. The reaction mixture was filtered. The molecular sieves were washed three times with dichloromethane. The combined filtrate was washed with a mixture of a saturated $Na_2S_2O_3$ solution and a saturated $NaHCO_3$ solution, washed with brine, dried with $MgSO_4$, filtered and the solvents of the filtrate evaporated. The residue was purified by column chromatography over silica gel (gradient elution: 0 - 10% MeOH in DCM) to give **25a** (103 mg, 27% yield). ESI-MS: m/z 921.4 $[M+H]^+$.

Step 2: preparation of **compound (*R) 31A, sodium salt** and **compound (*S) 31B, sodium salt**

Compound **25a** (103 mg, 0.1 mmol) was placed in methylamine, 33% solution in ethanol (6 mL, 58.4 mmol) was stirred at 45°C for 1 hour. The reaction mixture was evaporated to dryness under reduced pressure. The residue was triturated in 10 mL anhydrous acetonitrile. The precipitate was collected by filtration and washed with anhydrous acetonitrile. A purification/separation of the two epimers was performed with reverse phase preparative HPLC (Stationary phase: RP XBridge Prep C18 OBD-10μm,50×150mm, Mobile phase: 0.25% $NH_4HCO_3$ solution in water, MeOH). The solvents of the two pure fractions were removed by lyophilization. The residues were dissolved in water and filtered over a prewashed (water) column filled with a cationic sodum ion-exchange resin. The solvents of the resulting solutions were lyophilized to give compounds **31A** and **31B,** each as a white solid.

**Compound (*R) 31A, sodium salt** (42 mg, 57% yield). $^1H$ NMR (400 MHz, DMSO-$d_6$) δ ppm 3.36 - 3.51 (m, 2 H) 3.88 (m, J=9.8 Hz, 1 H) 4.12 - 4.23 (m, 2 H) 4.55 (d, J=1.0 Hz, 1 H) 5.19 (br t, J=13.0 Hz, 1 H) 5.26 - 5.61 (m, 3 H) 6.04 (br d, J=8.1 Hz, 1 H) 6.37 - 6.48 (m, 1 H) 6.53 (br s, 2 H) 7.35 (br s, 2 H) 8.16 (s, 1 H) 8.22 (d, J=2.8 Hz, 1 H) 8.34 (s, 1 H). $^{31}P$ NMR (162 MHz, DMSO-$d_6$) δ ppm 52.43 (s, 1 P); ESI-MS: m/z 694.3 $[M+H]^+$.

**Compound (*S) 31B, sodium salt** (3 mg, 4% yield). $^{31}P$ NMR (162 MHz, D2O) δ ppm 55.20 (s, 1 P); ESI-MS: m/z 694.3 $[M+H]^+$.

Example 26

Compound (*R) 7A and compound (*S) 7B

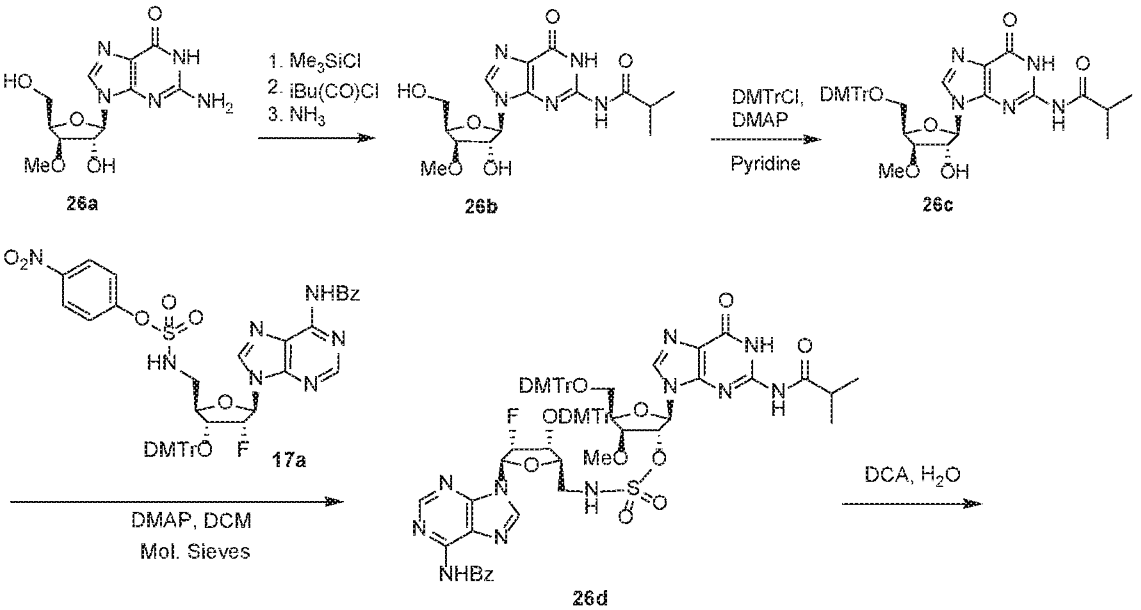


26a 26b 26c 17a 26d

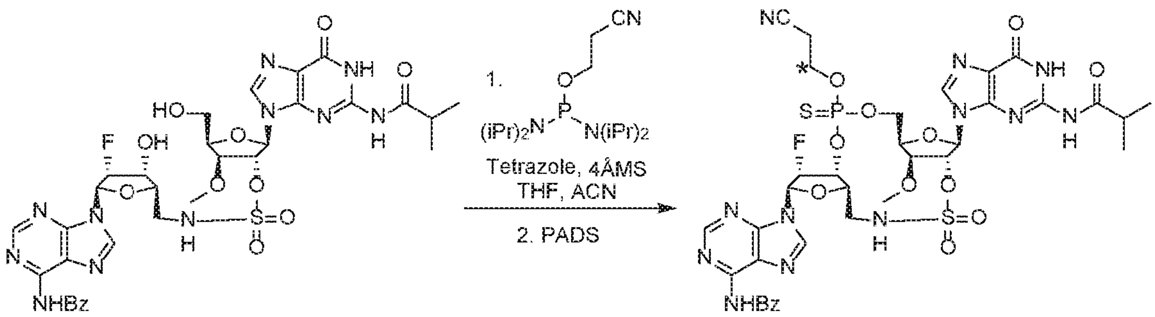


26e 26f

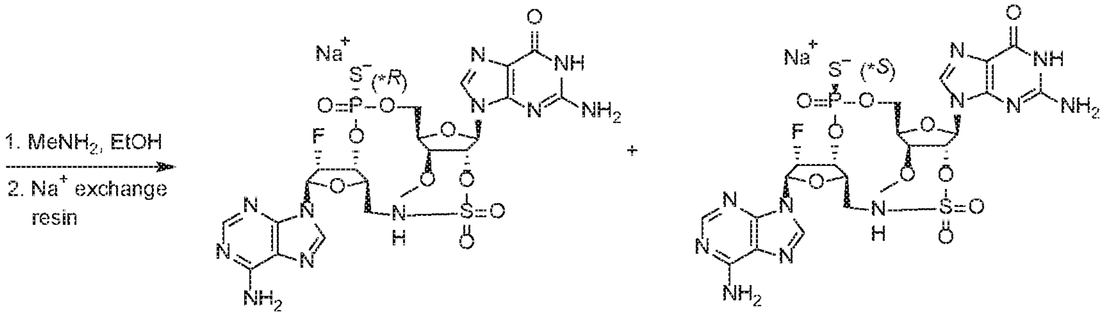


**Compound (*R) 7A, sodium salt** **Compound (*S) 7B, sodium salt**

Step 1: Preparation of compound **26b**

Chlorotrimethylsilane (5.53 mL, 43.7 mmol) was added dropwise to a solution **of 26a** [CAS 847648-20-8] (2.6 g, 8.74 mmol) in dry pyridine (26 mL) at 0 °C. The reaction solution was stirred at room temperature for 30 min after which it was cooled again to 0 °C. Isobutyryl chloride (4.58 mL, 43.7 mmol) was added dropwise over a period of 15 min and stirring was continued at room temperature until complete conversion. The reaction was cooled to 0 °C, water was added followed by the addition of aqueous ammonia (26%, 18 mL) after 20 min, stirring was continued at room temperature for 2 h. The reaction solution was neutralized with acetic acid and concentrated under reduced pressure. Purification was performed by column chromatography over silica gel (gradient elution: 0 - 7% MeOH in DCM) to give compound **26b** as an off-white solid (1.3 g, yield: 40%). $^1H$ NMR (500MHz DMSO-$d_6$) δ ppm: 12.10 (s, 1H), 11.71 (s, 1H), 7.96 (s, 1H), 5.93 (d, *J*= 4.1 Hz, 1H), 4.80 (t, *J*= 5.5 Hz, 1H), 4.50 (t, *J*= 2.1 Hz, 1H), 4.23 (q, *J* = 5.0 Hz, 1H), 3.78 (q, *J*= 2.1 Hz, 1H), 3.71 (m, 1H), 3.63 (m, 1H), 3.37 (s, 3H), 2.78 (m, 1H), 1.23 (d, *J* = 4.8 Hz, 1H), 1.12 (d, *J*= 6.9 Hz, 6H); ESI-MS: *m/z* 367.9 $[M+H]^+$.

Step 2: Preparation of compound **26c**

A solution of compound **26b** (1.75 g, 4.76 mmol) in dry pyridine (35 mL), to which DMAP (0.29 g, 2.38 mmol) and DMTrCl (2.41 g, 7.14 mmol) (portionwise) were added, was stirred at room temperature until complete conversion (ca. 1.5 h). The reaction mixture was quenched with methanol (15 mL) and concentrated under reduced pressure. The obtained residue was dissolved in ethyl acetate and washed with water. The organic layer was dried over anhydrous $Na_2SO_4$, filtered and concentrated under reduced pressure. Purification was performed by column chromatography over silica gel (gradient elution: 0 - 2.5% MeOH in DCM) to give compound **26c** as a pale brown foam (3.1 g, yield: 96%).

$^1H$ NMR (500 MHz, DMSO-$d_6$) δ ppm: 11.89 (d, *J* = 177.0 Hz, 2H), 7.75 (s, 1H), 7.39 (d, *J* = 7.6 Hz, 2H), 7.25 (m, 7H), 6.86 (m, 4H), 5.98 (d, *J* = 4.1 Hz, 1H), 5.83 (d, *J* = 2.1 Hz, 1H), 4.49 (m, 2H), 3.82 (q, *J* = 2.1 Hz, 1H), 3.73 (d, *J* = 2.8 Hz, 6H), 3.27 (s, 3H), 3.22 (m, 1H), 2.78 (m, 1H), 1.12 (dd, *J* = 6.9, 2.1 Hz, 6H); ESI-MS: *m/z* 669 $[M+H]^+$.

Step 3: Preparation of compound **26d**

A reaction flask was charged with DMAP (0.35 g, 2.9 mmol), dry DCM (10 mL) and activated 3Å molecular sieves. The resulting mixture was stirred at room temperature for at least 2 h under inert atmosphere. Simultaneously, a solution of compound **26c** (0.38 g, 0.57 mmol) and a solution of sulfamate **17a** (0.6 g, 0.69 mmol) each in dry DCM

(2 x 10 mL), were dried on activated 3Å molecular sieves (ca. 2 h). Both solutions (compound **26c** and sulfamate **17a** respectively) were successively transferred to the reaction flask. The resulting reaction mixture was stirred for 24 h. The molecular sieves were removed by filtration and thoroughly rinsed with dichloromethane. The filtrate was washed with saturated aqueous $NaHCO_3$, brine and saturated aqueous $NH_4Cl$, dried over $Na_2SO_4$, filtered, and concentrated under reduced pressure. The residue was purified by silica gel column chromatography (gradient elution: 0 - 1% MeOH in DCM) to give compound **26d** as a foam (0.44 g, yield: 55%). $^1$H NMR (500 MHz, DMSO-$d_6$) δ ppm: 12.12 (s, 1H), 11.69 (s, 1H), 11.22 (s, 1H), 8.63 (s, 1H), 8.45 (s, 1H), 8.02 (d, *J* = 6.9 Hz, 2H), 7.64 (q, *J* = 7.3 Hz, 2H), 7.55 (t, *J* = 7.6 Hz, 2H), 7.49 (d, *J* = 7.6 Hz, 2H), 7.36 (m, 8H), 7.23 (m, 8H), 6.92 (t, *J*= 9.6 Hz, 4H), 6.84 (dd, *J* = 13.8, 9.0 Hz, 4H), 6.28 (d, *J* = 19.3 Hz, 1H), 6.05 (d, *J* = 1.0 Hz, 1H), 5.15 (s, 1H), 4.97 (m, 1H), 4.85 (d, *J* = 22.0 Hz, 1H), 4.40 (q, *J* = 5.0 Hz, 1H), 3.98 (m, 2H), 3.70 (t, *J* = 3.1 Hz, 12H), 3.23 (d, *J* = 6.9 Hz, 2H), 3.13 (s, 3H), 2.79 (m, 2H), 2.65 (m, 2H), 1.10 (d, *J* = 5.5 Hz, 6H); ESI-MS: m/z 1406 $[M+H]^+$.

Step 4: Preparation of compound **26e**

A solution of compound **26d** (430 mg, 0.3 mmol) in DCM (12 mL), to which DCA (1.0 mL of 10% in DCM, 1.2 mmol) and water (27 μL, 1.5 mmol) were added, was stirred at room temperature until complete deprotection (ca. 1 h). The reaction mixture was quenched by the addition of pyridine (120 μL, 1.5 mmol) and some drops of methanol. The residue obtained after concentration under reduced pressure was purified by silica column chromatography (gradient elution: 0 - 8% MeOH in DCM) to give compound **26e** as a white powder (225 mg, yield: 92%). $^1$H NMR (500 MHz, DMSO-$d_6$) δ ppm: 12.11 (s, 1H), 11.67 (s, 1H), 11.25 (s, 1H), 8.73 (s, 1H), 8.57 (s, 1H), 8.04 (d, *J*= 6.9 Hz, 2H), 7.87 (s, 1H), 7.65 (t, *J*= 7.6 Hz, 1H), 7.55 (t, *J* = 7.9 Hz, 2H), 6.29 (dd, *J*= 19.6, 1.7 Hz, 1H), 6.04 (s, 1H), 5.92 (s, 1H), 5.58 (m, 1H), 5.22 (s, 1H), 4.92 (d, *J* = 10.3 Hz, 1H), 4.62 (m, 1H), 4.17 (q, *J* = 5.0 Hz, 1H), 4.10 (t, *J* = 2.1 Hz, 1H), 4.02 (td, *J* = 7.2, 2.5 Hz, 1H), 3.70 (m, 1H), 3.61 (m, 1H), 3.45 (m, 1H), 3.37 (s, 3H), 3.06 (q, *J*= 7.3 Hz, 1H), 2.77 (m, 1H), 1.12 (dd, *J* = 6.9, 2.1 Hz, 6H); ESI-MS: *m/z* 801.0 $[M+H]^+$.

Step 5: Preparation of compound **26f**

A solution of compound **26e** (220 mg, 0.27 mmol) and 1*H*-tetrazole (3.2 mL, 3 - 4% in MeCN, dried on 3Å molecular sieves before use) in 1:1 MeCN / THF (48 mL, pre-dried on activated 3Å molecular sieves, pre-dried on activated 3Å molecular sieves) was treated with activated 3Å molecular sieves for 1 h after which 2-cyanoethyl-N,N,N',N'-tetra(isopropyl)phosphorodiamidite (87 μL, 0.27 mmol) was added at once. The resulting reaction mixture was shaken at room temperature for 22 h. Phenylacetyl disulfide (PADS, 0.45 g, 1.5 mmol) was added and shaking was continued for an extra hour. Molecular sieves were removed by filtration and rinsed with dichloromethane. The combined filtrates were subsequently washed with a 1:1 mixture of saturated aqueous $Na_2S_2O_3$ and saturated aqueous $NaHCO_3$, and brine, dried with $MgSO_4$, filtered and concentrated under reduced pressure. The residue was purified by silica gel column chromatography (gradient elution: 0 - 10% MeOH in DCM) to give compound **26f** (55 mg, yield: 21%). ESI-MS: *m/z* 933.5 $[M+H]^+$.

Step 6: Preparation of **compound (*R) 7A, sodium salt** and **compound (*S) 7B, sodium salt**

Compound **26e** (55 mg, 0.059 mmol) was stirred in a 33% methylamine solution in ethanol (4 mL) at 45°C until complete conversion (ca. 1 h), after which the reaction solution was cooled to room temperature and concentrated under reduced pressure. The crude product was triturated in MeCN, the obtained precipitate was further purified by preparative reversed phase HPLC (Stationary phase: XBridge C18 OBD, 5 μm, 250 x 30 mm; Mobile phase: aqueous 0.25% ammonia bicarbonate (A) - MeOH (B); gradient elution) resulting in the separation of both P-epimers: **compound (*R) 7A, ammonium salt** as the first eluting isomer and **compound (*S) 7B, ammonium salt** as the second eluting isomer. Final conversion into the sodium salt was done by elution of an aqueous solution over a column packed with a cationic sodium ion-exchange resin to afford **compound (*R) 7A, sodium salt** (yield: 7 mg, 16%) as a white solid and **compound (*S) 7B, sodium salt** (yield: 4 mg, 9%) as a white solid.

**Compound (*R) 7A, sodium salt.** $^1$H NMR (400 MHz, $D_2O$) δ ppm 3.31 (br d, *J*=12.2 Hz, 1 H), 3.50 (s, 3 H), 3.52 - 3.57 (m, 1 H), 4.28 - 4.50 (m, 4 H), 4.74 (s, 1 H), 4.97 - 5.08 (m, 1 H), 5.29 (s, 1 H), 5.72 (dd, *J*=50.5, 3.7 Hz, 1 H), 6.15 (s, 1 H), 6.30 (d, *J*=18.7 Hz, 1 H), 7.76 (s, 1 H), 8.10 (s, 1 H), 8.21 (s, 1 H); $^{31}$P NMR (162 MHz, $D_2O$) δ ppm 55.05 (s, 1 P); ESI-MS: *m/z* 706.0 $[M+H]^+$.

**Compound (*S) 7B, sodium salt.** $^1$H NMR (400 MHz, $D_2O$) δ ppm 8.43 (br s, 1 H), 8.18 (s, 1 H), 7.84 (s, 1 H), 6.37 (d, *J*=19.1 Hz, 1 H), 6.14 (s, 1 H), 5.52 (dd, *J*=50.7, 4.3 Hz, 1 H), 5.26 (br s, 1 H), 4.87 - 4.99 (m, 2 H), 4.64 - 4.70 (m, 1 H), 4.49 (ddd, *J*=10.5, 4.9, 2.7 Hz, 1 H), 4.39 (br d, *J*=8.8 Hz, 1 H), 4.23 (dt, *J*=23.8, 10.7 Hz, 1 H), 3.57 (br dd, *J*=14.1, 3.5 Hz, 1 H), 3.47 (s, 3 H), 3.33 (br d, *J*=13.9 Hz, 1 H); $^{31}$P NMR (162 MHz, $D_2O$) δ ppm 56.94 (s, 1 P); ESI-MS: *m/z* 706.1 $[M+H]^+$.

Example 27

**Compounds (*R) 44A and (S*) 44B**

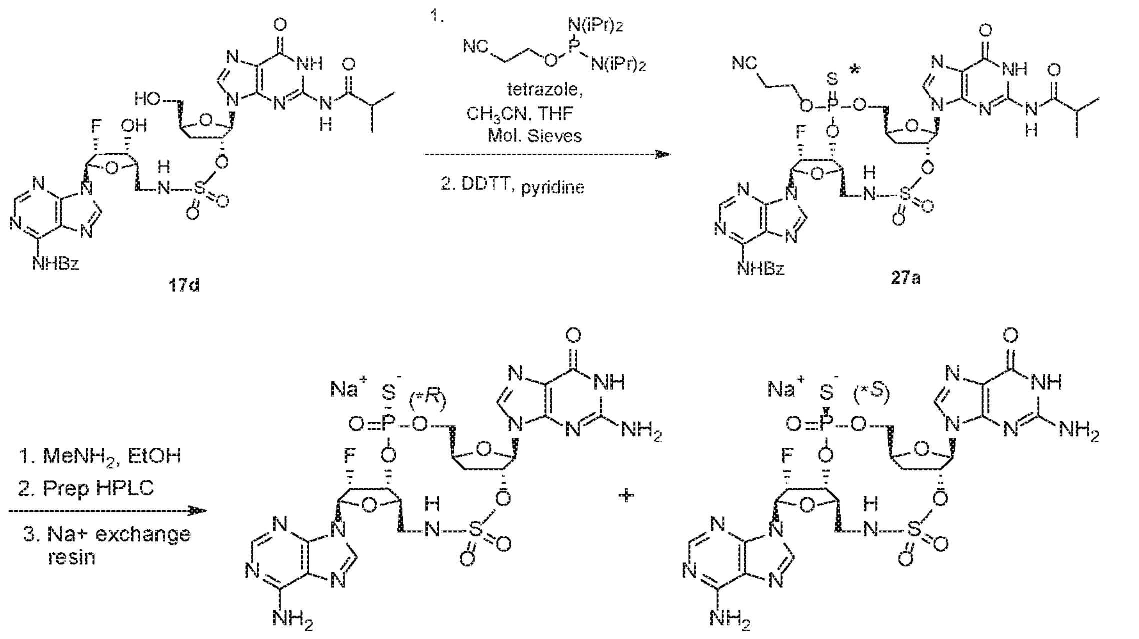


17d 27a

**Compound (*R) 44A, sodium salt** **Compound (*S) 44B, sodium salt**

Step 1: Preparation of compound **27a**

Note: THF was freshly distilled over Na/benzophenone and $CH_3CN$ was freshly distilled over $CaH_2$ before use.

To a solution of **17d** (400 mg, 0.518 mmol) in tetrahydrofuran (14 mL) was added 4Å molecular sieves (powder, 2g) and the mixture was stirred for 20 min. After 20 min, a solution of 1H-tetrazole (0.45 M in acetonitrile, 9.2 mL) was added at 25 °C, followed by the addition of a solution of 2-cyanoethyl-*N*,*N*,*N'*,*N'*-tetra(isopropyl)phosphorodiamidite (0.31 g, 1.037 mmol, diluted in 2 mL of THF) at 25 °C. After stirring for 1.5 hr at RT, (*E*)-N,N-dimethyl-N-(3-thioxo-3H-1,2,4-dithiazol-5-yl)formimidamide (DDTT, 0.453 g, 2.205 mmol, diluted in 10 mL Py) was added at 25 °C and the solution was stirred for 1.5 h. The reaction mixture was concentrated under reduced pressure to give a residue. The residue was purified by flash column chromatography over silica gel (0-15% MeOH in DCM) to give 27a (80 mg) as a light yellow solid. ESI-MS: m/z=902.9 $[M+H]^+$.

Step 2: Preparation of **compound (*R) 44A, sodium salt** and **compound (*S) 44B, sodium salt**

A solution of compound 27a (80 mg, 0.09 mmol) in methylamine (50% in EtOH, 5.0 mL) was stirred at 25 °C for 4 hours. The reaction mixture was concentrated under reduced pressure. Separation by reverse phase preparative HPLC (Column: Xbridge 150 x 30mm x 10μm, Condition: water (10 mM $NH_4HCO_3$)-$CH_3CN$ Begin B: 5, End B 20, Gradient Time(min): 7, 100%B Hold Time(min): 0, Flow Rate (mL/min): 25) afforded **compound (*R) 44A, ammonium salt** (19 mg) and **compound (*S) 44B, ammonium salt** (1.5 mg), both as a white solid. Final conversion of both compounds into their corresponding sodium salt was done by elution of an aqueous solution over a column packed with a cationic sodium ion-exchange resin.

**Compound (*R) 44A, sodium salt** (27 mg).

$^1H$ NMR (400 MHz, $D_2O$) $\delta$ ppm 8.22 (br, s, 1H), 8.09 (s, 1H), 7.29 (br, s, 1H), 6.57 -6.50 (m, 1H), 6.05-5.98 (m, 2H), 5.94-5.78 (m, 1H), 5.31-5.13 (m, 1H), 4.70 (br, d, J = 7.5 Hz, 1H), 4.60 (br, d, *J* = 8.8 Hz, 1H), 4.26 (br, dd, *J* = 4.3, 11.5 Hz, 1H), 4.19 - 4.11 (m, 1H), 3.85 (br, d, *J* = 13.3 Hz, 1H), 3.56 (br, d, *J* = 13.3 Hz, 1H), 2.81 - 2.63 (m, 2H);

$^{19}F$ NMR (376 MHz, $D_2O$) $\delta$-195.872 (s, IF) ppm; $^{31}P$ NMR (162 MHz, $D_2O$) $\delta$52.528 (s, IP) ppm; ESI-MS: *m/z* = 676.0 $[M+H]^+$.

**Compound (*S), 44B, sodium salt** (1.8 mg).

$^1H$ NMR (400 MHz, $D_2O$) $\delta$ppm 8.20 (s, 1H), 7.87 (s, 1H), 7.28 (s, 1H), 6.59-6.46 (m, 1H), 6.22-6.14 (m, 1H), 5.97 (d, *J* = 7.5 Hz, 1H), 5.53-5.34 (m, 2H), 4.66-4.54 (m, 2H), 4.34 (br, t, *J* = 11.5 Hz, 1H), 4.11 (br, dd, *J* = 4.6, 11.9 Hz, 1H), 3.77 (br, d, *J* = 13.3 Hz, 1H), 3.45 (br, d, *J* = 13.3 Hz, 1H), 3.02 (q, *J* = 7.3 Hz, 1H), 2.76-2.66 (m, 1H), 2.78 -2.65 (m, 1H), 2.58 (br, dd, J= 7.8, 12.5 Hz, 1H), 1.26-1.18 (m, 1H), 1.27-1.17 (m, 1H), 1.22 (br t, J= 7.3 Hz, 1H), 1.32 - 1.10 (m, 1H); $^{19}F$ NMR (376 MHz, $D_2O$) $\delta$ppm -195.902 (s, 1F); $^{31}P$ NMR (162 MHz, $D_2O$) $\delta$ppm 56.151 (s, IP); ESI-MS: *m/z* = 677.0 $[M+H]^+$.

Example 28

**Compounds (*R) 42A and (*S) 42B**

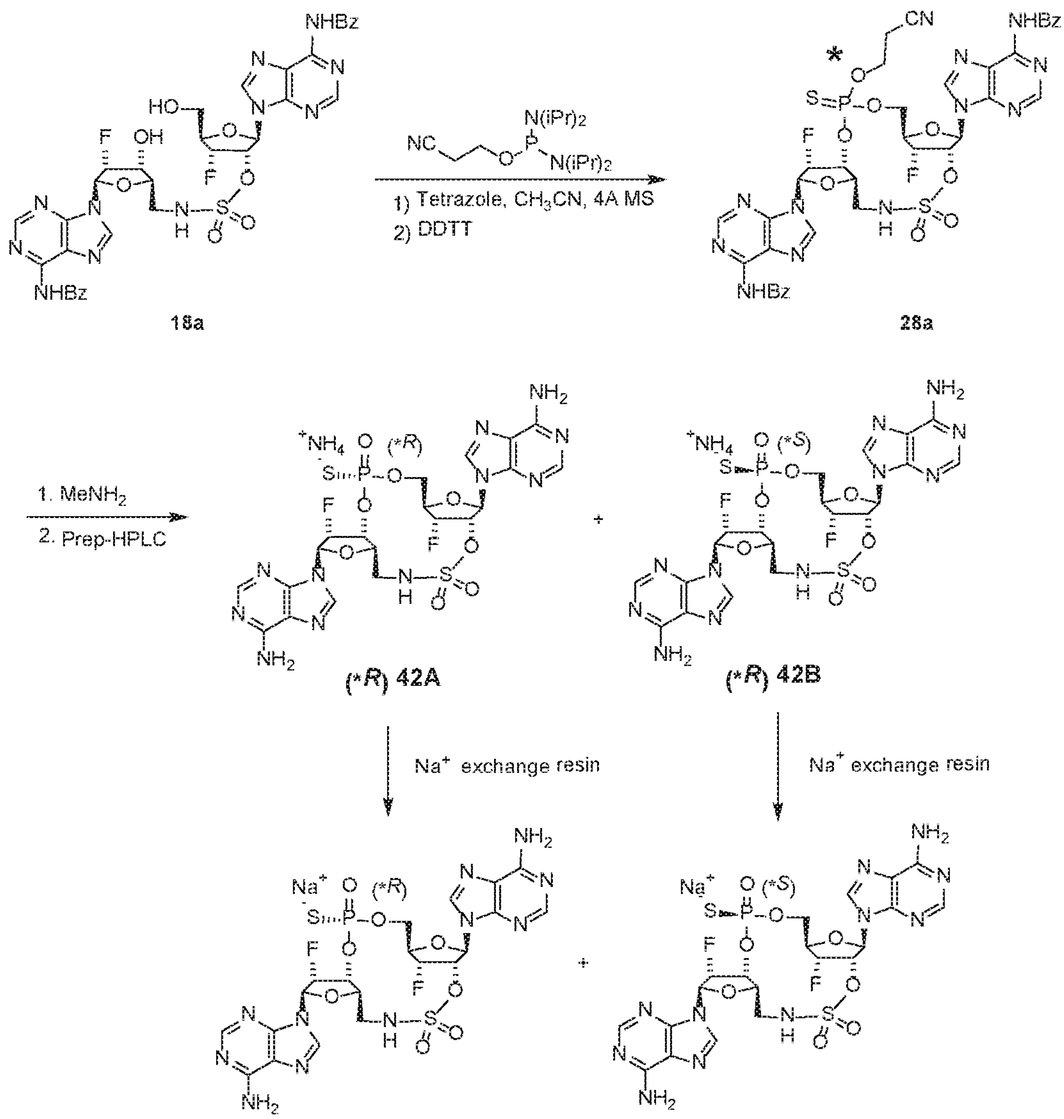


**Compound (*R) 42A, sodium salt** **Compound (*S) 42B, sodium salt**

Step 1: preparation of compound **28a**

Note: THF was freshly distilled over Na/benzophenone and $CH_3CN$ was freshly distilled over $CaH_2$ before use.

To a solution of **18a** (430 mg, 0.532 mmol, dried by lyophilization) in tetrahydrofuran (9 mL) was added 4Å Molecular Sieves (powder, 2g) and the mixture was stirred for 20 min. After 20 min, a solution of 1H-tetrazole (0.45 M in Acetonitrile, 9.46 mL, prepared by dissolved 945 mg of tetrazole (dried by lyophilization) in 30 mL of dry $CH_3CN$, followed by addition of 1.5 g of 4A MS and then stirred for 1hr under $N_2$ before use) was added at 25 °C, purged several times with $N_2$, followed by addition of a solution of 3-((bis(diisopropylamino)phosphino)oxy) propanenitrile (0288 mg, 0.958 mmol, diluted in 2 mL of THF) dropwise over 30 min at 25 °C to generate a white suspension. After stirring for 1.5 hr at RT, a solution of DDTT (655 mg, 3.19 mmol) in pyridine (10 mL) was added at 25 °C and the solution was stirred for 30 min. The reaction mixture was combined with another batch, filtered through a pad of diatomaceous earth, concentrated under reduced pressure to give a yellow residue. The residue was purified by flash column chromatography over silica gel (0-2% MeOH in DCM) to give **28a** (260 mg) as a white solid. ESI-MS: m/z = 939.1 $[M+H]^+$.

Step 2: preparation of **compound (****R***) 42A, ammonium salt** and **compound (****S***) 42B, ammonium salt**

A solution of compound **28a** (130 mg, 0.138 mmol) in methylamine (27 to 32% in EtOH, 5.0 mL) was stirred at RT for 2 hr. The reaction mixture was combined with another batch and the mixture was evaporated under reduced pressure to give a white solid; the solid was dissolved in water (30 mL), then washed with DCM (20 mL x 4). The aqueous layer was lyophilized to afford a white solid (190 mg). The white solid was purified by reverse phase preparative HPLC (Column: Xbridge 150 x 30mm x 5μm, Condition: water (10 mM $NH_4HCO_3$)-$CH_3CN$ Begin B: 5, End B 35, Gradient Time(min): 7, 100%B Hold Time(min): 0, Flow Rate (mL/min): 25) to give **compound (****R***) 42A, ammonium salt** (70 mg) as a white solid and **compound (****S***) 42B, ammonium salt** (12 mg) as a white solid.

**Compound (****R***) 42A, ammonium salt.** $^1H$ NMR (400 MHz, $D_2O$) $\delta$8.96 (s, 1H), 8.23 (d, J=7.1 Hz, 2H), 7.25 (s, 1H), 6.58 - 6.41 (m, 2H), 5.90 - 5.58 (m, 3H), 5.33 - 5.12 (m, 1H), 4.93 - 4.77 (m, 1H), 4.41 - 4.24 (m, 2H), 3.86 (br dd, J=2.8, 13.1 Hz, 1H), 3.66 (br d, J=13.2 Hz, 1H); $^{19}F$ NMR (376MHz, $D_2O$) $\delta$ppm -196.52 (s, IF), -198.72 (s, IF); $^{31}P$ NMR (162MHz, $D_2O$) $\delta$ppm 54.24 (s, IP); ESI-MS: m/z = 678.2 $[M+H]^+$.

**Compound (****S***) 42B, ammonium salt.** $^1H$ NMR (400 MHz, $D_2O$) $\delta$8.54 (br s, 1H), 8.15 (br s, 1H), 7.97 (br s, 1H), 7.14 (br s, 1H), 6.40 (br s, 2H), 5.95 - 5.71 (m, 1H), 5.94 - 5.69 (m, 1H), 5.68 - 5.33 (m, 1H), 5.70 - 5.32 (m, 1H), 5.19 (br s, 1H), 4.13 (br s, 2H), 3.72 (br s, 1H), 3.57 (br d, J=12.7 Hz, 1H); $^{19}F$ NMR (376MHz, $D_2O$) $\delta$ppm -195.66 (s, IF), -198.44 (s, IF); $^{31}P$ NMR (162MHz, $D_2O$) $\delta$ppm 55.09 (s, IP); ESI-MS: m/z = 678.1 $[M+H]^+$.

**Sodium salt conversion**

Dowex 50W × 8, 200-400 (12 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was pH neutral. The resin was transferred back into the beaker, 15% NaOH in deionized water solution was added, and mixture was gently stirred for 5 min, and decanted (1 ×). The resin was transferred to the column and washed with 15% NaOH in water (at least 4 CV), and then with deionized water until it was pH neutral. **Compound (****R***) 42A, ammonium salt** (92 mg in 20 mL/9 mL) was dissolved in minimum amount of deionized water and $CH_3CN$ (1:1, v/v), added to the top of the column, and eluted with deionized water. Appropriate fractions were pooled together and lyophilized to give **compound (****R***) 42A, sodium salt** (72.4 mg) as a white solid.

$^1H$ NMR(400 MHz, $D_2O$) $\delta$ ppm 8.96 (br s, 1H), 8.24 (br d, J=13.2 Hz, 2H), 7.13 (s, 1H), 6.62 - 6.41 (m, 2H), 5.95 - 5.64 (m, 3H), 5.27 - 5.06 (m, 1H), 5.02 - 4.89 (m, 1H), 4.63 (br d, J=8.3 Hz, 1H), 4.39 (br s, 2H), 3.92 (br d, J=11.5 Hz, 1H), 3.72 (br d, J=13.2 Hz, 1H); $^{19}F$ NMR (376MHz, $D_2O$) $\delta$ ppm -196.32 (s, IF), -198.94 (s, IF); $^{31}P$ NMR (162MHz, $D_2O$) $\delta$ ppm 54.05 (s, IP)
ESI-MS: m/z = 678.1 $[M+H]^+$.

Using the previous procedure for sodium salt conversion, **compound (****S***) 42B, sodium salt** was obtained (6.9 mg) as a white solid.

$^1H$ NMR (400 MHz, $D_2O$) $\delta$ ppm 8.63 (s, 1H), 8.23 (s, 1H), 8.08 (s, 1H), 7.29 (s, 1H), 6.56 - 6.39 (m, 2H), 5.95 - 5.77 (m, 1H), 5.73 - 5.58 (m, 1H), 5.58 - 5.45 (m, 1H), 5.58 - 5.44 (m, 1H), 5.36 - 5.22 (m, 1H), 4.91 - 4.78 (m, 1H), 4.53 (br dd, J=6.1, 11.0 Hz, 1H), 4.19 (br d, J=11.7 Hz, 1H), 4.25 - 4.13 (m, 1H), 3.79 (dd, J=4.0, 13.6 Hz, 1H), 3.71 - 3.70 (m, 1H), 3.66 - 3.66 (m, 1H), 3.68 - 3.59 (m, 1H); $^{19}F$ NMR (376MHz, $D_2O$) $\delta$ppm - 195.10 - 196.43 (m, IF), -198.17 (td, J=25.9, 52.5 Hz, IF); $^{31}P$ NMR(162MHz, $D_2O$) $\delta$ 55.26 (s, 1P); ESI-MS: m/z = 678.1 $[M+H]^+$.

Example 29

## Compound 45

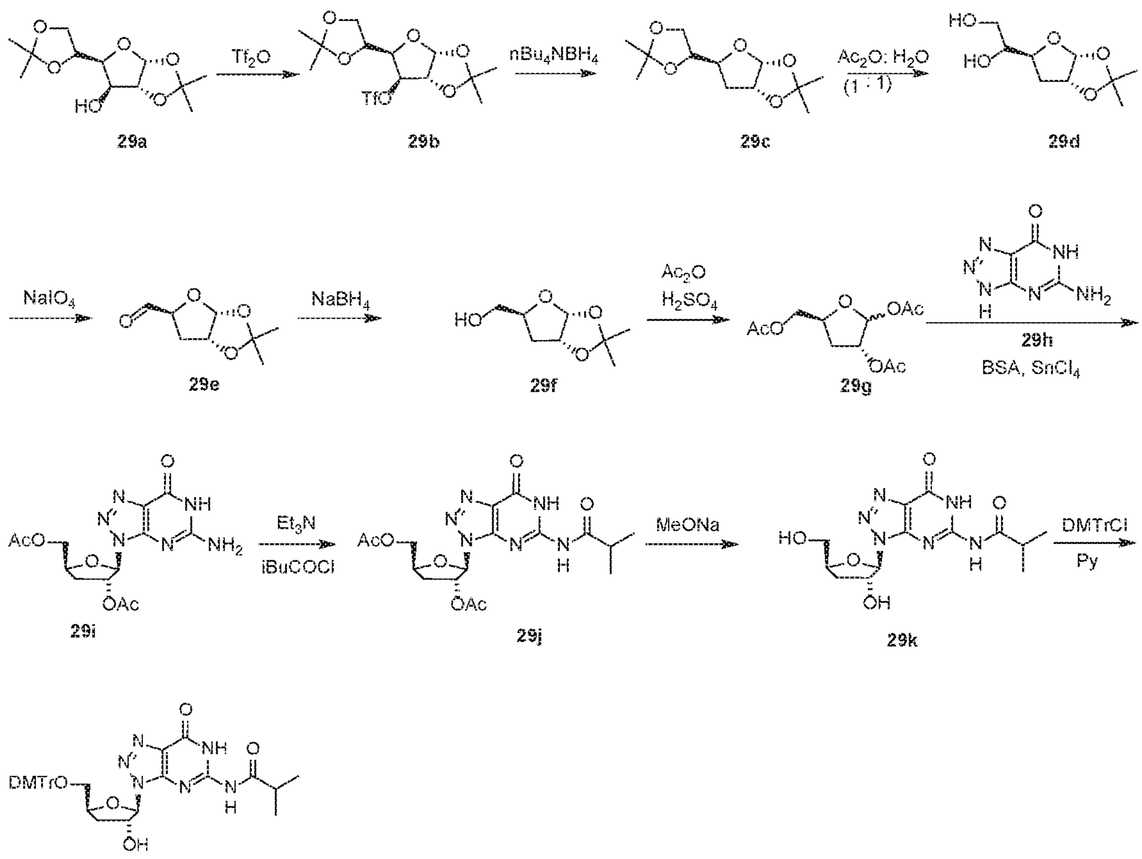


29a 29b 29c 29d 29e 29f 29g 29i 29j 29k

29l

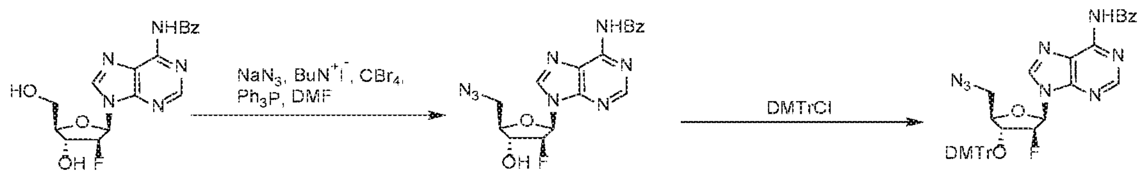


29m 29n 29o

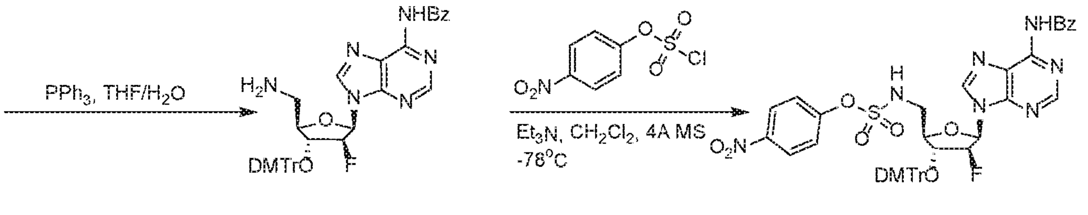


**29p** **29q**

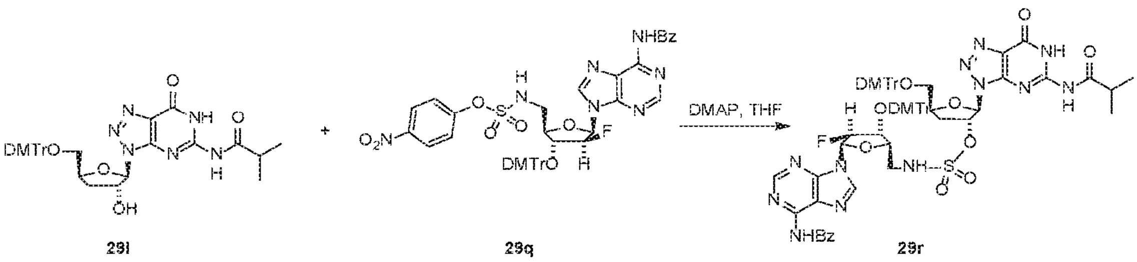


**29l** **29q** **29r**

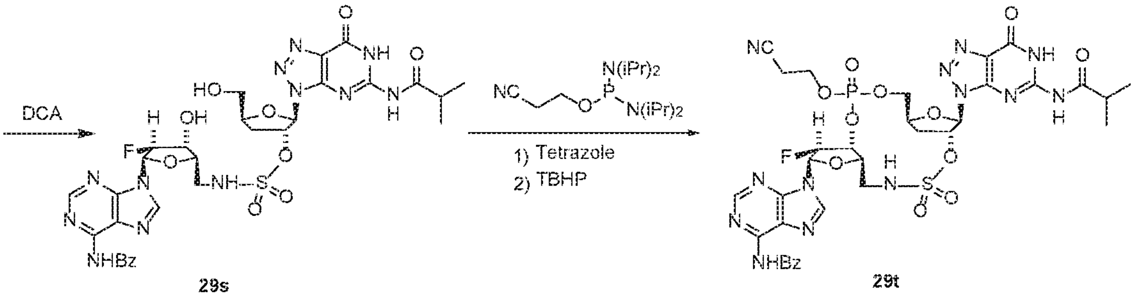


**29s** **29t**

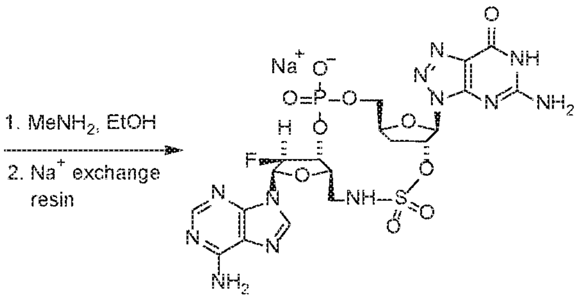


**Compound 45, sodium salt**

Step 1: preparation of compound **29b**

To a solution of compound **29a** (13 g, 49.94 mmol) in pyridine (10.06 mL, 124.86 mmol) and dichloromethane (300 mL) was added triflic anhydride (10.92 mL, 64.93 mmol) dropwise at 0°C. After stirring the reaction at 0 °C for about 1 h, the mixture was diluted with DCM (100 mL), washed with water (200 mL), brine (200 mL), dried with anhydrous $MgSO_4$, filtered and evaporated to dryness to give 29b (20.4 g) as a yellow oil, used into the next step without any further purification. $^1$H NMR (400 MHz, $CDCl_3$) δ 6.07 (d, *J* = 3.6 Hz, 1H), 5.35 (d, *J* = 1.6 Hz, 1H), 4.85 (d, *J* = 3.8 Hz, 1H), 4.32 - 4.19 (m, 3H), 4.06 (dd, *J* = 3.90, 8.8 Hz, 1H), 1.61 (s, 3H), 1.51 (s, 3H), 1.42 (d, *J* = 2.6 Hz, 6H).

Step 2: Preparation of compound **29c**

To a solution of compound **29b** (20.4 g, 51.99 mmol) in toluene (500 mL) was added n-$Bu_4NBH_4$ (40.13 g, 155.98 mmol) dropwise at 25 °C. After stirring at 80 °C for 6 h, the reaction mixture was washed with water (200 mL), brine (200 mL), dried with anhydrous $MgSO_4$, filtered and evaporated to dryness to give a yellow oil. The resulting oil was purified by flash column chromatography over silica gel (0-20% EA in PE) to give compound **29c** (6.4 g) as a

colorless oil. $^1H$ NMR (400 MHz, $CDCl_3$) δ 5.83 (d, *J* = 3.8 Hz, 1H), 4.77 (t, *J* = 4.1Hz, 1H), 4.23 -4.08 (m, 3H), 3.89-3.76 (m, 1H), 2.19 (dd, *J* = 3.9, 13.4 Hz, 1H), 1.82-1.73 (m, 1H), 1.52 (s, 3H), 1.43 (s, 3H), 1.35 (d, *J* = 15.3 Hz, 6H).

Step 3: Preparation of compound **29d**

A solution of compound **29c** (13.4 g, 54.85 mmol) in AcOH/water (V/V,1:1, 200 mL) was stirred for 12 h at 25 °C. The reaction solution was concentrated and co-evaporated with toluene (2 x 40 mL) to give compound **29d** (11.4 g) as a colorless oil, used directly for the next step without any further purification.

Step 4: Preparation of compound **29e**

To a solution of compound **29d** (11.4 g, 8.81 mmol) in MeOH (200 mL) and water (100 mL) was added sodium periodate (17.91 g, 83.73 mmol). After stirring for 2 h at 25 °C, the reaction mixture was filtered and the filtrate diluted with aqueous saturated $Na_2S_2O3$ (50 mL); the mixture was then concentrated to remove MeOH. The residue was partitioned with 2-Me-THF (200 mL), the aqueous layer was extracted with 2-Me-THF (5 x 100 mL). The organic layers were then combined, washed with brine, dried (anhydrous $Na_2SO_4$), filtered and concentrated under reduced pressure to give compound 29e (11g) as a yellow oil, used directly for the next step without further purification.

Step 5: Preparation of compound **29f**

To a solution of compound **29e** (11 g, 9.29 mmol) in MeOH (200 mL) was added sodium borohydride (2.9 g, 76.66 mmol). After stirring for 1 hr at 0 °C, the reaction mixture was quenched with aqueous saturated $NH_4Cl$ (60 mL), and the mixture was concentrated to remove MeOH. The residue was lyophilized to give crude **29f**. The crude product was mixed with MeOH/DCM (v/v, 10:1, 200 mL), then filtered and concentrated under reduced pressure give a residue purified by flash column chromatography over silica gel (0-60% PE in EA) to afford compound **29f** (5.3 g) as a yellow solid. $^1H$ NMR (400 MHz, $CDCl_3$) δ 5.83 (d, *J* = 3.7 Hz, 1H), 4.77 (t, J= 4.2Hz, 1H), 4.41 - 4.30 (m, 1H), 3.90 (br d, *J* = 12.0 Hz, 1H), 3.57 (br d, J= 12.0Hz, 1H), 2.01 (dd, *J* = 4.5, 13.3 Hz, 1H), 1.89 - 1.81 (m, 2H), 1.33 (s, 3H).

Step 6: Preparation of compound **29g**

Acetic anhydride (6.51 mL, 68.9 mmol) and concentrated sulfuric acid (36.91 uL, 0.69 mmol) were added to a stirred solution of compound **29f** (1.2 g, 6.89 mmol) in acetic acid (39.4 mL, 688.89 mmol) at 0 °C. After stirring for 1 h rt, the reaction mixture was quenched with cold water (100 mL), stirred for 30 min rt and extracted with ethyl acetate (3x 60 mL). The organic layer was successively washed with saturated aqueous sodium bicarbonate solution (3 x 90 mL), brine solution (2 x 90 mL), dried over anhydrous sodium sulfate, filtered and concentrated under reduced pressure to give a residue. The residue (combined with silica gel: 4 g) was purified by flash column chromatography over silica (0%-40% EA in PE, V/V) to give compound **29g** (870 mg) as a colorless oil. $^1H$ NMR (400 MHz, $CD_3CN$) δ 6.03 (s, 1H), 5.11 (d, *J* = 4.6 Hz, 1H), 4.49 (dtd, J= 3.3, 6.5, 9.6 Hz, 1H), 4.19 (dd, *J* = 3.3, 11.9 Hz, 1H), 4.00 (dd, *J* = 6.6, 12.0 Hz, 1H), 2.03 - 1.99 (m, 11H). The same reaction was repeated several times and all batches combined.

Step 7: Preparation of compound **29i**

A solution of compound **29h** (0.672 g, 4.42 mmol) and BSA (3.13 g, 15.37 mmol) in anhydrous $CH_3CN$ (12 mL) was stirred at 85 °C for 1 h and then cooled to 0 °C. It was then added dropwise at 0 °C to compound **29g** (1 g, 3.84 mmol) and $SnCl_4$ (3.003 g, 11.53 mmol) under stirring; after stirring the mixture at 26 °C for 24 hr., the crude mixture was combined with another batch, cooled down and diluted with EA (300 mL). The organic layer was successively washed with aqueous saturated $NaHCO_3$ (4 x 200 mL), brine (2 x 150 mL), dried over anhydrous sodium sulfate, filtered and concentrated under reduced pressure to give a residue. The residue (combined with silica gel: 10 g) was purified by flash column chromatography over silica: 20 g) (0%-10% MeOH in DCM, V/V) to give compound **29i** (3.52 g) as a white foam. $^1H$ NMR (400 MHz, $CD_3OD-d_4$) δ 6.22 (s, 1H), 5.75 (br, d, *J*= 5.5 Hz, 1H), 4.65 (br dd, *J*= 3.6, 9.7 Hz, 1H), 4.31 (dd, J=3.3, 12.0 Hz, 1H), 4.12 (dd, *J* = 5.6, 11.9 Hz, 1H), 3.35 (s, 3H), 2.80 (ddd, J= 5.8,10.0,14.1 Hz, 1H), 2.33 (br, dd, *J*= 6.0, 13.8 Hz, 1H), 2.12 (s, 3H), 1.97 - 1.93 (m, 3H); ESI -MS: m/z = 352.9 $[M + H]^+$.

Step 8: Preparation of compound **29j**

To a solution of compound **29i** (2 g, 5.677 mmol) in DCM (20 mL) was added drop-wise $Et_3N$ (1.72 g, 17.03 mmol) and isobutyryl chloride (1.21 g, 11.35 mmol) at rt. After stirring at 25 °C for 2 h, the reaction was quenched by water (50 mL), washed with brine (2 x 50 mL), dried over $Na_2SO_4$, filtered, and concentrated under reduced pressure

to give compound **29j** (2.39 g), used directly for the next step without further purification.

Step 9: Preparation of compound **29k**

To a solution of compound **29j** (2.39 g, 5.67 mmol) in THF (25 mL) was added drop-wise sodium methanolate (1.23 g, 22.71 mmol) at 0 °C. After stirring at 25 °C for 2 h more sodium methanolate was added dropwise (1.23 g, 22.71 mmol). After stirring at 25 °C for for 0.5 h, the mixture was diluted with saturated water/$CH_3COOH$ (1:1,4 mL) to adjust to pH 7, then concentrated under reduced pressure to give a residue. The residue (combined with silica gel: 10 g) was purified by flash column chromatography over silica (0%-10% MeOH in DCM, V/V) to give compound **29k** (770 mg) as a white foam. $^1H$ NMR (400 MHz, DMSO-$d_6$) δ 12.09 (br s, 2H), 5.99 (s, 1H), 5.75 (br d, *J*= 2.9 Hz, 1H), 4.80 - 4.70 (m, 2H), 4.49 - 4.36 (m, 1H), 3.51 - 3.40 (m, 2H),2.79 (td, J= 6.8, 13.6 Hz, 1H), 2.43 - 2.30 (m, 1H), 2.07 (dd, *J* = 6.7,12.6 Hz, 1H), 1.13 (d, *J*= 6.6 Hz, 6H); ESI -MS: m/z = 339.1[M + H]$^+$.

Step 10: Preparation of compound **29l**

DMTrCl (1.20 g, 3.54 mmol) was added to a solution of compound **29k** (1 g, 2.95 mmol) in Py (10 mL). After stirring at 25 °C for 12 hr, the mixture was partitioned between EA (150 mL) and water (100 mL). The mixture was filtered and the organic layer was successively washed with brine (100 mL), dried over anhydrous $Na_2SO_4$, filtered and concentrated to dryness to get the crude residue (oil). The residue (combined with silica gel, 5 g) was purified by flash column chromatography over silica (0%-10% MeOH in DCM, V/V) to give compound **291** (1.52 g) as a yellow foam. $^1H$ NMR (400 MHz, CD3CN) δ 7.32 (dd, J=1.6, 7.9 Hz, 2H), 7.22 - 7.10 (m, 7H), 6.73 (dd, J=8.8, 13.9 Hz, 4H), 6.13 (s, 1H), 4.93 (br s, 1H),4.77 - 4.57 (m, 1H), 3.77 (br s, 1H), 3.73 (d, J=3.9 Hz, 6H), 3.23 - 3.04 (m, 2H), 2.70 (spt, J=6.9 Hz, 1H), 2.57 (ddd, J=5.3, 9.8, 13.3 Hz, 1H), 2.08 (dd,J=6.4, 13.4 Hz, 1H), 1.19 (d, J=6.8 Hz, 6H).

ESI -MS: m/z = 663.3[M + Na]$^+$.

Step 11: Preparation of compound **29n**

Tetrabutylammonium iodide (0.89 g, 2.41 mmol), triphenylphosphine (4.74 g, 18.08 mmol), carbon tetrabromide (5.996 g, 18.080 mmol), $NaN_3$ (3.330 g, 51.223 mmol) were added to a solution of compound **29m** (4.5 g, 12.05 mmol, CAS# 144924-99-2cb) in DMF (70 mL) at 30 °C. After stirring at 30 °C for 24 h, more tetrabutyl-ammonium iodide (0.89 g, 2.41 mmol), triphenylphosphine (4.74 g, 18.00 mmol) and carbon tetrabromide (5.99 g, 18.08 mmol) were added to the solution. After stirring for another 24 h, the mixture was diluted with aqueous saturated $Na_2CO_3$ (150 mL) to adjust the mixture to pH 9, and the mixture was extracted with ethyl acetate (200 mL x 3). The organic layers were then combined and successively washed with brine (100 mL), dried with anhydrous $Na_2SO_4$, filtered and evaporated under reduced pressure to give a yellow solid. The reaction was repeated several times and all batches were combined. The residue (combined with silica gel: 30 g) was purified by flash column chromatography over silica gel (silica gel: 120 g)(gradient elution: 0 - 100% ethyl acetate in petroleum to 0 - 20% methanol in ethyl acetate) to give compound **29n** (9.5 g) as a yellow solid. $^1H$ NMR (400 MHz, DMSO-$d_6$) δ ppm 11.28 (br, s, 1H), 8.78 (s, 1H), 8.54 (d, *J* = 2.4 Hz, 1H), 8.08 - 8.02 (m, 2H), 7.68 - 7.62 (m, 1H), 7.58 - 7.52 (m, 2H), 6.66 - 6.58 (m, 1H), 6.18 (br s, 1H), 5.46 - 5.24 (m, 1H), 4.56 (td, *J*= 4.4, 19.1 Hz, 1H), 4.08 - 4.04 (m, 1H), 3.72 - 3.68 (m, 1H); $^{19}F$ NMR (376 MHz, DMSO-$d_6$) δ ppm -197.05 (s, 1H); ESI-MS: *m*/*z* 398.9 [M + H]$^+$.

Step 12: Preparation of compound **29o**

To a solution of compound **29n** (8.5 g, 21.33 mmol) and DMAP (1.3 g, 10.67 mmol) in pyridine (90 mL) was added DMTrCl (14.46 g, 42.67 mmol) at 25 °C. After stirring at 80 °C for 14 h, the mixture was partitioned between DCM (200 mL) and water (50 mL). The organic layer was washed with brine (100 mL), dried over anhydrous $Na_2SO_4$, filtered and the solvent evaporated under reduced pressure to give the crude **29o.** It was combined with another batch and purified; the residue was purified by flash column chromatography over silica gel (PE/ (EA/DCM = 1/1) from 10% to 100%) to afford compound **29o** (9.5 g) as a light yellow solid. $^1H$ NMR (400 MHz, $CDCl_3$) δ 9.00 (br, s, 1H), 8.84 (br, s, 1H), 8.20 (br, s, 1H), 8.02 (br, d, *J* = 7.6 Hz, 2H), 7.64-7.58 (m, 1H), 7.54 (br, t, *J* = 7.5 Hz, 2H), 7.46 (br, d, *J* = 7.1 Hz, 2H), 7.40 - 7.34 (m, 5H), 7.30 (br, d, *J* = 7.3 Hz, 1H), 6.94 - 6.84 (m, 4H), 6.58 - 6.48 (m, 1H), 4.48 - 4.34 (m, 2H), 4.18 - 4.06 (m, 1H), 3.38 (br d, *J*= 13.0 Hz, 1H), 3.18 (br, dd, J= 5.9, 13.9 Hz, 1H); $^{19}F$ NMR (376 MHz, $CDCl_3$) δ -196.176 (s, 1F); ESI-MS: *m*/*z* 701.2 [M + H]$^+$.

Step 13: Preparation of compound **29p**

To a solution of compound **29o** (10.0 g, 14.27 mmol) in THF (100 mL) was added triphenylphosphine (5.24 g,

1.99 mmol) at 25 °C. After stirring the solution at 40 °C for 2 h, water (50 mL) was added to the solution at 40 °C and the mixture was stirred for 12 h. The reaction mixture was partitioned between DCM (200 mL) and brine (2 x 50 mL). The organic layer was concentrated under reduced pressure to afford a yellow solid (10.0 g). The yellow solid (combined with silica gel: 20 g) was purified by flash column chromatography over silica (DCM/MeOH = 0% to 20%) to give compound **29p** (6.6 g) as a white solid. $^1H$ NMR (400 MHz, DMSO-$d_6$) δ ppm 8.74 (s, 1H), 8.46 (d, *J* = 2.7 Hz, 1H), 8.06 - 7.98 (m, 2H), 7.64 (d, *J*= 7.3 Hz, 1H), 7.58 - 7.50 (m, 2H), 7.48 (d, *J*= 7.6 Hz, 2H), 7.38 - 7.32 (m, 6H), 7.30 - 7.26 (m, 1H), 6.94 (dd, *J* = 3.4, 8.8 Hz, 4H), 6.50 - 6.40 (m, 1H), 4.38 - 4.32 (m, 1H), 4.24 - 4.08 (m, 2H), 3.74 (d, *J*= 2.9 Hz, 6H), 2.68 - 2.54 (m, 2H); $^{19}F$ NMR (376 MHz, DMSO-$d_6$) δ ppm -195.79 (s, 1F); ESI-MS: *m/z* 675.3 $[M+H]^+$.

Step 14: Preparation of compound **29q**

4-Nitrophenol (0.92 g, 6.67 mmol) and triethylamine (1.35, 13.34 mmol) was added to the solution of compound **29p** (1.5 g, 2.22 mmol) in DCM (40 mL). The resulting solution was stirred at -78 °C and a solution of 4-nitrophenyl chlorosulfate (1.58 g, 6.67 mmol) in DCM (5 mL) was added at -78 °C. After stirring at -78 °C for 1 h, the reaction mixture was concentrated under reduced pressure to give a residue. The residue (combined with silica gel: 4 g) was purified by flash column chromatography over silica (0-100% PE in EA) to afford compound **29q** (1.4 g) as a light yellow solid, stored at -20 °C. $^1H$ NMR (400 MHz, $CDCl_3$) δ ppm 8.94 (br, s, 1H), 8.64 (s, 1H), 8.48 (br, s, 1H), 8.18 - 8.08 (m, 2H), 8.00 (br, d, *J* = 7.8 Hz, 2H), 7.96 (s, 1H), 7.66-7.60 (m, 1H), 7.58 -7.52 (m, 2H), 7.44 (br, d, *J*= 7.8 Hz, 2H), 7.38-7.28 (m, 7H), 6.92-6.84 (m, 4H), 6.40- 6.26 (m, 1H), 4.48-4.42 (m, 2H), 4.28 (br, s, 1H), 3.86-3.76 (m, 6H), 3.56 (br, d, *J* = 13.2 Hz, 1H), 3.32 (br, d, *J*= 13.7 Hz, 1H); $^{19}F$ NMR (376 MHz, $CDCl_3$) δ ppm -193.08 (s, IF); ESI-MS: *m/z* 876.6 $[M + H]^+$.

Step 15: Preparation of compound **29r**

A solution of compound **29l** (800 mg, 1.25 mmol), compound **29q** (1.64 g, 1.87 mmol) and activated 4Å molecular sieves (3 g) in THF (24 mL) was stirred at room temperature for 0.5 h under $N_2$. DMAP (762.73 mg, 6.24 mmol) was then added in one portion. After stirring at RT for 2 h under $N_2$, then 45 °C for 12 h under $N_2$, the reaction mixture was diluted with EA (100 mL), then filtered through a pad of diatomaceous earth. The filtrate was washed with aqueous saturated $NaHCO_3$ (6 x 50 mL). The organic layer was successively washed with brine (50 mL), dried over anhydrous $Na_2SO_4$, filtered and concentrated under reduced pressure to give a residue. The residue (combined with silica gel: 5 g) was purified by flash column chromatography over silica (0%-10% MeOH in DCM, V/V) to give compound 29r (1.4 g) as a white solid. ESI -MS: m/z = 1377.1 $[M + H]^+$.

Step 16: Preparation of compound 29s

To a solution of compound 29r (1.4 g, 1.01 mmol) in DCM (24 mL) was added dichloroacetic acid (262 mg, 2.03 mmol) and water (0.183 mL) at rt. After stirring at rt for 3 h under $N_2$, pyridine (0.8 g, 10.16 mmol) was added to the solution. After stirring for 2 h, the mixture was concentrated under reduced pressure to give a residue. The residue (combined with silica gel: 6 g) was purified by flash column chromatography over silica (MeOH/DCM = 0 to 10%) to give compound 29s (570 mg) as a white solid. $^1H$ NMR (400 MHz, DMSO-$d_6$) δ ppm 12.15-11.97 (m, 2H), 11.22 (s, 1H), 8.78 (t, J= 5.9 Hz, 1H), 8.67 (s, 1H), 8.41 (d, *J* = 1.5 Hz, 1H), 8.09-8.00 (m, 2H), 7.69-7.61 (m, 1H), 7.60-7.51 (m, 2H), 6.51 (dd, *J*= 3.8, 17.6 Hz, 1H), 6.34 (s, 1H), 6.23 (d, *J* = 4.0 Hz, 1H), 5.95 (s, 1H), 5.33-5.13 (m, 1H), 4.76 (t, J= 5.5 Hz, 1H), 4.51-4.33 (m, 2H), 4.18-4.02 (m, 2H), 3.54-3.36 (m, 3H), 2.90-2.73 (m, 1H), 2.69-2.56 (m, 1H), 2.40 (br, dd, *J*= 6.0, 14.3 Hz, 1H), 1.13 (dd, *J* = 6.9, 10.2 Hz, 6H); $^{19}F$ NMR (376 MHz, DMSO-d6) *δ* ppm -197.57 (s, IF); ESI -MS: m/z = 773.3 $[M + H]^+$.

Step 17: Preparation of compound 29t

THF was freshly distilled over Na/benzophenone and $CH_3CN$ was freshly distilled over $CaH_2$ before use.

Vacuum-dried compound 29s (100 mg, 0.13 mmol) was co-evaporated with a mixture of $CH_3CN$ and THF (2/2 mL x 3), then dissolved in a mixture of THF (5 mL). It was then added 4Å MS (500 mg) and a solution of 1H-tetrazole (2.301 mL, 0.45 M, prepared by dissolving 315 mg of tetrazole in 10 mL of dry $CH_3CN$, followed by addition of 500 mg of 4Å MS and then stirred for 1 h under $N_2$ before use). The resulting white suspension was stirred for 5 min at RT under $N_2$. A solution of 2-cyanoethyl tetraisopropylphosphoro diamidite (78.01 mg, 0.26 mmol) in THF (1 mL) was then added drop-wise over 5 min vial a syringe. The resulting white suspension was further stirred for 1 hr at 35 °C under $N_2$. A solution of TBHP (0.118 mL, 0.65 mmol, 5~6M in decane) was added via a syringe and stirred for another 30 min. The mixture was diluted with DCM (20 mL), then filtered through a pad of diatomaceous earth and concentrated to give crude product as a colorless oil. The crude product (combined with silica gel: 2 g) was purified by flash column chromatography over silica (0%-6% MeOH in DCM, V/V) to give compound 29t (72 mg) as a white solid. ESI -MS: m/z = 887.9 $[M + H]^+$.

Step 18: Preparation of **Compound** 45, **sodium salt**

Compound **29t** (72 mg, 0.082 mmol) was combined with methylamine (5.0 mL); after stirring at RT for 4 h, the reaction mixture was concentrated under pressure to give a crude product. The crude product was purified by reverse phase preparative HPLC (Column: Xbridge 10μm 150 x 30mm, Condition: water (10mM $NH_4HCO_3$)-ACN B: 0, End B 25, Gradient Time (min): 7, 100%B Hold Time (min): 0, FlowRate (mL/min): 25) to give **compound 45** ammonium salt (6.9 mg) as a white solid. $^1H$ NMR (400 MHz, $D_2O$) δ ppm 8.12 (s, 1H), 7.67 (br, s, 1H), 6.52 (br, dd, *J* = 4.9, 11.7 Hz, 1H), 6.31 (br, d, *J* = 7.0 Hz, 1H), 6.18-6.04 (m, 1H), 5.64-5.43 (m, 1H), 5.32 -5.20 (m, 1H), 4.37 (br, d, *J* = 3.5 Hz, 1H), 4.15-4.03 (m, 2H), 3.57-3.46 (m, 2H), 2.83 - 2.71 (m, 1H), 2.67-2.56 (m, 1H); $^{19}F$ NMR (376 MHz, $D_2O$) δ ppm -195.88 (s, 1F); $^{31}P$ NMR (162 MHz, $D_2O$) δ ppm -1.513 (s, IP); ESI -MS: *m*/*z* = 661.1101 $[M + H]^+$.

**Sodium salt conversion:** Dowex 50W × 8, 200-400 (8 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was pH neutral. The resin was transferred back into the beaker, 15% NaOH in deionized water solution was added, and mixture was gently stirred for 5 min, and decanted (1 x). The resin was transferred to the column and washed with 15% NaOH in WATER (at least 4 CV), and then with deionized water until it was pH neutral. **Compound 45, ammonium salt** (6.9 mg) was dissolved in a minimal amount of deionized water and $CH_3CN$ (1:1, v/v, 5 mL), added to the top of the column, and eluted with deionized water. Appropriate fractions were pooled together and lyophilized to give **compound 45, sodium salt** (2 mg) as a white solid. $^1H$ NMR (400 MHz, $D_2O$) δ ppm 8.20 (s, 1H), 7.74 (br, s, 1H), 6.58 (dd, J= 5.1, 11.7 Hz, 1H), 6.37 *(d, J*= 7.1 Hz, 1H), 6.24-6.13 (m, 1H), 5.69-5.51 (m, 1H), 5.42-5.29 (m, 1H), 4.85-4.83 (m, 1H), 4.70 (br, dd, *J* = 2.3, 6.2 Hz, 1H), 4.43 (br, d, *J* = 3.4 Hz, 1H), 4.23- 4.10 (m, 2H), 3.64-3.50 (m, 2H), 2.91-2.78 (m, 1H), 2.73-2.64 (m, 1H); $^{19}F$ NMR (376 MHz, $D_2O$) δ ppm -195.902 (s, IF); $^{31}P$ NMR (162 MHz, $D_2O$) δ ppm -1.54 (s, IP); ESI -MS: *m*/*z* = 661.2 $[M+H]^+$.

<u>Example 30</u>

**Compound (*R) 50A**

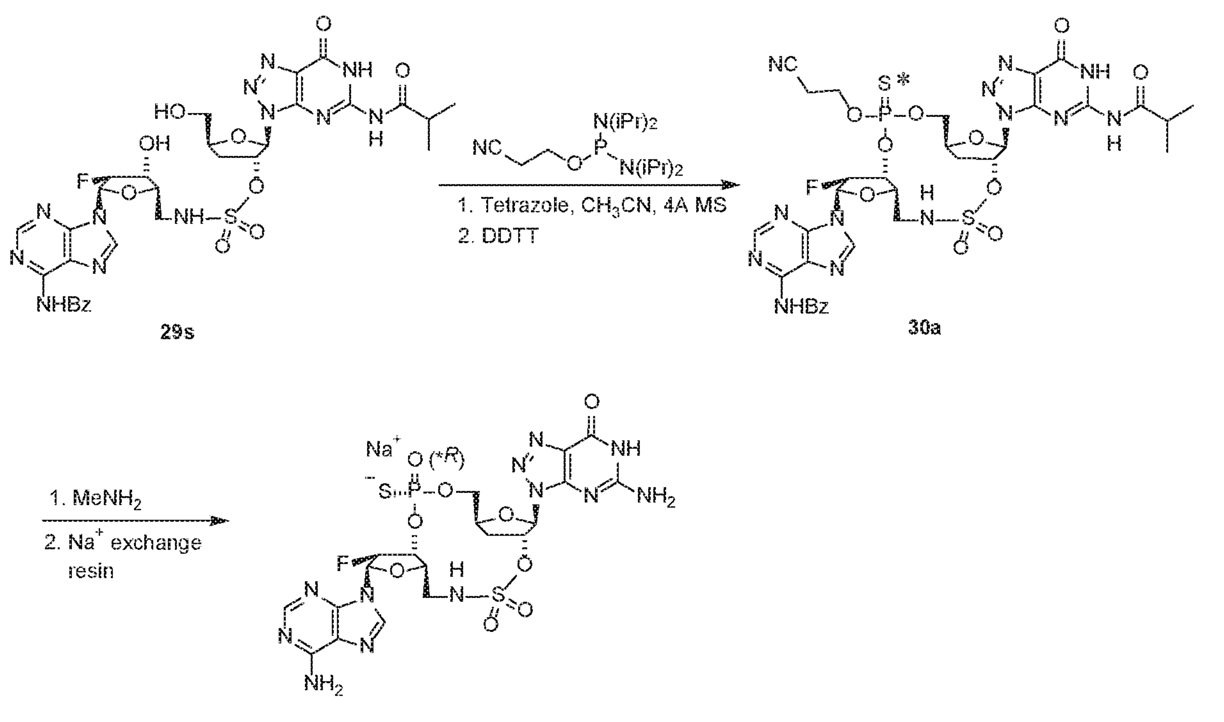


**Compound (*R) 50A, sodium salt**

Step 1: Preparation of compound **30a**

Note: THF was freshly distilled over Na/benzophenone and $CH_3CN$ was freshly distilled over $CaH_2$ before use.

Compound **29s** (200 mg, 0.26 mmol) was dissolved in THF (6 mL) and $CH_3CN$ (10 mL), to which were added 0.3 g of 4Å MS (powder) and a solution of 1H-tetrazole (4.6 mL, 0.45 M, dried on activated molecular sieves before use). A solution of 2-cyanoethyl-*N,N,N',N'*-tetra(isopropyl)phosphorodiamidite (156 mg, 0.52 mmol) in THF (0.9 mL) was added drop-wise over 10 min vial a syringe. The resulting white suspension was further stirred for 2 hr at 35 °C under argon. A solution of DDTT (264.9 mg, 1.29 mmol) in pyridine (10 mL) was then added into the above solution at 35 °C. After stirring the reaction at 35 °C for 1 h, the mixture was diluted with EtOAc (20 mL), filtered through a pad of diatomaceous earth, the pad washed with EtOAc and the filtrate evaporated under reduced pressure to give the crude product The residue was purified by silica gel column chromatography (MeOH in DCM=0% to 7 %) to give compound 30a (80 mg) as a white solid. ESI -MS: m/z = 904.2 $[M+H]^+$.

Step 2: Preparation of **compound (****R***) 50A, sodium salt**

Compound **30a** (80 mg) was treated with $MeNH_2$ (5 ml, in EtOH, 30%) at 25 °C. After stirring the reaction at 25 °C for 3 h, the solvent was evaporated under reduced pressure to give a residue. The residue was dissolved in a mixture of $H_2O/CH_3CN$ (30 mL) and washed with DCM (3x15 mL). The aqueous phase was lyophilized to give the crude product (71 mg) as a yellow solid. The residue was purified by reverse preparative HPLC (Method: Column, Waters Xbridge Prep OBD 5 $\mu$m C18 150x30; Condition water (10mM NH4HCO3)(A)-ACN(B) Begin B 0 End B 23; Gradient Time(min) 7; 100%B Hold Time(min) 0 FlowRate (ml/min) 25) to give **compound (****R***) 50A, ammonium salt** (8 mg, 9%) as a white solid. $^1$H NMR (400 MHz, $D_2O$) $\delta$ 8.63 (br s, 1H), 8.51 (d, J=1.7 Hz, 1H), 6.94 (br d, J=18.8 Hz, 1H), 6.65 (br d, J=3.4 Hz, 1H), 6.39 (br d, J=2.9 Hz, 1H), 6.20 - 6.00 (m, 1H), 5.71 (br t, J=11.4 Hz, 1H), 5.06 (br s, 1H), 4.70 - 4.60 (m, 1H), 4.56 - 4.47 (m, 1H), 4.04 - 3.96 (m, 1H), 3.94 - 3.85 (m, 1H), 3.27 - 3.17 (m, 1H), 3.16 - 3.03 (m, 1H); $^{19}$F NMR (376 MHz, $D_2O$) $\delta$ ppm -195.682

$^{31}$P NMR (162 MHz, $D_2O$) $\delta$ ppm 54.318; ESI -MS: m/z 677.2 $[M+H]^+$.

**Conversion to sodium salt**

Dowex 50W $\times$ 8, 200-400 (2 mL, H form) was added to a beaker and washed with deionized water (10 mL). Then to the resin was added 15% $H_2SO_4$ in deionized water, the mixture was gently stirred for 5 min, and decanted (15 mL). The resin was transferred to a column with 15% $H_2SO_4$ in deionized water and washed with 15% $H_2SO_4$ (at least 4 CV), and then with deionized water until it was pH neutral. The resin was transferred back into the beaker, 15% NaOH in deionized water solution was added, and mixture was gently stirred for 5 min, and decanted (1 $\times$). The resin was transferred to the column and washed with 15% NaOH in water (at least 4 CV), and then with deionized water until it was pH neutral. **Compound (****R***) 50A, ammonium salt** (4 mg) was dissolved in a minimal amount of deionized water: $CH_3CN$ (3:1, v/v, 8 mL), added to the top of the column, and eluted with deionized water. Appropriate fractions were pooled together and lyophilized to give **compound (****R***)** 50A, **sodium salt** (2.4 mg, 58%) as a white solid.

$^1$H NMR (400 MHz, $D_2O$) $\delta$ ppm 8.25 (s, 1H), 7.88 (s, 1H), 6.59 (dd, J=4.3, 15.5 Hz, 1H), 6.33 (d,J=6.8 Hz, 1H), 6.15 - 6.01 (m, 1H), 5.86 - 5.66 (m, 1H), 5.54 - 5.41 (m, 1H), 4.47 (br d, J=3.9 Hz, 1H), 4.29 - 4.12 (m, 2H), 3.65 - 3.54 (m, 2H), 2.88 - 2.79 (m, 1H), 2.76 - 2.69 (m, 1H); $^{19}$F NMR (376 MHz, $D_2O$) $\delta$ ppm -194.41

$^{31}$P NMR (162 MHz, $D_2O$) $\delta$ ppm 53.83; ESI-MS: m/z = 677.1 $[M+H]^+$.

Example 31

**Compound 51**

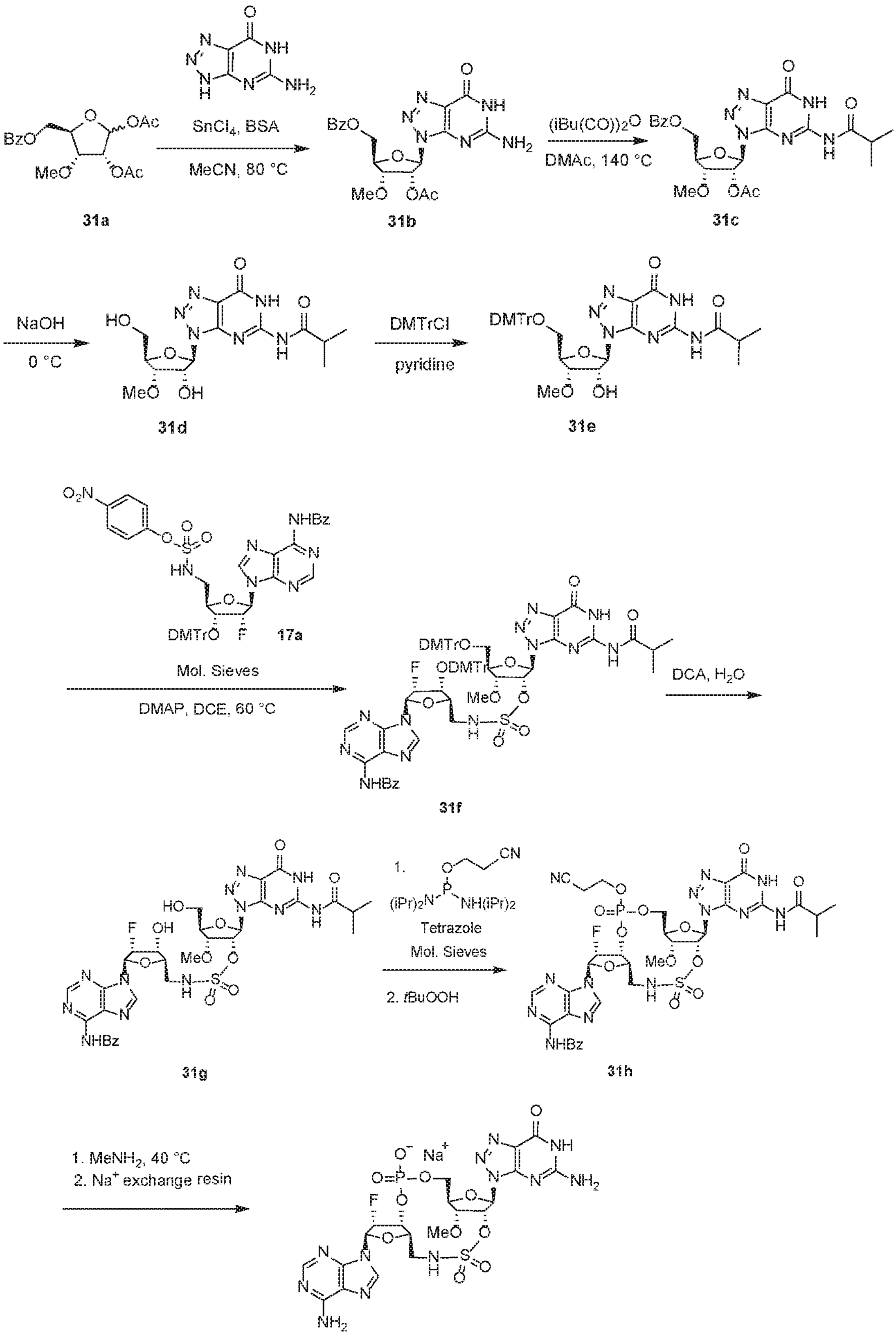


31a 31b 31c 31d 31e 17a 31f 31g 31h

**Compound 51, sodium salt**

Step 1: Preparation of compound **31b**

Bis(trimethylsilyl)acetamide (BSA, 93.95 g, 461.9 mmol) was added dropwise to a suspension of 8-azaguanine (11.71 g, 77.0 mmol) in anhydrous MeCN (340 mL) at 25 °C. The reaction mixture was stirred at 80 °C for 3 h and then cooled to room temperature. A solution of 1,2-di-O-acetyl-5-benzoyl-3-O-methyl-D-ribofuranose **31a** [CAS 10300-21-7] (13.56 g, 38.5 mmol) in anhydrous MeCN (65 mL) was added followed by the dropwise addition of $SnCl_4$ (80.21 g, 307.9 mmol). The homogeneous solution was stirred at 80 °C for 30 min, then cooled to room temperature and poured into an ice-cold 5% aqueous $NaHCO_3$ solution (800 mL). EtOAc (800 mL) was added and stirring was continued for 10 min. The reaction mixture was filtered and the filtrate was transferred to a separatory funnel. The two layers were separated and the water layer was extracted with EtOAc (200 mL x 2). The combined organic layers were washed with 5% aqueous $NaHCO_3$ (600 mL x 2), dried over $Na_2SO_4$, and evaporated to dryness in vacuo to give crude compound **31b** (11.50 g). The crude product was used directly in the next reaction without further purification. $^1H$ NMR (600 MHz, DMSO-$d_6$) $\delta$ ppm 11.09 (br s, 1 H), 7.88 (d, *J*=7.2 Hz, 2 H), 7.61 - 7.68 (m, 1 H), 7.44 - 7.52 (m, 2 H), 7.04 (br s, 2 H), 6.17 (d, *J*=1.7 Hz, 1 H), 5.90 (dd, *J*=5.0, 2.1 Hz, 1 H), 4.66 (dd, *J*=7.1, 4.9 Hz, 1 H), 4.53 - 4.58 (m, 1 H), 4.35 - 4.42 (m, 2 H), 3.41 (s, 3 H), 2.14 (s, 3 H); ESI-MS: *m/z* 445.5 $[M+H]^+$.

Step 2: preparation of compound **31c**

To a solution of the above crude compound **31b** (11.50 g) in anhydrous dimethylacetamide (57.5 mL) was added dropwise isobutyric anhydride (6.14 g, 38.8 mmol). The reaction mixture was stirred at 140 °C for 2 h, then cooled to room temperature and diluted with EtOAc (300 mL). The resulting solution was washed with 10% aqueous $NH_4Cl$ (300 mL x 3) and brine (300 mL). The organic layer was dried over $Na_2SO_4$ and concentrated to dryness in vacuo to give crude compound **31c** (13.30 g). The crude product was used directly in the next reaction without further purification.

Step 3: preparation of compound **31d**

To a solution of crude compound **31c** (11.98 g, from Step 2) in THF (42 mL), MeOH (35 mL) and $H_2O$ (11 mL), was added 5 N NaOH (11 mL, 55 mmol) at 0 °C. The reaction mixture was stirred at 0 °C for 4 h. The pH of the reaction mixture was adjusted to 6.5 with formic acid. The resulting solution was concentrated in vacuo to dryness and the residue was triturated with MeCN and $H_2O$ several times to give compound **31d** (1.55 g, yield: 14% from 31b). $^1H$ NMR (600 MHz, DMSO-$d_6$) $\delta$ ppm 12.07 (br s, 2 H), 5.97 (d, *J*=4.4 Hz, 1 H), 5.68 (br s, 1 H), 4.92 (br s, 1 H), 4.84 (br s, 1 H), 4.01 - 4.08 (m, 2 H), 3.52 - 3.59 (m, 1 H), 3.44 - 3.50 (m, 1 H), 3.43 (s, 3 H), 2.79 (spt, *J*=6.8 Hz, 1 H), 1.13 (d, *J*=6.8 Hz, 6 H); ESI-MS: *m/z* 369.5 $[M+H]^+$.

Step 4: preparation of compound 31e

To a solution of compound 31d (1.10 g, 3.0 mmol) in anhydrous pyridine (20.0 mL) was added a solution of DMTrCl (1.32 g, 3.9 mmol) in pyridine (3.0 mL) at 0 °C. The resulting mixture was stirred at room temperature for 12 h after which it was diluted with EtOAc and washed with saturated $NaHCO_3$, water, and brine. The organic layer was dried over $Na_2SO_4$, filtered, and concentrated to dryness at 40 °C. The residue was purified by silica gel column chromatography (gradient elution: 20 - 50% EtOAc in heptane) to give compound 31e as a white solid (1.52 g, yield: 76%). $^1H$ NMR (500 MHz, DMSO-$d_6$) $\delta$ ppm 11.87 (br s, 1 H), 7.24 - 7.28 (m, 2 H), 7.10 - 7.24 (m, 7 H), 6.76 - 6.82 (m, 4 H), 6.06 (d, *J*=2.7 Hz, 1 H), 5.79 (br d, *J*=4.6 Hz, 1 H), 5.01 (br s, 1 H), 4.18 - 4.24 (m, 2 H), 3.71 (s, 6 H), 3.36 (s, 3 H), 3.15 - 3.20 (m, 1 H), 3.05 (br dd, *J*=10.0, 3.9 Hz, 1 H), 2.80 (spt, *J*=6.8 Hz, 1 H), 1.13 (d, *J*=6.9 Hz, 6 H); ESI-MS: *m/z* 671.4 $[M+H]^+$.

Step 5: preparation of compound 31f

Compound 31e (1.30 g, 1.94 mmol), DMAP (546 mg, 4.47 mmol) and sulfamate 17a (1.18 g, 1.33 mmol) were dissolved separately in dry DCE ($3 \times 3$ mL). Each solution was dried with 3Å activated molecular sieves by stirring under $N_2$ overnight To the solution of sulfamate 17a in DCE, were respectively added the solution of DMAP in DCE and the solution of compound 31e in DCE (added via a syringe). The reaction mixture was stirred at 60 °C under $N_2$ for 4 h. This mixture was diluted with DCM and filtered, the filtrate was washed with 2% aqueous acetic acid, 5% aqueous $NaHCO_3$, and brine solution consecutively, and then evaporated to dryness under reduced pressure. The residue was dissolved in DCM and purified by column chromatography over silica gel (gradient elution: 0.6 - 1% EtOH in DCM) to give compound 31f as a yellow solid (0.53 g, yield: 28%). ESI-MS: m/z 1407.6 $[M+H]^+$.

Step 5: preparation of compound 31g

A solution compound **31f**(480 mg, 0.34 mmol) in DCM (5 mL) to which water (30.8 mg, 1.71 mmol) and DCA (220 mg, 1.71 mmol) were added, was stirred for 4 h at room temperature. Next, the reaction mixture was washed with 5% aqueous $NaHCO_3$ and brine. The organic phase was dried over $Na_2SO_4$ and concentrated to dryness under reduced pressure. The residue was purified by column chromatography over silica gel (gradient elution: 1 - 5% EtOH in DCM) to give compound 31g as a white solid (150 mg, yield: 55%). $^{1}$H NMR (500 MHz, DMSO-$d_6$) δ ppm 11.90 (br s, 1 H), 11.27 (br s, 1 H), 8.74 (s, 1 H), 8.62 (s, 1 H), 8.04 (d, *J*=7.6 Hz, 2 H), 7.61 - 7.70 (m, 1 H), 7.51 - 7.59 (m, 2 H), 6.38 (d, *J*=19.5 Hz, 1 H), 6.29 (d, *J*=2.3 Hz, 1 H), 5.51 - 5.69 (m, 2 H), 4.90 (br s, 1 H), 4.63 (ddd, *J*=20.4, 7.6, 4.6 Hz, 1 H), 4.47 (br t, *J*=5.5 Hz, 1 H), 4.00 - 4.12 (m, 2 H), 3.55 - 3.61 (m, 1 H), 3.42 - 3.47 (m, 2 H), 3.39 (s, 3 H), 3.21 - 3.29 (m, 1 H), 2.77 (spt, *J*=6.7 Hz, 1 H), 1.11 (d, *J*=6.9 Hz, 6 H); ESI-MS: *m*/*z* 803.7 $[M+H]^+$.

Step 6: preparation of compound 31h

A solution of compound 31g (100 mg, 0.125 mmol) and 1*H*-tetrazole (2.22 mL, 0.45 M solution in MeCN, 0.998 mmol) in dry THF (2 mL) was treated with 4Å molecular sieves for 1 h under $N_2$ after which 2-cyanoethyl-*N,N,N',N'*-tetra(isopropyl)- phosphorodiamidite (67.5 mg, 0.22 mmol) in dry MeCN (0.6 mL) was added dropwise over 15 min (note: THF was freshly distilled over Na/benzophenone and MeCN was freshly distilled over $CaH_2$ before use). The resulting reaction mixture was stirred for 90 min at room temperature. A solution of *t*BuOOH (199 µL, 0.99 mmol) was added and stirring was continued for another 30 min. The reaction mixture was filtered through a pad of Diatomaceous earth and concentrated. The residue was purified by silica column chromatography (gradient elution: 0 - 10% MeOH in DCM) to give compound 31h as a white solid (32 mg, yield: 30%). ESI-MS: *m*/*z* 918.4 $[M+H]^+$.

Step 7: preparation of **compound** 51, **sodium salt**

Compound **31h** (32 mg, 0.035 mmol) was stirred in a 33% methylamine solution in ethanol (12 mL) at 40 °C for 2.5 h. The reaction mixture was concentrated under reduced pressure. The residue was dissolved in water, washed with DCM and lyophilized. The crude product was purified by preparative reversed phase HPLC (Stationary phase: Xbridge OBD C18, 5 µm, 150 x 30 mm; Mobile phase: 10 mM aqueous ammonia bicarbonate (A) - MeCN (B); gradient elution). Final conversion into the sodium salt was done by elution of an aqueous solution over a column packed a cationic sodium ion-exchange resin to give **compound** 51, **sodium salt** as a white fluffy solid after lyophilization (14 mg, yield: 55%). $^{1}$H NMR (400 MHz, $D_2O$) δ ppm 8.15 (br s, 1H), 7.10 (br s, 1H), 6.51 - 6.45 (m, 1H), 6.41 (br s, 1H), 6.16 (br s, 1H), 5.36 - 5.12 (m, 2H), 4.61 (br d, *J* = 2.4 Hz,1H), 4.52 (br d, *J*= 8.8 Hz, 1H), 4.38 (d, *J*= 4.8 Hz, 1H), 4.23 (dd, *J* = 2.4,4.8 Hz, 2H), 3.77 (br d, *J* = 13.2 Hz, 1H), 3.57 (s, 3H), 3.42 (br d, *J*= 12.8 Hz, 1H); $^{31}$P NMR (162 MHz, $D_2O$) δ ppm -1.81 (s, IP); $^{19}$F NMR (376 MHz, $D_2O$) δ ppm - 196.87-197.38 (m, 1F); ESI-MS: *mlz* 691.0 $[M+H]^+$.

<u>Example 32</u>

**Compound 25**

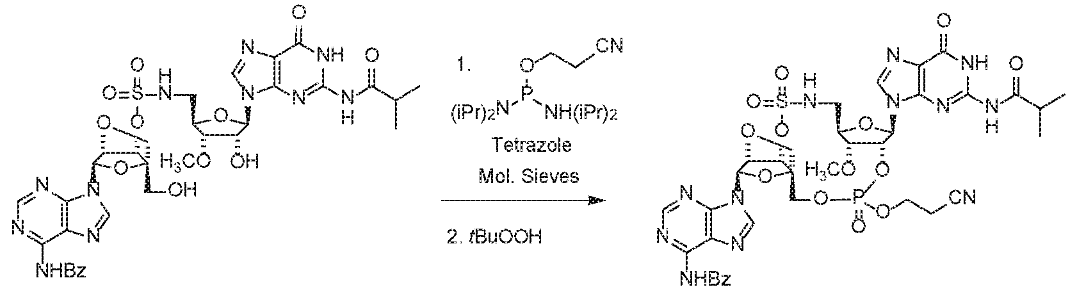


3q 32a

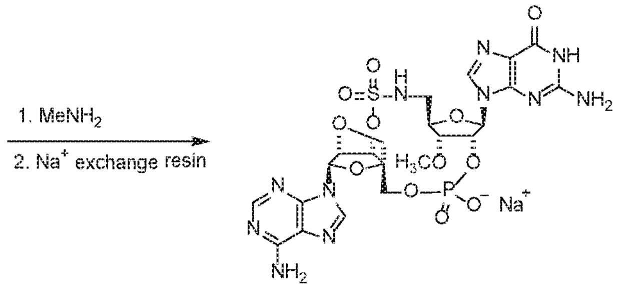


**Compound 25, sodium salt**

Preparation of **compound 32a**

THF was freshly distilled over Na/benzophenone and $CH_3CN$ was freshly distilled over $CaH_2$.

Vacuum-dried diol **3q** (250 mg, 0.307 mmol) was co-evaporated with a mixture of $CH_3CN$ / THF (8/5 mLx3) and dissolved in a mixture of $CH_3CN$ / THF (7.5/5 mL). It was added 600 mg of activated 4Å Molecular Sieves and a solution of 1H-tetrazole in $CH_3CN$ (5.47 mL, 0.45M, prepared by dissolving 630 mg of tetrazole in 20 mL of dry $CH_3CN$, followed by addition of 600 mg of 4Å Molecular Sieves and then stirred for 1hr under Argon before use). After stirring the white suspension for 1hr at 8°C under Argon, a solution of 2-Cyanoethyl N,N,N',N'-tetraisopropylphosphorodiamidite in $CH_3CN$ (4.69 mL, 0.49 mmol, 0.105M in $CH_3CN$, prepared by dissolving 506 mg of amidite in 16 mL of $CH_3CN$, followed by addition of 600 mg of 4Å Molecular Sieves and then stirred for 1hr under Argon before use) was added dropwise over 60 min. The resulting white suspension was stirred for 1hr at 8°C under Argon. More $CH_3CN$ (5 mL) was added and after stirring for 1hr at 30°C, additional tetrazole (1.36 mL, 0.61 mmol) was added. After stirring for an additional 2 hr, TBHP (0.5 mL, 2.5 mmol, 5M in decane) was added rapidly. After stirring for 30 min, the mixture was filtered through a pad of diatomaceous earth; the filtrate was combined with another batch and concentrated under reduced pressure to give a residue dissolved in DCM (8 mL) and purified by flash column chromatography on silica gel (0 - 6% MeOH in DCM, 25 mL/min) to afford 32a (136 mg) as a white solid. ESI-MS: m/z = 927 $[M+H]^+$.

The above solid was combined with another batch and purified further by reverse phase preparative HPLC (Column: Xtimate 5 μm C18 150 x 25; mobile phase water (10mM $NH_4HCO_3$)-ACN. Begin B 23, End B 53; Flow Rate: 25 mL/min Gradient Time: 9 min followed by B 100 for 3 min); desired fractions were collected and lyophilized to generate 36a as a white solid. $^1H$ NMR (400 MHz, $CD_3CN$) $\delta$ 9.36 (td, J=3.7, 7.2 Hz, 2H), 8.72 - 8.60 (m, 1H), 8.73 - 8.56 (m, 1H), 8.18 (s, 1H), 8.12 - 7.96 (m, 3H),7.86 (s, 1H), 7.82 (s, 1H), 7.71 - 7.62 (m, 1H), 7.62 - 7.51 (m, 2H), 6.21 (s, 1H), 6.06 - 5.94 (m, 1H), 5.46 - 5.37 (m, 1H), 5.37 - 5.29 (m, 1H), 5.28- 5.21 (m, 1H), 4.82 (dd, J=6.2, 11.1 Hz, 1H), 4.63 (dd, J=3.9, 11.2 Hz, 1H), 4.48 (dd, J=2.8, 11.4 Hz, 1H), 4.45 - 4.38 (m, 1H), 4.35 - 4.19 (m, 2H), 4.17 - 4.07 (m, 2H), 4.03 - 3.93 (m, 1H), 3.89 - 3.79 (m, 1H), 3.68 - 3.58 (m, 2H), 3.59 - 3.50 (m, 1H), 2.80 (t,J=5.9 Hz, 1H), 2.54 (td, J=5.2, 17.2Hz, 1H), 2.42 - 2.27 (m, 1H), 1.10 - 1.02 (m, 3H), 0.88 (d, *J*=6.8 Hz, 1H), 0.83 (d, J=6.8 Hz, 1H);
$^{31}P$ NMR (162MHz, $CD_3CN$) = 3.07 (s, 1P), 3.15 (s, 1P); ESI-MS: m/z = 927.3 $[M+H]^+$.

Preparation of **compound 25, sodium salt**

A solution of compound **32a** (30.5 mg, 0.033 mmol) in $MeNH_2$ (27-30% in EtOH, 5 mL) was stirred at 5°C for

4 hr. The reaction mixture was concentrated under reduced pressure to give a residue; the residue was partitioned between DCM / water (10 / 15 mL). The aqueous layer was washed with DCM (8 mL x 3) and lyophilized. The crude compound was suspended in 6 mL of ethyl acetate, sonicated (3min) and centrifuged (5min). The above supernate was collected and the previous procedure repeated twice. The precipitate was partitioned between DCM / water (10 / 15 mL). The aqueous layer was extracted with DCM (10 mL x 2) and lyophilized to give a solid. Final conversion into the sodium salt was done by elution of an aqueous solution over a column packed a cationic sodium ion-exchange resin to give **compound 25, sodium salt** as a white fluffy solid after lyophilization (60.1 mg). $^{1}H$ NMR (400 MHz, $D_2O$) $\delta$8.15 - 7.90 (m, 1H), 7.83 (br s, 2H), 7.69 (br s, 1H), 6.90 - 6.72 (m, 1H), 6.08 - 5.82 (m, 1H), 4.45 - 4.15(m,3H), 4.16 - 3.88 (m, 1H), 3.28 (t, J=6.8 Hz, 1H), 2.87 (t, J=6.7 Hz, 1H), 2.68 - 2.59 (m, 1H), 2.46 (s, 4H); $^{31}P$ NMR (162MHz, $D_2O$) $\delta$ - 2.4 (s, IP); ESI-MS: m/z = 700 $[M+H]^+$.

<u>Example 33</u>

**Compounds (*R) 23A and (*S) 23B**

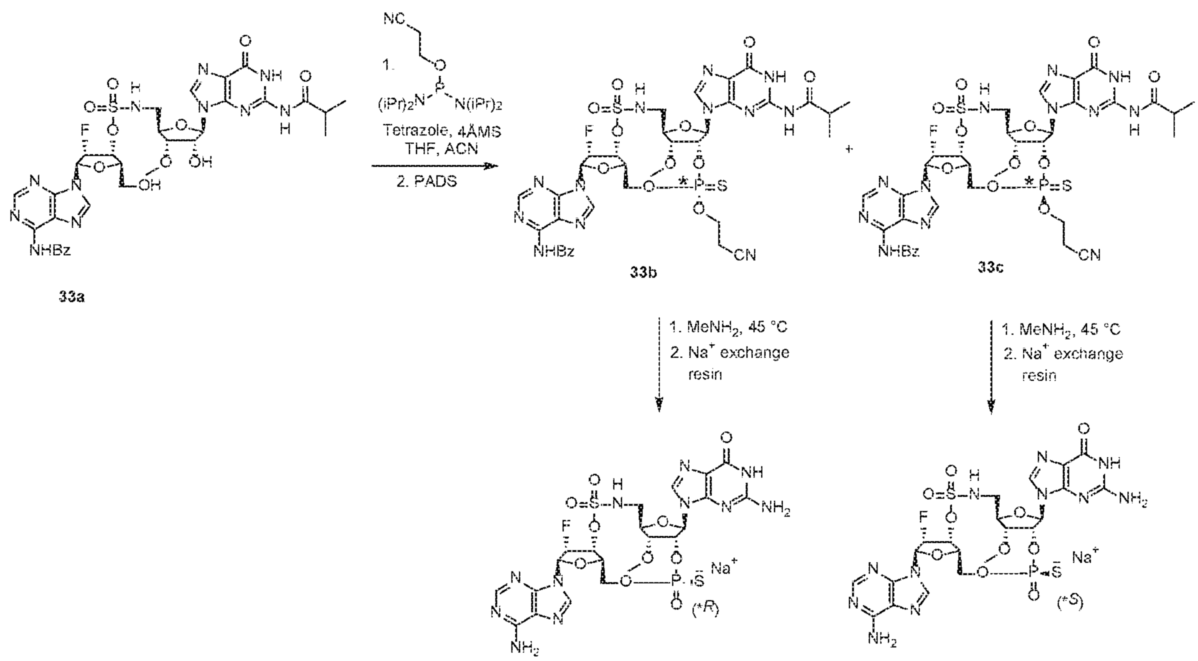


**Compound (R*) 23A, sodium salt** **Compound (S*) 23B, sodium salt**

Step 1: Preparation of compounds **33b** and 33c

A solution of compound 33a (600 mg, 0.75 mmol) and 1H-tetrazole (8.97 mL of a 3 - 4% in MeCN, dried on 3Å molecular sieves before use) in 1:1 MeCN / THF (110 mL, dried on 3Å molecular sieves before use) was treated with activated 3Å molecular sieves under $N_2$ for 1 h after which 2-cyanoethyl-*N,N,N',N'*-tetra(isopropyl)phosphorodiamidite (240 μL, 0.75 mmol) was added at once. The reaction mixture was shaken at room temperature for 3.5 h. An extra amount of 2-cyanoethyl-*N,N,N',N'*-tetraisopropyl-phosphorodiamidite (0.12 mL, 0.37 mmol) was added and shaking was continued overnight. Next, phenylacetyl disulfide (PADS, 0.45 g, 1.5 mmol) was added, the reaction mixture was shaken for an extra 18 hours. Molecular sieves were removed by filtration and rinsed with dichloromethane. The combined filtrates were subsequently washed with a 1:1 mixture of saturated aqueous $Na_2S_2O_3$ and saturated aqueous NaHCO3, and brine, dried with MgSO4, filtered and concentrated under reduced pressure. The residue was purified by silica column chromatography (gradient elution: 0 - 10% MeOH in DCM) to give compound 33b (122 mg, yield: 17%) as the first eluting isomer and compound 33c (39 mg, yield: 5%) as the second eluting isomer. Compound 33b ESI-MS: *m/z* 933.5 $[M+H]^+$; Compound 33c ESI-MS: *m/z* 933.6 $[M+H]^+$.

Step 2: Preparation of **compound (*R**) 23A, sodium salt** and **compound (*S**) 23B, sodium salt**

Compound **33b** (122 mg, 0.13 mmol) was stirred in a 33% methylamine solution in ethanol (7 mL) at 45 °C until complete conversion (ca.1 h). The reaction mixture was cooled to room temperature and concentrated under reduced pressure. The resulting crude product was triturated in MeCN followed by preparative reversed phase HPLC purification (Stationary phase: XBridge C18 OBD, 5 μm, 150 × 50 mm; Mobile phase: aqueous 0.25% ammonia bicarbonate (A) - MeOH (B); gradient elution) to give **compound (*R**) 23A.** Final conversion into the sodium salt was done by elution of an aqueous solution over a column packed with a cationic sodium ion-exchange resin to give **compound (*R**) 23A, sodium salt** as a white fluffy solid after lyophilization (13 mg, yield: 14%). $^{1}H$ NMR (400 MHz, $D_2O$) δ ppm 8.23 (s, 1 H), 8.19 (s, 1 H), 7.82 (br s, 1 H), 6.44 (d, *J*=15.5 Hz, 1 H), 5.92 (d, *J*=8.5 Hz, 1 H), 5.65 (dd, *J*=50.6, 3.4 Hz, 1 H), 5.42 (br s, 1 H), 5.17 (ddd, *J*=21.8, 9.2, 3.4 Hz, 1 H), 4.66 - 4.71 (m, 1 H), 4.46 - 4.58 (m, 2 H), 4.15 (br s, 1 H), 4.00 (br dd, *J*=12.2,4.1 Hz, 1 H), 3.69 - 3.90 (m, 2 H), 3.60 (s, 3 H); $^{31}P$ NMR (162 MHz, $D_2O$) δ ppm 52.18 (s, 1 P); ESI-MS: *m/z* 706.4 $[M+H]^+$.

Compound **33c** (39 mg, 0.035 mmol) was stirred in a 33% methylamine solution in ethanol (2 mL) at 45 °C until complete conversion (ca.1 h). The reaction mixture was cooled to room temperature and concentrated under reduced pressure. The resulting crude product was triturated in MeCN followed by preparative reversed phase HPLC purification (Stationary phase: XBridge C18 OBD, 10 μm, 150 × 50 mm; Mobile phase: aqueous 0.25% ammonia bicarbonate (A) - MeOH (B); gradient elution) to give **compound (*S**) 23B.** Final conversion into the sodium salt was done by elution of an aqueous solution over a column packed with a cationic sodum ion-exchange resin to give **compound (*S**) 23B, sodium salt** as a white fluffy solid after lyophilization (18 mg, yield: 70%). $^{1}H$ NMR (400 MHz, $D_2O$) δ ppm 8.43 (s, 1 H), 8.20 (s, 1 H), 7.88 (br s, 1 H), 6.52 (d, *J*=15.9 Hz, 1 H), 5.97 (d, *J*=8.5 Hz, 1 H), 5.81 (dd, *J*=50.5, 3.7 Hz, 1 H), 5.45 - 5.60 (m, 1 H), 5.33 - 5.44 (m, 1 H), 4.67 - 4.73 (m, 1 H), 4.49 - 4.53 (m, 1 H), 4.43 (dt, *J*=11.7, 3.7 Hz, 1 H), 4.17 (br s, 1 H), 4.05 - 4.11 (m, 1 H), 3.78 (m, *J*=9.8 Hz, 2 H), 3.62 (s, 3 H); $^{31}P$ NMR (162 MHz, $D_2O$) δ ppm 56.24 (s, 1 P); ESI-MS: *m/z* 706.4 $[M+H]^+$.

Example 34

**Compound (*R) 32A and compound (*S) 32B**

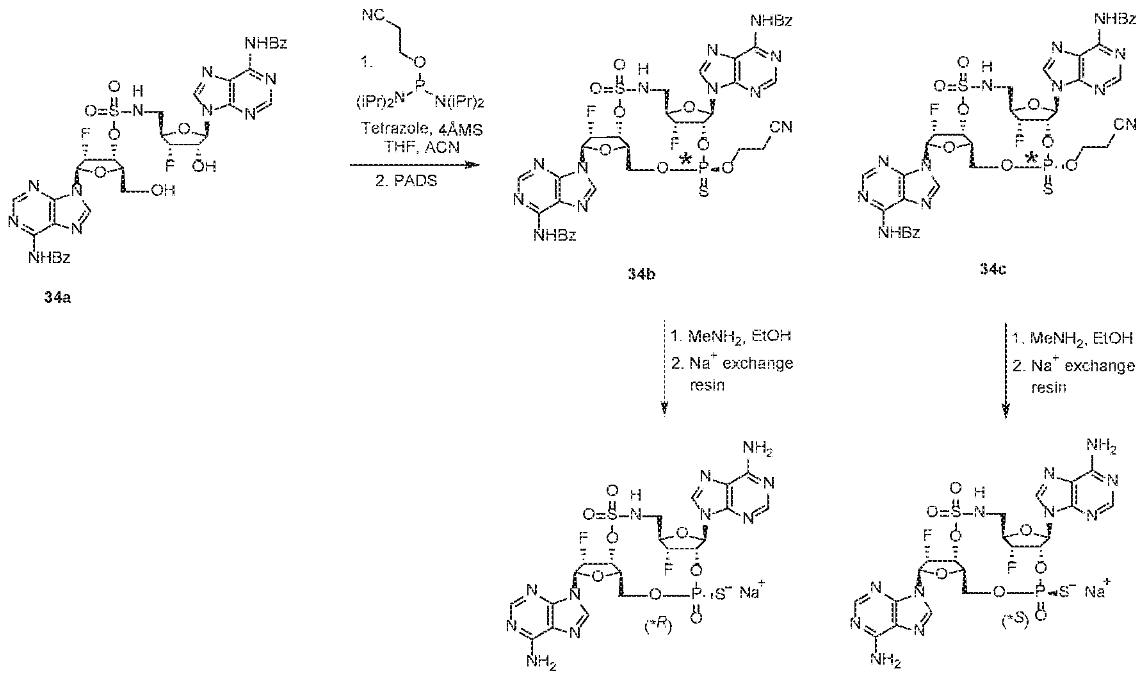


34a 34b 34c

**Compound (*R) 32A, sodium salt Compound (*S) 32b, sodium salt**

Step 1: Preparation of compound **34b** and compound **34c**

Compound **34a** (0.8 g, 0.99 mmol) was dissolved in a mixture of anhydrous ACN (85 mL) and anhydrous THF (85 mL). 1H-tetrazole (11.5 mL, 3.96 mmol) and 3Å molecular sieves were added. The mixture was shaken for 1 hour at RT and then 2-cyanoethyl-N,N,N',N'-tetraisopropylphosphorodiamidite (0.31 mL, 0.99 mmol) was added at once via syringe. The reaction mixture was shaken at room temperature for 4.5 hours. An additional amount of 2-cyanoethyl-N,N,N',N'-tetraisopropylphosphorodiamidite (0.16 mL, 0.5 mmol) was added. The reaction mixture was shaken at RT for 2 days and then phenylacetyl disulfide (PADS, 0.6 g, 1.98 mmol) was added. The reaction mixture was shaken at RT for 18 hours. The reaction mixture was filtered. The molecular sieves were washed three times with dichloromethane. The combined filtrate was washed with a mixture of a saturated $Na_2S_2O_3$ solution and a saturated NaHCO3 solution, washed with brine, dried with $MgSO_4$, filtered and the solvents of the filtrate evaporated. The residue was purified by silica column chromatography (gradient elution: 0 - 10% MeOH in DCM) to give compound **34b** (224 mg, yield: 16%) as the first eluting isomer and compound **34c** (265 mg, yield: 11%) as the second eluting isomer.

Compound **34a** ESI-MS: *m*/*z* 939.5 $[M+H]^+$;
Compound **34b** ESI-MS: *m*/*z* 939.5 $[M+H]^+$.

Step 2: Preparation of **compound (*R), 33A sodium salt** and **compound 33b, sodium salt**

Compound **34a** (224 mg, 0.16 mmol) was stirred in a 33% methylamine solution in ethanol (6.5 mL) at 45 °C until complete conversion (ca.1 h). The reaction mixture was cooled to room temperature and concentrated under reduced pressure. The resulting crude product was triturated in MeCN followed by preparative reversed phase HPLC purification (Stationary phase: XBridge C18 OBD, 5 μm, 150 × 50 mm; Mobile phase: aqueous 0.25% ammonia bicarbonate (A) - MeOH (B); gradient elution) to give **compound (*R), 32A ammonium salt.** Final conversion into the sodium salt was done by elution of an aqueous solution over a column packed with cationic sodium ion-exchange resin to give **compound (*R), 32A sodium salt** as a white fluffy solid after lyophilization (31 mg, yield: 27%). $^1H$ NMR (400 MHz, DMSO-$d_6$) δ ppm 8.77 (br s, 1 H), 8.32 (s, 1 H), 8.18 (s, 1 H), 8.13 (s, 1 H), 7.34 (br s, 2 H), 7.18 (br s, 2 H), 6.22 (dd, *J*=15.3,2.4 Hz, 1 H), 6.01 (d, *J*=8.6 Hz, 1 H), 5.70 - 5.84 (m, 1 H), 5.59 (br d, *J*=51.7 Hz, 1 H), 5.30 (dd, *J*=54.9, 3.3 Hz, 1 H), 5.03 (br d, *J*=17.1 Hz, 1 H), 4.15 - 4.29 (m, 3 H), 3.70 - 3.79 (m, 1 H), 3.20 (d, *J*=15.4 Hz, 1 H), 3.00 - 3.11 (m, 1 H); $^{31}P$ NMR (162 MHz, DMSO-$d_6$) δ ppm 52.83 (s, 1 P); ESI-MS: *m*/*z* 678.4 $[M+H]^+$.

Compound **34b** (265 mg, 0.11 mmol) was stirred in a 33% methylamine solution in ethanol (2 mL) at 45 °C until complete conversion (ca.1 h). The reaction mixture was cooled to room temperature and concentrated under reduced pressure. The resulting crude product was triturated in MeCN followed by preparative reversed phase HPLC purification (Stationary phase: XBridge C18 OBD, 5 μm, 150 × 50 mm; Mobile phase: aqueous 0.25% ammonia bicarbonate (A) - MeOH (B); gradient elution) to give **compound (*S) 32b, ammonium salt.** Final conversion into the sodium salt was done by elution of an aqueous solution over a column packed with a cationic Na ion-exchange resin to give **compound (*S) 32b, sodium salt** as a white fluffy solid after lyophilization (20 mg, yield: 25%).

$^1H$ NMR (400 MHz, $D_2O$) δ ppm 8.22 (s, 1 H), 8.20 (s, 1 H), 8.04 (s, 1 H), 7.83 (s, 1 H), 6.45 (br d, *J*=15. 7 Hz, 1 H), 6.27 (br d, *J*=8.2 Hz, 1 H), 5.91 (br d, *J*=51.3 Hz, 1 H), 5.52 - 5.74 (m, 1 H), 5.23 - 5.51 (m, 2 H), 4.50 - 4.80 (solvent peak overlap, 4 H), 4.00 - 4.43 (m, 1 H), 3.59 - 3.98 (m, 1 H); $^{31}P$ NMR (162 MHz, $D_2O$) δ ppm 56.05 (s, 1 P); ESI-MS: m/z 678.1 $[M+H]^+$.

<u>Example 35</u>

## Compound 26

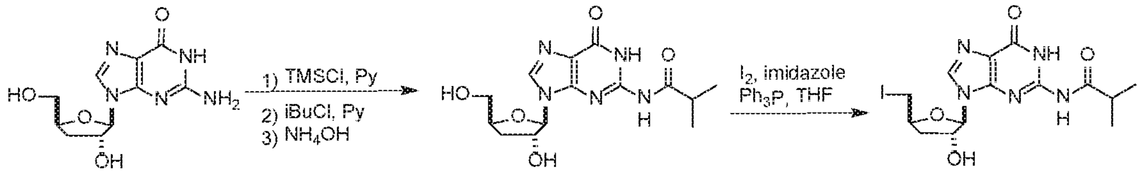


35a 35b 35c

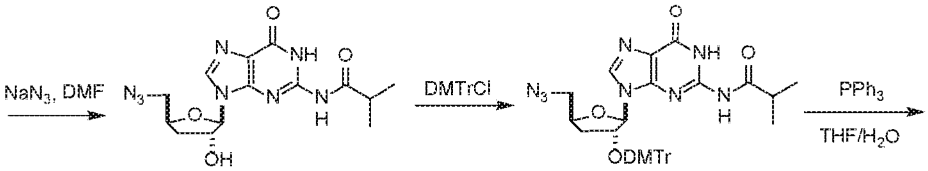


35d 35e

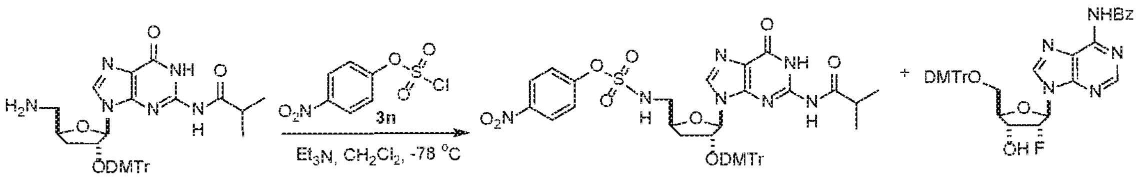


35f 35g 35h

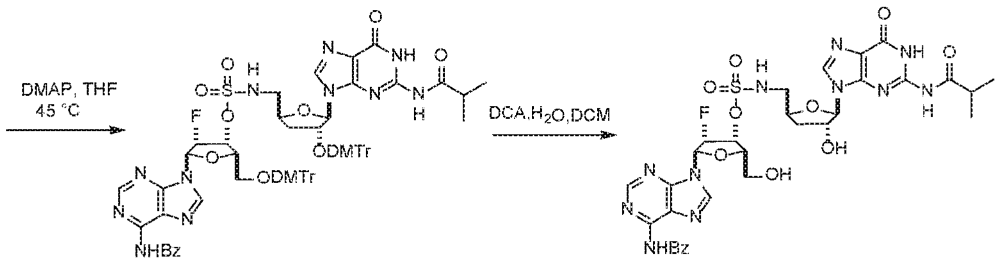


35i 35j

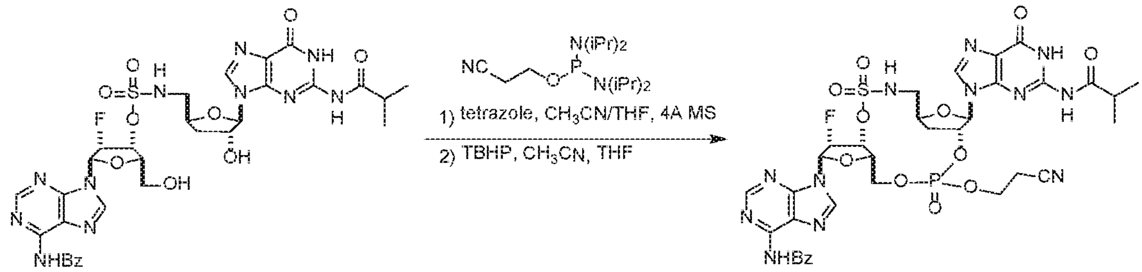


35j 35k

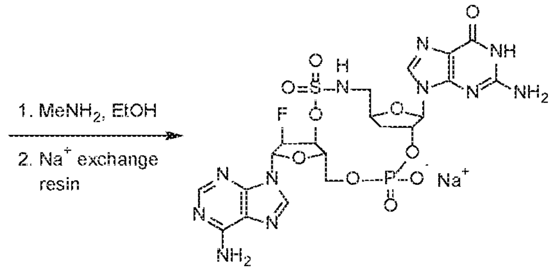


**Compound 26, ammonium salt** **Compound 26, sodium salt**

Step 1: preparation of Compound **35b**

Compound **35a** was co-evaporated with pyridine (30 mL) twice before use. To a stirred suspension of compound **35a** (3 g, 11.22 mmol) in pyridine (50 mL) was added dropwise via a pressure equalizing dropping funnel at 0 °C TMSC1 (7.2 mL, 56.73 mmol) over 30 min resulting in a white suspension, then further stirred at RT for 1 hr. Isobutyryl chloride (2.4 g, 22.52 mmol) was then added to the reaction mixture via a syringe at 0 °C under $N_2$ over 15 min. The resulting suspension was stirred at RT overnight under $N_2$ atmosphere. Water (15 mL) was added to the the reaction mixture at 0 °C, then ammonium hydroxide (17.3 mL, 25%) was added vial a pressure equalizing dropping funnel at 0 °C over 10 min. The resulting clear solution was further stirred at RT for 1 hr. The reaction mixture was concentrated under reduced pressure to give a residue. The residue was purified by flash column chromatography on silica gel (0% to 20% MeOH in DCM) to give **35b** as a yellow solid. $^1H$ NMR (DMSO-$d_6$, 400 MHz) $\delta$ ppm 12.12 (s, 1H), 11.71 (s, 1H), 8.30 (s, 1H), 5.79 (d, J = 1.6 Hz, 1H), 4.46-4.50 (m, 1H), 4.30-4.38 (m, 1H), 3.68 (br dd, J= 12.0, 3.2 Hz, 3H), 3.52 (br dd, J = 12.0, 3.6 Hz, 1H), 2.73-2.84 (m, 1H), 2.18-2.28 (m, 1H), 1.90 (ddd, J=13.2, 6.0, 2.0 Hz, 1H), 1.11 (d, J=6.8 Hz, 6H); ESI-MS: m/z 338.1 $[M+H]^+$.

Step 2: preparation of Compound **35c**

To a stirred solution of compound **35b** (3.4 g, crude), triphenylphosphine (7.93 g, 30.23 mmol) and imidazole (2.75 g, 40.4 mmol) in THF (35 mL) was added at 0 °C a solution of $I_2$ (7.68 g, 30.26 mmol) in THF (20 mL). After stirring the reaction overnight at 35 °C the mixture was filtered and the filtrate was concentrated, then diluted with DCM (150 mL) and washed with aqueous saturated $Na_2SO_3$ (100 mL $\times$ 2). The organic layer was dried with $Na_2SO_4$, filtered and concentrated under reduced pressure to give a residue. The residue was purified by flash column chromatography on silica gel (DCM:MeOH = 1:0 ~ 10:1) to give the compound **35c** (1.55 g) as a yellow solid. $^1H$ NMR (DMSO-$d_6$, 400 MHz) $\delta$ ppm 12.11 (s, 1H), 11.66 (s, 1H), 8.17 (s, 1H), 5.82 (d, J = 2.4 Hz, 1H), 5.74-5.76 (m, 1H), 4.65-4.70 (m, 1H), 4.30-4.39 (m, 1H), 3.43-3.55 (m, 2H), 2.78 (quin, J = 6.8 Hz, 1H), 2.12-2.25 (m, 2H), 1.12 (d, J = 6.8 Hz, 6H); ESI-MS: m/z 447.9 $[M+H]^+$.

Step 3: preparation of Compound **35d**

$NaN_3$ (800 mg, 12.3 mmol) was added to a stirred solution of compound **35c** (1.73 g, 3.87 mmol) in DMF (25 mL) under $N_2$. After stirring the reaction at 80 °C for 3 hr, the mixture was diluted with DCM (100 mL) and washed with brine (50 mL $\times$ 2). The organic layer was dried with anhydrous $Na_2SO_4$ and evaporated under reduced pressure to give the residue. The residue was purified by flash column chromatography on silica gel (DCM:MeOH = 1:0 ~ 10:1) to give the compound **35d** (1.27 g) as a yellow solid. ESI-MS: m/z 363.1 $[M+H]^+$.

Step 4: preparation of Compound **35e**

Compound **35d** was co-evaporated with pyridine (20 mL) twice before use.

To a solution of compound **35d** (1.27 g) in pyridine (15 mL) was added DMAP (215 mg, 1.76 mmol) and DMTrCl (1.781 g, 5.257 mmol) at 0 °C. After stirring the reaction at RT overnight, the mixture was diluted with $CH_2Cl_2$ (80 mL), then washed successively with aqueous saturated NaHCO3 (50 mL × 3). The organic layer was collected, dried with $Na_2SO_4$, filtered and concentrated under reduced pressure to give a residue. The residue was purified by flash column chromatography on silica gel (0-85% EtOAc in Petroleum ether) to give compound **35e** (1.61 g) as a yellow solid. $^1H$ NMR (DMSO-$d_6$, 400 MHz) $\delta$ 12.05 (s, 1H), 11.59 (s, 1H), 7.96 (s, 1H), 7.36 (d, J = 7.2 Hz, 2H), 7.22-7.29 (m, 2H), 7.12-7.22 (m, 5H), 6.76 (d, J =8.8 Hz, 2H), 6.69 (d, J = 8.8 Hz, 2H), 5.55 (d, J = 2.4 Hz, 1H), 4.72 (br d, J = 6.4 Hz, 1H), 4.42-4.51 (m, 1H), 3.64 (d, J = 12.4 Hz, 6H), 3.47-3.54 (m, 1H), 3.37 (br d, J = 6.4 Hz, 1H), 2.79 (quin, J = 6.8 Hz, 1H), 2.03-2.14 (m, 1H), 1.85-1.94 (m, 1H), 1.13 (t, J = 6.8 Hz, 6H); ESI-MS: m/z 665.2 $[M+H]^+$.

Step 5: preparation of compound **35f**

To a solution of compound **35e** (1.61 g, 2.42 mmol) in THF (16 mL) was added $Ph_3P$ (889 mg, 3.39 mmol) in one portion, then the mixture was stirred at 40°C for 2 hr under $N_2$. water (8 mL) was added to the mixture, then it was further stirred at 40°C overnight. The mixture was combined with another crude batch and diluted with $CH_2Cl_2$ (100 mL), water (80 mL), and extracted with $CH_2Cl_2$ (100 mL × 2). Organic layers were combined, dried with $Na_2SO_4$, filtered and concentrated under reduced pressure to give a residue. The residue was purified by flash column chromatography on silica gel (0~8% MeOH in DCM) to give compound **35f** (1.5 g, 2.34 mmol) as a white solid. $^1H$ NMR (DMSO-$d_6$, 400 MHz) $\delta$7.92 (s, 1H), 7.36 (d, J = 7.2 Hz, 2H), 7.13-7.28 (m, 7H), 6.76 (d, J = 8.8 Hz, 2H), 6.70 (d, J = 9.2 Hz, 2H), 5.59 (d, J =1.6 Hz, 1H), 4.56-4.87 (m, 5H), 4.26 (dq, J = 10.0, 5.2 Hz, 1H), 3.65 (d, J = 11.6 Hz, 6H), 2.78 (spt, J = 6.8 Hz, 1H), 2.58-2.72 (m, 2H), 1.99-2.11 (m,1H), 1.73 (br dd, J = 13.2, 5.6 Hz, 1H), 1.13 (t, J = 6.8 Hz, 6H); ESI-MS: m/z 639.2 $[M+H]^+$.

Step 6: preparation of compound **35g**

A solution of 4-nitrophenyl chlorosulfate **3n** (1.67 g, 7.04 mmol) in dry $CH_2Cl_2$ (3 mL) was added rapidly to a mixture of compound **35f** (1.5 g, 2.35 mmol), 4-nitrophenol (980.1 mg, 7.04 mmol), $Et_3N$ (1.96 mL, 14.14 mmol) in dry $CH_2Cl_2$ (27 mL) under $N_2$ at -78 °C, then warmed to RT naturally over 2 hr. The mixture was combined with another crude batch, diluted with $CH_2Cl_2$ (100 mL) and washed with aqueous saturated NaHCO3 (100 mLx5). The organic layer was collected, dried with anhydrous $Na_2SO_4$, filtered and concentrated under reduced pressure to give a residue. The residue was purified by flash column chromatography on silica gel (0~90% EtOAc in petroleum ether) to give the compound 35g (1.6 g) as a yellow solid. $^1H$ NMR (DMSO-$d_6$, 400 MHz) $\delta$ 12.05 (s, 1H), 11.55 (s, 1H), 8.91 (t, J = 5.6 Hz, 1H), 8.20-8.27 (m, 2H), 7.93 (s, 1H), 7.43-7.51 (m, 2H), 7.34 (d, J = 7.2 Hz, 2H), 7.21-7.28 (m, 2H), 7.11-7.21 (m, 5H), 7.11-7.21 (m, 1H), 6.75 (d, J = 8.8 Hz, 2H), 6.68 (d, J = 8.8 Hz, 2H), 5.55 (d, J = 2.0 Hz, 1H), 4.67 (br d, J = 6.4 Hz, 1H), 4.34-4.44 (m, 1H), 3.64 (d, J = 12.4 Hz, 6H), 3.30-3.37 (m, 1H), 3.18-3.27 (m, 1H), 2.78 (spt, J = 6.8 Hz, 1H), 1.99-2.10 (m, 1H), 1.89 (br dd, J = 12.4, 5.6 Hz, 1H), 1.10-1.15 (m, 6H); ESI-MS: m/z 840.3 $[M+H]^+$.

Step 7: preparation of compound **35i**

A suspension of compound **35h** (910 mg, 1.347 mmol), compound **35g** (1.6 g, 1.905 mmol) and Molecular seives (3 g) in THF (40 mL) was stirred under $N_2$ for 30 min at RT, followed by addition of DMAP (659 mg, 5.39 mmol), then stirred at 45 °C overnight under $N_2$. The reaction mixture was filtered through a pad of diatomaceous earth and the filtrate was concentrated under reduced pressure to give a yellow residue, which was dissolved in DCM (60 mL), then washed with aqueous saturated NaHCO3 (40 mLx3). The organic layer was collected, dried with anhydrous $Na_2SO_4$, filtered and concentrated under reduced pressure to give a yellow residue. The residue was purified by flash column chromatography on silica gel (Petroleum ether:EtOAc = 1:0~1:4) to give compound **35i** (1.13 g) as a white solid, which was confirmed by LCMS. ESI-MS: m/z 1377.3 $[M+H]^+$.

Step 8: preparation of compound **35j**

To a stirred solution of compound **35i** (1.13 g, 0.77 mmol) in DCM (30 mL) was added water (140 mg, 7.77 mmol) and DCA (220 mg, 1.7 mmol). The yellow mixture was stirred at RT overnight It was then added MeOH (5 mL), followed by addition of pyridine (244.5 mg, 4 eq) resulting in yellow solution, which was further stirred for 15 min; the solution was worked up with another crude batch and concentrated under reduced pressure to give a residue. The residue was purified by flash column chromatography on silica gel (DCM:MeOH = 1:0~10:1) to give compound **35j** (630

mg) as a white solid. $^1H$ NMR (DMSO-$d_6$, 400 MHz) $\delta$ 12.11 (s, 1H), 11.64 (s, 1H), 11.28 (s, 1H), 8.77 (s, 1H), 8.70 (s, 1H), 8.62 (br t, J = 6.0 Hz, 1H), 8.14 (s, 1H), 8.04 (d, J = 7.2 Hz, 2H), 7.62-7.69 (m, 1H), 7.52-7.59 (m, 2H), 6.48 (dd, J = 16.8, 2.8 Hz, 1H), 5.76-5.96 (m, 2H), 5.72 (d, J = 4.0 Hz, 1H), 5.30-5.43 (m, 2H), 4.60 (br s, 1H), 4.36-4.45 (m, 1H), 4.28-4.34 (m, 1H), 3.74-3.84 (m, 1H), 3.58-3.68 (m, 1H), 3.23-3.35 (m, 2H), 2.71-2.83 (m, 1H), 2.17-2.28 (m, 1H), 2.00-2.09 (m, 1H), 1.11 (dd, J = 6.8, 1.6 Hz, 6H); $^{19}F$ NMR (DMSO-$d_6$, 376MHz) $\delta$-202.81 (dt, J=52.1, 16.5 Hz, IF); ESI-MS: m/z 772.3 $[M+H]^+$.

Step 9: preparation of compound **35k**

THF was freshly distilled over Na/ benzophenone and $CH_3CN$ was freshly distilled over $CaH_2$ before use. To a solution of compound **35j** (120 mg, 0.15 mmol) in THF (3 mL), was added 800 mg of 4A MS (powder) and a solution of 1H-tetrazole (3.45 mL, 0.45 M, prepared by dissolving 945 mg of tetrazole (dried by lyophilization) in 30 mL of dry $CH_3CN$, followed by addition of 1 g of 4A MS and then stirred for 1hr under $N_2$ before use); the mixture was purged with $N_2$. A solution of 2-cyanoefhyl-N,N,N',N'-tetraisopropylphosphorodiamidite (84.36 mg, 0.28 mmol) in THF (0.8 mL) was added drop-wise over 25 min vial a syringe, then stirred at room temperature for 1.5 hr. A solution of TBHP (0.25 mL, 1.24 mmol, 5M) was added and stirred for another 30 min. The reaction mixture was concentrated under reduced pressure to give a residue. The residue was purified by flash column chromatography on silica gel (DCM:MeOH = 1:0~10:1) to give **39k** a white solid. (68 mg). ESI-MS: m/z 887.4 $[M+H]^+$.

Step 10: preparation of **compound 26, ammonium salt**

A solution of compound **35k** (68 mg, 0.077 mmol) in $MeNH_2$/EtOH (5 mL) was stirred at rt. for 2 hr. After stirring the reaction mixture at 40°C for 1h, the volatile was evaporated and the obtained white solid was dissolved in a mixture of WATER (20 mL), then extracted with DCM (10 mL × 4). The aqueous layer was lyophilized and purified by reverse phase preparative HPLC (Column:Xbridge 150×30mm×10μm, Condition: A: water (10mM $NH_4HCO_3$)-ACN: MeCN, beginning: B 0%, End B:30% ; Flow Rate 25 mL/min.) to give **compound 26, ammonium salt** as a white solid (18.8 mg).

$^1H$ NMR (DMSO-$d_6$, 400 MHz) $\delta$ ppm 10.78 (br s, 1H), 8.84 (br d, *J*= 6.8 Hz, 1H), 8.45 (s, 1H), 8.25 (s, 1H), 7.86 (s, 1H), 6.67 (br s, 1H), 6.44 (br d, J= 18.0 Hz,1H), 5.91 (s, 1H), 5.62 (br s, 1H), 5.49 (br d, J= 3.2 Hz, 1H), 5.30 - 5.16 (m, 2H), 4.72 (br s, 1H), 4.44 (br d, *J*= 9.4 Hz, 1H), 4.48 - 4.40 (m, 1H), 4.29 (br d, *J*= 12.4 Hz, 1H), 4.21 (br t, *J* = 10.8 Hz, 1H), 3.89 (br d, *J*= 12.0 Hz, 1H), 3.45 - 3.33 (m, 1H), 2.92 (br d, *J* = 11.2 Hz, 1H), 1.94 (br t, *J* = 10.8 Hz, 1H); $^{19}F$ NMR(376MHz, DMSO-$d_6$) $\delta$ ppm -198.95 (br s, IF); $^{31}P$ NMR (162 MHz, DMSO-$d_6$) $\delta$ ppm -4.86 (br s, IP); ESI-MS: m/z 660.3 $[M+H]^+$.

**Conversion to sodium salt**

Dowex 50W × 8, 200-400 (5 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 ×). The resin was transferred to the column and washed with 15% NaOH in water (at least 4 CV), and then with deionized water until it was neutral. **Compound 26 ammonium salt** (10 mg) were dissolved in minimum amount of deionized water and $CH_3CN$ (1:1, v/v, 3 mL), added to the top of the column, and eluted with deionized water. Appropriate fractions were pooled together and lyophilized to **compound 26, sodium salt** (6.1 mg) as a white solid $^1H$ NMR (400 MHz, $D_2O$) $\delta$ ppm 8.22 (br s, 1H), 8.11 (br s, 1H), 7.75 (s, 1H), 6.40 (br d, J= 16.8 Hz, 1H), 5.88 (br s, 1H), 5.73 (br s, 1H), 5.60 (br s, 1H), 5.24 -5.04 (m, 1H), 4.59 (br d, J= 8.8 Hz, 1H), 4.49 (br s, 1H), 4.42 (br d, *J* = 12.0 Hz, 1H), 4.13 (br d, *J* = 11.8 Hz, 1H), 3.69 (br d, *J* = 13.6 Hz, 1H), 3.41 (br d, J= 8.0 Hz, 1H), 2.76 (br s, 1H), 2.26 - 2.19 (m, 1H), 2.22 (br d, J= 6.8 Hz, 1H); $^{19}F$ NMR (376 MHz, $D_2O$) $\delta$ ppm -200.45 (br s, IF); $^{31}P$ NMR (162MHz, $D_2O$) $\delta$ ppm -2.84 (s, IP); ESI-MS: m/z 660.0 $[M+H]^+$.

Example 36

**Compound 37**

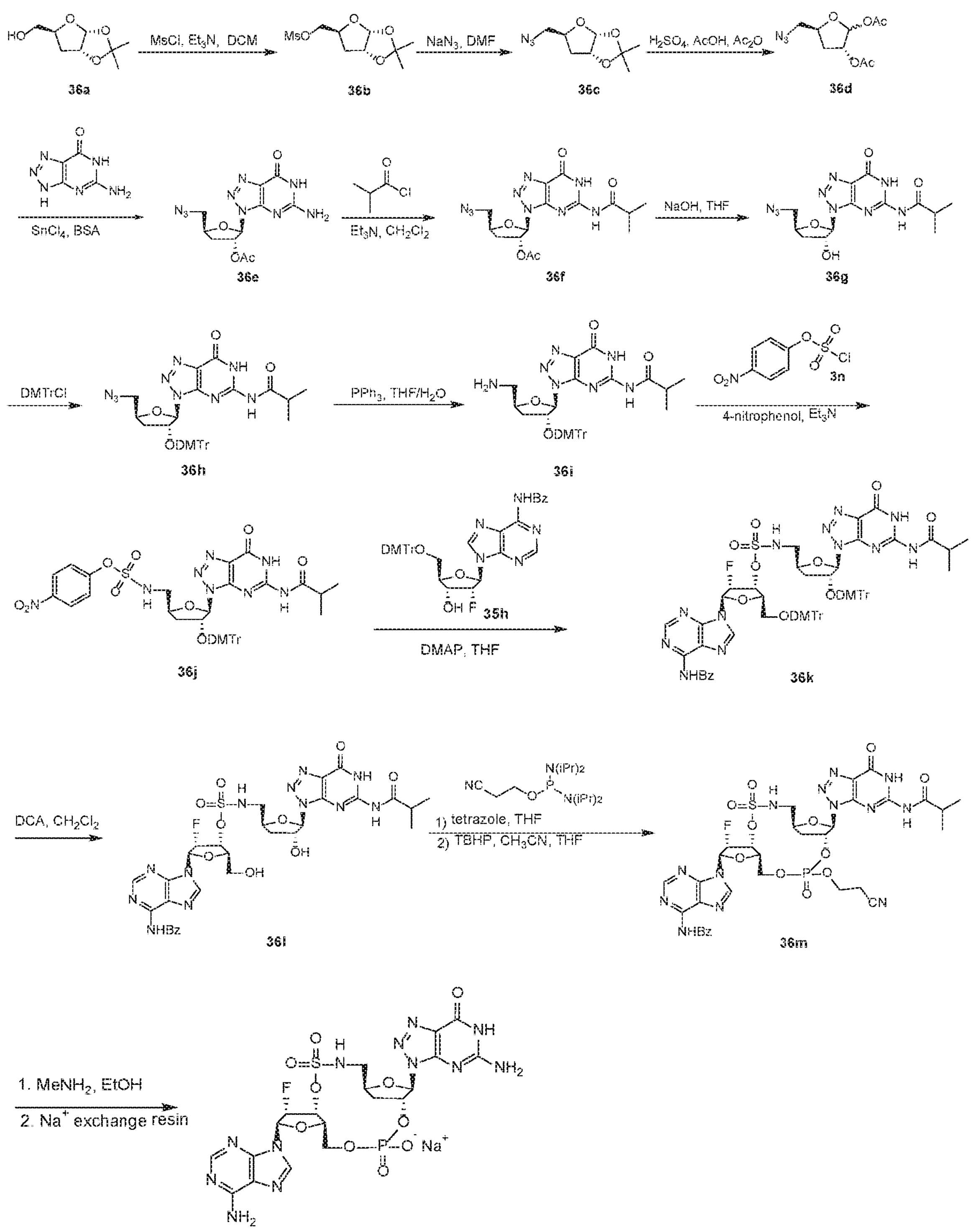


**Compound 37, sodium salt**

Step 1: preparation of compound **36b**

To a solution of compound **36a** (4.2 g, 24.11 mmol) in DCM (40 mL) was added TEA (4.88 g, 48.22 mmol) and MsCl (4.97 g, 43.4 mmol) dropwise for 10 mins. After stirring at 25 °C for 2 hr., the mixture was partitioned between

DCM (100 mL) and water (50 mL). The organic layer was washed with brine (3 × 50 mL), dried over anhydrous $Na_2SO_4$, filtered and the solvent evaporated under reduced pressure to give a residue. The residue (combined with silica gel: 10 g) was purified by flash column chromatography on silica gel (PE/EA from 10% to 100% and DCM/MeOH = 0% to 5 %) to give compound **36b** (5.6 g) as a light yellow solid. $^1H$ NMR (400 MHz, $CDCl_3$) δ ppm 5.84 (d, J=2.4 Hz, 1H), 4.78 (t, J=3.4 Hz, 1H), 4.51 -4.40 (m, 2H), 4.29-4.22 (m, 1H), 3.09-3.05 (m, 3H), 2.18-2.09 (m, 1H), 1.84-1.73 (m, 1H), 1.52 (s, 3H), 1.33 (s, 3H).

Step 2: preparation of compound **36c**

To a solution of compound **36b** (5.6 g, 22.19 mmol) in DMF (55 mL) was added $NaN_3$ (4.25 g, 65.37 mmol) at 25 °C. After stirringat 100 °C for 3 hrs., the mixture was diluted with aqueous saturated NaHCO3 (100 mL) and extracted with ethyl acetate (3 × 100 mL). The organic layer was washed with brine (100 mL), dried over anhydrous $Na_2SO_4$, filtered and evaporated under reduced pressure to give a yellow solid. The solid (combined with silica gel: 10 g) was purified by flash column chromatography on silica gel (silica gel: 20 g) (gradient elution: 0 -100% ethyl acetate in petroleum then 0-20% methanol in ethyl acetate) to give compound **36c** (3.8 g) as a yellow solid. $^1H$ NMR (400 MHz, $CDCl_3$) δ ppm 5.85 (d, J=3.4 Hz, 1H), 4.77 (t, J=4.2 Hz, 1H), 4.45 - 4.35 (m, 1H), 3.59 (dd, J=3.7, 13.2 Hz, 1H), 3.28 (dd, J=4.6, 13.2 Hz, 1H), 2.09 (dd, J=4.4, 13.4 Hz, 1H), 1.79 (ddd, J=4.8, 10.7, 13.4 Hz, 1H), 1.52 (s, 3H), 1.33 (s, 3H).

Step 3: preparation of compound **36d**

Acetic anhydride (18.03 mL, 190.75 mmol) and concentrated sulfuric acid (0.104 mL, 1.91 mmol) were added to a stirred solution of compound **36c** (3.8 g, 19.08 mmol) in acetic acid (109.1 mL, 1907.56 mmol) at 0 °C. After stirring for 2 h at 25 °C, the reaction mixture was quenched with cold water (100 mL) and stirred for 30 min at 25 °C. The mixture was extracted with ethyl acetate (3 × 200 mL). Organic layers were combined and successively washed with saturated aqueous sodium bicarbonate solution (3 × 300 mL), brine (2 × 200 mL), dried over anhydrous $Na_2SO_4$, filtered and concentrated under reduced pressure to give a residue. The residue (combined with silica gel: 10 g) was purified by flash column chromatography on silica gel (0-100% PE in EA) to give compound **36d** (3.2 g) as a yellow oil. $^1H$ NMR (400 MHz, $CDCl_3$) δ ppm 6.21 - 6.14 (m, 1H), 5.22 (d, J=5.0 Hz, 1H), 4.58- 4.50 (m, 1H), 3.57 (dd, J=4.0, 13.1Hz, 1H), 3.25 (dd, J=4.6, 13.2 Hz, 1H), 2.29-2.19 (m, 1H), 2.12-2.10 (m, 1H), 2.09 (s, 6H).

Step 4: preparation of compound **36e**

A solution of 5-amino-3H-[1,2,3]triazolo[4,5-d]pyrimidin-7(6H)-one (2.37 g, 15.6 mmol) and BSA (11.041 g, 54.273 mmol) in anhydrous $CH_3CN$ (200 mL) was stirred at 80 °C for 1 h and then cooled to 0 °C. It was then added a solution of compound **36d** (3.3 g, 13.57 mmol) in anhydrous $CH_3CN$ (80 mL) followed by SnCl4 (10.6 g, 40.7 mmol). After stirring for at 26 °C for 48 hours, the mixture was cooled and diluted with EtOAc (100 mL), then added into aqueous saturated NaHCO3 (500 mL) dropwise at 0 °C. Organic layer was washed with brine (2 × 100 mL), dried over anhydrous $Na_2SO_4$, filtered and concentrated under pressure to give a residue. The residue (combined with silica gel: 10 g) was purified by flash column chromatography on silica gel (2%-10% MeOH in DCM, V/V) to give compound **36e** (6.4 g) as a yellow foam. $^1H$ NMR (400 MHz, DMSO-$d_6$) δ ppm 11.08 (br, s, 1H), 7.28 (br, s, 1H), 6.68 (br, s, 1H), 6.10 (s, 1H), 5.68 (d, J=5.5 Hz, 1H), 4.61-4.50 (m, 1H), 3.58 (dd, J=3.1, 13.4 Hz, 1H), 3.34-3.29 (m, 1H), 2.71-2.59 (m, 1H), 2.33-2.24 (m, 1H), 2.09 (s, 3H); ESI-MS: m/z=336.1 $[M+H]^+$.

Step 5: preparation of compound **36f**

To a solution of compound **36e** (4.8 g, 14.32 mmol) in DCM (48 mL) was added triethylamine (4.34 g, 42.95 mmol) and DMAP (504.37 mg, 4.13 mmol) at 25 °C. After stirring for 5 min, the mixture was cooled down at 0 °C and isobutyryl chloride (3.05 g, 28.63 mmol) was added to the solution in 10 min. After stirring at 25 °C for 2 hours, the mixture was diluted with EtOAc (100 mL), washed with brine (3 × 100 mL), dried over anhydrous $Na_2SO_4$, filtered and concentrated under pressure to give compound **36f** (4.8 g) as a yellow solid which was directly used into next step without any further purification.

Step 6: preparation of compound **36g**

To a solution of compound **36f** (6.5 g) in THF/MeOH/water (240/150/45 mL) was added NaOH (0.5 M in water, 64.14 mL) at 0 °C. Then the solution was stirred at 0 °C for 1 hour. The solution was acidified with acetic acid (~2 mL) to pH 7. The solution was concentrated under pressure to give a yellow solid (6 g). The residue (combined with silica gel: 10 g) was purified by flash column chromatography on silica gel (silica gel: 40 g) (DCM/MeOH=1/0 to 5/1) to give

compound **36g** (4.5 g) as a light yellow solid.

$^1$H NMR (400 MHz, $CD_3OD$) δ ppm 6.20 (s, 1H), 4.94 (d, J=5.0 Hz, 1H), 4.73 - 4.65 (m, 1H), 3.52-3.46 (m, 1H), 3.38-3.33 (m, 1H), 2.78-2.61 (m, 2H), 2.20 (ddd, J=1.3, 6.0, 13.3 Hz, 1H), 1.23 (d, J=7.0 Hz, 6H); ESI-MS: m/z=364.2 $[M+H]^+$

Step 7: preparation of compound **36h**

A solution of compound **36g** (5 g, 13.76 mmol) in Py (50 mL) was treated with DMAP (841 mg, 6.88 mmol) at 0 °C for 10 min. After 10 min, DMTrCl (9.3 g, 27.52 mmol) was added and then the solution was stirred at 80 °C for 12 hours. The reaction mixture was diluted with EA (50 mL) and washed with brine (3 × 80 mL). The organic layer was dried over anhydrous $Na_2SO_4$, filtered and concentrated under pressure to give a yellow solid (10 g). The yellow solid (combined with silica gel: 15 g) was purified by flash column chromatography on silica gel (0-100% PE in EA) to give compound **36h** (8.0 g) as a light yellow solid. $^1$H NMR (400 MHz, $CDCl_3$) δ ppm12.01 (br, s, 1H), 8.35 (br, s, 1H), 7.45-7.42 (m, 2H), 7.28-7.24 (m, 7H), 6.73-6.66 (m, 4H), 5.10 (d, J=5.8 Hz, 1H), 4.84-4.74 (m, 1H), 3.83 -3.77 (m, 1H), 3.71 (d, J=6.3 Hz, 6H), 3.43-3.35 (m, 1H), 3.29-3.23 (m, 1H), 2.68- 2.56 (m, 2H), 2.40 (dd, J=5.9, 13.2 Hz, 1H), 1.31 (dd, J=6.9, 11.7 Hz, 6H). ESI-MS: m/z=688.1 $[M+Na]^+$.

Step 8: preparation of compound **36i**

A solution of compound **36h** (8.0 g, 12.02 mmol) in EA/EtOH (1/1, 350 mL) was treated with Pd/C (4.5 g); after stirring the mixture under $H_2$ atmosphere (15 psi) for 2 hr, thsolution was filtered and concentrated under reduced pressure to give a residue. The residue (combined with silica gel: 10 g) was purified by flash column chromatography on silica gel (silica gel: 40 g) (DCM/MeOH=1/0 to 5/1) to give compound **36i** (6.0 g) as a light yellow solid. ESI-MS: m/z=640.4 $[M+H]^+$

Step 9: preparation of compound **36j**

A solution of compound **36i** (6.0 g, 8.44 mmol) in DCM (130 mL) was treated with 4-nitrophenol (3.52 g, 25.32 mmol) and triethylamine (5.12 g, 50.65 mmol). After cooling down to -78 °C, a solution of 4-nitrophenyl chlorosulfate (6.02 g, 25.32 mmol) in DCM (20 mL) was added at -78 °C. After stirring at -78 °C for 1 h, the mixture was filtered and the filtrate diluted with DCM (300 mL). Organic layer was washed with aqueous saturated $NaHCO_3$ (3 × 150 mL) and concentrated under reduced pressure to give a residue (10 g). The residue was purified by flash column chromatography on silica gel (gradient elution: 0 - 100% ethyl acetate in petroleum) to give compound **36j** (5.9 g) as a yellow solid. $^1$H NMR (400 MHz, $CD_3CN$) δ ppm 12.07 (br, s, 1H), 9.39 (br, s, 1H), 8.22-8.17 (m, 2H), 7.40-7.36 (m, 4H), 7.24-7.14 (m, 7H), 6.76-6.66 (m, 4H), 5.76 (d, *J*=38 Hz, 1H), 4.97-4.90 (m, 1H), 4.68-4.60 (m, 1H), 3.68 (d, *J*=8.3 Hz, 6H), 3.46-3.39 (m, 1H), 3.32 -3.25 (m, 1H), 2.65 (td, *J*=7.0, 13.7 Hz, 1H), 2.40-2.32 (m, 1H), 2.22-2.19 (m, 1H), 1.22 (d, *J*=7.0 Hz, 3H), 1.21-1.18 (m, 3H).

ESI-MS: m/z=863.2 $[M+Na]^+$

Step 10: preparation of compound **36k**

A solution of compound **36j** (1.18 g, 1.40 mmol), compound **35h** (0.73 g, 1.08 mmol) and 4A MS (1 g) in DCE ( 21 mL) was stirred under $N_2$ for 30 min at RT, followed by addition of DMAP (660.41 mg, 5.41 mmol). After stirring the reaction at 45 °C (oil temperature) for 12 hr., the mixture was filtered and the filtrate partitioned between DCM (100 mL) and brine (100 mL). Organic layer was successively washed with aqueous saturated NaHCO3 (3 × 100 mL), dried over anhydrous $Na_2SO_4$, filtered and the evaporated under reduced pressure to give a residue (4 g). The residue (combined with silica gel: 6 g) was purified by flash column chromatography on silica gel (PE/ EA from 10% to 100% and DCM/MeOH=0% to 5 %) to give compound **36k** (1.2 g) as a light yellow solid.

$^1$H NMR (400 MHz, $CD_3CN$) δ 11.88 (br, s, 1H), 9.42 (br, s, 1H), 9.09 (br, s, 1H), 8.38 (s, 1H), 8.05 (s, 1H), 7.98-7.85 (m, 1H), 7.76 (br, d, *J*=7.6 Hz, 1H), 7.47 -7.39 (m, 1H), 7.35-7.27 (m, 2H), 7.12 (br, d, *J*=7.3 Hz, 2H), 7.07 (br, d, *J*=6.4 Hz, 2H), 7.02 - 6.86 (m, 11H), 6.72 (br, d, *J*=8.6 Hz, 1H), 6.59 - 6.40 (m, 8H), 6.13 - 6.03 (m, 1H), 5.83 (br, s, 0.5H), 5.73-5.62 (m, 1H), 5.46 (br, s, 1H), 4.67 (br, s, 1H), 4.33 (br, s, 1H), 3.99 (br, s, 1H), 3.67-3.38 (m, 11H), 3.25 (br, d, *J*=11.2 Hz, 1H), 3.10 (br, d, *J*=14.4 Hz, 1H), 3.00 (br, d, *J*=8.1 Hz, 1H), 2.89 (br, d, *J*=6.1 Hz, 1H), 2.51-2.41 (m, 1H), 2.14 -2.05 (m, 1H), 1.75-1.72 (m, 6H); $^{19}$F NMR (376 MHz, $CD_3CN$) δ ppm -200.072 (s, IF); ESI-MS: m/z=1377.8 $[M+H]^+$

Step 11: preparation of compound **36l**

A solution of compound **36k** (1.2 g, 0.78 mmol) in DCM (30 mL) was treated with water (141.25 mg, 7.84 mmol) and DCA (202.19 mg, 1.57 mmol) resulting in a red solution. After stirring at 25 °C for 12 hrs., MeOH (5 mL) was added to the mixture until turned clear followed by addition of pyridine (620.19 mg, 7.84 mmol). The mixture was stirred at RT for 2 hrs, then concentrated under pressure to give a residue. The residue (combined with silica gel: 2 g) was purified by flash column chromatography on silica gel (0-8% DCM in MeOH) to give compound **36l** (0.54 g) as a white solid. $^{1}H$ NMR (400 MHz, DMSO-$d_6$) δ 12.26 (s, 1H), 11.92 (s, 1H), 11.27 (s, 1H), 8.77 (s, 1H), 8.69 (s, 1H), 8.53 (br, t, *J*=5.8 Hz, 1H), 8.05 (br, d, *J*=7.5 Hz, 2H), 7.69-7.63 (m, 1H), 7.60-7.53 (m, 2H), 6.46 (dd, *J*=2.6, 16.7 Hz, 1H), 6.04 (s, 1H), 5.92 (br, s, 0.5H), 5.86 (br, d, *J*=3.8 Hz, 1H), 5.79 (br, d, *J*=3.3 Hz, 0.5H), 5.37-5.26 (m, 2H), 4.75 (br, s, 1H), 4.60-4.49 (m, 1H), 4.27 (br, s, 1H), 4.10 (br, s, 1H), 3.77 (br, d, *J*=12.5 Hz, 1H), 3.60 (br, dd, *J*=4.8, 8.0 Hz, 1H), 2.79 (td, *J*=6.7, 13.7 Hz, 1H), 2.19 (br, dd, *J*=6.3, 12.5 Hz, 1H), 1.13 (d, *J*=6.8 Hz, 6H). $^{19}F$ NMR (376MHz, DMSO-d6) δ ppm -202.787 (s, 1F); ESI-MS: m/z= 773.3 $[M+H]^+$

Step 12: preparation of compound **36m**

Note: THF was freshly distilled over Na/benzophenone and $CH_3CN$ was freshly distilled over $CaH_2$ before use.

A solution of compound **36l** (100 mg, 0.13 mmol) in THF (2 mL) was treated with 4A MS (powder) (1g); after stirring for 20 min, a solution of 1H-tetrazole (0.45 M) in $CH_3CN$ (2.3 mL) was added at 25 °C. It was then added a solution of 2-cyanoethyl-*N,N,N',N'*-tetra(isopropyl)phosphorodiamidite (0.078 g, 0.26 mmol, diluted in 2 mL of THF) at 25 °C and the mixture stirred for 1.5 h, followed by addition of tert-butyl hydroperoxide (0.12 mL, 0.65 mmol) at 25 °C. After stirring for 1.5 hr, the mixture was diluted with DCM (20 mL), filtered through a pad of diatomaceous earth and concentrated to afford the crude product as a yellow solid (1.0 g). The crude (combined with silica gel: 2 g) was purified by flash column chromatography on silica gel (0-15% MeOH in $CH_2Cl_2$) to give compound 36m (50 mg) as a light yellow solid. ESI-MS: m/z=888.3 $[M+H]^+$

Step 13: preparation of **compound 37, sodium salt**

A solution of compound **36m** (50 mg, 0.056 mmol) in EtOH (2.5 mL) was treated with methylamine (2.5 mL, 33% in EtOH). After stirring at 25 °C for 3 hrs., the solution was concentrated under reduced pressure to give a residue. The residue was purified by reverse phase preparative HPLC (Column: Waters Xbridge Prep OBD C18 5μm 150x30, Condition: water (10mM $NH_4HCO_3$)-ACN B: 0, End B 30, Gradient Time (min): 7, 100%B Hold Time (min): 1, FlowRate (mL/min): 25) to give **compound 37 ammonium salt** (12.2 mg) as a white solid. $^{1}H$ NMR(400 MHz, $D_2O$) δ 8.58 (s, 1H), 8.45 (s, 1H), 6.66 (d, J=18.1 Hz, 1H), 6.46 (s, 1H), 5.94-5.75 (m, 1H), 5.51-5.41 (m, 1H), 5.37 (br, s, 1H), 4.63 (br, d, J=12.5 Hz, 1H), 4.35 (br, d, J=13.4 Hz, 1H), 3.92 (br, dd, J=3.7, 14.4 Hz, 1H), 3.50 -3.36 (m, 1H), 3.16 (br, d, J=12.0 Hz, 1H), 2.65-2.55 (m, 1H); $^{19}F$ NMR (376MHz, $D_2O$) δ ppm -199.359 (s, IF) ; $^{31}P$ NMR (162MHz, $D_2O$) δ ppm -3.307 (s, IP); ESI-MS: m/z=661.3 $[M+H]^+$

**Conversion to sodium salt**

Dowex 50W × 8, 200-400 (10 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 x). The resin was transferred to the column and washed with 15% NaOH in water (at least 4 CV), and then with deionized $H2_2O$ until it was neutral. **Compound 37, ammonium salt** (28 mg) was dissolved in deionized water/MeCN (20 mL/5 mL), added to the top of the column, and eluted with deionized water. Appropriate fractions were pooled together and lyophilized to **compound 37, sodium salt** (24.5 mg) as a white solid.

$^{1}H$ NMR (400 MHz, $D_2O$) = 8.63 (br, s, 1H), 8.48 (br, s, 1H), 6.71 (br, d, J=17.6 Hz, 1H), 6.51 (br, s, 1H), 5.97 - 5.79 (m, 1H), 5.54 - 5.40 (m, 2H), 4.69 (br, d, J=13.3 Hz, 1H), 4.41 (br, d, J=13.1 Hz, 1H), 3.97 (br, d, J=13.8 Hz, 1H), 3.54-3.41 (m, 1H), 3.22 (br, d, J=12.5 Hz, 1H), 2.66 (br, s, 1H); $^{19}F$ NMR (376MHz, $D_2O$) ppm -199.443 (s, IF); $^{31}P$ NMR (162MHz, $D_2O$) ppm -3.283 (s, IP); ESI-MS: m/z 661.2 $[M+H]^+$

Example 37

## Compound 47

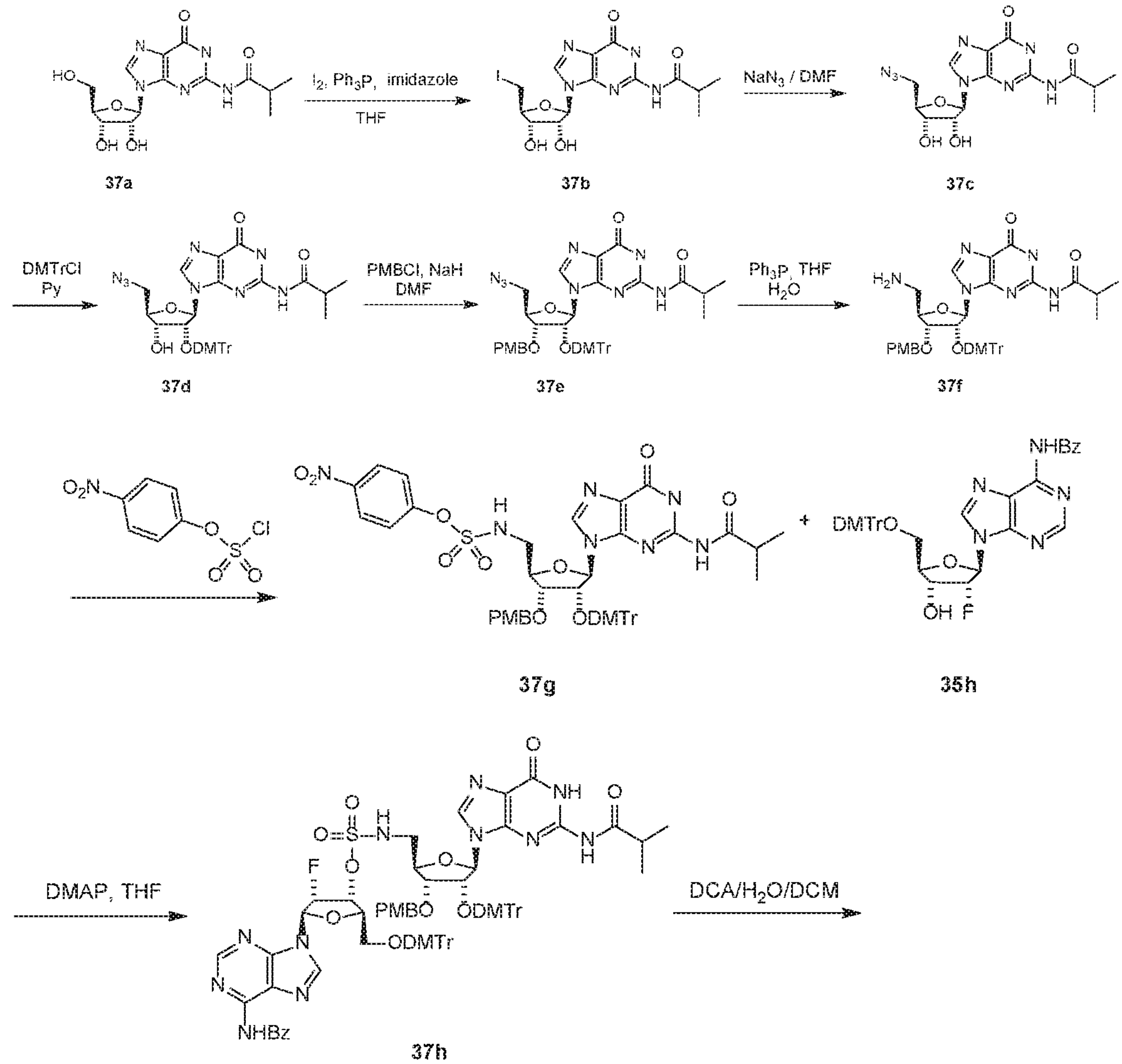


37a 37b 37c

37d 37e 37f

37g 35h

37h

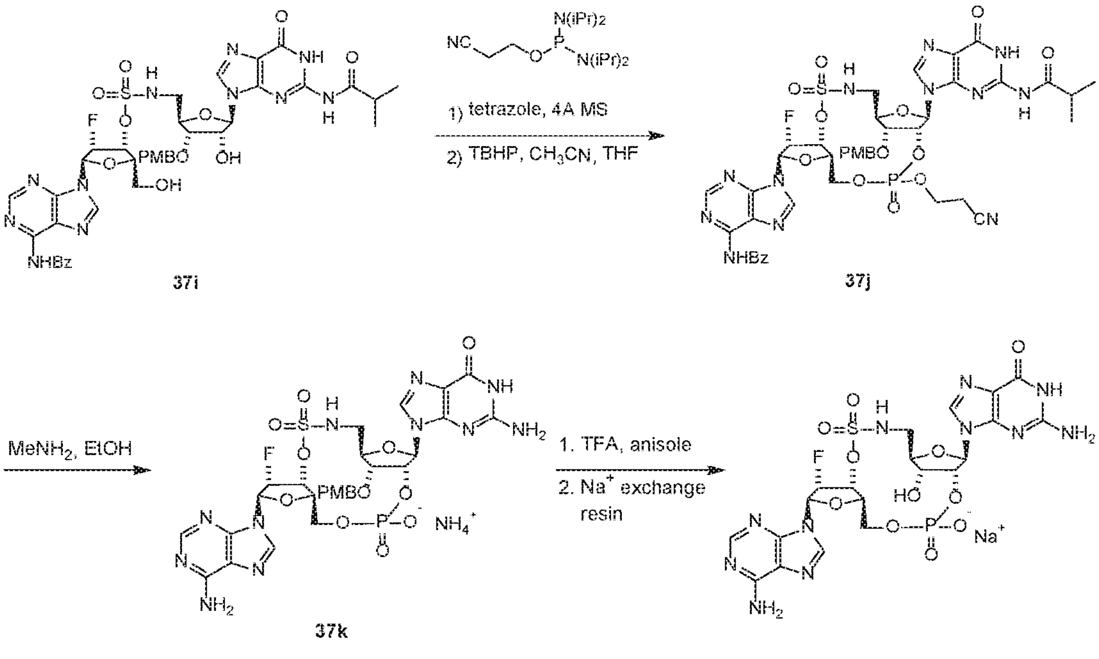


37i

37j

37k

**Compound 47, sodium salt**

Step 1: preparation of compound **37b**

To a stirred solution of compound **37a** (10 g, 28.3 mmol), triphenylphosphine (22.27 g, 84.91 mmol), imidazole (7.71 g, 113.25 mmol) in THF (100 mL), was added a solution of iodine (21.55 g, 84.91 mmol) in THF (100 mL) at 0 °C. After stirring at 35°C overnight, the reaction mixture was filtered, concentrated under reduced pressure and diluted with DCM (400 mL). Organic layer was washed with aqueous saturated $Na_2SO_3$ (200 mL × 2), dried with anhydrous $Na_2SO_4$, filtered and concentrated under reduced pressure to give a residue. The residue was purified by flash column chromatography on silica gel (DCM: MeOH = 1:0~10:1) to give compound **37b** (6 g) as a white solid. $^1H$ NMR (DMSO-$d_6$, 400 MHz) $\delta$ 12.10 (s, 1H), 11.65 (s, 1H), 8.26 (s, 1H), 5.83 (d, J = 6.0 Hz, 1H), 5.66 (br d, J = 5.6 Hz, 1H), 5.45 (br d, J = 4.4 Hz,1H), 4.68 (q, J = 5.6 Hz, 1H), 4.11 (br d, J = 3.2 Hz, 1H), 3.92-4.02 (m, 1H), 3.58 (br dd, J = 10.4, 6.0 Hz, 1H), 3.43 (br dd, J = 10.4, 6.8 Hz, 1H), 2.76 (dt, J = 13.6, 6.8 Hz, 1H), 1.12 (br d, J = 6.8 Hz, 6H); ESI-MS: m/z 463.9 $[M+H]^+$.

Step 2: preparation of compound **37c**

A solution of compound **37b** (6 g, 12.95 mmol) in DMF (100 mL) was treated with $NaN_3$ (2.47 g, 37.99 mmol) under $N_2$. After stirring at 80 °C for 3 hr., the mixture was diluted with DCM (400 mL ) and washed with brine (300 mL × 2). The organic layer was dried with anhydrous $Na_2SO_4$, filtered and concentrated under reduced pressure to give a residue. The residue was purified by flash column chromatography on silica gel (DCM : MeOH = 1:0~10:1) to give the compound **37c** (4 g) as a white solid. $^1H$ NMR (DMSO-$d_6$, 400 MHz) $\delta$ 12.10 (s, 1H), 11.64 (s, 1H), 8.26 (s, 1H), 5.84 (d, J = 6.0 Hz, 1H), 5.65 (d, J = 6.0 Hz, 1H), 5.38 (d, J = 4.8 Hz, 1H),4.60 (q, J = 5.6 Hz, 1H), 4.09-4.11 (m, 1H), 4.02 (dt, J = 7.2, 3.6 Hz, 1H), 3.62-3.72 (m, 1H), 3.52-3.60 (m, 1H), 2.77 (sept, J = 6.8 Hz, 1H), 1.12 (d, J = 6.8 Hz, 6H); ESI-MS: m/z= 379.0 $[M+H]^+$

Step 3: preparation of compound **37d**

Compound **37c** was co-evaporated with pyridine (80 mL) twice before use. To a solution of compound **37c** (4 g, 10.57 mmol) in pyridine (40 mL) was added DMAP (646 mg, 5.29 mmol) and DMTrCl (5.38 g, 15.88 mmol) at 0 °C. After stirring at RT overnight, the reaction mixture was diluted with $CH_2Cl_2$ (200 mL). The organic layer was successively washed with aqueous saturated NaHCO3 (150 mL × 3), dried with anhydrous $Na_2SO_4$, filtered and concentrated under reduced pressure to give a residue. The residue was purified by flash column chromatography on silica gel (0~90% EtOAc in Petroleum ether) to give the crude product as a yellow. The crude product was purified by reverse phase preparative HPLC (Phenomenex Synergi Max-RP 10 μm 250 × 50mm; mobile phase: Water-ACN from 30 % to 70 %; flow rate: 100 mL/min) to give the compound **37d** (4.15 g) as a white solid. $^1H$ NMR (DMSO-$d_6$, 400 MHz) $\delta$ 12.02 (s, 1H), 11.43 (br s, 1H), 7.97 (s, 1H), 7.42 (d, J = 6.8 Hz, 2H), 7.28 (d, J = 8.8 Hz, 2H), 7.11-7.22 (m, 5H), 6.71 (d, J = 9.2

Hz, 2H), 6.63 (d, J = 9.2 Hz, 2H), 5.66 (d, J = 5.2 Hz, 1H), 5.48 (d, J = 5.6 Hz, 1H), 4.80 (br t, J = 4.8 Hz, 1H), 4.05 (dt, J = 7.2, 4.0 Hz, 1H), 3.65 (d, J = 11.6 Hz, 6H), 3.56 (br dd, J = 13.2, 7.2 Hz, 1H), 3.44-3.51 (m, 1H), 3.39 (dd, J = 13.2, 4.0 Hz, 1H), 2.79 (quin, J = 6.8 Hz, 1H), 1.14 (dd, J = 6.8, 2.3 Hz, 6H); ESI-MS: m/z= 681.4 $[M+H]^+$

Step 4: preparation of compound **37e**

NaH (60% in mineral oil, 189.5 mg, 4.74 mmol) was added to a suspension of compound **37d** (2.15 g, 3.16 mmol) in DMF (20 mL) at 0 °C. After stirring at 0 °C for 0.5 hr, a solution of 4-methoxybenzyl chloride (0.512 mL, 3.8 mmol) in DMF (10 mL) was added dropwise. After addition was complete, the reaction mixture was stirred at 0 °C for 1 hr and quenched with water (80 mL) and extracted with EtOAc (100 mL × 2). The organic layer was dried with anhydrous $Na_2SO_4$, filtered and evaporated under reduced pressure to give a residue. The residue was combined with another batch and purified by reverse phase preparative HPLC (YMC Triart C18, 7μm 250x50mm; mobile phase: water (10mM $NH_4HCO_3$)-ACN from 55 % to 90 %; flow rate: 90 mL/min) to give the compound **37e** (1.5 g, 1.83 mmol) as a white solid and compound **37d** (2.16 g, 3.09 mmol) as a yellow solid. $^1H$ NMR (DMSO-$d_6$, 400 MHz) δ 12.04 (s, 1H), 11.42 (br s, 1H), 8.11 (s, 1H), 7.32 (d, J = 8.4 Hz, 2H), 7.22-7.28 (m, 2H), 7.14-7.20 (m, 5H), 7.05 (d, J =9.2 Hz, 2H), 6.96 (d, J = 8.8 Hz, 2H), 6.74 (d, J = 8.8 Hz, 2H), 6.66 (d, J = 8.8 Hz, 2H), 6.02 (d, J = 7.6 Hz, 1H), 4.87 (br s, 1H), 4.34 (d, J = 10.4 Hz,1H), 4.22 (dd, J = 7.2, 5.2 Hz, 1H), 4.00 (br d, J = 10.4 Hz, 1H), 3.78 (s, 3H), 3.69 (d, J = 9.6 Hz, 7H), 3.27 (dd, J = 12.8, 4.8 Hz, 1H), 2.76 (spt, J = 6.8 Hz, 1H), 2.61 (br d, J = 3.6 Hz, 1H), 1.09-1.17 (m, 6H); ESI-MS: m/z= 801.4 $[M+H]^+$.

Step 5: preparation of compound **37f**

$Ph_3P$ (673 mg, 2.566 mmol) was added to a solution of compound **37e** (1.5 g, 1.83 mmol) in THF (15 mL) in one portion. The mixture was stirred at 40°C for 2 hr under $N_2$ followed by addition of water (7.5 mL). After stirring at 40°C ON, the mixture was diluted with DCM (50 mL), water (40 mL) and extracted with DCM (50 mL × 2). Organic layers were then combined, was dried with anhydrous $Na_2SO_4$, filtered and evaporated under reduced pressure to give a residue. The residue was purified by flash column chromatography on silica gel (0~8% MeOH in DCM) to give compound **37f** (1.27 g) as a white solid

$^1H$ NMR (DMSO-$d_6$, 400 MHz) δ 8.06 (s, 1H), 7.31 (d, J = 8.4 Hz, 2H), 7.22-7.27 (m, 2H), 7.13-7.22 (m, 5H), 7.04 (d, J = 8.8 Hz, 2H), 6.96 (d, J = 8.4 Hz, 2H), 6.74 (d, J = 9.2 Hz, 2H), 6.66 (d, J = 8.8 Hz, 2H), 5.99 (d, J = 7.6 Hz, 1H), 4.78 (br s, 1H), 4.30 (d, J = 10.4 Hz, 1H), 4.04 (t, J = 5.6 Hz, 1H), 3.96 (d, J = 10.4 Hz, 1H), 3.75-3.80 (m, 3H), 3.69 (d, J = 8.4 Hz, 6H), 2.73-2.85 (m, 1H), 2.66 (br d, J = 4.4 Hz, 1H), 2.62 (br d, J = 6.0 Hz, 2H), 1.12 (t, J = 6.4 Hz, 6H); ESI-MS: m/z= 775.3 $[M+H]^+$

Step 6: preparation of compound **37g**

A solution of 4-nitrophenyl chlorosulfate (1.17 g, 4.92 mmol) in dry DCM (3 mL) was added to a mixture of compound **37f** (1.27 g, 1.64 mmol), 4-nitrophenol (685 mg, 4.92 mmol), $Et_3N$ (1.37 mL, 9.88 mmol) in dry DCM (27 mL) under $N_2$ at -78 °C, then warmed to room temperature naturally over 2 hr. The reaction mixture was worked up with another batch, diluted with DCM (100 mL) and washed with aqueous saturated NaHCO3 (100 mL × 5). The organic layer was collected, dried with anhydrous $Na_2SO_4$, filtered and evaporated under reduced pressure to give a residue. The residue was purified by flash column chromatography on silica gel (0~90% EtOAc in PE) to give the compound **37g** (1.55 g, 1.59 mmol) as a yellow solid. $^1H$ NMR (DMSO-$d_6$, 400 MHz) 12.05 (s, 1H), 11.49 (br s, 1H), 8.88 (t, J = 6.0 Hz, 1H), 8.26-8.31 (m, 2H), 8.13 (s, 1H), 7.47-7.53 (m, 2H), 7.29 (d, J =8.8 Hz, 2H), 7.20-7.25 (m, 2H), 7.14-7.20 (m, 5H), 7.03 (d, J = 8.8 Hz, 2H), 6.95 (d, J = 8.8 Hz, 2H), 6.74 (d, J = 8.8 Hz, 2H), 6.67 (d, J = 8.8 Hz, 2H), 5.95 (d, J = 7.2 Hz, 1H), 4.72 (br s, 1H), 4.25 (d, J = 10.4 Hz, 1H), 4.18 (br t, J = 5.6 Hz, 1H), 3.96 (br d, J = 10.4 Hz, 1H), 3.77 (s, 3H), 3.69 (d, J = 6.8 Hz, 6H), 3.24-3.44 (m, 2H), 2.91 (br d, J = 3.6 Hz, 1H), 2.69-2.79 (m, 1H), 1.12 (t, J = 6.4 Hz, 6H); ESI-MS: m/z= 976.3 $[M+H]^+$

Step 7: preparation of compound **37h**

A suspension of compound **37g** (766 mg, 1.13 mmol), compound **35h** (1.55 g, 1.59 mmol) and MS (3 g) in THF (40 mL) was stirred under $N_2$ for 30 min at RT, followed by addition of DMAP (554 mg, 4.53 mmol), then stirred at 45 °C ON under $N_2$. The reaction mixture was filtered through a pad of diatomaceous earth and the filtrate was concentrated under reduced pressure to give a yellow residue; the residue was dissolved in 60 mL of DCM and washed with aqueous saturated NaHCO3 (40 mL × 3). The organic layer was dried with anhydrous $Na_2SO_4$, filtered and evaporated under reduced pressure to give a residue. The residue was purified by flash column chromatography on silica gel (PE : EtOAc = 1:0~0:1) to give compound **37h** (360 mg) as a yellow solid. ESI-MS: m/z= 757.4 $[M/2 +H]^+$

Step 8: preparation of compound **37i**

A solution of compound **37h** (360 mg, 0.24 mmol) in DCM (10 mL) was treated with water (43 mg, 2.39 mmol) and DCA (67.5 mg, 0.52 mmol) resulting in a yellow solution. After stirring at RT for 6 hr, MeOH (2 mL) was added to the mixture, followed by addition of pyridine (75.5 mg, 4 eq); the resulting solution was stirred for 15 min and concentrated to give a residue. The residue was purified by flash column chromatography on silica gel (DCM: MeOH = 1:0-10:1) to give the compound **37i** (130 mg) as a white solid. ESI-MS: m/z= 908.4 $[M+H]^+$

Step 9: preparation of compound **37j**

NOTE: THF was freshly distilled over Na/benzophenone and $CH_3CN$ was freshly distilled over $CaH_2$ before use. To a solution of compound **37i** (130 mg, 0.14 mmol, dried by lyophilization) in THF (3 mL) was added 4A MS (powder, 800mg) followed by a solution of 1H-tetrazole (3.18 mL, 0.45 M, prepared by dissolving 945 mg of tetrazole (dried by lyophilization) in 30 mL of dry $CH_3CN$, followed by addition of 1 g of 4A MS and then stirred for 1hr under $N_2$ before use); after purging several times the vessel with $N_2$, a solution of 2-cyanoethyl-*N,N,N',N'*-tetra(isopropyl)phosphorodiamidite (77.69 mg, 0.26 mmol) in THF (0.8 mL) was added drop-wise over 25 min vial a syringe, then stirred at room temperature for 1.5 hr, followed by addition of tert-butyl hydroperoxide (0.23 mL, 1.13 mmol, 5M). After stirring for another 30 min, the reaction mixture was concentrated under reduced pressure to give a residue. The residue was purified by flash column chromatography on silica gel (DCM: MeOH =1:0 ~ 10:1, $R_f$= 0.4) to give compound **37j** (46 mg) as a white solid. ESI-MS: m/z= 1023.5 and 1023.4 $[M+H]^+$

Step 10: preparation of compound **37k**

A solution of compound **37j** (46 mg, 0.045 mmol) was treated with $MeNH_2$/EOH (5 mL) at 40°C for 2.5 hr. The reaction mixture was concentrated under reduced pressure to give a residue. The residue was purified by reverse phase preparative HPLC (Column: Waters Xbridge Prep OBD C18 5μm 150x30, Condition: A: water (10mM $NH_4HCO_3$)-ACN: MeCN, beginning: B 5%, End B: 25%; Flow Rate (mL/min) 25.) to give compound **37k** (12 mg) as a white solid.
ESI-MS: m/z= 796.3 $[M+H]^+$

Step 11: preparation of **compound 48, sodium salt**

A solution of anisole (15.9 mg, 0.14 mmol) in TFA (0.113 mL, 57.3 mmol) was cooled down to 0 °C and added to compound **37k** (12 mg, 0.015 mmol). After stirring the at 0 °C for 2.5 hr, the TFA was removed by blowing flow of nitrogen gas at 0°C. The remaining reaction mixture was quenched with methylamine, 33% solution in EtOH (0.113 mL) at 0°C. The reaction mixture was evaporated to dryness and partitioned between DCM and WATER (20 mL × 3/10 mL). The aqueous layer was lyophilized to give a white residue purified by reverse preparative HPLC (Column: Waters Xbridge Prep OBD 5μm C18 150x30, Condition: A: water (10mM $NH_4HCO_3$)-ACN: MeCN, beginning: B 5%, End B: 35%; Flow Rate (mL/min) 25.) to give **compound 47, ammonium salt** (7.1 mg, 0.010 mmol) as white solid. $^1H$ NMR (400 MHz, $D_2O$) δ 8.59 (s, 1H), 8.47 (s, 1H), 8.16 (s, 1H), 6.76 (d, *J* = 16.8 Hz, 1H), 6.28 (d, *J* = 8.4 Hz, 1H), 6.07 (br d, *J* = 3.6 Hz, 1H), 5.94 (br d, *J* = 4.4 Hz, 1H), 5.63 (br dd, *J* = 3.2, 8.8 Hz, 1H), 5.58 (br dd, *J* = 4.4, 8.8 Hz, 1H), 5.49 (br d, *J* = 5.6 Hz, 1H), 4.93 (br d, *J* = 8.8 Hz, 1H), 4.67 (br s, 1H), 4.39(br d, *J* = 15.6 Hz, 1H), 4.13 - 4.07 (m, 1H), 3.96 (br d, *J* = 14.4 Hz, 1H); $^{31}P$ NMR (162 MHz, $D_2O$) δ ppm -2.21 (s, IP); $^{19}F$ NMR (376 MHz, $D_2O$) δ 200.3-200.4 (m, IF); ESI-MS: m/z= 676.0 $[M+H]^+$.

**Conversion to sodium salt**

Dowex 50W × 8, 200-400 (3 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 x). The resin was transferred to the column and washed with 15% NaOH in water (at least 4 CV), and then with deionized water until it was neutral. **Compound 47, ammonium salt** (7.1 mg) was dissolved in deionized water/MeCN (3 mL/3 mL), added to the top of the column, and eluted with deionized water Appropriate fractions were pooled together and lyophilized to **compound 47, sodium salt** (3.4 mg) as a white solid.
$^1H$ NMR (400 MHz, $D_2O$) δ ppm 8.57 (s, 1H), 8.44 (s, 1H), 8.13 (s, 1H), 6.73 (d, *J* = 16.6 Hz, 1H), 6.25 (d, *J* = 8.8 Hz, 1H), 6.04 (br d, *J* = 3.6 Hz, 1H), 5.91 (br d, *J* = 4.0 Hz, 1H), 5.60 (br dd, J= 3.6, 9.2 Hz, 1H), 5.55 (br dd, J = 4.0, 9.2 Hz, 1H), 5.49 - 5.42 (m, 1H), 4.90 (br d, *J* = 9.2 Hz, 1H), 4.67 - 4.64 (m, 1H), 4.39 -4.33 (m, 1H), 4.08 (br d, J= 14.4

Hz, 1H), 3.93 (br d, *J* = 14.8 Hz, 1H); $^{31}P$ NMR (162 MHz, $D_2O$) δ ppm -2.26 (s, IP); $^{19}F$ NMR (376MHz, $D_2O$) δ ppm -200.34-200.47 (m,1F); ESI-MS: m/z= 676.1 $[M+H]^+$

Example 38

**Compound (*R) 46A and compound (*S) 46B**

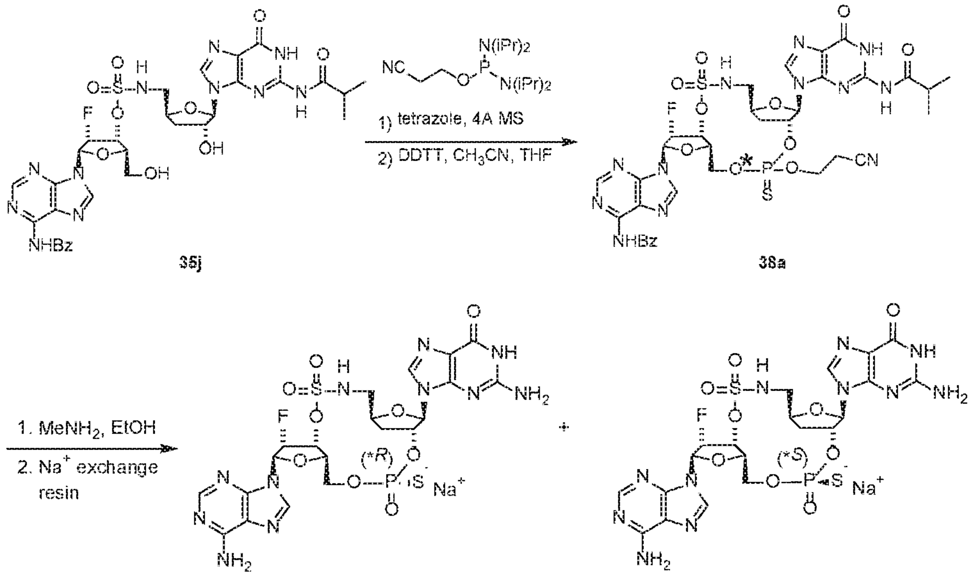


35j

38a

**Compound (*R) 46A, sodium salt** **Compound 46B, sodium salt**

Step 1: preparation of compound **38a**

NOTE: THF was freshly distilled over Na/benzophenone and $CH_3CN$ was freshly distilled over $CaH_2$ before use. To a solution of compound **35j** (300 mg, 0.39 mmol, dried by lyophilization) in THF (6 mL) was added 4A MS (powder, 800 mg) and a solution of 1H-tetrazole (8.64 mL, 0.45 M, prepared by dissolved 945 mg of tetrazole (dried by lyophilization) in 30 mL of dry $CH_3CN$, followed by addition of 1 g of 4A MS and then stirred for 1hr under $N_2$ before use); after purging several times the reaction flask with $N_2$. a solution of 2-cyanoethyl-*N,N,N',N'*-tetra(isopropyl)phosphorodiamidite (210.9 mg,0.7 mmol) in THF (2 mL) was added dropwise over 20 min vial a syringe, then stirred at room temperature for 1.5 hr. It was then added DDTT (638.53 mg, 3.11 mmol) and the mixture stirred for another 30 min. The reaction mixture was concentrated under reduced pressure to give a residue. The residue was purified by flash column chromatography on silica gel (DCM: MeOH = 1: 0-10:1) to give compound **38a** as a white solid. (372 mg). ESI-MS: m/z= 903.2 and 903.4 $[M+H]^+$

Step 2: preparation of **compound (*R) 46A, sodium salt** and **compound 46B (*S), sodium salt**

Compound **38a** (372 mg) was treated with $MeNH_2$/EtOH (15 mL) at 40 °C for 2.5 hr. After stirring at 40°C for 1h, the reaction mixture was concentrated to give a white solid combined with another batch. The solid was dissolved in water (40 mL) and washed with DCM (20 mL × 4), The aqueous layer was lyophilized and purified by reverse phase preparative HPLC (Xtimate C18 5μm 150x25mm, Condition: A: water(10mM $NH_4HCO_3$)-ACN: MeCN, beginning B: 12%, End B:42% ; Flow Rate(mL/min) 25.) to give white solid **compound (*R) 46A, ammonium salt** as a white solid (18.5 mg) and **compound (*S), 46B ammonium salt** as a white solid (35.2 mg).

**Compound (*R) 46A, ammonium salt:** $^1H$ NMR (400 MHz, DMSO-$d_6$) δ ppm 8.69 (s, 1H), 8.30 (s, 1H), 7.89 (s, 1H), 6.46 (d, J= 16.0 Hz, 1H), 5.95 (s, 1H), 5.48 (br d, *J* = 2.8 Hz, 1H), 5.35 (br d, *J* = 2.8Hz, 1H), 5.10 (br dd, *J* = 3.2, 9.4 Hz, 1H), 5.04 (br dd, *J* = 3.2, 9.2 Hz, 1H), 4.67 (br s, 1H), 4.47 (br d, *J* = 9.2 Hz, 1H), 4.36 (br *d, J* = 12.8 Hz, 1H), 4.19 (brt, *J* = 10.8 Hz, 1H), 3.85 (brd, *J* = 13.6 Hz, 1H), 3.62 (br d, J= 3.6 Hz, 1H), 3.02 (br d, J= 10.4 Hz, 1H), 1.89 (br t, J= 10.8 Hz, 1H); $^{31}P$ NMR (162MHz, DMSO-$d_6$) δ ppm 50.5 (br s, IP); $^{19}F$ NMR (376MHz, DMSO-$d_6$) δ ppm -199.86

(td, *J* = 19.6, 49.6 Hz, IF)

**Compound (****S***) 46B, ammonium salt:** $^1$H NMR (400 MHz, DMSO-$d_6$) $\delta$ ppm 8.48 (s, 1H), 8.30 (s, 1H), 7.88 (s, 1H), 6.45 (d, *J* = 17.2 Hz, 1H), 5.90 (s, 1H), 5.58 (br d, J = 3.2 Hz, 1H), 5.45 (br d, *J* = 3.2Hz, 1H), 5.17 (br d, J= 6.4 Hz, 1H), 5.12 (br d, J= 9.2 Hz, 1H), 4.75 (br s, 1 H), 4.54 - 4.45 (m, 2H), 4.25 - 4.16 (m, 1H), 3.78 (br d, *J*= 13.6 Hz, 1H), 3.38(br dd, *J* = 11.2, 13.6 Hz, 1H), 3.01 (br d, *J* = 10.4 Hz, 1H), 1.93 (br t, J= 10.8 Hz, 1H); $^{31}$P NMR (162MHz, DMSO-$d_6$) $\delta$ ppm 52.02 (br s, 1P); $^{19}$F NMR (376MHz, DMSO-$d_6$) $\delta$ ppm -199.30-199.90 (m, IF).

**Conversion to compound (****R***) 46A, sodium salt**

Dowex 50W $\times$ 8, 200-400 (4 mL, H form) was added to a beaker and washed with deionized water (30 mL). Then to the resin was added 15% $H_2SO_4$ in deionized water, the mixture was gently stirred for 5 min, and decanted (15 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 $\times$). The resin was transferred to the column and washed with 15% NaOH in water (at least 4 CV), and then with deionized water until it was neutral. **Compound (****R***) 46A, ammonium salt** (18.5 mg) was dissolved in deionized water/MeCN (8 mL/2 mL), added to the top of the column, and eluted with deionized water. Appropriate fractions were pooled together and lyophilized to **compound (****R***) 46A,** sodium salt (13.7 mg) as a white solid. $^1$H NMR (400 MHz, $D_2O$) $\delta$ ppm 8.27 (br s, 1H), 7.97 (br s, 1H), 7.72 (br s, 1H), 6.35 (br d, *J* = 16.4 Hz, 1H), 5.77 (br s, 1H), 5.70 - 5.49 (m, 1H), 5.26 (br s, 2H),4.59 - 4.44 (m, 2H), 4.36 (br d, *J* = 11.2 Hz, 1H), 4.05 (br s, 1H), 3.60 - 3.38 (m, 2H), 2.71 (br s, 1H), 2.28 (br s, 1H); $^{31}$P NMR (162MHz, $D_2O$) $\delta$ 54.42 (br s, IP); $^{19}$F NMR (376MHz, $D_2O$) $\delta$ -200.41 (br s, 1F); ESI-MS: m/z= 676.0 $[M+H]^+$

**Conversion to compound (****S***) 46B, sodium salt**

Dowex 50W $\times$ 8, 200-400 (10 mL, H form) was added to a beaker and washed with deionized water (30 mL). Then to the resin was added 15% $H_2SO_4$ in deionized water, the mixture was gently stirred for 5 min, and decanted (25 mL). The resin was transferred to a column with 15% $H_2SO_4$ in deionized waterand washed with 15% $H_2SO_4$ (at least 4 CV), and then with deionized wateruntil it was neutral. The resin was transferred back into the beaker, 15% NaOH in deionized watersolution was added, and mixture was gently stirred for 5 min, and decanted (1 $\times$). The resin was transferred to the column and washed with 15% NaOH in water (at least 4 CV), and then with deionized water until it was neutral. **Compound (****S***) 46B, ammonium salt** (35.2 mg) was dissolved in deionized water/MeCN (10 mL/8 mL), added to the top of the column, and eluted with deionized water. Appropriate fractions were pooled together and lyophilized to **compound (****S***) 46B, sodium salt** (33.1 mg) as a white solid. $^1$H NMR (400 MHz, $D_2O$) $\delta$ ppm 8.72 (br s, 1H), 8.73 - 8.68 (m, 1H), 8.58 (br s, 1H), 8.14 (br s, 1H), 6.81 (br d, *J* = 17.6 Hz, 1H), 6.26 (br s, 1H), 6.07 - 5.89 (m, 1H), 5.67 - 5.53 (m, 2H), 4.94 (br d, *J* = 6.8 Hz, 2H), 4.82 (br s, 1H), 4.42 (br d, *J* = 12.4 Hz, 1H), 4.09 (br d, *J* = 14.4 Hz, 1H), 4.14 - 4.04 (m, 1H), 3.82 -3.69 (m, 1H), 3.23 (br s, 1H), 2.58 (br s, 1H), 2.37 (br s, 1H); $^{31}$P NMR (162 MHz, $D_2O$) $\delta$ ppm 53.58 (br s, IP); $^{19}$F NMR (376MHz, $D_2O$) $\delta$ ppm -199.69 (br s, IF); ESI-MS: m/z= 676.0 $[M+H]^+$

Example 39

## Compound 35

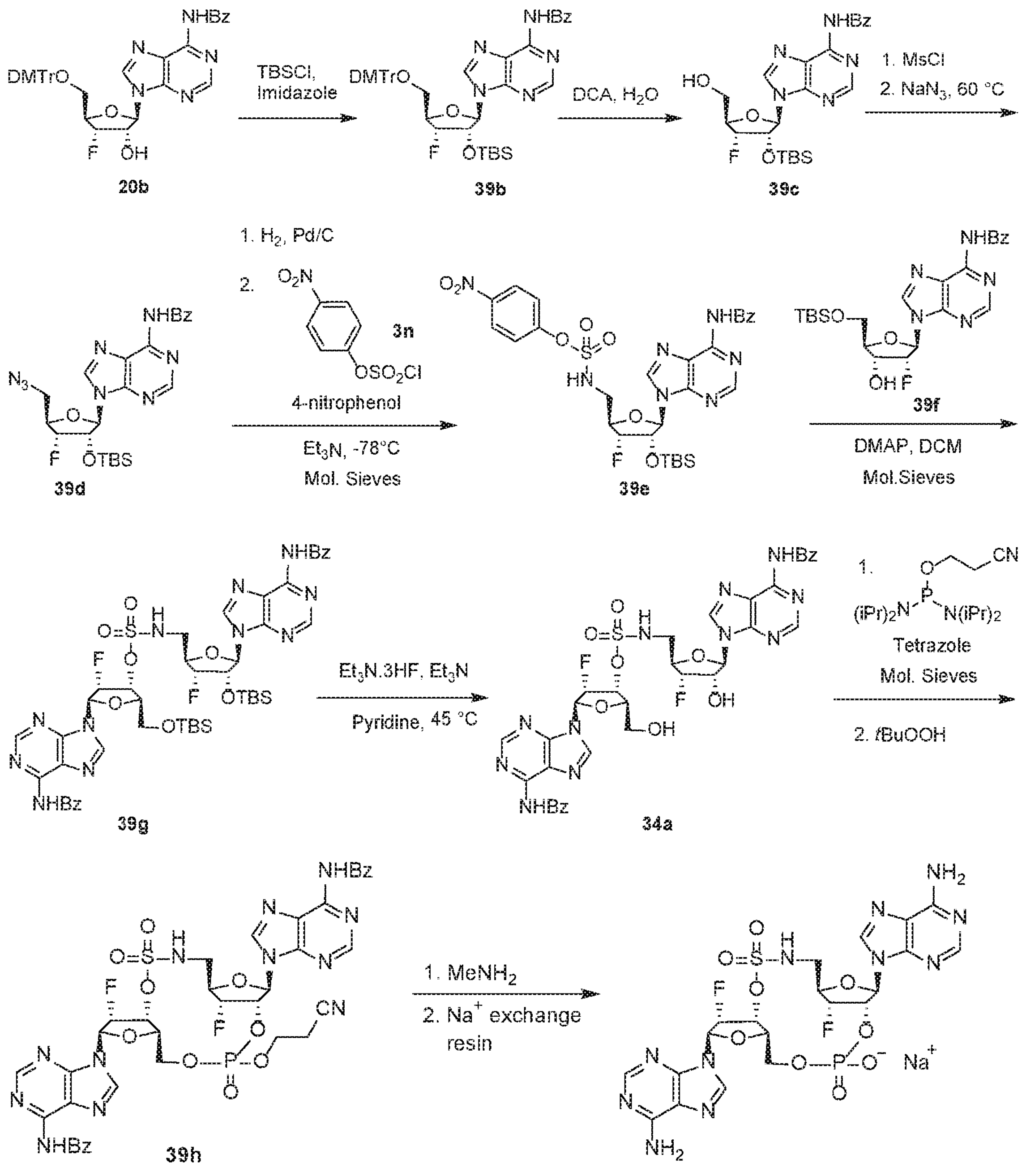


**Compound 35, sodium salt**

Step 1: Preparation of compound **39b**

Imidazole (2.36 g, 34.6 mmol) and TBSCl (3.48 g, 23.1 mmol) were successively added to a solution of compound **20b** [CAS 170871-87-1 ] (7.8 g, 11.5 mmol) in dry DCM (101 mL). The reaction mixture was stirred at room temperature until complete conversion (ca. 3 h) after which the reaction solution was diluted with DCM and washed with water. The organic layer was dried over anhydrous $Na_2SO_4$, filtered and concentrated under reduced pressure to give crude compound **39b** as a white foam (9.2 g, crude).

$^1H$ NMR (500 MHz DNISO-$d_6$) $\delta$ ppm: 0.20 (s, 3 H), 0.24 (s, 3 H), 0.96 (s, 9 H), 3.60 (d, *J*=5.5 Hz, 2 H), 3.97 (d, *J*=2.1 Hz, 6 H), 4.69 (m, 1 H), 5.4 (dd, *J*=54.4, 4.1 Hz, 1 H), 5.63 (dq, *J*=21.3, 3.9 Hz, 1 H), 6.35 (d, *J*=7.6 Hz, 1 H),

7.10 (m, 4 H), 7.47 (q, *J*=7.3 Hz, 1 H), 7.52 (m, 6 H), 7.66 (d, *J*=7.6 Hz, 2 H), 7.80 (t, *J*=7.6 Hz, 2 H), 7.89 (t, *J*=7.6 Hz, 1 H), 8.29 (d, *J*=6.9 Hz, 2 H), 8.83 (s, 1H) 8.89 (s, 1 H), 11.49 (s, 1 H); ESI-MS: *m/z* 790.4 $[M+H]^+$.

Step 2: Preparation of compound **39c**

A solution of crude compound **39b** (9.2 g) in DCM (250 mL), to which water (1 mL, 57.6 mmol) and DCA (38 mL of a 10% solution in DCM, 46.1 mmol) were added, was stirred at room temperature until complete conversion (ca.1 h). The reaction mixture was quenched with pyridine (4.6mL, 57.6 mmol) and methanol (5 mL), and subsequently concentrated under reduced pressure. The residue was purified by column chromatography over silica gel (gradient elution: 0-5% MeOH in DCM) to give compound **39c** as a white foam (4.4 g). $^1$H NMR (500 MHz DMSO-$d_6$) $\delta$ ppm: -0.26 (s, 3 H), -0.06 (s, 3 H), 0.72 (s, 9 H), 3.72 (m, 2 H), 4.36 (dt, J=26.6, 4.0 Hz, 1 H), 5.10 (m, 2 H), 5.22 (d, J=4.1 Hz, 1 H), 5.39 (s, 1 H), 6.13 (d, J=7.6 Hz, 1 H), 7.55 (t, J=7.6 Hz, 2 H), 7.65 (t, J=7.6 Hz, 1 H), 8.05 (d, J=6.9 Hz, 2 H), 8.77 (d, J=9.6 Hz, 2 H), 11.24 (s, 1 H); ESI-MS: *m/z* 488.2$[M+H]^+$.

Step 3: Preparation of compound **39d**

Mesyl chloride (1.04 mL, 13.55 mmol) was added dropwise to a solution of compound **39c** (4.4 g, 9.03 mmol) in dry pyridine (44 mL) at 0° C. The reaction mixture was stirred at 0° C until complete conversion (ca. 3 h) after which it was quenched with methanol and concentrated under reduced pressure. The obtained residue was dissolved in EtOAc and washed with saturated aqueous $NaHCO_3$. The organic layer was dried over anhydrous $Na_2SO_4$, filtered and concentrated under reduced pressure to give the mesylated product. The crude procuct was dissolved in dry DMF (50 mL) followed by the addition of sodium azide (4.51 g, 69.38 mmol). The reaction mixture was stirred at 60° C for 5 h after which it was cooled to room temperature, diluted with EtOAc, and washed with saturated aqueous $NaHCO_3$ and water. The organic layer was dried over anhydrous $Na_2SO_4$, filtered and concentrated under reduced pressure. Purification was done by column chromatography over silica gel (gradient elution: 0- 2% MeOH in DCM) to give compound **39d** as a white foam (4 g, yield: 86.5%). $^1$H NMR (500 MHz DMSO-$d_6$) $\delta$ ppm: -0.25 (s, 3 H), -0.02 (s, 3 H), 0.72 (s, 9 H), 3.65 (dd, *J*=13.1, 4.1 Hz, 1 H), 3.92 (q, *J*=6.9 Hz, 1 H), 4.50 (dq, *J*=25.3, 3.7 Hz, 1 H), 5.20 (dd, *J*=53.7, 4.8 Hz, 1 H), 5.35 (dq, *J*=22.0, 3.9 Hz, 1 H), 6.15 (d, *J*=7.6 Hz, 1 H), 7.56 (t, *J*=7.9 Hz, 2 H), 7.65 (t, *J*=7.2 Hz, 1 H), 8.05 (d, *J*=6.9 Hz, 2 H), 8.80 (d, *J*=2.4 Hz, 2 H), 11.24 (s, 1 H); ESI-MS: *m/z* 513.2 $[M+H]^+$.

Step 4: Preparation of compound **39e**

A solution of compound **39d** (4 g, 7.8 mmol) in MeOH (40 mL) was hydrogenated under atmospheric pressure at room temperature on Pd/C (20% on carbon, 0.4 g). The reaction mixture was filtered over Diatomaceous earth, the Diatomaceous earth was rinsed with MeOH. The filtrate was evaporated under reduced pressure to give the compound amine as a white foam. The crude product (dried by co-evaporation with anhydrous toluene) was dissolved in DCM (59 mL) followed by the addition of 4-nitrophenol (3.2 g, 24 mmol), $Et_3N$ (6.5 mL, 46.8 mmol) and activated molecular sieves. The resulting mixture was cooled to -78°C under $N_2$ after which a solution of 4-nitrophenyl chlorosulfate (5.5 g, 23.4 mmol) in DCM (20 mL) was added dropwise, stirring was continued until complete conversion (ca. 3 h). The reaction mixture was warmed to room temperature and washed with saturated aqueous $NaHCO_3$ and water. The organic phase was dried with anhydrous $Na_2SO_4$, filtered and concentrated under reduced pressure. The crude product was purified by silica column chromatography (gradient elution: 0 - 50% EtOAc in hexane) to give compound **39e** as a yellow foam (3.5 g, yield: 65 %). $^1$H NMR (500 MHz DMSO-$d_6$) $\delta$ ppm: -0.28 (d, *J*=15.8 Hz, 3 H), -0.05 (d, *J*=6.2 Hz, 3 H), 0.71 (s, 9 H), 3.66 (t, *J*=5.9 Hz, 2 H), 4.42 (d, *J*=26.2 Hz, 1 H), 5.29 (m, 2 H), 6.14 (t, *J*=7.2 Hz, 1 H), 7.55 (m, 4 H), 7.65 (t, *J*=7.2 Hz, 1 H), 8.05 (d, *J*=7.6 Hz, 2 H), 8.30 (m, 2 H), 8.69 (s, 1 H), 8.76 (d, *J*=7.6 Hz, 1 H), 9.22 (t, *J*=5.9 Hz, 1 H), 11.27 (s, 1 H); ESI-MS: *m/z* 688.29 $[M+H]^+$.

Step 5: Preparation of compound **39g**

A reaction flask was charged with DMAP (2.59 g, 21.2 mmol), dry DCM (60 mL) and activated 3Å molecular sieves. The resulting mixture was stirred at room temperature for at least 2 h under inert atmosphere. Simultaneously, a solution of compound **39f** (2.05 g, 4.2 mmol) and a solution of sulfamate **39e** (3.5 g, 5.1 mmol) each in dry DCM (2 $\times$ 60 mL), were dried on activated 3Å molecular sieves (ca. 2 h). Both solutions (compound **39f** and sulfamate **39e** respectively) were successively transferred to the reaction flask. The resulting reaction mixture was stirred for 24 h. The molecular sieves were removed by filtration and thoroughly rinsed with dichloromethane. The filtrate was washed with saturated aqueous $NaHCO_3$ and saturated aqueous $NH_4Cl$. The combined organic phases were dried over $Na_2SO_4$, filtered, and concentrated under reduced pressure. The residue was purified by silica column chromatography (gradient elution: 0 - 2% MeOH in DCM) to give compound **39g** as an off-white foam (2.9 g, yield: 66%).

$^1H$ NMR (500 MHz DMSO-$d_6$) δ ppm: -0.26 (s, 3 H), -0.05 (t, *J*=11.0 Hz, 9 H), 0.71 (s, 9 H), 0.77 (s, 9 H), 3.56 (m, 2 H), 3.85 (dd, *J*=12.4, 3.4 Hz, 1 H), 3.98 (d, *J*=9.6 Hz, 1 H), 4.33 (t, *J*=3.4 Hz, 1 H), 4.46 (dt, *J*=25.2, 6.0 Hz, 1 H), 5.18 (d, *J*=4.1 Hz, 1 H), 5.31 (m, 2 H), 5.55 (dq, *J*=17.9, 4.1 Hz, 1 H), 5.98 (dd, *J*=51.0, 2.8 Hz, 1 H), 6.15 (d, *J*=7.6 Hz, 1 H), 6.51 (dd, *J*=18.9, 1.7 Hz, 1 H), 7.55 (t, *J*=7.6 Hz, 4 H), 7.65 (t, *J*=7.6 Hz, 2H), 8.04 (dd, *J*=7.9, 1.7 Hz, 4 H), 8.59 (s, 1 H), 8.75 (d, *J*=8.3 Hz, 3 H), 8.86 (t, *J*=6.2 Hz, 1 H), 11.26 (d, *J*=4.1 Hz, 2 H); ESI-MS: *m*/*z* 1036.5 $[M+H]^+$.

Step 6: Preparation of compound **34a**

A solution of compound **39g** (2.9 g, 2.8 mmol) in pyridine (55 mL) to which $Et_3N$ (19.5 mL, 140 mmol) and $Et_3N.3HF$ (4.5 mL, 28 mmol) were added, was stirred at 45 °C until complete conversion (ca. 5 h). The reaction mixture was cooled to room temperature, isopropoxytrimethylsilane (19.8 mL, 112 mmol) was added and stirring was continued overnight. The residue obtained after concentration under reduced pressure was suspended in DCM, some drops of methanol were added. The suspension was stirred for 20 min, after which the precipitate was collected by filtration and dried under high vacuum to give compound **34a** as an off-white powder (1.8 g, yield: 79%). $^1H$ NMR (500 MHz DMSO-$d_6$) δ ppm: 3.51 (dd, *J*=46.1, 13.8 Hz, 2 H), 3.65 (td, *J*=9.0, 3.4 Hz, 1 H), 3.81 (q, *J*=4.1 Hz, 1 H), 4.34 (t, *J*=3.12 Hz, 1 H), 4.41 (dt, *J*=25.9, 6.0 Hz, 1 H), 5.21 (m, 2 H), 5.40 (m, 2 H), 5.92 (dt, *J*=51.2, 3.8 Hz, 1 H), 6.11 (d, *J*=8.3 Hz, 1 H), 6.15 (d, *J*=6.2 Hz, 1 H), 6.50 (dd, *J*=17.2, 2.8 Hz,1 H), 7.55 (t, *J*=7.6 Hz, 4 H), 7.65 (t, *J*=7.6 Hz, 2 H), 8.04 (dd, *J*=7.6, 2.1 Hz, 4 H), 8.70 (s, 1 H), 8.76 (d, *J*=7.6 Hz, 3 H), 8.85 (s, 1 H), 11.27 (s, 2 H); ESI-MS: *m*/*z* 808.3 $[M+H]^+$.

Step 7: Preparation of compound **39h**

A solution of compound **34a** (890 mg, 1.10 mmol) and 1*H*-tetrazole (12.86 mL of a 3 - 4% in MeCN, dried on 3Å molecular sieves before use) in dry THF (50 mL) was pre-treated with activated 3Å molecular sieves for 2 h under $N_2$. 2-Cyanoethyl-*N,N,N',N'*-tetra(isopropyl)phosphorodiamidite (365 mg, 1.21 mmol) was added at once. The reaction mixture was shaken for 1 h after which an extra portion of 2-cyanoethyl-*N,N,N',N'*-tetra(isopropyl)phosphorodiamidite (166 mg, 0.55 mmol) was added. Shaking was continued overnight Addition of an extra amount of 2-cyanoethyl-*N,N,N',N'*-tetra(isopropyl)phosphorodiamidite (33 mg, 0.11 mmol) followed by shaking for a an additional day was needed to obtain full conversion. *t*BuOOH (341 μL of 5.5 M solution in decane, 1.87 mmol) was added and shaking was continued for 1 h. The molecular sieves were removed by filtration and extensively rinsed with DCM The filtrate was washed with brine, saturated $NaHCO_3$ and brine respectively, dried with $MgSO_4$, filtered and concentrated under reduced pressure. The residue was purified by silica column chromatography (gradient elution: 0 - 5% MeOH in DCM) to give compound 39h (168 mg, yield: 16.5%). ESI-MS: *m*/*z* 923.4 $[M+H]^+$.

Step 8: Preparation of **compound 35, sodium salt**

Compound **39h** (168 mg, 0.182 mmol) was stirred in a 33% methylamine solution in ethanol (10 mL) at room temperature for 2 h. The reaction mixture was concentrated under reduced pressure. The crude product was triturated in MeCN followed by preparative reversed phase HPLC purification (Stationary phase: XBridge C18 OBD, 5 μm, 250 × 30 mm; Mobile phase: aqueous 0.25% ammonia bicarbonate (A) - MeCN (B); gradient elution) to give **compound 35, ammonium salt**. Conversion into the sodium salt was done by elution of an aqueous solution over a column packed with a cationic sodium ion-exhange resin to give **compound 35, sodium salt** as a white fluffy solid after lyophilization (25 mg, yield: 20%). $^1H$ NMR (600 MHz, DMSO-$d_6$, 67 °C) δ ppm 10.60 (br s, 1 H), 8.32 (br s, 1 H), 8.27 (s, 1 H), 8.14 (s, 1 H), 8.13 (s, 1 H), 7.32 (br s, 2 H), 7.14 (br s, 2 H), 6.29 (dd, *J*=15.4, 4.4 Hz, 1 H), 6.05 (d, *J*=8.8 Hz, 1 H), 6.00 - 6.14 (m, 1 H), 5.48 - 5.59 (m, 2 H), 5.27 (dd, *J*=53.0, 3.6 Hz, 1 H), 4.46 - 4.53 (m, 1 H), 4.42 - 4.46 (m, 1 H), 4.08 (dt, *J*=11.7, 7.5 Hz, 1 H), 3.73 (ddd, *J*=11.7, 6.0, 3.3 Hz, 1 H), 3.69 (br d, *J*=14.7 Hz, 1 H), 3.49 (br d, *J*=14.4 Hz, 1 H); $^{31}P$ NMR (162 MHz, DMSO-$d_6$) δ ppm -1.33 (s, 1 P); ESI-MS: *m*/*z* 660.3 $[M-H]^-$.

<u>Example 40</u>

Compound 53

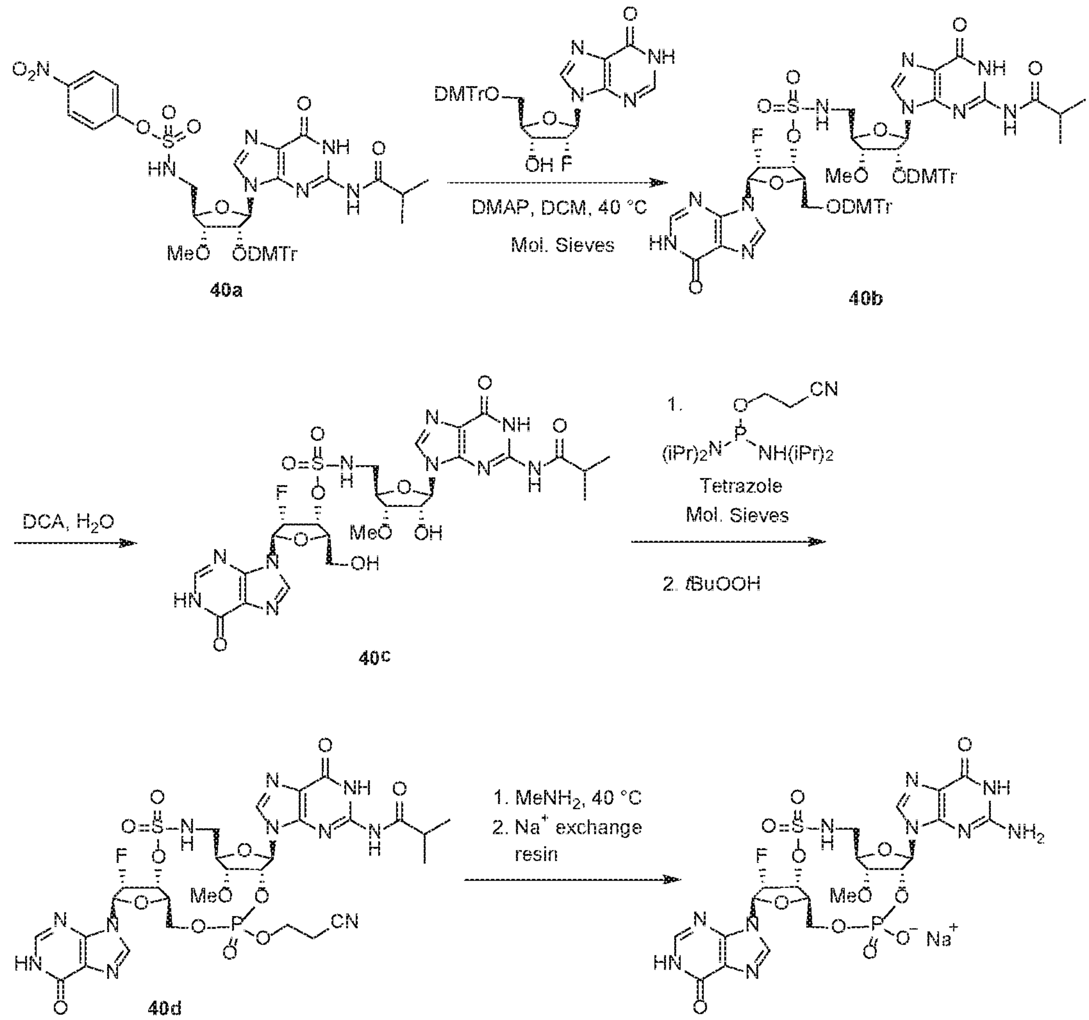


40a 40b 40c 40d

**Compound 53, sodium salt**

Step 1: preparation of compound **40b**

A reaction flask was charged with DMAP (1.43 g, 11.7 mmol), dry DCM (9 mL) and activated 4Å molecular sieves. The resulting mixture was stirred at room temperature for 3 h under inert atmosphere. Simultaneously, a solution of 5'-*O*-DMTr-2'-F-deoxyinosine [CAS 1951424-83-1] (1.47 g, 2.57 mmol) and a solution of sulfamate **40a** (2.0 g, 2.34 mmol), each in dry DCM (2 × 9 mL), were dried on activated 4Å molecular sieves (ca. 3 h). Both solutions were successively transferred to the reaction flask. The resulting reaction mixture was stirred at 40 °C overnight. The molecular sieves were removed by filtration and thoroughly rinsed with DCM The filtrate was washed with saturated aqueous $NaHCO_3$, the aqueous phase was then extracted with DCM. The combined organic phases were dried over $Na_2SO_4$, filtered, and concentrated under reduced pressure. The residue was purified by silica column chromatography (gradient elution: 1 - 3% MeOH in DCM) to give pure compound **40b** (2.01 g, 65.9%). ESI-MS: *m*/*z* 1303.8 $[M+H]^+$.

Step 2: preparation of compound **40c**

Compound **40b** (2.0 g, 1.53 mol) was dissolved in DCM (77 mL) to which water (140 μL, 7.65 mmol) and DCA (490 μL, 5.98 mmol) were added. The reaction mixture was stirred at room temperature for 2 h after which pyridine (620 μL, 7.65 mmol) and some MeOH were added. The resulting mixture was partially concentrated under reduced pressure and transferred to a silica column for purification (gradient elution: 0 - 18% MeOH in DCM) to give compound **40c** (0.92 g, 86%).

$^1H$ NMR (300 MHz, DMSO-$d_6$) δ ppm 12.48 (br s, 1 H), 12.08 (br s, 1 H), 11.60 (br s, 1 H), 8.63 (br t, *J*=5.6 Hz, 1 H), 8.34 (s, 1 H), 8.23 (s, 1 H), 8.09 (d, *J*=3.2 Hz, 1 H), 6.31 (dd, *J*=16.4, 2.9 Hz, 1 H), 5.58 - 5.88 (m, 3 H), 5.37 (br t, *J*=5.0 Hz, 1 H), 5.18 - 5.30 (m, 1 H), 4.65 (q, *J*=5.9 Hz, 1 H), 4.23 - 4.33 (m, 1 H), 4.05 (q, *J*=5.0 Hz, 1 H), 3.84 (t,

*J*=4.1 Hz, 1 H), 3.70 - 3.81 (m, 1 H), 3.54 - 3.68 (m, 1 H), 3.42 (s, 3 H), 3.20 - 3.40 (m, 2 H), 2.75 (spt, *J*=6.9 Hz, 1 H), 1.12 (br d, *J*=7.0 Hz, 3 H), 1.11 (br d, *J*=7.0 Hz, 3 H);
ESI-MS: *m*/*z* 699.4 $[M+H]^+$.

Step 3: preparation of compound **40d**

A solution of compound **40c** (200 mg, 0.286 mmol) and 1*H*-tetrazole (5.09 mL mL of a 0.45 M solution in MeCN, 2.29 mmol) in dry MeCN (8 mL) was treated with 4Å molecular sieves for 1 h under $N_2$ after which 2-cyanoethyl-*N,N,N',N'*-tetra(isopropyl)-phosphorodiamidite (173 mg, 0.57 mmol) in dry MeCN (1.0 mL) was added dropwise over 10 min (note: THF was freshly distilled over Na/benzophenone and MeCN was freshly distilled over $CaH_2$ before use). The resulting reaction mixture was stirred for 2 h at room temperature. A solution of *t*BuOOH (300 μL, 1.5 mmol) was added and stirring was continued for another 30 min. The reaction mixture was filtered through a pad of Diatomaceous earth and concentrated. The residue was purified by silica column chromatography (gradient elution: 0 - 10% MeOH in DCM) to give compound **40d** as a white solid (206 mg, 71%). ESI-MS: *m*/*z* 814.4 $[M+H]^+$.

Step 4: preparation of **compound 53, sodium salt**

Compound **40d** (206 mg, 0.253 mmol) was stirred in a 33% methylamine solution in ethanol (10 mL) at 40 °C for 3 h. The reaction mixture was concentrated under reduced pressure. The residue was dissolved in water, washed with DCM and lyophilized. The crude product was purified by preparative reversed phase HPLC (Stationary phase: Xbridge OBD C18, 5 μm, 150 × 30 mm; Mobile phase: 10 mM aqueous ammonia bicarbonate (A) - MeCN (B); gradient elution). Final conversion into the sodium salt was done by elution of an aqueous solution over a column packed with Dowex 50WX8 Na ion-exchange resin to give **compound 53, sodium salt** as a white fluffy solid after lyophilization (13 mg, 8%). $^1$H NMR (400 MHz, $D_2O$) δ ppm 8.39 (s, 1H), 8.18 (s, 1H), 8.06 (s, 1H), 6.63 (d, *J*=19.1 Hz, 1H), 6.12 (d, *J*=8.5 Hz, 1H),6.01 - 5.84 (m, 1H), 5.66 (ddd, *J*=4.1, 8.7,19.1 Hz, 1H), 5.54 - 5.44 (m, 1H), 4.75 - 4.69 (m, 1H), 4.66 (s, 1H), 4.51 (br d, *J*=11.5 Hz, 1H),4.26 (d, *J*=4.8 Hz, 1H), 4.24 - 4.17 (m, 1H), 4.03 - 3.94 (m, 1H), 3.91 - 3.81 (m, 1H), 3.77 (s, 3H); $^{31}$P NMR (162 MHz, $D_2O$) δ ppm -2.020 (s, IP);
$^{19}$F NMR (376.5 MHz, $D_2O$) δ ppm -198.634 (s, IF); ESI-MS: m/z = 691.2 $[M+H]^+$.

Example 41

Compound 54

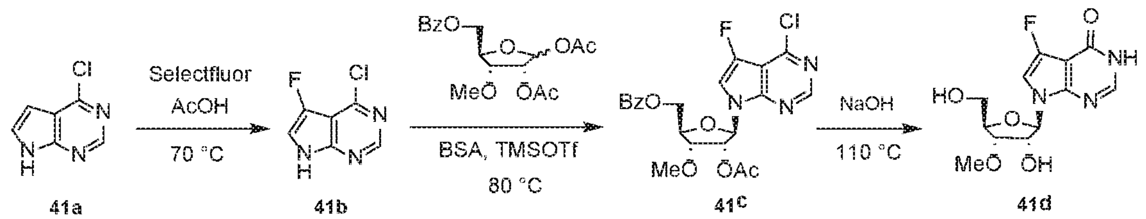


41a 41b 41c 41d

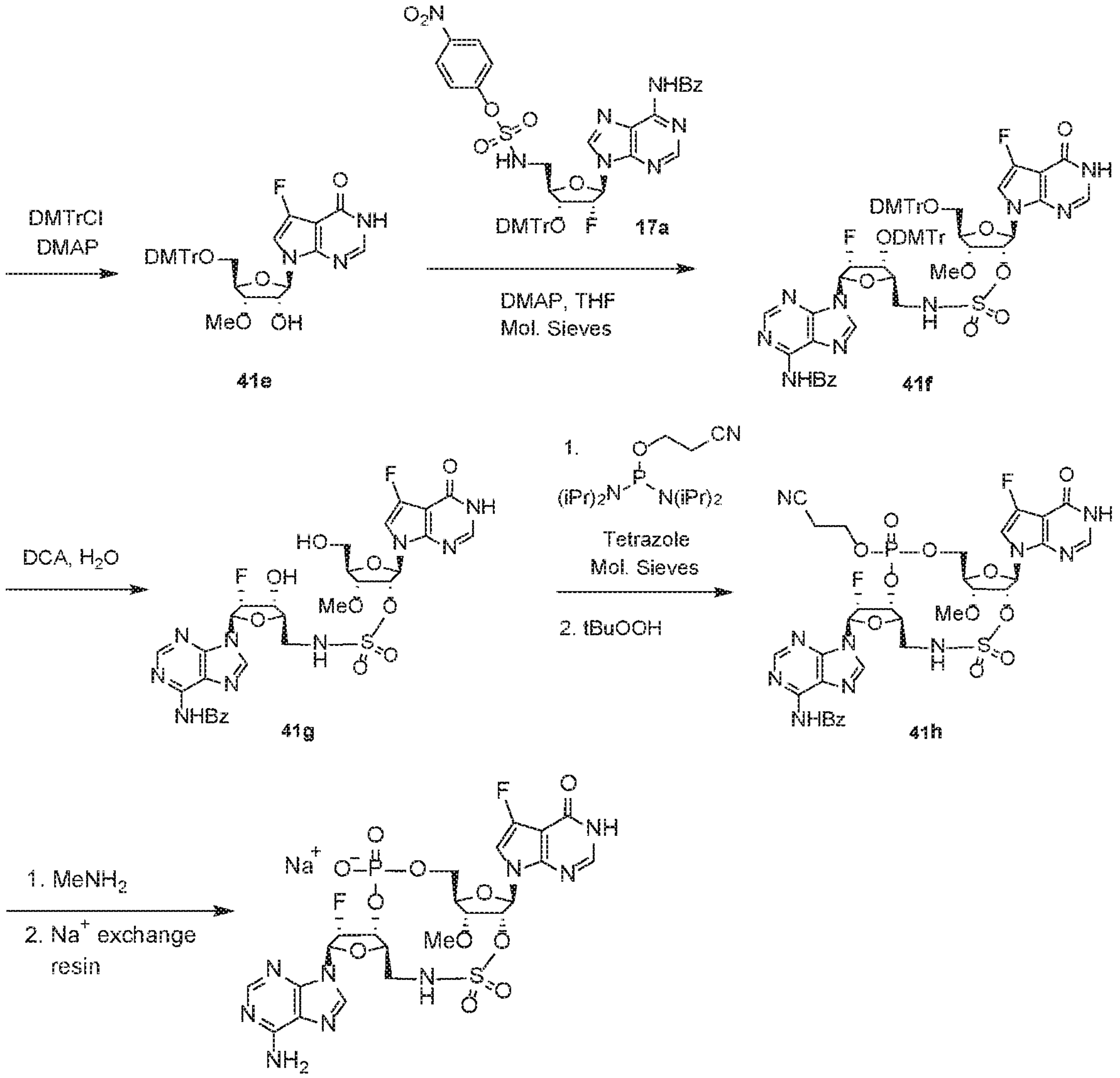


**Compound 54, sodium salt**

Step 1: preparation of compound **41b**

Selectfluor (34.6 g, 97.7 mmol) and AcOH (100 mL) were added to a solution of 4-chloro-7H-pyrrolo[2,3-d]pyrimidine [CAS 3680-69-1] (**41a**,10.0 g, 65.1 mmol) in dry MeCN (500 mL), the mixture was stirred at 70 °C until complete conversion (ca. 16 h). After cooling to room temperature, the reaction mixture was concentrated under reduced pressure and co-evaporated with anhydrous toluene. The resulting solid was dissolved in a DCM / EtOAc (1:1) solvent mixture, the obtained solution was filtered through a pad of Diatomaceous earth which was thoroughly washed. The combined filtrates were concentrated under reduced pressure. Purification was done by column chromatography over silica gel (gradient elution: 0-1% MeOH in DCM) to give compound **41b** as an off-white solid (5.2 g, yield: 47%). $^1H$ NMR (500 MHz, DMSO-$d_6$) δ ppm 12.49 (s, 1H), 8.63 (s, 1H), 7.73 (t, *J* = 2.8 Hz, 1H); ESI-MS: *m/z* 169.8 $[M-H]^-$.

Step 2: preparation of compound **41c**

Bis(trimethylsilyl)acetamide (BSA, 3.15 mL, 12.8 mmol) was added to suspension of compound **41b** (2.0 g, 11.6 mmol) in dry MeCN (80 mL). After stirring for 10 min, 1,2-di-*O*-acetyl-3-*O*-methyl-5-*O*-benzoyl-D-ribofuranose ([10300-21-7], 4.53 g, 12.8 mmol) and trimethylsilyl trifluoromethanesulfonate (TMSOTf, 2.34 mL, 12.8 mmol) were successively added. The reaction mixture was stirred 15 min at room temperature and then transferred to a pre-heated oil-bath at 80 °C, stirring was continued for 90 min. After cooling to room temperature, the reaction mixture was diluted with EtOAc and sequentially washed with saturated aqueous $NaHCO_3$ and brine. The organic layer was dried over anhydrous $Na_2SO_4$, filtered and concentrated under reduced pressure. Purification was done by column chromatography

over silica gel (gradient elution: 0-10% EtOAc in hexane) to give compound **41c** as an off-white powder (1.6 g, yield: 30 %). $^1H$ NMR (500 MHz, DMSO-$d_6$) δ ppm 8.66 (s, 1 H), 7.99 (d, *J*=1.4 Hz, 1 H), 7.95 (d, *J*=7.6 Hz, 2 H), 7.65 - 7.71 (m, 1 H), 7.47 - 7.56 (m, 2 H), 6.42 (d, *J*=4.1 Hz, 1 H), 5.78 (t, *J*=4.8 Hz, 1 H), 4.65 (dd, *J*=12.2, 3.5 Hz, 1 H), 4.51 (dd, *J*=12.2, 5.1 Hz, 1 H), 4.41 - 4.45 (m, 1 H), 4.35 - 4.40 (m, 1 H), 3.41 (s, 3 H), 2.09 (s, 3 H); ESI-MS: *m/z* 464.0 $[M+H]^+$.

Step 3: preparation of compound **41d**

2M aqueous NaOH (16 mL) was added to a solution of compound **41c** (1.6 g, 3.4 mmol) in dioxane (16 mL). The reaction mixture was stirred at 110 °C until complete conversion (ca.3 h), after which it was cooled to room temperature and neutralized with 1M aqueous HCl. The residue obtained after concentration under reduced pressure was purified by column chromatography over silica gel (gradient elution: 0 - 7% MeOH in DCM) to give compound **41d** as an off-white powder (0.32 g, yield: 45%). $^1H$ NMR (500 MHz, DMSO-$d_6$) δ ppm 12.11 (br s, 1 H), 7.92 (s, 1 H), 7.34 (d, *J*=2.1 Hz, 1 H), 6.04 (dd, *J*=6.2, 1.4 Hz, 1 H), 5.42 (d, *J*=6.2 Hz, 1 H), 5.08 (br t, *J*=5.2 Hz, 1 H), 4.33 - 4.43 (m, 1 H), 3.96 (q, *J*=3.8 Hz, 1 H), 3.77 (dd, *J*=5.0, 3.4 Hz, 1 H), 3.56 - 3.65 (m, 1 H), 3.49 - 3.55 (m, 1 H), 3.39 (s, 3 H); ESI-MS: *m/z* 299.9 $[M+H]^+$.

Step 4: preparation of compound **41e**

A solution of compound **41d** (0.51 g, 1.7 mmol) in dry pyridine (8 mL), to which DMAP (0.1 g, 0.8 mmol) and DMTrCl (0.92 g, 2.7 mmol) (portionwise) were added, was stirred at room temperature for 16 h. The reaction mixture was quenched with methanol (5 mL) and concentrated under reduced pressure. The obtained residue was dissolved in EtOAc and washed with water. The organic layer was dried over anhydrous $Na_2SO_4$, filtered and concentrated under reduced pressure. Purification was done by column chromatography over silica gel (gradient elution: 0 - 2% MeOH in DCM) to give compound **41e** as an off-white foam (0.75 g, yield: 74%). $^1H$ NMR (500 MHz, DMSO-$d_6$) δ ppm 12.13 (br s, 1 H), 7.93 (s, 1 H), 7.35 - 7.41 (m, 2 H), 7.26 - 7.32 (m, 2 H), 7.22 - 7.26 (m, 5 H), 7.18 (d, *J*=2.1 Hz, 1 H), 6.83 - 6.90 (m, 4 H), 6.06 (dd, *J*=4.8, 1.4 Hz, 1 H), 5.54 (d, *J*=6.2 Hz, 1 H), 4.47 (q, *J*=5.4 Hz, 1 H), 4.05 (q, *J*=4.4 Hz, 1 H), 3.87 (t, *J*=5.2 Hz, 1 H), 3.74 (s, 6 H), 3.33 (s, 3 H), 3.20 (d, *J*=4.1 Hz, 2 H); ESI-MS: *m/z* 602.3 $[M+H]^+$.

Step 5: preparation of compound **41f**

Compound **41e** (1.2 g, 2.03 mmol), sulfamate **17a** (2.1 g, 2.43 mmol) and DMAP (1.2 g, 10.14 mmol) were each separately dissolved in dry DCM (3 × 30.0 mL). Each solution was dried with 3Å activated molecular sieves by stirring under $N_2$ for at least 2 h. To the DMAP solution, were respectively added the solution of compound **41e** in DCM and the solution of sulfamate **17a** in DCM. The resulting reaction mixture was stirred for 36 h. The molecular sieves were removed by filtration and thoroughly rinsed with dichloromethane. The filtrate was washed with saturated aqueous $NaHCO_3$, brine and saturated aqueous $NH_4Cl$. The combined organic phases were dried over $Na_2SO_4$, filtered, and concentrated under reduced pressure. The residue was purified by silica column chromatography (gradient elution: 0 - 1 % MeOH in DCM) to give compound **41f** (1.3 g, yield: 49%). ESI-MS: *m/z* 1338.77 $[M+H]^+$.

Step 6: preparation of compound **41g**

A solution of compound **41f** (1.3 g, 0.9 mmol) in DCM (36.5 mL), to which DCA (3.2 mL of 10% in DCM, 3.8 mmol) and water (90 μL, 4.8 mmol) were added, was stirred at room temperature until complete deprotection. The reaction mixture was quenched by the addition of pyridine (0.4 mL, 4.8 mmol) and some drops of methanol. The residue obtained after concentration under reduced pressure was suspended in dichloromethane, filtered and dried to get compound **41g** as an off-white powder (0.56 g, yield: 78%).

$^1H$ NMR (500 MHz, DMSO-$d_6$) δ ppm 12.18 (br s, 1 H), 11.26 (br s, 1 H), 8.75 (s, 1 H), 8.62 (s, 1 H), 8.49 (br t, *J*=5.9 Hz, 1 H), 8.06 (d, *J*=7.6 Hz, 2 H), 7.93 (br d, *J*=2.1 Hz, 1 H), 7.63 - 7.70 (m, 1 H), 7.53 - 7.60 (m, 2 H), 7.34 (s, 1 H), 6.37 (br d, *J*=19.3 Hz, 1 H), 6.31 (br d, *J*=5.5 Hz, 1 H), 5.86 (br s, 1 H), 5.59 (br d, *J*=53.7 Hz, 1 H), 5.25 (br s, 1 H), 5.20 (br t, *J*=5.5 Hz, 1 H), 4.59 (br d, *J*=18.6 Hz, 1 H), 4.03 - 4.15 (m, 2 H), 3.98 (m, *J*=2.1 Hz, 1 H), 3.60 - 3.67 (m, 1 H), 3.53 - 3.59 (m, 1 H), 3.38 (s, 3 H), 3.11 - 3.27 (m, 2 H); ESI-MS: *m/z* 734.2 $[M+H]^+$.

Step 7: preparation of compound **41h**

A solution of compound **41g** (100 mg, 0.136 mmol) in dry MeCN / DMF (4 mL, 1:3) was treated with 4Å molecular sieves for 20 min under $N_2$ after which 1*H*-tetrazole (2.42 mL of a 0.45 M solution in MeCN, 1.09 mmol) and 2-cyanoethyl-*N,N,N',N'*-tetra(isopropyl)-phosphorodiamidite (82 mg, 0.27 mmol) in dry MeCN (1.0 mL) was added (note: MeCN was freshly distilled over $CaH_2$ before use). The resulting reaction mixture was stirred for 1.5 h at room temperature. A solution

of *t*BuOOH (126 μL, 0.69 mmol) was added and stirring was continued for another 30 min. The reaction mixture was diluted with DCM, then filtered through a pad of Diatomaceous earth and concentrated. The residue was purified by flash column chromatography over silica gel (gradient elution: 0 - 10% MeOH in DCM) to give compound **41h** (50 mg, purity 77%).

ESI-MS: *m/z* 849.3 $[M+H]^+$.

Step 8: preparation of **compound 54, sodium salt**

The above compound **41h** was stirred in a 30% methylamine solution in ethanol (5 mL) at room temperature for 3 h. The reaction mixture was concentrated under reduced pressure. The residue was dissolved in water, washed with DCM and lyophilized. The crude product was purified by preparative reversed phase HPLC (Stationary phase: Xbridge OBD C18, 5 μm, 150 × 30 mm; Mobile phase: 10 mM aqueous ammonia bicarbonate (A) - MeCN (B); gradient elution). Final conversion into the sodium salt was done by elution of an aqueous solution over a column packed with Dowex 50WX8 Na ion-exchange resin to give **compound 54, sodium salt** as a white fluffy solid after lyophilization (24 mg, yield: 25% from 41g). $^1H$ NMR (400 MHz, $D_2O$) δ ppm 8.22 (br s, 1H), 7.94 (s, 1H), 7.40 (s, 1H), 7.30 (br s, 1H), 6.46 - 6.34 (m, 2H), 5.39 (br dd, J=4.6, 8.1 Hz, 2H), 5.23 (br s, 1H), 5.08-4.93 (m, 1H), 4.63 (br s, 1H), 4.48 (br d, J=8.6 Hz, 1H), 4.41 (br d, J=3.9 Hz, 1H), 4.32-4.25 (m, 1H), 4.25 - 4.18 (m, 1H), 3.77 (br d, J=11.7 Hz, 1H), 3.59 (s, 3H), 3.54 (br d, J=13.0 Hz, 1H); $^{19}F$ NMR (376MHz, $D_2O$) δ ppm -164.506 (s, IF), -197.262 (s, 1F); $^{31}P$ NMR (162MHz, D2O) δ ppm -1.850 (s, IP); ESI-MS: m/z=692.1 $[M+H]^+$.

Example 42

Compound 5

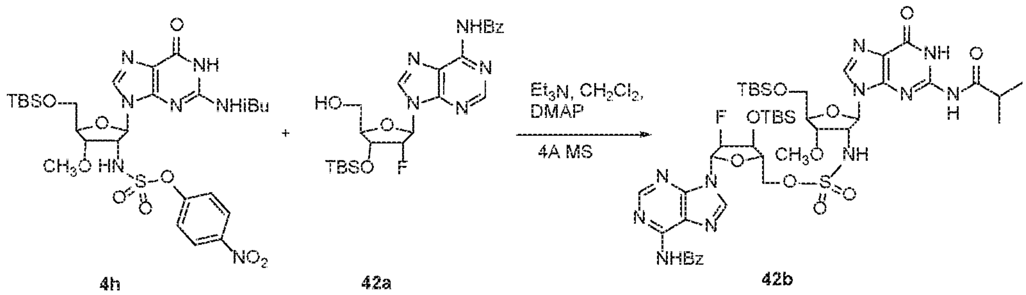


4h 42a 42b

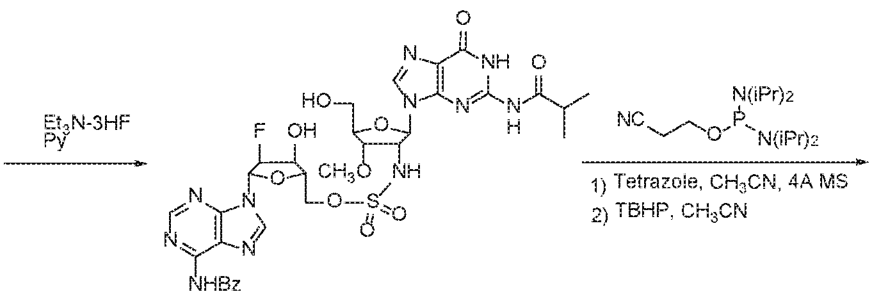


**42c**

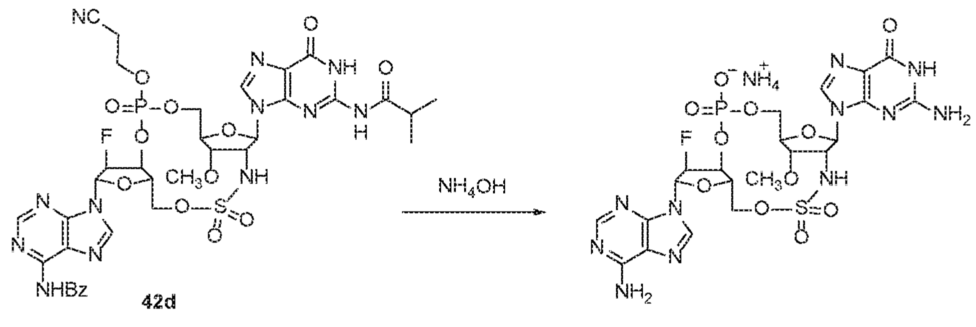


**42d**

**Compound 5, ammonium salt**

Step 1: preparation of compound **42b**

A mixture of compound **4h** (520 mg, 0.76 mmol), compound **42a** (372 mg, 0.76 mmol) and 4Å MS (1 g) in THF (20 mL) was stirred at 20°C for 1h under $N_2$. Then DMAP (466 mg, 3.81 mmol) was added. The resulting yellow suspension was stirred at 20°C for 17 h under $N_2$. The reaction mixture was filtered and concentrated under reduced pressure (<40°C) to give a residue purified by column chromatography over silica gel (gradient eluent: petroleum ether/ethyl acetate from 100/0 to 0/100 then dichloromethane / methanol from 100/0 to 100/10) to give compound **42b** (460 mg) as a pale yellow solid.

$^1H$ NMR (400 MHz, $CD_3OD$) δ ppm 8.69 (s, 1 H) 8.40 (s, 1 H) 8.08 - 8.13 (m, 3 H) 7.64-7.71 (m, 1 H) 7.54 - 7.62 (m, 2 H) 6.28 (dd, J=19.07, 1.47 Hz, 1 H) 5.94 (d, J=8.07 Hz, 1 H) 5.57 (d, J=4.40 Hz, 1 H) 5.44 (d, J=2.93 Hz, 1 H) 4.92 - 5.00 (m, 1 H) 4.61 (dd, J=8.07, 5.38 Hz, 1 H) 4.22 (br dd, J=8.19, 2.81 Hz, 2 H) 4.13 - 4.17 (m, 1 H) 4.06 (br dd, J=7.09, 3.18 Hz, 1 H) 3.84 - 3.91 (m, 2 H) 3.77 - 3.82 (m, 1 H) 3.37 (s, 3 H) 2.69 (dt, J=13.69, 6.85 Hz, 1 H) 1.20 (t, J=6.60 Hz, 6 H) 0.87 - 0.96 (m, 17 H) 0.17 - 0.21 (m, 6 H) 0.08 (d, J=11.00 Hz, 6 H); ESI-MS: *m/z*=1031.2[M+H]$^+$

Step 2: preparation of compound **42c**

A solution of compound **42b** (660 mg, 0.64 mmol), triethylamine (648.2 mg, 6.4 mmol) and trithylamine trihydrofluoride (516.3 mg, 3.20 mmol) in pyridine (20 mL) was stirred at 10°C for 17 hours. The reaction mixture was filtered and concentrated under reduced pressure at 30°C to give a crude oil purified by reverse phase preparative HPLC (Column: Agela DuraShell 5μm, 150 mm × 25 mm; mobile phase: A: water (10mM $NH_4HCO_3$) - B: MeCN; gradient elution: A (93%) : B (7%) to A (63%) and B (37%) over 9 min; Flow Rate 25 ml/min). The pure fractions were collected and lyophilized to dryness to give compound **42c** (230 mg) as white solid.

$^1H$ NMR (400 MHz, DMSO-d6) δ ppm 12.08 (s, 1 H) 11.63 (s, 1 H) 11.26 (s, 1 H) 8.69-8.81 (m, 2 H) 8.56 (s, 1 H) 8.18 (s, 1 H) 8.05 (d, J=7.28 Hz, 2 H) 7.62 - 7.71 (m, 1 H) 7.50 - 7.61 (m, 2 H) 6.38 (d, J=19.83 Hz, 1 H) 5.99 (d, J=6.53 Hz, 1 H) 5.84 (d, J=8.78 Hz, 1 H) 5.46 - 5.68 (m, 1 H) 5.15 (t, J=5.27 Hz, 1 H) 4.59 - 4.79 (m, 2 H) 4.21 - 4.32 (m, 1 H) 4.13 - 4.19 (m, 1 H) 4.03 - 4.11 (m, 2 H) 3.85 (d, J=5.52 Hz, 1 H) 3.55 - 3.63 (m, 1 H) 3.45-3.53 (m, 1 H) 3.19 (s, 3 H) 2.75 (quin, J=6.71 Hz, 1 H) 1.11 (dd, J=6.78, 2.76 Hz, 5 H) 1.08 - 1.14 (m, 1 H); ESI-MS: *m/z*-801.9[M+H]$^+$

Step 3: preparation of compound **42d**

To a solution of compound **42c** (310 mg, 0.38 mmol) in $CH_3CN$/THF (1:1, v/v, 18 mL) was added 4Å molecular sieve (1 g) and 1*H*-tetrazole in $CH_3CN$ (7 mL, 3.15 mmol, 0.45M in CH3CN). After stirring the mixture at 25 °C for 0.5 h, 2-cyanoethyl N,N,N',N'-tetraisopropylphosphorodiamidite (190 mg, 0.63 mmol) in $CH_3CN$ was added to the mixture.

After stirring the mixture at 30°C for 2 h, 1*H*-tetrazole in $CH_3CN$ (1.8 mL, 0.81 mmol, 0.45M in $CH_3CN$) was added to the mixture. After stirring the mixture at 30°C for 0.5 h, TBHP (0.4 mL, 2 mmol, 5M in decane) was added to the reaction. After stirring the mixture at 30°C for 2 h, the reaction was filtered and concentrated under reduced pressure to give crude compound **42d** (850 mg); ESI-MS: *m*/*z*=917.1 $[M+H]^+$

Step 3: preparation of **compound 5, ammonium salt**

**Compound 42d** (1000 mg, crude) was treated with methanamine in EtOH (33%) (10 mL) at RT. Aftre stirring the reaction mixture at 20°C for 2 h, the mixture was filtered and concentrated under reduced pressure. The residue was dissolved in water (30 mL) and extracted with acetate ethyl (10 mL × 2). The aqueous phase was lyophilized to dryness to give a residue purified by reverse phase preparative HPLC (Column: Agela DuraShell 5μm, 150 mm × 25 mm; mobile phase: A: water (0.04%$NH_3^{-}H_2O$+10mM $NH_4HCO_3$) - B: MeCN; gradient elution: A (100%) : B (0%) to A (90%) and B (10%) over 10 min; Flow Rate 25 ml/min). The pure fractions were collected and lyophilized to dryness to give **compound 5, ammonium salt** (111 mg).

$^1H$ NMR (400 MHz, $D_2O$) δ ppm 7.98 (s, 1 H) 7.56 - 7.70 (m, 2 H) 6.20 (br d, J=14.92 Hz, 1 H) 5.71 (br d, J=9.05 Hz, 1 H) 5.18 - 5.40 (m, 2 H) 4.98 (br s, 1 H) 4.85 (br d, J=4.89 Hz, 1 H) 4.55 (br d, J=10.27 Hz, 1 H) 4.44 - 4.52 (m, 1 H) 4.39 (br s, 1 H) 4.19 (br d, J=11.25 Hz, 1 H) 4.05 - 4.16 (m, 2 H) 4.01 (br d, J=4.40 Hz, 1 H) 3.34 - 3.43 (m, 1 H) 3.35 (s, 3 H); $^{31}P$ NMR (162 MHz, $D_2O$) δ ppm -2.37 - -0.06 (m, 1 P); $^{19}F$ NMR (376 MHz, $D_2O$) δ ppm -202.54 (br s, 1 F); ESI-MS: *m*/*z*=689.9 $[M+H]^+$

Example 43

**Compound 20**

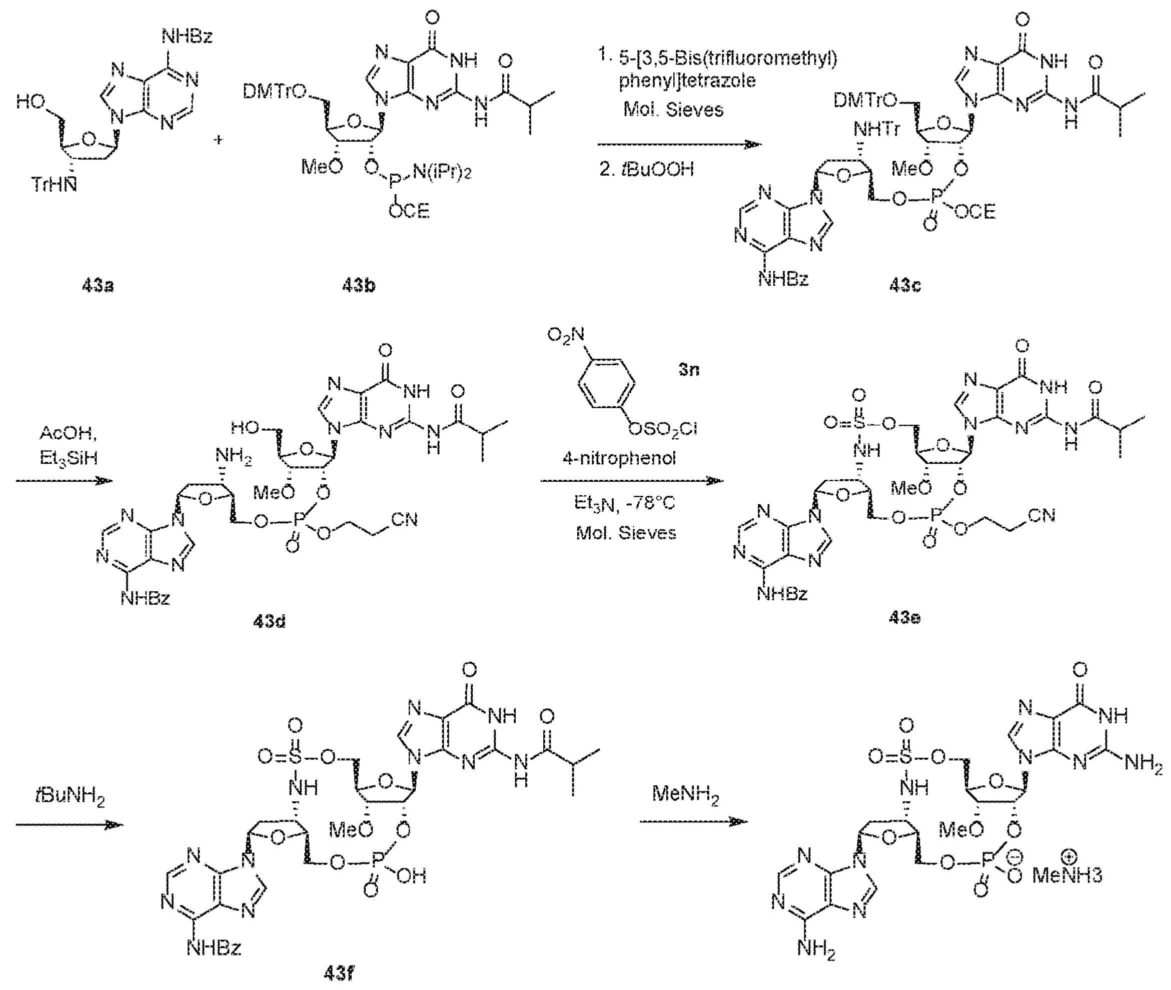


**Compound 20, methylammonium**

Step 1: Preparation of compound **43c**

5-[3,5-Bis(trifluoromethyl)phenyl]tetrazole (9.65 mL of a 0.25 M solution in MeCN, 2.41 mmol, solution dried on molecular sieves before use) was added to a solution of *N*-benzoyl-2',3'-dideoxy-3'-[(triphenylmethyl)amino]-adenosine **43a** [CAS 195375-63-4] (720 mg, 1.21 mmol) in MeCN (8 mL). Activated molecular sieves were added and the resulting mixture was stirred for 1 h under $N_2$. Next, a solution of 5'-*O*-(4,4-dimethoxytrityl)-*N*2-isobutyryl-3'-*O*-methyl-guanosine-2'-(2-cyanoethyl-N,N-diisopropyl-phosphoramidite) **43b** [CAS 179479-04-0 ] (2201 mg, 2.53 mmol) in MeCN (25 mL), dried on activated molecular sieves, was transferred to the above mixture. The resulting reaction solution was stirred for 1 h at room temperature after which *t*BuOOH (1.10 mL of a 5.5 M solution in decane, 6.03 mmol) was added, stirring was continued for an extra hour. The reaction mixture was filtered and concentrated under reduced pressure. Water and ethyl acetate were added, the water phase was separated and extracted with ethyl acetate. The combined organic phases were dried with anhydrous $Na_2SO_4$ and concentrated under reduced pressure. Purification was done by column chromatography over silica gel (gradient elution: 0 - 100% EtOAc in petroleum ether, followed by 0 -10% MeOH in EtOAc) to give compound **43c** (2.3 g).

ESI-MS: *m/z* 1381.4 $[M+H]^+$.

Step 2: Preparation of compound **43d**

A solution of the above compound **43c** (1.3 g, impure) in MeCN (15 mL), to which AcOH (80% in water, 15 mL) and triethylsilane (4.8 mL, 30.22 mmol) were added, was stirred at room temperature overnight The reaction mixture was concentrated under reduced pressure. The residue was purified by preparative reversed phase HPLC (Stationary phase: Xtimate C18, 5 μm, 150 × 25 mm; Mobile phase: 10 mM aqueous ammonia bicarbonate (A) - MeCN (B); gradient elution) to give a product mixture (260 mg) of compound **43d** and its cyanoethyl deprotected analogue. ESI-MS: *m/z*

838.2 [M+H]$^+$ & 784.2 [M+H]$^+$ (cyanoethyl deprotected compound).

Step 3: Preparation of compound **43e**

A mixture of compound **43d** and its cyanoethyl deprotected analogue (400 mg, ~ 0.49 mmol) was dissolved in pyridine / DCM (1:1, mL), followed by the addition of $Et_3N$ (403 mg, 3.98 mmol), 4-nitrophenol (346 mg, 2.49 mmol) and activated 4Å molecular sieves. The resulting solution was cooled to -78°C and stirred for 1 h under an inert atmosphere. To this, a solution of 4-nitrophenyl chlorosulfate (592 mg, 2.49 mmol) in DCM (5 mL), dried on activated 4Å molecular sieves (1 h) before use, was added. The reaction mixture was stirred at -78°C for 2 h after which it was warmed to room temperature and stirred for an additional 1.5 h. The solvent was removed under reduced pressure. Water and ethyl acetate were added, the water phase was separated and extracted with ethyl acetate. The combined organic layers were dried with anhydrous $Na_2SO_4$ and concentrated under reduced pressure. Purification was done by column chromatography over silica gel (gradient elution: 0 - 100% EtOAc in petroleum ether, followed by 0 - 10% MeOH in EtOAc) to give impure compound **43e** which was used as such without any further purification. ESI-MS: *m*/*z* 899.3 [M+H]$^+$.

Step 4: Preparation of compound **43f**

*Tert*-butylamine (200 μL, 1.88 mmol) was added to a solution of compound **43e** (100 mg, impure) in ethanol (1 mL). The reaction mixture was concentrated under reduced pressure and the crude product purified by preparative reversed phase HPLC (Stationary phase: Agela Durashell C18, 5 μm, 150 × 25 mm, Mobile phase: 10 mM aqueous ammonia bicarbonate (A) - MeCN (B); gradient elution) to give pure compound **43f** (7 mg). ESI-MS: *m*/*z* 846.2 [M+H]$^+$.

Step 5: Preparation of **compound 20**

Compound **43f** (6 mg, 7.09 μmol) was stirred in a 33% methylamine solution in ethanol (1 mL) at room temperature for 1 h. The reaction mixture was concentrated under reduced pressure and the residue triturated in MeCN to give **compound 20** (2 mg, yield: 33%).

$^1$H NMR (600 MHz, DMSO-$d_6$) δ ppm 8.37 (s, 1 H), 8.17 (s, 1 H), 7.89 (s, 1 H), 7.29 (br s, 2 H), 6.53 (br s, 2 H), 6.36 (dd, *J*=9.7, 5.4 Hz, 1 H), 5.76 (d, *J*=9.1 Hz, 1 H), 5.08 (td, *J*=9.7,4.3 Hz, 1 H), 4.73 (br d, *J*=5.1 Hz, 1 H), 4.37 (d, *J*=11.2 Hz, 1 H), 4.29 (s, 1 H), 4.16 - 4.22 (m, 2 H), 4.15 (br dd, *J*=11.8, 2.1 Hz, 1 H), 3.94 (d, *J*=4.1 Hz, 1 H), 3.52 - 3.57 (m, 1 H), 3.50 (s, 3 H), 3.12 - 3.19 (m, 1 H), 2.44 - 2.49 (m, 1 H); $^{31}$P NMR (162 MHz, DMSO-$d_6$) δ ppm 0.97 (s, 1 P); ESI-MS: *m*/*z* 672.3 [M+H]$^+$.

Example 44

**Compound (*R) 10A**

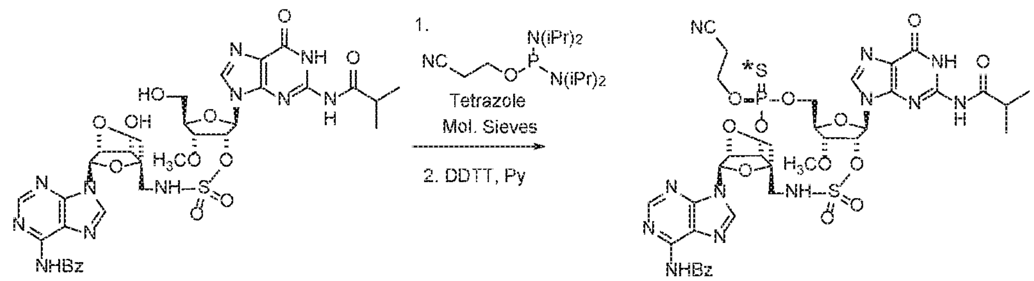


6g

44a

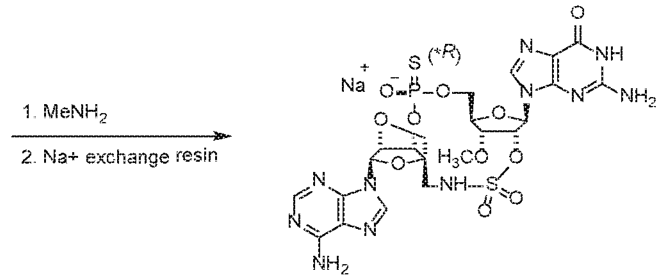


**Compound (*R) 10A, sodium salt**

Step 1: preparation of compound **44a**

Compound **6g** (95 mg, 0.117 mmol) was co-evaporated with mixture of anhydrous Toluene: Acetonitrile (1:1, v/v, 3 × 30 mL) then dissolved in anhydrous THF (10 mL). 4 Å Molecular sieves powder (0.3 g) and 0.45 M Tetrazole in $CH_3CN$ (2.0 mL, 0.936 mmol) were added and the resulting heterogeneous mixture was bubbled with Argon for 4 min. After stirring this mixture at RT for 10 min, a solution of 2-cyanoethyl N,N-diisopropyl-chlorophosphoramidite in $CH_3CN$ (57 mg, 0.18 mmol, in 3.0 mL $CH_3CN$) was added over 30 min at RT. After stirring the reaction for 1.5 hr, the molecular sieves were removed by filtration and washed with THF (15 mL). The filtrate was used directly in the next step. A solution of DDTT (119 mg, 0.585 mmol dissolved in 5 mL of pyridine) was added. After stirring the reaction mixture at RT for 30 min, the mixture was diluted with EtOAc (30 mL) and washed with saturated aqueous $NaHCO_3$ (1 × 20 mL) and brine (1 × 20 mL). Aqueous layer was back extracted with EtOAc (1 × 40 mL). Combined organic layers were evaporated to dryness, the resulting crude material was purified by flash column chromatography on silica gel (MeOH in DCM: 0 to 15%, v/v) to generate compound 44a (58 mg); ESI-MS: m/z 943 $[M+H]^+$.

Step 2: preparation of **compound (*R) 10A, sodium salt**

A solution of compound **44a** (58 mg, 0.06 mmol) in $MeNH_2$ (33% in EtOH, 10 mL) was stirred at 40°C for 2 hr 30 min, concentrated under reduced pressure.

The resulting crude solid was washed with DCM (15 mL) and the precipitate was filtered off and purified by reverse phase preparative HPLC (column: column: Synergi 4μm, Hydro RP, 250 mm × 4.6 mm, Mobile Phase: Buffer A: 50 mM Triethylammonium acetate in WATER; Buffer B: 50 mM triethylammonium acetate in $CH_3CN$; gradient: 0-40% of B over 30 min, flow rate: 24 mL/min) to afford **compound (*R) 10A** (15.9 mg) as a TEAA salt. Final conversion into the sodium salt was done by elution of an aqueous solution over a column packed with a cationic sodium ion-exchange resin. to afford

Step 3: preparation of **compound (*R) 10A, sodium salt**

Dowex 50W × 8, 200-400 (5 mL, H form) was added to a beaker and washed with deionized water (30 mL). 15% $H_2SO_4$ in deionized water was added to the resin; the mixture was gently stirred for 5 min, and decanted (30 mL). The resin was transferred to a column with 15% $H_2SO_4$ in deionized water and washed with 15% $H_2SO_4$ (at least 4 CV [Column Volume]), and then with deionized water until neutral pH (paper pH). The resin was transferred back into the beaker, 15% NaOH in deionized water solution was added, and the mixture was gently stirred for 5 min, and decanted (1 ×). The resin was transferred to the column and washed with 15% NaOH in water (at least 4 CV), and then with

deionized water until neutral pH. Compound **16** (15.9 mg) were dissolved in minimum amount of deionized water, added to the top of the column, and eluted with deionized water. Appropriate fractions were pooled together (UV) and lyophilized to give **compound (*R) 10A, sodium salt** (15.1 mg).

$^1$H NMR (400 MHz, $D_2O$): $\delta$8.33 (s, 1H), 7.99 (s, 1H), 7.71 (s, 1H), 6.30-6.42 (m, 2H), 6.22 (d, $J$ = 8.0 Hz, 1H), 5.43 (s, 1H), 5.27 (s, 1H), 4.84-4.98 (m, 1H), 4.60-4.70 (m, 1H), 4.56 (d, $J$ = 3.6 Hz, 1H), 4.32 (d, $J$ = 8.4 Hz, 1H), 4.12 (d, $J$ = 8.0 Hz, 1H), 4.01-4.11 (m, 1H), 3.81 (d, $J$ = 11.6 Hz, 1H), 3.69 (s, 3H), 3.55 (d, $J$ = 11.6 Hz, 1H) ; $^{31}$P NMR (162 MHz, $D_2O$): $\delta$ 54.07 (s, IP); ESI-MS: *m/z*: 714.7 [M-1]$^-$.

Example 45

**Compound 17**

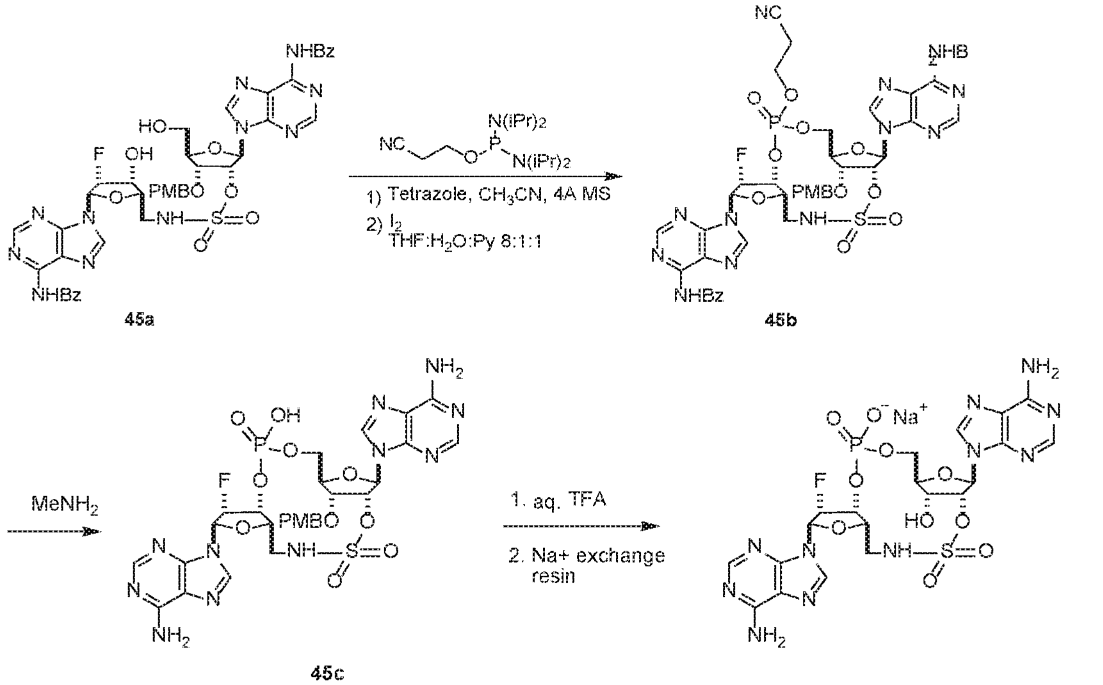


45a 45b 45c

**Compound 17, sodium salt**

Preparation of compound **45b**

Compound **45a** (200 mg, 0.216 mmol) was co-evaporated with mixture of anhydrous Toluene: MeCN (1:1, v/v, 3 × 30 mL) then dissolved in anhydrous THF (20 mL). 4 Å Molecular sieves powder (0.8 g) and 0.45 M Tetrazole in MeCN (3.8 mL, 1.72 mmol) were added. The resulting heterogeneous mixture was bubbled with Argon for 4 min. After stirring this mixture at RT for 10 min, a solution of 2-cyanoethyl-*N,N,N',N'*-tetra-(isopropyl)phosphorodiamidite (105 mg, 0.345 mmol) in MeCN (3 mL) was added to this over 30 min at RT. After stirring the reaction for 90 min, filtered off then washed with THF (15 mL). The resulting mixture (MS: m/z 1007 [M+H]$^+$) was used directly in the next step. 0.5 M Iodine (in THF:water:Py 8:1:1, v/v/v) was added to this until the color persists. After stirring at RT for 30 min, reaction mixture then diluted EtOAc (30 mL), excess iodine was quenched with aqueous saturated $Na_2S_2O_3$ (until discoloration). Phases were separated; organic phase was washed with aqueous saturated. $NaHCO_3$ (1 × 20 mL), aqueous saturated NaCl (1 × 20 mL). Aqueous phase was back extracted with EtOAc (1 × 20 mL). The combined organic phase were evaporated to dryness, the resulting crude material was purified by flash column chromatography on silica gel (0-15% MeOH in DCM, v/v) to give **45b** (135 mg). ESI-MS: m/z 1023 [M+H]$^+$.

Preparation of Compound **45c**

Compound **45b** (135 mg) was subjected to 33% methylamine solution in ethanol (10 mL) at RT. After stirring at 40 °C for 2 hr, the reaction mixture was concentrated under reduced pressure. The resulting crude solid was washed

with DCM (15 mL) and the precipitate was filtered off and to give **45c** (98 mg) as a solid. ESI-MS: m/z 796 $[M+H]^+$.

Preparation of **Compound 17 sodium salt**

Compound **45c** (98 mg) was subjected to 80% aqueous TFA (4 mL) at 0 C. After stirring for 1 hr at 0 C, the reaction mixture was concentrated under vacuum to give the crude product as colorless oil. The crude was purified by reverse phase preparative HPLC (column: Synergi 4μm, Hydro RP, 250 mm × 30 mm, Mobile Phase: Buffer A: 50 mM Triethylammonium acetate in WATER; Buffer B: 50 mM Triethylammoniumacetate in $CH_3CN$, gradient: 0-40% of B over 30 min, flow rate 24 mL/min) to give **compound 17** (24.2 mg) as a TEAA salt. ESI-MS: m/z: 674 $[M-1]^-$,

**Preparation of sodium salt**

Dowex 50W × 8, 200-400 (5 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 ×). The resin was transferred to the column and washed with 15% NaOH in water at least 4 CV), and then with deionized water until it was neutral. **Compound 17** TEAA salt (1:1, v/v) was dissolved in minimum amount of deionized water and MeCN (1:1, v/v), added to the top of the column, and eluted with deionized water. Appropriate fractions were pooled together and lyophilized to give **compound 17, sodium salt** (22.9 mg).

$^1H$ NMR (400 MHz, $D_2O$) $\delta$ppm 8.31 (s, 1H), 7.95 (s, 1H), 7.76 (s, 1H), 6.74 (s, 1H), 6.15-6.25 (m, 2H), 5.45-5.55 (m, 1H), 5.25 (d, J = 4.8 Hz, 0.5H), 5.13 (d, J = 4.8 Hz, 0.5H), 4.75-4.90 (m, 1H), 4.65 (s, 1H), 4.35-4.45 (m, 1H), 4.33 (d, J = 9.6 Hz, 1H), 4.10-4.25 (m, 2H), 3.55-3.70 (dd, J = 3.2, 13.2 Hz, 1H), 3.38 (d, J = 12 Hz, 1H).

$^{31}P$ NMR (162 MHz, $D_2O$) $\delta$-1.895 ppm (s, IP); $^{19}F$ NMR (379 MHz, $D_2O$) $\delta$-196.91 ppm (broad peak, IF); ESI-MS: m/z: 658.5 $[M-1]^-$.

Example 46

**Compound 18**

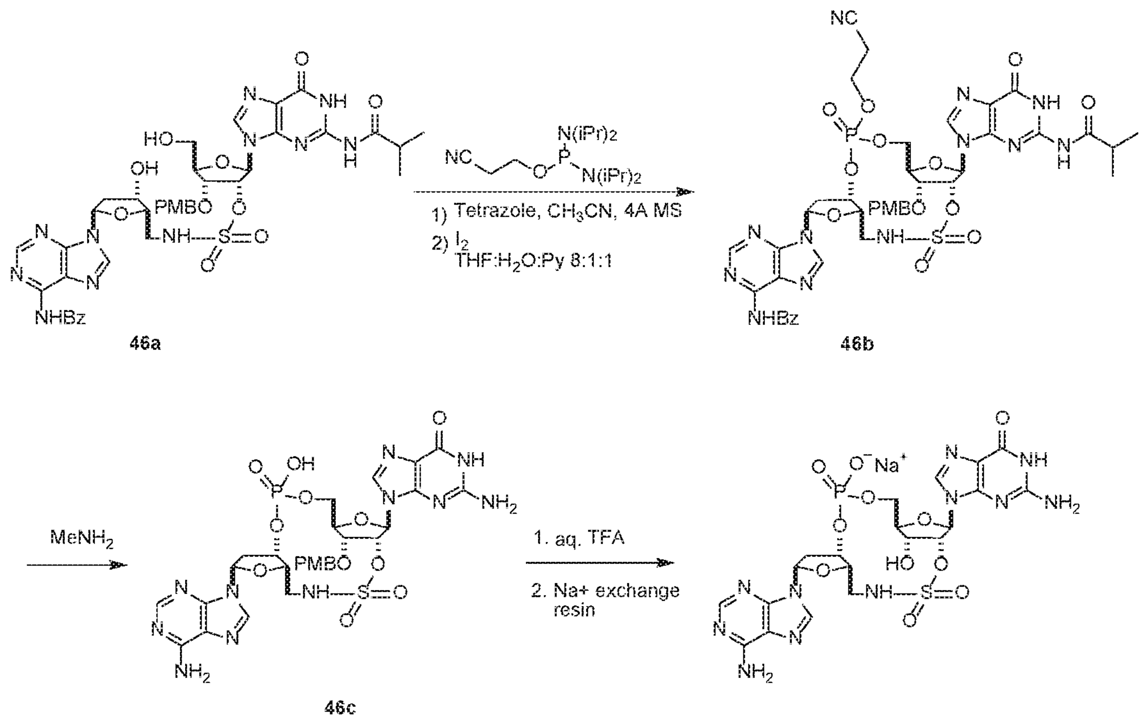


**Compound 18, sodium salt**

Preparation of compound **46b**

Compound **46a** (110 mg, 0.123 mmol) was co-evaporated with mixture of anhydrous Toluene: Acetonitrile (1:1, v/v, 3 × 30 mL) then dissolved in anhydrous THF (14 mL). 4 Å Molecular sieves powder (0.5 g) and 0.45 M Tetrazole in MeCN (2.1 mL, 0.989 mmol) were added to this. The resulting heterogeneous mixture was bubbled with Ar for 4 min. After stirring this mixture at RT for 10 min, a solution of 2-cyanoethyl-*N,N,N',N'*-tetra(isopropyl)phosphorodiamidite (60 mg, 0.197 mmol) in MeCN (3.0 mL) was added to over 30 min at RT. After stirring for 90 min at RT, the reaction mixture was filtered off then washed with THF (15 mL). The resulting mixture (MS: m/z 989 $[M+H]^+$) was used directly into the next step. 0.5 M Iodine (in THF:Water:Py 8:1:1, v/v/v) was added to this until the color persists. After stirring at RT for 30 min, the reaction mixture was diluted with EtOAc (30 mL), excess iodine was quenched with aqueous saturated $Na_2S_2O_3$ (until discoloration). Phases were separated; organic phase was washed with aqueous saturated $Na_2S_2O_3$ (until discoloration). Phases were separated; organic phase was washed with aqueous saturated. $NaHCO_3$ (1 × 20 mL), aqueous saturated NaCl (1 × 20 mL). Aqueous phase was back extracted with EtOAc (1 × 20 mL). The combined organic phase were evaporated to dryness, the resulting crude material was purified by flash column chromatography on silica gel (0-15% MeOH in dichloromethane, v/v) to give compound **46b** (77 mg). ESI-MS: m/z 1005.8 $[M+H]^+$.

Preparation of compound **46c**

Compound **46b** (77 mg) was subjected to 33% methylamine solution in ethanol (6 mL) at RT. After stirring at 40 °C for 2 hr, the reaction mixture was concentrated under reduced pressure. The resulting crude solid was washed with DCM (15 mL) and the precipitate was filtered off and to give compound **46c** (43 mg). ESI-MS: m/z 778 $[M+H]^+$.

Preparation of **compound 18, sodium salt**

Compound **46c** (43 mg) was subjected to 80% aqueous TFA (2 mL). After stirring at RT for 1 hr, the reaction mixture was concentrated under vacuum to give a residue as a colorless oil. The residue was purified by reverse phase preparative HPLC (column: Synergi 4μm, Hydro RP, 250 mm × 30 mm, Mobile Phase: Buffer A: 50 mM Triethylammonium acetate in water; Buffer B: 50 mM Triethylammoniumacetate in $CH_3CN$, gradient: 0-40% of B over 30 min, flow rate 24 mL/min) to give **compound 18** (6.4 mg) as a TEAA salt

Dowex 50W × 8, 200-400 (5 mL, H form) was added to a beaker and washed with deionized water (30 mL). Then to the resin was added 15% $H_2SO_4$ in deionized water, the mixture was gently stirred for 5 min, and decanted (30 mL). The resin was transferred to a column with 15% $H_2SO_4$ in deionized water and washed with 15% $H_2SO_4$ (at least 4 CV), and then with deionized water until it was neutral. The resin was transferred back into the beaker, 15% NaOH in deionized water solution was added, and mixture was gently stirred for 5 min, and decanted (1 ×). The resin was transferred to the column and washed with 15% NaOH in water (at least 4 CV), and then with deionized water until it was neutral. **compound 18** (6.4 mg) TEAA salt was dissolved in minimum amount of deionized water and MeCN (1:1, v/v), added to the top of the column, and eluted with deionized water. Appropriate fractions were pooled together and lyophilized to give **compound 18, sodium salt** (5.9 mg).

$^1H$ NMR (400 MHz, $D_2O$) $\delta$ ppm 7.93 (s, 1H), 7.84 (s, 1H), 7.21 (s, 1H), 6.32 (dd, J = 2.8; 4.8 Hz, 1H), 6.05 (d, J = 8.4 Hz, 1H), 5.92-6.01 (m, 1H), 5.15-5.25 (m, 1H), 4.61 (d, J = 12 Hz, 1H), 4.30-4.36 (m, 1H), 3.90-4.20 (m, 3H), 3.50-3.58 (dd, J = 3.2, 13.2 Hz, 1H), 3.20-3.33 (dd, J = 2, 13.2 Hz, 1H), 2.60-2.75 (m, 2H). $^{31}P$ NMR (162 MHz, $D_2O$) $\delta$ ppm -0.838 (s, IP); ESI-MS: m/z: 656.8 $[M-1]^-$.

Example 47

## Compound (*R) 21A

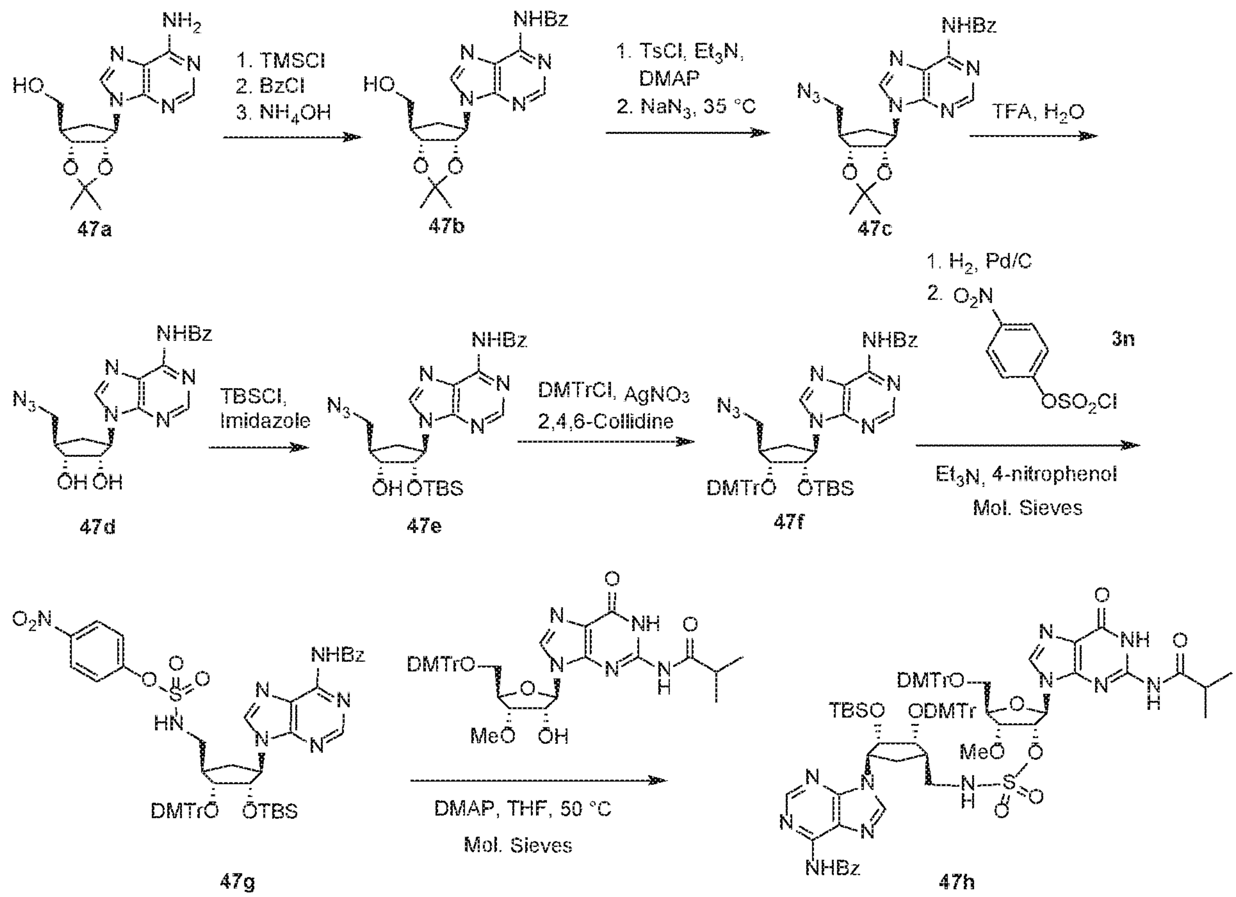

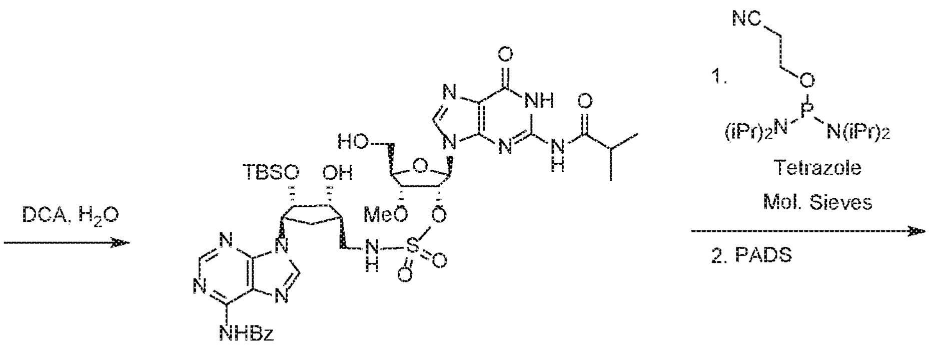


47i

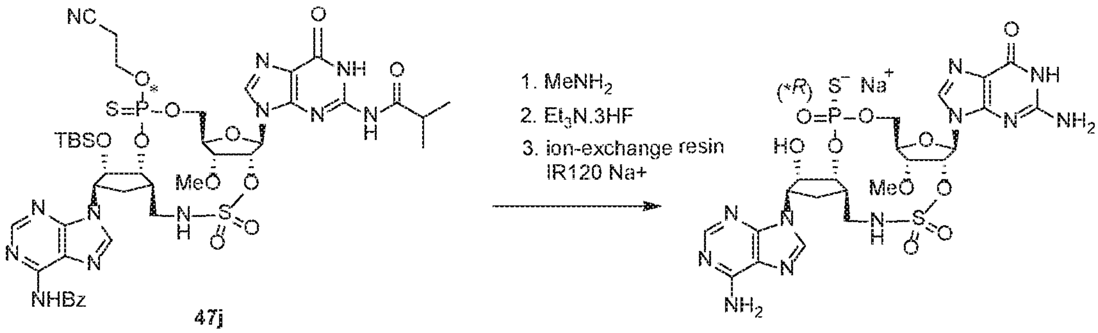


47j

Compound (*R) 21A, sodium salt

Step 1: Preparation of compound **47b**

Chlorotrimethylsilane (2.08 mL, 16.38 mmol) was dropwise added to a solution of compound **47a** [CAS 174171-97-2] (1 g, 3.28 mmol) in dry pyridine (60 mL) at 0 °C. The reaction mixture was stirred at 0 °C for 2 h after which benzoyl chloride (1.89 mL, 16.38 mmol) was added dropwise. Stirring was continued at room temperature for 3 h. The reaction solution was cooled to 0 °C, water (30 mL) was added followed by the addition of aqueous ammonia (30 mL of 25% solution), stirring was continued for 90 min at 0 °C. The reaction solution was extracted with EtOAc, the combined organic layers were dried with $Na_2SO_4$, filtered and concentrated under reduced pressure. Purification was done by column chromatography over silica gel (gradient elution: 50 - 100% EtOAc in petroleum ether) to give compound **47b** as a white solid (1.1 g, yield: 82%).

$^1H$ NMR (400 MHz, DMSO-$d_6$) $\delta$ ppm 11.17 (s, 1 H), 8.75 (s, 1 H), 8.64 (s, 1 H), 8.05 (d, *J*=7.3 Hz, 2 H), 7.61 - 7.68 (m, 1 H), 7.46 - 7.59 (m, 2 H), 5.02 - 5.09 (m, 1 H), 4.90 - 5.01 (m, 1 H), 4.83 (t, *J*=5.3 Hz, 1 H), 4.59 (dd, *J*=7.2, 3.9 Hz, 1 H), 3.53 (br t, *J*=5.0 Hz, 2 H), 2.17 - 2.41 (m, 3 H), 1.50 (s, 3 H), 1.25 (s, 3 H); ESI-MS: *m/z* 410.2 $[M+H]^+$.

Step 2: Preparation of compound **47c**

DMAP (448 mg, 3.66 mmol) and tosyl chloride (2.79 g, 14.65 mmol) were added to a solution of compound **47b** (3 g, 7.32 mmol) and $Et_3N$ (3.07 mL, 21.98 mmol) in DCM (6 mL) under ice cooling. The reaction mixture was stirred at room temperature for 4 h. The mixture was quenched with water and extracted with DCM. The organic layer was washed with brine, dried with anhydrous $Na_2SO_4$ and evaporated under reduced pressure to give the tosylated product. The crude product was dissolved in DMF (15 mL) followed by the addition of sodium azide (1.88 g, 28.91 mmol), the resulting reaction mixture was stirred at 35 °C for 16 h. Next, the reaction mixture was cooled to room temperature, diluted with saturated aqueous $Na_2CO_3$, and extracted with EtOAc. The organic layer was separated, washed with brine, dried with anhydrous $Na_2SO_4$ and evaporated under reduced pressure. The crude product was purified by silica column chromatography (gradient elution: 0-25% EtOAc in petroleum ether) to give compound **47c** as a yellow solid (2.1 g, yield: 61%).

$^1H$ NMR (400 MHz, DMSO-$d_6$) $\delta$ ppm 1.25 (s, 3 H), 1.51 (s, 3 H), 2.24 - 2.48 (m, 3 H), 3.53 (dd, *J*=12.3, 6.3 Hz, 1 H), 3.60 (dd, *J*=12.3, 5.3 Hz, 1 H), 4.02 (q, *J*=7.2 Hz, 1 H), 4.58 (dd, *J*=7.2,4.6 Hz, 1 H), 4.93 - 5.03 (m, 1 H), 5.03 - 5.11 (m, 1 H), 7.51 - 7.59 (m, 2 H), 7.60 - 7.71 (m, 1 H), 8.04 (d, *J*=7.5 Hz, 2 H), 8.64 (s, 1 H), 8.75 (s, 1 H), 11.19 (br s, 1 H);

ESI-MS: *m/z* 435.1 $[M+H]^+$.

Step 3: Preparation of compound **47d**

TFA (100 mL, 75% in water) was added to a solution of compound **47c** (3.47 g, 7.99 mmol) in DCM (20 mL), the resulting mixture was stirred at room temperature for 1 h. The reaction was concentrated under reduced pressure, the obtained residue was dissolved in EtOAc and washed with saturated aqueous $K_2CO_3$, the water layer was separated and extracted with EtOAc. The combined organic layers were dried with anhydrous $Na_2SO_4$ and evaporated under vacuum to give compound **47d** as a white solid (3.25 g, crude) which was used as such in the next step.
ESI-MS: *m/z* 395.0 $[M+H]^+$.

Step 4: Preparation of compound **47e**

Compound **47d** (3.25 g) was dissolved in DMF (22 mL), followed by the addition of imidazole (1.57 g, 23.0 mmol) and TBSCl (1.39 g, 9.20 mmol). A second reaction was done in parallel on the same scale. Both reaction mixtures were stirred at room temperature overnight after which EtOAc and brine were added. The organic phase was separated and the water layer extracted with EtOAc. The combined organic layers were dried with anhydrous $Na_2SO_4$, filtered and evaporated under reduced pressure to give the crude regio-isomeric mixture. Separation was done by preparative reversed phase HPLC (Stationary phase: Phenomenex Synergi, 10 $\mu$m Max-RP, 250 $\times$ 50 mm; Mobile phase: WATER (A)-MeCN (B); gradient elution) to give compound **47e** (2.0 g, yield: 24%) as the first eluting isomer and the 3'-TBS-protected regio-isomer (**47ea**, structure not shown, 2.5 g, yield: 31%) as the second eluting isomer.
$^1H$ NMR (400 MHz, DMSO-$d_6$) $\delta$ ppm 11.13 (s, 1 H), 8.72 (s, 1 H), 8.61 (s, 1 H), 8.04 (*br* d, *J*=7.5 Hz, 2 H), 7.61 - 7.68 (m, 1 H), 7.51 - 7.58 (m, 2 H), 4.91 - 5.01 (m, 1 H), 4.78 (d, *J*= 5.3 Hz, 1 H), 4.52 (dd, *J*=8.5, 5.5 Hz, 1 H), 3.79 - 3.86 (m, 1 H), 3.54 - 3.64 (m, 2 H), 2.34 (dt, *J*=12.6, 8.5 Hz, 1 H), 2.16 - 2.28 (m, 1 H), 1.94 - 2.04 (m, 1 H), 0.64 (s, 9 H),-0.16 (s, 3 H), -0.38 (s, 3 H);
ESI-MS: *m/z* 509.2 $[M+H]^+$.

Step 5: Preparation of compound **47f**

DMTrCl (394 mg, 1.16 mmol), $AgNO_3$ (492 mg, 2.90 mmol) and 2,4,6-collidine (351 mg, 2.90 mmol) were added to a solution of compound **47e** (300 mg, 0.59 mmol) in DCM (6 mL), the resulting mixture was stirred at room temperature overnight. The reaction was quenched with brine, the water layer was separated and extracted with DCM The combined organic layers were dried with anhydrous $Na_2SO_4$, filtered and evaporated under vacuum. The crude product was purified by silica column chromatography (gradient elution: 0 -100% EtOAc in petroleum ether) to give compound **47f** (430 mg, yield: 90%) as a yellow solid.
$^1H$ NMR (400 MHz, DMSO-$d_6$) $\delta$ ppm -0.68 (s, 3 H), -0.22 (s, 3 H), 0.67 - 0.75 (m, 9 H), 0.80 - 0.89 (m, 1 H), 1.54 - 1.66 (m, 1 H), 2.00 - 2.08 (m, 1 H), 2.29 - 2.39 (m, 1 H), 3.21 (dd, *J*=12.0, 5.9 Hz, 1 H), 0.00 (br dd, *J*=3.9, 1.0 Hz, 1 H), 3.74 (s, 3 H), 3.75 (s, 3 H), 4.58 (dd, *J*=9.3, 3.9 Hz, 1 H), 5.37 (q, *J*=9.3 Hz, 1 H), 6.83 - 6.96 (m, 4 H), 7.20 - 7.27 (m, 1 H), 7.28 - 7.35 (m, 2 H), 7.36 - 7.44 (m, 4 H), 7.50 - 7.60 (m, 4 H), 7.61 - 7.67 (m, 1 H), 8.05 (br d, *J*=7.6 Hz, 2 H), 8.62 - 8.69 (m, 1 H), 8.71 (s, 1 H), 11.12 (br s, 1 H);
ESI-MS: *m/z* 811.3 $[M+H]^+$.

Step 6: Preparation of compound **47g**

A suspension of compound **47f** (3.23 g, 3.98 mmol) in EtOAc (150 mL) was stirred under $H_2$ (15 psi) at 30 °C overnight in the presence of Pd/C (10% on carbon, 3.0 g). The catalyst was removed by filtration over Diatomaceous earth, the filtrate was concentrated under reduced pressure to give the compound amine which was immediately used as such in the next step. The crude product was dissolved in DCM (110 mL), followed by the addition of 4-nitrophenol (3.53 g, 25.38 mmol), $Et_3N$ (2.12 mL, 15.23 mmol) and activated molecular sieves. The resulting mixture was cooled to -78 °C under $N_2$ atmosphere after which 4-nitrophenyl chlorosulfate (3.62 g, 15.23 mmol) in DCM (20 mL) was added dropwise, the reaction solution was allowed to warm to room temperature and stirred overnight. The mixture was diluted with DCM and filtered through a pad of Diatomaceous earth. The filtrate was washed with saturated aqueous $NaHCO_3$, dried with $Na_2SO_4$ and concentrated. The crude product was purified by silica column chromatography (gradient elution: 0-50% EtOAc in petroleum ether) to give compound **47g** (2.6 g, yield: 67%).
$^1H$ NMR (400 MHz, DMSO-$d_6$) $\delta$ ppm -0.62 (s, 3 H), -0.30 (s, 3 H), 0.65 (s, 9 H), 1.92-2.11 (m, 2 H), 2.44 (br dd, *J*=9.9, 3.5 Hz, 1 H), 2.85 - 3.01 (m, 1 H), 3.04 - 3.15 (m, 1 H), 3.45 (br d, *J*=3.9 Hz, 1 H), 3.72 (s, 3 H), 3.72 (s, 3 H), 4.68 (br dd, *J*=9.5, 3.9 Hz, 1 H), 5.47 (q, *J*=9.5 Hz, 1 H), 6.87 (s, 3 H), 7.19 - 7.25 (m, 1 H), 7.26 - 7.34 (m, 2 H), 7.35-7.48 (m, 5 H), 7.51 - 7.60 (m, 3 H), 7.62 - 7.70 (m, 1 H), 8.05 (br d, *J*=7.3 Hz, 2 H), 8.33 (d, *J*=9.0 Hz, 2 H), 8.67 (d, *J*=7.1 Hz, 1 H), 8.80 (br t, *J*=5.7 Hz, 1 H), 11.15 (s, 1 H); ESI-MS: *m/z* 986.6 $[M+H]^+$.

Step 7: Preparation of compound **47h**

Activated molecular sieves were added to a solution of compound **47g** (1 g, 1.07 mmol) and 5'-O-(4,4'-Dimethoxytrityl)-N2-isobutyryl-3'-O-methyl-D-guanosine [CAS 103285-33-2] (717 mg, 1.01 mmol) in dry THF (25 mL), the resulting mixture was stirred at room temperature for 2 h under nitrogen. Next, a solution of DMAP (619 mg, 5.07 mmol) in THF (10 mL), pre-dried by stirring for 2 h in the presence of activated molecular sieves, was added to initiate the reaction. The resulting reaction mixture was stirred at 50 °C overnight. Molecular sieves were removed by filtration and the filtrate washed with saturated aqueous $NaHCO_3$. The aqueous phase was extracted with DCM. The combined organic layers were dried with $Na_2SO_4$, filtered and concentrated under reduced pressure. The crude product was purified by silica column chromatography (gradient elution: 0 - 2% MeOH in DCM) to give compound **47h** (875 mg, yield: 54%). ESI-MS: *m/z* 759.2 $[M+2H]/2^+$.

Step 8: Preparation of compound **47i**

A solution of compound **47h** (875 mg, 0.56 mmol) in DCM (40 mL) was treated with DCA (0.19 mL, 2.25 mmol) in the presence of water (0.05 mL, 2.81 mmol) for 1 h. The reaction mixture was quenched by the addition of pyridine (0.23 mL, 2.81 mmol) in methanol (2 mL), followed by concentration under reduced pressure. The resulting residue was dissolved in DCM, the obtained solution was washed with water and brine, dried with anhydrous $Na_2SO_4$, filtered and concentrated under reduced pressure. The crude product was purified by silica column chromatography (gradient elution: 0-5% MeOH in DCM) to give compound **47i** as a white solid (465 mg, yield: 91%).

$^1H$ NMR (400 MHz, DMSO-$d_6$) δ ppm 12.11 (br s, 1 H), 11.58 (br s, 1 H), 11.09 (br s, 1 H), 8.70 (s, 1 H), 8.57 (s, 1 H), 8.44 (br s, 1 H), 8.29 (s, 1 H), 8.05 (d, *J*=7.3 Hz, 2 H), 7.59 - 7.70 (m, 1 H), 7.47 - 7.58 (m, 2 H), 6.10 (d, *J*=6.5 Hz, 1 H), 5.41 (dd, *J*=6.5, 5.3 Hz, 1 H), 5.29 (t, *J*=5.3 Hz, 1 H), 4.91 (q, *J*=9.3 Hz, 1 H), 4.54 (br d, *J*=3.7 Hz, 1 H), 4.48 (dd, *J*=8.7, 5.5 Hz, 1 H), 4.22 (dd, *J*=4.9, 2.4 Hz, 1 H), 4.15 (q, *J*=3.3 Hz, 1 H), 3.76 (br s, 1 H), 3.54 - 3.73 (m, 2 H), 3.49 (s, 3 H), 2.97 (dd, *J*=13.4, 7.7 Hz, 1 H), 2.81 - 2.90 (m, 1 H), 2.75 (spt, *J*=6.8 Hz, 1 H), 2.18 - 2.29 (m, 1 H), 2.02 - 2.15 (m, 1 H), 1.74 - 1.87 (m, 1 H), 1.11 (d, *J*=6.5 Hz, 3 H), 1.08 (d, *J*=6.9 Hz, 3 H), 0.63 (s, 9 H), -0.17 (s, 3 H), -0.41 (s, 3 H); ESI-MS: *m/z* 912.7 $[M+H]^+$.

Step 9: Preparation of compound **47j**

A solution of compound **47i** (280 mg, 0.31 mmol) and 1*H*-tetrazole (3.58 mL of a 3-4% in MeCN, pre-dried on activated 3Å molecular sieves) in MeCN / THF (1:1, 44 mL, pre-dried on activated 3Å molecular sieves) was treated with activated 3Å molecular sieves for 2 h under $N_2$ after which 2-cyanoethyl-*N,N,N',N'*-tetra(isopropyl)phosphorodiamidite (97 μL, 0.31 mmol) was added at once. The resulting reaction mixture was shaken at room temperature for 3 h. PADS (0.19 g, 0.61 mmol) was added and shaking was continued for an extra hour. Molecular sieves were removed by filtration and rinsed with dichloromethane. The combined filtrates were washed with saturated aqueous $NaHCO_3$ and brine, dried with MgSO4, filtered and concentrated under reduced pressure. The residue was purified by silica column chromatography (gradient elution: 0 - 10% MeOH in DCM) to give compound **47j** (91 mg, yield: 26%) as a single diastereomer.

$^{31}P$ NMR (162 MHz, DMSO-$d_6$) δ ppm 66.65 (s, 1 P);
ESI-MS: *m/z* 1043.7 $[M+H]^+$.

Step 10: Preparation of **compound (*R) 21A, sodium salt**

Compound **47j** (86 mg, 0.077 mmol) was stirred in a 33% methylamine solution in ethanol (4 mL) at 45 °C until complete conversion (ca. 1 h), after which the reaction solution was cooled to room temperature and concentrated under reduced pressure. The residue was dissolved in pyridine (4 mL), followed by the addition of $Et_3N$ (320 μL, 2.32 mmol) and triethylamine trihydrofluoride (200 μL, 1.16 mmol). The reaction mixture was stirred at 45 °C for 18 h. Isopropoxytrimethylsilane (0.82 mL, 4.65 mmol) was added and stirring was continued at room temperature for 18 h. The residue obtained after concentration under reduced pressure was triturated in anhydrous acetonitrile, the obtained precipitate was further purified by preparative reversed phase HPLC (Stationary phase: XBridge C18 OBD, 10 μm, 150 × 50 mm, Mobile phase: 0.25% aqueous ammonia bicarbonate (A) - MeOH (B); gradient elution) to give **compound (*R) 21A** as a single diastereomer. Final conversion into the sodium salt was done by elution of an aqueous solution over a column packed with a cationic Na ion-exchange resin affording **compound (*R) 21A, sodium salt** as a white fluffy solid (27 mg, yield: 46%).

$^1H$ NMR (400 MHz, $D_2O$) δ ppm 8.36 (s, 1 H), 8.18 (s, 1 H), 7.87 (s, 1 H), 6.10 (d, *J*=8.1 Hz, 1 H), 5.68 (dd, *J*=8.3, 4.3 Hz, 1 H), 4.83 - 4.95 (m, 2 H), 4.67 - 4.72 (m, 1 H), 4.61 (m, *J*=2.0 Hz, 1 H), 4.39 (d, *J*=4.5 Hz, 1 H), 4.27 (ddd, *J*=11.8, 6.5, 2.4 Hz, 1 H), 4.18 (ddd, *J*=12.2, 4.5, 1.6 Hz, 1 H), 3.55 (s, 3 H), 3.36 (br d, *J*=4.8 Hz, 2 H), 2.55 - 2.65

(m, 2 H), 1.92 - 2.03 (m, 1 H);

$^{31}P$ NMR (162 MHz, $D_2O$) δ ppm 54.55 (s, 1 P);

ESI-MS: *m*/*z* 702.4 $[M+H]^+$.

Example 48

Compound (*R) 43A

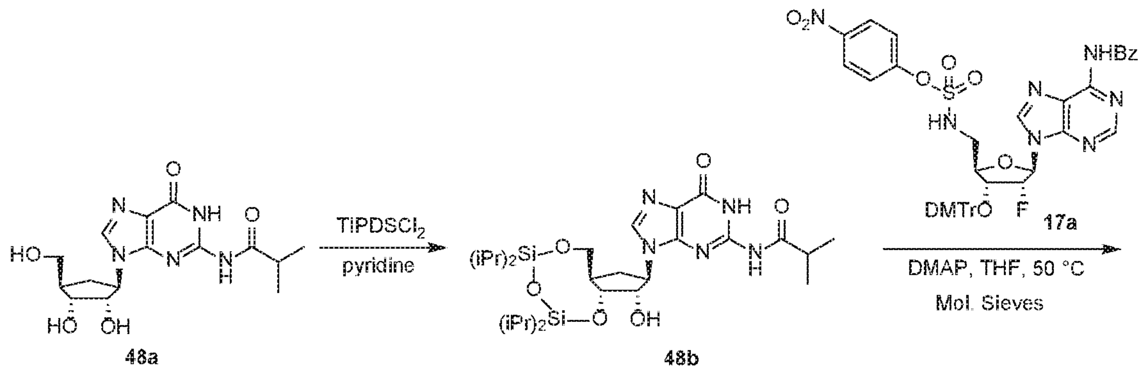

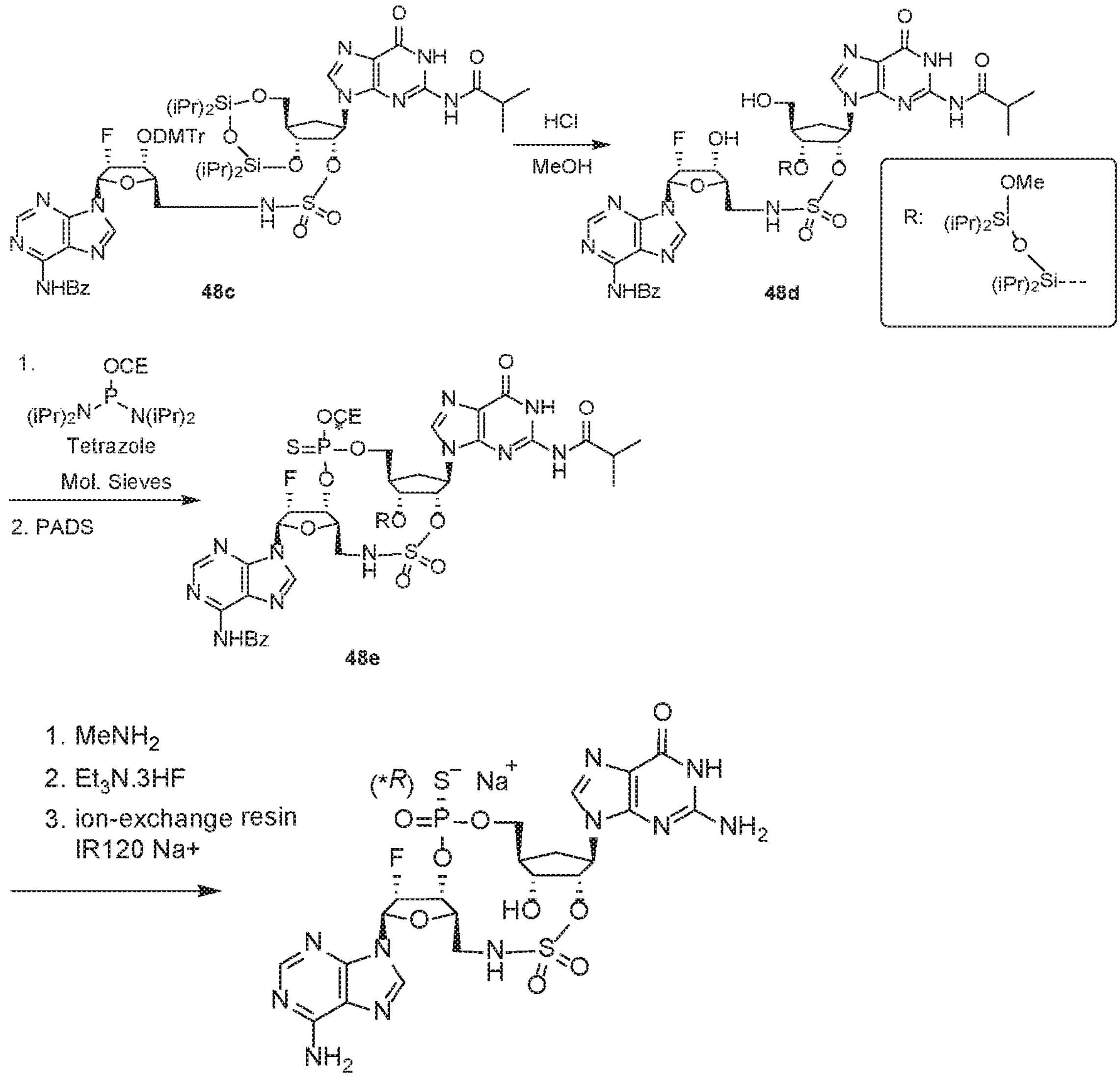


**Compound (*R) 43A, sodium salt**

Step 1: Preparation of compound **48b**

$TIPDSCl_2$ (765 μL, 2.39 mmol) was added to a solution of compound **48a** [CAS 1834500-50-3] (0.6 g, 1.71 mmol) in pyridine (25 mL). The reaction mixture was stirred for 2 h after which it was poured into saturated aqueous $NaHCO_3$ and extracted with EtOAc. The combined organic layers were dried with anhydrous $Na_2SO_4$ and concentrated under reduced pressure. The residue product was purified by silica column chromatography (gradient elution: 0 - 100% EtOAc in petroleum ether followed by 0 - 10% MeOH in EtOAc) to give compound **48b** as a white solid (550 mg, yield: 54%).

$^1H$ NMR (400 MHz, DMSO-$d_6$) δ ppm 12.06 (br s, 1 H), 11.56 (br s, 1 H), 8.05 (s, 1 H), 4.85 (d, *J*=7.3 Hz, 1 H), 4.51 - 4.64 (m, 1 H), 4.29 (q, *J*=7.2 Hz, 1 H), 4.14 - 4.23 (m, 1 H), 3.90 (dd, *J*=11.5, 3.0 Hz, 1 H), 3.76 (dd, *J*=11.6, 8.1 Hz, 1 H), 2.78 (spt, *J*=6.7 Hz, 1 H), 2.10 - 2.29 (m, 2 H), 1.42 - 1.58 (m, 1 H), 1.13 (d, *J*=7.0 Hz, 6 H), 0.99 - 1.10 (m, 28 H); ESI-MS: *m/z* 594.4 $[M+H]^+$.

Step 2: Preparation of compound **48c**

Activated molecular sieves were added to a solution of compound **48b** (350 mg, 0.59 mmol) and sulfamate **17a** (745 mg, 0.85 mmol) in dry THF (20 mL), the resulting mixture was stirred at room temperature for 2 h under nitrogen. Next, a solution of DMAP (347 mg, 2.84 mmol) in THF (5 mL), pre-dried by stirring in the presence of activated molecular sieves, was added to initiate the reaction. The reaction mixture was stirred at 50 °C overnight. The molecular sieves were removed by filtration and the filtrate washed with saturated aqueous $NaHCO_3$. The aqueous phase was extracted with DCM. The combined organic layers were dried with $Na_2SO_4$, filtered and concentrated under reduced pressure.

Compound **48c** (401 mg, yield: 51%) was obtained as a yellow solid after two rounds of purification by silica column chromatography (round 1: gradient elution: 0 - 2% MeOH in DCM, round 2: 0 - 100% EtOAc in petroleum ether followed by 0 - 10% MeOH in EtOAc).

$^1$H NMR (400 MHz, DMSO-$d_6$) δ ppm 12.00 (s, 1 H), 11.44 (s, 1 H), 11.25 (s, 1 H), 8.59 (s, 1 H), 8.51 (s, 1 H), 8.20 (br t, *J*=5.4 Hz, 1 H), 8.03 (d, *J*=7.3 Hz, 2 H), 8.00 (s, 1 H), 7.62 - 7.70 (m, 1 H), 7.55 (t, *J*=7.5 Hz, 2 H), 7.46 (d, *J*=7.5 Hz, 2 H), 7.28 - 7.39 (m, 6 H), 7.18 - 7.26 (m, 1 H), 6.84 - 6.92 (m, 4 H), 6.30 (dd, *J*=17.9, 3.0 Hz, 1 H), 4.98 - 5.04 (m, 1 H), 4.84 - 4.95 (m, 1 H), 4.56 - 4.72 (m, 1 H), 4.55 (br s, 1 H), 4.39 (t, *J*=5.5 Hz, 1 H), 3.73 - 3.93 (m, 3 H), 3.71 (s, 3 H), 3.70 (s, 3 H), 2.88 - 3.00 (m, 1 H), 2.73 (spt, *J*=6.8 Hz, 1 H), 2.57 - 2.68 (m, 1 H), 2.20 - 2.34 (m, 2 H), 1.54 - 1.66 (m, 1 H), 1.09 (d, *J*=6.8 Hz, 6 H), 0.86 - 1.07 (m, 28 H); ESI-MS: *m/z* 1329.8 [ M+2H]/2$^+$

Step 3: Preparation of compound **48d**

A solution of compound **48c** (1.3 g, 0.98 mmol) in dry MeOH (55 mL, dried on molecular sieves) was treated with HCl (0.19 mL of 2 M in $Et_2O$, 13.7 mmol) for 4 h. The reaction mixture was quenched by the addition of saturated aqueous $NaHCO_3$ followed by extraction with DCM The organic phase was dried with anhydrous $MgSO_4$, filtered and concentrated under reduced pressure. The crude product was purified by silica column chromatography (gradient elution: 0 - 5% MeOH in DCM) to give compound **48d** (670 mg, yield: 65%).

$^1$H NMR (400 MHz, DMSO-$d_6$) δ ppm 1.01 - 1.08 (m, 28 H), 1.12 (d, *J*=6.8 Hz, 3 H), 1.67 - 1.75 (m, 1 H), 2.20 - 2.32 (m, 1 H), 2.43 - 2.54 (m, 1 H), 2.62 (br d, *J*=14.3 Hz, 1 H), 2.79 (spt, *J*=6.9 Hz, 1 H), 2.85 - 2.96 (m, 1 H), 3.51 (s, 3 H), 3.52 - 3.57 (m, 2 H), 3.75 - 3.83 (m, 1 H), 4.39 - 4.48 (m, 1 H), 4.50 (d, *J*=4.2 Hz, 1 H), 5.02 (q, *J*=9.6 Hz, 1 H), 5.09 (t, *J*=4.5 Hz, 1 H), 5.25 (dd, *J*=9.8, 4.1 Hz, 1 H), 5.52 (ddd, *J*=52.4, 4.4, 2.6 Hz, 1 H), 5.68 (br d, *J*=5.9 Hz, 1 H), 6.30 (dd, *J*=18.5, 2.6 Hz, 1 H), 7.56 (t, *J*=7.7 Hz, 2 H), 7.61 - 7.69 (m, 1 H), 8.02 - 8.07 (m, 2 H), 8.11 (br s, 1 H), 8.14 (s, 1 H), 8.57 (s, 1 H), 8.71 (s, 1 H), 11.22 (br s, 1 H), 11.33 (br s, 1 H), 12.09 (br s, 1 H); ESI-MS: *m/z* 1060.7 [M+H]$^+$

Step 4: Preparation of compound **48e**

A solution of compound **48d** (640 mg, 0.60 mmol) and 1*H*-tetrazole (7.05 mL of a 3 - 4% in MeCN, dried on 3Å molecular sieves before use) in dry THF (47.5 mL, dried on 3Å molecular sieves before use) was treated with activated 3Å molecular sieves for 2 h under $N_2$ after which 2-cyanoethyl-*N,N,N',N'*-tetra(isopropyl)phosphorodiamidite (200 mg, 0.66 mmol) was added in one portion. The reaction mixture was shaken for 3 h. An additional amount of 2-cyanoethyl-*N,N,N',N'*-tetra(isopropyl)phosphorodiamidite (73 mg, 0.24 mmol) was added and shaking was continued overnight. Pyridine (19.5 mL) and PADS (456 mg, 1.53 mmol) were added, the reaction mixture was shaken for an extra hour. The molecular sieves were removed by filtration and rinsed with dichloromethane. The filtrate was washed with brine and concentrated under reduced pressure. The crude product was purified by column chromatography over silica gel (gradient elution: 0 - 15% MeOH in DCM) give compound **48e** (250 mg, yield: 35%). ESI-MS: *m/z* 1138.7 [M+H]$^+$.

Step 5: Preparation of **compound (****R***) 43A, sodium salt**

Compound **48e** (250 mg, 0.21 mmol) was stirred in a 33% methylamine solution in ethanol (10 mL) at room temperature until complete conversion (ca. 4 h), after which the reaction solution was concentrated under reduced pressure and triturated in MeCN. The precipitate was dissolved in a mixture of pyridine (1.7 mL) and $Et_3N$ (1.5 mL). Triethylamine trihydrofluoride (141 μL, 0.84 mmol) was added, the resulting reaction mixture was stirred at room temperature until complete conversion (note: precipitation of desired product observed). Isopropoxytrimethylsilane (596 μL, 3.36 mmol) was added and stirring was overnight. The residue, obtained after concentration under reduced pressure, was triturated in MeCN, the obtained precipitate was further purified by preparative reversed phase HPLC (Stationary phase: XBridge C18 OBD, 10μm, 150 × 50 mm, Mobile phase: 0.25% ammonia bicarbonate (A) - MeCN (B); gradient elution) to **compound (****R***) 43A, ammonium salt** as a single P-epimer. Final conversion into the sodium salt was done by elution of an aqueous solution over a column packed with a cationic Na ion-exchange resin affording **compound (****R***) 43A, sodium salt** as a white fluffy solid (56 mg, yield: 37.5%). $^1$H NMR (600 MHz, DMSO-$d_6$, 61 °C) δ ppm 10.24 (br s, 1 H), 8.34 (br s, 1 H), 8.07 (s, 1 H), 7.87 (s, 1 H), 7.26 (s, 3 H), 6.34 (dd, *J*=18.6, 2.2 Hz, 1 H), 6.23 (br s, 2 H), 5.69 (d, *J*=51.8 Hz, 1 H), 5.44 - 5.54 (m, 1 H), 5.39 (dddd, *J*=17.3, 9.8, 7.2, 4.7 Hz, 1 H), 5.18 (br s, 1 H), 5.06 (q, *J*=9.8 Hz, 1 H), 4.30 - 4.38 (m, 1 H), 4.29 (br s, 1 H), 3.99 - 4.10 (m, 1 H), 3.86 (dt, *J*=11.0, 4.0 Hz, 1 H), 3.63 (dd, *J*=13.2, 4.5 Hz, 1 H), 3.36 (dd, *J*=13.6, 3.7 Hz, 1 H), 2.45 (dt, *J*=13.5, 10.2 Hz, 1 H), 2.20 (m, *J*=9.1, 4.5 Hz, 1 H), 1.80 - 1.91 (m, 1 H); $^{31}$P NMR (162 MHz, DMSO-$d_6$) δ ppm 51.63 (s, 1 P); ESI-MS: *m/z* 690.1 [M+H]$^+$.

Example 49

## Compound 24

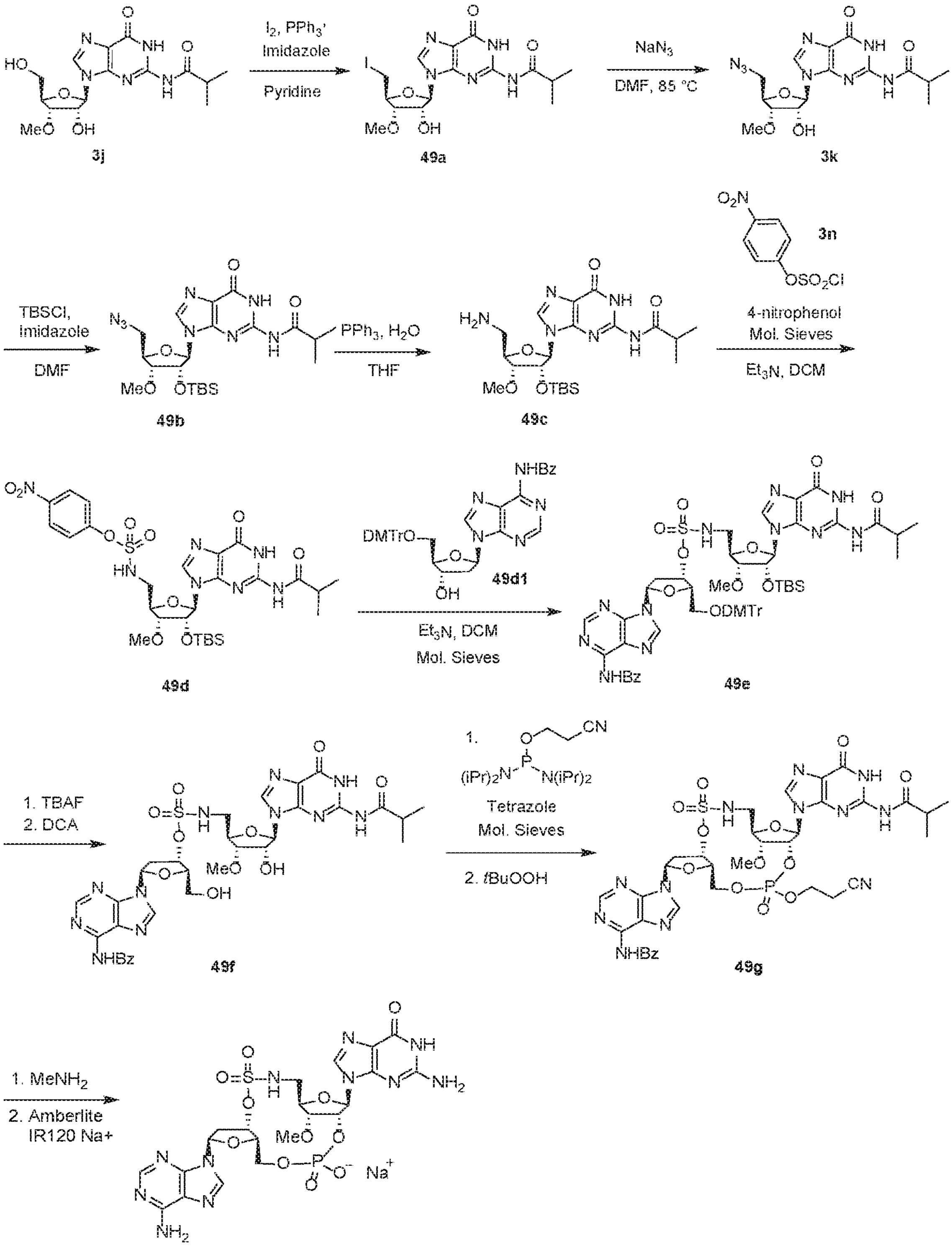


3j, 49a, 3k, 3n, 49b, 49c, 49d, 49d1, 49e, 49f, 49g

## Compound 24, sodium salt

Step 1: Preparation of compound **49a**

To a solution of *N*2-isobutyryl-3'-O-methyl-guanosine **3j** (50 g, 136 mmol) in anhydrous pyridine (500 mL), were

added imidazole (18.52 g, 272 mmol), triphenylphosphine (53.51 g, 204 mmol), and iodine (51.78 g, 204 mmol). The reaction mixture was stirred under N2 at 0~5°C for 3 h. The solution was then evaporated to dryness and the residue was dissolved in DCM (500 mL). Saturated aqueous $NaHCO_3$ was added and the mixture was stirred for 30 min. The precipitate was filtered and dried under vacuum to afford compound **49a** as a white powder (56 g, yield: 86%).

$^1$H NMR (400 MHz, DMSO-$d_6$) δ ppm 11.94 (br s, 2 H), 8.24 (s, 1 H), 5.82 (d, *J* = 6.5 Hz, 1 H), 5.69 (br s, 1 H), 4.84 (dd, *J* = 5.0, 6.5 Hz, 1 H), 4.10 (m, 1 H), 3.83 (dd, *J* = 3.0, 4.5 Hz, 1 H), 3.58 (dd, *J* = 7.0, 10.5 Hz, 1 H), 3.47 (dd, *J* = 7.0, 10.5 Hz, 1 H), 3.46 (s, 3 H), 2.74 (spt, *J*= 6.5 Hz, 1 H), 1.12 (d, *J* = 6.5 Hz, 6 H); ESI-MS: *m/z* 477.8 [M+H]$^+$.

Step 2: Preparation of compound **3k**

Sodium azide (22.48 g, 345.8 mmol) was added to a solution of compound **49a** (55 g, 115.2 mmol) in anhydrous DMF (100 mL) and stirred at 85 °C for 4 h under $N_2$. The reaction solution was then cooled down to 25 °C, poured into water (2 L), and stirred for 30 min. The precipitate was collected by filtration and dried to give compound **3k** (41 g, yield: 91%).

$^1$H NMR (400 MHz, DMSO-$d_6$) δ ppm 12.09 (br s, 1 H), 11.63 (br s, 1 H), 8.26 (s, 1 H), 5.84 (d, J= 6.0 Hz, 1 H), 5.68 (d, *J* = 5.0 Hz, 1 H), 4.76 (m, 1 H), 4.14 (m, 1 H), 3.84 (t, *J* = 4.5 Hz, 1 H), 3.69 (dd, *J* = 8.0, 13.0 Hz, 1 H), 3.57 (dd, *J* = 4.0, 13.0 Hz, 1 H), 3.43 (s, 3H), 2.77 (spt, J= 7.0 Hz, 1 H), 1.13 (d, J= 7.0 Hz, 6 H); ESI-MS: *m/z* 393.0 [M+H]$^+$.

Step 3: Preparation of compound **49b**

A mixture of compound **3k** (40.00 g, 101.94 mmol), imidazole (10.41 g, 152.9 mmol) and TBSCl (18.44 g, 122.4 mmol) in anhydrous DMF (100 mL) was stirred at 25°C for 17 h under $N_2$. The reaction solution was evaporated to dryness at 55 °C under reduced pressure. The residue was dissolved in EtOAc and washed with water. The organic phase was dried with $Na_2SO_4$, filtered, and the filtrate was evaporated. The crude product was purified by silica column chromatography (gradient elution: 20 - 40% EtOAc in heptane) to give compound **49b** (45 g, yield: 87%).

$^1$H NMR (500MHz, DMSO-d6) δ ppm 12.08 (br s, 1 H), 11.59 (br s, 1 H), 8.29 (s, 1 H), 5.87 (d, *J* = 7.5 Hz, 1 H), 4.86 (dd, *J* = 4.5, 7.5 Hz, 1 H), 4.17 (m, 1 H), 3.84 - 3.79 (m, 2 H), 3.56 (dd, *J* = 4.5, 13.0 Hz, 1 H), 3.45 (s, 3 H), 2.77 (spt, *J* = 7.0 Hz 1 H), 1.12 (d, *J* = 6.5 Hz, 6 H), 0.74 (s, 9 H), -0.02 (s, 3 H), -0.23 (s, 3 H); ESI-MS: *m/z* 507.6 [M+H]$^+$.

Step 4: Preparation of compound **49c**

Triphenylphosphine (31.06 g, 118.4 mmol) was added to a solution of compound **49b** (40 g, 78.95 mmol) in THF (400 mL). The reaction mixture was stirred at 25 °C for 10 min after which water (5.68 g, 315.2 mmol) was added dropwise over 30 min. TsOH (27.19 g, 157.9 mmol) was added and stirring was continued for 10 min. The reaction solution was evaporated under reduced pressure. The residue was dissolved in DCM (400 mL) and washed with water (3 × 400 mL). The organic phase was dried with $Na_2SO_4$, filtered and evaporated. The crude material was purified by silica gel column chromatography (gradient elution: 1% - 5% MeOH in DCM) to give compound **49c** as the tosylate salt (35 g, yield: 68%).

$^1$H NMR (400 MHz, DMSO-d6) δ ppm 9.38 (br s, 5 H), 8.33 (s, 1 H), 7.49 (d, *J* = 8.5 Hz, 2H), 7.12 (d, *J* = 8.5 Hz, 2 H), 5.88 (d, *J* = 7.0 Hz, 1H), 4.85 (dd, *J* = 5.0 and 7.0 Hz, 1 H), 4.20 (m, 1 H), 3.92 (dd, *J* = 2.0 and 4.5 Hz, 1 H), 3.46 (s, 3H), 3.31-3.17 (m, 2 H), 2.77 (spt, *J* = 7.0 Hz, 1H), 2.29 (s, 3 H), 1.12 (d, *J* = 7.0 Hz, 6 H), 0.74 (s, 9H), -0.01 (s, 3H), -0.25 (s, 3 H); ESI-MS: *m/z* 481.6 [M+H]$^+$.

Step 5: Preparation of compound **49d**

Compound **49c** (TsOH salt, 34 g, 52.1 mmol), 4-nitrophenol (72.48 g, 521 mmol) and $Et_3N$ (63.21 g, 625 mmol) were dissolved in DCM (800 ml) and cooled to -78 °C. A solution of 4-nitrophenyl chlorosulfate (24.76 g, 104.2 mmol) in DCM (50 mL) was added dropwise over 30 min. The reaction mixture was warmed to 0 °C, diluted with DCM (800 mL), and washed with 1 M aqueous $NaH_2PO_4$ (3 × 800mL). The organic phase was dried with $Na_2SO_4$, filtered, and concentrated under reduced pressure. The crude material was purified by silica gel column chromatography (gradient elution: 1 - 5% DCM in MTBE) to give compound **49d** (19 g, yield: 53.5%).

$^1$H NMR (500MHz, CDC13) δ ppm 9.49 (br s, 1 H), 8.32 (d, *J* = 9.0 Hz, 2 H), 7.63 (s, 1 H), 7.47 (d, *J* = 9.0 Hz, 2 H), 5.64 (d, *J* = 8.0 Hz, 1 H), 4.95 (dd, *J* = 5.5, 8.0 Hz, 1 H), 4.38 (s, 1 H), 3.84 (d, *J* = 5.5 Hz, 1 H), 3.64 (s, 2 H), 3.55 (s, 3 H), 2.51 (spt, *J* = 7.0 Hz, 1 H), 1.18 (d, *J* = 7.0 Hz, 3 H), 1.12 (d, *J* = 7.0 Hz, 1 H), 0.77 (s, 9 H), -0.10 (s, 3H), -0.31 (s, 3H); ESI-MS: *m/z* 682.7 [M+H]$^+$.

Step 6: Preparation of compound **49e**

Compound **49d** (18 g, 26.4 mmol), *N*6-benzoyl-5'-*O*-(4,4'-dimethoxytrityl)-2'-deoxyadenosine (34.56 g, 52.55 mmol), and $Et_3N$ (13.32 g, 131.6 mmol) were separately dissolved in dry DCM (3 × 72 mL). Each solution was dried on 3Å activated molecular sieves by stirring under $N_2$ for 4 h. Next, the $Et_3N$ solution was added to the N6-benzoyl-5'-O-(4,4'-dimethoxytrityl)-2'-deoxyadenosine (**49d1**) solution, followed by the addition of the sulfamate **49d** solution (transferred via a syringe). The resulting reaction mixture was stirred at 25 °C for 17 h. The molecular sieves were removed by filtration, the filtrate was evaporated under reduced pressure. The crude product 49e was used as such in the next step.

Step 7: Preparation of compound **49f**

The above crude product **49e** was dissolved in THF (190 mL) and treated with TBAF (14.57 g, 55.73 mmol) at 25 °C for 16 h. The reaction mixture was evaporated to dryness under reduced pressure. The residue was dissolved in DCM, washed with 2% aqueous acetic acid (3 × 190 mL) and evaporated. The crude product was dissolved in DCM (190 mL) and treated with DCA (17.97 g, 139.4 mmol) and water (2.5 mL, 138.7 mmol). The reaction mixture was stirred at 25 °C until complete deprotection and then washed with 5% aqueous $NaHCO_3$. The residue obtained after evaporation under reduced pressure was purified by reverse phase chromatograph using MeOH and water as eluent to give compound **49f** (4 g) as an off-white solid.

$^1$H-NMR (500MHz, DMSO-d6) δ (ppm) 12.07 (s, 1H), 11.59 (s, 1H), 11.19 (s, 1H), 8.75 (s, 1H), 8.68 (s, 1H), 8.47 (t, J= 5.5 Hz, 1H), 8.26 (s, 1H), 8.05 (d, J= 7.5 Hz, 2H), 7.65 (t, *J* = 7.0 Hz, 1H), 7.55 (t, *J* = 7.5 Hz, 2H), 6.51 (t, *J* = 6.5 Hz, 1H), 5.83 (d, *J* = 6.0 Hz, 1H), 5.64 (d, *J* = 6.0 Hz, 1H), 5.27-5.23 (m, 2H), 4.67 (q, J= 5.5 Hz, 1H), 4.24 (m, 1H), 4.08 (m, 1H), 3.87 (m, 1H), 3.60 (m, 2H), 3.44 (s, 3H), 3.44-3.28 (m, 2H), 3.10 (m, 1H), 2.76 (m, 2H), 1.12 (d, *J* = 3.0 Hz, 6H); ESI-MS: *m/z* 784.1 $[M+H]^+$.

Step 8: Preparation of compound **49g**

A solution of compound **49f** (387 mg, 0.49 mmol) in dry MeCN / THF (1:1, 24 mL, dried on 3Å molecular sieves before use) was treated with 3Å molecular sieves for 2 h after which it was transferred to a reaction flask. 1*H*-tetrazole (4.32 mL of a 3 - 4% solution in MeCN, dried on 3Å molecular sieves before use) was added, followed by the addition of 2-cyanoethyl-*N*,*N*,*N'*,*N'*-tetra(isopropyl)phosphorodiamidite (172 μL, 0.54 mmol). The resulting reaction mixture was stirred for 2 h after which an additional amount of 2-cyanoethyl-*N*,*N*,*N'*,*N'*-tetra(isopropyl)phosphorodiamidite (78 μL, 0.25 mmol) was added, stirring was continued overnight *t*BuOOH (161 μL of 5.5 M solution in decane, 0.89 mmol) was added, the reaction solution was stirred for an extra 90 min after which it was filtered and concentrated under reduced pressure. Purification was done by silica column chromatography (gradient elution: 0 - 7.5% MeOH in DCM) to give compound **49g** (158 mg, yield: 31%). ESI-MS: *m/z* 899.5 $[M+H]^+$.

Step 9: Preparation of **compound 24, sodium salt**

Compound **49g** (158 mg, 0.155 mmol) was stirred in a 33% methylamine solution in ethanol (2 mL) at room temperature until complete conversion (ca. 2 h). The reaction mixture was concentrated under reduced pressure. The resulting crude product was triturated in MeCN followed by preparative reversed phase HPLC purification (Stationary phase: XBridge C18 OBD, 10 μm, 150 × 50 mm; Mobile phase: aqueous 0.25% ammonia bicarbonate (A) - MeOH (B); gradient elution). Final conversion into the sodium salt was done by elution of an aqueous solution over a column packed with a cationic Na ion-exchange resin to give **compound 24, sodium salt** as a white fluffy solid after lyophilization.

$^1$H NMR (400 MHz, DMSO-$d_6$, 81 °C) δ ppm 10.19 (br s, 1 H), 8.24 (s, 1 H), 8.17 (s, 1 H), 8.19 (br s, 1 H), 6.92 (s, 1 H), 6.33 (dd, *J*=9.4, 5.6 Hz, 1 H), 6.19 (br s, 2 H), 5.74 (d, *J*=8.8 Hz, 1 H), 5.36 - 5.60 (m, 2 H), 4.05 - 4.24 (m, 3 H), 3.82 (d, *J*=4.4 Hz, 1 H), 3.53 - 3.63 (m, 1 H), 3.50 (s, 3 H), 3.26 (br s, 2 H), 3.01 - 3.11 (m, 1 H, signal under water peak), 2.87 (dd, *J*=14.1, 5.5 Hz, 1 H);
$^{31}$P NMR (126 MHz, DMSO-$d_6$) δ ppm 0.84 (s, 1 P); ESI-MS: *m/z* 672.3 $[M+H]^+$.

Example 50

## Compound (*R) 19A and compound (*S) 19B

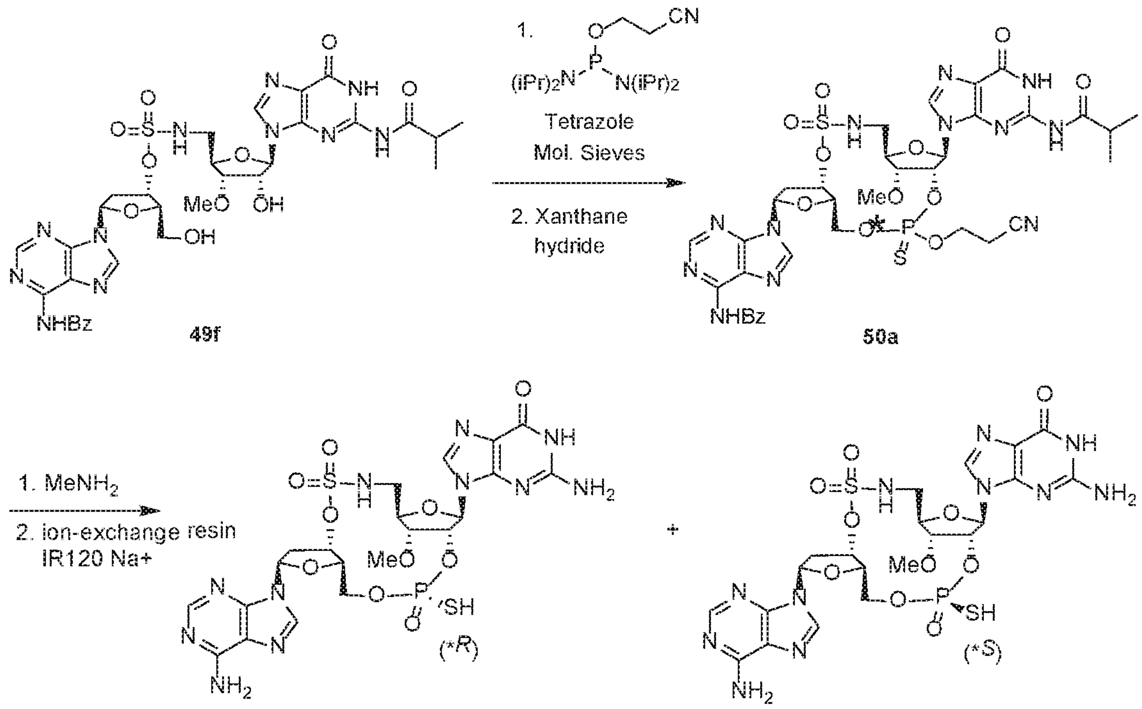


49f

50a

(*R)

(*S)

**Compound (*R) 19A, sodium salt Compound (*S) 19B, sodium salt**

Step 1: Preparation of compound **50a**

A solution of **49f** (1 g, 1.3 mmol) and 1*H*-tetrazole (0.2 g, 3.1 mmol) in dry DCM (40 mL) was pre-treated with 3Å molecular sieves for 3 h before the addition of 2-cyanoethyl-*N,N,N',N'*-tetra(isopropyl)phosphorodiamidite (0.4 g, 1.3 mmol) in one portion. The resulting reaction mixture was stirred at 35 °C for 1 h. Xanthane hydride (0.22 g, 1.43 mmol) was added and stirring was continued for 30 min. The reaction solution was diluted with DCM and washed with brine, 5% aqueous $NaHCO_3$ and water. The organic phase was dried with $Na_2SO_4$ and filtered. The filtrate was evaporated to dryness under reduced pressure. The crude was purified by column chromatography on silica gel (gradient elution: 5 - 10% MeOH in DCM) to give 600 mg of a mixture of compound 50a and its cyanoethyl-deprotection product. ESI-MS: *m/z* 916.0 $[M+H]^+$.

Step 2: Preparation of **compound (*R) 19A, sodium salt** and **compound (*S) 19B, sodium salt**

The above product **50a** was stirred in a 33% methylamine solution in ethanol (13 mL) at room temperature until complete conversion. The reaction mixture was concentrated under reduced pressure. The crude product was purified by reversed phase C18 column using MeOH and water as mobile phase to give the P-epimeric mixture (60 mg, yield: 7% from compound 50a). Both isomers were separated by preparative reversed phase HPLC (Stationary phase: XBridge C18 OBD, 10 μm, 250 × 50 mm; Mobile phase: aqueous 0.25% ammonia bicarbonate (A) - MeOH (B); gradient elution) to give **compound (*R) 19A, ammonium salt** as the first eluting isomer and **compound (*S) 19B, ammonium salt** as the second eluting isomer. Final conversion into the sodium salt was done by elution of an aqueous solution over a column packed with a cationic Na ion-exchange resin.

**Compound (*R) 19A, sodium salt:**

$^1H$ NMR (600 MHz, DMSO-$d_6$, 81 °C) δ ppm 9.17 (br s, 1 H), 8.24 (s, 1 H), 8.16 (s, 1 H), 7.96 (br s, 1 H), 6.97 (s, 2 H), 6.38 (dd, *J*=9.1, 5.4 Hz, 1 H), 6.20 (br s, 2 H), 5.77 (d, *J*=8.9 Hz, 2 H), 5.51 (br s, 1 H), 4.44 - 4.51 (m, 1 H), 4.26 (br s, 1 H), 4.18 (br s, 1 H), 3.78 (d, *J*=4.6 Hz, 1 H), 3.56 - 3.62 (m, 1 H), 3.53 (s, 3 H), 3.20 - 3.50 (m, 3 H), 2.90 (dd, *J*=14.5, 5.4 Hz, 1 H); ESI-MS: *m/z* 687.1 $[M+H]^+$.

**Compound (*S) 19B, sodium salt:**

$^1$H NMR (600 MHz, DMSO-$d_6$, 81 °C) δ ppm 10.03 (br s, 1 H), 8.29 (s, 1 H), 8.18 (s, 1 H), 8.00 (br s, 1 H), 6.97 (br s, 2 H), 6.38 (dd, *J*=9.4, 5.4 Hz, 1 H), 6.26 (br s, 2 H), 5.78 (d, *J*=8.9 Hz, 1 H), 5.50 (br s, 1 H), 5.48 (br s, 1 H), 4.22 - 4.34 (m, 1 H), 4.15 (br s, 1 H), 4.09 (q, *J*=11.2 Hz, 1 H), 3.85 (d, *J*=4.5 Hz, 1 H), 3.60 - 3.71 (m, 1 H), 3.55 (s, 3 H), 3.41 - 3.48 (m, 1 H), 3.34 - 3.40 (m, 1 H), 3.23 - 3.34 (m, 1 H), 2.87 (dd, *J*=14.5, 5.4 Hz, 1 H); $^{31}$P NMR (162 MHz, DNEO-$d_6$) δ ppm 55.50 (s, 1 P); ESI-MS: *m*/*z* 678.1.1 [M+H]$^+$.

Example 51

Compound 22

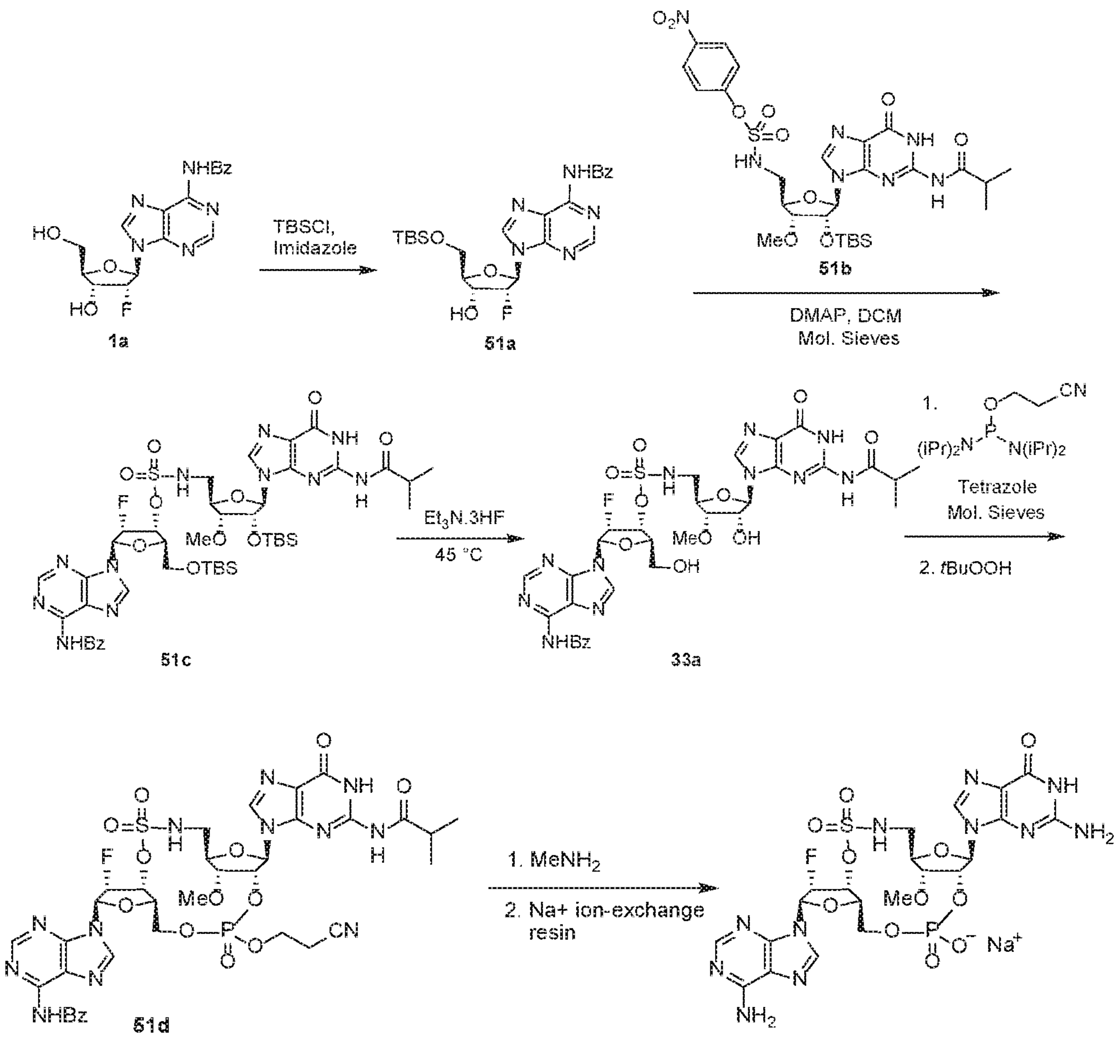


Compound 22, sodium salt

Step 1: Preparation of compound **51a**

Imidazole (3.94 g, 57 mmol) and TBSCl (7.18 g, 470 mmol) were added to a solution of *N*6-benzoyl-2'-deoxy-2'-fluoroadenosine **1a** [CAS 136834-20-3] (12.7 g, 34.0 mmol) in DMF (105 mL) at 0 °C. The reaction mixture was stirred at room temperature until complete conversion (2 h). The reaction mixture was diluted with EtOAc and washed with saturated aqueous $NH_4Cl$ and water. The organic phase was dried with anhydrous $Na_2SO_4$, filtered and concentrated

under reduced pressure. Purification was done by column chromatography over silica gel (gradient elution: 0 - 1 % MeOH in DCM) to give compound **51a** as white solid (12 g, yield: 71%). $^{1}$H NMR (500 MHz DMSO-$d_6$) δ ppm - 0.04 (s, 3 H), -0.02 (s , 3 H), 0.82 (s, 9 H), 3.81 (dd, *J* =11.7, 4.1 Hz, 1 H), 3.99 (m, 2 H), 4.62 (ddd, *J*=21.7, 11.7, 6.9 Hz, 1 H), 5.54 (dq, *J*=52.8, 2.0 Hz, 1 H), 5.77 (m, 1 H), 6.38 (dd, *J*=18.6, 1.4 Hz, 1 H), 7.55 (t, *J*=7.9 Hz, 2 H), 7.65 (t, *J*=7.2 Hz, 1 H), 8.04 (d, *J*=6.9 Hz, 2 H), 8.57 (s, 1 H), 8.75 (s 1 H); ESI-MS: *m/z* 488.0 $[M+H]^+$.

Step 2: Preparation of compound **51c**

A reaction flask was charged with DMAP (2.03 g, 16.6 mmol), dry DCM (40 mL) and activated 3Å molecular sieves. The resulting mixture was stirred at room temperature for at least 2 h under inert atmosphere. Simultaneously, a solution of compound **51a** (1.6 g, 3.3 mmol) and a solution of sulfamate **51b** (2.5 g, 3.66 mmol) each in dry DCM (2 × 40 mL), were dried on activated 3Å molecular sieves (ca. 2 h). Both solutions (compound **51a** and sulfamate **51b** respectively) were successively transferred to the reaction flask. The resulting reaction mixture was stirred for 36 h. The molecular sieves were removed by filtration and thoroughly rinsed with dichloromethane. The filtrate was washed with saturated aqueous $NaHCO_3$, brine and saturated aqueous $NH_4Cl$, dried over $Na_2SO_4$, filtered, and concentrated under reduced pressure. The residue was purified by silica column chromatography (gradient elution: 0 - 1% MeOH in DCM) to give compound **51c** as an off-white foam (1.8 g, yield: 53%).

$^{1}$H NMR (500 MHz DMSO-$d_6$) δ ppm -0.25 (s, 3 H), -0.050 (s, 3 H), -0.015 (d, *J*=2.8 Hz, 6 H), 0.73 (s, 9 H), 0.79 (s, 9 H), 1.11 (dd, *J*=6.9, 1.4 Hz, 6 H), 2.75 (m, 1 H), 3.40 (q, *J*=6.9 Hz, 1 H), 3.45 (s, 3H), 3.48 (q, *J*=6.9 Hz, 1 H), 3.85 (dd, *J*=11.7, 3.4 Hz, 2 H), 3.98 (dd, *J*=12.4, 2.1 Hz, 1 H), 4.13 (m, 1 H), 4.33 (t, *J*=3.4 Hz, 1 H), 4.71 (dd, *J*=6.9, 4.8 Hz, 1 H), 5.49 (dq, *J*=17.6, 3.9 Hz, 1 H), 5.86 (d, *J*=6.9 Hz, 1 H), 6.51 (dd, *J*=18.6, 2.1 Hz, 1 H), 7.56 (t, *J*=7.6 Hz, 2 H), 7.66 (t, *J*=7.2 Hz, 1 H), 8.05 (d, *J*=7.6 Hz, 2 H), 8.30 (s, 1 H), 8.59 (s, 1 H), 8.71 (t, *J*=6.2 Hz, 1 H), 8.75 (s, 1 H), 11.26 (s, 1 H), 11.65 (s, 1 H), 12.08 (s, 1 H); ESI-MS: *m/z* 1030.4 $[M+H]^+$.

Step 3: Preparation of compound **33a**

$Et_3N$ (12.1 mL, 87.37 mmol) and $Et_3N.3HF$ (2.84 mL, 17.4 mmol) were added to a solution of compound **51c** (1.8 g, 1.74 mmol) in pyridine (34 mL). The reaction mixture was stirred at 45 °C until complete conversion (ca. 5 h) and then cooled to room temperature. Isopropoxytrimethylsilane (12.4 mL, 69.9 mmol) was added and stirring was continued overnight The crude product obtained after concentration under reduced pressure was purified by silica column chromatography (gradient elution: 0 - 7% MeOH in DCM) to give compound **33a** as an off-white foam (1.2 g, yield: 86%).

$^{1}$H NMR (500 MHz DMSO-$d_6$) δ ppm 1.11 (q, *J*=3.2 Hz, 6 H), 2.75 (m, 1 H), 3.40 (s, 1 H), 3.43 (s, 3 H), 3.64 (d, *J*=12.4 Hz, 1 H), 3.80 (d, *J*=12.4 Hz, 1 H), 3.86 (t, *J*=4.5 Hz, 1 H), 4.07 (m, 1 H), 4.32 (t, *J*=3.1 Hz, 1 H), 4.67 (q, *J*=5.7 Hz, 1 H), 5.36 (td, *J*=10.7, 4.6 Hz, 2 H), 5.66 (d, *J*=6.2 Hz, 1 H), 5.88 (m, 2 H), 6.49 (dd, *J*= 16.5, 2.8 Hz, 1 H), 7.56 (t, *J*=7.9 Hz, 2 H), 7.66 (t, *J*=7.6 Hz, 1 H), 8.05 (d, *J*=7.6 Hz, 2 H), 8.25 (s, 1 H), 8.66 (s, 1 H), 8.74 (d, *J*=32.4 Hz, 2 H), 11.27 (s, 1 H), 11.62 (s, 1 H), 12.08 (s, 1 H); ESI-MS: *m/z* 802.1 $[M+H]^+$.

Step 4: Preparation of compound **51d**

A solution of compound **33a** (500 mg, 0.624 mmol) and 1*H*-tetrazole (5.46 mL of a 3 - 4% in MeCN, dried on 3Å molecular sieves before use) in dry THF (25 mL, dried on 3Å molecular sieves before use) was treated with 3Å molecular sieves for 2 h after which 2-cyanoethyl-*N*,*N*,*N'*,*N'*-tetra(isopropyl)phosphorodiamidite (206 mg, 0.686 mmol) was added in one portion. The resulting reaction mixture was shaken overnight. An additional amount of 2-cyanoethyl-*N*,*N*,*N'*,*N'*-tetra(isopropyl)phosphorodiamidite (94 mg + 38 mg, 0.312 mmol + 0.125 mmol) was added in two portions with a 5 h time interval, shaking was continued for an extra day to obtain full conversion. *t*BuOOH (227 μL of 5.5 M solution in decane, 1.25 mmol) was added and the reaction mixture was shaken for an extra hour. The molecular sieves were removed by filtration and extensively rinsed with DCM. The filtrate was concentrated and the obtained residue purified by silica column chromatography (gradient elution: 0 - 7.5% MeOH in DCM) to afford compound **51d** (110 mg, yield: 19%). ESI-MS: *m/z* 917.5 $[M+H]^+$.

Step 5: Preparation of **compound 22, sodium salt**

Compound **51d** (110 mg, 0.12 mmol) was stirred in a 33% methylamine solution in ethanol (10 mL) at room temperature until complete conversion (ca. 2 h). The reaction mixture was concentrated under reduced pressure. The resulting crude product was triturated in MeCN followed by preparative reversed phase HPLC purification (Stationary phase: XBridge C18 OBD, 5 μm, 250 × 30 mm; Mobile phase: aqueous 0.25% ammonia bicarbonate (A) - MeCN (B); gradient elution) to give **compound 22, ammonium salt.** Conversion into the sodium salt was done by elution of an aqueous solution over a column packed with a cationic sodium ion-exchange resin to give **compound 22, sodium salt**

as a white fluffy solid after lyophilization (52 mg, yield: 60%).

$^1$H NMR (600 MHz, DMSO-$d_6$, 60 °C) δ ppm 10.67 (br s, 1 H), 9.13 (br s, 1 H), 8.37 (s, 1 H), 8.18 (s, 1 H), 7.99 (br s, 1 H), 7.24 (br s, 2 H), 6.33 (dd, *J*=15.9, 3.3 Hz, 1 H), 6.29 (br s, 2 H), 5.95 (br d, *J*=50.9 Hz, 1 H), 5.76 (d, *J*=8.9 Hz, 1 H), 5.59 (br s, 1 H), 5.18 - 5.25 (m, 1 H), 4.47 (br s, 1 H), 4.20 (dt, *J*=11.9, 6.1 Hz, 1 H), 4.14 (br s, 1 H), 3.94 (br d, *J*=4.4 Hz, 1 H), 3.75 - 3.81 (m, 1 H), 3.52 (s, 3 H), 3.47 - 3.55 (m, 1 H), 3.42 (s, 1 H);

$^{31}$P NMR (162 MHz, DMSO-$d_6$) δ ppm -1.28 (s, 1 P);
ESI-MS: *m/z* 690.3 [M+H]$^+$.

<u>Example 52</u>

**Compound 55**

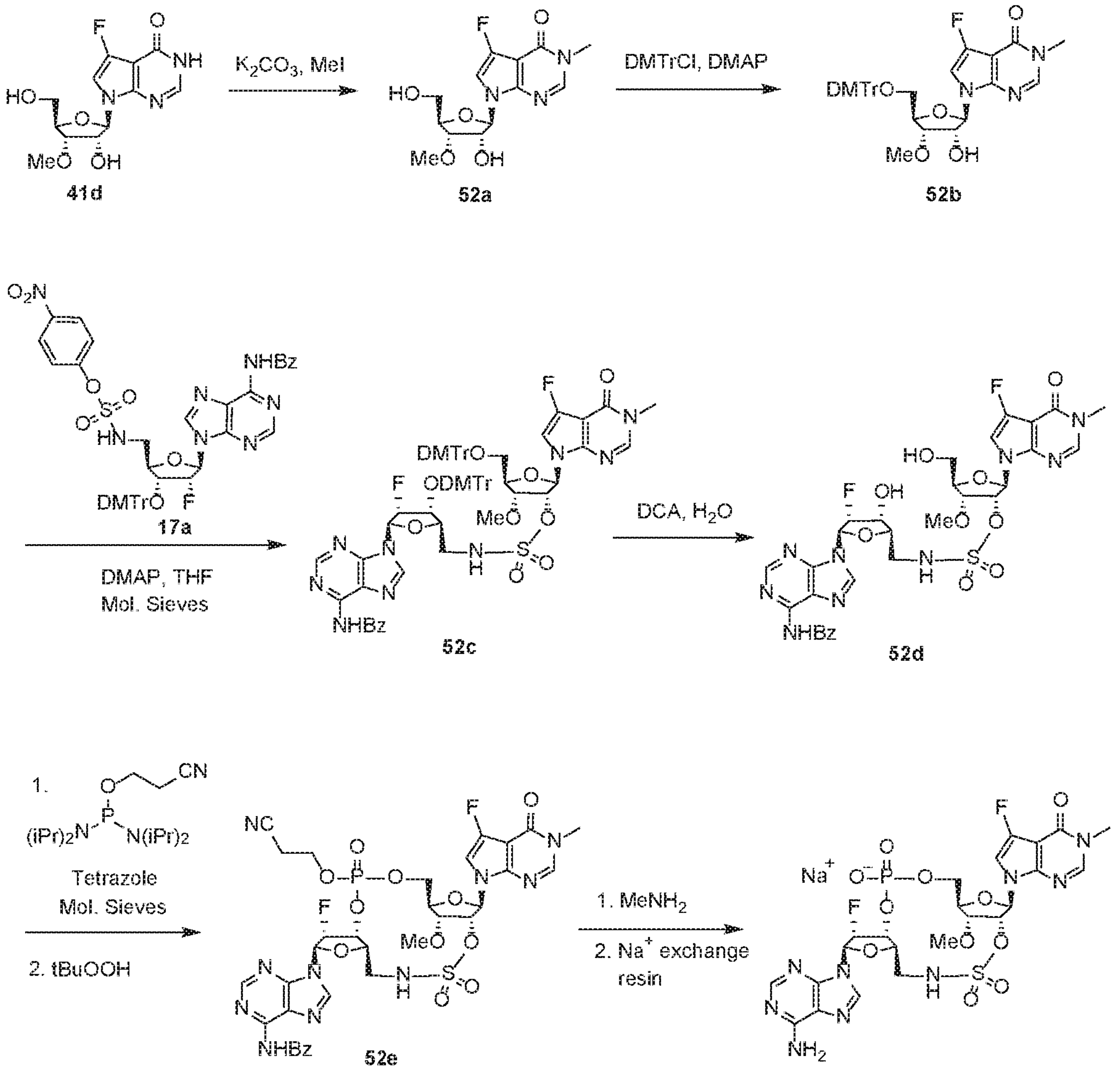


**Compound 55, sodium salt**

Step 1: preparation of compound **52a**

Potassium carbonate (0.32 g, 2.3 mmol) was added to a solution of compound **41d** (0.55 g, 1.8 mmol) in dry DMF (6 mL). The resulting suspension was cooled to 0 °C, after which iodomethane (0.15 mL, 2.3 mmol) was added.

The reaction mixture was warmed to room temperature and stirred until complete conversion (ca. 2.5 h). The residue obtained after concentration under reduced pressure was purified by column chromatography over silica gel (gradient elution: 0 - 4% MeOH in DCM) to give compound **52a** as a white powder (0.45 g, yield: 79%).

$^1$H NMR (500 MHz, DMSO-$d_6$) δ ppm 8.25 (s, 1 H), 7.36 (s, 1 H), 6.02 (d, *J*=5.5 Hz, 1 H), 5.43 (d, *J*=6.9 Hz, 1 H), 5.09 (t, *J*=5.5 Hz, 1 H), 4.39 (q, *J*=6.0 Hz, 1 H), 3.97 (q, *J*=3.4 Hz, 1 H), 3.73 - 3.80 (m, 1 H), 3.57 - 3.64 (m, 1 H), 3.50 - 3.55 (m, 1 H), 3.45 (s, 3 H), 3.40 (s, 3 H); ESI-MS: *m/z* 314 $[M+H]^+$.

Step 2: preparation of compound **52b**

A solution of compound **52a** (0.85 g, 2.7 mmol) in dry pyridine (12.7 mL), to which DMAP (0.16 g, 1.3 mmol) and DMTrCl (1.46 g, 4.3 mmol) (portionwise) were added, was stirred at room temperature for 4 h. The reaction mixture was quenched with methanol (9 mL) and concentrated under reduced pressure. The obtained residue was dissolved in EtOAc and washed with water. The organic layer was dried over anhydrous $Na_2SO_4$, filtered and concentrated under reduced pressure. Purification was done by column chromatography over silica gel (gradient elution: 0 - 0.2% MeOH in DCM) to give compound **52b** as an off-white foam (1.4 g, yield: 85%).

$^1$H NMR (500 MHz, DMSO-$d_6$) δ ppm: 8.24 (s, 1H), 7.37 (d, *J* = 7.6 Hz, 2H), 7.29 (t, *J* = 7.6 Hz, 2H), 7.22 (m, 6H), 6.87 (dd, *J* = 9.0, 2.1 Hz, 4H), 6.04 (d, *J* = 4.8 Hz, 1H), 5.53 (d, *J* = 6.2 Hz, 1H), 4.47 (q, *J* = 5.5 Hz, 1H), 4.05 (q, J= 4.4 Hz, 1H), 3.85 (t, *J* = 4.8 Hz, 1H), 3.72 (d, *J* = 9.6 Hz, 6H), 3.45 (s, 3H), 3.35 (s, 3H), 3.20 (d, *J* = 4.8 Hz, 2H); ESI-MS: *m/z* 638.2 $[M+Na]^+$.

Step 3: preparation of compound **52c**

Compound **52b** (0.9 g, 1.46 mmol), sulfamate **17a** (1.53 g, 1.75 mmol) and DMAP (0.9 g, 7.31 mmol) were each separately dissolved in dry DCM (3 × 25.0 mL). Each solution was dried with 3Å activated molecular sieves by stirring under $N_2$ for at least 2 h. To the DMAP solution, were respectively added the solution of compound **52b** in DCM and the solution of sulfamate **17a** in DCM The resulting reaction mixture was stirred for 36 h. The molecular sieves were removed by filtration and thoroughly rinsed with dichloromethane. The filtrate was washed with saturated aqueous $NaHCO_3$, brine and saturated aqueous $NH_4Cl$. The combined organic phases were dried over $Na_2SO_4$, filtered, and concentrated under reduced pressure. The residue was purified by silica column chromatography (gradient elution: 0 - 0.4% MeOH in DCM) to give compound **52c** (0.38 g, yield: 19%) and a more polar fraction (0.8 g) containing mono and di DMTr-deprotected product.

Step 4: preparation of compound **52d**

A solution of compound **52c** (0.38 g, 0.28 mmol) in DCM (10.6 mL), to which DCA (0.9 mL of 10% in DCM, 1.1 mmol) and water (30 μL, 1.4 mmol) were added, was stirred at room temperature until complete deprotection. The reaction mixture was quenched by the addition of pyridine (0.1 mL, 1.4 mmol) and some drops of methanol, and concentrated under reduced pressure. A similar reaction protocol was applied on the above 0.8 g polar fraction. The residue of both reactions were combined for purification by silica column chromatography (gradient elution: 0 - 6% MeOH in DCM) to give compound **52d** as a white powder (0.4 g, yield: 62%).

$^1$H NMR (500 MHz, DMSO-$d_6$) δ ppm 11.27 (br s, 1 H), 8.74 (s, 1 H), 8.62 (s, 1 H), 8.54 (br t, *J*=5.9 Hz, 1 H), 8.23 (s, 1 H), 8.06 (d, *J*=7.6 Hz, 2 H), 7.64 - 7.70 (m, 1 H), 7.52 - 7.59 (m, 2 H), 7.36 (br s, 1 H), 6.36 (dd, *J*=19.3, 2.1 Hz, 1 H), 6.28 (d, *J*=6.2 Hz, 1 H), 5.90 (br s, 1 H), 5.58 (br d, *J*=52.7 Hz, 1 H), 5.22 (t, *J*=5.5 Hz, 1 H), 4.52 - 4.64 (m, 1 H), 4.07 - 4.14 (m, 2 H), 3.95 - 4.01 (m, 1 H), 3.64 (dd, *J*=12.4, 3.4 Hz, 1 H), 3.56 (dd, *J*=11.7, 2.8 Hz, 1 H), 3.43 (s, 3 H), 3.38 (s, 3 H), 3.12 - 3.24 (m, 2 H); ESI-MS: *m/z* 748.5 $[M+H]^+$.

Step 5: preparation of compound **52e**

A solution of compound **52d** (100 mg, 0.134 mmol) and 1*H*-tetrazole (2.38 mL of a 0.45 M solution in MeCN, 1.14 mmol) in dry MeCN / DMF (5 mL, 4:1) mixture was treated with 4Å molecular sieves for 10 min under $N_2$, after which a solution of 2-cyanoethyl-*N*,*N*,*N'*,*N'*-tetra(isopropyl)phosphorodiamidite (80 mg, 0.27 mmol) in dry MeCN (1.0 mL) was added (note: MeCN was freshly distilled over $CaH_2$ before use). The resulting reaction mixture was stirred for 1.5 h at room temperature. Next, a solution of *t*BuOOH (126 μL of 5~6 M in decane, 0.67 mmol) was added and stirring was continued for another 30 min. The reaction mixture was diluted with DCM, filtered through a pad of Diatomaceous earth and concentrated. The residue was purified by flash column chromatography over silica gel (gradient elution: 0 - 7% MeOH in DCM) to give compound **52e.**

ESI-MS: *m/z* 863.1 $[M+H]^+$.

Step 6: preparation of **compound 55, sodium salt**

The above compound **52e** was stirred in a 30% methylamine solution in ethanol (10 mL) at room temperature for 2.5 h. The reaction mixture was concentrated under reduced pressure. The residue was dissolved in a water / MeCN (4:1) mixture, washed with DCM and concentrated. The crude product was purified by preparative reversed phase HPLC (Stationary phase: Xbridge OBD C18, 5 μm, 150 × 30 mm; Mobile phase: 10 mM aqueous ammonia bicarbonate (A) - MeCN (B); gradient elution) to give **compound 55, ammonium salt** (25 mg, yield: 26% from **52d**). Final conversion into the sodium salt was done by elution of an aqueous solution over a column packed with Dowex 50WX8 Na ion-exchange resin.

$^{1}H$ NMR (400 MHz, $D_2O$) δ 8.13 (br s, 1H), 8.00 (br d, *J*=5.6 Hz, 1H), 7.42 (br s, 1H), 7.06 (br s, 1H), 6.42 - 6.33 (m, 2H), 5.41 (br s, 1H), 5.35 - 5.16 (m, 1H), 5.05 - 4.88 (m, 1H), 4.65 (br s, 1H), 4.53 - 4.40 (m, 2H), 4.33 - 4.20 (m, 2H), 3.88 - 3.76 (m, 1H), 3.60 (s, 4H), 3.48 (s, 3H)

$^{19}F$ NMR (376MHz, $D_2O$) δ -165.090 (s, IF), -196.605 (s, IF); $^{31}P$ NMR (162MHz, $D_2O$) δ -1.899 (s, IP); ESI -MS: *m*/*z* 706.0 $[M+H]^+$.

Example 53

# Compound 27

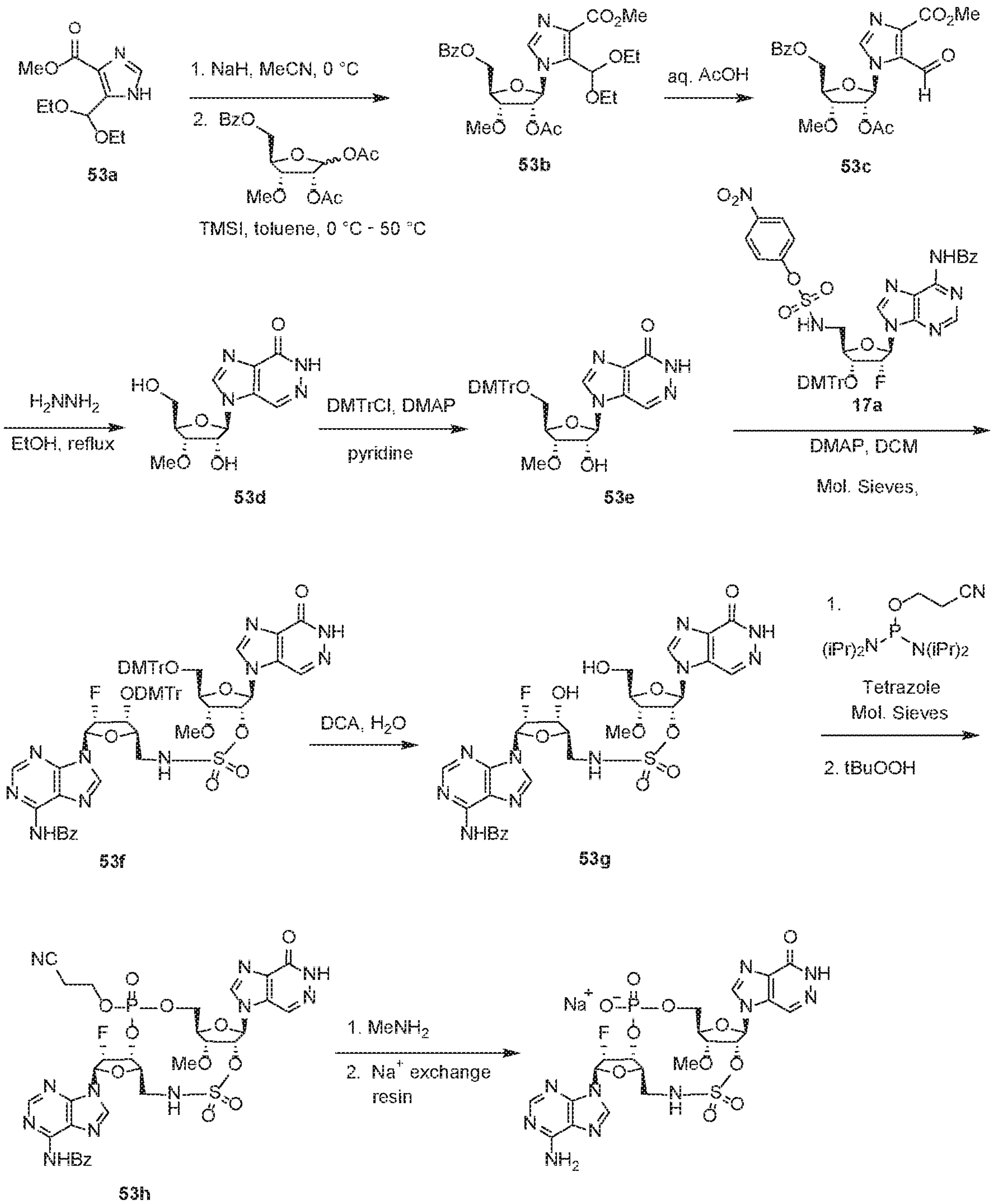


53a, 53b, 53c, 17a, 53d, 53e, 53f, 53g, 53h

**Compound 27, sodium salt**

Step 1: preparation of compound **53b**

A reaction flask charged with methyl 5-(diethoxymethyl)-1H-imidazole-4-carboxylate **53a** [CAS 85109-99-5] (3.24 g, 14.1 mmol) and dry MeCN (120 mL) was cooled in an ice-bath. Sodium hydride (1.23 g of a 55% dispersion in mineral oil, 28.5 mmol) was added, the resulting mixture was stirred at rt for 45 min.

In a separate flask, iodotrimethylsilane (3.23 mL, 22.6 mmol) was added to a solution of 1,2-di-*O*-acetyl-3-*O*-methyl-5-*O*-benzoyl-D-ribofuranose ([10300-21-7], 5 g, 14.1 mmol) in dry toluene (50 mL). The resulting solution was stirred for 20 min at rt after which it was slowly transferred to the above reaction flask cooled in an ice-bath. The resulting

reaction mixture was stirred at 50 °C until full conversion (ca. 3.5 h). After cooling to rt, EtOAc was added. The organic phase was washed with water and brine, dried with anhydrous $Na_2SO_4$, filtered, and concentrated under reduced pressure. Purification was done by column chromatography over silica (gradient elution: 0 - 40% EtOAc in hexane) to give compound **53b** as colourless gum (4.3 g, yield: 58%)

$^1H$ NMR (500 MHz DMSO-$d_6$) δ ppm 8.0 (m, 3H), 7.70 (t, *J*=7.6Hz,1H,), 7.56 (m, 2H), 6.36 (m, 1H), 6.23 (s, 1H), 5.49 (dd, *J*=4.1, 2.8 Hz, 1H), 4.63 (m, 2H), 4.26(m, 2H), 3.78 (s, 3H), 3.68 (m, 1H), 3.47 (m, 1H) 2.11 (s, 2H) 1.11 (m, 6H);
ESI-MS: *m*/*z* 521.2 $[M+H]^+$.

Step 2: preparation of compound **53c**

A solution of compound **53b** (4.3 g, 8.25 mmol) in 80% aqueous AcOH (50 mL) was stirred at rt for 16 h. Next, the reaction solution was poured into ice cold water and extracted with DCM The organic layer was washed with water, dried over anhydrous $Na_2SO_4$, filtered and concentrated under reduced pressure. Purification was done by column chromatography over silica (gradient elution: 0-30% EtOAc in hexane) to give compound **58c** as a white solid (1.7 g, yield: 52%).

$^1H$ NMR (500 MHz DMSO-$d_6$) δ ppm -10.27 (s, 1H), 8.34 (s, 1H), 8.00 (m, 2H), 7.70 (m, 1H), 7.55 (t, *J*=7.9 Hz, 2H), 6.40 (d, *J*=2.1 Hz, 1H), 5.54 (q, *J*=2.3 Hz, 1H), 4.65 (t, *J*=4.1 Hz, 2H), 4.34 (m, 1H), 4.19 (dd, *J* =7.9, 5.2 Hz, 1H), 3.87 (s, 1H).

Step 3: preparation of compound **53d**

Hydrazine (1 M in THF, 362 mL, 362 mmol) was added to a solution of compound **53c** (8.1 g, 18.12 mmol) in anhydrous EtOH (200 mL). The reaction mixture was stirred at reflux temperature allowing the THF to evaporate completely (the reaction was very slow in the presence of THF). After completion of the reaction (ca. 72 h), the solvent was removed under reduced pressure. The residue was purified by column chromatography over silica (gradient elution: 0 - 10% MeOH in DCM). The obtained product was dissolved in water and the pH adjusted to 5 by the addition of Amberlite® IR120 hydrogen form. The resin was removed by filtration and rinsed with water, the filtrate was evaporated and dried under high vacuum to give compound **53d** as a pale yellow solid (3.5 g, 68%).

$^1H$ NMR (500 MHz DMSO-$d_6$) δ ppm 12.75 (s, 1H), 8.65 (s, 1H), 8.54 (s, 1H), 5.92 (d, *J*=6.9 Hz, 1H), 5.75 (s, 1H) 4.39 (t, *J*=5.9 Hz, 1H), 4.11 (q, *J*=3.0 Hz, 1H), 3.83 (q, *J*=2.8 Hz, 2H), 3.69 (dd, *J*=12.1, 3.1 Hz, 2H), 3.64 (dd, *J*=12.1, 3.1 Hz, 2H), 3.42 (s, 3H), 3.16 (s, 1H); ESI-MS: *m*/*z* 283.1 $[M+H]^+$.

Step 4: preparation of compound **53e**

DMAP (0.75 g, 6.18 mmol) and DMTrCl (6.6 g, 19.7 mmol) (portionwise) were added to a solution of compound **53d** (3.5 g, 12.3 mmol, dried by co-evaporation with anhydrous toluene and dry pyridine) in dry pyridine (52 mL). The resulting reaction mixture was stirred for 4 h, after which it was quenched by the addition of methanol (10 mL) and concentrated under reduced pressure. The residue was purified by column chromatography over silica (gradient elution: 0 - 2% MeOH in DCM) to give compound **53e** as pale pink foam (4.8 g, yield: 67%). $^1H$ NMR (500 MHz DMSO-$d_6$) δ ppm: 12.76 (s, 1H), 8.50 (d, *J*=16.5 Hz, 2H), 7.28 (m, 4H), 7.20 (m, 5H) 6.84 (d, *J*=9.0 Hz, 4H), 5.99 (d, *J*=4.8 Hz, 1H), 5.82 (d, *J*=5.5 Hz, 1H), 4.67 (d, *J*=5.5 Hz, 1H) 4.19 (q, *J*=4.1 Hz, 1H), 3.94 (t, *J*=4.8 Hz, 1H), 3.73 (s, 6H), 3.37 (s, 3H), 3.23 (q, *J*=3.4 Hz, 2H); ESI-MS: m/z 585.3 $[M+H]^+$.

Step 5: preparation of compound **53f**

Compound **53e** (0.53 g, 0.9 mmol), sulfamate **17a** (0.876 g, 1.08 mmol) and DMAP (0.55 g, 4.5 mmol) were separately dissolved in dry DCM (3 × 20 mL). Each solution was dried with 3Å activated molecular sieves by stirring under $N_2$ for at least 2 h. To the DMAP solution, were respectively added the solution of compound **53e** and the solution of sulfamate **17a.** The resulting reaction mixture was stirred for 36 h. The molecular sieves were removed by filtration and thoroughly rinsed with DCM. The filtrate was washed with saturated aqueous $NaHCO_3$ and saturated aqueous $NH_4Cl$, dried over $Na_2SO_4$, filtered, and concentrated under reduced pressure. The residue was purified by silica column chromatography (gradient elution: 0 - 2% MeOH in DCM) to give compound **53f** as a pale yellow foam (0.45 g, yield: 38%).

ESI-MS: *m*/*z* 1321.5 $[M+H]^+$.

Step 5: preparation of compound **53g**

A solution of compound **53f** (0.75 g, 0.52 mmol) in DCM (20 mL), to which DCA (1.75 mL of 10% in DCM, 2.1

mmol) and water (47 μL, 2.6 mmol) were added, was stirred at rt until complete deprotection (ca. 2 h). The reaction mixture was quenched by the addition of pyridine (0.2 mL, 2.6 mmol) and some drops of methanol, and concentrated under reduced pressure. The residue was purified by silica column chromatography (gradient elution: 0 - 7% MeOH in DCM) to give compound **53g** as an off-white powder (0.33 g, yield: 82%).

$^1H$ NMR (500 MHz, DMSO-$d_6$) δ ppm 12.77 (s, 1 H), 11.24 (s, 1 H), 8.75 (s, 1 H), 8.60 - 8.64 (m, 3 H), 8.60 (s, 1 H), 8.04 (d, *J*=7.6 Hz, 2 H), 7.63 - 7.67 (m, 1 H), 7.53 - 7.58 (m, 2 H), 6.34 (d, *J*=4.8 Hz, 1 H), 6.37 (dd, *J*=20.0, 2.1 Hz, 1 H), 5.89 (d, *J*=6.2 Hz, 1 H), 5.59 (ddd, *J*=52.3, 4.8, 2.1 Hz, 1 H), 5.46 (t, *J*=4.8 Hz, 1 H), 5.18 (t, *J*=4.8 Hz, 1 H), 4.56 - 4.65 (m, 1 H), 4.13 - 4.18 (m, 2 H), 4.00 (td, *J*=7.4, 3.1 Hz, 1 H), 3.76 (s, 1 H), 3.60 - 3.69 (m, 1 H), 3.36 (s, 3 H), 3.26 - 3.31 (m, 1 H), 3.15 - 3.23 (m, 1 H); ESI-MS: *m/z* 717.3 $[M+H]^+$.

Step 6: preparation of compound **53h**

Note: THF was freshly distilled over Na/benzophenone and MeCN was freshly distilled over $CaH_2$ before use.

A solution of compound **53g** (100 mg, 0.14 mmol) and 1*H*-tetrazole (2.48 mL of a 0.45 M solution in MeCN, 1.12 mmol) in dry DMF (7 mL) was treated with 4Å molecular sieves for 30 min under $N_2$. It was then added dropwise a solution of 2-cyanoethyl-*N*,*N*,*N'*,*N'*-tetra(isopropyl)phosphorodiamidite (75 mg, 0.25 mmol) in dry THF (1.0 mL). After stirring the mixture at rt for 3 h, additional solution of 2-cyanoethyl-*N*,*N*,*N'*,*N'*-tetra(isopropyl)phosphorodiamidite (21 mg, 0.07 mmol) in dry THF (0.5 mL) was added. The resulting suspension was further stirred for 16 h. *t*BuOOH (140 μL of 5~6 M in decane, 0.70 mmol) was added and the reaction was stirred for an extra 30 min. The mixture was partially concentrated under reduced pressure. The residue was filtered and the filtrate was poured under stirring into $H_2O$ (20 mL) to give a suspension. The solid was collected, dissolved in DCM/MeOH (3 mL/1 mL) and purified by preparative thin layer chromatography (DCM:MeOH = 8:1). The desired fractions were collected and triturated in MeCN/DMSO (10 mL/2 mL) for 2 h to give a suspension. After filtration, the filtrate was evaporated to dryness to give crude compound **53h** as a solution in DMSO (2 mL) used directly into the next step.

ESI-MS: *m/z*=832.1 $[M+H]^+$

Step 7: preparation of **compound 27, sodium salt**

The previous solution of compound **53h** in DMSO (2 mL) was treated with $MeNH_2$ (30% in EtOH, 3 mL) and stirred at rt for 2 hrs. The mixture was evaporated to dryness, combined with another batch and purified by reverse phase preparative HPLC (Column Waters Xbridge Prep OBD 5μm C18 150×30; Condition water(10mM $NH_4HCO_3$) (A)-ACN (B) Begin B 0, End B 30; Gradient Time(min) 7, 100%B Hold Time (min) 1 Flow Rate (ml/min) 25) to give after lyophilization **compound 27, ammonium salt** (5 mg) as a white solid.

$^1H$ NMR (400 MHz, D2O) δ ppm 8.63 (s, 1 H), 8.45 (s, 1 H), 7.98 (s, 1 H), 6.77 (s, 1 H), 6.24 - 6.33 (m, 2 H), 5.11 - 5.34 (m, 3 H), 4.55 (br s, 1 H), 4.28 - 4.42 (m, 2 H), 4.23 (br s, 1 H), 4.08 - 4.20 (m, 1 H), 3.61 (br d, J=12.96 Hz, 1 H), 3.44 (s, 3 H), 3.33 (br d, J=13.69 Hz, 1H)

$^{19}F$ NMR (376 MHz, D2O) δ ppm -198.29 - -197.44 (m, 1 F)

$^{31}P$ NMR (162 MHz, D2O) δ ppm -2.34 (s, 1 P)

ESI-MS: *m/z*=675.3 $[M+H]^+$

**Conversion to sodium salt**

Dowex 50W × 8, 200-400 (10 mL, H form) was added to a beaker and washed with de-ionized $H_2O$ (30 mL). Then to the resin was added 15% $H_2SO_4$ in de-ionized $H_2O$ (30 mL), the mixture was gently stirred for 15 min, and decanted (30 mL). The resin was transferred to a column with 15% $H_2SO_4$ in de-ionized $H_2O$ and washed with 15% $H_2SO_4$ (at least 4 CV), and then with de-ionized $H_2O$ until it was neutral. The resin was transferred back into the beaker, 15% NaOH in de-ionized $H_2O$ (30 mL) solution was added, and mixture was gently stirred for 15 min, and decanted (1 ×). The resin was transferred to the column and washed with 15% NaOH in $H_2O$ (at least 4 CV), and then with de-ionized $H_2O$ until it was neutral. **compound 27, ammonium salt** (5 mg) was dissolved in de-ionized $H_2O$ (3 mL), added to the top of the column, and eluted with de-ionized $H_2O$. Desired fractions were pooled together and lyophilized to give **compound 27, sodium salt** (2.1 mg) as a white solid.

$^1H$ NMR (400 MHz, D2O) δ ppm 8.75 (s, 1 H), 8.52 (br s, 1 H), 7.84 - 8.36 (m, 1 H), 6.65 - 7.24 (m, 1 H), 6.38 (br d, J=19.07 Hz, 2 H), 5.28 - 5.46 (m, 1 H), 5.26 (br dd, J=8.53, 4.77 Hz, 1 H), 4.81 - 4.93 (m, 1 H), 4.63 (br s, 1 H), 4.42 (br dd, J=10.54, 7.78 Hz, 2 H), 4.19 - 4.34 (m, 2 H), 3.58 - 3.75 (m, 1 H), 3.54 (s, 3 H), 3.30 - 3.48 (m, 1 H)

$^{19}F$ NMR (377 MHz, D2O) δ ppm -197.75 (br s, 1 F)

$^{31}P$ NMR (162 MHz, D2O) δ ppm -2.15 (br s, 1 P)

ESI-MS: *m/z*=675.2 $[M+H]^+$

Example 54

Compound 3

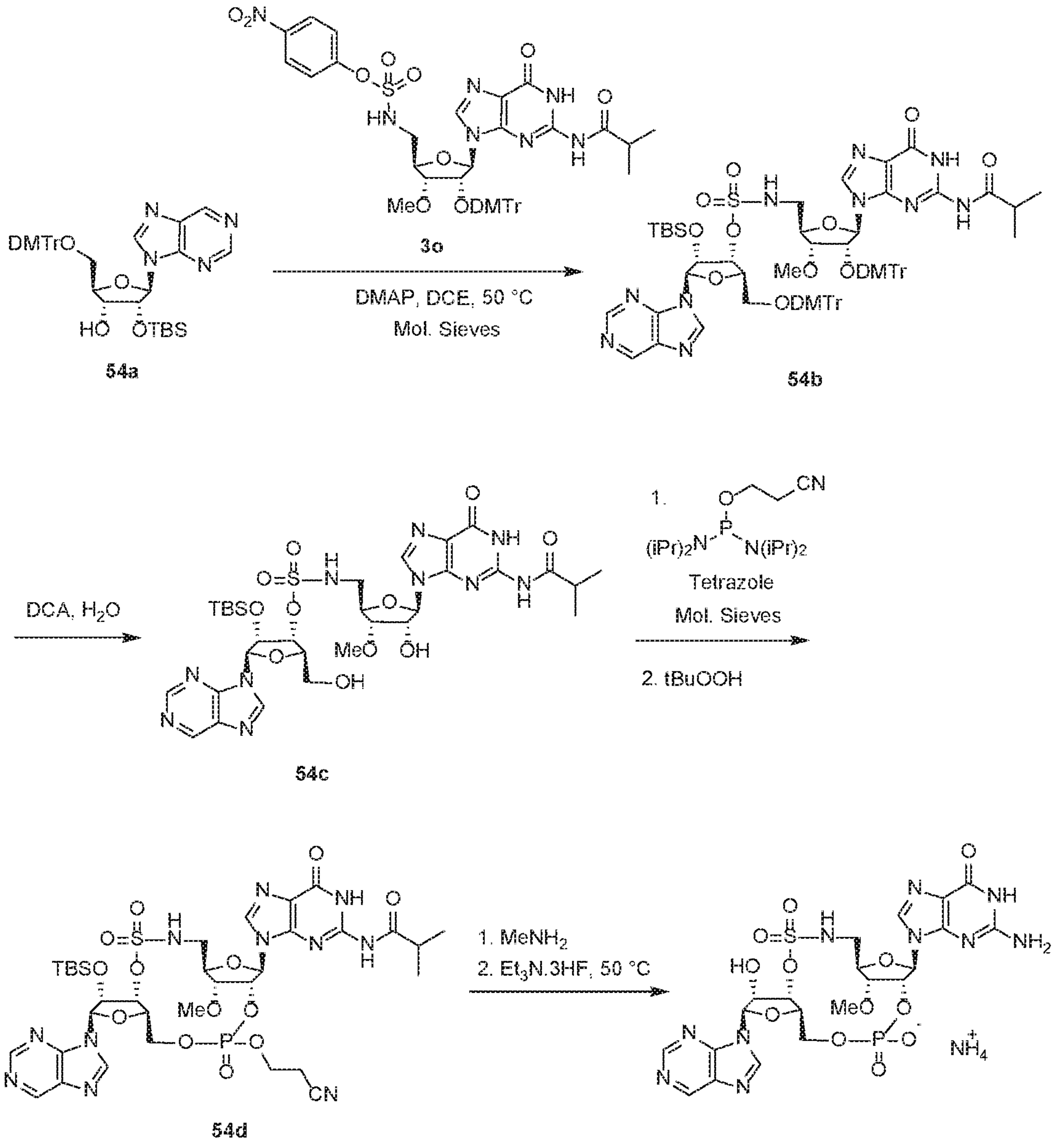

54a
54b
54c
54d

Compound 3, ammonium salt

Step 1: preparation of compound 54b

Compound **54a** (1 g, 1.5 mmol) and sulfamate **3o** (1.3 g, 1.5 mmol) were dissolved in DCE (10 mL). 4Å Molecular sieves (powder) was added and the reaction mixture was stirred at room temperature for 2 h under $N_2$. A mixture of DMAP (913 mg, 7.5 mmol) and 4Å molecular sieves powder (0.5 g) in DCE (2 mL) was stirred at room temperature for 6 h under $N_2$ and then added to the above mixture. (Note: DCE was dried on activated 3Å molecular sieves before use.). The reaction mixture was stirred at 50 °C overnight. The molecular sieves were removed by filtration and rinsed with DCM The filtrate was washed with saturated aqueous $NaHCO_3$, the aqueous phase was extracted with DCM.The combined organic layers were dried with $Na_2SO_4$, filtered and evaporated under vacuum. The residue was purified by

silica column chromatography (gradient elution: 1 - 2% MeOH in DCM) to give 1.8 g (yield: 83%) a mixture of compound **54b** and its 2'-coupled regioisomer (structure not shown), the latter one being the result of compound **54a** partially undergoing a 2'→3' TBS-shift.

ESI-MS: *m*/*z* 1097.5 [M-DMTr+H]$^+$.

Step 2: preparation of compound **54c**

The above product mixture (2.6 g, 1.84 mmol) was dissolved in DCM (30 mL), to which DCA (610 μL, 7.40 mmol) and water (330 μL, 1.84 mmol) were added. The reaction mixture was stirred at room temperature until complete deprotection (ca. 1 h) after which it was quenched by the addition of pyridine (1.5 mL, 18.4 mmol) in methanol (4 mL) and subsequently washed with water. The organic layer was dried with anhydrous $Na_2SO_4$, filtered and concentrated under reduced pressure. The residue was purified by preparative reversed phase HPLC (Stationary phase: XBridge C18 OBD, 5 μm, 150 × 30 mm; Mobile phase: $H_2O$ (A) - MeCN (B); gradient elution) to give pure compound **54c** as a white solid (600 mg, yield: 40%).

$^1$H NMR (400 MHz, DMSO-*d*$_6$) δ ppm 12.09 (br s, 1 H), 11.62 (br s, 1 H), 9.26 (s, 1 H), 8.99 (s, 1 H), 8.89 (s, 1 H), 8.45 (br s, 1 H), 8.24 (s, 1 H), 6.10 (d, *J*=6.0 Hz, 1 H), 5.82 (d, J=6.0 Hz, 1 H), 5.71 (d, *J*=6.3 Hz, 1 H), 5.48 (br s, 1 H), 4.91 - 5.00 (m, 2 H), 4.72 (q, *J*=5.8 Hz, 1 H), 4.29 - 4.40 (m, 1 H), 4.03 - 4.12 (m, 1 H), 3.87 (br t, *J*=4.3 Hz, 1 H), 3.60 - 3.74 (m, 2 H), 3.44 (s, 3 H), 3.35 - 3.42 (m, 1 H), 3.29 (dd, *J*=14.1, 7.8 Hz, 1 H), 2.75 (spt, *J*=6.8 Hz, 1 H), 1.11 (d, *J*=6.8 Hz, 3 H), 1.09 (d, *J*=6.9 Hz, 3 H), 0.63 (s, 9H), -0.08 (s, 3 H), -0.34 (s, 3 H);
ESI-MS: *m*/*z* 795.4 [M+H]$^+$.

Step 3: preparation of compound **54d**

A solution of compound **54c** (400 mg, 0.5 mmol) and 1*H*-tetrazole (8.9 mL of a 0.45 M solution in MeCN, 4.03 mmol) in a mixture of dry THF (48 mL) and dry DMF (4 mL) was treated with 4Å molecular sieves for 30 min under $N_2$ after which 2-cyanoethyl-*N*,*N*,*N'*,*N'*-tetra(isopropyl)phosphorodiamidite (273 mg, 0.91 mmol) in dry THF (4 mL) was added dropwise over 10 min. (Note: THF was freshly distilled over Na/benzophenone and MeCN was freshly distilled over $CaH_2$ before use.) The resulting reaction mixture was stirred for 3 h at room temperature. Next, a solution of *t*BuOOH (500 μL of 5~6 M solution in decane, 2.5 mmol) was added and stirring was continued for another 30 min. The reaction mixture was filtered through a pad of Celite and concentrated. The residue was dissolved in DCM and washed with water. The organic layer was dried on $Na_2SO_4$, filtered and concentrated under reduced pressure. The crude product was purified by flash chromatography on silica gel (gradient elution: 1 - 10% MeOH in DCM) to give compound **54d** (130 mg, 23% yield) as a white solid.

ESI-MS: *m*/*z* 910.5 [M+H]$^+$.

Step 3: preparation of **compound 3, ammonium salt**

Compound **54d** (58 mg) was stirred in a 30% methylamine solution in ethanol (2 mL) at room temperature for 2 h. The reaction mixture was concentrated under reduced pressure. The residue was dissolved in pyridine (2 mL) after which $Et_3N$ (30 mg, 0.27 mmol) and triethylamine trihydrofluoride (21.7 mg, 0.13 mmol) were added, the resulting mixture was stirred at 50 °C for 1 day. The reaction solution was cooled to room temperature, isopropoxytriethylsilane (0.1 mL, 0.54 mmol) was added and stirring was continued overnight. The residue, obtained after concentration under reduced pressure, was purified by preparative reversed phase HPLC (Stationary phase: XBridge C18 OBD, 5 μm, 150 × 25 mm; Mobile phase: 10 mM aqueous ammonia bicarbonate (A) - MeCN (B); gradient elution) to give **compound 3, ammonium salt** (4 mg).

$^1$H NMR (400 MHz, D2O) δ ppm 9.26 (s, 1 H), 9.02 (s, 1 H), 8.74 (s, 1 H), 7.94 (s, 1 H), 6.40 (d, *J*=1.5 Hz, 1 H), 6.01 (d, *J*=8.5 Hz, 1 H), 5.39 (td, *J*=9.1, 5.3 Hz, 1 H), 5.25 (dd, *J*=7.9, 4.5 Hz, 1 H), 5.06 - 5.11 (m, 1 H), 4.55 (s, 1 H), 4.48 (br d, *J*=12.0 Hz, 1 H), 4.09 - 4.18 (m, 2 H), 3.86 (br d, *J*=14.6 Hz, 1 H), 3.72 (br d, *J*=14.3 Hz, 1 H), 3.65 (s, 3 H); $^{31}$P NMR (162 MHz, D2O) δ ppm -2.22 (s, 1 P); ESI-MS: m/z 673.2 [M+H]$^+$.

Example 55

**Compound 39**

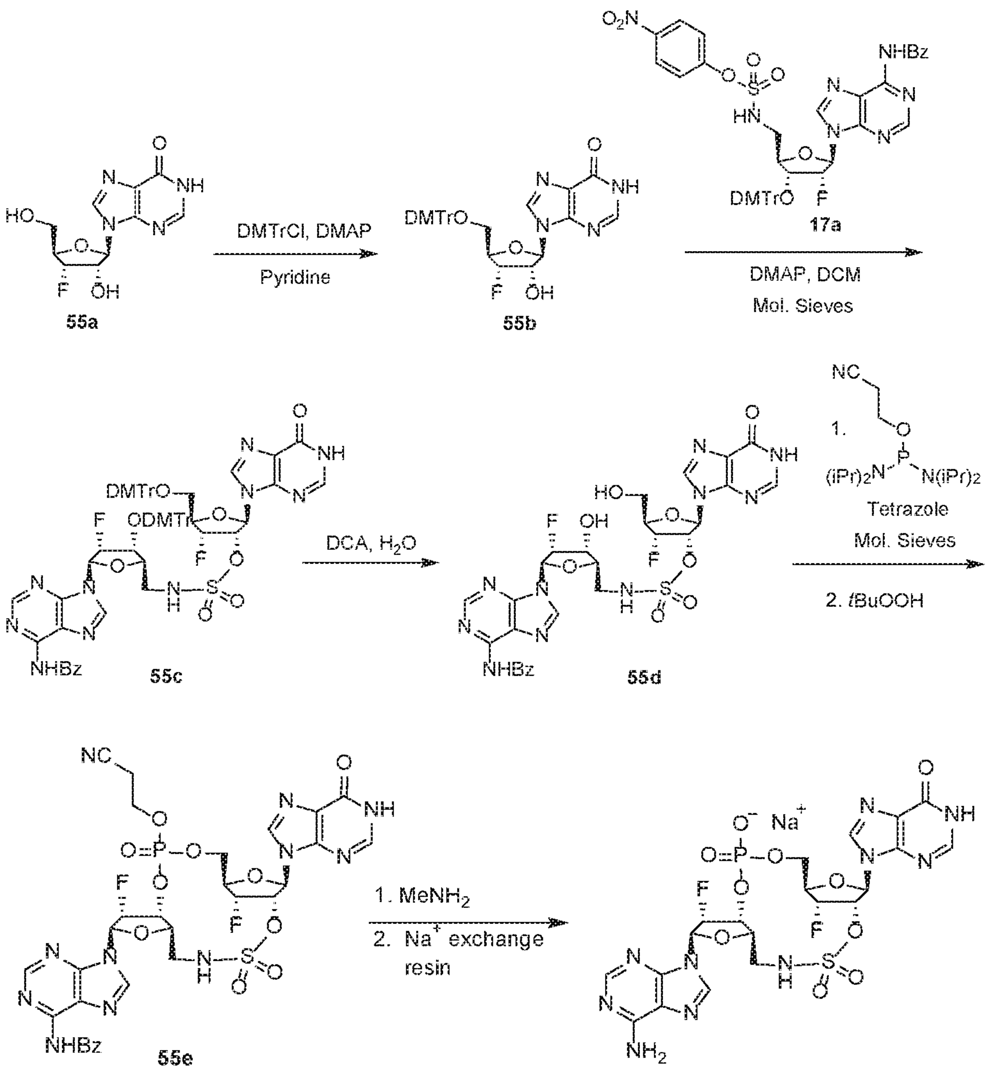


**Compound 39, sodium salt**

Step 1: Preparation of compound **55b**

A solution of 3'-deoxy-3'-fluoroinosine **55a** [CAS 117517-20-1] (2.2 g, 8.14 mmol) in dry pyridine (33 mL), to which DMAP (0.49 g, 4.0 mmol) and DMTrCl (4.4 g, 13 mmol) (portion wise) were added, was stirred at room temperature until complete conversion (ca. 2.5 h). The reaction mixture was quenched with methanol (10 mL) and concentrated under reduced pressure. The obtained residue was dissolved in ethyl acetate and washed with water. The organic layer was dried over anhydrous $Na_2SO_4$, filtered and concentrated under reduced pressure. Purification was done by column chromatography over silica gel (gradient elution: 0 - 2% MeOH in DCM) to give compound **55b** as an off-white foam (3.3 g, yield: 71%).

$^1H$ NMR (500 MHz DMSO-$d_6$) δ ppm: 8.22 (d, J=2.1 Hz, 1H), 7.93 (d, J=1.4 Hz, 1H), 7.36 (d, J=8.3 Hz, 2H), 7.28 (t, J=7.6 Hz, 2H), 7.23 (m, 5H), 6.86 (dd, J=8.3, 6.2 Hz, 4H), 5.92 (d, J=7.6 Hz, 1H), 5.14 (dd, J=54.1, 4.5 Hz, 1H), 5.02 (dq, J=23.2, 3.9 Hz, 1H), 4.35 (dt, J=25.9, 4.3 Hz, 1H), 3.74 (s, 6H), 3.29 ( dq, J=37.5, 5.2 Hz, 2H); ESI-MS:*m/z* 572.0 $[M+H]^+$.

Step 2: Preparation of compound **55c**

Compound **55b** (0.66 g, 1.15 mmol), sulfamate **17a** (1.21 g, 1.38 mmol) and DMAP (0.704 g, 5.76 mmol) were

each separately dissolved in dry DCM (3 × 20.0 mL) and dried on activated 3Å molecular sieves for at least 2 h under an inert atmosphere. Next, the compound **55b** and sulfamate **17a** solutions were successively transferred to the reaction flask containing the DMAP solution. The resulting reaction mixture was stirred for 24 h. The molecular sieves were removed by filtration and thoroughly rinsed with dichloromethane. The filtrate was successively washed with saturated aqueous $NaHCO_3$ and saturated aqueous $NH_4Cl$, dried over $Na_2SO_4$, filtered, and concentrated under reduced pressure. The residue was purified by silica column chromatography (gradient elution: 0 - 2% MeOH in DCM) to give compound **55c** as an off-white foam (0.475 g, yield: 31%).

$^1H$ NMR (500 MHz DMSO-$d_6$) δ ppm: 12.47 (s, 1H), 11.23 (s, 1H), 8.66 (s, 1H), 8.56 (s, 1H), 8.48 (s, 1H), 8.19 (s, 1H), 8.03 (d, J=7.6 Hz, 2H), 7.84 (s, 1H), 7.65 (t, J=7.2 Hz, 1H), 7.55 (t, J=7.6 Hz, 2H), 7.48 (d, J=7.6 Hz, 2H ), 7.36 (q, J=4.8 Hz, 4H), 7.32 (m, 4H), 7.23 (t, J=7.6 Hz, 4H), 7.19 (t, J=7.9 Hz, 5H), 6.90 (dd, J=9.0, 6.9 Hz, 4H), 6.81 (dd, J=9.0, 6.2 Hz, 4H), 6.34 (m, 1H), 6.23 (d, J=6.2 Hz, 1H), 5.78 (m, 2H), 5.50 (d, J=53.7 Hz, 1H), 4.72 (m, 2H), 4.43 (d, J=24.1 Hz, 1H), 4.01 (t, J=7.9 Hz, 1H), 3.74 (s, 1H), 3.70 (t, J=2.4 Hz, 12H), 3.25 (dd, J=10.7, 3.8 Hz, 1H), 2.99 (d, J=13.1 Hz, 1H), 2.70 (dd, J=14.5, 9.0 Hz, 1H); ESI-MS: *m/z* 1310.0 $[M+H]^+$.

Step 3: Preparation of compound **55d**

A solution of compound **55c** (0.453 g, 0.34 mmol) in DCM (12.6 mL), to which DCA (1.14 mL of 10% in DCM, 1.38 mmol) and water (31 μL, 1.72 mmol) were added, was stirred at room temperature until complete deprotection (ca. 2 h). The reaction mixture was quenched by the addition of pyridine (0.14 mL, 1.72 mmol) and some drops of methanol. The resulting suspension was stirred for 20 min, filtered and dried to get compound **55d** (0.21 g, yield: 86%).

$^1H$ NMR (500 MHz DMSO-$d_6$) δ ppm: 12.5 (d, *J*=3.4 Hz, 1H), 11.25 (s, 1H), 8.75 (s, 1H), 8.69 (t, *J*=6.0 Hz, 1H), 8.61 (s, 1H), 8.35 (s, 1H), 8.09 (d, *J*=3.4 Hz, 1H), 8.05 (d, 7.6 Hz, 2H), 7.66 (t, *J*= 7.2 Hz, 1H), 7.56 (t, *J*=7.6 Hz, 2H), 6.32 (dd, *J*=20.0, 2.1 Hz, 1H), 6.21 (d, *J*=7.6 Hz, 1H), 5.85 (d, *J*=6.2 Hz, 1H), 5.60 (m, 1H), 5.49 (m, 3H), 5.34 (d, *J*=4.8 Hz, 1H), 4.57 (m, 1H), 4.40 (dt, *J*=26.9, 3.4 Hz, 1H), 3.92 (q, *J*=6.0 Hz, 1H), 3.65 (t, *J*=4.1 Hz, 2H), 3.17 (t, *J*=6.2 Hz, 2H); ESI-MS: *m/z* 706.0 $[M+H]^+$.

Step 4: Preparation of compound **55e**

A solution of compound **55d** (260 mg, 0.37 mmol) and 1H-tetrazole (2.15 mL of a 3 - 4% in MeCN, dried on activated 3Å molecular sieves) in DMF / THF (1:2, 30 mL, dried on activated 3Å molecular sieves) was treated with 3Å molecular sieves for 2 h under an inert atmosphere after which 2-cyanoethyl-*N,N,N',N'*-tetra(isopropyl) phosphorodiamidite (129 μL, 0.41 mmol) was added in one portion. The resulting reaction mixture was shaken at room temperature overnight An additional amount of 2-cyanoethyl-*N,N,N',N'*-tetra(isopropyl)phosphorodiamidite (23 μL, 0.074 mmol) was added and shaking was continued for an extra day to obtain full conversion. *t*BuOOH (134 μL of 5.5 M solution in decane, 0.74 mmol) was added and the reaction mixture was shaken for an extra hour. Molecular sieves were removed by filtration and rinsed with dichloromethane. The filtrate was extensively washed with water, dried with MgSO4, filtered and concentrated under reduced pressure. The residue was purified by silica column chromatography (gradient elution: 0 - 5% MeOH in DCM) to give compound **55e** (20 mg, yield: 7%). ESI-MS: *m/z* 820.4 $[M+H]^+$.

Step 5: Preparation of **compound 39, sodium salt**

Compound **55e** (20 mg, 0.024 mmol) was stirred in a 33% methylamine solution in ethanol (10 mL) at room temperature until complete conversion (~1 h). The reaction mixture was concentrated under reduced pressure. The resulting crude product was triturated in MeCN followed by preparative reversed phase HPLC purification (Stationary phase: XBridge C18 OBD, 5 μm, 250 x 30 mm; Mobile phase: aqueous 0.25% ammonia bicarbonate (A) - MeCN (B); gradient elution) to give **compound 39, ammonium salt.** Final conversion into the sodium salt was done by elution of an aqueous solution over a column packed with a cationic Na ion-exchange resin to afford **compound 39, sodium salt** as a white fluffy solid after lyophilization (13.5 mg, yield: 80%).

$^{31}P$ NMR (162 MHz, DMSO-$d_6$) δ ppm -2.06 (s, 1 P); ESI-MS: *m/z* 663.3 $[M+H]^+$.

Example 56

## Compound 40

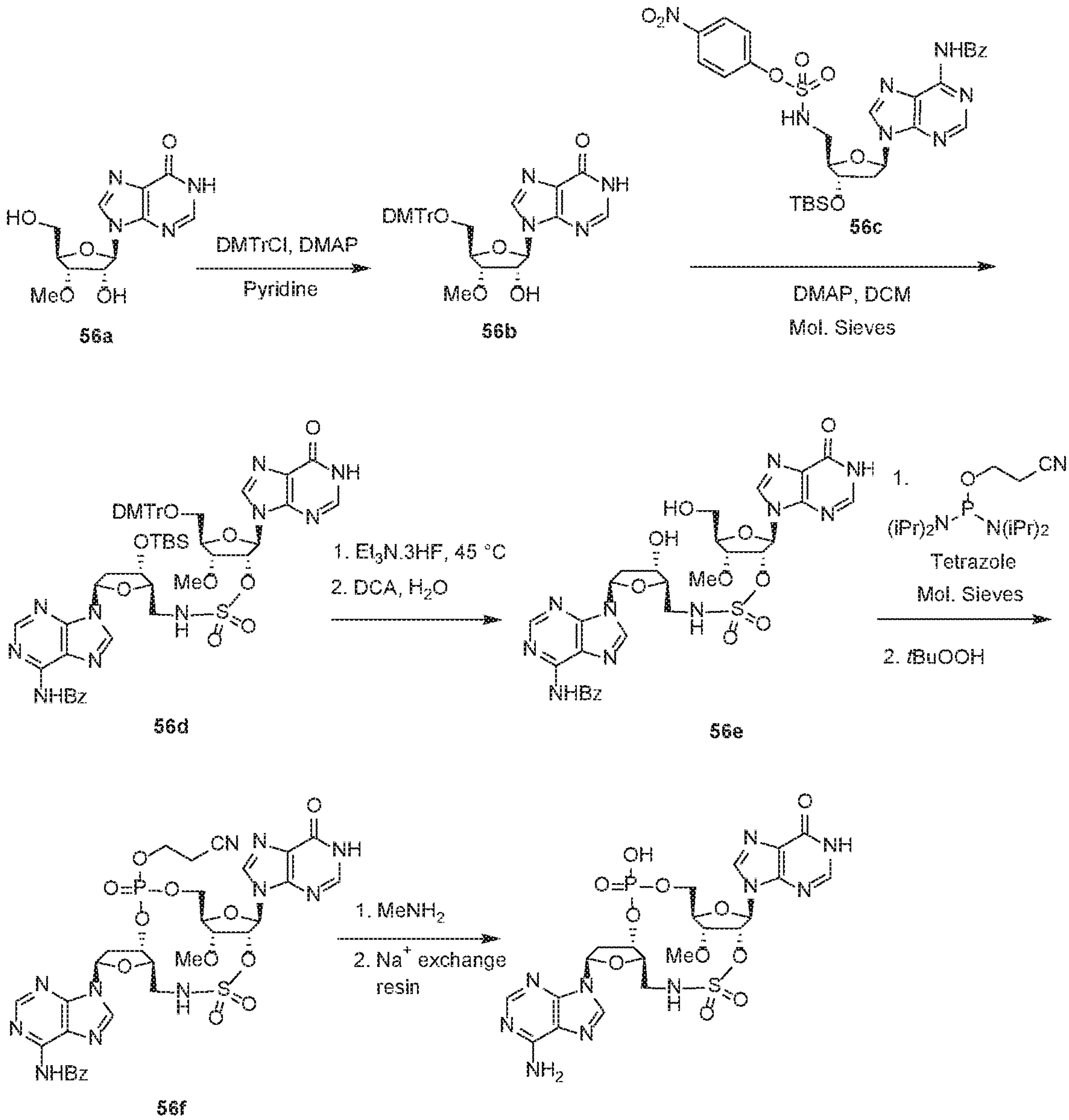


**Compound 40, sodium salt**

Step 1: preparation of compound **56b**

A solution of 3'-(*O*-methyl)inosine **(56a,** 1.5 g, 5.31 mmol, CAS#75479-64-0 ) in dry pyridine (30 mL), to which DMAP (0.32 g, 2.65 mmol) and DMTrCl (2.69 g, 7.97 mmol) (portion wise) were added, was stirred at room temperature until complete conversion. The reaction mixture was quenched with methanol (15 mL) and concentrated under reduced pressure. The obtained residue was dissolved in ethyl acetate and washed with water. The organic layer was washed with water, dried with anhydrous $Na_2SO_4$, filtered and concentrated under reduced pressure. Purification was done by column chromatography over silica gel (gradient elution: 0 - 2.5% MeOH in DCM) to give compound **56b** as a pale brown foam (2.19 g, yield: 71%). $^1H$ NMR (500 MHz, DMSO-$d_6$) δ ppm: 12.35 (s, 1H), 8.22 (s, 1H), 8.00 (s, 1H), 7.34 (d, *J* = 7.6 Hz, 2H), 7.27 (t, J= 7.6 Hz, 2H), 7.21 (m, 5H), 6.84 (m, 4H), 5.89 (d, *J* = 4.8 Hz, 1H), 5.62 (d, *J* = 6.2 Hz, 1H), 4.78 (q, *J* = 5.3 Hz, 1H), 4.13 (q, *J* = 4.6 Hz, 1H), 3.99 ( t, *J* = 5.2 Hz, 1H), 3.73 (s, 6H), 3.37 (s, 3H), 3.22 (d, *J* = 4.1 Hz, 2H); ESI-MS: *m*/*z* 585 $[M+H]^+$.

Step 2: preparation of compound **56d**

A reaction flask was charged with DMAP (0.76 g, 6.2 mmol), dry DCM (25 mL) and activated 3Å molecular sieves. The resulting mixture was stirred at room temperature for at least 2 h under inert atmosphere. Simultaneously, a solution of compound **56b** (0.73 g, 1.24 mmol) and a solution of compound **56c** (1.0 g, 1.49 mmol), each in dry DCM (2 x 25 mL), were dried on activated 3Å molecular sieves (ca. 2 h). Both solutions (compound **56b** and compound **56c** respectively) were successively transferred to the reaction flask. The resulting reaction mixture was stirred for 24 h. The molecular sieves were removed by filtration and thoroughly rinsed with dichloromethane. The filtrate was washed with saturated aqueous $NaHCO_3$, brine and saturated aqueous $NH_4Cl$, dried over $Na_2SO_4$, filtered, and concentrated under reduced pressure. The residue was purified by silica column chromatography (gradient elution: 0 - 2% MeOH in DCM) to give compound **56d** (0.54 g, yield: 39%). $^1H$ NMR (500 MHz DMSO-$d_6$) δ ppm: 8.72 (s, 1H), 8.66 (s, 1H), 8.27 (s, 1H), 8.09 (m, 1H), 8.05 (m, 2H), 7.99 (s, 1H), 7.65 (m, 1H), 7.56 (t, *J* = 7.6 Hz, 2H), 7.29 (d, *J* = 6.9 Hz, 2H), 7.24 (t, *J* = 7.6 Hz, 2H), 7.18 (m, 5H), 6.87 (d, *J* = 9.0 Hz, 1H), 6.81 (dd, *J* = 9.0, 4.1 Hz, 4H), 6.46 (m, 1H), 6.24 (d, *J* = 3.4 Hz, 1H), 5.66 (m, 1H), 4.63 (t, *J* = 2.8 Hz, 1H), 4.54 (m, 1H), 4.11 (m, 1H), 3.93 (m, 1H), 3.71 (s, 6H), 3.32 (d, *J* = 8.3 Hz, 12H), 3.26 (m, 3H), 3.19 (m, 3H), 2.99 (m, 1H), 2.58 (m, 4H), 2.35 (m, 1H), 0.89 (s, 9H), 0.11 (s, 6H); ESI-MS: *m/z* 1116 $[M+H]^+$.

Step 3: preparation of compound **56e**

$Et_3N$ (2.65 mL, 19.0 mmol) and $Et_3N.3HF$ (0.31 mL, 1.9 mmol) were added to a solution of compound **56d** (0.53 g, 0.47 mmol) in pyridine (9.5 mL). The reaction mixture was stirred at 45 °C until complete conversion (ca. 5 h) and then cooled to room temperature. Isopropoxytrimethylsilane (1.34 mL, 7.6 mmol) was added and stirring was continued overnight Concentration under reduced pressure gave the crude 5'-deprotected compound which was re-dissolved in DCM (13.3 mL). Water (40 μL, 2.37 mmol) and dichloroacetic acid (1.57 mL of 10% in DCM, 1.9 mmol) were added, the resulting reaction mixture was stirred for 1 h (full conversion) after which it was quenched by the addition of pyridine (200 μL, 2.37 mmol) and some drops of methanol. The residue obtained after concentration under reduced pressure was purified by silica column chromatography (gradient elution: 0 - 9% MeOH in DCM) to give compound **56e** (220 mg, yield: 66.6% two-step). $^1H$ NMR (500 MHz DMSO-$d_6$) δ ppm: 12.43 (br s, 1H), 11.20 (br s, 1H), 8.72 (s, 1H), 8.63 (s, 1H), 8.57 (br t, *J* = 5.5 Hz, 1H), 8.33 (s, 1H), 8.04 (m, 3H), 7.65 (t, *J* = 7.2 Hz, 1H), 7.56 (t, *J* = 7.9 Hz, 2H), 6.43 (t, *J* = 6.9 Hz, 1H), 6.17 (d, *J* = 5.5 Hz, 1H), 5.45 (m, 2H), 5.26 (t, *J* = 5.5 Hz, 1H), 4.38 (m, 1H), 4.23 (t, *J* = 4.1 Hz, 1H), 4.12 (q, *J* = 3.7 Hz, 1H), 3.84 (m, 1H), 3.68 (m, 1H), 3.57 (m, 1H), 3.40 (s, 3H), 3.12 (m, 2H), 2.84 (m, 1H), 2.33 (m, 1H); ESI-MS: *m/z* 699 $[M+H]^+$.

Step 4: preparation of compound **56f**

A solution of compound **56e** (200 mg, 0.286 mmol) and 1*H*-tetrazole (5.09 mL of a 0.45 M solution in MeCN, 0.99 mmol, dried on 4Å molecular sieves before use) in dry MeCN / THF (1:2, 9 mL) was treated with 4Å molecular sieves for 30 min under $N_2$ after which 2-cyanoethyl-*N,N,N',N'*-tetra(isopropyl)phosphorodiamidite (155 mg, 0.52 mmol) in dry MeCN (2 mL) was added dropwise over 10 min (note: THF was freshly distilled over Na/benzophenone and MeCN was freshly distilled over $CaH_2$ before use). The resulting reaction mixture was stirred for 30 min at room temperature. A solution of *t*BuOOH (286 μL of 5~6 M solution in decane, 1.43 mmol) was added and stirring was continued for another 30 min. The mixture was diluted with DCM (20 mL), filtered through a pad of Diatomaceous earth and concentrated. The crude product was purified by silica column chromatography (gradient elution: 0 - 8% MeOH in DCM) to give compound **56f** (130 mg, yield: 44% (purity: ~80% LCUV)) as a white solid. ESI-MS: *m/z* = 814.3 $[M+H]^+$.

Step 5: preparation of **compound 40, sodium salt**

The above compound **22f** (130 mg, 0.072 mmol) was stirred in a 30% methylamine solution in ethanol (15 mL) at room temperature for 2 h. The reaction mixture was concentrated under reduced pressure. The residue was dissolved in water, washed with DCM and lyophilized. The crude product was purified by preparative reversed phase HPLC (Stationary phase: Xbridge OBD C18, 5 μm, 150 x 30 mm; Mobile phase: 10 mM aqueous ammonia bicarbonate (A) - MeCN (B); gradient elution). Final conversion into the sodium salt was done by elution of an aqueous solution over a column packed with a cation sodium ion-exchange resin to give **compound 40, sodium salt** as a white fluffy solid after lyophilization (5.8 mg, yield: 3% from **22f**). $^1H$ NMR (400 MHz, $D_2O$) δ ppm 8.39 (s, 1 H), 7.91 (br d, J=13.2 Hz, 2 H), 7.24 (br s, 1 H), 6.26 (br s, 1 H), 6.18 (br d, J=8.3 Hz, 1 H), 5.49 (br s, 1 H), 4.87 - 5.06 (m, 1 H), 4.51 (br s, 1 H), 4.30 (br d, J=3.7 Hz, 1 H), 4.10 (br s, 3 H), 3.42 (s, 3 H), 3.38 (br s, 2 H), 2.53 - 2.75 (m, 2 H);
$^{31}P$ NMR (162 MHz, $D_2O$) δ ppm -1.23 (s, 1 P); ESI-MS: *m/z* = 657.0 $[M+H]^+$.

## Biological Examples

### *In Vitro Assays*

### Example 1

STING SPA binding assay

The human STING SPA binding assay measures displacement of tritium labeled 2',3'cGAMP (cyclic (guanosine-(2' -> 5')-monophosphate-adenosine-(3' -> 5')-monophosphate) to biotinylated STING protein. A soluble version of recombinant STING was expressed in E.coli that lacks the four transmembrane domains and contains residues 139-379 of Q86WV6 with an R at position 232 (H232R). Based on the allele frequency of 58% of the population, H232R is considered to be a wild type (Yi, et al., "Single Nucleotide Polymorphisms of Human STING can affect innate immune response to cyclic dinucleotides" PLOS ONE. 2013, 8(10), e77846). The STING construct has an N-terminal HIS tag, followed by a TEV protease cleavage site and an AVI tag to allow directed biotinylation by BirA biotin ligase (Beckett et al., A minimal peptide substrate in biotin holoenzyme synthetase-catalyzed biotinylation. (1999) Protein Science 8, 921-929). The HIS tag is cleaved after purification and prior to biotinylation.

The assay was run in 1536-well plates in a total volume of 8 $\mu$L per well by adding 8 nM [$^3$H]-2'3'-cGAMP and 40 nM biotin-STING protein in assay buffer [25mM HEPES (Corning 25-060-C1) pH 7.5, 150 mM NaCl (Sigma S5150), 0.5 mg/mL BSA (Gibco 15260-037), 0.001% Tween-20 (Sigma P7949), molecular grade water (Corning 46-000-CM)]. Test compounds (80 nL) were added with an acoustic dispenser (EDC Biosystems) in 100% DMSO for a final assay concentration of 1% DMSO. Plates were centrifuged for 1 min and incubated for 60 min at room temperature. Finally, (2 $\mu$L) polystyrene streptavidin SPA beads (PerkinElmer RPNQ0306) were added and plates were sealed and centrifuged for 1 min at room temperature. Plates were dark adapted for 2 h and read on a ViewLux (Perkin Elmer) for 12 min per plate. A saturation binding curve for [$^3$H]-2'3'-cGAMP showed a $K_D$ of 3.6 $\pm$ 0.3 nM for binding to STING, comparable to reported values for the natural ligand (Zhang et al., Cyclic GMP-AMP containing mixed phosphodiester linkages is an endogenous high-affinity ligand for STING.

Other natural ligands including cyclic-di-GMP also returned values in this assay within the expected range. Reference compound is cGAMP and results are reported as percent inhibition and $IC_{50}$ values. Binding to mouse STING used a construct similar to the one described above containing residues 138-378 of Q3TBT3.

Full length human STING binding assay

Human STING from residues 1-379 of Q86WV6 with an R at position 232 (H232R) with an N-terminal 6HIS tag followed by a FLAG tag, a TEV protease cleavage site and an AVI tag for biotinylation was recombinantly expressed in HEK293-EXPI cells. Purified membranes were prepared from these cells and STING expression was confirmed and quantified by immunoblot STING containing membranes were combined with test compound in a Greiner 384-well assay plate and incubated at room temperature for one hour in the same assay buffer used for the STING SPA binding assay. Next, [$^3$H]-2'3'-cGAMP was added and plates were incubated for 30 min at room temperature. Reactions were transferred to a prewashed Pall 5073 filter plate and each well was washed 3 times with 50 $\mu$L assay buffer. Filter plates were dried at 50 °C for 1 h. To each well, 10 $\mu$L of Microscint scintillation fluid was added and plates were sealed and read on a TopCount (Perkin Elmer) for 1 min per well.

STING SPR binding assay

Compounds were analyzed on an S200 biacore SPR instrument (GE Healthcare). E.coli produced truncated STING protein was immobilized on a series S streptavidin chip via biotin capture (GE Healthcare #BR100531) with. Compounds were screened at 1:2 dilutions from 100 uM to 0.195 uM in run buffer (10mM HEPES, pH 7.4, 150mM NaCl, 0.005% P20, ImM TECEP). Steady state affinity and kinetic evaluations were carried out using 1:1 binding model (STING was treated as a dimer). Run parameters were as follows: 60 sec on, 300 sec off for the IFM compounds, cyclic-di-GMP (60sec on/60sec off), thiol isomer 1 (60 sec on/300 sec off) and cGAMP (60sec on/1200sec off) with a flow rate of 50$\mu$L/min and data collection at 40 Hz at 25 °C.

STING human cell reporter assay

Agonism of the human STING pathway is assessed in THP1-ISG cells (Invivogen, cat #thp-isg) derived from human THP1 monocyte cell line by stable integration of an interferon regulatory factor (IRF)-inducible SEAP reporter construct. THP1-Blue ISG cells express a secreted embryonic alkaline phosphatase (SEAP) reporter gene under the

control of an ISG54 minimal promoter in conjunction with five interferon (IFN)-stimulated response elements. As a result, THP1-Blue ISG cells allow the monitoring of IRF activation by determining the activity of SEAP. The levels of IRF-induced SEAP in the cell culture supernatant are readily assessed with alkaline phosphatase detection medium, a SEAP detection reagent These cells are resistant to Zeocin. 2'3'cGAMP was used as a positive control in this assay. To run the assay, 60,000 cells were dispensed in 30 μL/well of a white, opaque bottom tissue culture treated 384-well plate.

Test compounds were added in a volume of 10 μL (1% DMSO final concentration). Compounds are initially prepared in 100% DMSO, spotted on an compound dilution plate and then diluted in media prior to transfer. The assay was incubated for 24 h at 37 °C, 5% $CO_2$ then plates were centrifuged at 1200 rpm (120x g) for 5 min. After final incubation, 90 μL of alkaline phosphatase detection medium-substrate was added to each well of a new 384-well clear plate and 10 μL of the cell supernatant was transferred from the assay plate to the new alkaline phosphatase detection medium-plate using a Biomek FX and mixed 4 times. Plates were incubated at RT for 20 min then absorbance at 655 nm was determined on the Tecan Safire2.

STING mouse cell reporter assay

Agonism of the mouse STING pathway is assessed in RAW Lucia cells (Invivogen,cat # rawl-isg) derived from mouse RAW-264.7 macrophage cell line by stable integration of an interferon-inducible Lucia luciferase reporter construct. RAW Lucia cells express a secreted luciferase reporter gene under the control of an ISG54 minimal promoter in conjunction with five interferon (IFN)-stimulated response elements. As a result, RAW Lucia cells allow the monitoring of IRF activation by determining the activity of luciferase. The levels of IRF-induced luciferase in the cell culture supernatant are readily assessed with QUANTI-Luc™, a luciferase detection reagent. These cells are resistant to Zeocin. 2'3'cGAMP is used as a positive control in this assay. To run the assay, 100,000 cells were dispensed in 90μL/well of a clear, flat bottom tissue culture treated 96-well plate. Test compounds were added in a volume of 10gL. The assay was incubated for 24 and 48 hours at 37°C, 5% CO2. After incubation, 20μL of the cell supernatant from the assay plate was transferred to a new 96-well white plate and 50uL of QUANTI-Luc substrate was added. The plate was incubated, shaking, at RT for 5 minutes then luminescence was read on an EnVision 2104 with 0.1s integration time.

Human interferon-B induction assay

THP1-Blue ISG cells are used to measure the secretion of IFN-β into the culture supernatant following STING pathway activation. To run the assay, anti-IFN-β capture antibodies were coated on 96 well MultiArray plates (Mesoscale Discovery). After a one hour incubation, plates were washed and 50 μL supernatant from the STING human cell reporter assay plates or IFN-β standards were mixed with 20 μL Sulfotag-conjugated detection antibody in the coated plates. Plates were incubated, shaking for 2 h, washed, and read buffer was applied. Electrochemiluminescence was measured on the SectorImager.

STING cell signaling pathway assessment

Agonism of the STING pathway was measured in THP1 BLUE ISG cells by western blot of phospho-STING(S366), phospho-TBK1(S172) and phospho-IRF3(S396). Briefly, 5 million cells in 90 μL nucleofection buffer were mixed with 10 μL test compounds. These mixtures were electroporated using program V-001 on an Amaxa Nucleofector (Lonza). Cells were transferred into 12 well plates with fresh media and allowed to recover for one hour at 37 °C, 5% $CO_2$. Cells were then washed in cold HBSS and lysed in RIPA buffer. Samples were total protein normalized and either diluted in ProteinSimple sample buffer or LDS loading buffer. Samples were heat denatured at 95°C for 5 min, then PeggySue (ProteinSimple) was used to measure phospho- and total STING and IRF3 while the NuPAGE (Invitrogen) system was used to measure TBK1. Data was analyzed using Compass or Licor Odyssey software, respectively.

STING in vivo activity

For all studies, female Balb/c mice were obtained from Charles River Labs (Wilmington, MA) and used when they were 6-8 weeks of age and weighed approximately 20 g. All animals were allowed to acclimate and recover from any shipping-related stress for a minimum of 5 days prior to experimental use. Reverse osmosis chlorinated water and irradiated food (Laboratory Autoclavable Rodent Diet 5010, Lab Diet) were provided ad libitum, and the animals were maintained on a 12 h light and dark cycle. Cages and bedding were autoclaved before use and changed weekly. All experiments were carried out in accordance with The Guide for the Care and Use of Laboratory Animals and were approved by the Institutional Animal Care and Use Committee of Janssen R & D, Spring House, PA. Each experimental group contained 8 mice. In vivo efficacy in a mouse CT26 tumor model was determined by implanting 500,000 CT26 colon carcinoma tumor cells subcutaneously into Balb/c mice and allowing tumors to establish to 100-300 $mm^3$. Com-

pounds were injected intratumorally formulated in phosphate buffered saline in a volume of 0.1 mL per injection. Mice were administered 0.05 mg every three days for a total of three doses. Efficacy was measured as the percent tumor growth inhibition (TGI) calculated by the reduction in size of the Treated tumor volume (T) over the Control tumor volume (C) according to the following formula: ((C-T)/(C))* 100 when all control animals were still on study. Cures were defined as the number of animals with no measurable tumor detected 10 tumor volume doubling times (TVDT) after the last dose was administered.

The resultant data are presented in Table 2.

Table 2.

| Cpd No. | hSTING SPA IC50 (μM)* | human cell reporter EC50 (μM)* | SPR human STING KD (μM) | human IFN-β (ranking value) | In vivo activity (%TGI) | In vivo activity (cures) ** |
|---|---|---|---|---|---|---|
| **c-GAMP** | 0.024 | 0.53 | 0.0055 | - | - | - |
| **3', 3'cGMP** | 8.62 | 8.7 | 3.97 | - | - | - |
| **3', 3'cAMP** | 73.06 | 17.83 | 5.99 | 539 | - | - |
| **1** | 0.0046 | 0.077 | 0.0012 | 7930 | 108.9 | 6 |
| **6** | 0.011 | 0.99 | 0.0012 | 6762 | - | - |
| **8** | 11.68 | 2.38 | - | 5539 | - | - |
| **9** | 2.79 | 0.11 | 0.28 | 8104 | - | - |
| **11** | 0.015 | 0.03 | 0.0065 | 14896 | - | - |
| **12** | 0.22 | 0.14 | - | - | - | - |
| **15** | 0.36 | 0.043 | - | 15611 | - | |
| **17** | 21.58 | 0.25 | 5.14 | 17298 | - | - |
| **18** | 1.14 | 0.11 | - | 15249 | - | - |
| **20** | 0.047 | -0.22 | 0.0082 | 6045 | - | - |
| **22** | 0.092 | 0.026 | - | - | - | - |
| **24** | 5 | 0.011 | - | 11534 | - | - |
| **25** | 29.2 | 0.1 | 3.48 | 6595 | 88.9 | - |
| **26** | 28.41 | 1.02 | - | - | - | - |
| **28** | 0.19 | 0.099 | - | 12969 | - | - |
| **29** | 0.29 | 0.053 | | 16254 | - | - |
| **30** | >100 | 13.05 | - | - | - | - |
| **33** | 0.39 | 0.17 | - | - | - | - |
| **34** | 4.26 | 0.025 | - | - | - | - |
| **35** | >100 | 7.08 | - | - | - | - |
| **36** | >100 | 2.46 | - | - | - | - |
| **37** | 12.36 | 1.35 | - | - | - | - |
| **39** | 0.021 | 0.065 | - | - | - | - |
| **40** | 0.066 | 0.038 | - | - | - | - |
| **41** | >100 | 22.53 | - | - | - | - |
| **45** | 76.17 | 1.84 | - | - | - | - |
| **47** | 1.3 | -0.039 | - | - | - | - |

(continued)

| Cpd No. | hSTING SPA IC50 (μM)* | human cell reporter EC50 (μM)* | SPR human STING KD (μM) | human IFN-β (ranking value) | In vivo activity (%TGI) | In vivo activity (cures) ** |
|---|---|---|---|---|---|---|
| **48** | >100 | 6.66 | - | - | - | - |
| **49** | 37.09 | 0.26 | - | - | - | - |
| **51** | 0.011 | - | - | - | - | - |
| **52** | 0.32 | - | - | 18191 | - | - |
| **53** | 4.01 | - | - | 11036 | - | - |
| **54** | 0.75 | - | - | 18578 | - | - |
| **55** | 0.22 | - | - | 25628 | - | - |

human IFN-β ranking value was determined by total cumulative IFN-β induction in pg/ml over the dose range tested (0.78 to 50uM) in THP-1 cells after a 24 hour exposure
* $IC_{50}$ and $EC_{30}$ are means of at least three values.
** 8 mice per group
( - ) not done,

Biological Example 2

STING primary human PBMC cytokine induction assay

Agonism of the human STING pathway is assessed in primary human peripheral blood mononuclear cells (PBMC) derived from human whole blood. 1 pint (approximately 420 mL) of fresh donor blood (AllCells Inc., Alameda, CA) is layered over Lymphocyte Separation Medium (1.077-1.080 g/mL, Corning, Manassas, VA), then centrifuged at 500g for 20 min at RT without applying break. The PBMC collected at the interface between serum and Lymphocyte Separation Medium are harvested, washed, then counted. PBMC are composed of subtypes of lymphocytes and monocytes, such as B cells, T cells, etc., and these subtypes have been characterized in the literature to express different levels of the STING protein. In response to STING agonists, such as 2'3'-cGAMP, these cells become activated and are induced to express a variety of proinflammatory and antiviral cytokines. Also, upon stimulation with STING agonists, these cells upregulate activation markers. The levels of cytokine induction can be measured by a variety of methods including ELISA, Luminex and MSD. The levels of activation marker upregulation can be measured by flow cytometry.

To run the assay, 1,000,000 cells may be dispensed into 225 μL/well of flat-bottom, tissue culture treated, 96-well plates. Test compounds may be added in a volume of 25 μL at 10x concentration. Some compounds may be solubilized in 100% DMSO and the final concentration of DMSO in the cultures receiving these compounds may be 1%. The assay may be incubated for 48 h at 37 °C, 5% $CO_2$. 200 μl of supernatants may be harvested without disturbing cells on the bottom of the plate, then frozen at -20 °C until time of Luminex measurement Luminex assays may be performed using G-CSF, IFNα2, IFNγ, IL-1b, IL-6, IL-10, IL-12 (p40), IL-12 (p70), TNFa from MILLIPLEX MAP Human Cytokine/Chemokine Magnetic Bead Panel - Immunology Multiplex Assay kit and IFNβ1 analyte from MILLIPLEX MAP Human Cytokine/Chemokine Magnetic Bead Panel IV kit (EMD Millipore, Billerica, MA), following the manufacturer's protocol. Cytokine induction may be measured using a Luminex FlexMAP 3D® instrument (Luminex Corporation, Radnor, PA). Analysis of collected Luminex data may be performed using MILLIPLEX Analyst software (EMD Millipore).

Suppression of HBV virus in PHH cells using conditioned media from STING activated primary human PBMC

Primary human hepatocytes can be infected with hepatitis B virus and during an established infection, will produce viral proteins such as HBsAg and HBeAg that can be detected by ELISA. Therapeutic treatment with compounds such as entecavir can suppress HBV reproduction, which can be measured by decreased viral protein production. (# of cells) $4\times10^5$ cells/well primary human hepatocytes (BioReclamation, Westbury, NY) may be dispensed into 500 μL/well of flat-bottom, tissue culture treated, 24-well plates. 24 h later, cells may be infected with 30-75 moi of HBV. On the next day, the PHH may be washed 3x and fresh maintenance media may be added to the cells. Concurrently, PBMC may be isolated as described previously. To stimulate the PBMC, 10,000,000 cells may be dispensed into 400 μL/well of flat-bottom, tissue culture treated, 24-well plates. Test compounds may be added in a volume of 100 μL, then the cultures may be incubated for 48 h at 37 °C, 5% $CO_2$. Supernatants may be harvested. Cells may be measured for activation

marker upregulation using flow cytometery. Briefly, cells may be stained with fluorescently labeled antibodies directed to CD56, CD19, CD3, CD8a, CD14, CD69, CD54, CD161, CD4 and CD80. Samples may be analyzed on an Attune NxT flow cytometer (Thermo Fisher, Carlsbad, CA)

From the stimulated PBMC cultures, a portion of supernatant may be reserved for cytokine detection by Luminex, as described previously. The rest of the supernatant may be divided in half, and one aliquot may be stored at 4°C for use on d8 of the assay. The other aliquot of supernatant may be diluted 1:1 with 2X PHH media, then may be added to the d4 infected PHH cells. After 96 h, the spent media may be changed and supernatant may be added at a dilution of 1:1 with 2X PHH media. At this point an interim measurement of HBsAg may be performed using an HBsAg ELISA kit (Wantai Bio-pharm, Beijing, China). Following 96 h, the media may be collected and HBsAg may be measured.

While the foregoing specification teaches the principles of the present invention, with examples provided for the purposes of illustration, it will be understood that the practice of the invention encompasses the usual variations, adaptations and/or modifications as come within the scope of the following claims

## Claims

1. A compound of Formula (I)

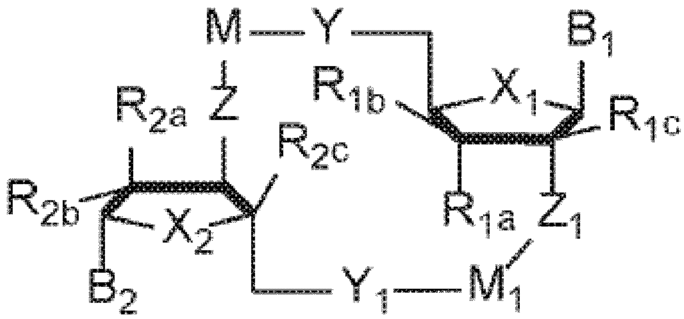

Formula (I)

which is a compound selected from the group consisting of the following compounds:

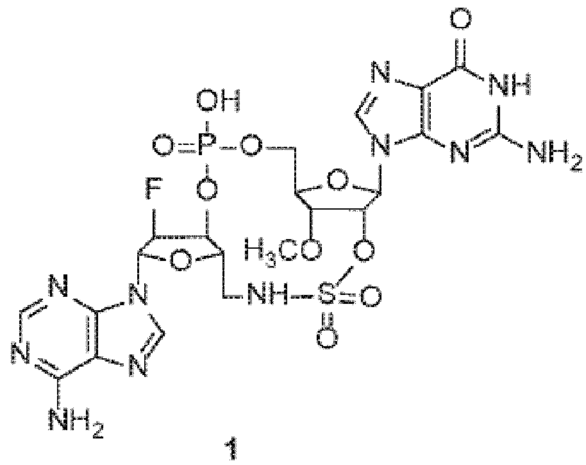

1

,

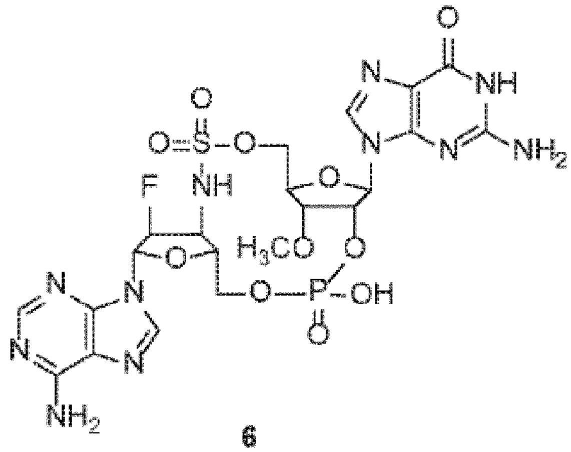

6

,

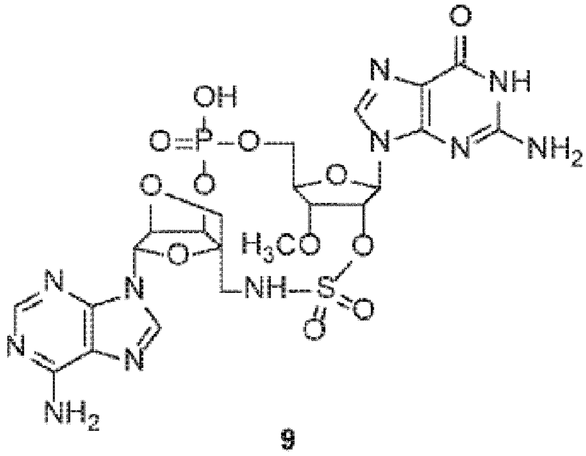

9

,

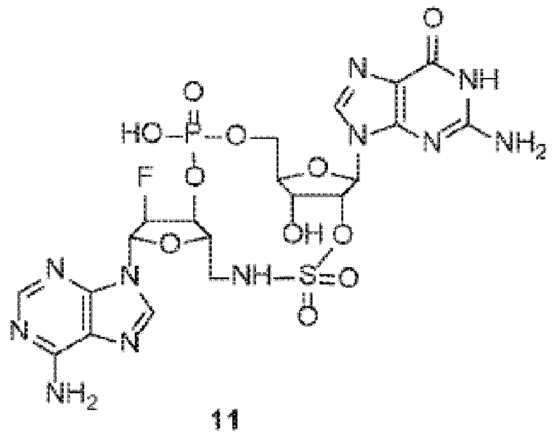

11

,

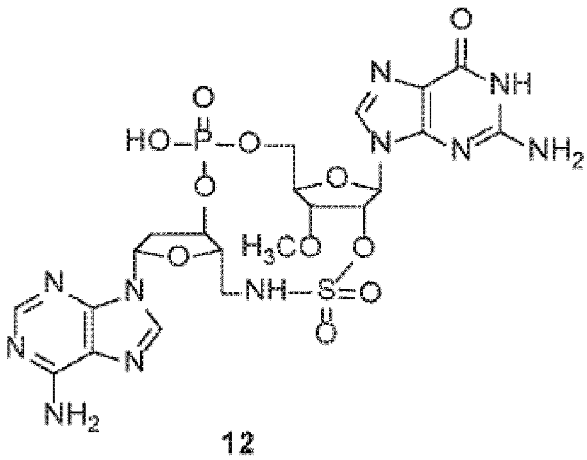

12

,

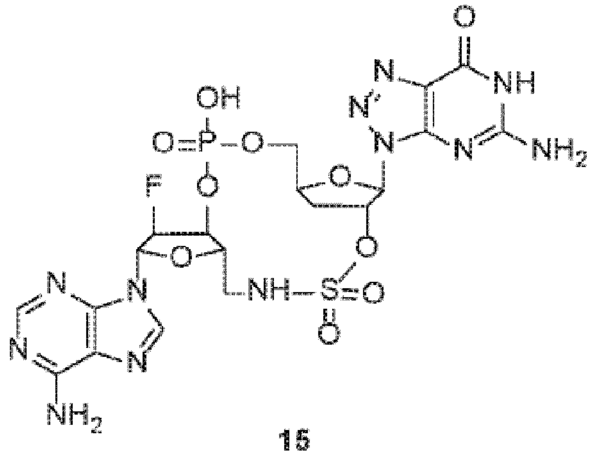

15

,

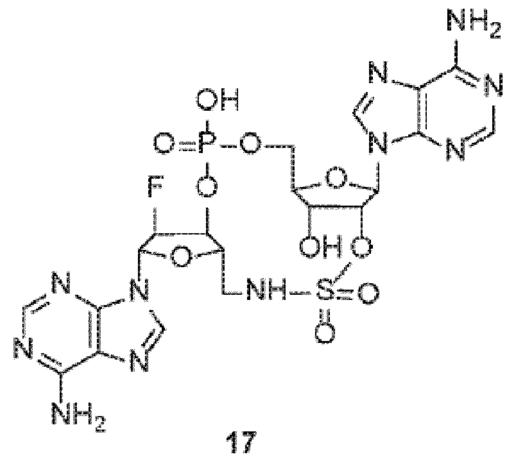

17

,

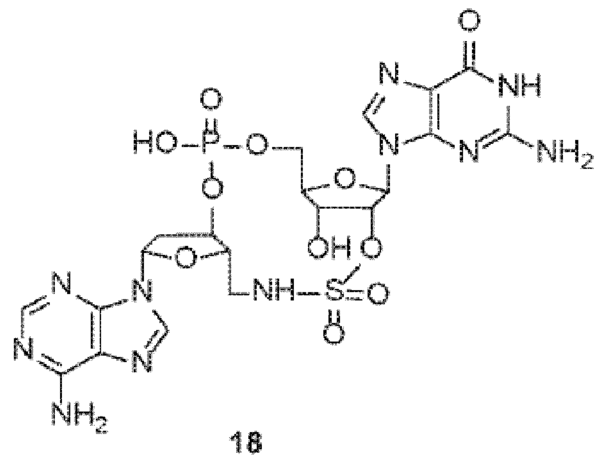

18

,

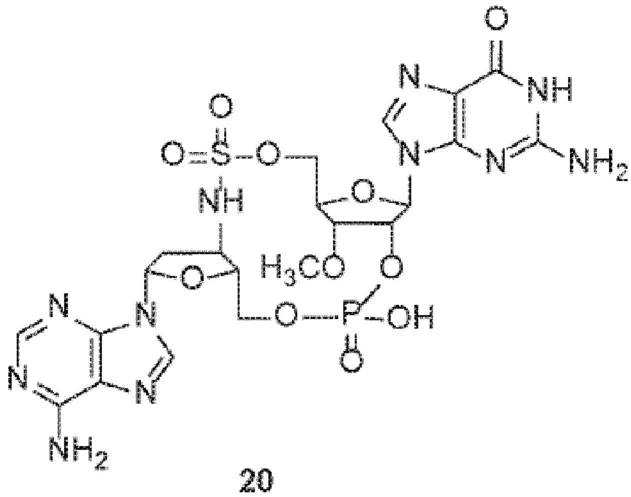

20

,

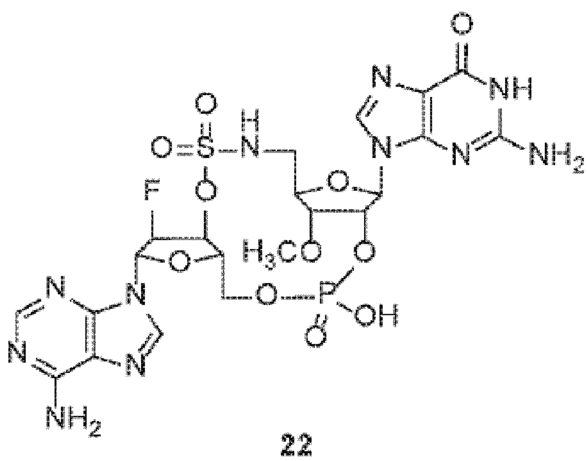

22

,

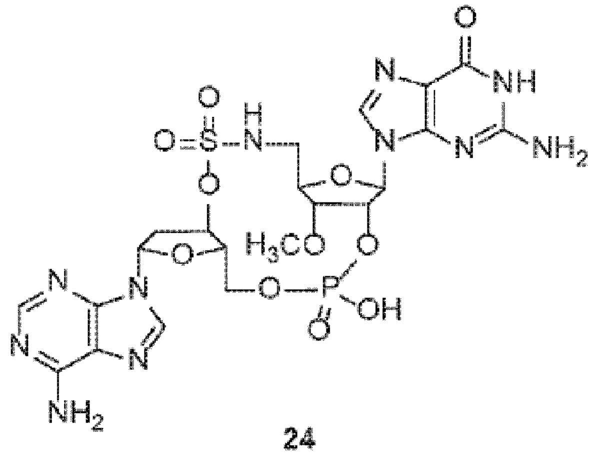

24

,

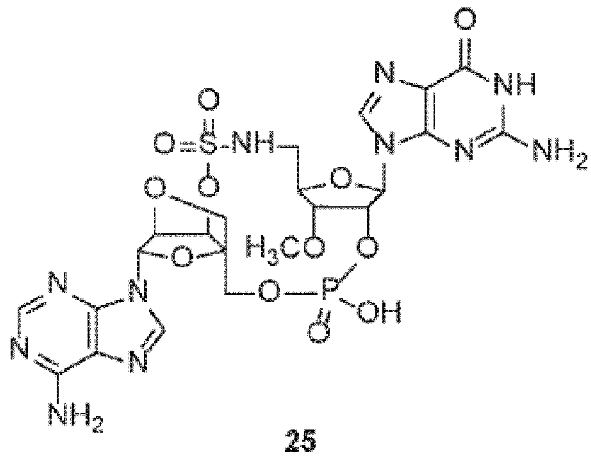

25

,

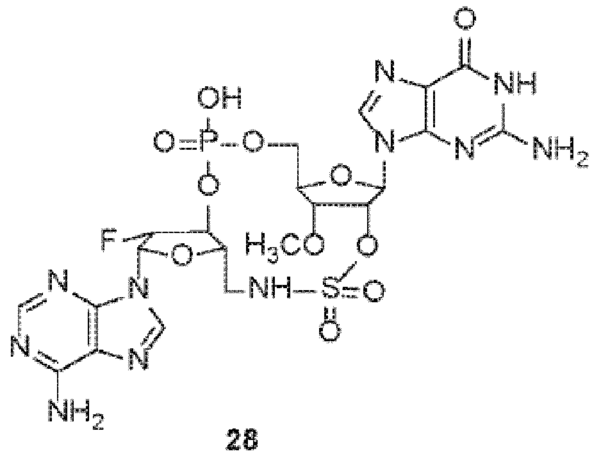

28

,

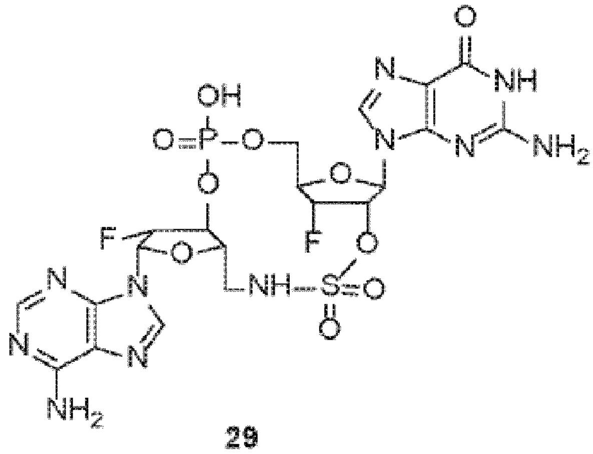

29

,

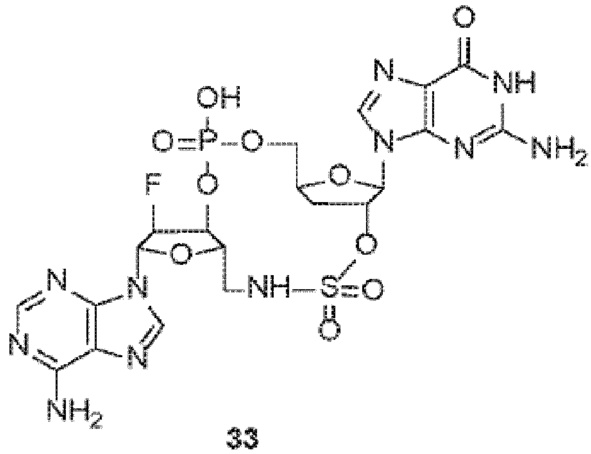

33

,

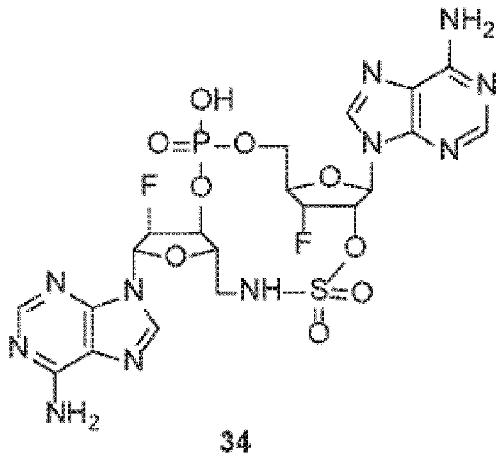

34

,

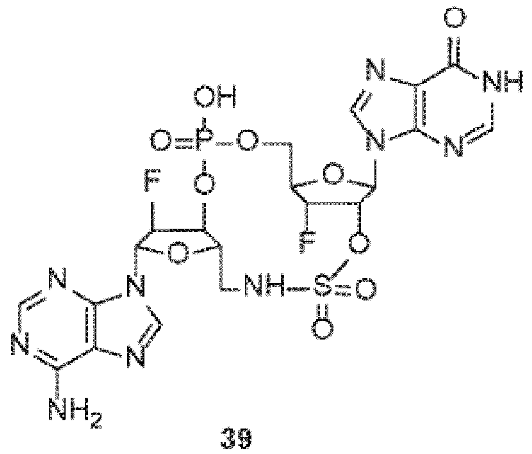

39

,

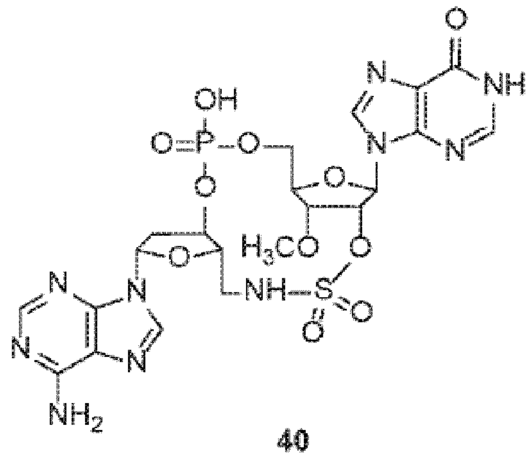

40

,

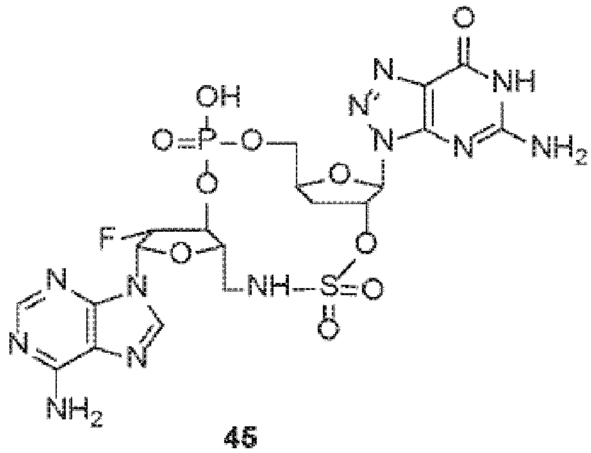

45

,

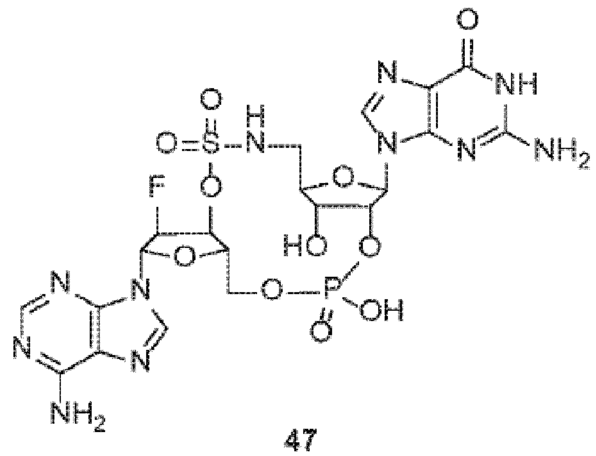

47

,

and

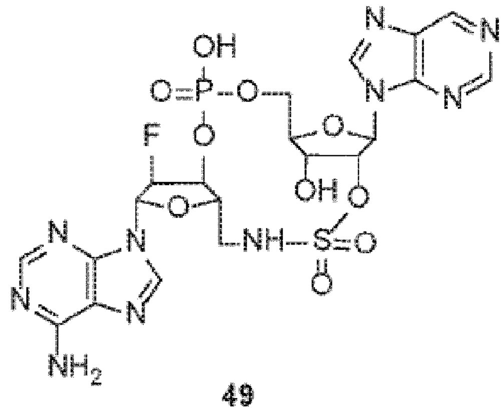

49

,

or a pharmaceutically acceptable salt form thereof.

**2.** The compound of claim 1 selected from the group consisting of

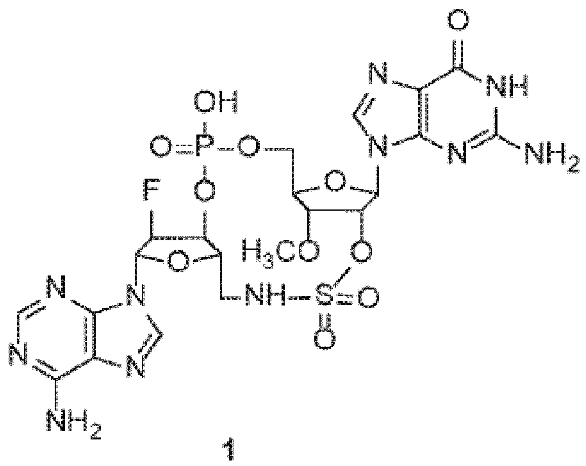

1

,

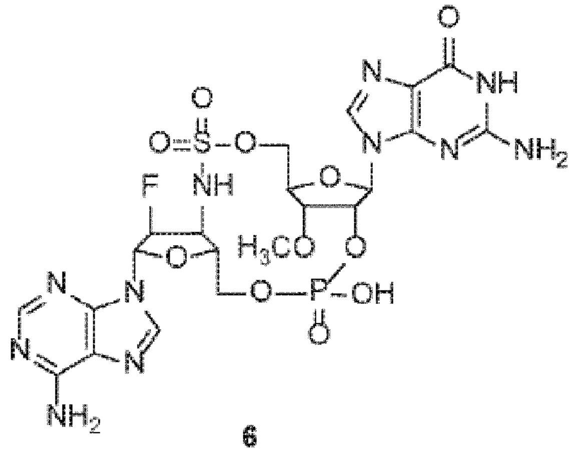

6

,

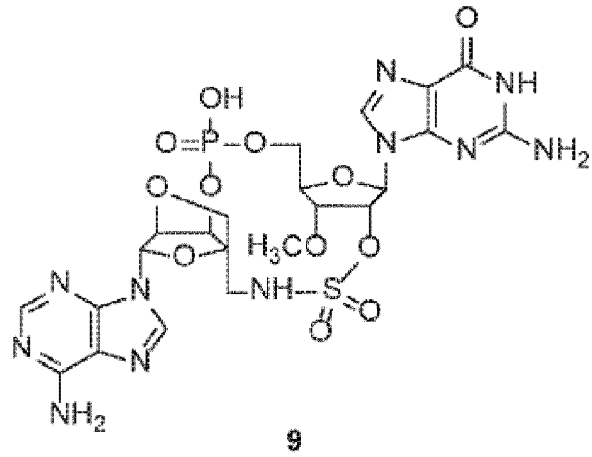

9

,

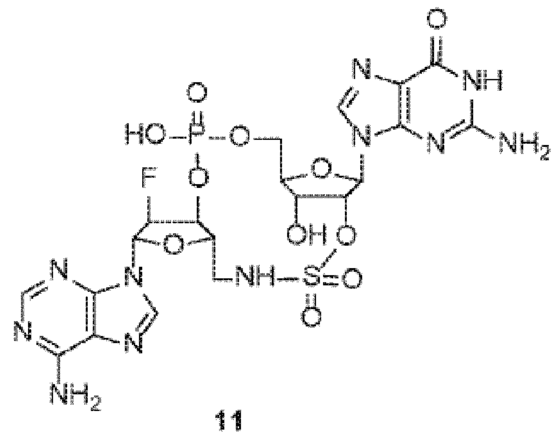

11

,

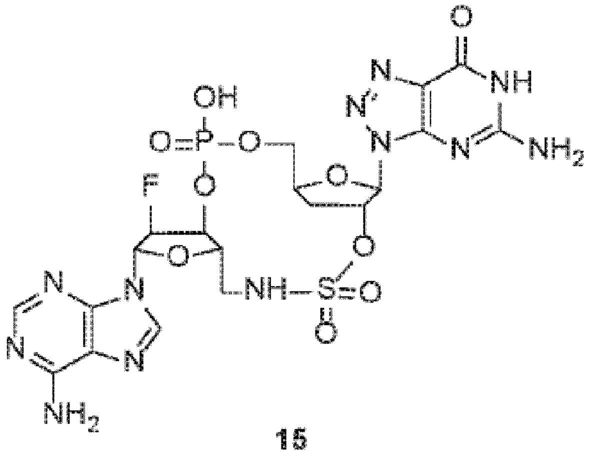

15

,

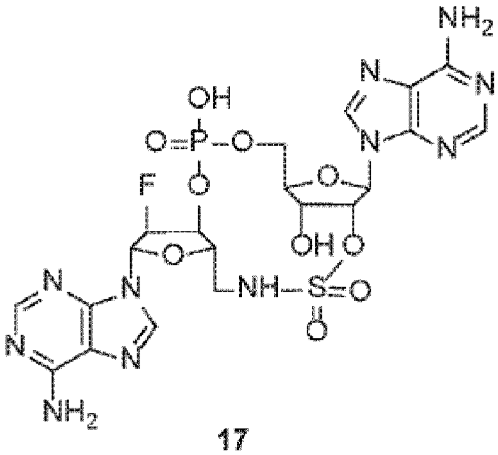

17

,

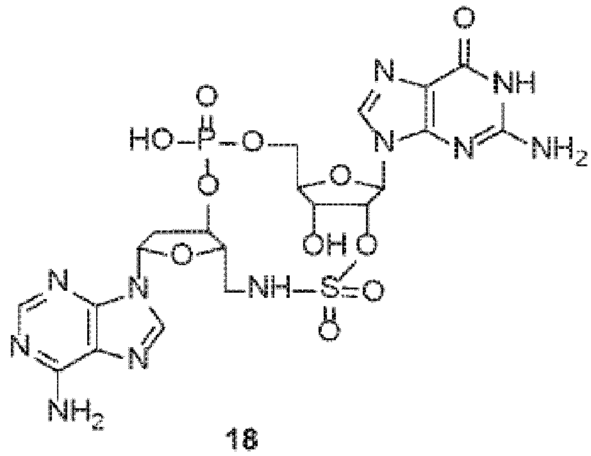

18

,

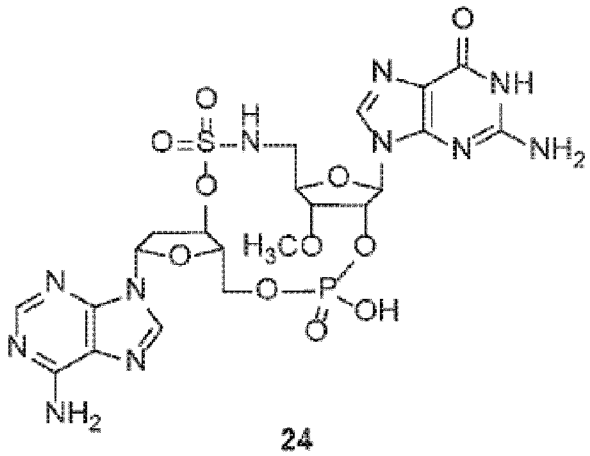

24

,

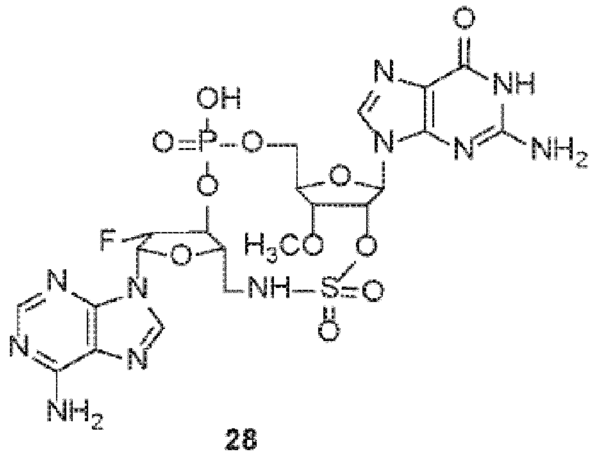

28

, and

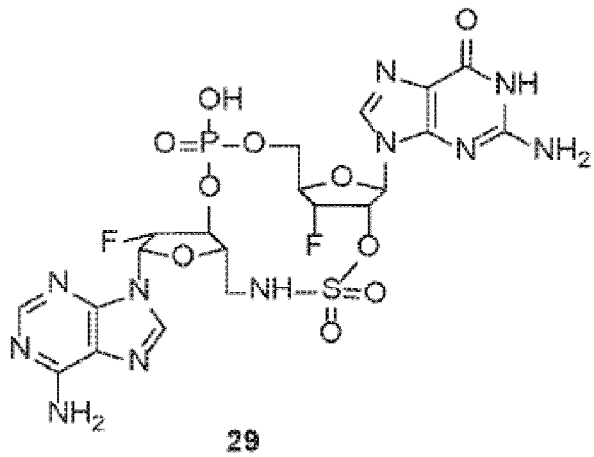

29

,

or a pharmaceutically acceptable salt form thereof.

3. A pharmaceutical composition comprising a compound of claim 1 or claim 2 and at least one of a pharmaceutically acceptable carrier, a pharmaceutically acceptable excipient, and a pharmaceutically acceptable diluent.

4. The pharmaceutical composition of claim 3, wherein the composition is a solid oral dosage form.

5. The pharmaceutical composition of claim 3, wherein the composition is a syrup, an elixir or a suspension.

**6.** The compound of claim 1 or 2 for use in treating a disease, syndrome, or condition modulated by STING.

**7.** The compound of claim 1 or 2 for use in treating a disease, syndrome, or condition, wherein said disease, syndrome, or condition is affected by the agonism of STING.

**8.** The compound for use according to claim 6 or claim 7, wherein said disease, syndrome, or condition is cancer.

**9.** The compound for use according to claim 8, wherein said cancer is melanoma, colon cancer, breast cancer, prostate cancer, lung cancer, or fibrosarcoma.

**10.** The compound for use according to claim 7, wherein said disease, syndrome, or condition is a viral infection.

**11.** The compound for use according to claim 10, wherein the viral infection is hepatitis B.

**12.** The compound of claim 1 or claim 2, and an oncolytic virus or anti-cancer vaccine for use in treating a disease, syndrome, condition, or disorder, wherein said disease, syndrome, condition, or disorder is affected by the agonism of STING.

**13.** The compound for use according to claim 12, wherein the anti-cancer vaccine is an antigen vaccine, whole cell vaccine, dendritic cell activating vaccine, DNA vaccine, Bacillus Calmette-Guérin (BCG) vaccine, Sipuleucel-T (Provenge), Talimogene laherparepvec (T-Vec; Imlygic™), oncolytic virus based vaccine, or adenovirus based vaccine.

**Patentansprüche**

**1.** Verbindung der Formel (1),

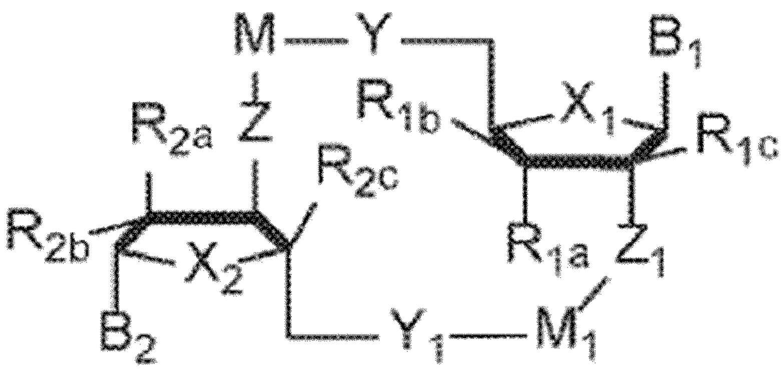

Formel (1)

welche eine Verbindung ist, ausgewählt aus der Gruppe bestehend aus den folgenden Verbindungen:

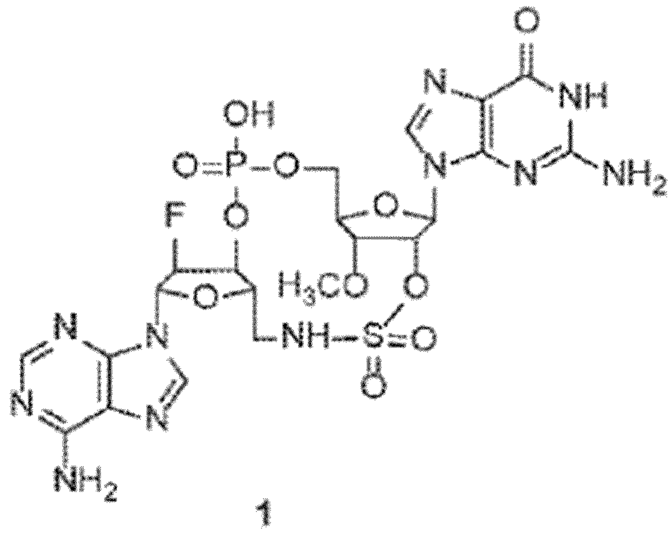

1

,

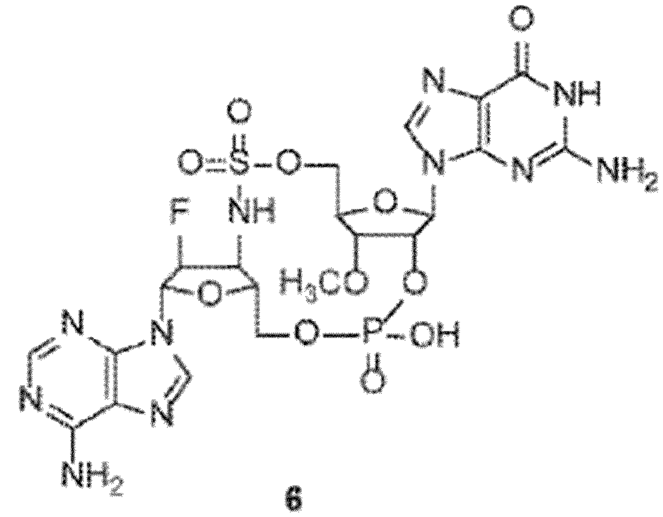

6

,

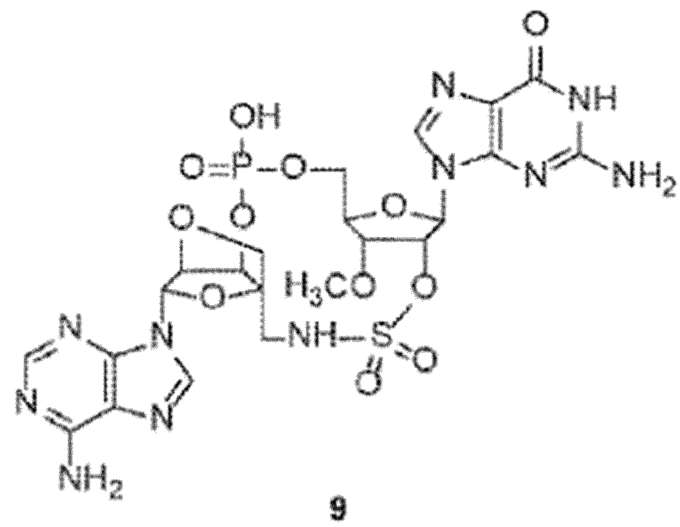

9

,

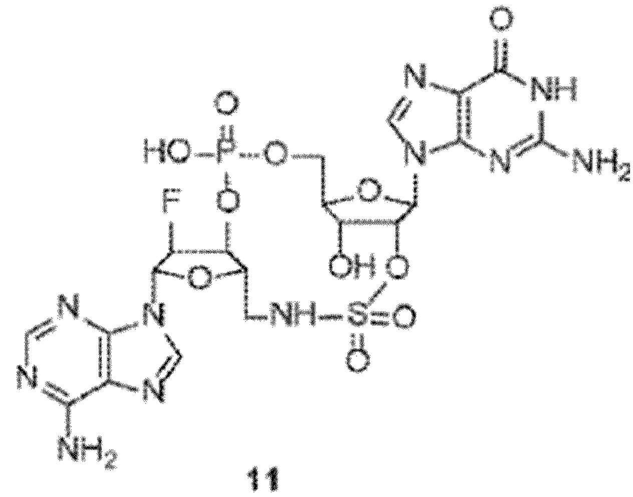

**11**

,

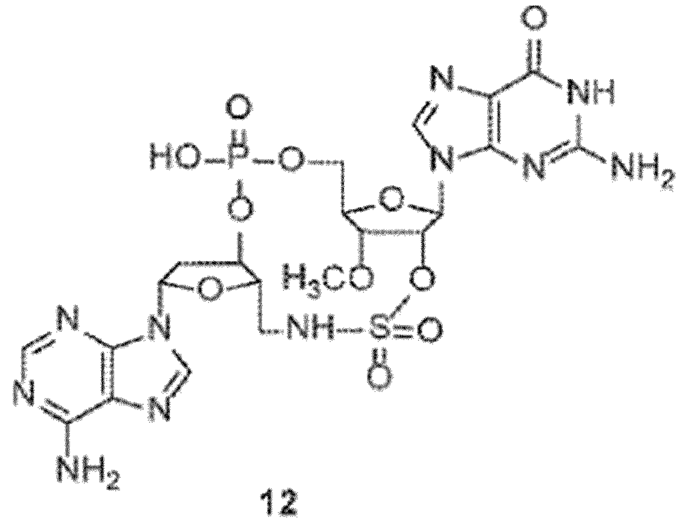

**12**

,

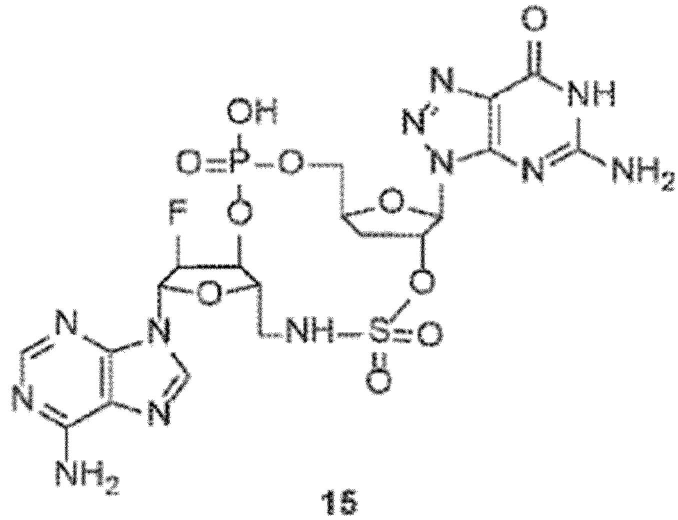

**15**

,

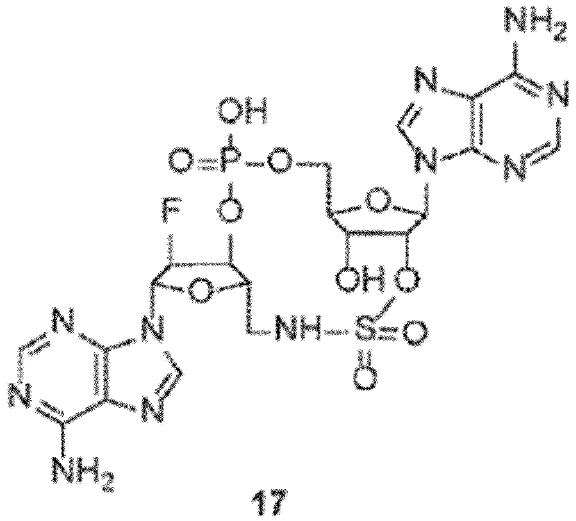

17

,

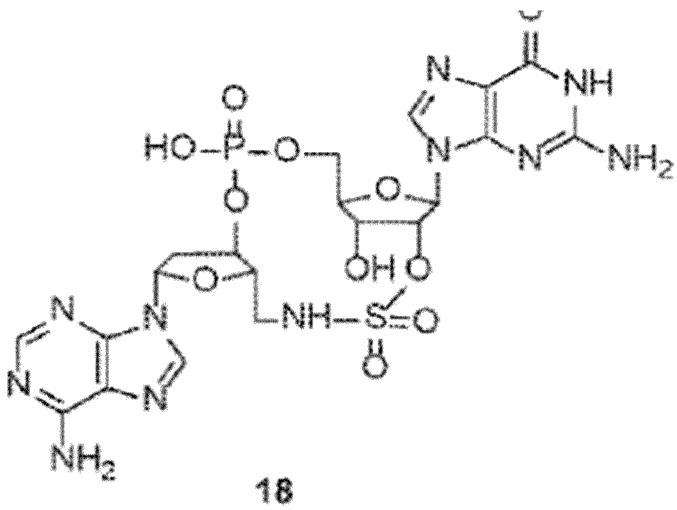

18

,

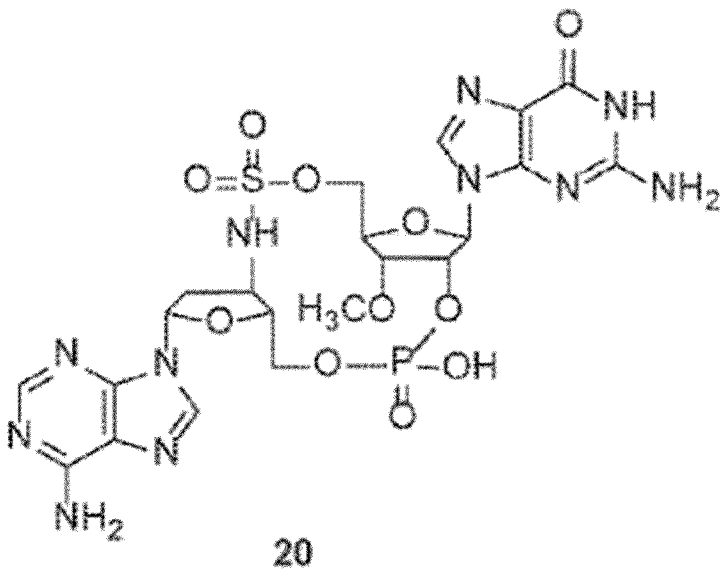

20

,

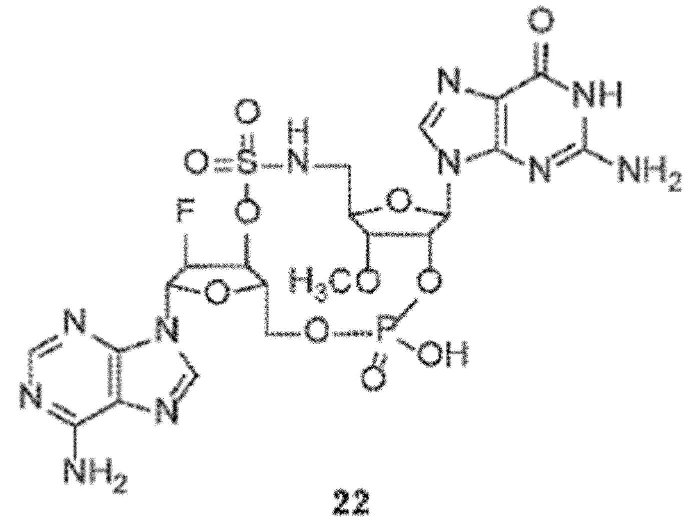

22

,

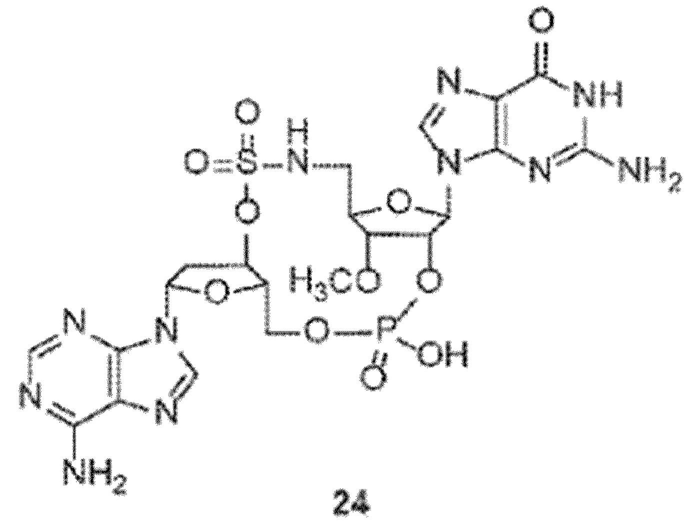

24

,

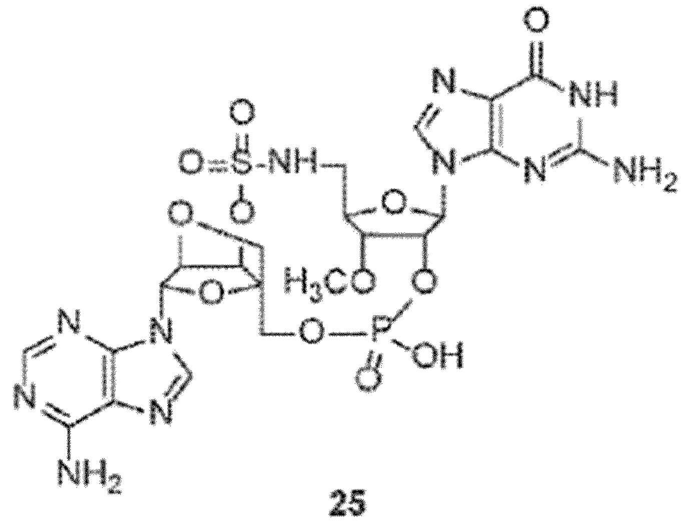

25

,

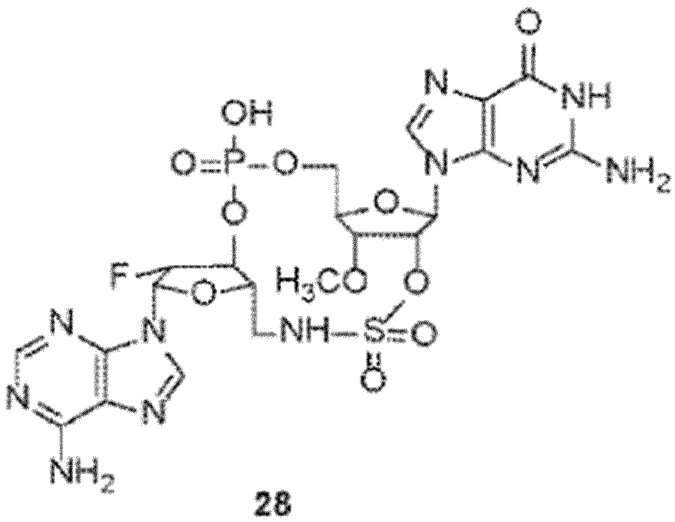

28

,

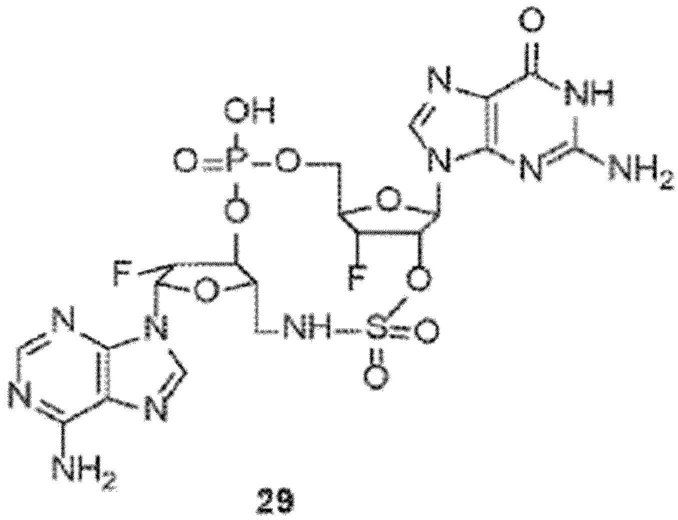

29

,

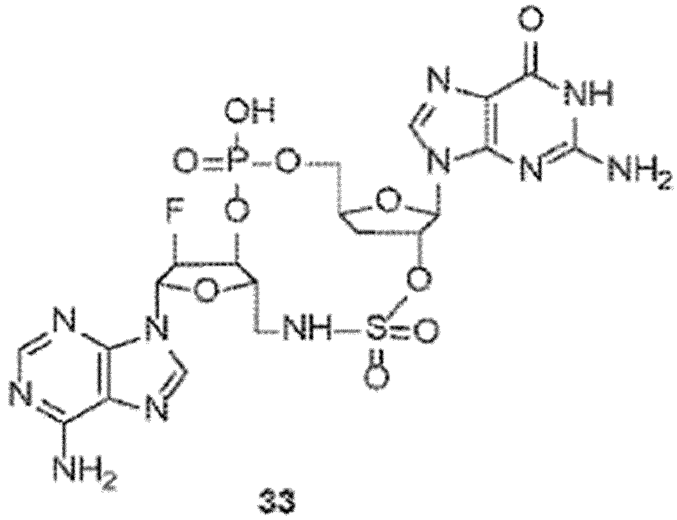

33

,

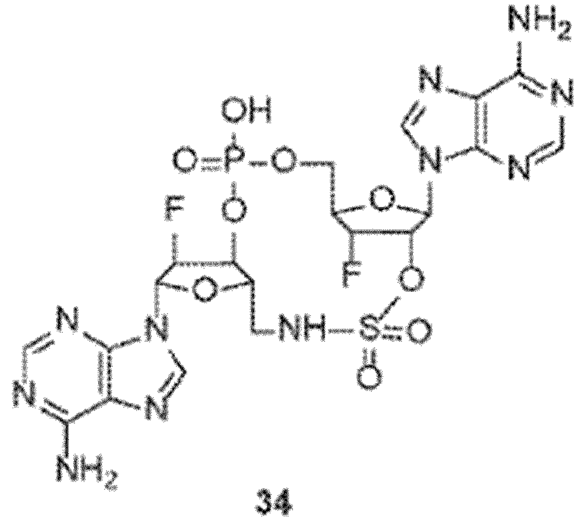

34

,

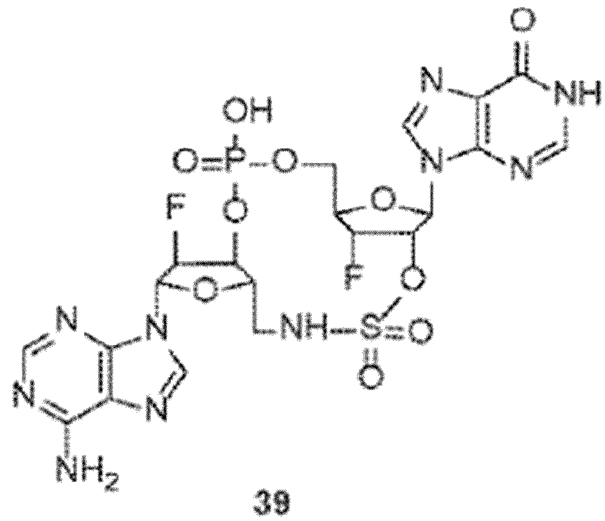

39

,

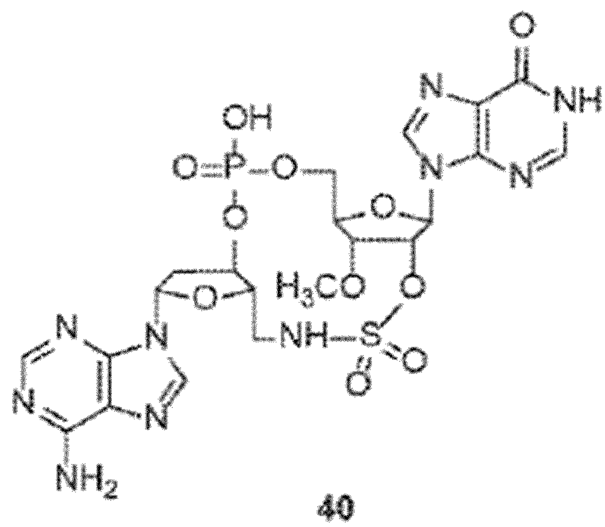

40

,

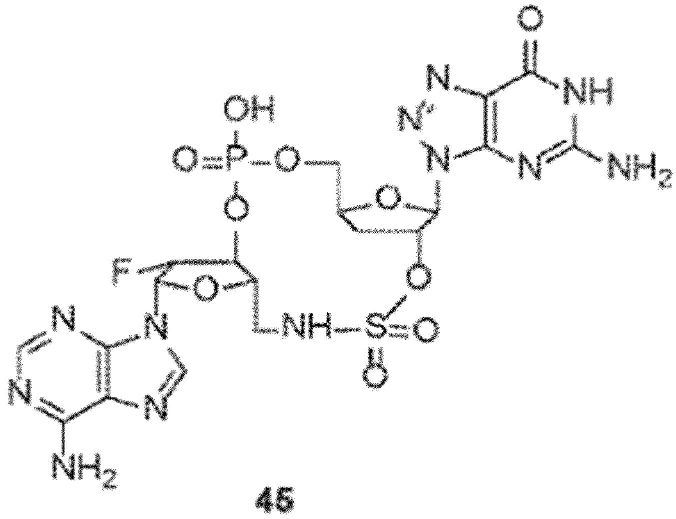

45

,

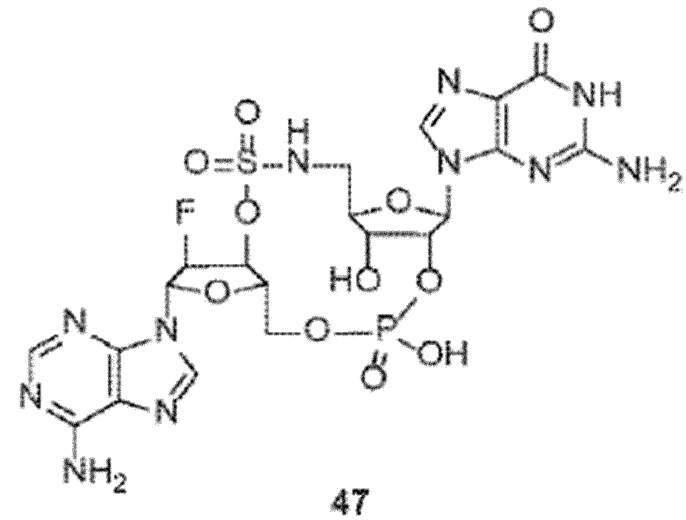

47

und

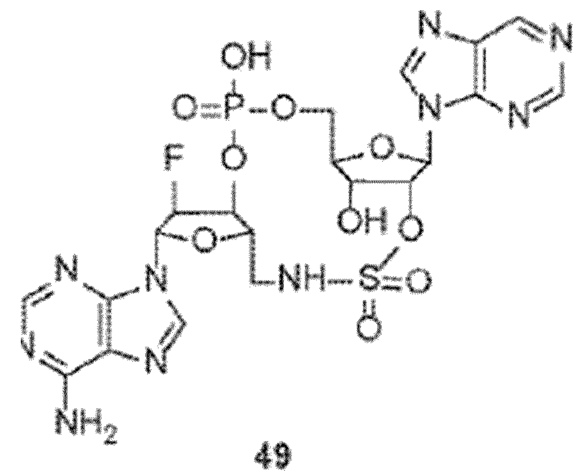

49

oder eine pharmazeutisch annehmbare Salzform davon.

**2.** Verbindung nach Anspruch 1, ausgewählt aus der Gruppe bestehend aus:

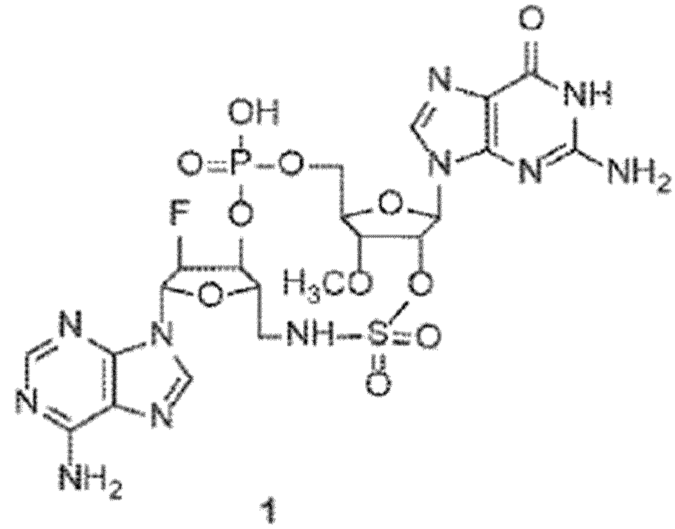

1

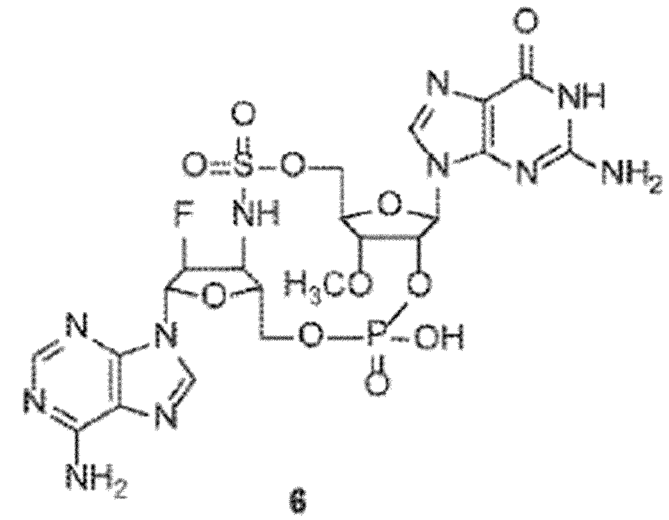

6

,

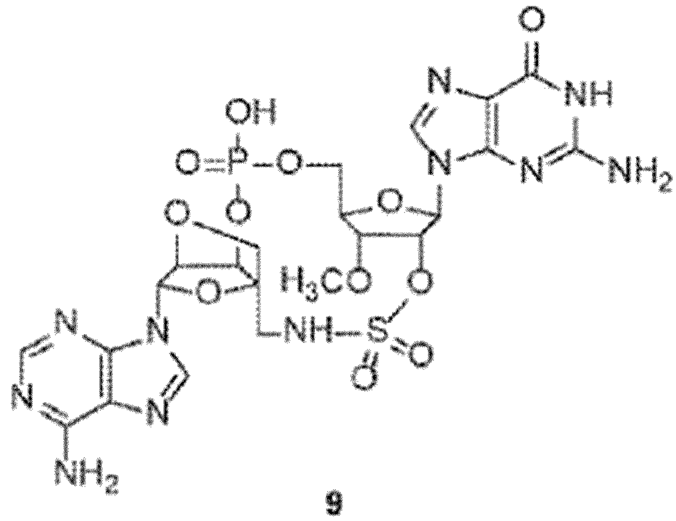

9

,

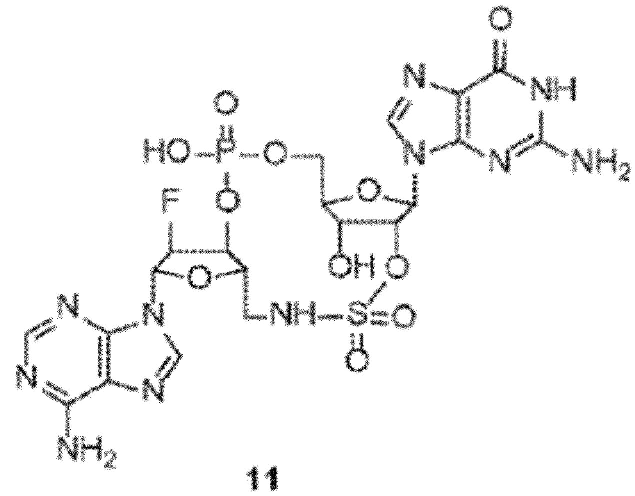

11

,

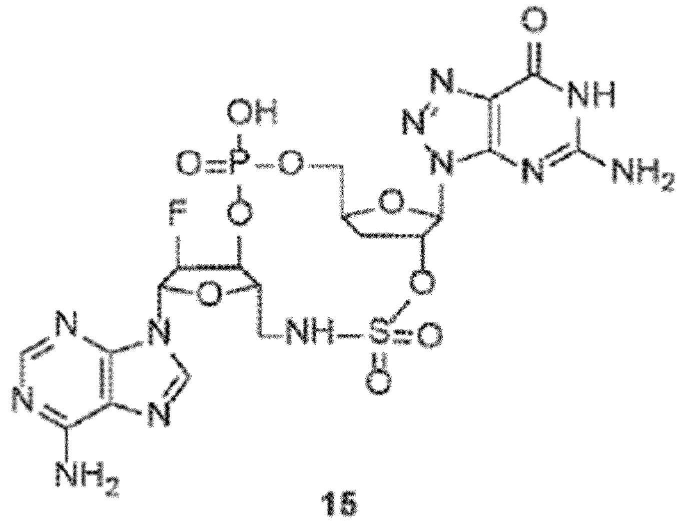

15

,

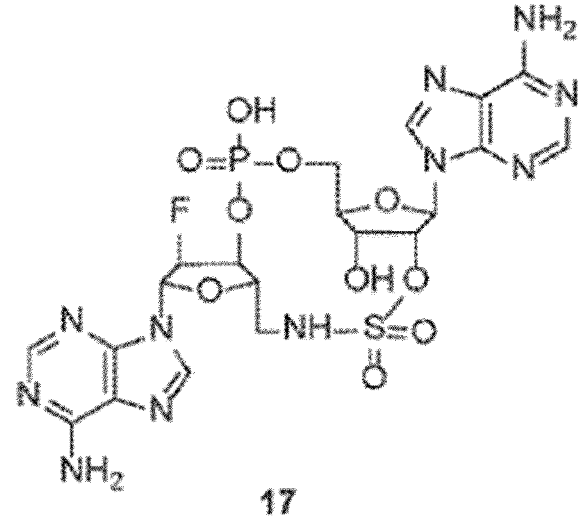

17

,

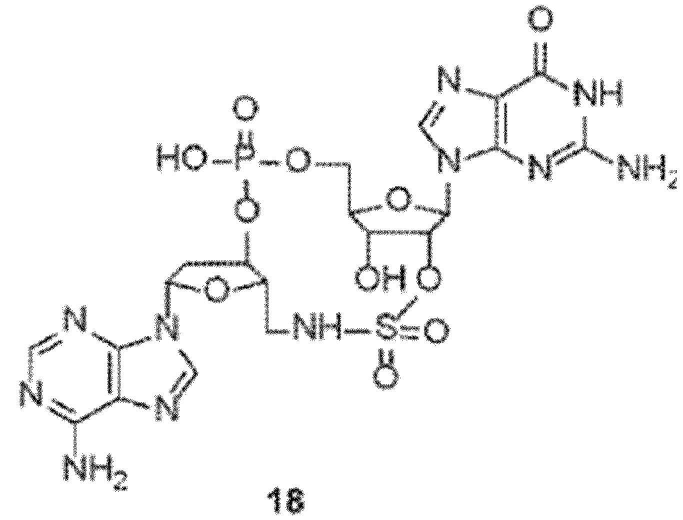

18

,

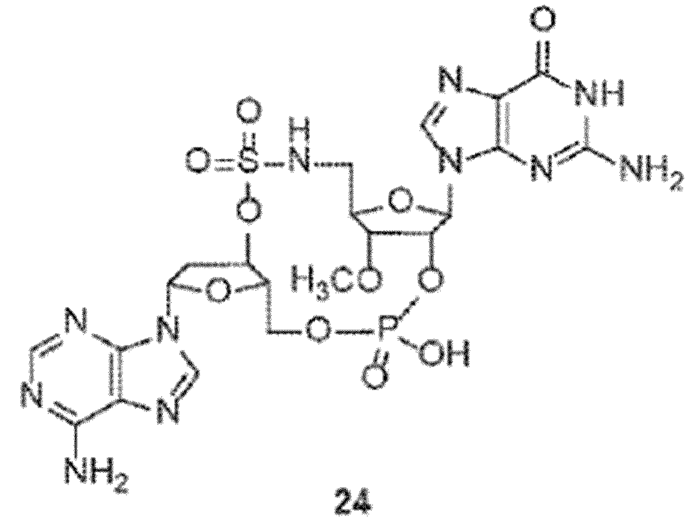

24

,

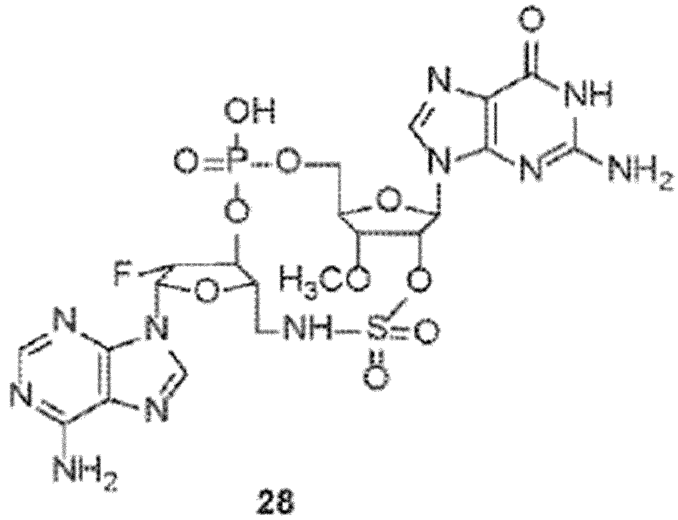

28

und

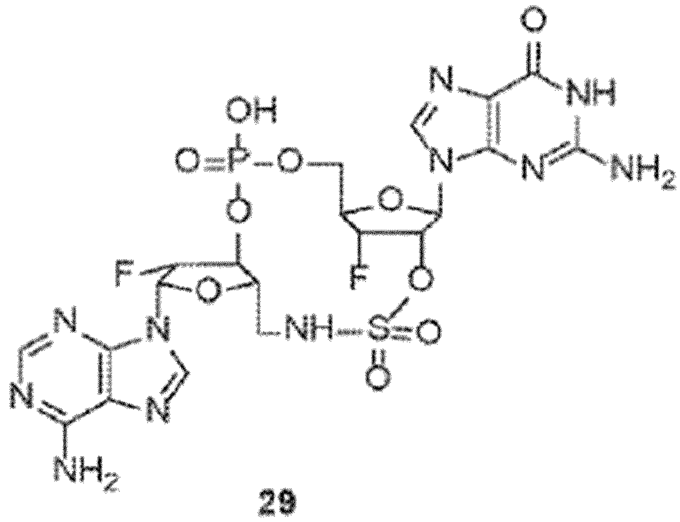

**29**

oder eine pharmazeutisch annehmbare Salzform davon.

3. Pharmazeutische Zusammensetzung, umfassend eine Verbindung nach Anspruch 1 oder 2 und einen pharmazeutisch annehmbaren Träger, einen pharmazeutisch annehmbaren Hilfsstoff und/oder einen pharmazeutisch annehmbaren Verdünner.

4. Pharmazeutische Zusammensetzung nach Anspruch 3, wobei die Zusammensetzung eine feste orale Dosierungsform ist.

5. Pharmazeutische Zusammensetzung nach Anspruch 3, wobei die Zusammensetzung ein Sirup, ein Elixier oder eine Suspension ist.

6. Verbindung nach Anspruch 1 oder 2 zur Verwendung bei der Behandlung einer Krankheit, eines Syndroms oder eines Zustands, moduliert durch STING.

7. Verbindung nach Anspruch 1 oder 2 zur Verwendung bei der Behandlung einer Krankheit, eines Syndroms oder eines Zustands, wobei die Krankheit, das Syndrom oder der Zustand durch den Agonismus von STING beeinflusst wird.

8. Verbindung zur Verwendung nach Anspruch 6 oder 7, wobei die Krankheit, das Syndrom oder der Zustand Krebs ist.

9. Verbindung zur Verwendung nach Anspruch 8, wobei der Krebs Melanom, Dickdarmkrebs, Brustkrebs, Prostatakrebs, Lungenkrebs oder Fibrosarkom ist.

10. Verbindung zur Verwendung nach Anspruch 7, wobei die Krankheit, das Syndrom oder der Zustand eine virale Infektion ist.

11. Verbindung zur Verwendung nach Anspruch 10, wobei die Virusinfektion Hepatitis B ist.

12. Verbindung nach Anspruch 1 oder 2 und ein Impfstoff gegen ein onkolytisches Virus oder einen Krebs zur Verwendung bei der Behandlung einer Krankheit, eines Syndroms, eines Zustands oder einer Störung, wobei die Krankheit, das Syndrom, der Zustand oder die Störung durch den Agonismus von STING beeinflusst wird.

13. Verbindung zur Verwendung nach Anspruch 12, wobei der Antikrebsimpfstoff ein Antigenimpfstoff, Ganzzellimpfstoff, ein dendritische Zellen aktivierender Impfstoff, ein DNA-Impfstoff, ein BCG(Bacillus Calmette-Guérin)-Impfstöff, Sipuleucel-T (Provenge), Talimogen laherparepvec (T-Vec; Imlygic™), ein auf onkolytischen Viren basierender Impfstoff oder ein auf Adenovirus basierender Impfstoff ist.

## Revendications

1. Composé de formule (I)

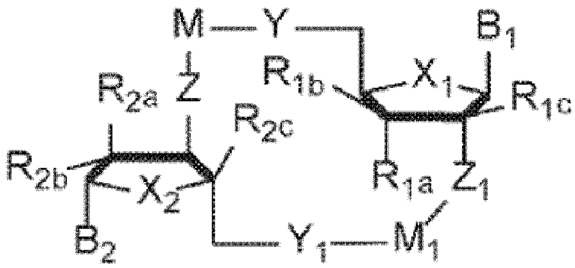

formule (1)

qui est un composé choisi dans le groupe constitué par les composés suivants :

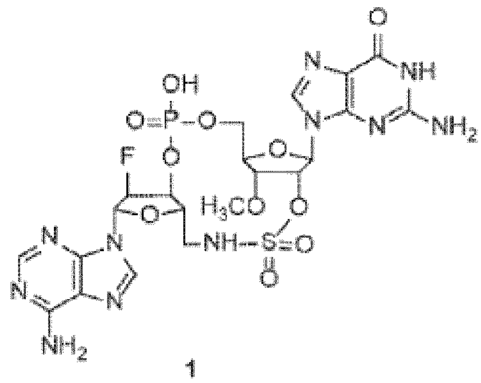

1

,

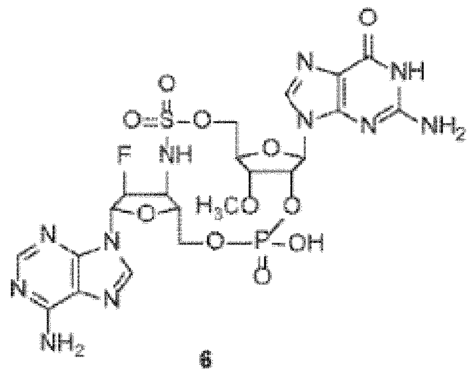

6

,

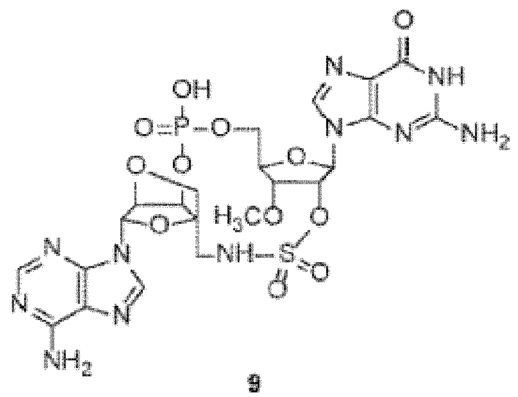

9

,

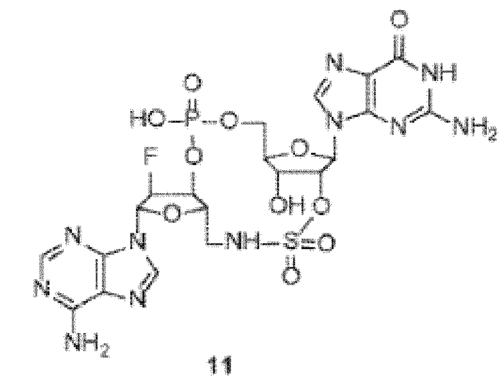

11

,

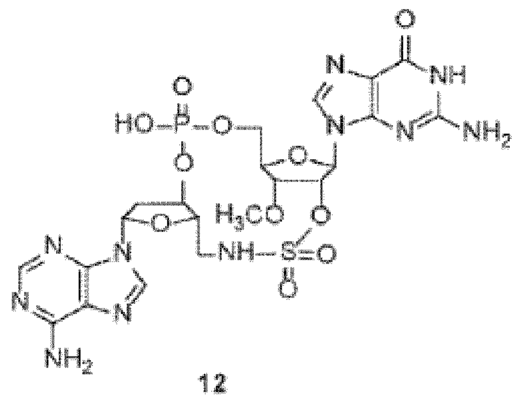

12

,

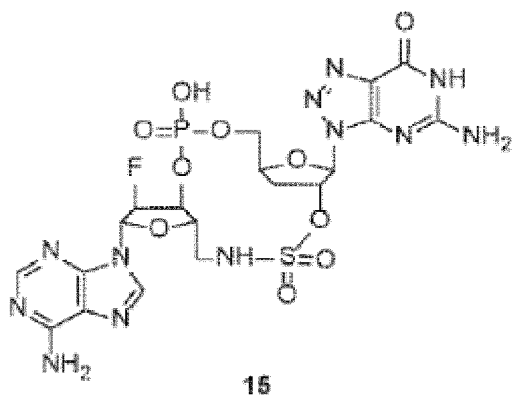

15

,

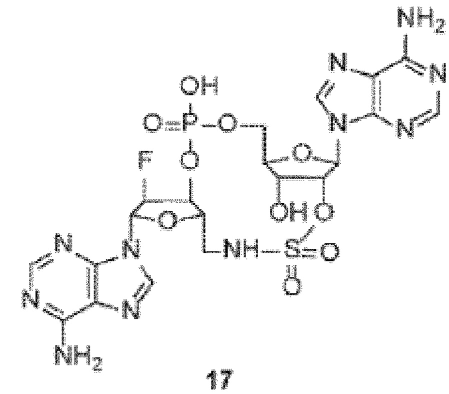

17

,

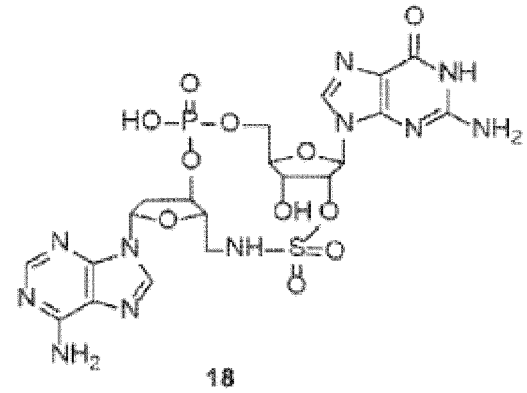

18

,

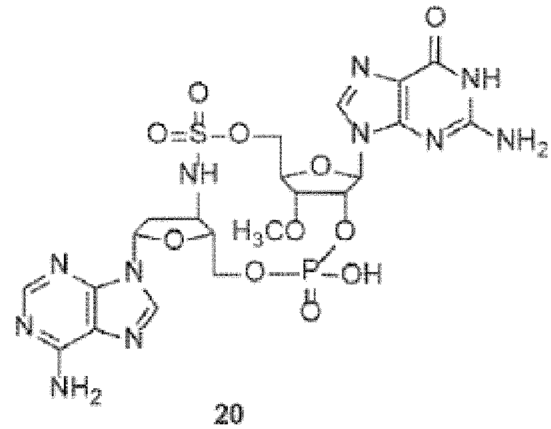

20

,

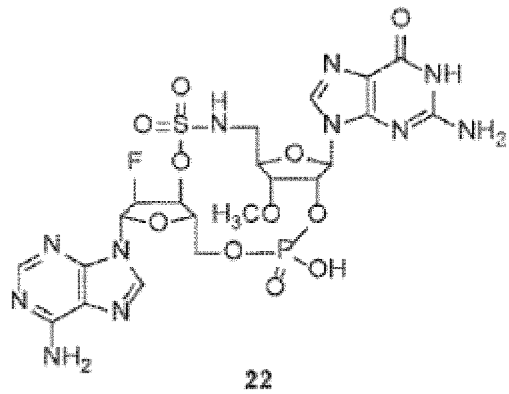

22

,

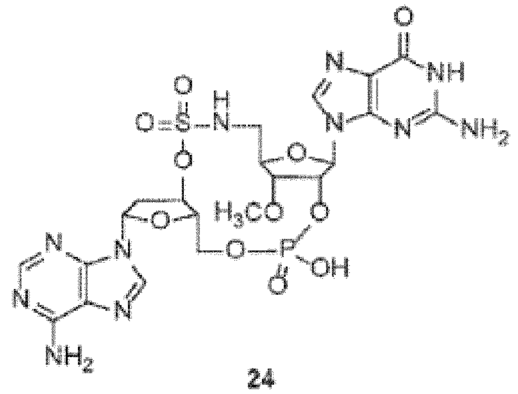

24

,

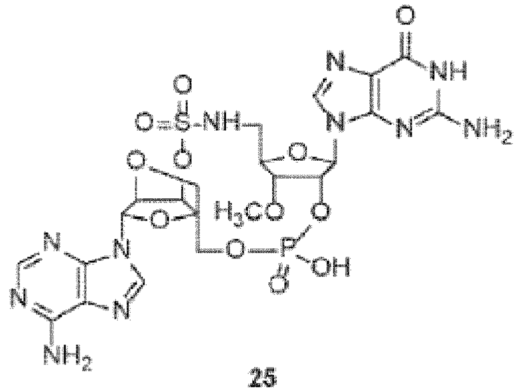

25

,

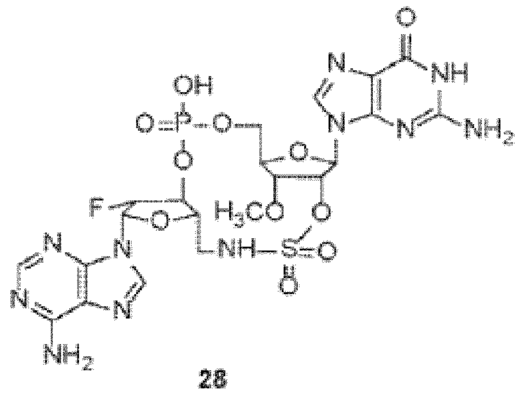

28

,

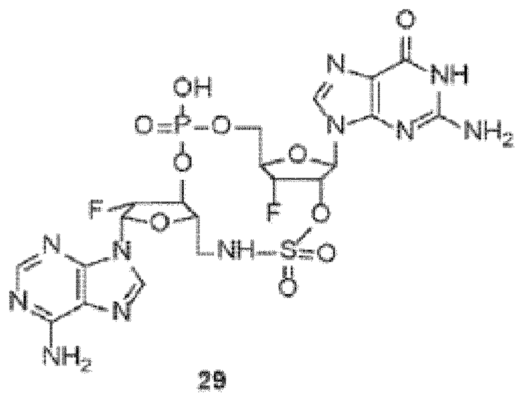

29

,

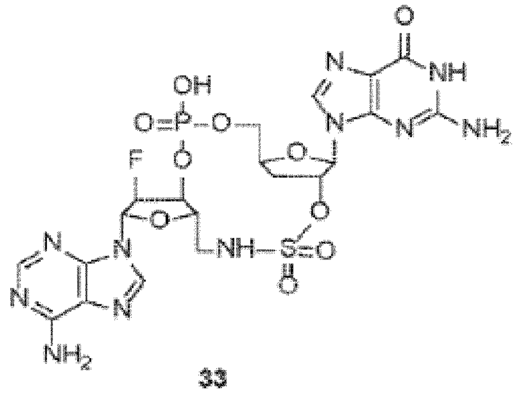

33

,

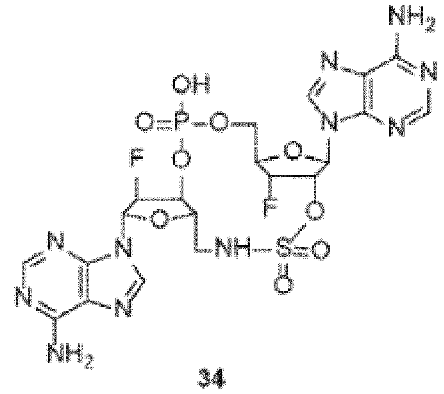

34

,

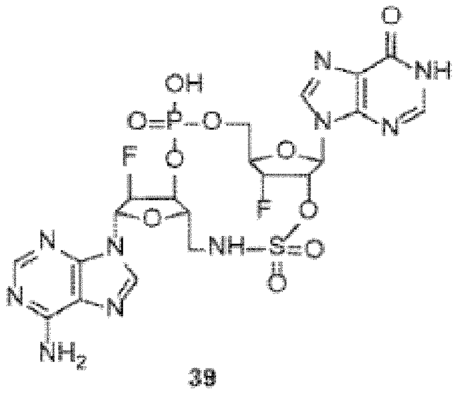

39

,

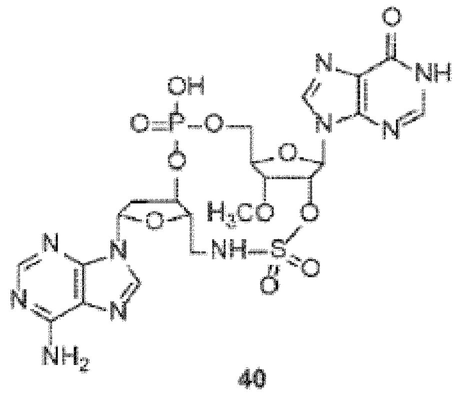

40

,

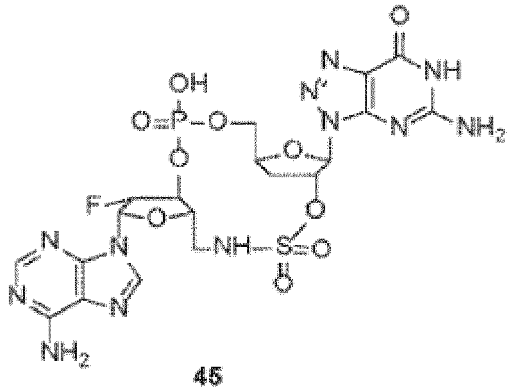

45

,

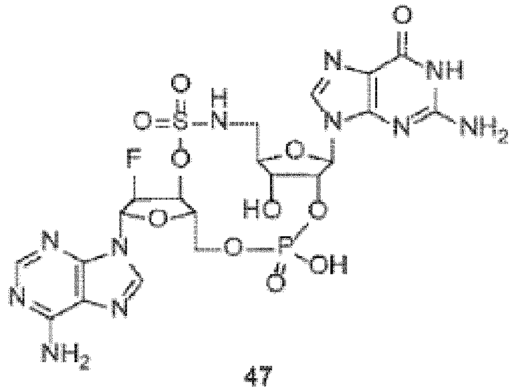

47

,

et

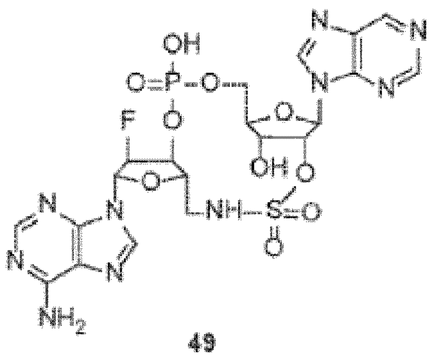

49

.

ou une forme de sel pharmaceutiquement acceptable correspondante.

**2.** Composé selon la revendication 1 choisi dans le groupe constitué par

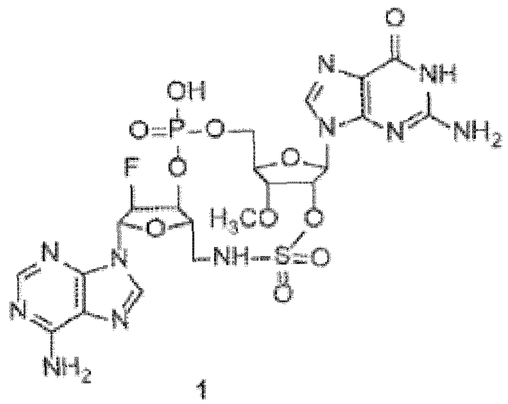

1

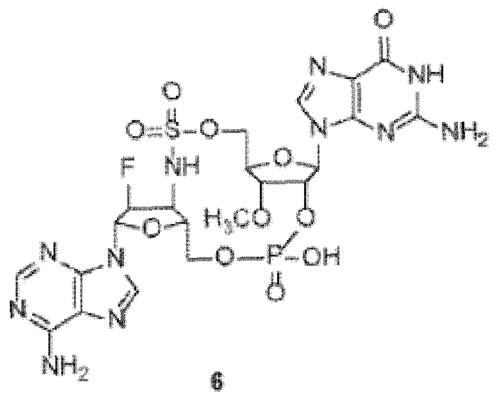

6

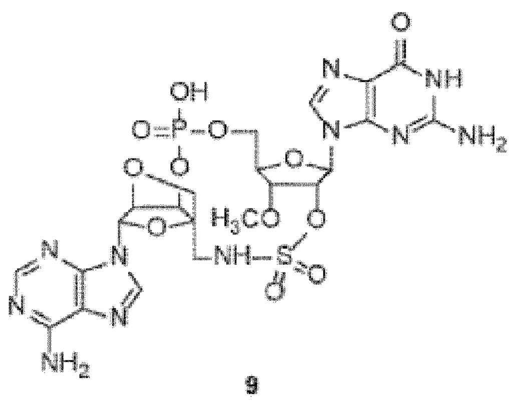

9

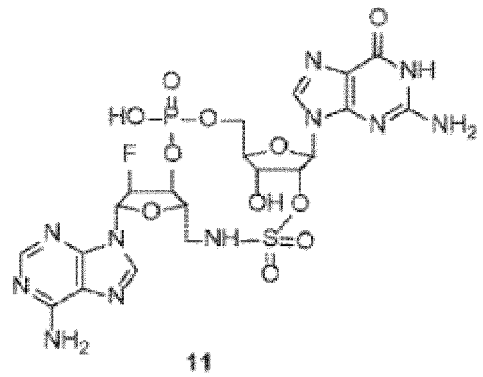

11

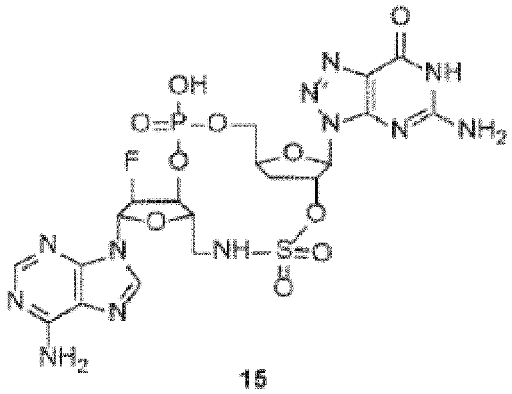

15

,

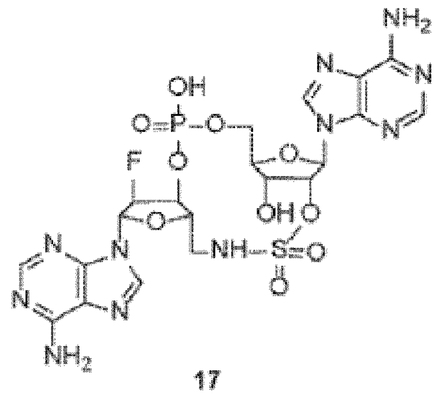

17

,

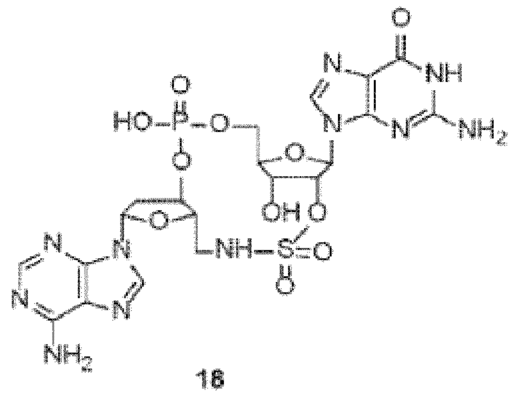

18

,

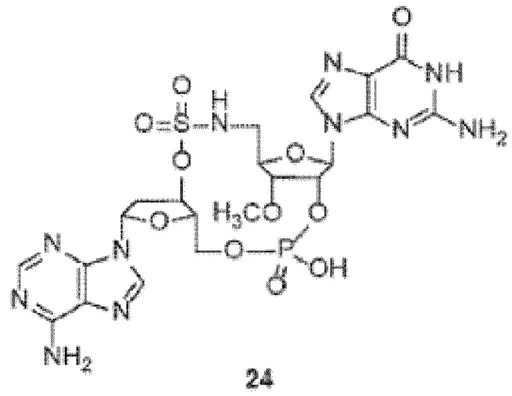

24

,

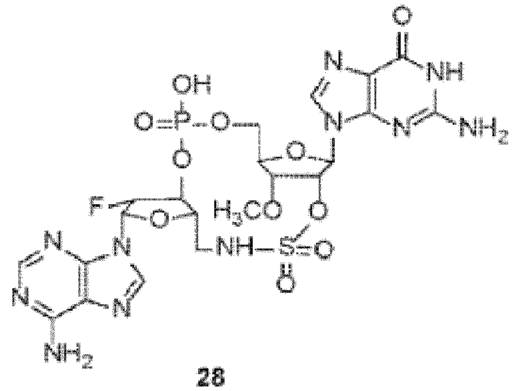

28

, et

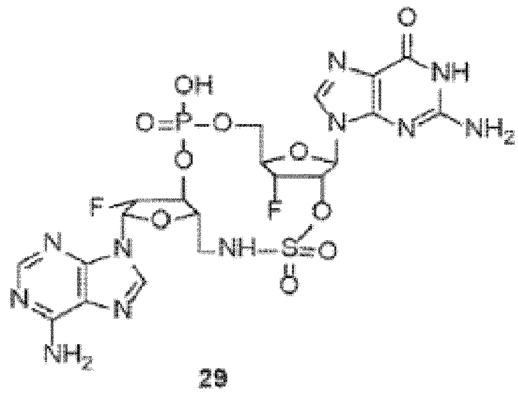

29

,

ou une forme de sel pharmaceutiquement acceptable correspondante.

3. Composition pharmaceutique comprenant un composé selon la revendication 1 ou la revendication 2 et l'un parmi un support pharmaceutiquement acceptable, un excipient pharmaceutiquement acceptable et un diluant pharmaceutiquement acceptable.

4. Composition pharmaceutique selon la revendication 3, la composition étant une forme de dosage orale solide.

5. Composition pharmaceutique selon la revendication 3, la composition étant un sirop, un élixir ou une suspension.

6. Composé selon la revendication 1 ou 2 pour une utilisation dans le traitement d'une maladie, d'un syndrome ou d'une affection modulé(e) par STING.

7. Composé selon la revendication 1 ou 2 pour une utilisation dans le traitement d'une maladie, d'un syndrome ou d'une affection, ladite maladie, ledit syndrome ou ladite affection étant affecté(e) par l'agonisme de STING.

8. Composé pour une utilisation selon la revendication 6 ou la revendication 7, ladite maladie, ledit syndrome ou ladite affection étant un cancer.

9. Composé pour une utilisation selon la revendication 8, ledit cancer étant un mélanome, un cancer du côlon, un cancer du sein, un cancer de la prostate, un cancer du poumon ou un fibrosarcome.

10. Composé pour une utilisation selon la revendication 7, ladite maladie, ledit syndrome ou ladite affection étant une infection virale.

11. Composé pour une utilisation selon la revendication 10, l'infection virale étant l'hépatite B.

12. Composé selon la revendication 1 ou la revendication 2, et un virus oncolytique ou un vaccin anticancéreux pour une utilisation dans le traitement d'une maladie, d'un syndrome, d'une affection ou d'un trouble, ladite maladie, ledit syndrome, ladite affection ou ledit trouble étant affecté(e) par l'agonisme de STING.

13. Composé pour une utilisation selon la revendication 12, le vaccin anticancéreux étant un vaccin antigénique, un vaccin à cellules entières, un vaccin activateur de cellules dendritiques, un vaccin à ADN, un vaccin du bacille de Calmette-Guérin, Sipuleucel-T (Provenge), Talimogene laherparepvec (T-Vec ; Imlygic™), un vaccin à base de virus oncolytique ou un vaccin à base d'adénovirus.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62599111
- WO 2017161349 A
- WO 2020016782 A1
- WO 2013019906 A
- WO 2010077634 A1
- US 8383796 B
- WO 2010027827 A
- WO 2011066342 A
- WO O1002369 A
- WO 02068470 A
- WO 02010192 A


### Non-patent literature cited in the description


- **ZHONG B et al.** The Adaptor Protein MITA Links Virus-Sensing Receptors to IRF3 Transcription Factor Activation. *Immunity,* 2008, vol. 29, 538-550
- **LIU S et al.** Phosphorylation of innate immune adaptor proteins MAVS, STING, and TRIF induces IRF3 activation. *Science,* 2015, 2630-2637
- **CHEN H et al.** Activation of STAT6 by STING Is Critical for Antiviral Innate Immunity. *Cell,* 2011, vol. 14, 433-446
- **CORRALES, L. et al.** Direct activation of STING in the tumor microenvironment leads to potent and systemic tumor regression and immunity. *Cell Reports,* 2015, vol. 11, 1-13
- **KONNO, H. et al.** Cyclic dinucleotides trigger ULK1 (ATG1) phosphorylation of STING to prevent sustained innate immune signaling. *Cell,* 2013, vol. 155, 688-698
- **SUN, L. et al.** Cyclic GMP-AMP Synthase Is a Cytosolic DNA Sensor That Activates the Type I Interferon Pathway. *Science,* 2013, vol. 339, 786-791
- **BHAT N ; FITZGERALD KA.** Recognition of Cytosolic DNA by cGAS and other STING-dependent sensors. *Eur J Immunol,* March 2014, vol. 44 (3), 634-40
- **ZHANG X et al.** Cyclic GMP-AMP Containing Mixed Phosphodiester Linkages Is An Endogenous High-Affinity Ligand for STING. *Molecular Cell,* 2013, vol. 51, 226-235
- **DANILCHANKA, O ; MEKALANOS, JJ.** Cyclic Dinucleotides and the Innate Immune Response. *Cell,* 2013, vol. 154, 962-970
- **LIU S-Y et al.** Systematic identification of type I and type II interferon-induced antiviral factors. *Proc. Natl. Acad. Sci.,* 2012, vol. 109, 4239-4244
- **J.F.W. MCOMIE.** Protective Groups in Organic Chemistry. Plenum Press, 1973
- **T.W. GREENE ; P.G.M. WUTS.** Protective Groups in Organic Synthesis. John Wiley & Sons, 1991
- **T.W. GREENE ; P.G.M. WUTS.** Protective Groups in Organic Synthesis. John Wiley & Sons, 1999
- **VAN DER JEUGHT et al.** *Oncotarget,* 2015, vol. 6 (3), 1359-1381
- **MARABELLE, A. et al.** *Clinical Cancer Research,* 2014, vol. 20 (7), 1747-1756
- **HOLFORD ; SCHEINER.** *Clin. Pharmacokinet,* vol. 6, 429-453
- **LOEWE ; MUISCHNEK.** *Arch. Exp. Pathol Pharmacol.,* 1926, vol. 114, 313-326
- **CHOU ; TALALAY.** *Adv. Enzyme Regul.,* 1984, vol. 22, 27-55
- **DUBENSKY et al.** *Therapeutic Advances in Vaccines,* 2013, vol. 1, 131-143
- *CHEMICAL ABSTRACTS,* 153186-10-8
- *CHEMICAL ABSTRACTS,* 160107-07-3
- *J. Chem. Soc., Perkin Trans.,* 2002, vol. 1, 485-495
- *CHEMICAL ABSTRACTS,* 103285-33-2
- *CHEMICAL ABSTRACTS,* 2241580-02-7
- *CHEMICAL ABSTRACTS,* 2086765-82-2
- *CHEMICAL ABSTRACTS,* 129054-67-7
- *CHEMICAL ABSTRACTS,* 117517-20-1
- *CHEMICAL ABSTRACTS,* 5399-87-1
- *CHEMICAL ABSTRACTS,* 847648-20-8
- *CHEMICAL ABSTRACTS,* 144924-99-2
- *CHEMICAL ABSTRACTS,* 10300-21-7
- *CHEMICAL ABSTRACTS,* 170871-87-1
- *CHEMICAL ABSTRACTS,* 1951424-83-1
- *CHEMICAL ABSTRACTS,* 3680-69-1
- *CHEMICAL ABSTRACTS,* 195375-63-4
- *CHEMICAL ABSTRACTS,* 179479-04-0

- *CHEMICAL ABSTRACTS,* 174171-97-2
- *CHEMICAL ABSTRACTS,* 1834500-50-3
- *CHEMICAL ABSTRACTS,* 136834-20-3
- *CHEMICAL ABSTRACTS,* 85109-99-5
- *CHEMICAL ABSTRACTS,* 75479-64-0
- **YI et al.** Single Nucleotide Polymorphisms of Human STING can affect innate immune response to cyclic dinucleotides. *PLOS ONE,* 2013, vol. 8 (10), e77846
- **BECKETT et al.** A minimal peptide substrate in biotin holoenzyme synthetase-catalyzed biotinylation. *Protein Science,* 1999, vol. 8, 921-929